(19)

Europäisches
Patentamt
European
Patent Office
Office européen
des brevets

(11)  **EP 3 750 911 A1**

(12)  **EUROPEAN PATENT APPLICATION**

(43) Date of publication:
**16.12.2020  Bulletin 2020/51**

(51) Int Cl.:
***C07K 14/005*** *(2006.01)*

(21) Application number: **19201176.5**

(22) Date of filing: **02.10.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.06.2019  US 201962861360 P**

(71) Applicants:
• **Westfälische Wilhelms-Universität Münster 48149 Münster (DE)**
• **Yeda Research and Development Co., Ltd. 76100 Rehovot (IL)**

(72) Inventors:
• **Mootz, Henning D.**
  **48161 Münster (DE)**
• **Bhagawati, Maniraj**
  **30167 Hannover (DE)**
• **Terhorst, Tobias**
  **48149 Münster (DE)**
• **Pietrokovski, Shmuel**
  **76100 Rehovot (IL)**

(74) Representative: **Viering, Jentschura & Partner mbB**
**Patent- und Rechtsanwälte**
**Hamborner Straße 53**
**40472 Düsseldorf (DE)**

(54)  **CYSTEINE-FREE INTEINS**

(57)  The present invention relates to an isolated polypeptide or to a composition of isolated polypeptides comprising (a) at least one first intein amino acid sequence (a1) and at least one first extein amino acid sequence (a2); wherein the at least one first intein amino acid sequence is an N-terminal sequence of a split intein or fragment thereof; wherein the at least one first extein sequence is fused to the N-terminus of the at least one first intein sequence and has a length of at least 1; and (b) at least one second intein amino acid sequence (b1) and at least one second extein sequence (b2); wherein the at least one second intein sequence is a C-terminal sequence of a split intein or fragment thereof; wherein the at least one second extein sequence is fused to the C-terminus of the at least one second intein sequence and has a length of at least 1; wherein the catalytic motifs of the at least one first intein amino acid sequence and/or of the at least one second intein amino acid sequence are free of cysteine residues; wherein the first and second intein amino acid sequence specifically interact with each other. Additionally, the invention relates to at least one isolated nucleic acid molecule comprising at least one nucleotide sequence encoding an isolated polypeptide according to the invention or a homolog, variant or complement thereof. Finally, the invention pertains to a method for modifying amino acid sequences or to a use of said isolated polypeptide(s) or said at least one isolated nucleic acid molecule.

EP 3 750 911 A1

SEQUENCE LISTING

```
<110>  Westfaelische Wilhelms-Universitaet Muenster
       Yeda Research & Development Co. Ltd

<120>  Cysteine-free inteins

<130>  P76264

<160>  286

<170>  PatentIn version 3.5

<210>  1
<211>  120
<212>  PRT
<213>  T4-like bacteriophage of Aeromonas salmonicida

<400>  1

Ser Val Val Gly Asp Thr Ile Ile Asp Val Ser Gly Lys Lys Met Thr
1               5                   10                  15


Ile Ala Glu Phe Tyr Asp Ser Thr Pro Asp Val Phe Met Arg Arg Asn
            20                  25                  30


Asp Glu Ala Arg Asp Trp Val Lys Arg Val Gly Gly Lys Thr Ser Leu
            35                  40                  45


Ser Val Asn Thr Tyr Ser Gly Glu Val Glu Arg Lys Asn Ile Asn Tyr
        50                  55                  60


Ile Met Lys His Thr Val Lys Lys Arg Met Phe Lys Ile Lys Ala Gly
65                  70                  75                  80


Gly Lys Glu Val Ile Val Thr Ala Asp His Ser Val Met Val Lys Arg
                85                  90                  95


Asp Gly Lys Ile Ile Asp Val Lys Pro Thr Glu Met Lys Gln Thr Asp
            100                 105                 110


Arg Val Val Lys Trp Met Leu Thr
            115                 120


<210>  2
<211>  39
<212>  PRT
<213>  T4-like bacteriophage of Aeromonas salmonicida

<400>  2

Met Ile Glu Phe Ile Glu Phe Glu Ile Glu Asp Leu Gly Val Met Glu
1               5                   10                  15
```

```
Ile Asp Val Tyr Asp Ile Glu Val Asp Gly Asn His Asn Phe Phe Gly
            20                  25                  30

Asn Asp Ile Leu Val His Asn
            35


<210>  3
<211>  26
<212>  PRT
<213>  Artificial sequence

<220>
<223>  modified IntN (CLN) from T4-like bacteriophage of Aeromonas
       salmonicida

<400>  3

Ser Val Val Gly Asp Thr Ile Ile Asp Val Ser Gly Lys Lys Met Thr
1                   5                   10                  15

Ile Ala Glu Phe Tyr Asp Ser Thr Pro Asp
            20                  25


<210>  4
<211>  129
<212>  PRT
<213>  Artificial sequence

<220>
<223>  modified IntC (CLC) from T4-like bacteriophage of Aeromonas
       salmonicida

<400>  4

Glu Ala Arg Asp Trp Val Lys Arg Val Gly Gly Lys Thr Ser Leu Ser
1                   5                   10                  15

Val Asn Thr Tyr Ser Gly Glu Val Glu Arg Lys Asn Ile Asn Tyr Ile
            20                  25                  30

Met Lys His Thr Val Lys Lys Arg Met Phe Lys Ile Lys Ala Gly Gly
            35                  40                  45

Lys Glu Val Ile Val Thr Ala Asp His Ser Val Met Val Lys Arg Asp
     50                  55                  60

Gly Lys Ile Ile Asp Val Lys Pro Thr Glu Met Lys Gln Thr Asp Arg
65                  70                  75                  80

Val Val Lys Trp Met Leu Thr Gly Ser His Met Ile Glu Phe Ile Glu
                85                  90                  95

Phe Glu Ile Glu Asp Leu Gly Val Met Glu Ile Asp Val Tyr Asp Ile
```

57

100                         105                         110

Glu Val Asp Gly Asn His Asn Phe Phe Gly Asn Asp Ile Leu Val His
        115                     120                     125

Asn

<210>   5
<211>   120
<212>   PRT
<213>   Aeromonas phage Aes123

<400>   5

Ser Val Val Gly Asp Thr Ile Ile Asp Val Ser Gly Lys Lys Met Thr
1                   5                   10                  15

Ile Ala Glu Phe Tyr Asp Ser Thr Pro Asp Val Phe Met Arg Arg Asn
        20                      25                      30

Asp Glu Ala Arg Asp Trp Val Lys Arg Val Gly Gly Lys Thr Ser Leu
        35                      40                      45

Ser Val Asn Thr Tyr Ser Gly Glu Val Glu Arg Lys Asn Ile Asn Tyr
        50                      55                      60

Ile Met Lys His Thr Val Lys Lys Arg Met Phe Lys Ile Lys Ala Gly
65                      70                      75                      80

Gly Lys Glu Val Ile Val Thr Ala Asp His Ser Val Met Val Lys Arg
                85                      90                      95

Asp Gly Lys Ile Ile Asp Val Lys Pro Thr Glu Met Lys Gln Thr Asp
                100                     105                     110

Arg Val Val Lys Trp Met Leu Thr
        115                     120

<210>   6
<211>   39
<212>   PRT
<213>   Aeromonas phage Aes123

<400>   6

Met Ile Glu Phe Ile Glu Phe Glu Ile Glu Asp Leu Gly Val Met Glu
1                   5                   10                  15

Ile Asp Val Tyr Asp Ile Glu Val Asp Gly Asn His Asn Phe Phe Gly
        20                      25                      30

58

Asn Asp Ile Leu Val His Asn
35

<210> 7
<211> 120
<212> PRT
<213> Aeromonas phage Aes144

<400> 7

Ser Val Val Gly Asp Thr Ile Ile Asp Val Ser Gly Lys Lys Met Thr
1               5                   10                  15

Ile Ala Glu Phe Tyr Asp Ser Thr Pro Asp Val Phe Met Arg Arg Asn
            20                  25                  30

Asp Glu Ala Arg Asp Trp Val Lys Arg Val Gly Gly Lys Thr Ser Leu
            35                  40                  45

Ser Val Asn Thr Tyr Ser Gly Glu Val Glu Arg Lys Asn Ile Asn Tyr
        50                  55                  60

Ile Met Lys His Thr Val Lys Lys Arg Met Phe Lys Ile Lys Ala Gly
65                  70                  75                  80

Gly Lys Glu Val Ile Val Thr Ala Asp His Ser Val Met Val Lys Arg
                85                  90                  95

Asp Gly Lys Ile Ile Asp Val Lys Pro Thr Glu Met Lys Gln Thr Asp
            100                 105                 110

Arg Val Val Lys Trp Met Leu Thr
        115                 120

<210> 8
<211> 39
<212> PRT
<213> Aeromonas phage Aes144

<400> 8

Met Ile Glu Phe Ile Glu Phe Glu Ile Glu Asp Leu Gly Val Met Glu
1               5                   10                  15

Ile Asp Val Tyr Asp Ile Glu Val Asp Gly Asn His Asn Phe Phe Gly
            20                  25                  30

Asn Asp Ile Leu Val His Asn
            35

59

```
<210>   9
<211>   120
<212>   PRT
<213>   Aeromonas phage Aes508

<400>   9

Ser Val Val Gly Asp Thr Ile Ile Asp Val Ser Gly Lys Lys Met Thr
1               5                   10                  15


Ile Ala Glu Phe Tyr Asp Ser Thr Pro Asp Val Phe Met Arg Arg Asn
            20                  25                  30


Asp Glu Ala Arg Asp Trp Val Lys Arg Val Gly Gly Lys Thr Ser Leu
            35                  40                  45


Ser Val Asn Thr Tyr Ser Gly Glu Val Glu Arg Lys Asn Ile Asn Tyr
        50                  55                  60


Ile Met Lys His Thr Val Lys Lys Arg Met Phe Lys Ile Lys Ala Gly
65                  70                  75                  80


Gly Lys Glu Val Ile Val Thr Ala Asp His Ser Val Met Val Lys Arg
                85                  90                  95


Asp Gly Lys Ile Ile Asp Val Lys Pro Thr Glu Met Lys Gln Thr Asp
            100                 105                 110


Arg Val Val Lys Trp Met Leu Thr
            115                 120


<210>   10
<211>   39
<212>   PRT
<213>   Aeromonas phage Aes508

<400>   10

Met Ile Glu Phe Ile Glu Phe Glu Ile Glu Asp Leu Gly Val Met Glu
1               5                   10                  15


Ile Asp Val Tyr Asp Ile Glu Val Asp Gly Asn His Asn Phe Phe Gly
            20                  25                  30


Asn Asp Ile Leu Val His Asn
            35


<210>   11
<211>   120
<212>   PRT
<213>   Aeromonas phage Aes509
```

<400> 11

Ser Val Val Gly Asp Thr Ile Ile Asp Val Ser Gly Lys Lys Met Thr
1               5                   10                  15

Ile Ala Glu Phe Tyr Asp Ser Thr Pro Asp Val Phe Met Arg Arg Asn
            20                  25                  30

Asp Glu Ala Arg Asp Trp Val Lys Arg Val Gly Gly Lys Thr Ser Leu
        35                  40                  45

Ser Val Asn Thr Tyr Ser Gly Glu Val Glu Arg Lys Asn Ile Asn Tyr
    50                  55                  60

Ile Met Lys His Thr Val Lys Lys Arg Met Phe Lys Ile Lys Ala Gly
65                  70                  75                  80

Gly Lys Glu Val Ile Val Thr Ala Asp His Ser Val Met Val Lys Arg
                85                  90                  95

Asp Gly Lys Ile Ile Asp Val Lys Pro Thr Glu Met Lys Gln Thr Asp
                100                 105                 110

Arg Val Val Lys Trp Met Leu Thr
            115                 120

<210> 12
<211> 39
<212> PRT
<213> Aeromonas phage Aes509

<400> 12

Met Ile Glu Phe Ile Glu Phe Glu Ile Glu Asp Leu Gly Val Met Glu
1               5                   10                  15

Ile Asp Val Tyr Asp Ile Glu Val Asp Gly Asn His Asn Phe Phe Gly
            20                  25                  30

Asn Asp Ile Leu Val His Asn
            35

<210> 13
<211> 120
<212> PRT
<213> Aeromonas phage Aes512

<400> 13

Ser Val Val Gly Asp Thr Ile Ile Asp Val Ser Gly Lys Lys Met Thr
1               5                   10                  15

Ile Ala Glu Phe Tyr Asp Ser Thr Pro Asp Val Phe Met Arg Arg Asn
                20                      25                  30

Asp Glu Ala Arg Asp Trp Val Lys Arg Val Gly Gly Lys Thr Ser Leu
            35                  40                  45

Ser Val Asn Thr Tyr Ser Gly Glu Val Glu Arg Lys Asn Ile Asn Tyr
        50                  55                  60

Ile Met Lys His Thr Val Lys Lys Arg Met Phe Lys Ile Lys Ala Gly
65                  70                  75                      80

Gly Lys Glu Val Ile Val Thr Ala Asp His Ser Val Met Val Lys Arg
                85                  90                  95

Asp Gly Lys Ile Ile Asp Val Lys Pro Thr Glu Met Lys Gln Thr Asp
            100                 105                 110

Arg Val Val Lys Trp Met Leu Thr
            115             120

<210>  14
<211>  39
<212>  PRT
<213>  Aeromonas phage Aes512

<400>  14

Met Ile Glu Phe Ile Glu Phe Glu Ile Glu Asp Leu Gly Val Met Glu
1               5                   10                  15

Ile Asp Val Tyr Asp Ile Glu Val Asp Gly Asn His Asn Phe Phe Gly
                20                  25                  30

Asn Asp Ile Leu Val His Asn
            35

<210>  15
<211>  141
<212>  PRT
<213>  Aeromonas phage Aes516

<400>  15

Ser Val Val Gly Asp Thr Ile Ile Asp Val Ser Gly Lys Lys Met Thr
1               5                   10                  15

Ile Ala Glu Phe Tyr Asp Ser Thr Pro Asp Val Phe Met Arg Arg Asn
                20                  25                  30

Asp Glu Ala Arg Asp Trp Val Lys Arg Val Gly Gly Lys Thr Ser Leu
        35                  40                  45

Ser Val Asn Thr Tyr Ser Gly Glu Val Glu Arg Lys Asn Ile Asn Tyr
        50                  55                  60

Ile Met Lys His Thr Val Lys Lys Arg Met Phe Lys Ile Lys Ala Gly
65                  70                  75                  80

Gly Lys Glu Val Ile Val Thr Ala Asp His Ser Val Met Val Lys Arg
                85                  90                  95

Asp Gly Lys Ile Ile Asp Val Lys Pro Thr Glu Met Lys Gln Thr Asp
            100                 105                 110

Arg Val Val Val Trp Asp Asn Glu Tyr Leu Ala Asn Lys Asp Gly Thr
        115                 120                 125

Ile Asn Arg Ile Val Glu Glu Ile Tyr Asp Arg Val Tyr
    130                 135                 140

<210> 16
<211> 39
<212> PRT
<213> Aeromonas phage Aes516

<400> 16

Met Ile Glu Phe Ile Glu Phe Glu Ile Glu Asp Leu Gly Val Met Glu
1               5                   10                  15

Ile Asp Val Tyr Asp Ile Glu Val Asp Gly Asn His Asn Phe Phe Gly
            20                  25                  30

Asn Asp Ile Leu Val His Asn
            35

<210> 17
<211> 120
<212> PRT
<213> Artificial sequence

<220>
<223> modified IntN from Aeromonas phage Aes517

<220>
<221> misc_feature
<222> (74)..(74)
<223> Xaa can be any naturally occurring amino acid

<400> 17

Ser Val Val Gly Asp Thr Ile Ile Asp Val Ser Gly Lys Lys Met Thr
1               5                   10                  15

Ile Ala Glu Phe Tyr Asp Ser Thr Pro Asp Val Phe Met Arg Arg Asn
            20                  25                  30

Asp Glu Ala Arg Asp Trp Val Lys Arg Val Gly Gly Lys Thr Ser Leu
            35                  40                  45

Ser Val Asn Thr Tyr Ser Gly Glu Val Glu Arg Lys Asn Ile Asn Tyr
        50                  55                  60

Ile Met Lys His Thr Val Lys Lys Arg Xaa Phe Lys Ile Lys Ala Gly
65                  70                  75                  80

Gly Lys Glu Val Ile Val Thr Ala Asp His Ser Val Met Val Lys Arg
                85                  90                  95

Asp Gly Lys Ile Ile Asp Val Lys Pro Thr Glu Met Lys Gln Thr Asp
            100                 105                 110

Arg Val Val Lys Trp Met Leu Thr
            115                 120


<210>  18
<211>  39
<212>  PRT
<213>  Aeromonas phage Aes517

<400>  18

Met Ile Glu Phe Ile Glu Phe Glu Ile Glu Asp Leu Gly Val Met Glu
1               5                   10                  15

Ile Asp Val Tyr Asp Ile Glu Val Asp Gly Asn His Asn Phe Phe Gly
            20                  25                  30

Asn Asp Ile Leu Val His Asn
            35


<210>  19
<211>  126
<212>  PRT
<213>  Unknown

<220>
<223>  IntN from Russia Kulunda-steppe soda lake Tanatar-5 brine
       environmental genomics

<400>  19

Ser Val Ala His Asp Ser Leu Ile Arg Ile Ser Arg Asp Asn Gly Thr
1               5                   10              15

Val Gln Asn Thr Thr Ile Glu Glu Leu Phe Leu Gln Gly Asn Glu Tyr
            20                  25                  30

Trp Glu Ser Asn Gly Lys Glu Tyr Ser Leu Asn Ser Asp Ile Lys Ile
        35                  40                  45

Ala His Thr Gly Ser Ser Gly Val Leu Asn Phe Val Asn Tyr Asn Tyr
        50                  55                  60

Val Tyr Arg His Lys Val Lys Asn Lys Ala Arg Tyr Arg Val Thr Thr
65              70                  75                  80

Ser Asn Gly Lys Ser Val Val Val Thr Asp Asp His Ser Val Met Ile
                85                  90                  95

Met Gln Asp Gly Arg Leu Ile Glu Lys Lys Pro Ser Glu Ile Lys Gln
            100                 105                 110

Gly Asp Leu Val Ile Thr Ile Val Asp Ser Asp Thr Ser Thr
            115                 120                 125


<210> 20
<211> 43
<212> PRT
<213> Unknown

<220>
<223> IntC from Russia Kulunda-steppe soda lake Tanatar-5 brine
      environmental genomics

<400> 20

Met Asp Ala Lys Val Ser Glu Val Val Gly Val Glu Arg Leu Asp Asp
1               5                   10              15

Phe Asp Asp Glu Tyr Val Tyr Asp Ile Gly Val Ala Asn Asp Asp Pro
            20                  25                  30

Tyr Phe Phe Ala Asn Asp Ile Leu Val His Asn
        35                  40


<210> 21
<211> 123
<212> PRT
<213> Rattus norvegicus

<400> 21

Ser Gln Ser Ala Leu Thr Ile Asn Tyr Leu Asp Gln Glu Lys Met Thr


65

```
        1                 5                      10                      15


        Val Glu Asp Met Phe Asn Lys Leu Lys Tyr Glu Asn Asn Asp Val Val
                    20                  25                  30


        Leu Arg Val Ser Asn Gly Ser Glu Val Val Pro Val Lys Asn His Thr
                    35                  40                  45


        Thr Lys Thr Phe Ile Met Lys Glu Gly Val Ile Asp Arg Pro Leu Lys
                    50                  55                  60


        Tyr Ile Met Arg His Lys Val Thr Lys Ser Lys Trp Arg Leu Arg Thr
        65                  70                  75                  80


        Glu Ser Gly Lys Glu Ile Ile Val Thr Gly Asp His Ser Leu Met Val
                    85                  90                  95


        Leu Arg Asp Asn Glu Leu Ile Ser Leu Lys Pro Lys Asp Val Asn Pro
                    100                 105                 110


        Lys Thr Asp Lys Ile Ile Thr Ile Lys Asp Val
                    115                 120


        <210>  22
        <211>  42
        <212>  PRT
        <213>  Rattus norvegicus

        <400>  22

        Met Asn Tyr Asn Ile Glu Asn Ile Ala Val Ile Glu Gln Ile Glu Asp
        1                 5                      10                      15


        Phe Gln Asp Glu Tyr Val Tyr Asp Leu Glu Val Glu Asp Thr His Thr
                    20                  25                  30


        Phe Phe Gly Asn Asp Ile Leu Ile His Asn
                    35                  40


        <210>  23
        <211>  123
        <212>  PRT
        <213>  Rattus norvegicus

        <400>  23

        Ser Gln Ser Ala Leu Thr Ile Asn Tyr Leu Asp Gln Glu Lys Met Thr
        1                 5                      10                      15


        Val Glu Asp Met Phe Asn Lys Leu Lys Tyr Glu Asn Asn Asp Val Val
                    20                  25                  30
```

Leu Arg Val Ser Asn Gly Ser Glu Val Val Pro Val Lys Asn His Thr
35                  40                  45

Thr Lys Thr Phe Ile Met Lys Glu Gly Val Ile Asp Arg Pro Leu Lys
50                  55                  60

Tyr Ile Met Arg His Lys Val Thr Lys Ser Lys Trp Arg Leu Arg Thr
65                  70                  75                  80

Glu Ser Gly Lys Glu Ile Ile Val Thr Gly Asp His Ser Leu Met Val
85                  90                  95

Leu Arg Asp Asn Glu Leu Ile Ser Leu Lys Pro Lys Asp Val Asn Pro
100                 105                 110

Lys Thr Asp Lys Ile Ile Thr Ile Lys Asp Val
115                 120

<210>   24
<211>   42
<212>   PRT
<213>   Rattus norvegicus

<400>   24

Met Asn Tyr Asn Ile Glu Asn Ile Ala Val Ile Glu Gln Ile Glu Asp
1                   5                   10                  15

Phe Gln Asp Glu Tyr Val Tyr Asp Leu Glu Val Glu Asp Thr His Thr
20                  25                  30

Phe Phe Gly Asn Asp Ile Leu Ile His Asn
35                  40

<210>   25
<211>   122
<212>   PRT
<213>   Prevotella megaphage Lak-B4

<400>   25

Ser Gln Thr Ala Asp Thr Gln Val Val Ile Asp Asn Lys Val Phe Ser
1                   5                   10                  15

Met Glu Gly Phe Phe Thr Lys Ala Lys Tyr Glu Asn Asp Asp Val Val
20                  25                  30

Ile Lys Leu Gln Asn Gly Ser Glu Val Ile Pro Val His Asn His Asp
35                  40                  45

67

```
Thr Leu Ser Tyr Lys Asp His Asp Tyr Lys Thr Ile Thr Arg Pro Ile
    50                  55                  60

Asn Tyr Ile Met Arg His Lys Val Thr Lys Asp Lys Phe Arg Leu Lys
65                  70                  75                  80

Thr Lys Ser Gly Lys Glu Leu Ile Val Thr Gly Asp His Ser Ile Met
                85                  90                  95

Val Ile Arg Asn Asn Glu Leu Ile Ser Val Pro Ala Arg Glu Ile Lys
            100                 105                 110

Lys Ser Asp Lys Ile Ile Thr Leu Asp Arg
        115                 120
```

```
<210>  26
<211>  44
<212>  PRT
<213>  Prevotella megaphage Lak-B4

<400>  26
```

```
Met Asn Tyr Asn Ile Gln Val Glu Asp Ile Asp Val Ile Glu Gln Leu
1               5                   10                  15

Glu Asp Phe Asn Asp Glu Tyr Val Tyr Asp Ile Glu Val Asp Asp Thr
            20                  25                  30

His Thr Phe Phe Ala Asn Glu Ile Leu Ala His Asn
        35                  40
```

```
<210>  27
<211>  131
<212>  PRT
<213>  Prevotella megaphage Lak-C1

<400>  27
```

```
Ser Gln Leu Gly Ser Thr Gln Phe Arg Val Asp Asn Asn Ile Thr Thr
1               5                   10                  15

Met Glu Asp Phe Phe Ile Lys Ala Lys Tyr Glu Asn Asn Asp Val Val
            20                  25                  30

Ile Lys Leu Thr Asn Gly Ser Glu Ile Ile Pro Val His Asn His Met
        35                  40                  45

Thr Leu Ser Tyr Lys Asp His Asp Tyr Lys Thr Ser Glu Arg Pro Ile
    50                  55                  60
```

Asn Tyr Ile Met Arg His Lys Val Thr Lys Asp Lys Phe Arg Leu Lys
65                  70              75              80

Thr Lys Ser Gly Lys Glu Leu Ile Val Thr Gly Asp His Ser Ile Met
            85                  90                  95

Val Ile Arg Asp Asn Glu Leu Ile Ser Leu Pro Ala Arg Glu Ile Lys
        100                 105                 110

Lys Ser Asp Lys Ile Ile Thr Ile Ala Gly Asp Asn Phe Thr Lys Ala
        115                 120                 125

Gly Asp Lys
    130


<210> 28
<211> 44
<212> PRT
<213> Prevotella megaphage Lak-C1

<400> 28

Met Asn Tyr Asp Ile Gln Ile Glu Asp Ile Asp Val Ile Glu Gln Leu
1               5               10                  15

Glu Asp Phe Asn Asp Glu Tyr Val Tyr Asp Ile Glu Val Asp Asp Thr
        20                  25                  30

His Thr Phe Phe Ala Asn Asp Ile Leu Ala His Asn
        35                  40


<210> 29
<211> 122
<212> PRT
<213> Prevotella megaphage Lak-B1

<400> 29

Ser Gln Thr Ala Asp Thr Gln Val Val Ile Asp Asn Lys Val Phe Ser
1               5               10                  15

Met Glu Gly Phe Phe Thr Lys Ala Lys Tyr Glu Asn Asp Asp Val Val
        20                  25                  30

Ile Lys Leu Gln Asn Gly Ser Glu Val Ile Pro Val His Asn His Asp
        35                  40                  45

Thr Leu Ser Tyr Lys Asp His Asp Tyr Lys Thr Ile Thr Arg Pro Ile
    50                  55                  60

Asn Tyr Ile Met Arg His Lys Val Thr Lys Asp Lys Phe Arg Leu Lys

69

```
                 65                      70                      75                      80


          Thr Lys Ser Gly Lys Glu Leu Ile Val Thr Gly Asp His Ser Ile Met
                          85                  90                  95


          Val Ile Arg Asn Asn Glu Leu Ile Ser Val Pro Ala Arg Glu Ile Lys
                          100                 105                 110


          Lys Ser Asp Lys Ile Ile Thr Leu Asp Arg
                          115                 120


          <210>  30
          <211>  44
          <212>  PRT
          <213>  Prevotella megaphage Lak-B1

          <400>  30

          Met Asn Tyr Asn Ile Gln Val Glu Asp Ile Asp Val Ile Glu Gln Leu
          1               5                   10                  15


          Glu Asp Phe Asn Asp Glu Tyr Val Tyr Asp Ile Glu Val Asp Asp Thr
                          20                  25                  30


          His Thr Phe Phe Ala Asn Glu Ile Leu Ala His Asn
                          35                  40


          <210>  31
          <211>  124
          <212>  PRT
          <213>  Prevotella megaphage Lak-A2

          <400>  31

          Ser Thr Ser Trp Lys Ser Ser Ile Tyr Val Asp Ser Val Lys Leu Lys
          1               5                   10                  15


          Val Gln Asp Ala Phe Asn Lys Phe Lys Tyr Glu Asn Asn Asp Thr Val
                          20                  25                  30


          Leu Lys Leu Asn Asn Gly Gln Glu Ile Val Pro Val His Asn His Asn
                          35                  40                  45


          Ile Leu Ser Tyr Val Asp His Asp Ala Glu Ala Thr Tyr Arg Pro Ile
                          50                  55                  60


          Lys Tyr Ile Met Arg His Lys Val Tyr Asn Lys Ser Arg Phe Arg Ile
          65                  70                  75                  80


          Lys Ser Lys Ser Gly Lys Glu Leu Glu Val Thr Gly Asp His Ser Met
                          85                  90                  95
```

Met Ile Ile Arg Asn Asn Glu Leu Ile Thr Val Lys Ala Lys Asp Ile
            100                     105                 110

Leu Lys Thr Asp Lys Ile Ile Thr Ile Ala Gly Asp
            115                 120

<210> 32
<211> 44
<212> PRT
<213> Prevotella megaphage Lak-A2

<400> 32

Met Asn Tyr Asp Ile Gln Ile Glu Asp Ile Asp Val Ile Glu Gln Leu
1                   5                   10                  15

Glu Asp Phe Asn Asp Glu Tyr Val Tyr Asp Ile Glu Val Asp Asp Thr
            20                  25                  30

His Thr Phe Phe Ala Asn Asp Ile Leu Ala His Asn
            35                  40

<210> 33
<211> 122
<212> PRT
<213> Prevotella megaphage Lak-A1

<400> 33

Ser Gln Ile Gly Ser Thr Gln Phe Tyr Val Asp Asn Asn Ile Thr Thr
1                   5                   10                  15

Met Glu Asp Phe Phe Thr Lys Ala Lys Tyr Glu Asn Asn Asp Val Val
            20                  25                  30

Ile Lys Leu Gln Asn Gly Ser Glu Val Val Pro Val His Asn His Asn
            35                  40                  45

Thr Leu Thr Tyr Ile Asp His Asp Phe Lys Thr Ser Glu Arg Pro Ile
        50                  55                  60

Asn Tyr Ile Met Arg His Lys Val Thr Lys Asp Lys Phe Arg Leu Lys
65                  70                  75                  80

Thr Lys Ser Gly Lys Glu Leu Ile Val Thr Gly Asp His Ser Ile Met
                85                  90                  95

Val Ile Arg Asn Asn Glu Leu Ile Ser Leu Pro Ala Arg Glu Ile Lys
            100                 105                 110

```
Asn Thr Asp Lys Ile Ile Thr Leu Asp Lys
        115                 120
```

<210> 34
<211> 44
<212> PRT
<213> Prevotella megaphage Lak-A1

<400> 34

```
Met Asn Tyr Asp Ile Gln Ile Glu Asp Ile Asp Val Ile Glu Gln Leu
1               5                   10                  15

Glu Asp Phe Asn Asp Glu Tyr Val Tyr Asp Ile Glu Val Asp Asp Thr
            20                  25                  30

His Thr Phe Phe Ala Asn Asp Ile Leu Ala His Asn
            35                  40
```

<210> 35
<211> 117
<212> PRT
<213> Pseudomonas phage vB_PaeM_PA5oct

<400> 35

```
Ser Val Asp Gly Ser Thr Ile Leu Asn Thr Ser Leu Gly Lys Ile Thr
1               5                   10                  15

Ile Glu Glu Leu Phe Asn Val Ser Asp Lys His Val Val His Ala Glu
            20                  25                  30

Lys Glu Phe Ala Ser Asn Glu Asp Val Met Val Met Ser Trp Asp Asn
            35                  40                  45

Ser Ala Lys Gln Pro Tyr Met Gly His Ile Asn Tyr Val Tyr Arg His
        50                  55                  60

Glu Val Glu Lys Glu Leu Phe Glu Ile Glu Asp Asn Asn Gly Asn Lys
65                  70                  75                  80

Val Ile Val Thr Glu Asp His Ser Ile Met Val Ile Arg Asn Ala Glu
                85                  90                  95

Leu Leu Glu Val Lys Pro Ala Glu Leu Thr Asp Ser Asp Ile Ile Leu
            100                 105                 110

Ser Ile Val Tyr Glu
            115
```

```
<210>  36
<211>  85
<212>  PRT
<213>  Pseudomonas phage vB_PaeM_PA5oct

<400>  36

Met His Asn Leu Thr Lys His Leu Leu Gly Val Phe Ser Ala Lys Thr
1               5                   10                  15

Glu Asp Glu Glu Tyr Lys Ser Ala Lys Arg Ala Leu Glu Glu Leu Asn
            20                  25                  30

Lys Asn Ile Lys Glu Arg Asp Pro Asn Lys Phe Asn Val Ser Leu Gly
        35                  40                  45

Lys Val Ser Lys Val Thr Asn Leu Gly Lys Lys Lys Gln Tyr Val Tyr
    50                  55                  60

Asp Ile Gly Met Lys Asn Pro Asp Asn Pro Tyr Phe Phe Gly Asn Asn
65                  70                  75                  80

Ile Leu Val His Asn
                85


<210>  37
<211>  131
<212>  PRT
<213>  Unknown

<220>
<223>  IntN from megaphage from human gut metagenome Denmark fecal
       sample

<400>  37

Ser Gln Leu Gly Ser Thr Gln Phe Arg Val Asp Asn Asn Ile Thr Thr
1               5                   10                  15

Met Glu Asp Phe Phe Ile Lys Ala Lys Tyr Glu Asn Asn Asp Val Val
            20                  25                  30

Ile Lys Leu Thr Asn Gly Ser Glu Ile Ile Pro Val His Asn His Met
        35                  40                  45

Thr Leu Ser Tyr Lys Asp His Asp Tyr Lys Thr Ser Glu Met Pro Ile
    50                  55                  60

Asn Tyr Ile Met Arg His Lys Val Thr Lys Asp Lys Phe Arg Leu Lys
65                  70                  75                  80

Thr Lys Ser Gly Lys Glu Leu Ile Val Thr Gly Asp His Ser Ile Met
```

                          85                    90                    95


        Val Ile Arg Asp Asn Glu Leu Ile Ser Leu Pro Ala Arg Glu Ile Lys
                    100                   105                   110


        Lys Ser Asp Lys Ile Ile Thr Ile Ala Gly Asp Asn Phe Thr Lys Ala
                    115                   120                   125


        Glu Asp Lys
            130


        <210>   38
        <211>   44
        <212>   PRT
        <213>   Unknown

        <220>
        <223>   IntC from megaphage from human gut metagenome Denmark fecal
                sample

        <400>   38

        Met Asn Tyr Asp Ile Gln Ile Glu Asp Ile Asp Val Ile Glu Gln Leu
        1                   5                   10                  15


        Glu Asp Phe Asn Asp Glu Tyr Val Tyr Asp Ile Glu Val Asp Asp Thr
                    20                  25                  30


        His Thr Phe Phe Ala Asn Asp Ile Leu Ala His Asn
                35                  40


        <210>   39
        <211>   124
        <212>   PRT
        <213>   Unknown

        <220>
        <223>   IntN from megaphage from human gut metagenome Denmark fecal
                sample

        <400>   39

        Ser Thr Ser Trp Lys Ser Ser Ile Tyr Val Asp Ser Val Lys Leu Lys
        1                   5                   10                  15


        Val Gln Ala Ala Phe Asn Lys Phe Lys Tyr Glu Asn Asn Asp Thr Val
                    20                  25                  30


        Leu Lys Leu Asn Asn Gly Gln Glu Ile Val Pro Val His Asn His Asn
                35                  40                  45


        Ile Leu Ser Tyr Val Asp His Asp Ala Glu Ala Thr Tyr Arg Pro Ile
                50                  55                  60

```
Lys Tyr Ile Met Arg His Lys Val Tyr Asn Lys Ser Arg Phe Arg Ile
65              70              75                      80


Lys Thr Lys Ser Gly Lys Glu Leu Glu Val Thr Gly Asp His Ser Met
                85              90                      95


Met Ile Ile Arg Asn Asn Glu Leu Ile Thr Val Lys Ala Lys Asp Ile
                100             105             110


Leu Lys Thr Asp Lys Ile Ile Thr Ile Thr Gly Asp
        115             120
```

<210> 40
<211> 44
<212> PRT
<213> Unknown

<220>
<223>    IntC from megaphage from human gut metagenome Denmark fecal
         sample

<400> 40

```
Met Asn Tyr Asp Ile Gln Ile Glu Asp Ile Asp Val Ile Glu Gln Leu
1               5               10              15


Glu Asp Phe Asn Asp Glu Tyr Val Tyr Asp Ile Glu Val Asp Asp Thr
            20              25              30


His Thr Phe Phe Ala Asn Asp Ile Leu Ala His Asn
            35              40
```

<210> 41
<211> 131
<212> PRT
<213> Unknown

<220>
<223>    IntN from megaphage from human gut metagenome Tanzanian Hadza
         hunter-gatherer fecal sample

<400> 41

```
Ser Gln Leu Gly Ser Thr Gln Phe Arg Val Asp Asn Asp Ile Thr Thr
1               5               10              15


Met Glu Asp Phe Phe Val Lys Ala Lys Tyr Glu Asn Asn Asp Val Val
            20              25              30


Ile Lys Leu Gln Asn Gly Ser Glu Val Val Pro Val His Asn His Asn
        35              40                      45
```

```
Thr Leu Thr Tyr Lys Asp His Asp Tyr Lys Thr Ile Thr Arg Pro Ile
    50              55              60

Asn Tyr Ile Met Arg His Lys Val Ser Lys Asp Lys Phe Arg Leu Lys
65              70              75              80

Thr Lys Ser Gly Lys Glu Leu Ile Val Thr Gly Asp His Ser Ile Met
            85              90              95

Val Ile Arg Asn Asn Glu Leu Val Ser Val Ala Ala Arg Glu Ile Lys
            100             105             110

Lys Thr Asp Lys Ile Ile Thr Ile Ala Gly Asp Asn Phe Asn Lys Ala
        115             120             125

Gly Asp Lys
        130
```

```
<210>  42
<211>  44
<212>  PRT
<213>  Unknown

<220>
<223>  IntC from megaphage from human gut metagenome Tanzanian Hadza
       hunter-gatherer fecal sample

<400>  42
```

```
Met Asn Tyr Asp Ile Gln Ile Glu Asp Ile Asp Val Ile Glu Gln Leu
1               5               10              15

Glu Asp Phe Asn Asp Glu Tyr Val Tyr Asp Ile Glu Val Asp Asp Thr
            20              25              30

His Thr Phe Phe Ala Asn Asp Ile Leu Ala His Asn
        35              40
```

```
<210>  43
<211>  122
<212>  PRT
<213>  Unknown

<220>
<223>  IntN from megaphage from human gut metagenome Denmark fecal
       sample

<400>  43
```

```
Ser Gln Thr Ala Asp Thr Gln Leu Phe Ile Asp Asn Lys Glu Ile Ser
1               5               10              15
```

```
Met Glu Glu Phe Phe Arg Thr Ala Lys Tyr Glu Asn Asn Asp Val Val
            20                  25                  30

Ile Lys Leu Gln Asn Gly Ser Glu Val Val Pro Val His Asn His Asn
            35                  40                  45

Thr Leu Ser Tyr Lys Asp His Asp Tyr Lys Thr Ile Glu Arg Pro Ile
        50                  55                  60

Asn Tyr Ile Met Arg His Lys Val Thr Lys Asp Lys Phe Arg Leu Lys
65                  70                  75                  80

Thr Lys Ser Gly Lys Glu Leu Ile Val Thr Gly Asp His Ser Ile Met
                85                  90                  95

Val Ile Arg Asn Asn Glu Leu Val Ser Leu Pro Ala Arg Glu Ile Lys
                100                 105                 110

Asn Thr Asp Lys Ile Ile Thr Leu Asp Lys
        115                 120
```

```
<210>  44
<211>  44
<212>  PRT
<213>  Unknown

<220>
<223>  IntC from megaphage from human gut metagenome Denmark fecal
       sample

<400>  44
```

```
Met Asn Tyr Asp Ile Gln Ile Glu Asp Ile Asp Val Ile Glu Gln Leu
1                   5                   10                  15

Glu Asp Phe Asn Asp Glu Tyr Val Tyr Asp Ile Glu Val Asp Asp Thr
            20                  25                  30

His Thr Phe Phe Ala Asn Asp Ile Leu Ala His Asn
        35                  40
```

```
<210>  45
<211>  131
<212>  PRT
<213>  Unknown

<220>
<223>  IntN from megaphage from human gut metagenome Bangladeshi
       cholera-succession

<400>  45
```

```
Ser Gln Leu Gly Ser Thr Gln Phe Arg Val Asp Asn Asn Ile Thr Thr
```

```
        1                 5                 10                15


        Met Glu Asp Phe Phe Ile Lys Ala Lys Tyr Glu Asn Asn Asp Val Val
                    20                25                30


        Ile Lys Leu Thr Asn Gly Ser Glu Ile Ile Pro Val His Asn His Met
                    35                40                45


        Thr Leu Ser Tyr Lys Asp His Asp Tyr Lys Thr Ser Glu Arg Pro Ile
                50                55                60


        Asn Tyr Ile Met Arg His Lys Val Thr Lys Asp Lys Phe Arg Leu Lys
        65                70                75                80


        Thr Lys Ser Gly Lys Glu Leu Ile Val Thr Gly Asp His Ser Ile Met
                        85                90                95


        Val Ile Arg Asp Asn Glu Leu Ile Ser Leu Pro Ala Arg Glu Ile Lys
                    100               105               110


        Lys Ser Asp Lys Ile Ile Thr Ile Ala Gly Asp Asn Phe Thr Lys Ala
                    115               120               125


        Gly Asp Lys
                130


        <210>   46
        <211>   44
        <212>   PRT
        <213>   Unknown

        <220>
        <223>   IntC from megaphage from human gut metagenome Bangladeshi
                cholera-succession

        <400>   46

        Met Asn Tyr Asp Ile Gln Ile Glu Asp Ile Asp Val Ile Glu Gln Leu
        1                 5                 10                15


        Glu Asp Phe Asn Asp Glu Tyr Val Tyr Asp Ile Glu Val Asp Asp Thr
                    20                25                30


        His Thr Phe Phe Ala Asn Asp Ile Leu Ala His Asn
                    35                40


        <210>   47
        <211>   156
        <212>   PRT
        <213>   Unknown
```

<220>
<223>   Contiguous intein from sheep gut metagenome

<400>   47

Ser Ile Arg Gly Asp Ser Leu Leu Ser Ile Asn Asn Lys Thr Ile Ser
1               5                   10                  15

Ile Ser Asn Phe Phe Asn Tyr Ser Glu Gly Ser Ile Lys Thr Asn Gly
                20                  25                  30

Asp Glu Lys Tyr Ile Lys His Leu Ser Arg Asp Tyr Phe Thr Thr Thr
        35                  40                  45

Tyr Asp Asp Gly Asn Ile Ile Glu Thr Lys Val Asn Tyr Val Met Lys
        50                  55                  60

His Lys Thr Lys Lys Arg Met Tyr Lys Leu Lys Val Val Asn Lys Glu
65                  70                  75                  80

Ile Val Val Thr Glu Asp His Ser Ile Met Ile Glu Arg Asp Asn Asn
                85                  90                  95

Leu Ile Glu Gly Ser Val Lys Asp Leu His Ser Asn Asp Lys Ile Ile
                100                 105                 110

Val Tyr Asn Lys Asn Ala Val Ile Lys Ser Glu Asn Trp Gln Val Glu
        115                 120                 125

Asp Leu Gly Ile Ile Glu Asp Tyr Val Tyr Asp Ile Glu Thr Glu Asn
        130                 135                 140

His Met Phe Phe Gly Asn Asp Ile Leu Val His Asn
145                 150                 155

<210>   48
<211>   115
<212>   PRT
<213>   Artificial sequence

<220>
<223>   modified IntN from Campylobacter phage CP21

<220>
<221>   misc_feature
<222>   (43)..(43)
<223>   Xaa can be any naturally occurring amino acid

<400>   48

Ser Val Val Gly Asp Ser Ile Ile Lys Val Asn Gly Lys Asn Ile Lys
1               5                   10                  15

Ile Glu Asp Phe Tyr Asp Ser Ile Lys Val Asp Pro Ile Val Thr Lys
20              25              30

Ser Gly Asn Asn Val Lys Leu Val Asp Asn Xaa Phe Thr Glu Ser Val
35              40              45

Asn Lys Asn Leu Gln Ile Glu Thr Lys Lys Ile Asn Tyr Ile Met Lys
50              55              60

His Lys Val Lys Lys Glu Phe Phe Lys Ile Lys Val Asn Asn Lys Glu
65              70              75              80

Val Val Val Thr Glu Asp His Ser Ile Met Val Leu Arg Asn Ser Glu
85              90              95

Leu Ile Glu Val Lys Pro Arg Asp Ile Lys Asn Gly Asp Leu Ile Ile
100             105             110

Leu Asn Asp
115

<210> 49
<211> 39
<212> PRT
<213> Campylobacter phage CP21

<400> 49

Met Ile Val Thr Glu Asn Phe Gln Val Glu Ser Leu Gly Ile Gln Glu
1           5               10              15

Leu Asp Val Tyr Asp Ile Glu Val Asp Ser Asn His Asn Phe Phe Ala
20              25              30

Asn Asp Ile Leu Val His Asn
35

<210> 50
<211> 345
<212> PRT
<213> Artificial sequence

<220>
<223> modified contiguous intein from Natronorubrum bangense JCM 10635

<220>
<221> misc_feature
<222> (120)..(120)
<223> Xaa can be any naturally occurring amino acid

<220>
<221>   misc_feature
<222>   (211)..(211)
<223>   Xaa can be any naturally occurring amino acid

<220>
<221>   misc_feature
<222>   (300)..(300)
<223>   Xaa can be any naturally occurring amino acid

<400>   50

Ser Val Pro Met Asn Glu Pro Ile Leu Ile Arg Asp Glu Asn Gly Ser
1               5                   10                  15


Ile Asp Ile Val Glu Ile Gln Glu Leu Asp Gly Arg Asp Gly Asp Val
            20                  25                  30


Glu Val Trp Thr Glu Lys Gly Phe Thr Arg Val Lys Arg Val Ile Arg
            35                  40                  45


Lys Pro Asn Arg Lys Lys Leu Tyr Thr Ile Arg Thr Lys Lys Gly Val
        50                  55                  60


Val His Ala Thr Glu Asp His Ser Leu Val Arg Ala Asp Gly Ser Glu
65                  70                  75                  80


Val Glu Pro Gly Glu Leu Gln Glu Gly Glu Ser Leu Leu His Arg Asn
                85                  90                  95


Val Ser Asp Ala Ser Thr Asp Val Gln Thr Asp Leu Ser Leu Asp Arg
                100                 105                 110


Ala Trp Leu Tyr Gly Phe Phe Xaa Gly Asp Gly Ser Ser Gly Asp Tyr
            115                 120                 125


Ala Tyr Asp His Pro Lys Asn Thr Asp Trp Asp Thr Arg Lys Thr Ser
        130                 135                 140


Trp Ser Leu Asn Asn Asn Asn Arg Glu Leu Leu Gln Arg Ala Ala Val
145                 150                 155                 160


Ala Leu Ser Lys Glu Phe Gly Val Asn Ser Arg Ile Asn Glu Thr Leu
                165                 170                 175


Glu Ser Ser Gly Thr Tyr Lys Leu Gln Pro Ser Asn Asn Gly Lys Arg
            180                 185                 190


Gly Ala Gly Ser Asn Gly Met Leu Pro Ser Leu Val Lys His Phe Asn
        195                 200                 205

```
Glu Thr Xaa Tyr Thr Pro Ser Arg Gln Lys Arg Val Pro Gln Asp Val
    210              215              220

Leu Asn Gly Asp Thr Gln Ala Ile Gln Ala Phe Leu Asp Gly Tyr Met
225              230              235                  240

Ala Ala Asp Gly His Val Gly Ser Arg Tyr Ser Lys Arg Phe His Glu
            245              250                  255

Ala Asp Thr Arg His Gln Pro Leu Ala Ser Gly Leu Val Phe Leu Leu
        260              265              270

Gln Arg Ile Gly Tyr Thr Phe Asn Ile Asn Val Arg Gln Val Glu Arg
        275              280              285

Asp Gly Gly Val Thr Glu Tyr Tyr Lys Leu Arg Xaa Gln Thr Ser His
    290              295              300

Arg Gly Asp Pro Asn Glu Val Lys Lys Ile Val Asp Tyr Glu Tyr Asp
305              310              315                  320

Gly Glu Tyr Val Tyr Asp Leu Glu Thr Glu Asn His His Phe His Ala
            325              330                  335

Gly Ala Gly Asn Ile Ile Val His Asn
        340              345

<210>  51
<211>  138
<212>  PRT
<213>  Unknown

<220>
<223>  IntN from Russia Kulunda-steppe soda lake Tanatar-5 brine
       environmental genomics

<400>  51

Ser Val Val Gly Asn Ser Val Ile Ser Val Asn Gly Lys Lys Ile Asn
1               5               10                  15

Ile Glu Asp Tyr Tyr Asp Arg Ile Asp Asn Asn Phe Ile Lys Asn Asp
        20              25              30

Gln Phe Asn Asp Asp Tyr Val Lys Val Val Asp Asn Gly Asp Thr Thr
        35              40              45

Gln Ser Ile Asn Lys Asp Gly Lys Leu Glu Asn Lys Pro Ile Asn Tyr
    50              55              60
```

```
Ile Met Lys His Arg Val Arg Lys Glu Met Phe Arg Ile Asp Asp Ser
65                  70                  75                  80


Ser Gly Asn Ser Val Ile Val Thr Glu Asp His Ser Val Ile Val Arg
                85                  90                  95


Asp Lys Lys Thr Lys Glu Ile Leu Asp Val Lys Pro Lys Glu Leu Asn
            100                 105                 110


Pro Lys Lys His Glu Ile Ile Asn Ile Ile Ala Asn Asp Thr Asp Ser
            115                 120                 125


Gly Gly Ile Tyr Gly Val Asp Asn Arg Lys
        130                 135
```

```
<210>  52
<211>  42
<212>  PRT
<213>  Artificial sequence

<220>
<223>  modified IntC from Russia Kulunda-steppe soda lake Tanatar-5
       brine environmental genomics


<220>
<221>  misc_feature
<222>  (39)..(39)
<223>  Xaa can be any naturally occurring amino acid

<400>  52
```

```
Met Ser Lys Ile Lys Phe Asp Asp Glu Phe Ser Val Lys Ser Leu Gly
1               5                   10                  15


Ile Val Asp Asn Tyr Val Tyr Asp Ile Glu Val Glu Asp Asn His Asn
                20                  25                  30


Phe Phe Ala Asn Asn Ile Xaa Val His Asn
        35                  40
```

```
<210>  53
<211>  120
<212>  PRT
<213>  Artificial sequence

<220>
<223>  modified IntN from oil-polluted sediment collected from 3
       locations (0.5 km, 0.7 km and 0.9 km) around wellhead MC252 after
       the Deepwater Horizon oil spill


<220>
<221>  misc_feature
```

<222> (57)..(57)
<223> Xaa can be any naturally occurring amino acid

<400> 53

Ser Val Ala Phe Asn Ser Ile Ile Glu Ile Asp Gly Ile Lys Asp Thr
1               5               10                  15

Ile Glu Ser Trp Phe Asn Lys Leu Ala Glu Glu His Gly Arg His Val
            20              25                  30

Asp Gly Glu Lys Glu Phe Thr Lys Ile Ser Ser Leu Asp Leu His Thr
        35              40                  45

Pro Thr Tyr Asp Val Ala Tyr Asp Xaa Met Val Asp Lys Pro Leu Met
        50              55                  60

Thr Ile Tyr Arg His Lys Ile Glu Lys Lys Met Tyr Thr Val Thr Ser
65              70                  75                  80

Val Asp Gly His Ser Val Thr Thr Thr Ala Asp His Ser Leu Met Val
            85              90                  95

Met Arg Gly Gly Asn Ile Ile Glu Ile Ile Pro Thr Asp Ile Leu Ser
        100             105                 110

Gly Asp Gln Leu Val Ile Phe Glu
        115             120


<210> 54
<211> 46
<212> PRT
<213> Unknown

<220>
<223> IntC from oil-polluted sediment collected from 3 locations (0.5
      km, 0.7 km and 0.9 km) around wellhead MC252 after the Deepwater
      Horizon oil spill

<400> 54

Met His Glu Arg Lys Tyr Lys Leu Val Asp Ile Ala Lys Val Glu Glu
1               5               10                  15

Val Lys Tyr Thr Asp Glu Tyr Val Tyr Asp Val Val Met Phe Glu Pro
            20              25                  30

Glu Ser Pro Tyr Phe Val Ala Asn Asp Ile Leu Val His Asn
        35              40                  45


<210> 55
<211> 116

```
<212>   PRT
<213>   Artificial sequence

<220>
<223>   modified IntN from oil-polluted sediment collected from 3
        locations (0.5 km, 0.7 km and 0.9 km) around wellhead MC252 after
        the Deepwater Horizon oil spill


<220>
<221>   misc_feature
<222>   (46)..(46)
<223>   Xaa can be any naturally occurring amino acid

<400>   55

Ser Val Val Gly Asp Thr Ser Ile Tyr Ile Asp Gly Asn Pro Ile Lys
1                   5                   10                  15


Ile Glu Asp Leu Tyr Asp Lys Thr Gln Gly Glu Leu Ile Glu Arg Gly
            20                  25                  30


Asp Tyr Asp Phe Ile Lys Lys Pro Asn Gln Asn Ile Leu Xaa Arg Ala
            35                  40                  45


Phe Asp Thr Lys Lys Gln Arg Val Val Glu Gln Lys Val Asn Tyr Ile
        50                  55                  60


Met Lys His Lys Val Lys Lys Lys Met Phe Lys Ile Lys Tyr Lys Gly
65                  70                  75                  80


Lys Glu Val Lys Val Thr Gln Asp His Ser Val Ile Ile Gln Arg Glu
                85                  90                  95


Asp Leu Phe Ile Ser Val Thr Pro Glu Gln Ile Lys Lys Gly Asp Lys
            100                 105                 110


Ile Ile Ile Ile
        115



<210>   56
<211>   42
<212>   PRT
<213>   Artificial sequence

<220>
<223>   modified IntC from oil-polluted sediment collected from 3
        locations (0.5 km, 0.7 km and 0.9 km) around wellhead MC252 after
        the Deepwater Horizon oil spill


<220>
<221>   misc_feature
<222>   (14)..(15)
<223>   Xaa can be any naturally occurring amino acid
```

<400> 56

```
Met Thr Ser Phe Glu Leu Ile Glu Asp Phe Glu Val Glu Xaa Xaa Gly
1               5                   10                  15

Glu Glu Glu Ile Trp Val Tyr Asp Ile Glu Val Glu Glu His His Asn
            20                  25                  30

Phe Phe Ala Asn Asp Ile Leu Val His Asn
        35                  40
```

<210> 57
<211> 156
<212> PRT
<213> Artificial sequence

<220>
<223> modified contiguous intein from Anaerobic digester Denmark WWTP
      Viborg metagenome

<220>
<221> misc_feature
<222> (111)..(111)
<223> Xaa can be any naturally occurring amino acid

<220>
<221> misc_feature
<222> (124)..(124)
<223> Xaa can be any naturally occurring amino acid

<400> 57

```
Ser Val His Ser Asp Ser Ile Val His Thr Asp Lys Gly Ser Ile Lys
1               5                   10                  15

Ile Glu Asp Trp Tyr Asn Lys Asn Lys Thr Asn Gly Gly Thr Thr Leu
            20                  25                  30

Glu Gly His Glu Ser Val Leu Thr Asn Asp Lys Ile Leu Asn Trp Asp
        35                  40                  45

Asn Glu Leu Tyr Phe Ala Pro Val Lys Arg Ile Ile Arg His Lys Val
        50                  55                  60

Thr Lys Pro Lys Trp Lys Ile Lys Thr Lys Ser Gly Lys Glu Ile Ile
65                  70                  75                  80

Ile Thr Asn Asp His Ser Leu Ile Val Phe Arg Asn Asn Glu Lys Leu
                85                  90                  95

Glu Ile Lys Pro Gln Gly Val Ile Tyr Gln Asp Lys Val Leu Xaa Leu
            100                 105                 110
```

```
Gly Thr Lys Phe Tyr Phe Asp Glu Ile Glu Ser Xaa Glu Glu Met Gly
        115             120             125
```

```
Thr Phe Asp Asn Glu Tyr Val Tyr Asp Val Glu Val Asp Asp Asp Thr
        130             135             140
```

```
His Thr Phe Ile Ala Asn Asp Ile Leu Val His Asn
145             150             155
```

<210> 58
<211> 121
<212> PRT
<213> Artificial sequence

<220>
<223> modified IntN from Tara Oceans expedition station TARA_022 Ionian
      sea (N=39.8386, E=17.4155) on 2009-11-16T08:16, depth of 3-7 m,
      size-fractionated (<-0.22 micrometres)

<220>
<221> misc_feature
<222> (29)..(29)
<223> Xaa can be any naturally occurring amino acid

<400> 58

```
Ser Val Asp Gly Lys Ser Thr Val Arg Ala Asn Gly Lys Asn Val Ser
1               5               10              15
```

```
Ile Glu Asn Leu Tyr Ser Glu Leu Glu Ser Asp Gly Xaa Asn Thr Ile
            20              25              30
```

```
Ile Thr Asp Phe Thr Gly Arg Gln Phe Val Phe Pro Lys Asp Val Lys
        35              40              45
```

```
Leu Pro Tyr Tyr Asn Glu Ser Asn Lys Lys Ile Glu Asn Gly Asn Val
    50              55              60
```

```
Glu Tyr Ile Glu Lys His Arg Val Lys Lys Lys Met Phe Lys Ile Arg
65              70              75              80
```

```
Ser Ser Asn Gly Lys Ser Val Ile Ile Thr Glu Asp His Ser Ile Met
            85              90              95
```

```
Val Met Arg Asn Lys Lys Leu Ile Lys Ile Thr Pro Asp Lys Leu Lys
        100             105             110
```

```
Lys Ser Asp Lys Leu Val Thr Leu Leu
    115             120
```

<210> 59
<211> 42
<212> PRT
<213> Artificial sequence

<220>
<223> modified IntC from Tara Oceans expedition station TARA_022 Ionian
sea (N=39.8386, E=17.4155) on 2009-11-16T08:16, depth of 3-7 m,
size-fractionated (<-0.22 micrometres)


<220>
<221> misc_feature
<222> (13)..(13)
<223> Xaa can be any naturally occurring amino acid

<220>
<221> misc_feature
<222> (29)..(29)
<223> Xaa can be any naturally occurring amino acid

<400> 59

```
Met Lys Tyr Lys Ile Glu Asp Ile Glu Glu Ile Glu Xaa Leu Gly Glu
1               5                   10                  15


Val Asp Gln Asp Val Tyr Asp Ile Gly Met Lys Asp Xaa Pro His Thr
            20                  25                  30


Phe Phe Ala Asn Asp Ile Leu Val His Asn
            35                  40
```


<210> 60
<211> 168
<212> PRT
<213> Artificial sequence

<220>
<223> modified contiguous intein from C-1 sludge sample from hydrolytic
chamber (first chamber) of Anaerobic Baffled Reactor in India


<220>
<221> misc_feature
<222> (2)..(2)
<223> Xaa can be any naturally occurring amino acid

<220>
<221> misc_feature
<222> (129)..(129)
<223> Xaa can be any naturally occurring amino acid

<400> 60

```
Ser Xaa Gln Asn Asp Thr Leu Ile Tyr Val Asp Asn Val Lys Met Ser
1               5                   10                  15


Ile Glu Asp Val Tyr Ile Met Met Lys Glu Glu Asn Phe Asp Ser Gly
```

|  |  | 20 |  |  |  | 25 |  |  |  | 30 |  |  |  |
|---|---|---|---|---|---|---|---|---|---|---|---|---|---|

Ile Val Thr Ser Asn Gly Ser Tyr Ile Ile Pro Asn Arg Phe Gly His
        35                40                45

Thr Ile Lys Thr Tyr Asp Glu Lys Thr Lys Lys Phe Ile Tyr Lys Pro
        50                55                60

Ile Lys Tyr Ile Met Arg His Lys Val Ser Lys Ala Lys Tyr Lys Ile
65                70                75                80

Thr Thr Ser Ser Gly Lys Ser Val Ile Val Thr Gly Asp His Ser Ile
                85                90                95

Met Ile Leu Arg Asn Asn Lys Leu Gln Ser Ile Lys Ala Lys Asp Ile
        100                105                110

Asn Ile Lys Thr Asp Lys Thr Ile Ser Val Ile Asp Asn Gln Leu Asp
        115                120                125

Xaa Ile Ile Glu Asp Ile Ala Ser Val Glu Gln Leu Glu Asp Phe Asn
        130                135                140

Asp Glu Tyr Val Tyr Asp Val Glu Val Glu Asp Thr His Thr Phe Val
145                150                155                160

Gly Asn Asp Ile Leu Leu His Asn
                165

```
<210>  61
<211>  116
<212>  PRT
<213>  Artificial sequence

<220>
<223>  modified IntN from Anaerobic digester Denmark WWTP Viborg
       metagenome


<220>
<221>  misc_feature
<222>  (47)..(47)
<223>  Xaa can be any naturally occurring amino acid

<400>  61
```

Ser Val Asp Gly Ser Thr Leu Ile Arg Thr Asn Ile Gly Ile Leu Pro
1                5                10                15

Ile Lys Ile Leu Phe Asp Lys Phe Ser Lys Lys Ser Lys Ile Lys Thr
        20                25                30

Glu Ser Tyr Gly His Glu Met Ile Asp Val Leu Asn Leu Glu Xaa Leu
        35                  40                  45

Thr Leu Lys Asp Asn Lys Ile Lys Met Gly Lys Ile Lys Arg Leu Ile
        50                  55                  60

Arg His Lys Thr Asn Lys Lys Met Tyr Lys Ile Arg Val Asn Gly Lys
65              70                  75                  80

Glu Ile Ile Thr Thr Glu Asp His Gly Ile Met Val Gln Arg Asp Gly
            85                  90                  95

Asn Leu Ile Arg Ile Ser Pro Lys Glu Ile Lys Lys Gly Asp Leu Met
            100                 105                 110

Val Asn Val Ile
            115


<210>  62
<211>  43
<212>  PRT
<213>  Unknown

<220>
<223>  IntC from Anaerobic digester Denmark WWTP Viborg metagenome

<400>  62

Met Lys Tyr Glu Leu Ser Pro Ile Glu Ser Ile Glu Val Val Asn Asp
1               5                   10                  15

Arg Phe Asp Tyr Val Tyr Asp Ile Glu Met Asp Asp Thr Thr Asp His
            20                  25                  30

Val Phe Phe Gly Asn Asp Ile Leu Val His Asn
            35                  40


<210>  63
<211>  128
<212>  PRT
<213>  Artificial sequence

<220>
<223>  modified IntN from Switchgrass-associated bovine rumen microbial
       communities from Urbana, Illinois, USA


<220>
<221>  misc_feature
<222>  (24)..(24)
<223>  Xaa can be any naturally occurring amino acid

<220>

```
<221>  misc_feature
<222>  (37)..(37)
<223>  Xaa can be any naturally occurring amino acid

<220>
<221>  misc_feature
<222>  (54)..(54)
<223>  Xaa can be any naturally occurring amino acid

<220>
<221>  misc_feature
<222>  (101)..(101)
<223>  Xaa can be any naturally occurring amino acid

<400>  63
```

Ser Val Ser Ala Asp Ser Asn Ile Lys Ile Ser Tyr Ser Asp Thr Gly
1               5                   10                  15

Ser Lys Lys Asp Ile Lys Val Xaa Asp Leu Phe Thr Glu Leu Lys Tyr
            20                  25                  30

Asn Asn Asn Asp Xaa Val Ile Ile Thr Gly Asn Gly Ser Glu Val Val
        35                  40                  45

Pro Val Lys Asp Ile Xaa Ala Gln Thr Tyr Ser Thr Glu Lys Asp Lys
        50                  55                  60

Val Ile Phe Arg Pro Val Asn Tyr Ile Met Arg His Lys Val Lys Lys
65                  70                  75                  80

Ser Arg Phe Arg Ile Thr Thr Glu Ser Gly Lys Gln Ile Ile Val Thr
                85                  90                  95

Gly Asp His Ser Xaa Met Val Val Arg Asp Asn Glu Leu Ile Ser Val
            100                 105                 110

Lys Ala Lys Asp Ile Lys Met Ser Asp Lys Ile Ile Thr Val Asp Asn
            115                 120                 125

```
<210>  64
<211>  49
<212>  PRT
<213>  Unknown

<220>
<223>  IntC from Switchgrass-associated bovine rumen microbial
       communities from Urbana, Illinois, USA

<400>  64
```

Met Val Met Asp Phe Lys Glu Thr Asn Tyr Lys Ile Glu Ser Ile Ala
1               5                   10                  15

Ala Ile Glu Gln Ile Glu Asp Phe Asp Glu Glu Tyr Val Tyr Asp Leu
20                              25                      30

Glu Ile Asp Asp Thr His Met Phe Phe Ala Asn Asp Ile Leu Val His
        35                      40                      45

Asn

<210>  65
<211>  129
<212>  PRT
<213>  Artificial sequence

<220>
<223>  modified IntN from Rattus norvegicus (Denmark: Riget) gut
       metagenome

<220>
<221>  misc_feature
<222>  (99)..(99)
<223>  Xaa can be any naturally occurring amino acid

<400>  65

Ser Val Ala Gly Asp Thr Lys Val Asp Ile Ser Ser Ala Asp Ile Lys
1               5                   10                  15

Lys Arg Ile Asp Ile Ala Asp Leu Phe Thr Lys Ala Lys Tyr Leu Asn
        20                      25                      30

Asp Asp His Val Leu Ser Val Ser Asn Gly Ser Glu Val Ile Pro Gly
        35                      40                      45

Asn Gly Ile Leu Ile Arg Ala Tyr Asp Lys Glu Leu Asp Met Ala Val
        50                      55                      60

Tyr Lys Pro Met Lys Tyr Val Met Arg His Lys Val Ser Lys Ala Arg
65                  70                      75                  80

Phe Arg Ile Lys Thr Glu Ser Gly Lys Glu Val Ile Val Thr Gly Asp
                85                      90                      95

His Ser Xaa Ile Val Leu Arg Asn Gly Glu Leu Ile Asp Ile Lys Ala
            100                     105                     110

Lys Asp Ile Asn Lys Glu Thr Asp Lys Ile Ile Thr Ile Asn Ser Lys
        115                     120                     125

Lys

<210> 66
<211> 47
<212> PRT
<213> Artificial sequence

<220>
<223> modified IntC from Rattus norvegicus (Denmark: Riget) gut metagenome

<220>
<221> misc_feature
<222> (35)..(35)
<223> Xaa can be any naturally occurring amino acid

<400> 66

Met Asp Phe Lys Glu Lys Asn Tyr Lys Ile Glu Ser Ile Ala Glu Ile
1               5                   10                  15

Glu Gln Leu Asp Asp Phe Glu Asp Glu Tyr Val Tyr Asp Val Glu Val
            20                  25                  30

Asp Asp Xaa His Asn Phe Phe Ala Asn Asp Val Leu Val His Asn
            35                  40                  45

<210> 67
<211> 115
<212> PRT
<213> Artificial sequence

<220>
<223> modified IntN from Campylobacter coli strain NC_C3306

<220>
<221> misc_feature
<222> (43)..(43)
<223> Xaa can be any naturally occurring amino acid

<400> 67

Ser Val Val Gly Asp Ser Ile Ile Lys Val Asn Gly Glu Asn Ile Lys
1               5                   10                  15

Ile Glu Asp Phe Tyr Asp Ser Ile Lys Val Asp Pro Ile Val Thr Lys
            20                  25                  30

Ser Gly Asn Asn Val Lys Leu Val Asp Asn Xaa Phe Thr Glu Ser Val
            35                  40                  45

Asn Lys Asn Leu Gln Ile Glu Thr Lys Lys Ile Asn Tyr Ile Met Lys
        50                  55                  60

His Arg Val Lys Lys Glu Phe Phe Lys Ile Lys Val Asn Asn Lys Glu
65                    70                75                      80

Val Ile Val Thr Glu Asp His Ser Ile Met Ile Leu Arg Asn Ser Glu
                85                    90                    95

Leu Ile Glu Val Lys Pro Arg Asp Ile Lys Thr Gly Asp Ser Ile Ile
                100                    105                110

Leu Asn Val
        115

<210> 68
<211> 39
<212> PRT
<213> Campylobacter coli strain NC_C3306

<400> 68

Met Ile Val Thr Glu Asn Phe Gln Val Glu Ser Leu Gly Ile Gln Glu
1                5                    10                    15

Leu Asp Val Tyr Asp Ile Glu Val Asp Ser Asn His Asn Phe Phe Ala
                20                    25                    30

Asn Asp Ile Leu Val His Asn
                35

<210> 69
<211> 153
<212> PRT
<213> Artificial sequence

<220>
<223> modified contiguous intein from Campylobacter jejuni SO-54

<220>
<221> misc_feature
<222> (43)..(43)
<223> Xaa can be any naturally occurring amino acid

<400> 69

Ser Val Val Gly Asp Ser Ile Ile Lys Val Asn Gly Lys Asn Ile Lys
1                5                    10                    15

Ile Glu Asp Phe Tyr Asp Ser Ile Lys Val Asp Pro Ile Val Thr Lys
                20                    25                    30

Ser Gly Asn Asn Val Lys Leu Val Asp Asn Xaa Phe Thr Glu Ser Val
        35                    40                    45

94

Asn Lys Asn Leu Gln Ile Glu Thr Lys Lys Ile Asn Tyr Ile Met Lys
            50                  55                  60

His Lys Val Lys Lys Glu Phe Phe Lys Ile Lys Val Asn Asn Lys Glu
65                  70                  75                  80

Val Val Val Thr Glu Asp His Ser Ile Met Val Leu Arg Asn Ser Glu
                85                  90                  95

Leu Ile Glu Val Lys Pro Arg Asp Ile Lys Ile Asp Asp Ile Leu Ile
            100                 105                 110

Leu Ile Asp Ser Lys Ser Ser Asp Phe Gln Val Glu Ser Leu Gly Ile
            115                 120                 125

Gln Glu Leu Asp Val Tyr Asp Ile Glu Val Asp Ser Asn His Asn Phe
    130                 135                 140

Phe Ala Asn Asp Ile Leu Val His Asn
145                 150

<210>  70
<211>  115
<212>  PRT
<213>  Artificial sequence

<220>
<223>  modified IntN from Campylobacter jejuni CFSAN038801

<220>
<221>  misc_feature
<222>  (43)..(43)
<223>  Xaa can be any naturally occurring amino acid

<400>  70

Ser Val Val Gly Asp Ser Ile Ile Lys Val Asn Gly Glu Asn Ile Lys
1               5                   10                  15

Ile Glu Asp Phe Tyr Asp Ser Ile Lys Val Asp Pro Ile Val Thr Lys
            20                  25                  30

Ser Gly Asn Asn Val Lys Leu Val Asp Asn Xaa Phe Thr Glu Ser Val
        35                  40                  45

Asn Lys Asn Leu Gln Ile Glu Thr Lys Lys Ile Asn Tyr Ile Met Lys
    50                  55                  60

His Arg Val Lys Lys Glu Phe Phe Lys Ile Lys Val Asn Asn Lys Glu
65                  70                  75                  80

95

Val Ile Val Thr Glu Asp His Ser Ile Met Val Leu Arg Asn Ser Glu
            85                  90                  95

Leu Ile Glu Val Lys Pro Arg Asp Ile Lys Thr Gly Asp Ser Ile Ile
            100                 105                 110

Leu Asn Asp
        115

<210> 71
<211> 39
<212> PRT
<213> Campylobacter jejuni CFSAN038801

<400> 71

Met Ile Val Thr Glu Asn Phe Gln Val Glu Ser Leu Gly Ile Gln Glu
1               5                   10                  15

Leu Asp Val Tyr Asp Ile Glu Val Asp Ser Asn His Asn Phe Phe Ala
            20                  25                  30

Asn Asp Ile Leu Val His Asn
        35

<210> 72
<211> 153
<212> PRT
<213> Artificial sequence

<220>
<223> modified contiguous intein from Campylobacter coli CFSAN038801

<220>
<221> misc_feature
<222> (43)..(43)
<223> Xaa can be any naturally occurring amino acid

<400> 72

Ser Val Val Gly Asp Ser Ile Ile Lys Val Asn Gly Glu Asn Ile Lys
1               5                   10                  15

Ile Glu Asp Phe Tyr Asp Ser Ile Lys Val Asp Pro Ile Val Thr Lys
            20                  25                  30

Ser Gly Asn Asn Val Lys Leu Val Asp Asn Xaa Phe Thr Glu Ser Val
        35                  40                  45

Asn Lys Asn Leu Gln Ile Glu Thr Lys Lys Ile Asn Tyr Ile Met Lys
        50                  55                  60

```
His Arg Val Lys Lys Glu Phe Phe Lys Ile Lys Val Asn Asn Lys Glu
65                  70              75                  80

Val Ile Val Thr Glu Asp His Ser Ile Met Val Leu Arg Asn Ser Glu
                85              90                  95

Leu Ile Glu Val Lys Pro Arg Asp Ile Lys Ile Asp Asp Ile Leu Ile
        100             105             110

Leu Ile Asp Ser Lys Ser Ser Asp Phe Gln Val Glu Ser Leu Gly Ile
        115             120             125

Gln Glu Leu Asp Val Tyr Asp Ile Glu Val Asp Ser Asn His Asn Phe
    130             135             140

Phe Ala Asn Asp Ile Leu Val His Asn
145             150
```

```
<210>  73
<211>  162
<212>  PRT
<213>  Artificial sequence

<220>
<223>  modified contiguous intein from Rhizobiales bacterium NORP22


<220>
<221>  misc_feature
<222>  (49)..(49)
<223>  Xaa can be any naturally occurring amino acid

<220>
<221>  misc_feature
<222>  (76)..(76)
<223>  Xaa can be any naturally occurring amino acid

<400>  73
```

```
Ser Val Ala Gly Asp Ser Leu Ile Tyr Val Asn Asp Lys Lys Ile Lys
1               5               10              15

Ile Glu Asp Tyr Tyr Asn Ser Leu Glu Asn Asn Phe Ile Leu Asn Asp
            20              25              30

Thr Phe Asn Glu Asn Tyr Val Lys Glu Val Ser Gly Asp Thr Thr Lys
        35              40              45

Xaa Tyr Ile Glu Gly Ile Lys Asn Lys Lys Ile Asn Tyr Ile Met Lys
    50              55              60

His Lys Val Ser Lys Lys Met Tyr Arg Ile Lys Xaa Asn Gly Asn Phe
```

97

```
              65                    70                    75                    80


              Val Asp Val Thr Glu Asp His Ser Val Ile Val Lys Asn Lys Lys Thr
                              85                    90                    95


              Lys Lys Ile Ser Ser Ile Lys Pro Lys Asn Leu Asn Pro Asn Leu His
                              100                   105                   110


              Ser Ile Ile Asn Ile Lys Thr Lys Lys Ile Asp Glu Leu Ile Thr Asp
                              115                   120                   125


              Asp Phe Glu Val Glu Tyr Leu Gly Ile Ile Glu Asn Tyr Val Tyr Asp
                  130                   135                   140


              Ile Glu Val Glu Glu Ala His Asn Phe Phe Ala Asn Asp Ile Leu Val
              145                   150                   155                   160


              His Asn



              <210>  74
              <211>  119
              <212>  PRT
              <213>  Artificial sequence

              <220>
              <223>  modified IntN from human gut metagenome Denmark fecal sample


              <220>
              <221>  misc_feature
              <222>  (24)..(24)
              <223>  Xaa can be any naturally occurring amino acid

              <220>
              <221>  misc_feature
              <222>  (117)..(117)
              <223>  Xaa can be any naturally occurring amino acid

              <400>  74

              Ser Leu Val Gly Ser Ser Ile Ile Ile Val Asn Gly Lys Lys Ile Lys
              1                   5                   10                    15


              Ile Glu Asp Tyr Tyr Asn Gln Xaa Asn Gly Ile Leu Ile Lys Asn Asp
                              20                    25                    30


              Ile Asn Asn Gln Asn Phe Ile Lys Glu Ile Asp Asn Asp Asp Lys Gly
                              35                    40                    45


              Leu Ser Tyr Asp Ile Asn Asn Gln Gln Ile Val Asn Asn Lys Ile Lys
                              50                    55                    60
```

Tyr Ile Lys Lys His Lys Val Lys Lys Glu Phe Phe Lys Ile Ser Tyr
65                  70                  75                  80


Lys Asp Lys Glu Val Ile Ile Thr Glu Asp His Ser Val Met Ile Glu
                    85                  90                  95


Arg Asn Asp Lys Ile Ile Glu Ile Lys Pro Arg Glu Ile Lys Gln Gly
                100                 105                 110


Asp Lys Ile Ile Xaa Ile Gln
            115


<210>  75
<211>  41
<212>  PRT
<213>  Unknown

<220>
<223>  IntC from human gut metagenome Denmark fecal sample

<400>  75

Met Gln Ile Gln Lys Thr Thr Asp Phe Lys Ile Glu Ser Leu Gly Ile
1                   5                   10                  15


Gln Glu Gln Tyr Val Tyr Asp Ile Glu Ile Glu Asp Thr His Asn Phe
                20                  25                  30


Phe Ala Asn Thr Ile Leu Val His Asn
            35                  40


<210>  76
<211>  119
<212>  PRT
<213>  Artificial sequence

<220>
<223>  modified IntN from human gut metagenome Peru National Reserve
       Matses (Amazon) fecal sample


<220>
<221>  misc_feature
<222>  (31)..(31)
<223>  Xaa can be any naturally occurring amino acid

<220>
<221>  misc_feature
<222>  (77)..(77)
<223>  Xaa can be any naturally occurring amino acid

<220>
<221>  misc_feature
<222>  (114)..(114)
<223>  Xaa can be any naturally occurring amino acid

<400> 76

Ser Val Val Gly Asp Thr Ile Ile Asn Val Asn Gly Lys Pro Ile Thr
1               5               10              15

Ile Ala Asp Tyr Tyr Asn Ser Ile Val Pro Asn Tyr Ile Lys Xaa Asp
            20              25              30

Asp Ile Asn Lys Asn Tyr Ile Lys Arg Val Asp Asn Gly Asp Val Ala
            35              40              45

Leu Ala Tyr Asp Asn Gly Ile Val Gln Asn Lys Ile Lys His Val Met
        50              55              60

Lys His Thr Val Lys Lys Arg Met Tyr Lys Ile Lys Xaa Asn Gly Lys
65              70              75              80

Glu Val Ile Met Thr Glu Asp His Ser Ile Ile Val Asn Arg Asn Gly
                85              90              95

Lys Asn Ile Ser Val Ser Pro Lys Asp Ile Leu Lys Gly Asp Arg Leu
            100             105             110

Ile Xaa Leu Asn Asn Tyr Lys
        115

<210> 77
<211> 46
<212> PRT
<213> Artificial sequence

<220>
<223> modified IntC from human gut metagenome Peru National Reserve
      Matses (Amazon) fecal sample

<220>
<221> misc_feature
<222> (2)..(2)
<223> Xaa can be any naturally occurring amino acid

<220>
<221> misc_feature
<222> (8)..(8)
<223> Xaa can be any naturally occurring amino acid

<220>
<221> misc_feature
<222> (34)..(34)
<223> Xaa can be any naturally occurring amino acid

<400> 77

Met Xaa Lys Ile Tyr Thr Glu Xaa Asn Val Val Asp Asp Phe Glu Val

```
            1                5                      10                    15


        Glu Asp Leu Gly Val Gln Glu Leu Asp Val Tyr Asp Ile Glu Val Glu
                    20                  25                  30


        Glu Xaa His Asn Phe Phe Ala Asn Asp Ile Leu Val His Asn
                35                  40                  45


        <210>  78
        <211>  119
        <212>  PRT
        <213>  Artificial sequence

        <220>
        <223>  modified IntN from human gut metagenome Spanish infant fecal
               sample


        <220>
        <221>  misc_feature
        <222>  (31)..(31)
        <223>  Xaa can be any naturally occurring amino acid


        <220>
        <221>  misc_feature
        <222>  (77)..(77)
        <223>  Xaa can be any naturally occurring amino acid


        <220>
        <221>  misc_feature
        <222>  (114)..(114)
        <223>  Xaa can be any naturally occurring amino acid

        <400>  78

        Ser Val Val Gly Asp Thr Ile Ile Asn Val Asn Gly Lys Pro Ile Thr
        1                5                      10                    15


        Ile Ala Asp Tyr Tyr Asn Ser Ile Ala Pro Asn Tyr Ile Lys Xaa Asp
                    20                  25                  30


        Asp Ile Asn Lys Asn Tyr Ile Lys Arg Val Asp Asn Gly Asp Val Ala
                35                  40                  45


        Leu Ala Tyr Asp Asn Gly Ile Val Gln Asn Lys Ile Lys His Val Met
            50                  55                  60


        Lys His Thr Val Lys Lys Arg Met Phe Lys Ile Lys Xaa Asn Gly Lys
        65                  70                  75                    80


        Glu Val Ile Met Thr Glu Asp His Ser Ile Ile Val Asn Arg Asn Gly
                        85                  90                  95


        Lys Asn Ile Ser Val Ser Pro Lys Asp Ile Leu Lys Gly Asp Arg Leu
```

101

                    100                  105                        110


Ile Xaa Leu Asn Asn Tyr Lys
            115


<210>  79
<211>  46
<212>  PRT
<213>  Artificial sequence

<220>
<223>  modified IntC from human gut metagenome Spanish infant fecal
       sample


<220>
<221>  misc_feature
<222>  (2)..(2)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (34)..(34)
<223>  Xaa can be any naturally occurring amino acid

<400>  79

Met Xaa Lys Ile Tyr Thr Glu Xaa Asn Val Val Asp Asp Phe Glu Ile
1               5                   10                  15


Glu Asp Leu Gly Val Gln Glu Leu Asp Val Tyr Asp Ile Glu Val Glu
            20                  25                  30


Glu Xaa His Asn Phe Phe Ala Asn Gly Ile Leu Val His Asn
            35                  40                  45


<210>  80
<211>  122
<212>  PRT
<213>  Artificial sequence

<220>
<223>  modified IntN from megaphage from human gut metagenome Denmark
       fecal sample


<220>
<221>  misc_feature
<222>  (9)..(9)
<223>  Xaa can be any naturally occurring amino acid

<400>  80

```
Ser Gln Ile Gly Ser Thr Gln Phe Xaa Val Asp Asn Asn Ile Thr Thr
1               5                   10                  15


Met Glu Asp Phe Phe Thr Lys Ala Lys Tyr Glu Asn Asn Asp Val Val
            20                  25                  30


Ile Lys Leu Gln Asn Gly Ser Glu Val Val Pro Val His Asn His Asn
        35                  40                  45


Thr Leu Ser Tyr Lys Asp His Asp Tyr Lys Thr Ile Glu Arg Pro Ile
    50                  55                  60


Asn Tyr Ile Met Arg His Lys Val Thr Lys Asp Lys Phe Arg Leu Lys
65                  70                  75                  80


Thr Lys Ser Gly Lys Glu Leu Ile Val Thr Gly Asp His Ser Ile Met
                85                  90                  95


Val Ile Arg Asn Asn Glu Leu Val Ser Leu Pro Ala Arg Glu Ile Lys
            100                 105                 110


Lys Ser Asp Lys Ile Ile Thr Leu Asp Lys
        115                 120
```

```
<210>  81
<211>  44
<212>  PRT
<213>  Unknown

<220>
<223>  IntC from megaphage from human gut metagenome Denmark fecal
       sample

<400>  81
```

```
Met Asn Tyr Asn Ile Gln Val Glu Asn Ile Asp Val Ile Glu Gln Leu
1               5                   10                  15


Glu Asp Phe Asn Asp Glu Tyr Val Tyr Asp Ile Glu Val Asp Asp Thr
            20                  25                  30


His Thr Phe Phe Ala Asn Glu Ile Leu Ala His Asn
        35                  40
```

```
<210>  82
<211>  264
<212>  PRT
<213>  Artificial sequence

<220>
<223>  modified IntN from human gut metagenome Denmark fecal sample
```

```
<220>
<221>  misc_feature
<222>  (46)..(46)
<223>  Xaa can be any naturally occurring amino acid

<220>
<221>  misc_feature
<222>  (156)..(156)
<223>  Xaa can be any naturally occurring amino acid

<220>
<221>  misc_feature
<222>  (207)..(207)
<223>  Xaa can be any naturally occurring amino acid

<400>  82
```

Ser Ile Val Ala Ser Thr Met Ile Tyr Gly Asp Thr Phe Ala Gly Thr
1               5               10              15

Ile Glu Glu Leu Phe Lys Lys Ser Ala Glu Gly Arg Val Leu Lys Gln
            20              25              30

Thr Leu Asn Gly Thr Leu Met Thr Glu Ser Asp Val Lys Xaa Leu Asn
            35              40              45

Trp Ser Glu Ser Lys Gly Leu His Tyr Ala Pro Ile Val Tyr Ile Ser
    50              55              60

Lys His Leu Val Lys Lys Asn Met Trp Lys Leu Arg Gly Lys Ser Gly
65              70              75              80

Lys Glu Val Ile Val Thr Glu Asp His Ser Leu Ile Val Phe Arg Asp
                85              90              95

Gly Lys Lys Leu Glu Val Lys Pro Lys Glu Ile Leu Pro Thr Asp Lys
            100             105             110

Ile Leu Ile Val Phe Pro Leu Lys Gln Arg Ile Ala Leu Ile Ile Lys
            115             120             125

Ala Arg Glu Arg Gly Tyr Ser Leu Ser Ser Gln Glu Val Phe Asp Lys
    130             135             140

Met Arg Pro Ile Val Glu Glu Met Gly Tyr Thr Xaa Lys Tyr Ala Thr
145             150             155             160

His Gly Gly Glu His Val Val Ile Thr Leu Asp Lys Ser Tyr Phe Leu
            165             170             175

Asp Phe Tyr Ile Pro Glu Leu Lys Ile Gly Ile Glu Tyr Asn Gly Gly

```
            180                    185                    190


   Met Phe His Gly Asp Pro Arg Leu Tyr Lys Asp Asp Glu Tyr Xaa Asn
           195                200                205


   Pro Trp Asn Ile Asn Glu Thr Ala Lys Asp Met Arg Glu Arg Asp Gln
       210                215                220


   Gln Arg Tyr Asp Phe Leu Leu Asn Asn Tyr Gly Ile Lys Thr Tyr Ile
   225                230                235                240


   Ile Trp Glu Leu Asp Tyr Asn Gln Gly Leu Asp Val Glu Ser Phe Ile
                   245                250                255


   Arg Arg Ile Leu Asn Glu Ser Lys
                   260


   <210>  83
   <211>  47
   <212>  PRT
   <213>  Artificial sequence

   <220>
   <223>  modified IntC from human gut metagenome Denmark fecal sample


   <220>
   <221>  misc_feature
   <222>  (18)..(18)
   <223>  Xaa can be any naturally occurring amino acid

   <400>  83

   Met Arg Val Ser Glu Phe Glu Tyr Gln Phe Asp Glu Ile Glu Ser Ile
   1               5                   10                  15


   Glu Xaa Leu Gly Glu Thr Asn Glu Tyr Val Tyr Asp Ile Glu Val Ala
                   20                  25                  30


   Asp Glu Ser His Thr Phe Ile Ala Asn Asp Ile Leu Val His Asn
           35                  40                  45


   <210>  84
   <211>  170
   <212>  PRT
   <213>  Artificial sequence

   <220>
   <223>  modified contiguous intein from human gut metagenome Denmark
          Roux-en-Y gastric bypass surgery of morbidly obese patient


   <220>
   <221>  misc_feature
```

<222>    (85)..(85)
<223>    Xaa can be any naturally occurring amino acid

<220>
<221>    misc_feature
<222>    (109)..(109)
<223>    Xaa can be any naturally occurring amino acid

<220>
<221>    misc_feature
<222>    (138)..(138)
<223>    Xaa can be any naturally occurring amino acid

<400>    84

```
Ser Ile Ser Tyr Asp Ser Ile Ile Lys Thr Ser Arg His Pro Asn Gly
1               5                   10                  15


Ile Thr Ile Ser Glu Trp Tyr Lys Glu Asn Glu Asn Asn Ile Gly Glu
            20                  25                  30


Arg Thr Leu Ala Gly His Glu Ser Val His Thr Asp Asp Lys Ala Leu
        35                  40                  45


Asn Phe Asp Asp Asp Lys Leu Thr Phe Thr Asn Val Lys Arg Ile Ile
    50                  55                  60


Arg His Lys Val Ser Lys Pro Lys Trp Lys Leu Arg Thr Ser Ser Gly
65                  70                  75                  80


Lys Glu Ile Val Xaa Thr Asp Asn His Ser Leu Ile Val Phe Arg Asn
                85                  90                  95


Gly Thr Lys Lys Lys Val Lys Pro Ser Glu Ile Thr Xaa Glu Asp Lys
            100                 105                 110


Val Leu Thr Val Asn Leu Ser Thr Ser Ile Glu Glu Asn Ile Arg Tyr
        115                 120                 125


Val Gln Ser Phe Asp Asn Val Glu Ile Xaa Val Asn Ile Gly Glu Tyr
    130                 135                 140


Thr Asp Glu Tyr Val Tyr Asp Ile Glu Met Asp Asp Asp Ser His Thr
145                 150                 155                 160


Phe Ile Ala Asn Asp Ile Leu Val His Asn
                165                 170
```

<210>    85
<211>    12
<212>    PRT
<213>    Unknown

```
<220>
<223>  IntN from human gut metagenome Bangladeshi cholera-succession


<400>  85

Ser Ala Ala Phe Ser Thr Lys Ile Met Ile Lys Arg
1               5                   10


<210>  86
<211>  154
<212>  PRT
<213>  Artificial sequence

<220>
<223>  modified IntC from human gut metagenome Bangladeshi
       cholera-succession


<220>
<221>  misc_feature
<222>  (68)..(68)
<223>  Xaa can be any naturally occurring amino acid

<220>
<221>  misc_feature
<222>  (82)..(82)
<223>  Xaa can be any naturally occurring amino acid

<220>
<221>  misc_feature
<222>  (136)..(136)
<223>  Xaa can be any naturally occurring amino acid

<400>  86

Met Lys Gln Asp Ile Glu Ile Gly Arg Leu Phe Asp Glu Leu Leu Glu
1               5                   10                  15


Ser Gly Leu Glu Leu Lys Thr Arg Asn Gly Tyr Glu Tyr Met Glu Pro
            20                  25                  30


Lys Gly Ile Lys Ile Leu Thr Gly Gly Asn Arg Tyr Val Trp Leu Val
            35                  40                  45


Ala Ile Ser Arg His Arg Thr Pro Lys His Leu Val Arg Ile Ser Ile
        50                  55                  60


Thr Thr Ser Xaa Ser Gly Lys Tyr Asp Val Thr Val Thr Thr Asp His
65                  70                  75                  80


Val Xaa Met Val Met Asn Arg Asp His Phe Phe Asp Asn Val Asn Ala
                85                  90                  95


Lys Asn Leu Glu Ile Gly Asp Tyr Val Gln Val Tyr Asp Ala Gly Tyr
                100                 105                 110
```

107

Gly Lys Glu Val Leu Gly Ala Ile Ser Lys Ile Glu Asp Leu Gly Pro
        115                 120                 125

Thr Asp Asp Tyr Val Tyr Asp Xaa Glu Val Asp Asp Asn Gly His Thr
        130                 135                 140

Phe Tyr Gly Asn Ser Val Leu Leu His Asn
145                 150


<210>  87
<211>  14
<212>  PRT
<213>  Unknown

<220>
<223>  IntN from pig gut metagenome

<400>  87

Ser Ala Ile Tyr Ser Thr Lys Leu Arg Ile Arg Ile Lys Asp
1                   5                   10


<210>  88
<211>  172
<212>  PRT
<213>  Artificial sequence

<220>
<223>  modified IntC from pig gut metagenome


<220>
<221>  misc_feature
<222>  (84)..(84)
<223>  Xaa can be any naturally occurring amino acid

<220>
<221>  misc_feature
<222>  (101)..(101)
<223>  Xaa can be any naturally occurring amino acid

<220>
<221>  misc_feature
<222>  (149)..(149)
<223>  Xaa can be any naturally occurring amino acid

<220>
<221>  misc_feature
<222>  (162)..(162)
<223>  Xaa can be any naturally occurring amino acid

<400>  88

Met Glu Lys Gln Glu Glu Ile Gly Ala Leu Tyr Asp Lys Leu Val Ala
1                   5                   10                  15

```
Glu Gly Arg Lys Ile Val Asn Asp Gly Lys Tyr Glu Leu Val Asp Ala
            20                  25              30

Lys Gly Ile Glu Val Leu Ser Tyr Gly Gly Lys Thr Asp Asn Gly Arg
            35                  40              45

Tyr Ala Pro Ile Lys Tyr Val Ser Arg His Lys Thr Ser Lys Asn Leu
    50                  55              60

Val Glu Ile Thr Val Thr Ile Gly Ser Asp Ala Val Asp Gly Gly Gly
65                  70              75                  80

Leu Ala Ser Xaa Gln Lys Lys Arg Leu Glu Lys Lys Val Val Val Thr
            85                  90                  95

Thr Asp His Ile Xaa Met Lys Tyr Asp Arg Asp Arg Val Phe Gln Asn
            100                 105             110

Val Asp Ala Lys Asn Leu Thr Pro Gly Asp Tyr Val Ser Val Tyr Asp
        115             120             125

Ser Gly Tyr Gly Met Glu Thr Tyr Gly Ile Val Ser Ala Ile Lys Asn
    130             135             140

Leu Gly Pro Thr Xaa Glu Tyr Val Tyr Asp Leu Glu Val Asp Asp Thr
145             150             155                 160

His Xaa Phe Tyr Ala Asn Asp Val Leu Ile His Asn
            165             170
```

```
<210>  89
<211>  116
<212>  PRT
<213>  Artificial sequence

<220>
<223>  modified IntN from bovine gut rumen metagenome


<220>
<221>  misc_feature
<222>  (9)..(9)
<223>  Xaa can be any naturally occurring amino acid

<220>
<221>  misc_feature
<222>  (42)..(42)
<223>  Xaa can be any naturally occurring amino acid

<220>
<221>  misc_feature
<222>  (84)..(84)
<223>  Xaa can be any naturally occurring amino acid
```

<400> 89

Ser Val Val Asp Ser Lys Ile Arg Xaa Met Ser Gly Glu Lys Met Leu
1               5               10              15

Ser Asp Ile Phe Asn Glu Ser Lys Asn Gln Tyr Lys Thr Pro Phe Ile
        20              25              30

Thr Ser His Gly Ala Glu Leu Tyr Pro Xaa Asp Glu Arg Val Leu Asn
        35              40              45

Tyr Asp Asp Thr Gly Leu His Tyr Thr Arg Ile Lys Tyr Ile Ser Arg
        50              55              60

His Lys Val Asn Lys Pro Met Trp Arg Leu Arg Thr Lys Ser Gly Lys
65              70              75              80

Glu Ile Leu Xaa Thr Glu Asp His Ser Leu Val Val Tyr Arg Asp Gly
                85              90              95

Lys Lys Lys Ser Ile Lys Pro Asp Lys Ile Leu Pro Thr Asp Lys Ile
            100             105             110

Val Thr Ile Lys
            115


<210> 90
<211> 43
<212> PRT
<213> Unknown

<220>
<223> IntC from bovine gut rumen metagenome

<400> 90

Met Glu Ile Ile Phe Asp Asp Ile Glu Thr Ile Glu Cys Val Ser Asp
1               5               10              15

Gly Trp Asp Asp Tyr Val Tyr Asp Ile Glu Val Glu Asp Asp Ser His
            20              25              30

Thr Phe Ile Ala Asn Asp Ile Leu Val His Asn
            35              40


<210> 91
<211> 223
<212> PRT
<213> Artificial sequence

<220>

<223> modified contiguous intein from pig gut metagenome

<220>
<221> misc_feature
<222> (2)..(2)
<223> Xaa can be any naturally occurring amino acid

<220>
<221> misc_feature
<222> (93)..(93)
<223> Xaa can be any naturally occurring amino acid

<220>
<221> misc_feature
<222> (191)..(191)
<223> Xaa can be any naturally occurring amino acid

<400> 91

```
Ser Xaa Glu Lys Ser Thr Phe Ile Asn Ile Ser Asp Asn Val Ile Ser
1               5                  10                 15


Ile Glu Thr Asn Ser Gly Ser Lys Val Tyr Asn Lys Asn Asp Ile Val
            20                  25                  30


Lys Val Asn Arg Asn Gly Ile Glu Met Glu Ile Tyr Ala Ser Asp Ile
            35                  40                  45


Asn Glu Asn Asp Leu Ile Trp Glu Glu Ser Ile Lys Lys Ile Glu Lys
        50                  55                  60


Thr Asn Lys Ile Thr Ile Glu Gln Leu Tyr Asn Leu Gly Thr Gln Asp
65                  70                  75                  80


Met Gly Ser Thr Leu Ala Gly His Glu Ser Val Asn Xaa Asp His Asp
                85                  90                  95


Val Leu Asn Ile Lys Gln Asn Asp Leu Ser Tyr Ser Gly Asn Gly Val
            100                 105                 110


Asp Phe Thr Ser Tyr Tyr Ala Pro Val Lys Arg Val Ile Arg His Lys
            115                 120                 125


Val Thr Lys Ala Lys Trp Ser Leu Val Asp Ser Asp Asn Asn Glu Val
            130                 135                 140


Ile Val Thr Asn Asp His Ser Leu Met Val Leu Arg Asp Gly Asn Leu
145                 150                 155                 160


Gln Lys Ile Lys Pro Tyr Asp Val Asp Val Glu Asn Asp Phe Leu Val
                165                 170                 175
```

```
Thr Ile Ser Asp Gly Asn Ile Glu Ile Arg Asp Ile Val Arg Xaa Glu
            180                 185                 190

Gln Ile Gly Asn Phe Glu Asp Glu Tyr Val Tyr Asp Ile Glu Met Asn
            195                 200                 205

Asp Asp Thr His Thr Phe Val Ala Asn Asn Ile Leu Val His Asn
    210                 215                 220
```

<210> 92
<211> 141
<212> PRT
<213> Artificial sequence

<220>
<223> modified IntN from sheep gut metagenome

<220>
<221> misc_feature
<222> (4)..(4)
<223> Xaa can be any naturally occurring amino acid

<220>
<221> misc_feature
<222> (59)..(59)
<223> Xaa can be any naturally occurring amino acid

<220>
<221> misc_feature
<222> (101)..(101)
<223> Xaa can be any naturally occurring amino acid

<400> 92

```
Ser Val Ser Xaa Asp Thr Ile Ile Tyr Trp Gly Phe Ala Gly Ser Ala
1               5                 10                  15

Glu Gln Ser Thr Thr Ile Glu Glu Met Phe Asn Ile Ile Lys Glu Gln
            20                  25                  30

Asn Met Asp Thr Val Leu Asn Leu Glu Asn Gly Ser Glu Val Val Pro
            35                  40                  45

Thr Ser Glu Leu Thr Ala Ile Arg Thr Tyr Xaa Pro Ile Leu Asn His
    50                  55                  60

Val Thr Gln Lys Pro Val Lys Tyr Val Met Arg His Lys Val Lys Lys
65                  70                  75                  80

Ala Arg Tyr Arg Ile Thr Thr Ser Ser Gly Lys Gln Val Ile Val Thr
            85                  90                  95
```

Gly Asp His Ser Xaa Met Val Leu Arg Asp Gly Lys Leu Thr Ala Val
            100                 105                 110

Lys Ala Asn Glu Ile Asn Pro Asn Thr Asp Lys Ile Ile Ser Asp Lys
            115                 120                 125

His Tyr Arg Pro Lys Ile Asn Glu Glu Lys Asp Glu Gly
    130                 135                 140

<210> 93
<211> 75
<212> PRT
<213> Unknown

<220>
<223> IntC from sheep gut metagenome

<400> 93

Met Glu Ala Gln Lys Gln Asn Asn Asn Asn Glu Tyr Ile Asp Leu Asp
1               5                   10                  15

Ile Asp Glu Phe Asn Pro Val Val Gly Asp Val Ala Asp Thr Asp Gly
            20                  25                  30

Ser Asp Thr Tyr Glu Val Glu Asp Ile Val Ser Ile Glu Gln Leu Asp
        35                  40                  45

Asp Phe Asp Asp Glu Tyr Val Tyr Asp Val Glu Val Glu Asp Thr His
    50                  55                  60

Thr Phe Phe Ala Asn Asp Ile Leu Val His Asn
65                  70                  75

<210> 94
<211> 168
<212> PRT
<213> Artificial sequence

<220>
<223> modified contiguous intein from sheep gut metagenome

<220>
<221> misc_feature
<222> (6)..(6)
<223> Xaa can be any naturally occurring amino acid

<220>
<221> misc_feature
<222> (47)..(47)
<223> Xaa can be any naturally occurring amino acid

<220>
<221> misc_feature

```
<222>   (96)..(96)
<223>   Xaa can be any naturally occurring amino acid


<400>   94

Ser Thr Phe Ser Lys Xaa Leu Leu Leu Ile Asn Arg Asn Asn Lys Asn
1               5                   10                  15


Val Gln Ile Thr Ile Glu Asp Leu Phe Asn Glu Ser Leu Lys Gln Asn
                20                  25                  30


Gly Ile Lys Asp Ile Thr Asn Asn Gly His Glu Ile Val Lys Xaa Asn
            35                  40                  45


Asp Asp Val Leu Asn Trp Thr Glu Lys Asn Gly Leu Asn Phe Val Pro
        50                  55                  60


Ile Lys Trp Ile Met Arg His Lys Val Ser Lys Pro Met Phe Lys Ile
65                  70                  75                  80


Thr Thr Lys Ser Gly Lys Thr Ile Thr Val Thr Glu Asp His Ser Xaa
                85                  90                  95


Val Ile Phe Arg Asn Gly Glu Gln Ile Val Ile Lys Ala Lys Asp Ile
                100                 105                 110


Asn Lys Glu Thr Asp Lys Ile Leu Ser Val Ile Asn Glu Asn Glu Thr
            115                 120                 125


Tyr Gln Phe Glu Glu Ile Glu Asn Ile Glu Gln Val Glu Tyr Lys Asp
    130                 135                 140


Asp Tyr Val Tyr Asp Ile Glu Val Asp Asp Asn Ser His Thr Phe Ile
145                 150                 155                 160


Gly Asn Asp Ile Leu Val His Asn
                165


<210>   95
<211>   166
<212>   PRT
<213>   Artificial sequence

<220>
<223>   modified contiguous intein from sheep gut metagenome


<220>
<221>   misc_feature
<222>   (2)..(2)
<223>   Xaa can be any naturally occurring amino acid
```

<220>
<221>  misc_feature
<222>  (109)..(109)
<223>  Xaa can be any naturally occurring amino acid

<400>  95

Ser Xaa Ala Gly Asn Ser Ile Ile Glu Leu Asn Gly Arg Lys Met Ser
1               5                   10                  15

Ile Glu Asn Ala Phe Ser Tyr Leu Lys Glu Glu Asn Asp His Ile Val
            20                  25                  30

Leu Arg Thr Ser Asn Gly Ser Glu Val Val Pro Val Glu Asn Thr Thr
        35                  40                  45

Thr Lys Thr Tyr Asp Ser Leu Ser Lys Lys Ile Val Asp Arg Asp Val
    50                  55                  60

Lys Tyr Ile Met Arg His Lys Val Ser Lys Pro Lys Trp Lys Leu Thr
65                  70                  75                  80

Thr Ser Ser Gly Lys Met Ile Glu Val Thr Gly Asp His Ser Leu Met
            85                  90                  95

Val Met Arg Asp Gly Glu Leu Leu Ser Val Lys Ala Xaa Glu Val Asp
            100                 105                 110

Pro Lys Lys Asp Lys Ile Val Thr Tyr Ile Asn Thr Gln Glu Tyr Ile
    115                 120                 125

Ile Glu Asp Ile Glu Ser Ile Glu Gln Val Glu Asp Phe Asn Asp Glu
    130                 135                 140

Tyr Val Tyr Asp Ile Glu Val Asp Asp Thr His Thr Phe Phe Ala Asn
145                 150                 155                 160

Asp Ile Leu Val His Asn
                165

<210>  96
<211>  166
<212>  PRT
<213>  Artificial sequence

<220>
<223>  modified contiguous intein from sheep gut metagenome

<220>
<221>  misc_feature
<222>  (2)..(2)

<223> Xaa can be any naturally occurring amino acid

<220>
<221> misc_feature
<222> (109)..(109)
<223> Xaa can be any naturally occurring amino acid

<400> 96

Ser Xaa Ala Gly Asn Ser Ile Ile Glu Leu Asn Gly Arg Lys Met Ser
1               5                   10                  15

Ile Glu Asn Ala Phe Ser Tyr Leu Lys Glu Glu Asn Asp His Ile Val
            20                  25                  30

Leu Arg Thr Ser Asn Gly Ser Glu Val Val Pro Val Glu Asn Thr Thr
        35                  40                  45

Thr Lys Thr Tyr Asp Ser Leu Ser Lys Lys Ile Val Asp Arg Asp Val
    50                  55                  60

Lys Tyr Ile Met Arg His Lys Val Ser Lys Pro Lys Trp Lys Leu Thr
65                  70                  75                  80

Thr Ser Ser Gly Lys Met Ile Glu Val Thr Gly Asp His Ser Leu Met
            85                  90                  95

Val Met Arg Asp Gly Glu Leu Leu Ser Val Lys Ala Xaa Glu Val Asp
            100                 105                 110

Pro Lys Lys Asp Lys Ile Val Thr Tyr Ile Asn Thr Gln Glu Tyr Ile
        115                 120                 125

Ile Glu Asp Ile Glu Ser Ile Glu Gln Val Glu Asp Phe Asn Asp Glu
    130                 135                 140

Tyr Val Tyr Asp Ile Glu Val Asp Asp Thr His Thr Phe Phe Ala Asn
145                 150                 155                 160

Asp Ile Leu Val His Asn
                165

<210> 97
<211> 129
<212> PRT
<213> Artificial sequence

<220>
<223> modified IntN from sheep gut metagenome

<220>

```
<221>  misc_feature
<222>  (99)..(99)
<223>  Xaa can be any naturally occurring amino acid

<400>  97

Ser Val Ala Gly Asp Thr Lys Val Asp Ile Ser Ser Ala Asp Ile Lys
1               5                   10                  15


Lys Arg Ile Asp Ile Ser Glu Leu Phe Thr Lys Ala Lys Tyr Leu Asn
            20                  25                  30


Asp Asp His Val Leu Ser Val Ser Asn Gly Ser Glu Val Ile Pro Gly
            35                  40                  45


Asn Gly Ile Leu Ile Arg Ala Tyr Asp Lys Asp Leu Asp Met Ala Val
    50                  55                  60


Tyr Lys Pro Met Lys Tyr Val Met Arg His Lys Val Ser Lys Ala Arg
65                  70                  75                  80


Phe Arg Ile Lys Thr Glu Ser Gly Lys Glu Val Ile Val Thr Gly Asp
                85                  90                  95


His Ser Xaa Ile Val Leu Arg Asp Gly Glu Leu Ile Asp Ile Lys Ala
            100                 105                 110


Lys Asp Ile Asn Lys Glu Thr Asp Lys Ile Ile Thr Ile Asn Ser Lys
            115                 120                 125


Lys



<210>  98
<211>  47
<212>  PRT
<213>  Artificial sequence

<220>
<223>  modified IntC from sheep gut metagenome


<220>
<221>  misc_feature
<222>  (35)..(35)
<223>  Xaa can be any naturally occurring amino acid

<400>  98

Met Asp Phe Lys Glu Lys Asn Tyr Lys Ile Glu Ser Ile Ala Glu Ile
1               5                   10                  15


Glu Gln Leu Asp Asp Phe Glu Asp Glu Tyr Val Tyr Asp Val Glu Val
```

                    20                      25                      30

        Asp Asp Xaa His Asn Phe Phe Ala Asn Asp Val Leu Val His Asn
                35                      40                      45


        <210>  99
        <211>  166
        <212>  PRT
        <213>  Artificial sequence

        <220>
        <223>  modified contiguous intein from sheep gut metagenome


        <220>
        <221>  misc_feature
        <222>  (85)..(85)
        <223>  Xaa can be any naturally occurring amino acid

        <220>
        <221>  misc_feature
        <222>  (122)..(122)
        <223>  Xaa can be any naturally occurring amino acid

        <220>
        <221>  misc_feature
        <222>  (134)..(134)
        <223>  Xaa can be any naturally occurring amino acid

        <400>  99

        Ser Val Ser Gly Asp Thr Val Val Val Thr Lys Asn His Pro Asn Gly
        1               5                   10                  15


        Ile Ser Ile Glu Arg Leu Tyr Gly Glu Asn Ala Glu Tyr Ala Val Glu
                    20                      25                      30


        Phe Gly Asp Asn His Glu Ser Val Leu Thr Gly Asp Met Val Leu Asn
                35                      40                      45


        Tyr Tyr Asp Asn Glu Leu Tyr Val Ala Pro Val Ser Arg Ile Ile Arg
            50                      55                      60


        His Lys Val Thr Lys Asp Lys Tyr Leu Leu Lys Ala Phe Asn Ser Gly
        65                      70                      75                      80


        Asn Ala Val Glu Xaa Thr Ser Asp His Ser Leu Val Val Tyr Arg Gln
                        85                      90                      95


        Asn Gly Asp Glu Gly Lys Tyr Val Ser Ala Val Val Lys Pro Tyr Glu
                    100                     105                     110


        Ile Arg Asn Gly Asp Leu Val Val Thr Xaa Lys Asn Asp Ser Tyr Thr
                115                     120                     125

```
Phe Glu Glu Ala Thr Xaa Glu Lys Ile Gly Glu Tyr Asn Asp Glu Tyr
    130                 135                 140

Val Tyr Asp Ile Glu Met Asp Asp Leu Thr Ser Thr Phe Val Ala Asn
145                 150                 155                 160

Gly Ile Leu Val His Asn
                    165


<210>  100
<211>  14
<212>  PRT
<213>  Unknown

<220>
<223>  IntN from sheep gut metagenome

<400>  100

Ser Ser Val Phe Asp Thr Tyr Ile Asp Val Ile Glu Glu Asp
1               5                   10


<210>  101
<211>  159
<212>  PRT
<213>  Artificial sequence

<220>
<223>  modified IntC from sheep gut metagenome


<220>
<221>  misc_feature
<222>  (58)..(58)
<223>  Xaa can be any naturally occurring amino acid

<220>
<221>  misc_feature
<222>  (72)..(72)
<223>  Xaa can be any naturally occurring amino acid

<220>
<221>  misc_feature
<222>  (87)..(87)
<223>  Xaa can be any naturally occurring amino acid

<220>
<221>  misc_feature
<222>  (114)..(114)
<223>  Xaa can be any naturally occurring amino acid

<400>  101

Met Lys Leu Tyr Leu Glu Arg Leu Ser Met Thr Ser Val Lys Ile Gly
1               5                   10                  15
```

```
Glu Leu Tyr Asn Lys Tyr Leu Ala Lys Gly Tyr Glu Ile Val Asn Thr
            20                  25                  30

Pro Thr Gly His Glu Leu Ile Tyr Pro Lys Ser Leu Lys Val Arg Ser
            35                  40                  45

Ile Gly Asn Glu Phe Lys Lys Val Lys Xaa Leu Ser Arg His Arg Thr
            50                  55                  60

Ser Lys Pro Leu Val Arg Ile Xaa Phe Gln Lys Ser Glu Pro Leu Val
65                  70                  75                  80

Val Thr Thr Asp His Val Xaa Met Ala Tyr Asn Asp Asp Arg Met Leu
                85                  90                  95

Glu Asn Ile Ala Ser Lys Asp Leu Arg Val Gly Met Met Val Asp His
            100                 105                 110

Tyr Xaa Arg Thr Ser Asp Lys Glu Val Ile Asp Val Ile Thr Asn Ile
            115                 120                 125

Glu Pro Leu Gly Thr Thr Asp Asp Tyr Val Tyr Asp Leu Glu Val Glu
            130                 135                 140

Asp Glu Ser His Val Phe Tyr Ala Asn Asp Thr Leu Ile His Asn
145                 150                 155
```

```
<210>   102
<211>   124
<212>   PRT
<213>   Artificial sequence

<220>
<223>   modified IntN from sheep gut metagenome


<220>
<221>   misc_feature
<222>   (63)..(63)
<223>   Xaa can be any naturally occurring amino acid

<220>
<221>   misc_feature
<222>   (98)..(98)
<223>   Xaa can be any naturally occurring amino acid

<400>   102
```

```
Ser Ile Asp Gly Asn Ser Val Ile Asp Ile Asn Asp Glu Lys Ile Ala
1                   5                   10                  15


Ile Lys Asp Ala Phe Ala Ala Ile Lys Tyr Met Asn Ile Asp Thr Val
            20                  25                  30
```

```
Ile Met Leu Pro Asn Gly Thr Gln Val Val Pro Ala Pro Ser Asp Ile
        35                  40                  45


Thr Leu Thr Thr Lys Thr Tyr Asp Ala Ser Thr Asp Thr Val Xaa Asp
        50                  55                  60


Lys Gln Ile Arg Tyr Ile Met Arg His Lys Val Lys Lys Ser Lys Tyr
65                  70                  75                  80


Lys Ile Thr Thr Glu Ser Gly Lys Glu Val Ile Val Thr Gly Asp His
                85                  90                  95


Ser Xaa Met Val Val Arg Asp Gly Ile Leu Ile Ser Val Thr Ala Gln
            100                 105                 110


Glu Ile Asn Pro Glu Thr Asp Lys Ile Ile Thr Ile
            115                 120
```

```
<210>  103
<211>  51
<212>  PRT
<213>  Artificial sequence

<220>
<223>  modified IntC from sheep gut metagenome

<400>  103

Met Leu Lys Ile Met Asp Phe Ser Gln Thr Asn Tyr Lys Val Glu Asn
1               5                   10                  15


Ile Lys Ser Ile Glu Val Leu Pro Asp Phe Asp Asp Glu Tyr Val Tyr
        20                  25                  30


Asp Ile Glu Val Gly Glu Thr His Met Phe Phe Ala Asn Glu Ile Leu
        35                  40                  45


Val His Asn
    50
```

```
<210>  104
<211>  171
<212>  PRT
<213>  Artificial sequence

<220>
<223>  modified contiguous intein from sheep gut metagenome


<220>
<221>  misc_feature
```

121

<222> (139)..(139)
<223> Xaa can be any naturally occurring amino acid

<400> 104

Ser Val Ser Lys Asp Thr Ile Ile Arg Thr Arg Leu His Pro Asp Gly
1               5                   10                  15


Ile Met Ile Glu Asp Phe Tyr Asp Glu Asn Ser Ser Asn Lys Gly Glu
            20                  25                  30


Asp Thr Arg Ala Gly His Glu Ser Val His Thr Ser Asp Gln Val Leu
        35                  40                  45


Asn Phe Asn Gly Ser Ile Leu Thr Asn Thr Ser Asn Leu Tyr Tyr Gly
    50                  55                  60


Asn Val Lys Arg Ile Ile Arg His Lys Val Ser Lys Pro Lys Trp Thr
65                  70                  75                  80


Ile Thr Thr Arg Phe Gly His Ser Val Ser Val Thr Asn Asp His Ser
                85                  90                  95


Leu Met Val Leu Arg Asp Asp Lys Leu Tyr Lys Val Lys Pro Ser Glu
            100                 105                 110


Val Lys Pro Gly Asp Glu Ala Met Ser Val Glu Val Met Tyr Gly Asp
        115                 120                 125


Ile Val Glu Gly Ala Asp Lys Ile Thr Ser Xaa Val His Thr Gly Glu
    130                 135                 140


Tyr Asn Asp Glu Tyr Val Tyr Asp Ile Glu Met Asp Asp Asp Asn His
145                 150                 155                 160


Thr Phe Phe Ala Asn Asp Ile Leu Val His Asn
                165                 170


<210> 105
<211> 166
<212> PRT
<213> Artificial sequence

<220>
<223> modified contiguous intein from sheep gut metagenome


<220>
<221> misc_feature
<222> (2)..(2)
<223> Xaa can be any naturally occurring amino acid

<220>
<221>  misc_feature
<222>  (109)..(109)
<223>  Xaa can be any naturally occurring amino acid

<400>  105

Ser Xaa Ala Gly Asn Ser Ile Ile Glu Leu Asn Gly Arg Lys Met Ser
1               5               10              15

Ile Glu Asn Ala Phe Ser Tyr Leu Lys Glu Glu Asn Asp His Ile Val
            20              25              30

Leu Arg Thr Ser Asn Gly Ser Glu Val Val Pro Val Glu Asn Thr Thr
            35              40              45

Thr Lys Thr Tyr Asp Ser Leu Ser Lys Lys Ile Val Asp Arg Asp Val
    50              55              60

Lys Tyr Ile Met Arg His Lys Val Ser Lys Pro Lys Trp Lys Leu Thr
65              70              75              80

Thr Ser Ser Gly Lys Met Ile Glu Val Thr Gly Asp His Ser Leu Met
            85              90              95

Val Met Arg Asp Gly Glu Leu Leu Ser Val Lys Ala Xaa Glu Val Asp
            100             105             110

Pro Lys Lys Asp Lys Ile Val Thr Tyr Ile Asn Thr Gln Glu Tyr Ile
    115             120             125

Ile Glu Asp Ile Glu Ser Ile Glu Gln Val Glu Asp Phe Asn Asp Glu
    130             135             140

Tyr Val Tyr Asp Ile Glu Val Asp Asp Thr His Thr Phe Phe Ala Asn
145             150             155             160

Asp Ile Leu Val His Asn
                165

<210>  106
<211>  124
<212>  PRT
<213>  Artificial sequence

<220>
<223>  modified IntN from sheep gut metagenome

<220>
<221>  misc_feature
<222>  (63)..(63)

<223> Xaa can be any naturally occurring amino acid

<220>
<221> misc_feature
<222> (98)..(98)
<223> Xaa can be any naturally occurring amino acid

<400> 106

Ser Ile Asp Gly Asn Ser Val Ile Asp Ile Asn Asp Asn Arg Ile Ala
1               5                   10                  15

Ile Arg Asp Ala Phe Ala Ala Ile Lys Gln Met Asn Gln Asp Thr Val
            20                  25                  30

Ile Val Leu Pro Asn Gly Thr Gln Val Val Pro Ala Pro Ser Asp Val
            35                  40                  45

Glu Leu Thr Thr Lys Thr Tyr Asp Ala Asn Thr Asp Thr Ile Xaa Asp
        50                  55                  60

Met Pro Ile Lys Tyr Ile Met Arg His Lys Val Arg Lys Ala Lys Tyr
65                  70                  75                  80

Lys Ile Thr Thr Glu Ser Gly Lys Gln Val Ile Val Thr Gly Asp His
                85                  90                  95

Ser Xaa Met Val Ile Arg Asp Gly Val Leu Ile Ser Val Thr Ala Gln
            100                 105                 110

Glu Ile Asn Pro Glu Thr Asp Lys Ile Ile Thr Ile
        115                 120

<210> 107
<211> 47
<212> PRT
<213> Artificial sequence

<220>
<223> modified IntC from sheep gut metagenome

<220>
<221> misc_feature
<222> (4)..(4)
<223> Xaa can be any naturally occurring amino acid

<400> 107

Met Glu Phe Xaa Gln Asn Asn Tyr Lys Val Glu Asn Ile Lys Ser Ile
1               5                   10                  15

Glu Val Leu Pro Asp Phe Glu Asp Glu Asp Val Tyr Asp Leu Glu Val
            20                  25                  30

Gly Gly Thr His Met Phe Phe Ala Asn Asp Ile Leu Val His Asn
        35                  40                  45

<210> 108
<211> 128
<212> PRT
<213> Artificial sequence

<220>
<223> modified IntN from sheep gut metagenome

<220>
<221> misc_feature
<222> (48)..(48)
<223> Xaa can be any naturally occurring amino acid

<220>
<221> misc_feature
<222> (52)..(52)
<223> Xaa can be any naturally occurring amino acid

<220>
<221> misc_feature
<222> (97)..(97)
<223> Xaa can be any naturally occurring amino acid

<400> 108

Ser Leu Ala Lys Lys Ser Leu Leu Leu Ile Lys Asp Thr Lys Asn Ile
1               5               10              15

Lys Asn Lys Ile Thr Ile Glu Asp Leu Phe Asn Gln Ser Leu Asp Lys
            20                  25                  30

Asn Gly Leu Ser Asp Ile Thr Gln Asn Asn Gln Glu Ile Val Lys Xaa
            35                  40                  45

Asp Gln Gln Xaa Leu Asn Trp Thr Lys Glu Asn Gly Leu Gln Tyr Val
        50                  55                  60

Pro Ile Lys Tyr Ile Met Arg His Lys Val Ser Lys Glu Gln Phe Lys
65                  70                  75                  80

Ile Lys Thr Lys Ser Gly Lys Glu Ile Ile Val Thr Gly Asp His Ser
                85                  90                  95

Xaa Ile Val Phe Arg Asn Gly Lys Gln Leu Thr Ile Lys Ala Arg Asp
            100                 105                 110

Ile Asn Lys Ser Thr Asp Lys Ile Leu Ser Ile Ile Asn Asn Glu Glu
            115                 120                 125

<210> 109
<211> 44
<212> PRT
<213> Unknown

<220>
<223> IntC from sheep gut metagenome

<400> 109

Met Leu Glu Tyr Gln Phe Glu Glu Ile Glu Ser Ile Glu Gln Leu Asp
1               5                   10                  15

Asn Phe Asn Asp Glu Tyr Val Tyr Asp Ile Glu Val Asp Asp Asn Ser
            20                  25                  30

His Thr Phe Ile Ala Asn Asp Ile Leu Val His Asn
        35                  40

<210> 110
<211> 169
<212> PRT
<213> Artificial sequence

<220>
<223> modified contiguous intein from sheep gut metagenome

<220>
<221> misc_feature
<222> (4)..(4)
<223> Xaa can be any naturally occurring amino acid

<220>
<221> misc_feature
<222> (48)..(48)
<223> Xaa can be any naturally occurring amino acid

<220>
<221> misc_feature
<222> (97)..(97)
<223> Xaa can be any naturally occurring amino acid

<400> 110

Ser Thr Phe Xaa Lys Ser Leu Leu Leu Ile Lys Asp Asn Lys Asn Ile
1               5                   10                  15

Glu Asn Lys Val Thr Ile Glu Ser Leu Phe Asn Lys Ser Leu Met Asp
            20                  25                  30

Asn Gly Leu Thr Asp Leu Thr Gln Asn Asn Gln Glu Ile Val Lys Xaa
        35                  40                  45

Asp Tyr Ser Thr Leu Asn Trp Thr Glu Glu Lys Gly Leu Glu Tyr Val
        50                  55                  60

126

```
Pro Ile Lys Tyr Ile Met Arg His Lys Val Ser Lys Gln Gln Phe Lys
65              70              75                          80
```

```
Ile Lys Thr Lys Ser Gly Lys Glu Ile Ile Val Thr Gly Asp His Ser
                85              90                  95
```

```
Xaa Ile Val Phe Arg Asn Gly Glu Glu Thr Val Val Lys Ala Lys Asp
            100             105             110
```

```
Ile Asn Lys Asp Thr Asp Lys Ile Leu Ser Ile Ile Thr Phe Asn Lys
        115             120             125
```

```
Tyr Gln Ile Glu Asn Ile Lys Ser Ile Glu Gln Leu Glu Asp Phe Asp
    130             135             140
```

```
Asn Glu Tyr Val Tyr Asp Ile Glu Val Asp Asp Asp Ser His Thr Phe
145             150             155                         160
```

```
Ile Ala Asn Asp Ile Leu Val His Asn
                165
```

```
<210>  111
<211>  15
<212>  PRT
<213>  Unknown

<220>
<223>  IntN from sheep gut metagenome

<400>  111
```

```
Ser Val His Gly Lys Thr His Val Phe Ile Arg Ser Ile Lys Asn
1               5                   10                  15
```

```
<210>  112
<211>  168
<212>  PRT
<213>  Artificial sequence

<220>
<223>  modified IntC from sheep gut metagenome


<220>
<221>  misc_feature
<222>  (96)..(96)
<223>  Xaa can be any naturally occurring amino acid

<220>
<221>  misc_feature
<222>  (150)..(150)
<223>  Xaa can be any naturally occurring amino acid
```

<400> 112

Met Gln Glu Ala Lys Ile Asp Ile Lys Ser Leu Tyr Asp Ser Leu Ala
1               5                   10                  15

Lys Lys Tyr Asp Val Gln His Lys Asn Ser Tyr Glu Val Ile Tyr Pro
            20                  25                  30

Lys Gly Tyr Glu Ile Lys Val Leu Gly Asn Lys Tyr Val Lys Leu Val
            35                  40                  45

Ala Met Ser Arg His Lys Thr Gln Lys His Leu Val Lys Ile Val Val
        50                  55                  60

Lys Ser Glu Lys Thr Ile Asp Ser Leu Asp Pro Ile Arg Gln Lys Ser
65                  70                  75                  80

Leu Leu Lys Lys Gln Asp Glu Val Val Val Thr Thr Asp His Ile Xaa
                85                  90                  95

Met Val Tyr Asn Asp Asp His Phe Phe Glu Asn Val Asn Ala Lys Asn
            100                 105                 110

Leu Lys Val Gly Asn Tyr Val Ser Val Tyr Asp Glu Ala Ser Asp Lys
            115                 120                 125

Glu Val Ile Gly Glu Ile Ala Ser Ile Glu Asp Leu Gly Met Thr Asp
    130                 135                 140

Asp Tyr Val Tyr Asp Xaa Glu Val Asp Asp Asp Ser His Ala Phe Tyr
145                 150                 155                 160

Ala Ser Asn Ile Leu Val His Asn
                165

<210> 113
<211> 171
<212> PRT
<213> Artificial sequence

<220>
<223> modified contiguous intein from sheep gut metagenome

<220>
<221> misc_feature
<222> (2)..(2)
<223> Xaa can be any naturally occurring amino acid

<220>
<221> misc_feature
<222> (111)..(111)

<223>   Xaa can be any naturally occurring amino acid

<220>
<221>   misc_feature
<222>   (139)..(139)
<223>   Xaa can be any naturally occurring amino acid

<400>   113

Ser Xaa Asn Phe Asp Thr Leu Ile Arg Thr Lys Asn Tyr Pro Asp Gly
1               5                   10                  15

Ile Thr Ile Glu Asp Phe Tyr Asn Ile Asn Ser Glu Asn Lys Gly Asp
            20                  25                  30

Thr Thr Leu Val Gly His Glu Ser Val Tyr Thr Thr Asp Lys Val Leu
        35                  40                  45

Asn Phe Lys Gly Asn Gln Leu Thr Asn Thr Ser Gln Leu Tyr Tyr Gly
        50                  55                  60

Asp Val Lys Arg Ile Ile Arg His Lys Val Ser Lys Pro Lys Trp Val
65                  70                  75                  80

Ile Thr Thr Arg Phe Gly His Ser Val Thr Val Thr Asn Asp His Ser
                85                  90                  95

Met Met Val Leu Arg Asp Asp Lys Leu Tyr Met Val Lys Pro Xaa Glu
            100                 105                 110

Ile His Pro Gly Asp Asp Val Leu Ser Leu Glu Val Met Tyr Gly Asp
        115                 120                 125

Phe Val Glu Gly Ser Asp Lys Val Thr Ser Xaa Leu His Val Gly Glu
        130                 135                 140

Tyr Glu Asp Glu Tyr Val Tyr Asp Ile Glu Met Gly Asp Asp Thr His
145                 150                 155                 160

Thr Phe Phe Ala Asn Asp Ile Leu Val His Asn
                165                 170

<210>   114
<211>   74
<212>   PRT
<213>   Unknown

<220>
<223>   IntN from sheep gut metagenome

<400>   114

```
Ser Val Ser Lys Asp Thr Ile Ile Arg Thr Lys Lys His Val Asp Gly
1               5                   10                  15

Ile Thr Ile Glu Asp Phe Tyr Glu Glu Asn Ser Glu Lys Asp Val Phe
            20                  25                  30

Glu Ile Lys Thr Glu Ser Gly Lys Ile Ile Arg Tyr Asn Lys Asn Asp
        35                  40                  45

Lys Val Lys Val Lys Arg Ser Asp Lys Ile Ile Tyr Val Tyr Pro Asp
    50                  55                  60

Asp Ile Leu Tyr Glu Asp Glu Ile Phe Thr
65                  70
```

```
<210>   115
<211>   159
<212>   PRT
<213>   Artificial sequence

<220>
<223>   modified IntC from sheep gut metagenome


<220>
<221>   misc_feature
<222>   (99)..(99)
<223>   Xaa can be any naturally occurring amino acid

<220>
<221>   misc_feature
<222>   (127)..(127)
<223>   Xaa can be any naturally occurring amino acid

<220>
<221>   misc_feature
<222>   (129)..(129)
<223>   Xaa can be any naturally occurring amino acid

<400>   115
```

```
Met Ser Gln Phe Glu Lys Ile Val Ser Ile Arg Lys Ile Arg Ser Ser
1               5                   10                  15

Asn Lys Gly Glu Ser Thr Leu Ala Gly His Glu Ser Val Tyr Thr Asp
            20                  25                  30

Asp Lys Val Leu Asn Phe Lys Gly Ser Ile Leu Thr Asn Ile Ser Gln
        35                  40                  45

Leu Tyr Tyr Gly Ser Val Lys Arg Ile Ile Arg His Lys Val Ser Lys
    50                  55                  60

Pro Lys Trp Thr Ile Thr Thr Arg Phe Gly His Ser Val Thr Val Thr
```

```
        65                    70                    75                    80


        Asn Asp His Ser Met Met Val Leu Arg Asp Asp Lys Leu Tyr Lys Val
                        85                    90                    95


        Lys Pro Xaa Glu Ile His Pro Asp Asp Tyr Val Leu Ser Leu Glu Ala
                        100                   105                   110


        Met Tyr Gly Asp Ile Val Glu Gly Ser Asp Lys Val Val Ser Xaa Leu
                        115                   120                   125


        Xaa Thr Gly Glu Tyr Asp Asn Glu Tyr Val Tyr Asp Ile Glu Met Asp
                        130                   135                   140


        Asp Asp Thr His Thr Phe Phe Ala Asn Asp Ile Leu Val His Asn
        145                   150                   155


        <210>  116
        <211>  170
        <212>  PRT
        <213>  Artificial sequence

        <220>
        <223>  modified contiguous intein from sheep gut metagenome


        <220>
        <221>  misc_feature
        <222>  (48)..(48)
        <223>  Xaa can be any naturally occurring amino acid

        <220>
        <221>  misc_feature
        <222>  (98)..(99)
        <223>  Xaa can be any naturally occurring amino acid

        <220>
        <221>  misc_feature
        <222>  (104)..(105)
        <223>  Xaa can be any naturally occurring amino acid

        <220>
        <221>  misc_feature
        <222>  (108)..(108)
        <223>  Xaa can be any naturally occurring amino acid

        <400>  116

        Ser Leu Ala Lys Asn Ser Leu Ile Leu Ile Glu Asp Asn Lys Asn Thr
        1               5                   10                    15


        Lys Asp Lys Ile Tyr Ile Glu Ser Leu Phe Asn Lys Ala Leu Arg Asp
                        20                    25                    30


        Asn Gly Leu Glu Asp Ile Ser Gln Asn Asn Gln Glu Ile Val Lys Xaa
```

```
            35                    40                    45


       Asp Asp Tyr Asn Val Leu Asn Trp Thr Lys Glu Asn Gly Leu Gln Tyr
           50              55              60


       Val Pro Ile Lys Tyr Ile Met Arg His Lys Val Ser Lys Pro Lys Phe
       65              70              75              80


       Lys Ile Lys Thr Lys Ser Gly Lys Glu Ile Ile Val Thr Gly Asp His
                   85              90              95


       Ser Xaa Xaa Val Phe Arg Asn Xaa Xaa Gln Leu Xaa Ile Lys Ala Lys
               100             105             110


       Asp Ile Asn Lys Asp Thr Asp Lys Ile Leu Ser Ile Ile Tyr Asp Met
               115             120             125


       Lys Tyr Ile Ile Glu Glu Ile Glu Ser Val Glu Gln Leu Asp Asn Phe
           130             135             140


       Asn Asp Glu Tyr Val Tyr Asp Ile Glu Val Asp Asp Asn Ser His Thr
       145             150             155             160


       Phe Ile Ala Asn Asp Ile Leu Val His Asn
                   165             170
```

```
       <210>  117
       <211>  120
       <212>  PRT
       <213>  Artificial sequence

       <220>
       <223>  modified IntN from marine sediment metagenome LCGC14


       <220>
       <221>  misc_feature
       <222>  (24)..(24)
       <223>  Xaa can be any naturally occurring amino acid

       <400>  117
```

```
       Ser Val Asp Ala Asp Thr Ile Ile Lys Thr Asn Tyr Gly Glu Met Thr
       1               5               10              15


       Ile Glu Asn Leu Phe Lys Ser Xaa Ser Ile Lys Gly Pro Ser Trp Ala
                   20              25              30


       Ile Asp Asp Gln Glu Phe Thr Ile Tyr Asp Gln Ile Gln Ile Leu Thr
               35              40              45
```

```
Tyr Asp Pro Lys Thr Asn Glu Glu Ile Tyr Arg Pro Phe Glu Tyr Val
    50                  55                  60

Tyr Arg His Lys Val Ser Lys Pro Arg Trp Lys Ile Ile Asp Glu Asn
65                  70                  75                  80

Gly Asn Glu Ile Ile Leu Thr Asn Asp His Ser Val Met Ile Glu Arg
                85                  90                  95

Asp Gly Lys Leu Ile Glu Ala Lys Pro Ser Glu Ile Asn Pro Asp Thr
                100                 105                 110

Asp Ile Leu Ile Thr Ile Gly Glu
        115                 120


<210>   118
<211>   42
<212>   PRT
<213>   Unknown

<220>
<223>   IntC from Marine sediment metagenome LCGC14

<400>   118

Met Val Glu Lys Leu Lys Ile Gln Lys Ile Glu Lys Leu Glu Asp Phe
1               5                   10                  15

Asp Asn Glu Tyr Val Tyr Asp Ile Ser Val Asp Lys Glu Thr Pro Tyr
                20                  25                  30

Phe Phe Gly Asn Asn Ile Leu Val His Asn
        35                  40


<210>   119
<211>   469
<212>   PRT
<213>   Artificial sequence

<220>
<223>   modified contiguous intein from Stanford USA drinking water
        system tap filter metagenome genome assembly


<220>
<221>   misc_feature
<222>   (106)..(106)
<223>   Xaa can be any naturally occurring amino acid

<220>
<221>   misc_feature
<222>   (111)..(111)
<223>   Xaa can be any naturally occurring amino acid

<220>
```

```
<221>  misc_feature
<222>  (121)..(121)
<223>  Xaa can be any naturally occurring amino acid

<220>
<221>  misc_feature
<222>  (240)..(240)
<223>  Xaa can be any naturally occurring amino acid

<220>
<221>  misc_feature
<222>  (284)..(284)
<223>  Xaa can be any naturally occurring amino acid

<220>
<221>  misc_feature
<222>  (290)..(290)
<223>  Xaa can be any naturally occurring amino acid

<220>
<221>  misc_feature
<222>  (304)..(304)
<223>  Xaa can be any naturally occurring amino acid

<220>
<221>  misc_feature
<222>  (415)..(415)
<223>  Xaa can be any naturally occurring amino acid

<220>
<221>  misc_feature
<222>  (423)..(423)
<223>  Xaa can be any naturally occurring amino acid

<220>
<221>  misc_feature
<222>  (444)..(444)
<223>  Xaa can be any naturally occurring amino acid

<400>  119
```

Ser Phe Ser Ser Ile Thr Pro Leu Leu Ile His Asp Asp His Gly Gln
1               5                   10                  15

Ile Asp Ile Val Gln Ala Lys Asp Leu Val Lys Pro Asn Asp Pro Ala
            20                  25                  30

Trp Met Ser Lys Glu Asp Phe Trp Lys Asn Tyr Asn Pro Asp Pro Val
            35                  40                  45

Ser Thr Thr Lys Ala Ser Ser Thr Ser Ile Ser Thr Thr Lys Thr Gly
            50                  55                  60

Arg Val Ser Lys Ala Thr Ser Ser Asn Ser Thr Thr Lys Pro Ser Gly
65                  70                  75                  80

Thr Ser Asn Thr Ile Ala Ile Glu Pro Thr Ser Thr Arg Gly Ser Arg
                85                  90                  95

```
        Ser Arg Thr Ser Arg Ser Ser Asn Ser Xaa Asp Ile Ala Ser Xaa Ser
                    100                 105                 110

        Asn Asp Asn Thr Ile Pro Gln Ser Xaa Ser Ser Glu Thr Pro Asp Thr
                    115                 120                 125

        Ser Gln Phe Ser Gly Ser Thr Gln Gln Tyr Met Asp Ile Arg Asp Lys
                    130                 135                 140

        Asn Tyr Asn Ile Trp Ser Glu Lys Gly Trp Thr Gln Ile Lys Tyr Ile
        145                 150                 155                 160

        Met Arg His Lys Thr Gly Lys Gln Met Tyr Arg Ile Asn Thr His Thr
                        165                 170                 175

        Gly Val Ile Asp Val Thr Glu Asp His Ser Leu Leu Asp Ile Lys Gly
                        180                 185                 190

        Asp Pro Val Thr Pro Asn Glu Val Lys Ile Gly Ser Glu Leu Leu His
                        195                 200                 205

        His Asp Leu Pro Asn Val Val Asn Glu His Gln Asn Val Ile Ile Asp
                    210                 215                 220

        Ala Asp Thr Ala Trp Leu Tyr Gly Phe Phe Tyr Ala Glu Gly Thr Xaa
        225                 230                 235                 240

        Gly Thr Tyr Gln Tyr Lys Lys Gly Pro Arg Ser Ser Trp Ser Ile Ser
                        245                 250                 255

        Asn Gln Asp Leu Ala Pro Met Asn Lys Ala Leu Glu Ile Leu Gln Arg
                        260                 265                 270

        Ile Glu Pro Asn Tyr Lys Phe Lys Ile Asp Asn Xaa Met Lys Ser Ser
                    275                 280                 285

        Ala Xaa His Lys Leu Ser Ala Arg Asn Gly Ser Thr Ser Lys Asn Xaa
            290                 295                 300

        Pro Arg Glu Tyr His Val Ser Ser Leu Val Glu Lys Tyr His Asn Met
        305                 310                 315                 320

        Phe His Ala Asp Ser Gly Gln Gln Arg His Asn Ile Lys Ser Asn Ser
                        325                 330                 335

        Tyr Thr Ala Leu Tyr Tyr Lys Lys Val Pro Lys Glu Ile Leu Asn Ala
```

```
                    340                    345                    350


          Ser Asn Asp Ile Lys Lys Ser Phe Tyr Asp Gly Tyr Tyr Val Gly Asp
                  355                    360                    365


          Gly Leu Lys Ala Thr Thr Ala Asn Glu Val Phe Asp Asn Lys Gly Gln
              370                    375                    380


          Ile Gly Ala Ala Gly Leu Tyr Tyr Ile Ala Ser Ala Leu Gly Tyr Asp
          385                    390                    395                    400


          Val Ser Leu Tyr Leu Arg Glu Asp Lys Pro Gln Ile Phe Arg Xaa Thr
                          405                    410                    415


          Leu Ser Lys Asp Thr Lys Xaa Val Lys Ser Val Gln Arg Lys Asn Arg
                  420                    425                    430


          Asn Ala Ile Lys Lys Ile Ile Pro Leu Gly Thr Xaa Asp Asp Tyr Val
                  435                    440                    445


          Tyr Asp Ile Glu Thr Glu Asn His His Phe Ala Ala Gly Ile Gly Arg
              450                    455                    460


          Met Val Val His Asn
          465


          <210>  120
          <211>  118
          <212>  PRT
          <213>  Artificial sequence

          <220>
          <223>  modified contiguous intein from Stanford USA drinking water
                 system tap filter metagenome genome assembly


          <220>
          <221>  misc_feature
          <222>  (42)..(42)
          <223>  Xaa can be any naturally occurring amino acid

          <220>
          <221>  misc_feature
          <222>  (74)..(74)
          <223>  Xaa can be any naturally occurring amino acid

          <220>
          <221>  misc_feature
          <222>  (82)..(82)
          <223>  Xaa can be any naturally occurring amino acid

          <400>  120

          Ser Val Asp Phe Gln Thr Leu Met Lys Thr Glu Lys Asp Glu Ile Thr
```

```
1                    5                         10                        15
```

Ile Glu Glu Leu Tyr Asn Arg Asn Ile Ile Asn Gly Ser Ala Gly Ile
            20                    25                    30

Thr Leu Asn Gly His Glu Ser Val Lys Xaa Thr Asp Ile Ile Leu Asn
            35                    40                    45

Tyr Ser Lys Ser Lys Gly Leu Tyr Phe Asn Asn Val Arg Arg Ile Ile
        50                    55                    60

Arg His Lys Val Ser Lys Glu Lys Trp Xaa Leu Lys Thr Thr Asn Gly
65                    70                    75                    80

Lys Xaa Ile Tyr Ile Thr Asn Asp His Ser Leu Ile Val Phe Arg Asp
                85                    90                    95

Gly Glu Lys Leu Glu Ile Lys Pro Lys Asp Val Leu Lys Ile Asp Lys
            100                   105                   110

Val Leu Thr Ile Lys Lys
            115

```
<210>  121
<211>  52
<212>  PRT
<213>  Artificial sequence

<220>
<223>  modified IntC from Lake Huron low oxygen high sulfur sink hole
       purple microbial mat bin unclassified.02

<220>
<221>  misc_feature
<222>  (14)..(14)
<223>  Xaa can be any naturally occurring amino acid

<400>  121
```

Met Met Glu Phe Glu Tyr Glu Leu Val Asp Ile Glu Ser Xaa Glu Leu
1                5                     10                    15

Val Gly Ile Phe Asp Asp Glu Tyr Val Tyr Asp Ile Glu Val Glu Glu
            20                    25                    30

Asp Glu Asn Asp Val Glu Asp Thr His Thr Phe Phe Gly Asn Asp Ile
            35                    40                    45

Leu Val His Asn
            50

```
<210>   122
<211>   125
<212>   PRT
<213>   Artificial sequence

<220>
<223>   modified IntN from Rattus norvegicus (Malaysia) gut metagenome


<220>
<221>   misc_feature
<222>   (45)..(46)
<223>   Xaa can be any naturally occurring amino acid

<220>
<221>   misc_feature
<222>   (96)..(96)
<223>   Xaa can be any naturally occurring amino acid

<400>   122


Ser Ser Ser Ser Asn Ser Asn Ile Tyr Ile Lys Arg Gly Asn Gln Val
1               5               10              15


Leu Lys Arg Thr Phe Ile Glu Leu Trp Arg Asp Thr Val Glu Glu His
            20              25              30


Met Pro Phe Gly Leu Gly Thr His Gly Gln Glu Leu Xaa Xaa Ser Asp
        35              40              45


Asp Tyr Val Leu Asn Tyr Asp Glu Lys Arg Gly Leu His Trp Val Pro
        50              55              60


Ile Lys Tyr Ile Met Lys His Gly Thr Lys Lys Arg Lys Phe Arg Val
65              70              75              80


Lys Thr Lys Ser Gly Lys Glu Ile Ile Val Thr Glu Asp His Ser Xaa
            85              90              95


Val Val Ile Arg Asn Gly Glu Lys Ile Ala Val Lys Ala Ser Glu Ile
            100             105             110


Asn Lys Asp Thr Asp Lys Ile Val Ser Ile Ser Thr Lys
        115             120             125


<210>   123
<211>   49
<212>   PRT
<213>   Rattus norvegicus

<400>   123


Met Ile Glu Glu Gly Phe Asp Tyr Ile Ile Glu Asp Ile Glu Ser Val
1               5               10              15
```

```
Glu Asp Ile Gly Phe Phe Asp Glu Asp Glu Pro Val Phe Asp Ile Glu
            20                  25              30

Val Glu Asp Asp Thr His Thr Phe Ile Gly Asn Asp Val Leu Val His
            35                  40              45

Asn
```

<210> 124
<211> 125
<212> PRT
<213> Artificial sequence

<220>
<223> modified IntN from Rattus norvegicus (Denmark: Riget) gut
      metagenome

<220>
<221> misc_feature
<222> (45)..(46)
<223> Xaa can be any naturally occurring amino acid

<220>
<221> misc_feature
<222> (96)..(96)
<223> Xaa can be any naturally occurring amino acid

<400> 124

```
Ser Ser Ser Ser Asn Ser Asn Leu Tyr Ile Lys Arg Gly Asn Gln Val
1               5                   10              15

Leu Lys Arg Thr Phe Ile Glu Leu Trp Arg Asp Thr Val Glu Glu His
            20                  25              30

Met Pro Phe Gly Leu Gly Thr His Gly Gln Glu Leu Xaa Xaa Ser Asp
            35                  40              45

Asp Tyr Val Leu Asn Tyr Asp Glu Lys Arg Gly Leu His Trp Val Pro
    50                  55                  60

Ile Lys Tyr Ile Met Lys His Gly Thr Lys Lys Arg Lys Phe Arg Val
65                  70                  75                  80

Lys Thr Lys Ser Gly Lys Glu Ile Ile Val Thr Glu Asp His Ser Xaa
            85                  90                  95

Val Val Ile Arg Asn Gly Glu Lys Ile Ala Val Lys Ala Ser Glu Ile
            100                 105                 110
```

Asn Lys Asp Thr Asp Lys Ile Val Ser Ile Ser Thr Lys
        115             120             125

<210> 125
<211> 49
<212> PRT
<213> Rattus norvegicus

<400> 125

Met Ile Glu Glu Gly Phe Asp Tyr Ile Ile Glu Glu Ile Glu Ser Val
1               5               10              15

Glu Asp Ile Gly Phe Phe Asp Glu Asp Glu Pro Val Phe Asp Ile Glu
            20              25              30

Val Glu Asp Asp Thr His Thr Phe Ile Gly Asn Asp Val Leu Val His
            35              40              45

Asn

<210> 126
<211> 198
<212> PRT
<213> Artificial sequence

<220>
<223> modified IntN from sheep gut metagenome

<220>
<221> misc_feature
<222> (24)..(24)
<223> Xaa can be any naturally occurring amino acid

<220>
<221> misc_feature
<222> (52)..(52)
<223> Xaa can be any naturally occurring amino acid

<220>
<221> misc_feature
<222> (178)..(178)
<223> Xaa can be any naturally occurring amino acid

<400> 126

Ser Phe Thr Glu Asp Thr Pro Val Phe Val Arg Asn Val Lys Asn Gly
1               5               10              15

Ala Ile Asp Ile Lys Pro Ile Xaa Glu Leu Ile Asp Glu Asn Lys Ile
            20              25              30

Glu Thr Asp Ala Leu Gly Arg Glu Tyr Asp Tyr Ser Pro Lys Pro Tyr

```
                35                      40                      45


        Gln Val Leu Xaa Arg Ser Gly Trp Val Thr Pro Ser Tyr Ile Tyr Arg
            50              55              60


        His Lys Thr Asp Lys Asp Ile Tyr Glu Ile Thr Asp Gly Asp Met Lys
        65              70              75              80


        Ile Glu Val Thr Glu Asp His Ser Leu Phe Asn Asp Lys Lys Glu Lys
                        85              90              95


        Ile Lys Pro Ser Glu Val Asn Asn Glu Thr His Leu Glu Tyr Phe Asn
                    100             105             110


        Asp Tyr Glu Val Phe Lys Lys Ala Lys Trp Leu Pro Ala Asp Thr Arg
                115             120             125


        Asn Pro His Phe Tyr Ala Lys Ala Leu Ala Asn Gly Lys Ile Asp Arg
            130             135             140


        Val Pro Ser Trp Phe Leu Asn Arg Pro Thr Lys Glu Gly Arg Glu Phe
        145             150             155             160


        Tyr Glu Val Phe Ile Lys Asn Tyr Arg Asp Asp Ile Gln Tyr Ser Lys
                        165             170             175


        Thr Xaa Leu Ala Gly Leu Tyr Phe Leu Lys Met Ile Ser Glu Val Ser
                    180             185             190


        Thr Tyr Ser Gly Ile Lys
                195


        <210>   127
        <211>   34
        <212>   PRT
        <213>   Unknown

        <220>
        <223>   IntC from sheep gut metagenome

        <400>   127

        Met Leu Glu Lys Thr Asn Lys Gly Lys Thr Ser Ala Tyr Val Tyr Asp
        1               5               10              15


        Ile Ser Leu Asp Gly Thr Val Val Asn Ala Leu Gly Met Asn Val Asn
                    20              25              30


        Ser Asn
```

<210> 128
<211> 195
<212> PRT
<213> Artificial sequence

<220>
<223> modified IntN from sheep gut metagenome

<220>
<221> misc_feature
<222> (52)..(52)
<223> Xaa can be any naturally occurring amino acid

<220>
<221> misc_feature
<222> (179)..(179)
<223> Xaa can be any naturally occurring amino acid

<400> 128

Ser Phe Thr Pro Asp Thr Pro Met Phe Ile Lys Tyr Lys Asp Ser Gly
1               5                   10                  15

Leu Ile Asp Ile Lys Pro Ile Glu Glu Leu Ile Asn Glu Lys Glu Ile
            20                  25                  30

Lys Ile Asp Ala Leu Gly Arg Glu Tyr Asp Tyr Ser Lys Lys Asp Tyr
            35                  40                  45

Tyr Val Leu Xaa Arg Ser Gly Trp Val Glu Pro Ser Tyr Ile Tyr Arg
    50                  55                  60

His Lys Thr Glu Lys Asp Ile Tyr Glu Ile Thr Asp Gly Glu Met Lys
65                  70                  75                  80

Val Glu Val Thr Glu Asp His Ser Leu Phe Asn Ser Lys Gln Glu Lys
                85                  90                  95

Ile Lys Pro Ser Glu Ile Thr Asn Lys Thr Glu Leu Glu Tyr Tyr Thr
            100                 105                 110

Glu Glu Val Arg Pro Asp Gly Tyr Thr Arg Tyr Phe Ala Thr Gln Ala
            115                 120                 125

Pro Gln Ala Lys Gln Met Ala Met Asp Leu Ala Asn Gly Lys Ile Asp
        130                 135                 140

Arg Val Pro Met Lys Val Leu Asn Tyr Gln Glu Val Tyr Gln Lys Ile
145                 150                 155                 160

```
Phe Tyr Asp Thr Phe Ile Glu Asn Tyr Lys Asn Asp Ile Lys Tyr Ser
            165                 170                 175


Lys Thr Xaa Leu Ala Gly Leu Gln Tyr Ile Arg Lys Met Leu Met Glu
            180                 185                 190


Lys Lys Phe
        195


<210>  129
<211>  33
<212>  PRT
<213>  Artificial sequence

<220>
<223>  modified IntC from sheep gut metagenome


<220>
<221>  misc_feature
<222>  (9)..(9)
<223>  Xaa can be any naturally occurring amino acid

<400>  129

Met Lys Ile Leu Asn Lys Gly Lys Xaa Leu Asp Tyr Val Tyr Asp Ile
1               5                   10                  15


Ser Leu Asp Gly Thr Val Val Asn Ala Leu Gly Met Asn Ile Leu Ser
            20                  25                  30


Asn


<210>  130
<211>  188
<212>  PRT
<213>  Artificial sequence

<220>
<223>  modified IntN from cow rumen metagenome


<220>
<221>  misc_feature
<222>  (50)..(50)
<223>  Xaa can be any naturally occurring amino acid

<220>
<221>  misc_feature
<222>  (153)..(153)
<223>  Xaa can be any naturally occurring amino acid

<220>
<221>  misc_feature
<222>  (185)..(185)
<223>  Xaa can be any naturally occurring amino acid
```

<400> 130

Ser Val Thr Glu Asp Thr Pro Leu Phe Val Arg Lys Asn Gly Met Ile
1               5                   10                  15

Asp Ile Lys Pro Ile Gly Glu Leu Phe Gly Ser Glu Asn Ile Glu Ser
            20                  25                  30

Tyr Pro Asp Gly Arg Glu Tyr Asp Arg Asn Glu Lys Asp Tyr Glu Val
        35                  40                  45

Leu Xaa Arg Ser Gly Trp Val Arg Pro Ser Tyr Ile Tyr Arg His Asn
    50                  55                  60

Thr Asp Lys Asp Val Tyr Lys Val Thr Gly Asp Gly Ile Glu Val Asp
65                  70                  75                  80

Ala Thr Arg Asp His Ser Leu Phe Asp Ala Asp Arg Asn Glu Ile Lys
                85                  90                  95

Pro Thr Glu Ile Ser Arg Gly Thr Lys Leu Glu Thr Tyr Ser Gly Asp
            100                 105                 110

Ser Leu Phe Asp Phe Asn Thr Ile Glu Leu Ser Asp Phe Ala Ile Asp
        115                 120                 125

Val Ala Ala Met Leu Val Met Thr Gly Lys Thr Asp Arg Ile Pro Asp
    130                 135                 140

Glu Ile Leu Asn Ala Thr Leu Glu Xaa Lys Ala Lys Phe Ile Asp Leu
145                 150                 155                 160

Leu Lys Asn Ala Glu Val Thr Thr Asp Arg Tyr Pro Lys Thr Leu Val
                165                 170                 175

Ala Gly Tyr Gln Tyr Ile Lys Asn Xaa Val Leu Leu
        180                 185

<210> 131
<211> 39
<212> PRT
<213> Artificial sequence

<220>
<223> modified IntC from cow rumen metagenome


<220>
<221> misc_feature
<222> (24)..(24)

<223> Xaa can be any naturally occurring amino acid

<400> 131

Met Gln Asn Asn Ile Ile Met Gln Ala Ile Lys Leu Lys Ser Glu Lys
1               5                   10                  15

Arg Thr Val Tyr Asp Ile Ser Xaa Asp Gly Thr Phe Val Asn Ala Leu
            20                  25                  30

Gly Met Asn Val Leu His Asn
        35

<210> 132
<211> 188
<212> PRT
<213> Artificial sequence

<220>
<223> modified IntN from Prevotella species CAG 1092 phage contig 445

<220>
<221> misc_feature
<222> (52)..(52)
<223> Xaa can be any naturally occurring amino acid

<220>
<221> misc_feature
<222> (83)..(83)
<223> Xaa can be any naturally occurring amino acid

<220>
<221> misc_feature
<222> (85)..(85)
<223> Xaa can be any naturally occurring amino acid

<220>
<221> misc_feature
<222> (120)..(120)
<223> Xaa can be any naturally occurring amino acid

<220>
<221> misc_feature
<222> (152)..(152)
<223> Xaa can be any naturally occurring amino acid

<400> 132

Ser Phe Thr Gly Asp Thr Pro Val Phe Ile Lys Tyr Asp Asn Thr Asn
1               5                   10                  15

Leu Ile Asp Ile Lys Pro Ile Ser Glu Leu Ile Asp Ile Asp Asn Ile
            20                  25                  30

Asp Lys Asp Val Leu Gly Arg Glu Tyr Asp Thr Ser Glu Lys Asn Tyr
        35                  40                  45

```
Ser Ile Leu Xaa Arg Ser Gly Trp Tyr Lys Pro Ser Tyr Ile Tyr Arg
    50                  55                  60

His Lys Thr Val Lys Asn Ile Tyr Arg Val Glu Asp Asn Thr Pro Ser
65                  70                  75                  80

Ala Gly Xaa Ile Xaa Asp Ile Thr Glu Asp His Ser Leu Phe Asn Asp
                85                  90                  95

Glu Arg Glu Lys Ile Lys Pro Ser Glu Ile Gly Gln Asn Thr Lys Leu
            100                 105                 110

Glu Tyr Lys Ser Lys Ile Phe Xaa Arg Arg Thr His Thr Ile Ser Asp
            115                 120                 125

Asp Lys Phe Asn Lys Leu Leu Asp Phe Thr Val Lys Phe Pro Ile Lys
        130                 135                 140

Ile Pro Ile Glu Ile Leu Asn Xaa Glu Ile Asn Thr Arg Lys Arg Phe
145                 150                 155                 160

Ala Tyr Glu Leu His Lys Arg Leu Lys Asp Ser Ile Asp Ile Gly His
            165                 170                 175

Tyr Ser Lys Val Phe Val Ala Gly Phe Lys Phe Leu
            180                 185
```

```
<210>  133
<211>  34
<212>  PRT
<213>  Prevotella species CAG 1092 phage

<400>  133
```

```
Met Ile Gln Val Glu Asn Ile Gly Lys Thr Asp Asp Tyr Val Tyr Asp
1                   5                   10                  15

Ile Ser Leu Asp Gly Thr Val Val Asn Ala Leu Gly Met Asn Val Leu
            20                  25                  30

Ser Asn
```

```
<210>  134
<211>  199
<212>  PRT
<213>  Artificial sequence

<220>
<223>  modified IntN from Lake Huron low oxygen high sulfur sink hole
```

purple microbial mat bin unclassified.01

```
<220>
<221>  misc_feature
<222>  (52)..(52)
<223>  Xaa can be any naturally occurring amino acid

<400>  134

Ser Phe Leu Tyr Asp Thr Pro Ile Tyr Ile Lys Tyr Lys Asn Ser Asp
1               5               10              15

Ile Ile Asp Ile Lys Ser Ile Gly Gly Val Phe Asn Ser Asn Glu Ser
            20              25              30

Asp Ile Asp Glu Leu Gly Arg Glu Tyr Asp Leu Ser Glu Lys Pro Tyr
        35              40              45

Gln Val Leu Xaa Arg Gly Gly Trp Ile Asp Val Asn Tyr Val Tyr Arg
        50              55              60

His Lys Thr Asp Lys Gln Ile His Arg Ile Ser Phe Asn Asp Gly Tyr
65              70              75              80

Val Asp Val Thr Ala Asp His Ser Val Phe Asn Glu Asn Lys Glu Lys
            85              90              95

Leu His Ser Lys Asp Val Ile Pro Asn Glu Thr Lys Leu Glu Met Ala
            100             105             110

Asn Leu Asp Tyr Ser Asn Phe Ile Ile Lys Gln Gln Asn Leu Asn Val
            115             120             125

Glu Thr Ile Glu Lys Met Ala Ser Val Leu Ala Leu Ser Val Asp Ile
        130             135             140

Asn Lys Lys Val Pro Ala Glu Ile Ile Asn Thr Asn Lys Glu Asn Gln
145             150             155             160

Ile Ile Phe Leu Asn Lys Phe Met Ser Val Thr Lys Leu Asn Lys Val
            165             170             175

Ser Gln Asp Asn Glu Asn Lys Val Leu Lys Ala Gly Ile Leu Phe Leu
            180             185             190

Val Asn Arg Thr Lys Asn Asn
            195

<210>  135
```

<211> 40
<212> PRT
<213> Artificial sequence

<220>
<223> modified IntC from Lake Huron low oxygen high sulfur sink hole purple microbial mat bin unclassified.01

<220>
<221> misc_feature
<222> (24)..(24)
<223> Xaa can be any naturally occurring amino acid

<400> 135

Met Asn Asn Leu Val Leu Gly Asn Glu Ile Ile Asp Asn Tyr Asp Pro
1               5                   10                  15

Glu Met Tyr Val Tyr Asp Ile Xaa Leu Asp Gly Thr Leu Ile Asn Ala
            20                  25                  30

Leu Gly Asn Asn Val Val Thr Gln
        35                  40

<210> 136
<211> 186
<212> PRT
<213> Artificial sequence

<220>
<223> modified IntN from sheep gut metagenome

<220>
<221> misc_feature
<222> (50)..(50)
<223> Xaa can be any naturally occurring amino acid

<400> 136

Ser Phe Thr Ser Asp Thr Pro Val Phe Ile Lys Tyr Asp Lys Ser Gly
1               5                   10                  15

Leu Ile Asp Ile Lys Ala Ile Ser Glu Leu Ile Gly Glu Thr Glu Val
            20                  25                  30

Asp Gly Leu Gly Arg Glu Tyr Asp Tyr Ser Glu Lys Asp Tyr Thr Val
            35                  40                  45

Leu Xaa Arg Ser Gly Trp Val Lys Pro Lys Tyr Ile Tyr Arg His Lys
        50                  55                  60

Thr Asp Lys Asp Ile Tyr Arg Val Glu Asp Asn Gly Ala Met Ile Asp
65                  70                  75                  80

```
Val Thr Glu Asp His Ser Leu Phe Asn Asp Lys Gln Glu Lys Ile Lys
                85                  90                  95

Pro Ser Glu Ile Asn Asp Glu Thr Lys Leu Glu Tyr Tyr Lys Asp Glu
               100                 105                 110

Ile Thr Pro Glu Gly Thr Met Lys Trp Leu Asn Glu Thr Arg Ala Arg
               115                 120                 125

Arg Leu Ala Lys Trp Ile Lys Asp Gly Thr Leu Thr Glu Val Pro Leu
               130                 135                 140

Pro Ile Leu Asn Ser Met Asn Arg Gly His Ile Asn Ala Phe Leu Asp
145                 150                 155                 160

Glu Leu Gly Asn Trp Asp Tyr Ser Lys Ser Ser Lys Val Leu Gln Ala
               165                 170                 175

Gly Ile Met Tyr Val Lys Asn Lys Arg Arg
               180                 185
```

```
<210>  137
<211>  34
<212>  PRT
<213>  Unknown

<220>
<223>  IntC from sheep gut metagenome

<400>  137
```

```
Met Ala Gln Ala Thr Lys Ile Asn Lys Thr Asp Asp Tyr Val Tyr Asp
1               5                   10                  15

Ile Ser Leu Asp Gly Thr Val Val Asn Ala Leu Gly Leu Asn Val Ile
                20                  25                  30

Ser Asn
```

```
<210>  138
<211>  185
<212>  PRT
<213>  Artificial sequence

<220>
<223>  modified IntN from sheep gut metagenome


<220>
<221>  misc_feature
<222>  (52)..(52)
```

<223> Xaa can be any naturally occurring amino acid

<220>
<221> misc_feature
<222> (155)..(155)
<223> Xaa can be any naturally occurring amino acid

<220>
<221> misc_feature
<222> (171)..(171)
<223> Xaa can be any naturally occurring amino acid

<220>
<221> misc_feature
<222> (184)..(184)
<223> Xaa can be any naturally occurring amino acid

<400> 138

Ser Phe Thr Glu Asp Thr Pro Leu Phe Ile Lys Tyr Asn Asn Ser Gly
1                5                  10                15

Leu Ile Asp Ile Lys Pro Ile Ser Glu Leu Val Asn Glu Asp Lys Ile
           20                  25                  30

Glu Phe Asp Gly Leu Gly Arg Glu Tyr Asp Tyr Ser Lys Lys Asp Phe
           35                  40                  45

Lys Val Leu Xaa Arg Ser Gly Trp Val Glu Pro Ser Tyr Ile Tyr Arg
        50                  55                  60

His Lys Thr Thr Lys Pro Ile Tyr Arg Ile Ser Asp Asp Glu Val Lys
65                  70                  75                  80

Met Ser Ile Asp Val Thr Glu Asp His Ser Leu Phe Asn Glu Lys Gln
                85                  90                  95

Glu Lys Ile Lys Pro Ser Glu Ile Asn Ser Asp Thr Lys Leu Glu Tyr
           100                 105                 110

Tyr Asn Asn Asn Ile Ser Asn Asn Gln Ile Val Ile Asp Glu Ile Arg
        115                 120                 125

Ile Asp Lys Tyr Ser Lys Leu Leu Ser Asn Gly Arg Leu Asn Ala Ile
    130                 135                 140

Pro Ile Asp Leu Leu Asn Ala Thr Val Glu Xaa Lys Lys Glu Phe Leu
145                 150                 155                 160

Ser Lys Met Asp Leu Ser Lys Val Ser Glu Xaa Lys Thr Leu Ile Ala
                165                 170                 175

```
Gly Ile Leu Tyr Leu Arg Ser Xaa Ile
            180                 185


<210>  139
<211>  36
<212>  PRT
<213>  Unknown

<220>
<223>  IntC from sheep gut metagenome

<400>  139

Met Leu Asn Ser Lys Lys Ile Lys Gly Lys Glu Glu Val Gly Val Val
1               5               10                  15


Tyr Asp Ile Ser Leu Asp Gly Thr Val Val Asn Ala Leu Gly Met Asn
            20              25                  30


Val Ile Ser Asn
            35


<210>  140
<211>  195
<212>  PRT
<213>  Artificial sequence

<220>
<223>  modified IntN from sheep gut metagenome


<220>
<221>  misc_feature
<222>  (49)..(49)
<223>  Xaa can be any naturally occurring amino acid

<220>
<221>  misc_feature
<222>  (174)..(174)
<223>  Xaa can be any naturally occurring amino acid

<400>  140

Ser Phe Thr Gly Asp Thr Pro Leu Phe Ile Lys Tyr Asn Asp Gly Lys
1               5               10                  15


Ile Asp Ile Lys Pro Ile Glu Glu Leu Ile Gly Glu Thr Glu Thr Asp
            20              25                  30


Ala Leu Gly Arg Glu Tyr Asp Tyr Ser Lys Lys Pro Tyr Lys Val Leu
            35              40                  45


Xaa Arg Ser Gly Trp Val Arg Pro Ser Tyr Ile Tyr Arg His Lys Thr
    50              55                  60
```

```
Asn Lys Pro Leu Tyr Thr Val Ser Glu Gly Asn Met Ser Val Thr Val
65              70              75              80


Thr Glu Asp His Ser Leu Phe Asn Asp Lys Gln Lys Lys Ile Lys Pro
                85              90              95


Ser Glu Ile Thr Glu Gly Thr Arg Leu Glu Tyr Tyr Thr Asp Lys Val
            100             105             110


Glu Thr Ser Thr Lys Gly Phe Ile His Leu Asn Glu Gln Arg Val Lys
        115             120             125


Ile Met Ala Lys Thr Leu Lys Asn Gly Val Ile Asn Arg Val Pro Ile
    130             135             140


Gln Leu Phe Asn Thr Asn Asn Ile Asp Ala Val Lys Thr Phe Leu Asn
145             150             155             160


Glu Leu Glu Gly Trp Asp Tyr Ser Asn Thr Ser Lys Thr Xaa Arg Ala
                165             170             175


Gly Ile Gln Phe Leu Lys Lys Lys Ile Asp Gly Phe Asn Phe His Asp
            180             185             190


Val Tyr Lys
        195
```

```
<210>  141
<211>  38
<212>  PRT
<213>  Artificial sequence

<220>
<223>  modified IntC from sheep gut metagenome


<220>
<221>  misc_feature
<222>  (12)..(12)
<223>  Xaa can be any naturally occurring amino acid

<400>  141

Met Asn Asn Ile Asn Ile Lys Lys Met Glu Asp Xaa Gln Asp Ile Gly
1               5               10              15


Val Val Tyr Asp Ile Ser Leu Asp Gly Thr Val Val Asn Ala Leu Gly
            20              25              30


Met Asn Val Ile Ser Asn
        35
```

```
<210>  142
<211>  193
<212>  PRT
<213>  Artificial sequence

<220>
<223>  modified IntN from sheep gut metagenome


<220>
<221>  misc_feature
<222>  (23)..(23)
<223>  Xaa can be any naturally occurring amino acid

<220>
<221>  misc_feature
<222>  (49)..(49)
<223>  Xaa can be any naturally occurring amino acid

<220>
<221>  misc_feature
<222>  (167)..(167)
<223>  Xaa can be any naturally occurring amino acid

<220>
<221>  misc_feature
<222>  (173)..(173)
<223>  Xaa can be any naturally occurring amino acid

<400>  142
```

Ser Phe Thr Ala Asp Thr Pro Leu Phe Ile Lys Tyr Asp Asp Gly Lys
1               5                   10                  15

Ile Asp Ile Lys Pro Ile Xaa Glu Leu Ile Gly Glu Thr Glu Thr Asp
            20                  25                  30

Lys Leu Gly Arg Glu Tyr Asp Tyr Ser Pro Lys Pro Tyr Arg Val Leu
        35                  40                  45

Xaa Arg Ser Gly Trp Met Arg Pro Ser Tyr Ile Tyr Arg His Lys Thr
    50                  55                  60

Asn Lys Pro Leu Tyr Glu Val Ser Glu Gly Asn Met Ser Ile Thr Val
65                  70                  75                  80

Thr Glu Asp His Ser Leu Phe Asn Asp Lys Gln His Lys Ile Lys Pro
                85                  90                  95

Ser Glu Ile Thr Glu Asn Thr Lys Leu Glu Tyr Tyr Lys Lys Ser Ile
            100                 105                 110

Glu Thr Asp Thr Lys Tyr Arg Trp Leu Thr Glu Asp Arg Ala Arg Arg
        115                 120                 125

Met Ser Lys Met Val Leu Asp Gly Thr Ile Asp Arg Val Pro Met Ala
          130                 135                 140

Ile Leu Asn Thr Glu Asn Leu Lys Ile Val Met Ala Phe Leu Lys Glu
145                 150                 155                 160

Trp Glu Tyr Met Pro Leu Xaa Thr Phe Ser Lys Thr Xaa Gln Ala Gly
                  165                 170                 175

Ile Asn Phe Leu Lys Met Lys Leu Tyr Gly Gln Asn Arg Asn Ile His
                  180                 185                 190

Lys


<210> 143
<211> 33
<212> PRT
<213> Unknown

<220>
<223> IntC from sheep gut metagenome

<400> 143

Met Lys Ile Lys Asn Ile Gly Ser Thr Ser Asp Tyr Val Tyr Asp Ile
1               5                   10                  15

Gln Leu Asp Gly Thr Val Val Asn Ala Leu Gly Met Asn Ile Ile Ser
              20                  25                  30

Asn


<210> 144
<211> 121
<212> PRT
<213> Unknown

<220>
<223> IntN from Antarctica Vida lake environmental sampling
      brine-hole-2

<400> 144

Ser Val Thr Gly Asp Thr Leu Ile Thr Leu Ser Asp Lys Lys Ile Ser
1               5                   10                  15

Ile Glu Asp Leu Phe Asn Arg Phe Asp Tyr Arg Val Ile Gln Asp Gln
              20                  25                  30

Gly Lys Glu Tyr Ala Ile Pro Arg Thr Glu Leu Glu Lys Glu Asn Asn


154

                35                        40                        45

Ala Val Leu Gly Tyr Asn Ala Phe Glu Asp Glu Ala Val Leu Gly Glu
        50                      55                      60

Ile Ala Tyr Val Met Arg His Lys Thr Lys Lys Lys Leu Tyr Glu Ile
65                      70                      75                      80

Glu Thr Asp Asp Gly Lys Lys Val Thr Val Thr Glu Asp His Ser Leu
                85                      90                      95

Ile Val Asp Arg His Gly Ile Thr Thr Glu Val Thr Pro Lys Asn Leu
                100                     105                     110

Glu Glu Asp Asp Leu Ile Ile Thr Ile
            115                     120

```
<210>  145
<211>  42
<212>  PRT
<213>  Artificial sequence

<220>
<223>  modified IntC from Antarctica Vida lake environmental sampling
       brine-hole-2


<220>
<221>  misc_feature
<222>  (13)..(13)
<223>  Xaa can be any naturally occurring amino acid

<400>  145
```

Met Asn Thr Ile Arg Thr Lys Val Lys Arg Val Thr Xaa Leu Gly Glu
1                   5                   10                      15

Val Asp Asp Tyr Val Tyr Asp Val Ser Met Val Arg Gln Asp Pro Phe
                20                      25                      30

Phe Phe Ala Asn Asp Ile Leu Val His Asn
            35                      40

```
<210>  146
<211>  187
<212>  PRT
<213>  Artificial sequence

<220>
<223>  modified IntN (partial) from sheep gut metagenome


<220>
<221>  misc_feature
```

<222> (49)..(49)
<223> Xaa can be any naturally occurring amino acid

<220>
<221> misc_feature
<222> (153)..(153)
<223> Xaa can be any naturally occurring amino acid

<220>
<221> misc_feature
<222> (173)..(173)
<223> Xaa can be any naturally occurring amino acid

<400> 146

```
Ser Phe Thr Gly Asp Thr Pro Leu Phe Ile Lys Tyr Asp Asn Gly Ile
1               5                   10              15


Ile Asp Ile Val Pro Ile Ser Asp Leu Ile Gly Asp Thr Glu Met Asp
            20              25              30


Asp Leu Gly Arg Glu Tyr Asp Val Ser Asp Lys Pro Tyr Lys Val Leu
        35              40              45


Xaa Arg Ser Gly Trp Val Lys Pro Glu Tyr Ile Tyr Arg His Lys Thr
    50              55              60


Lys Lys Pro Leu Tyr Glu Val Ser Asp Gly Asp Met Thr Val Met Val
65              70              75              80


Thr Glu Asp His Ser Leu Phe Asn Asn Lys Gln Glu Lys Ile Lys Pro
                85              90              95


Ser Glu Ile Asn Glu Lys Thr Lys Leu Glu Tyr Tyr Gly His Lys Ile
            100             105             110


Glu Ser Ser Tyr Glu Tyr Gly Trp Leu Asn Glu Lys Arg Ala Leu Arg
        115             120             125


Met Ala Lys Trp Leu Lys Asp Gly Thr Leu Asn Gln Val Pro Thr Pro
    130             135             140


Leu Leu Asn Thr Lys Lys Ile Asn Xaa Ile Lys Val Phe Leu Gly Glu
145             150             155             160


Leu Gln Gly Phe Asp Phe Asn Asn Ala Thr Lys Thr Xaa Arg Ala Gly
                165             170             175


Ile Leu Phe Leu Lys Glu Lys Leu Lys Asn Lys
            180             185
```

```
<210>  147
<211>  124
<212>  PRT
<213>  Artificial sequence

<220>
<223>  modified IntN (partial) from sheep gut metagenome


<220>
<221>  misc_feature
<222>  (16)..(16)
<223>  Xaa can be any naturally occurring amino acid

<400>  147
```

Ser Val Thr Glu Asp Ser Leu Ile Arg Thr Gly Ser Gly Asn Ile Xaa
1               5                   10                  15

Val Lys Asp Ile Trp Asp Lys Tyr Ser Lys Glu Ile Pro Glu Val Phe
            20                  25                  30

Glu Tyr Ser Asn Asn Phe Lys Thr Ala Tyr Leu Lys Met Asn Asp Lys
        35                  40                  45

Glu Tyr Ile Leu Asn Pro Val Met Thr Ile Leu Gly Ser Asp Asp Arg
    50                  55                  60

Leu Tyr Ile Pro Arg Tyr Ile Met Lys His Lys Thr Lys Lys Lys Leu
65                  70                  75                  80

Tyr Lys Ile Thr Thr Glu Ser Gly Lys Gln Val Thr Val Thr Glu Asp
            85                  90                  95

His Ser Leu Ile Val Val Arg Asp Asn Val Lys Thr Val Ile Lys Pro
        100                 105                 110

Thr Glu Leu Leu Asp Thr Asp Glu Val Ile Val Ile
        115                 120

```
<210>  148
<211>  43
<212>  PRT
<213>  Artificial sequence

<220>
<223>  modified IntC (partial) from sheep gut metagenome


<220>
<221>  misc_feature
<222>  (33)..(33)
<223>  Xaa can be any naturally occurring amino acid

<400>  148
```

```
Met Ile Asn Met Tyr Leu Glu His Ile Lys Ser Val Glu Val Leu Glu
1               5                   10                  15

Pro Thr Glu Asp Leu Asp Val Tyr Asp Leu Glu Leu Pro Val Asp His
                20                  25                  30

Xaa Phe Phe Ala Asn Asp Ile Leu Val His Asn
            35                  40
```

<210> 149
<211> 53
<212> PRT
<213> Artificial sequence

<220>
<223> modified IntC (partial) from sheep gut metagenome

<220>
<221> misc_feature
<222> (5)..(5)
<223> Xaa can be any naturally occurring amino acid

<220>
<221> misc_feature
<222> (43)..(43)
<223> Xaa can be any naturally occurring amino acid

<400> 149

```
Met Pro Val Ile Xaa Leu Lys Glu Ile Ile Lys Leu Tyr Lys Val Ser
1               5                   10                  15

Asn Ile Lys Ser Ile Glu Val Ile Thr Pro Glu Glu Pro Ile Asp Val
                20                  25                  30

Tyr Asp Ile Glu Met Pro Asp Asp Asp His Xaa Phe Phe Ala Asn Asp
            35                  40                  45

Ile Leu Val His Asn
        50
```

<210> 150
<211> 47
<212> PRT
<213> Artificial sequence

<220>
<223> modified IntC (partial) from sheep gut metagenome

<220>
<221> misc_feature
<222> (37)..(37)
<223> Xaa can be any naturally occurring amino acid

<400> 150

```
Met Gly Gly Glu Trp Glu Met Lys Ile Glu Ser Val Lys Ser Ile Glu
1               5                   10                  15

Val Val Thr Pro Glu Glu Pro Ile Asp Val Tyr Asp Ile Glu Met Pro
            20                  25                  30

Asp Asp Asp His Xaa Phe Phe Ala Asn Asp Ile Leu Val His Asn
        35                  40                  45
```

<210> 151
<211> 50
<212> PRT
<213> Artificial sequence

<220>
<223> modified IntC (partial) from sheep gut metagenome

<220>
<221> misc_feature
<222> (40)..(40)
<223> Xaa can be any naturally occurring amino acid

<400> 151

```
Met Leu Lys Tyr Ile Leu Glu Val Val Ser Met Asn Leu Ser Lys Ile
1               5                   10                  15

Lys Ser Val Glu Val Ile Thr Pro Glu Glu Pro Thr Glu Val Tyr Asp
            20                  25                  30

Ile Glu Leu Pro Val Asp His Xaa Phe Tyr Ala Asn Asp Ile Leu Val
            35                  40                  45

His Asn
        50
```

<210> 152
<211> 42
<212> PRT
<213> Artificial sequence

<220>
<223> modified IntC from Antarctica Vida lake environmental sampling
      brine-hole-2

<220>
<221> misc_feature
<222> (13)..(13)
<223> Xaa can be any naturally occurring amino acid

<400> 152

Met Asp Thr Asn Arg Ala Lys Val Lys Ser Val Lys Xaa Leu Gly Glu
1                   5                   10                  15

Val Asp Asp Tyr Val Tyr Asp Val Ser Met Lys Asp Gln Asp Pro Phe
                20                  25                  30

Phe Phe Ala Asn Gly Ile Leu Val His Asn
            35                  40

<210>  153
<211>  379
<212>  PRT
<213>  Artificial sequence

<220>
<223>  modified contiguous intein from Nanosalina species J07AB43

<220>
<221>  misc_feature
<222>  (151)..(151)
<223>  Xaa can be any naturally occurring amino acid

<220>
<221>  misc_feature
<222>  (154)..(154)
<223>  Xaa can be any naturally occurring amino acid

<220>
<221>  misc_feature
<222>  (187)..(187)
<223>  Xaa can be any naturally occurring amino acid

<220>
<221>  misc_feature
<222>  (213)..(213)
<223>  Xaa can be any naturally occurring amino acid

<220>
<221>  misc_feature
<222>  (277)..(277)
<223>  Xaa can be any naturally occurring amino acid

<220>
<221>  misc_feature
<222>  (379)..(379)
<223>  Xaa can be any naturally occurring amino acid

<400>  153

Ser Leu Asn Tyr Ser Arg Gln Val Val Val Lys Asn Pro Asn Asn Glu
1                   5                   10                  15

Ile Gln Phe Met Glu Val Gly Lys Phe Val Glu Asp Ile Asp Val Pro
                20                  25                  30

Glu Asn Tyr Glu Thr Leu Ala Trp Asp Glu Glu Lys Asp Arg Ser Val

```
                35                      40                      45

        Phe Lys Pro Val Lys Arg Ala Ile Met His Lys Tyr Asn Gly Asn Leu
            50                  55                  60

        Leu Arg Phe Asp Thr Ser Arg Gly Arg Thr Glu Val Thr Pro Gln His
        65                  70                  75                  80

        Ser Val Tyr Arg Tyr Glu Asp Gly Glu Ile Lys Leu Ala Asp Ala Glu
                        85                  90                  95

        Asp Leu Glu Glu Gly Asp Arg Leu Val Ser Leu Ser Glu Leu Pro Glu
                    100                 105                 110

        Thr Glu Lys Lys Tyr Ser Glu Gly Asp Thr Ile Asp Leu Ala Asp Leu
                    115                 120                 125

        Glu Tyr Glu Asn Ser Asp Leu Met Ala Tyr Arg Asp Lys Lys Lys Phe
            130                 135                 140

        Pro Ala Glu Lys Gly Glu Xaa Pro Tyr Xaa Gly Glu Val Tyr Tyr Leu
        145                 150                 155                 160

        Ser Ser His Val His Arg Asp His Gln Asp Arg Arg Ile Ala Leu Gly
                        165                 170                 175

        Glu Ala Ser Gln Asp Tyr Ser Tyr Ile Gly Xaa Lys Asn Ala Lys Ala
                    180                 185                 190

        Gly Lys Ile Pro Arg Phe Trp Lys Leu Thr Ser Glu Leu Ala Trp Ile
                    195                 200                 205

        Leu Gly Phe Tyr Xaa Gly Asp Gly Ser Ala Ser Leu Gly Asp Lys Gln
                    210                 215                 220

        Met Val Ser Phe Gly Gly Gln Asn Lys Glu Asn Ile Arg Arg Val Lys
        225                 230                 235                 240

        Arg Phe Phe Asp Gln Ile Leu Asp Glu Glu Leu Ser Ile Ile Glu Asp
                        245                 250                 255

        Val Asp Ser Arg Thr Gly Gly Lys Met Tyr Tyr Tyr Arg Ile Gln Arg
                    260                 265                 270

        Ile Pro Val Val Xaa Leu Phe Val Asn Gly Leu Gly Ala Gly Ser Gly
                    275                 280                 285
```

Ser Asp Gly Lys Lys Val Pro Ser Met Ile Ile Asn Gly Asp Lys Gly
    290                 295             300

Leu Arg Lys Ala Phe Val Glu Gly Tyr Phe Asp Ala Asp Gly Ser Arg
    305                 310             315                 320

Asp Lys Asp Tyr Asp Asp Arg Tyr Asp Ser Glu Asn Met Arg Phe Ser
                325             330             335

Thr Lys Ser Ser Tyr Leu Ala Asn Gln Val Gln Tyr Ile Leu Lys Gln
        340             345             350

Leu Asn Leu Gly Glu Asn Arg Tyr Val Glu Ile Ser Ala Met Ser Leu
        355             360             365

Ser Ser Ile Val Lys Ile Asn Leu Arg Ser Xaa
        370             375

<210> 154
<211> 351
<212> PRT
<213> Artificial sequence

<220>
<223> modified contiguous intein from Acanthamoeba castellanii
      mamavirus Hal-V

<220>
<221> misc_feature
<222> (88)..(88)
<223> Xaa can be any naturally occurring amino acid

<220>
<221> misc_feature
<222> (110)..(110)
<223> Xaa can be any naturally occurring amino acid

<220>
<221> misc_feature
<222> (147)..(147)
<223> Xaa can be any naturally occurring amino acid

<220>
<221> misc_feature
<222> (173)..(173)
<223> Xaa can be any naturally occurring amino acid

<220>
<221> misc_feature
<222> (259)..(259)
<223> Xaa can be any naturally occurring amino acid

<220>
<221> misc_feature
<222> (262)..(262)
<223> Xaa can be any naturally occurring amino acid

<220>
<221> misc_feature
<222> (272)..(272)
<223> Xaa can be any naturally occurring amino acid

<400> 154

Ser Val Thr Gly Asp Thr Pro Ile Ile Thr Arg His Gln Asn Gly Asp
1               5                   10                  15

Ile Asn Ile Thr Thr Ile Glu Glu Leu Gly Ser Lys Trp Lys Pro Tyr
            20                  25                  30

Glu Ile Phe Lys Ala His Glu Lys Asn Ser Asn Arg Lys Phe Lys Gln
            35                  40                  45

Gln Ser Gln Tyr Pro Thr Asp Ser Glu Val Trp Thr Ala Lys Gly Trp
        50                  55                  60

Ala Lys Ile Lys Arg Val Ile Arg His Lys Thr Val Lys Lys Ile Tyr
65                  70                  75                  80

Arg Val Leu Thr His Thr Gly Xaa Ile Asp Val Thr Glu Asp His Ser
                85                  90                  95

Leu Leu Asp Pro Asn Gln Asn Ile Ile Lys Pro Ile Asn Xaa Gln Ile
            100                 105                 110

Gly Thr Glu Leu Leu His Gly Phe Pro Glu Ser Asn Asn Val Tyr Asp
        115                 120                 125

Asn Ile Ser Glu Gln Glu Ala Tyr Val Trp Gly Phe Phe Met Gly Asp
    130                 135                 140

Gly Ser Xaa Gly Ser Tyr Gln Thr Lys Asn Gly Ile Lys Tyr Ser Trp
145                 150                 155                 160

Ala Leu Asn Asn Gln Asp Leu Asp Val Leu Asn Lys Xaa Lys Lys Tyr
                165                 170                 175

Leu Glu Glu Thr Glu Asn Ile Gln Phe Lys Ile Leu Asp Thr Met Lys
            180                 185                 190

Ser Ser Ser Val Tyr Lys Leu Val Pro Ile Arg Lys Ile Lys Tyr Met
        195                 200                 205

Val Asn Lys Tyr Arg Lys Ile Phe Tyr Asp Asn Lys Lys Tyr Lys Leu
    210                 215                 220

```
Val Pro Lys Glu Ile Leu Asn Ser Thr Lys Asp Ile Lys Asn Ser Phe
225             230             235             240

Leu Glu Gly Tyr Tyr Ala Ala Asp Gly Ser Arg Lys Glu Thr Glu Asn
                245             250             255

Met Gly Xaa Arg Arg Xaa Asp Ile Lys Gly Lys Ile Ser Ala Gln Xaa
            260             265             270

Leu Phe Tyr Leu Leu Lys Ser Leu Gly Tyr Asn Val Ser Ile Asn Ile
        275             280             285

Arg Ser Asp Lys Asn Gln Ile Tyr Arg Leu Thr Phe Ser Asn Lys Lys
    290             295             300

Gln Arg Lys Asn Pro Ile Ala Ile Lys Lys Ile Gln Leu Met Asn Glu
305             310             315             320

Thr Ser Asn Asp His Asp Gly Asp Tyr Val Tyr Asp Leu Glu Thr Glu
            325             330             335

Ser Gly Ser Phe His Ala Gly Val Gly Glu Met Ile Val Lys Asn
        340             345             350
```

<210> 155
<211> 351
<212> PRT
<213> Artificial sequence

<220>
<223> modified contiguous intein from Acanthamoeba polyphaga
     lentillevirus

<220>
<221> misc_feature
<222> (88)..(88)
<223> Xaa can be any naturally occurring amino acid

<220>
<221> misc_feature
<222> (110)..(110)
<223> Xaa can be any naturally occurring amino acid

<220>
<221> misc_feature
<222> (147)..(147)
<223> Xaa can be any naturally occurring amino acid

<220>
<221> misc_feature
<222> (173)..(173)
<223> Xaa can be any naturally occurring amino acid

<220>
<221>  misc_feature
<222>  (259)..(259)
<223>  Xaa can be any naturally occurring amino acid

<220>
<221>  misc_feature
<222>  (262)..(262)
<223>  Xaa can be any naturally occurring amino acid

<220>
<221>  misc_feature
<222>  (272)..(272)
<223>  Xaa can be any naturally occurring amino acid

<400>  155

Ser Val Thr Gly Asp Thr Pro Ile Ile Thr Arg His Gln Asn Gly Asp
1                5                   10                  15

Ile Asn Ile Thr Thr Ile Glu Glu Leu Gly Ser Lys Trp Lys Pro Tyr
            20                  25                  30

Glu Ile Phe Lys Ala His Glu Lys Asn Ser Asn Arg Lys Phe Lys Gln
            35                  40                  45

Gln Ser Gln Tyr Pro Thr Asp Ser Glu Val Trp Thr Ala Lys Gly Trp
            50                  55                  60

Ala Lys Ile Lys Arg Val Ile Arg His Lys Thr Val Lys Lys Ile Tyr
65                  70                  75                  80

Arg Val Leu Thr His Thr Gly Xaa Ile Asp Val Thr Glu Asp His Ser
                85                  90                  95

Leu Leu Asp Pro Asn Gln Asn Ile Ile Lys Pro Ile Asn Xaa Gln Ile
            100                 105                 110

Gly Thr Glu Leu Leu His Gly Phe Pro Glu Ser Asn Asn Val Tyr Asp
            115                 120                 125

Asn Ile Ser Glu Gln Glu Ala Tyr Val Trp Gly Phe Phe Met Gly Asp
            130                 135                 140

Gly Ser Xaa Gly Ser Tyr Gln Thr Lys Asn Gly Ile Lys Tyr Ser Trp
145                 150                 155                 160

Ala Leu Asn Asn Gln Asp Leu Asp Val Leu Asn Lys Xaa Lys Lys Tyr
                165                 170                 175

Leu Glu Glu Thr Glu Asn Ile Gln Phe Lys Ile Leu Asp Thr Met Lys
                180                 185                 190

```
Ser Ser Ser Val Tyr Lys Leu Val Pro Ile Arg Lys Ile Lys Tyr Met
        195                 200                 205

Val Asn Lys Tyr Arg Lys Ile Phe Tyr Asp Asn Lys Lys Tyr Lys Leu
        210                 215                 220

Val Pro Lys Glu Ile Leu Asn Ser Thr Lys Asp Ile Lys Asn Ser Phe
225                 230                 235                 240

Leu Glu Gly Tyr Tyr Ala Ala Asp Gly Ser Arg Lys Glu Thr Glu Asn
                245                 250                 255

Met Gly Xaa Arg Arg Xaa Asp Ile Lys Gly Lys Ile Ser Ala Gln Xaa
            260                 265                 270

Leu Phe Tyr Leu Leu Lys Ser Leu Gly Tyr Asn Val Ser Ile Asn Ile
            275                 280                 285

Arg Ser Asp Lys Asn Gln Ile Tyr Arg Leu Thr Phe Ser Asn Lys Lys
        290                 295                 300

Gln Arg Lys Asn Pro Ile Ala Ile Lys Lys Ile Gln Leu Met Asn Glu
305                 310                 315                 320

Thr Ser Asn Asp His Asp Gly Asp Tyr Val Tyr Asp Leu Glu Thr Glu
                325                 330                 335

Ser Gly Ser Phe His Ala Gly Val Gly Glu Met Ile Val Lys Asn
                340                 345                 350


<210>  156
<211>  351
<212>  PRT
<213>  Artificial sequence

<220>
<223>  modified contiguous intein from Mimivirus Rowbotham-Bradford


<220>
<221>  misc_feature
<222>  (88)..(88)
<223>  Xaa can be any naturally occurring amino acid

<220>
<221>  misc_feature
<222>  (110)..(110)
<223>  Xaa can be any naturally occurring amino acid

<220>
<221>  misc_feature
```

<222> (147)..(147)
<223> Xaa can be any naturally occurring amino acid

<220>
<221> misc_feature
<222> (173)..(173)
<223> Xaa can be any naturally occurring amino acid

<220>
<221> misc_feature
<222> (259)..(259)
<223> Xaa can be any naturally occurring amino acid

<220>
<221> misc_feature
<222> (262)..(262)
<223> Xaa can be any naturally occurring amino acid

<220>
<221> misc_feature
<222> (272)..(272)
<223> Xaa can be any naturally occurring amino acid

<400> 156

```
Ser Val Thr Gly Asp Thr Pro Ile Ile Thr Arg His Gln Asn Gly Asp
1               5                   10                  15

Ile Asn Ile Thr Thr Ile Glu Glu Leu Gly Ser Lys Trp Lys Pro Tyr
            20                  25                  30

Glu Ile Phe Lys Ala His Glu Lys Asn Ser Asn Arg Lys Phe Lys Gln
        35                  40                  45

Gln Ser Gln Tyr Pro Thr Asp Ser Glu Val Trp Thr Ala Lys Gly Trp
        50                  55                  60

Ala Lys Ile Lys Arg Val Ile Arg His Lys Thr Val Lys Lys Ile Tyr
65                  70                  75                  80

Arg Val Leu Thr His Thr Gly Xaa Ile Asp Val Thr Glu Asp His Ser
                85                  90                  95

Leu Leu Asp Pro Asn Gln Asn Ile Ile Lys Pro Ile Asn Xaa Gln Ile
                100                 105                 110

Gly Thr Glu Leu Leu His Gly Phe Pro Glu Ser Asn Asn Val Tyr Asp
                115                 120                 125

Asn Ile Ser Glu Gln Glu Ala Tyr Val Trp Gly Phe Phe Met Gly Asp
                130                 135                 140

Gly Ser Xaa Gly Ser Tyr Gln Thr Lys Asn Gly Ile Lys Tyr Ser Trp
145                 150                 155                 160
```

```
Ala Leu Asn Asn Gln Asp Leu Asp Val Leu Asn Lys Xaa Lys Lys Tyr
                165                 170                 175

Leu Glu Glu Thr Glu Asn Ile Gln Phe Lys Ile Leu Asp Thr Met Lys
                180                 185                 190

Ser Ser Ser Val Tyr Lys Leu Val Pro Ile Arg Lys Ile Lys Tyr Met
                195                 200                 205

Val Asn Lys Tyr Arg Lys Ile Phe Tyr Asp Asn Lys Lys Tyr Lys Leu
    210                 215                 220

Val Pro Lys Glu Ile Leu Asn Ser Thr Lys Asp Ile Lys Asn Ser Phe
225                 230                 235                 240

Leu Glu Gly Tyr Tyr Ala Ala Asp Gly Ser Arg Lys Glu Thr Glu Asn
                245                 250                 255

Met Gly Xaa Arg Arg Xaa Asp Ile Lys Gly Lys Ile Ser Ala Gln Xaa
                260                 265                 270

Leu Phe Tyr Leu Leu Lys Ser Leu Gly Tyr Asn Val Ser Ile Asn Ile
                275                 280                 285

Arg Ser Asp Lys Asn Gln Ile Tyr Arg Leu Thr Phe Ser Asn Lys Lys
    290                 295                 300

Gln Arg Lys Asn Pro Ile Ala Ile Lys Lys Ile Gln Leu Met Asn Glu
305                 310                 315                 320

Thr Ser Asn Asp His Asp Gly Asp Tyr Val Tyr Asp Leu Glu Thr Glu
                325                 330                 335

Ser Gly Ser Phe His Ala Gly Val Gly Glu Met Ile Val Lys Asn
                340                 345                 350
```

<210> 157
<211> 282
<212> PRT
<213> Artificial sequence

<220>
<223> modified contiguous intein from Marseilleviridae Montpellier MTP3

<220>
<221> misc_feature
<222> (86)..(86)
<223> Xaa can be any naturally occurring amino acid

```
<220>
<221>  misc_feature
<222>  (108)..(108)
<223>  Xaa can be any naturally occurring amino acid

<220>
<221>  misc_feature
<222>  (145)..(145)
<223>  Xaa can be any naturally occurring amino acid

<220>
<221>  misc_feature
<222>  (161)..(161)
<223>  Xaa can be any naturally occurring amino acid

<220>
<221>  misc_feature
<222>  (181)..(181)
<223>  Xaa can be any naturally occurring amino acid

<220>
<221>  misc_feature
<222>  (217)..(217)
<223>  Xaa can be any naturally occurring amino acid

<220>
<221>  misc_feature
<222>  (234)..(234)
<223>  Xaa can be any naturally occurring amino acid

<220>
<221>  misc_feature
<222>  (244)..(244)
<223>  Xaa can be any naturally occurring amino acid

<400>  157
```

Ser Val Thr Gly Asp Thr Pro Ile Met Ile Lys Asp Lys Asn Asn Asn
1               5               10              15

Ile Asn Ile Val Thr Ile Lys Glu Leu Gly Glu Lys Trp Lys Pro Tyr
            20              25              30

Asp Ile Phe Lys Ser His Glu Ile Asn Ser Asn Arg Lys Tyr Lys Gln
        35              40              45

Gln Ala Asp Phe Asn Gly Glu Val Trp Thr Ser Asn Gly Trp Ala Lys
    50              55              60

Ile Lys Arg Val Ile Arg His Lys Thr Val Lys Lys Leu Tyr Arg Val
65              70              75              80

Leu Thr Asn Thr Gly Xaa Ile Asp Val Thr Glu Asp His Ser Leu Leu
            85              90              95

Asp Thr Asn Lys Asn Ile Ile Lys Pro Ile Asp Xaa Lys Ile Gly Thr

169

```
                    100                      105                        110


        Glu Leu Leu His Gly Phe Pro Glu Ile Asn Asn Asn His Asn Lys Leu
                115                     120                 125


        Ser Leu Glu Ile Tyr Lys Glu Leu Asp Ile Thr Ser Glu Leu Phe Asp
            130                     135                 140


        Xaa Met Ile Glu Ser Asn Lys Lys Trp Asn Asn Glu Lys Met Lys Ala
        145                     150                 155                 160


        Xaa Phe Ile Gly Ser Glu Tyr Arg Lys Gln Asn Lys Asn Ile Ser Asn
                        165                 170                 175


        Glu Ile Leu Asn Xaa Ser Lys Lys Ile Lys Lys Tyr Phe Leu Leu Gly
                180                     185                 190


        Tyr Leu Gly Asn Asp Lys Glu Tyr Ile Thr Asn Asn Lys Ile Asn Ala
            195                     200                 205


        Gln Met Val Tyr Tyr Leu Met Lys Xaa Leu Gly Tyr Asn Ile Val Ile
            210                     215                 220


        Asp Leu Ile Glu Ser Ser Tyr Lys Leu Xaa Ile Val Asp Asn Ile Asn
        225                     230                 235                 240


        Lys Pro Tyr Xaa Ile Asn Lys Ile Ile Gln Leu Gln Asp Thr Ser Ile
                        245                 250                 255


        Asn Gly Glu Tyr Val Tyr Asp Leu Glu Thr Glu Ser Gly Thr Phe His
                260                     265                 270


        Ala Gly Ile Gly Glu Leu Ile Val Lys Asn
                275                     280



        <210>   158
        <211>   282
        <212>   PRT
        <213>   Artificial sequence

        <220>
        <223>   modified contiguous intein from Megavirus chiliensis


        <220>
        <221>   misc_feature
        <222>   (86)..(86)
        <223>   Xaa can be any naturally occurring amino acid

        <220>
        <221>   misc_feature
```

```
<222>  (108)..(108)
<223>  Xaa can be any naturally occurring amino acid

<220>
<221>  misc_feature
<222>  (145)..(145)
<223>  Xaa can be any naturally occurring amino acid

<220>
<221>  misc_feature
<222>  (181)..(181)
<223>  Xaa can be any naturally occurring amino acid

<220>
<221>  misc_feature
<222>  (213)..(213)
<223>  Xaa can be any naturally occurring amino acid

<220>
<221>  misc_feature
<222>  (217)..(217)
<223>  Xaa can be any naturally occurring amino acid

<220>
<221>  misc_feature
<222>  (234)..(234)
<223>  Xaa can be any naturally occurring amino acid

<220>
<221>  misc_feature
<222>  (244)..(244)
<223>  Xaa can be any naturally occurring amino acid

<400>  158
```

Ser Val Thr Gly Asp Thr Pro Ile Met Ile Lys Asp Lys Asn Asn Asn
1               5               10                  15

Ile Asn Ile Val Thr Ile Lys Glu Leu Gly Glu Lys Trp Lys Pro Tyr
            20                  25                  30

Asp Ile Phe Lys Ser His Glu Ile Asn Ser Asn Arg Lys Tyr Lys Gln
            35                  40                  45

Gln Ala Asp Phe Asn Gly Glu Val Trp Thr Ser Asn Gly Trp Ala Lys
        50                  55                  60

Ile Lys Arg Val Ile Arg His Lys Thr Val Lys Lys Leu Tyr Arg Val
65                  70                  75                  80

Leu Thr Asn Thr Gly Xaa Ile Asp Val Thr Glu Asp His Ser Leu Leu
                85                  90                  95

Asp Thr Asn Lys Asn Ile Ile Lys Pro Ile Asp Xaa Lys Ile Gly Thr
                100                 105                 110

```
Glu Leu Leu His Gly Phe Pro Glu Ile Asn Asn Asn His Asn Lys Leu
    115             120             125

Ser Leu Glu Ile Tyr Lys Glu Leu Asp Ile Thr Ser Glu Leu Phe Asp
    130             135             140

Xaa Met Ile Glu Ser Asn Lys Lys Trp Asn Asn Glu Lys Met Lys Ala
145             150             155             160

Tyr Phe Ile Gly Ser Glu Tyr Arg Lys Gln Asn Lys Asn Ile Ser Asn
                165             170             175

Glu Ile Leu Asn Xaa Ser Lys Lys Ile Lys Lys Tyr Phe Leu Leu Gly
            180             185             190

Tyr Leu Gly Asn Asp Lys Glu Tyr Ile Thr Asn Asn Lys Ile Asn Ala
        195             200             205

Gln Ile Ile Tyr Xaa Leu Met Lys Xaa Leu Glu Tyr Asn Ile Val Ile
    210             215             220

Asp Leu Ile Glu Ser Ser Tyr Lys Leu Xaa Ile Ile Asp Asn Ile Asn
225             230             235             240

Glu Pro Tyr Xaa Ile Asn Lys Ile Ile Gln Leu Gln Asp Thr Ser Ile
            245             250             255

Asn Gly Glu Tyr Val Tyr Asp Leu Glu Thr Glu Ser Gly Thr Phe His
            260             265             270

Ala Gly Ile Gly Glu Leu Ile Val Lys Asn
        275             280
```

```
<210>  159
<211>  282
<212>  PRT
<213>  Artificial sequence

<220>
<223>  modified contiguous intein from Megavirus courdo11


<220>
<221>  misc_feature
<222>  (86)..(86)
<223>  Xaa can be any naturally occurring amino acid

<220>
<221>  misc_feature
<222>  (108)..(108)
<223>  Xaa can be any naturally occurring amino acid
```

```
<220>
<221>  misc_feature
<222>  (145)..(145)
<223>  Xaa can be any naturally occurring amino acid

<220>
<221>  misc_feature
<222>  (161)..(161)
<223>  Xaa can be any naturally occurring amino acid

<220>
<221>  misc_feature
<222>  (181)..(181)
<223>  Xaa can be any naturally occurring amino acid

<220>
<221>  misc_feature
<222>  (217)..(217)
<223>  Xaa can be any naturally occurring amino acid

<220>
<221>  misc_feature
<222>  (234)..(234)
<223>  Xaa can be any naturally occurring amino acid

<220>
<221>  misc_feature
<222>  (244)..(244)
<223>  Xaa can be any naturally occurring amino acid

<400>  159
```

Ser Val Thr Gly Asp Thr Pro Ile Met Ile Lys Asp Lys Asn Asn Asn
1               5               10              15

Ile Asn Ile Val Thr Ile Lys Glu Leu Gly Glu Lys Trp Lys Pro Tyr
            20              25              30

Asp Ile Phe Lys Ser His Glu Ile Asn Ser Asn Arg Lys Tyr Lys Gln
            35              40              45

Gln Ala Asp Phe Asn Gly Glu Val Trp Thr Ser Asn Gly Trp Ala Lys
            50              55              60

Ile Lys Arg Val Ile Arg His Lys Thr Val Lys Lys Leu Tyr Arg Val
65              70              75              80

Leu Thr Asn Thr Gly Xaa Ile Asp Val Thr Glu Asp His Ser Leu Leu
                85              90              95

Asp Thr Asn Lys Asn Ile Ile Lys Pro Ile Asp Xaa Lys Ile Gly Thr
            100             105             110

Glu Leu Leu His Gly Phe Pro Glu Ile Asn Asn Asn His Asn Lys Leu
            115             120             125

```
Ser Leu Glu Ile Tyr Lys Glu Leu Asp Ile Thr Ser Glu Leu Phe Asp
    130             135             140

Xaa Met Ile Glu Ser Asn Lys Lys Trp Asn Asn Glu Lys Met Lys Ala
    145             150             155             160

Xaa Phe Ile Gly Ser Glu Tyr Arg Lys Gln Asn Lys Asn Ile Ser Asn
            165             170             175

Glu Ile Leu Asn Xaa Ser Lys Lys Ile Lys Lys Tyr Phe Leu Leu Gly
            180             185             190

Tyr Leu Gly Asn Asp Lys Glu Tyr Ile Thr Asn Asn Lys Ile Asn Ala
        195             200             205

Gln Met Val Tyr Tyr Leu Met Lys Xaa Leu Gly Tyr Asn Ile Val Ile
    210             215             220

Asp Leu Ile Glu Ser Ser Tyr Lys Leu Xaa Ile Val Asp Asn Ile Asn
225             230             235             240

Lys Pro Tyr Xaa Ile Asn Lys Ile Ile Gln Leu Gln Asp Thr Ser Ile
            245             250             255

Asn Gly Glu Tyr Val Tyr Asp Leu Glu Thr Glu Ser Gly Thr Phe His
            260             265             270

Ala Gly Ile Gly Glu Leu Ile Val Lys Asn
            275             280
```

```
<210>  160
<211>  282
<212>  PRT
<213>  Artificial sequence

<220>
<223>  modified contiguous intein from Megavirus courdo7

<220>
<221>  misc_feature
<222>  (86)..(86)
<223>  Xaa can be any naturally occurring amino acid

<220>
<221>  misc_feature
<222>  (108)..(108)
<223>  Xaa can be any naturally occurring amino acid

<220>
<221>  misc_feature
<222>  (145)..(145)
```

```
<223>  Xaa can be any naturally occurring amino acid

<220>
<221>  misc_feature
<222>  (161)..(161)
<223>  Xaa can be any naturally occurring amino acid

<220>
<221>  misc_feature
<222>  (181)..(181)
<223>  Xaa can be any naturally occurring amino acid

<220>
<221>  misc_feature
<222>  (217)..(217)
<223>  Xaa can be any naturally occurring amino acid

<220>
<221>  misc_feature
<222>  (234)..(234)
<223>  Xaa can be any naturally occurring amino acid

<220>
<221>  misc_feature
<222>  (244)..(244)
<223>  Xaa can be any naturally occurring amino acid

<400>  160

Ser Val Thr Gly Asp Thr Pro Ile Met Ile Lys Asp Lys Asn Asn Asn
1               5                   10                  15

Ile Asn Ile Val Thr Ile Lys Glu Leu Gly Glu Lys Trp Lys Pro Tyr
            20                  25                  30

Asp Ile Phe Lys Ser His Glu Ile Asn Ser Asn Arg Lys Tyr Lys Gln
            35                  40                  45

Gln Ala Asp Phe Asn Gly Glu Val Trp Thr Ser Asn Gly Trp Ala Lys
        50                  55                  60

Ile Lys Arg Val Ile Arg His Lys Thr Val Lys Lys Leu Tyr Arg Val
65                  70                  75                      80

Leu Thr Asn Thr Gly Xaa Ile Asp Val Thr Glu Asp His Ser Leu Leu
                85                  90                  95

Asp Thr Asn Lys Asn Ile Ile Lys Pro Ile Asp Xaa Lys Ile Gly Thr
            100                 105                 110

Glu Leu Leu His Gly Phe Pro Glu Ile Asn Asn Asn His Asn Lys Leu
            115                 120                 125

Ser Leu Glu Ile Tyr Lys Glu Leu Asp Ile Thr Ser Glu Leu Phe Asp
            130                 135                 140
```

```
Xaa Met Ile Glu Ser Asn Lys Lys Trp Asn Asn Glu Lys Met Lys Ala
145             150             155             160

Xaa Phe Ile Gly Ser Glu Tyr Arg Lys Gln Asn Lys Asn Ile Ser Asn
            165             170             175

Glu Ile Leu Asn Xaa Ser Lys Lys Ile Lys Lys Tyr Phe Leu Leu Gly
        180             185             190

Tyr Leu Gly Asn Asp Lys Glu Tyr Ile Thr Asn Asn Lys Ile Asn Ala
        195             200             205

Gln Met Val Tyr Tyr Leu Met Lys Xaa Leu Gly Tyr Asn Ile Val Ile
    210             215             220

Asp Leu Ile Glu Ser Ser Tyr Lys Leu Xaa Ile Val Asp Asn Ile Asn
225             230             235             240

Lys Pro Tyr Xaa Ile Asn Lys Ile Ile Gln Leu Gln Asp Thr Ser Ile
            245             250             255

Asn Gly Glu Tyr Val Tyr Asp Leu Glu Thr Glu Ser Gly Thr Phe His
            260             265             270

Ala Gly Ile Gly Glu Leu Ile Val Lys Asn
        275             280
```

```
<210>  161
<211>  282
<212>  PRT
<213>  Artificial sequence

<220>
<223>  modified contiguous intein from Megavirus terra1 TE1

<220>
<221>  misc_feature
<222>  (86)..(86)
<223>  Xaa can be any naturally occurring amino acid

<220>
<221>  misc_feature
<222>  (108)..(108)
<223>  Xaa can be any naturally occurring amino acid

<220>
<221>  misc_feature
<222>  (145)..(145)
<223>  Xaa can be any naturally occurring amino acid

<220>
```

```
<221>  misc_feature
<222>  (161)..(161)
<223>  Xaa can be any naturally occurring amino acid

<220>
<221>  misc_feature
<222>  (181)..(181)
<223>  Xaa can be any naturally occurring amino acid

<220>
<221>  misc_feature
<222>  (217)..(217)
<223>  Xaa can be any naturally occurring amino acid

<220>
<221>  misc_feature
<222>  (234)..(234)
<223>  Xaa can be any naturally occurring amino acid

<220>
<221>  misc_feature
<222>  (244)..(244)
<223>  Xaa can be any naturally occurring amino acid

<400>  161
```

Ser Val Thr Gly Asp Thr Pro Ile Met Ile Lys Asp Lys Asn Asn Asn
1               5                   10                  15

Ile Asn Ile Val Thr Ile Lys Glu Leu Gly Glu Lys Trp Lys Pro Tyr
            20                  25                  30

Asp Ile Phe Lys Ser His Glu Ile Asn Ser Asn Arg Lys Tyr Lys Gln
            35                  40                  45

Gln Ala Asp Phe Asn Gly Glu Val Trp Thr Ser Asn Gly Trp Ala Lys
        50                  55                  60

Ile Lys Arg Val Ile Arg His Lys Thr Val Lys Lys Leu Tyr Arg Val
65                  70                  75                  80

Leu Thr Asn Thr Gly Xaa Ile Asp Val Thr Glu Asp His Ser Leu Leu
                85                  90                  95

Asp Thr Asn Lys Asn Ile Ile Lys Pro Ile Asp Xaa Lys Ile Gly Thr
            100                 105                 110

Glu Leu Leu His Gly Phe Pro Glu Ile Asn Asn Asn His Asn Lys Leu
            115                 120                 125

Ser Leu Glu Ile Tyr Lys Glu Leu Asp Ile Thr Ser Glu Leu Phe Asp
        130                 135                 140

Xaa Met Ile Glu Ser Asn Lys Lys Trp Asn Asn Glu Lys Met Lys Ala

```
              145                150                155                160


         Xaa Phe Ile Gly Ser Glu Tyr Arg Lys Gln Asn Lys Asn Ile Ser Asn
                         165                170                175


         Glu Ile Leu Asn Xaa Ser Lys Lys Ile Lys Lys Tyr Phe Leu Leu Gly
                     180                185                190


         Tyr Leu Gly Asn Asp Lys Glu Tyr Ile Thr Asn Asn Lys Ile Asn Ala
                     195                200                205


         Gln Met Val Tyr Tyr Leu Met Lys Xaa Leu Gly Tyr Asn Ile Val Ile
                 210                215                220


         Asp Leu Ile Glu Ser Ser Tyr Lys Leu Xaa Ile Val Asp Asn Ile Asn
         225                230                235                240


         Lys Pro Tyr Xaa Ile Asn Lys Ile Ile Gln Leu Gln Asp Thr Ser Ile
                     245                250                255


         Asn Gly Glu Tyr Val Tyr Asp Leu Glu Thr Glu Ser Gly Thr Phe His
                     260                265                270


         Ala Gly Ile Gly Glu Leu Ile Val Lys Asn
                     275                280



         <210>  162
         <211>  265
         <212>  PRT
         <213>  Artificial sequence

         <220>
         <223>  modified contiguous intein from Moumouvirus Monve


         <220>
         <221>  misc_feature
         <222>  (107)..(107)
         <223>  Xaa can be any naturally occurring amino acid

         <400>  162

         Ser Val Thr Gly Asp Thr Pro Ile Ile Val Lys Leu Pro Asn Ser Asn
         1                 5                 10                15


         Asp Val Glu Ile Lys Thr Ile Gln Glu Leu Thr Thr Phe Trp Tyr Glu
                     20                25                30


         Tyr Asp Ala Phe Lys Ala Gly Asp Ser Asn Arg Lys Asp Lys Gln Gln
                     35                40                45
```

Ala Ile Leu Asp Tyr Glu Val Trp Thr Asp Lys Gly Trp Ala Lys Ile
        50                  55                  60

Lys Arg Val Ile Arg His Gln Thr Lys Lys Ser Ile Tyr Arg Val Lys
65                  70                  75                  80

Thr Asn Asn Gly Val Val Asp Val Thr Glu Asp His Ser Leu Leu Asn
                85                  90                  95

Thr Asp Lys Glu Ile Ile Lys Pro Leu Asp Xaa Asn Pro Asn Thr Lys
            100                 105                 110

Leu Leu His Gly Phe Met Glu Thr Asn Asn Ile Tyr Gln Asn Ile Thr
            115                 120                 125

Pro Ser Gln Ala Tyr Leu Leu Gly Leu Asn Phe Gly Lys Val Asp Val
        130                 135                 140

Tyr Trp Asp Ile Ile Asn Ala Gln Ser Ile Trp Asp Val Ile Asn Ile
145                 150                 155                 160

Ile Thr Asn Ala Thr Thr Lys Ile Lys Gln Glu Phe Ile Lys Gly Trp
                165                 170                 175

Lys Lys Gln Gly Phe Tyr Asn Ile Glu Asn Lys Val Glu Ala Gln Phe
            180                 185                 190

Leu Tyr Tyr Val Leu Lys Ser Leu Gly His Asn Val Asn Ile His Met
        195                 200                 205

Pro Ile Thr Lys Glu Asp Ile Tyr Arg Leu Ser Tyr Gly Lys Asn Ile
        210                 215                 220

Ser Asn Ile Asn Lys Thr Thr Ile Gln Tyr Leu Arg Glu Thr Tyr Asp
225                 230                 235                 240

Gly Glu Tyr Val Tyr Asp Leu Glu Thr Glu Ser Gly Thr Phe His Ala
                245                 250                 255

Gly Ile Gly Glu Met Ile Val Lys Asn
            260                 265

<210> 163
<211> 323
<212> PRT
<213> Artificial sequence

<220>
<223> modified contiguous intein from Ostreococcus tauri virus 1

179

```
<220>
<221>  misc_feature
<222>  (48)..(48)
<223>  Xaa can be any naturally occurring amino acid

<220>
<221>  misc_feature
<222>  (97)..(97)
<223>  Xaa can be any naturally occurring amino acid

<220>
<221>  misc_feature
<222>  (135)..(135)
<223>  Xaa can be any naturally occurring amino acid

<220>
<221>  misc_feature
<222>  (161)..(161)
<223>  Xaa can be any naturally occurring amino acid

<220>
<221>  misc_feature
<222>  (208)..(208)
<223>  Xaa can be any naturally occurring amino acid

<400>  163
```

Ser Val Thr Pro Asp Thr Pro Leu Leu Ile Arg Glu Asn Gly Glu Val
1               5               10                  15

Lys Thr Thr Arg Ile Asp Ser Leu Val Asp Leu Tyr Glu Val Arg Asp
            20              25                  30

Asp Gly Lys Glu Ile Ala Glu Ile Asp Ala Glu Val Trp Thr Glu Xaa
            35              40                  45

Gly Phe Thr Pro Ile Lys Gln Ile Val Arg His Lys Thr Thr Lys Asn
        50              55                  60

Ile His Arg Val Leu Thr His Thr Gly Ile Val Asp Val Thr Glu Asp
65              70                  75                  80

His Ser Leu Leu Leu Lys Asn Lys Glu Met Ile Lys Pro Ser Glu Val
                85                  90                  95

Xaa Leu Gly Thr Glu Leu Leu His Gly Asn Ser Leu Glu Ala Phe Gly
            100                 105                 110

Glu Thr His Thr Asp Val Thr Pro Glu Glu Ala Lys Val Met Gly Phe
            115                 120                 125

Phe Phe Gly Asp Gly Ser Xaa Gly His Tyr Asp Gly Lys Tyr Thr Trp
        130                 135                 140

Ala Leu Asn Asn Ala Asp Met Thr Phe Leu Glu Glu Met Ser Glu Leu
145              150              155              160

Xaa Pro Phe Glu Thr Arg Val Tyr Asp Thr Ile Gln Ser Ser Gly Val
                 165              170              175

Tyr Lys Leu Asn Ala Val Gly Asp Val Lys Ser Ile Ser Val Arg Tyr
         180              185              190

Arg Ser Leu Phe Tyr Asn Glu His Lys Glu Lys Val Val Pro Pro Xaa
         195              200              205

Ile Leu Gly Ala Pro Leu His Ile Val Gln Ser Phe Trp Asp Gly Tyr
     210              215              220

Tyr Met Ala Asp Gly Asp Lys Asp Val His Gly Tyr Thr Arg Met Asp
225              230              235              240

Ile Lys Gly Lys Glu Gly Ser Met Gly Met Tyr Ile Leu Gly Arg Arg
              245              250              255

Leu Gly Tyr Asn Val Ser Met Asn Thr Arg Thr Asp Lys Pro Asp Ile
         260              265              270

Phe Arg Gln Thr Trp Thr Thr Ser Ser Gln Arg Lys Asn Pro Ile Ala
         275              280              285

Ile Lys Lys Leu Glu Leu Leu Gly Glu Thr Glu Gly Tyr Val Tyr Asp
     290              295              300

Leu Thr Thr Gly Ser His His Phe His Val Gly Pro Gly Asp Leu Val
305              310              315              320

Val His Asn

<210> 164
<211> 317
<212> PRT
<213> Artificial sequence

<220>
<223> modified contiguous intein from unknown phycodnavirus KBvp-11

<220>
<221> misc_feature
<222> (19)..(19)
<223> Xaa can be any naturally occurring amino acid

<220>
<221> misc_feature
<222> (107)..(107)
<223> Xaa can be any naturally occurring amino acid

<220>
<221> misc_feature
<222> (135)..(135)
<223> Xaa can be any naturally occurring amino acid

<220>
<221> misc_feature
<222> (161)..(161)
<223> Xaa can be any naturally occurring amino acid

<220>
<221> misc_feature
<222> (208)..(208)
<223> Xaa can be any naturally occurring amino acid

<220>
<221> misc_feature
<222> (281)..(281)
<223> Xaa can be any naturally occurring amino acid

<400> 164

Ser Val Thr Pro Asp Thr Pro Leu Leu Ile Arg Gln Asp Gly Ile Val
1               5                   10                  15

Lys Thr Xaa Arg Ile Asp Ser Leu Val Asn Ala Tyr Glu Val Arg Asp
            20                  25                  30

Asp Gly Lys Glu Val Ala Thr Ile Asp Ala Glu Val Trp Thr Glu Lys
            35                  40                  45

Gly Phe Thr Pro Ile His Gln Ile Val Arg His Lys Thr Thr Lys Arg
        50                  55                  60

Ile His Arg Val Leu Thr His Thr Gly Val Val Asp Val Thr Glu Asp
65                  70                  75                  80

His Ser Leu Leu Leu Glu Asp Ala Lys Met Ile Thr Pro Lys Glu Val
                85                  90                  95

Gln Leu Gly Thr Lys Leu Leu His Gly Ser Xaa Val Asn Ala Ile Ile
            100                 105                 110

Asp Gly Thr Ser Arg Val Ser Val Asn Glu Ala Lys Val Met Gly Phe
            115                 120                 125

Phe Phe Gly Asp Gly Ser Xaa Gly Ala Tyr Asn Gly Lys Tyr Thr Trp
        130                 135                 140

```
Thr Leu Asn Asn Ala Asn Ile Gln Tyr Leu Asp Lys Met Ala Ser Leu
145                 150                 155                 160

Xaa Pro Phe Glu Thr Arg Ile Tyr Ala Thr Met Glu Ser Ser Gly Val
                165                 170                 175

Tyr Lys Leu Asn Ala Ile Gly Asp Val Lys Thr Ile Ser Leu Arg Tyr
                180                 185                 190

Arg Ser Leu Phe Tyr Asn Ala Ala Lys Glu Lys Val Ile Pro Pro Xaa
                195                 200                 205

Ile Leu Asn Ala Pro Glu Glu Val Val Lys Ala Phe Val Glu Gly Tyr
            210                 215                 220

Tyr Met Ala Asp Gly Asp Thr Arg Met Asp Ile Lys Gly Lys Glu Gly
225                 230                 235                 240

Ser Met Gly Met Phe Ile Leu Gly Lys Arg Leu Gly Tyr Asn Val Ser
                245                 250                 255

Ile Asn Thr Arg Ser Asp Lys Pro Asp Ile Tyr Arg Gln Thr Trp Thr
                260                 265                 270

Thr Tyr Ser Gln Arg Lys Glu Pro Xaa Ala Ile Lys Lys Leu Glu Phe
            275                 280                 285

Leu Glu Glu Thr Asp Gly Tyr Val Tyr Asp Leu Thr Thr Glu Ser His
    290                 295                 300

His Phe His Val Gly Pro Gly Glu Leu Val Val His Asn
305                 310                 315
```

<210> 165
<211> 359
<212> PRT
<213> Artificial sequence

<220>
<223> modified contiguous intein from Antarctica Vida lake
      environmental sampling brine-hole-2

<220>
<221> misc_feature
<222> (81)..(81)
<223> Xaa can be any naturally occurring amino acid

<220>
<221> misc_feature
<222> (191)..(191)

<223>   Xaa can be any naturally occurring amino acid

<220>
<221>   misc_feature
<222>   (207)..(207)
<223>   Xaa can be any naturally occurring amino acid

<220>
<221>   misc_feature
<222>   (333)..(333)
<223>   Xaa can be any naturally occurring amino acid

<400>   165

```
Ser Val Thr His Asp Thr Pro Ile Tyr Ile Lys Trp Lys Asp Ser Asn
1               5               10              15

Lys Leu Asp Ile Leu Pro Ile Ser Asp Ile Phe Asn Glu Asp Ser Glu
        20              25              30

Val Leu Asp Glu Glu Ser Leu Arg Asp Leu Glu Ile Lys Pro Tyr Glu
        35              40              45

Val Leu Thr Val Asn Gly Trp Lys Glu Ile Asn Tyr Val Tyr Arg His
        50              55              60

Glu Thr Asn Lys Lys Ile His Arg Ile Ser Thr Lys Asp Lys Leu Val
65              70              75              80

Xaa Val Thr Glu Asp His Ser Leu Phe Gln Asn Gly Lys Gln Ile Lys
        85              90              95

Pro Lys Asn Leu Lys Arg Gly Asp Val Leu Asp Val Arg Glu Leu Pro
        100             105             110

Thr Phe Glu Leu Asp Asp Asp Asn Asn Leu Asn Glu Asp Leu Phe Tyr
        115             120             125

Leu Tyr Gly Tyr Phe Leu Gly Asp Gly Ser Ala Thr Tyr Gly Asn Arg
        130             135             140

Lys Gln Tyr Tyr Lys Ser Lys Lys Thr Ser Lys Thr Asn Ile Asn Lys
145             150             155             160

Gly Lys Arg Ser Val Phe Lys Ile Ser Ser Ser Asn Tyr Asp Lys Leu
        165             170             175

Ile Arg Leu Gln Lys Ile Ile Lys Asp Asn Phe Asp Val Asn Xaa Lys
        180             185             190

Ile Lys Asp His Arg Glu Ser Ser Asn Val Tyr Asn Leu Ile Xaa Tyr
```

                195                      200                      205


Val Lys Lys Met Ser Val Lys Phe Ser Glu Asp Phe Tyr Thr Ser Tyr
    210                 215                 220


Lys Glu Lys Lys Ile Pro Asn Tyr Val Leu Asn Thr Asn Lys Lys Asn
225                 230                 235                 240


Lys Leu Ala Phe Leu Glu Gly Val Phe Ser Ser Asp Gly Tyr Gly Asp
                245                 250                 255


Thr Leu Glu Glu Val Ser Asp Ile Gly Met Lys Ser Gln Val Ala Met
            260                 265                 270


Ser Gly Ile Ser Tyr Ile Met Glu Thr Leu Ser Ile Asp Arg Glu Ile
        275                 280                 285


Lys Val Arg Lys Asp Lys Glu Asn Phe Ile Ser Leu Lys Leu Lys Asn
    290                 295                 300


Arg Asn Arg Ser Asn Ser Lys Phe Ala Asn Lys Ile Lys Met Lys Ser
305                 310                 315                 320


Asp Glu Val Trp Leu Asn Glu Val Ile Thr Asn Lys Xaa Glu Lys Asn
                325                 330                 335


Tyr Val Tyr Asp Ile Ser Thr Glu Asp Gly Thr Phe Ile Gly Gly Ile
            340                 345                 350


Gly Gly Val Asp Leu Lys Asn
        355


<210> 166
<211> 296
<212> PRT
<213> Artificial sequence

<220>
<223> modified contiguous intein from botany bay environmental sample BBAY15 library


<220>
<221> misc_feature
<222> (32)..(32)
<223> Xaa can be any naturally occurring amino acid

<220>
<221> misc_feature
<222> (43)..(43)
<223> Xaa can be any naturally occurring amino acid

<220>
<221> misc_feature
<222> (144)..(144)
<223> Xaa can be any naturally occurring amino acid

<220>
<221> misc_feature
<222> (149)..(149)
<223> Xaa can be any naturally occurring amino acid

<220>
<221> misc_feature
<222> (185)..(185)
<223> Xaa can be any naturally occurring amino acid

<220>
<221> misc_feature
<222> (209)..(209)
<223> Xaa can be any naturally occurring amino acid

<220>
<221> misc_feature
<222> (212)..(212)
<223> Xaa can be any naturally occurring amino acid

<220>
<221> misc_feature
<222> (223)..(223)
<223> Xaa can be any naturally occurring amino acid

<400> 166

Ser Val Thr Ala Glu Thr Pro Val Leu Val Lys Ser Pro Asp Gly Val
1               5                   10                  15

Val Gln Tyr Ile Gln Ile Ser Glu Leu Gly Asn Lys Trp Asp Lys Xaa
            20                  25                  30

Val Glu Asp Gly Lys Glu Glu Lys Glu Phe Xaa Ser Leu Glu Gly Lys
        35                  40                  45

Gly Trp Glu Ala Trp Ser Asp Asp Gly Trp Thr Pro Ile Lys Asn Val
    50                  55                  60

Ile Arg His Glu Leu Ala Pro His Lys Lys Val Phe Arg Val Leu Thr
65                  70                  75                  80

His Thr Gly Tyr Val Glu Val Thr Asp Asp His Ser Leu Leu Asn Asn
            85                  90                  95

Glu Lys Gly Ile Ile Lys Thr Asn Glu Ile Lys Asn Gly Gln Leu Leu
            100                 105                 110

Leu Gln Asn Glu Tyr Ile Glu Leu Asp Ser Lys Ile Asn Thr Ile Ser
        115                 120                 125

```
Glu Asp Glu Ala Arg Ile Met Gly Phe Phe Phe Gly Asp Gly Ser Xaa
    130                 135                 140

Gly Thr Tyr Asn Xaa Lys Ser Gly Lys Lys Ser Ser Trp Ala Leu Asn
145                 150                 155                 160

Asn Asn Asn Leu Ile Arg Leu Lys Lys Tyr Gln Asn Leu Phe Tyr Lys
            165                 170                 175

Asn Lys Asn Lys Val Ile Pro Asp Xaa Ile Phe Lys Ser Asn Lys Asn
            180                 185                 190

Val Arg Lys Ala Phe Phe Glu Gly Leu Tyr Asp Ala Asp Gly Asp Lys
        195                 200                 205

Xaa Gly Tyr Xaa Thr Arg Ile Asp Gln Lys Ser Met Ile Ser Xaa Ala
    210                 215                 220

Tyr Ile Tyr Lys Leu Gly Lys Ser Leu Asp Tyr Asn Val Ser Ile Asn
225                 230                 235                 240

Val His Ser Lys Lys Lys Asn Ile Phe Arg Leu Thr Phe Thr Thr Lys
            245                 250                 255

Lys Gln Arg Lys Ile Val Asn Ala Val Lys Lys Leu Met Lys Ser Asn
            260                 265                 270

Asn Gly Tyr Val Tyr Asp Leu Thr Thr Glu Ser Ser Leu Ser Ser Arg
            275                 280                 285

Ile Gly Ser Ile Ile Val His Asn
    290                 295
```

```
<210>   167
<211>   371
<212>   PRT
<213>   Artificial sequence

<220>
<223>   modified contiguous intein from Chrysochromulina ericina virus
        isolate 01


<220>
<221>   misc_feature
<222>   (20)..(20)
<223>   Xaa can be any naturally occurring amino acid

<220>
<221>   misc_feature
<222>   (42)..(42)
```

<223>   Xaa can be any naturally occurring amino acid

<220>
<221>   misc_feature
<222>   (58)..(58)
<223>   Xaa can be any naturally occurring amino acid

<220>
<221>   misc_feature
<222>   (112)..(112)
<223>   Xaa can be any naturally occurring amino acid

<220>
<221>   misc_feature
<222>   (142)..(142)
<223>   Xaa can be any naturally occurring amino acid

<220>
<221>   misc_feature
<222>   (147)..(147)
<223>   Xaa can be any naturally occurring amino acid

<220>
<221>   misc_feature
<222>   (172)..(172)
<223>   Xaa can be any naturally occurring amino acid

<220>
<221>   misc_feature
<222>   (196)..(196)
<223>   Xaa can be any naturally occurring amino acid

<220>
<221>   misc_feature
<222>   (271)..(271)
<223>   Xaa can be any naturally occurring amino acid

<400>   167

Ser Val Ala Ser Tyr Thr Pro Ile Tyr Val Arg Tyr Asn Lys Ser Ile
1               5                   10                  15

Ile Asp Ile Xaa Ser Val Glu Glu Leu Ala Glu Lys Tyr Gly Asn Gly
            20                  25                  30

Trp His Leu Glu Ser Pro Lys Glu Tyr Xaa Glu Leu Asn Asn Ile Glu
        35                  40                  45

Ser Trp Thr Glu Asn Gly Trp Thr Glu Xaa His Arg Val Ile Arg His
    50                  55                  60

Arg Leu Ala Pro Tyr Lys Lys Met Val Arg Ile Leu Thr His Thr Gly
65                  70                  75                  80

Leu Val Asp Val Thr Asp Asp His Ser Leu Val Lys Asn Thr Gly Glu
                85                  90                  95

Glu Ile Ser Pro Lys Asp Val Ser Ile Gly Thr Lys Leu Leu His Xaa
            100                 105                 110

Thr Met Ser Glu Asn Glu Ser Asn Ile Glu Ser Asp Ile Ser Ile Asp
            115                 120                 125

Glu Ala Arg Ile Met Gly Phe Phe Phe Gly Asp Gly Ser Xaa Gly Ile
            130                 135                 140

Tyr Asp Xaa Pro Ser Gly His Lys Ala Ser Trp Ala Leu Asn Asn Ser
145                 150                 155                 160

Asn Lys Glu Leu Ile Glu Lys Tyr Tyr Asn Leu Xaa Lys Ser Val Tyr
            165                 170                 175

Pro Glu Phe Glu Trp Lys Val Tyr Asp Thr Leu Asn Ser Ser Gly Val
            180                 185                 190

Tyr Lys Ile Xaa Phe Asn Lys Lys Ser Gly Ser Lys Ser Lys Ile Gln
            195                 200                 205

Phe Ile Glu Lys Tyr Arg Ser Met Leu Tyr Asn Lys Lys Ser Lys Ile
            210                 215                 220

Ile Pro Ser Glu Ile Ile Asn Gly Ser Ile Glu Leu Arg Lys Ser Phe
225                 230                 235                 240

Trp Glu Gly Leu Tyr Asp Ala Asp Gly Asp Lys Asp Lys Asn Gly Tyr
            245                 250                 255

Thr Arg Ile Asp Gln Lys Ser Gln Ile Ser Ala Ala Tyr Ile Xaa Trp
            260                 265                 270

Leu Ala Asn Ser Ile Gly Tyr Lys Thr Ser Leu Asn Ile Arg Asp Asp
            275                 280                 285

Lys Thr Asp Ile Tyr Arg Ile Thr Ala Thr Lys Asn Lys Gln Arg Arg
            290                 295                 300

Asp Gly Asp Lys Ile Lys Lys Ile Val Asn Ile Gln Asn Ser Ala Asn
305                 310                 315                 320

Ile Gln Asn Ser Ala Asn Ile Gln Asn Ser Val Asn Ile Gln Asn Ser
            325                 330                 335

Val Asn Ile Gln Asn Ser Lys Asp Asn Gln Asp Tyr Val Tyr Asp Leu
            340                 345                 350

```
Thr Thr Glu Asn His His His Phe Ala Ala Gly Ile Gly Asn Met Ile
        355                 360                 365


Val His Asn
        370



<210> 168
<211> 348
<212> PRT
<213> Artificial sequence

<220>
<223> modified contiguous intein from Antarctica Ace lake environmental
      sampling


<220>
<221> misc_feature
<222> (36)..(36)
<223> Xaa can be any naturally occurring amino acid

<220>
<221> misc_feature
<222> (47)..(47)
<223> Xaa can be any naturally occurring amino acid

<220>
<221> misc_feature
<222> (149)..(149)
<223> Xaa can be any naturally occurring amino acid

<220>
<221> misc_feature
<222> (154)..(154)
<223> Xaa can be any naturally occurring amino acid

<220>
<221> misc_feature
<222> (179)..(179)
<223> Xaa can be any naturally occurring amino acid

<220>
<221> misc_feature
<222> (277)..(277)
<223> Xaa can be any naturally occurring amino acid

<400> 168

Ser Val Ala Lys Tyr Thr Pro Val Tyr Val Lys Val Asp Gly Lys Leu
1               5               10                  15


Gln Ile Val Glu Met Glu Lys Leu Ala Glu Gln Tyr Gly Gly Asn Gln
            20              25                  30


Trp Thr Thr Xaa Leu Glu Glu Gly Lys Gln Glu Lys Glu Phe Xaa Glu
        35              40                  45
```

```
Leu Tyr Gly Val Glu Thr Trp Thr Asp Lys Gly Trp Thr Lys Leu His
    50              55                  60

Arg Ile Ile Arg His Gln Leu Ala Ser His Lys Lys Met Ile Arg Ile
65              70                  75                      80

Leu Thr His Thr Gly Met Val Asp Val Thr Asp Asp His Ser Leu Val
                85                  90                  95

Leu Glu Asp Gly Asn Glu Ile Ser Pro Lys Glu Val Asp Ile Gly Thr
            100                 105                 110

Lys Leu Leu His Lys Thr Leu Glu Tyr Glu Ser Pro Gln Phe Glu Val
        115                 120                 125

Glu Asn Asn Ile Ser Ala Asp Met Ala Lys Ile Tyr Gly Phe Phe Phe
    130                 135                 140

Gly Asp Gly Ser Xaa Gly Ile Tyr Asp Xaa Pro Ser Gly Lys Lys Ala
145             150                 155                     160

Ser Trp Ala Leu Asn Asn Ala Asn Glu Glu Leu Leu Asp Lys Tyr Ile
            165                 170                 175

Lys Leu Xaa Arg Lys Ser Tyr Pro Glu Phe Asp Trp Gln Ile Tyr Asp
            180                 185                 190

Thr Ile Glu Ser Ser Gly Val Tyr Lys Leu Thr Phe Asn Gly Asn Val
        195                 200                 205

Tyr Gly Asn Lys Ser Lys Phe Ile Glu Thr Tyr Arg Lys His Met Tyr
    210                 215                 220

Ser Gly Lys Ser Lys Ile Ile Pro Asp Phe Ile Leu Asn Gly Thr His
225                 230                 235                 240

Glu Ile Arg Glu Ala Phe Trp Glu Gly Leu Tyr Asp Ala Asp Gly Asp
            245                 250                 255

Lys Asp Lys Asn Gly Tyr Val Arg Ile Asp Gln Lys Asn Gln Ile Ser
            260                 265                 270

Ala Ala His Ile Xaa Trp Leu Ala Asn Ser Ile Gly Tyr Lys Thr Ser
        275                 280                 285

Ile Asn Thr Arg Thr Asp Lys Gln Asn Ile Tyr Arg Ile Thr Ala Thr
    290                 295                 300
```

Lys Gly Ala Gln Arg Lys Glu Gly Asn Ala Ile Lys Lys Leu Met Glu
305                     310                 315                 320

Leu Asp Tyr His Asp Tyr Val Tyr Asp Leu Thr Thr Glu Asn His His
                    325                 330                 335

Phe Ala Ala Gly Ile Gly Asn Met Ile Val His Asn
                    340                 345

```
<210>  169
<211>  290
<212>  PRT
<213>  Artificial sequence

<220>
<223>  modified contiguous intein (partial) from Antarctica Ace lake
       environmental sampling


<220>
<221>  misc_feature
<222>  (36)..(36)
<223>  Xaa can be any naturally occurring amino acid

<220>
<221>  misc_feature
<222>  (47)..(47)
<223>  Xaa can be any naturally occurring amino acid

<220>
<221>  misc_feature
<222>  (129)..(129)
<223>  Xaa can be any naturally occurring amino acid

<220>
<221>  misc_feature
<222>  (150)..(150)
<223>  Xaa can be any naturally occurring amino acid

<220>
<221>  misc_feature
<222>  (155)..(155)
<223>  Xaa can be any naturally occurring amino acid

<220>
<221>  misc_feature
<222>  (180)..(180)
<223>  Xaa can be any naturally occurring amino acid

<220>
<221>  misc_feature
<222>  (183)..(183)
<223>  Xaa can be any naturally occurring amino acid

<220>
<221>  misc_feature
<222>  (223)..(223)
<223>  Xaa can be any naturally occurring amino acid
```

```
<220>
<221>  misc_feature
<222>  (280)..(280)
<223>  Xaa can be any naturally occurring amino acid

<220>
<221>  misc_feature
<222>  (290)..(290)
<223>  Xaa can be any naturally occurring amino acid

<400>  169
```

Ser Val Ala Lys Tyr Thr Pro Val Tyr Val Arg Ala Asn Gly Gln Leu
1           5               10              15

Gln Ile Val Glu Met Glu Gln Leu Ala Glu Lys Tyr Gly Gly Asn Asn
        20              25              30

Trp Ser Lys Xaa Ile Glu Glu Gly Lys Gln Glu Lys Glu Phe Xaa Glu
        35              40              45

Leu Thr Asn Ile Glu Thr Trp Thr Asn Lys Gly Trp Thr Lys Leu Tyr
    50              55              60

Arg Ile Ile Arg His Lys Leu Ala Ser His Lys Lys Met Val Arg Ile
65              70              75              80

Leu Thr His Thr Gly Met Val Asp Val Thr Asp Asp His Ser Leu Leu
            85              90              95

Leu Glu Asp Gly Ser Glu Val Ser Pro Lys Asn Val Glu Ile Gly Thr
            100             105             110

Lys Leu Leu His Lys Thr Leu Lys His Asp Ala Leu Lys Pro Asn Ala
        115             120             125

Xaa Leu Asp Thr Val Ser Val Asp Met Ala Lys Ile Tyr Gly Phe Phe
    130             135             140

Phe Gly Asp Gly Ser Xaa Gly Ile Tyr Asn Xaa Pro Ser Gly Lys Lys
145             150             155             160

Ala Thr Trp Ala Leu Asn Asn Ser Asn Val Glu Leu Leu Asp Lys Tyr
            165             170             175

Ile Asn Leu Xaa Lys Lys Xaa Tyr Pro Glu Phe Thr Trp Gln Thr Tyr
            180             185             190

Asn Thr Ile Glu Ser Ser Gly Leu Tyr Lys Ile Thr Phe Asn Ser Asn
    195             200             205

```
Glu Tyr Gly Thr Lys Ser Arg Phe Ile Glu Thr Tyr Arg Asn Xaa Met
    210                 215                 220

Tyr Thr Gly Asn Ser Lys Ile Ile Pro Asp Phe Ile Leu Asn Gly Ser
225                 230                 235                 240

Asn Glu Ile Arg Glu Ala Phe Trp Glu Gly Met Tyr Asp Ala Ala Ala
                245                 250                 255

Asp Gly Asp Lys Asp Glu Asn Gly Tyr Val Arg Ile Asp Gln Lys Asn
            260                 265                 270

Gln Ile Ser Ala Ala His Ile Xaa Trp Leu Ala Asn Ser Met Asp Ile
            275                 280                 285

Arg Xaa
        290
```

```
<210>  170
<211>  328
<212>  PRT
<213>  Artificial sequence

<220>
<223>  modified contiguous intein from Bathycoccus species RCC1105 virus
       BpV2

<220>
<221>  misc_feature
<222>  (19)..(19)
<223>  Xaa can be any naturally occurring amino acid

<220>
<221>  misc_feature
<222>  (107)..(107)
<223>  Xaa can be any naturally occurring amino acid

<220>
<221>  misc_feature
<222>  (114)..(114)
<223>  Xaa can be any naturally occurring amino acid

<220>
<221>  misc_feature
<222>  (136)..(136)
<223>  Xaa can be any naturally occurring amino acid

<220>
<221>  misc_feature
<222>  (141)..(141)
<223>  Xaa can be any naturally occurring amino acid

<220>
<221>  misc_feature
<222>  (166)..(166)
```

<223> Xaa can be any naturally occurring amino acid

<220>
<221> misc_feature
<222> (213)..(213)
<223> Xaa can be any naturally occurring amino acid

<220>
<221> misc_feature
<222> (299)..(299)
<223> Xaa can be any naturally occurring amino acid

<400> 170

Ser Val Thr Pro Asp Thr Pro Leu Leu Ile Arg Gln Asn Gly Thr Val
1               5                   10                  15

His Thr Xaa Arg Ile Asp Ser Leu Val Asn Glu Tyr Thr Leu Arg Asp
            20                  25                  30

Asp Gly Lys Glu Ile Gly Tyr Ile Asn Ala Glu Val Trp Thr Glu Asn
            35                  40                  45

Gly Phe Thr Ser Ile Gln Gln Ile Val Arg His Lys Thr Asn Lys Asn
        50                  55                  60

Ile His Arg Val Val Thr His Thr Gly Ile Val Asp Val Thr Glu Asp
65                  70                  75                  80

His Ser Leu Leu Leu Glu Asn Lys Glu Ile Ala Lys Pro Thr Gln Val
                85                  90                  95

Gly Val Gly Thr Ala Leu Leu His Gly Asn Xaa Val Glu Ser Ile Asp
            100                 105                 110

Thr Xaa Thr Asp Thr Ser Ile Thr Lys Glu Glu Ala Lys Val Met Gly
        115                 120                 125

Phe Phe Phe Gly Asp Gly Ser Xaa Gly Thr Tyr Gln Xaa Arg Ser Gly
    130                 135                 140

Val Lys Ser Thr Trp Ala Leu Asn Asn Ser Lys Leu Glu Tyr Leu Glu
145                 150                 155                 160

Glu Met Gln Lys Leu Xaa Pro Phe Glu Thr Lys Ile Tyr Asp Thr Ile
                165                 170                 175

Lys Ser Ser Gly Val Tyr Lys Leu Asn Ala Lys Gly Leu Val Val Asp
            180                 185                 190

Ile Val Asn Lys Tyr Arg Asn Leu Phe Tyr Asn Ser His Lys Glu Lys

|     | 195 |     |     |     |     | 200 |     |     |     |     | 205 |     |     |     |
| --- | --- | --- | --- | --- | --- | --- | --- | --- | --- | --- | --- | --- | --- | --- |

Val Val Pro Ser Xaa Ile Leu Asn Ala Pro Leu Glu Ile Ile Lys Ser
    210                 215                 220

Phe Val Asp Gly Tyr Tyr Met Ala Asp Gly Asp Lys Asp Lys Asn Gly
225                 230                 235                 240

Tyr Thr Arg Met Asp Val Lys Gly Lys Glu Gly Ser Met Gly Met Tyr
                245                 250                 255

Met Leu Gly Arg Lys Leu Gly Tyr Asn Val Ser Ile Asn Thr Arg Thr
                260                 265                 270

Asp Lys Val Asn Val Phe Arg Gln Thr Trp Thr Lys Ser Leu Gln Arg
                275                 280                 285

Lys Ser Pro Thr Lys Ile Lys Lys Leu Glu Xaa Leu Gly Glu Thr Asp
    290                 295                 300

Gly Tyr Val Tyr Asp Leu Thr Thr Lys Ser His His Phe His Val Gly
305                 310                 315                 320

Pro Gly Asp Leu Val Val His Asn
                325

```
<210>  171
<211>  323
<212>  PRT
<213>  Artificial sequence

<220>
<223>  modified contiguous intein from unknown phycodnavirus KBvp-16


<220>
<221>  misc_feature
<222>  (19)..(19)
<223>  Xaa can be any naturally occurring amino acid

<220>
<221>  misc_feature
<222>  (94)..(94)
<223>  Xaa can be any naturally occurring amino acid

<220>
<221>  misc_feature
<222>  (107)..(107)
<223>  Xaa can be any naturally occurring amino acid

<220>
<221>  misc_feature
<222>  (135)..(135)
<223>  Xaa can be any naturally occurring amino acid
```

<220>
<221>  misc_feature
<222>  (161)..(161)
<223>  Xaa can be any naturally occurring amino acid

<220>
<221>  misc_feature
<222>  (189)..(189)
<223>  Xaa can be any naturally occurring amino acid

<220>
<221>  misc_feature
<222>  (208)..(208)
<223>  Xaa can be any naturally occurring amino acid

<400>  171

Ser Val Thr Pro Asp Thr Pro Leu Leu Leu Arg Ile Lys Gly Glu Val
1               5                   10                  15

Lys Thr Xaa Arg Ile Asp Ser Leu Val Glu Ser Tyr Glu Glu Arg Asp
            20                  25                  30

Asp Gly Lys Glu Val Ala Glu Ile Asp Ala Glu Val Trp Thr Glu Lys
            35                  40                  45

Gly Phe Thr Pro Ile Gln Gln Ile Val Arg His Lys Thr Thr Lys Asn
        50                  55                  60

Ile His Arg Val Leu Thr His Thr Gly Val Val Asp Val Thr Glu Asp
65                  70                  75                  80

His Ser Leu Leu Leu Glu Asn Lys Gln Met Ile Lys Pro Xaa Glu Val
                85                  90                  95

Ser Leu Gly Thr Asn Leu Leu His Gly Asp Xaa Val Tyr Gly Leu Asn
            100                 105                 110

Trp Asn Asp Thr Thr Val Ser Val Asn Glu Ala Lys Val Met Gly Phe
            115                 120                 125

Phe Phe Gly Asp Gly Ser Xaa Gly His Tyr Gly Asp Lys Tyr Thr Trp
        130                 135                 140

Ala Leu Asn Asn Ser Asn Val Asp Tyr Leu Ile Glu Met Gln Asn Leu
145                 150                 155                 160

Xaa Pro Phe Glu Thr Ser Ile Tyr Asp Thr Ile Glu Ser Ser Gly Val
            165                 170                 175

Tyr Lys Leu Asn Ala Lys Gly Asp Val Lys Asn Ile Xaa Glu Arg Tyr

```
                    180                    185                      190


        Arg Ser Met Phe Tyr Asn Ala His Lys Glu Lys Ile Val Pro Ser Xaa
                195                 200                 205


        Ile Leu Asn Ala Pro Ile Glu Val Val Glu Ser Phe Trp Glu Gly Tyr
            210                 215                 220


        Tyr Met Ala Asp Gly Asp Lys Asp Val His Gly Tyr Thr Arg Met Asp
        225                 230                 235                 240


        Ile Lys Gly Lys Glu Gly Ser Met Gly Met Phe Ile Leu Gly Lys Arg
                        245                 250                 255


        Leu Asn Tyr Asn Val Ser Leu Asn Thr Arg Lys Asp Lys Pro Asp Val
                260                 265                 270


        Phe Arg Gln Thr Trp Thr Lys Ser Thr Gln Arg Lys Ser Pro Asn Ala
                275                 280                 285


        Ile Lys Lys Leu Glu Leu Val Gly Glu Thr Glu Gly Tyr Val Tyr Asp
            290                 295                 300


        Leu Thr Thr Glu Ser His His Phe His Ile Gly Pro Gly Asp Leu Val
        305                 310                 315                 320


        Val His Asn
```

```
<210>  172
<211>  437
<212>  PRT
<213>  Artificial sequence

<220>
<223>  modified contiguous intein from Haloferax alexandrinus JCM 10717


<220>
<221>  misc_feature
<222>  (395)..(395)
<223>  Xaa can be any naturally occurring amino acid

<400>  172
```

```
        Ser Val Thr Gly Asp Arg Pro Val Val Val Arg Asp Pro Gly Gly Thr
        1                   5                   10                  15


        Val Arg Ile Leu Pro Ile Glu Asp Leu Phe Ala Arg Gly Thr Thr Glu
                    20                  25                  30
```

```
Ser Glu Val Leu Ile Ala Ala Asp Gly Asp Val Val Ala Ser Ala Thr
        35              40              45

Pro Gly Lys Thr Arg Arg Ala Leu Asp Gly Trp Asp Ala Leu Ser Val
        50              55              60

Asn Glu Ala Gly Glu Ala Glu Trp Gln Pro Ile Ala Gln Ala Ile Arg
65              70              75              80

His Lys Thr Asp Lys Pro Val Val Asn Leu Gln His Lys Phe Gly Glu
        85              90              95

Ser Thr Thr Thr Arg Asp His Ser Tyr Val Val Pro Gly Glu His Gly
        100             105             110

Leu Thr Thr Val Ser Pro Asp Asp Val Ala Glu Pro Tyr Arg Val Ser
        115             120             125

Gly Val Pro Asp Val Glu Pro Val Glu Gln Val Asp Val Tyr Glu Val
130             135             140

Leu Arg Gly Tyr Glu Arg Glu Tyr Glu Asp Gly Arg Ser Val Gly Ser
145             150             155             160

Asp Asn Ser Ile Thr Lys Arg Lys Gln Ile His Ala Asp Asp Glu Tyr
            165             170             175

Val Trp Phe Gly His Glu His His Arg Asp Val Asp Ser Thr Val Lys
        180             185             190

Val Lys Arg Phe Val Asp Ile Asp Ser Glu Asp Gly Ala Ala Leu Ile
        195             200             205

Arg Leu Leu Gly Ala Tyr Val Ser Glu Gly Ser Ala Ser Thr Gly Glu
210             215             220

Thr Ala Thr Ser Lys Phe Gly Ala Ser Ile Ala Glu Ser Asp Arg Glu
225             230             235             240

Trp Leu Ala Gln Leu Gln Arg Asp Tyr Ser Arg Leu Phe Glu Asn Thr
            245             250             255

Thr Ala Asp Ile Ile Thr Ser Asp Arg Arg Ala Glu Arg Thr Val Glu
        260             265             270

Tyr Gln Thr Asp Thr Gly Gly Ala Ser Val Thr Tyr Asn Asp Glu Thr
        275             280             285
```

```
Leu Lys Leu Gln Met Met Asn Glu Leu Ala Ala Val Phe Phe Arg Glu
    290             295             300

Phe Ala Gly Gln Thr Ser Arg Gly Lys Arg Ile Pro Ser Phe Val Phe
305             310             315             320

His Leu Pro Glu Glu Lys Gln Asp Leu Phe Leu Thr Leu Leu Val Glu
            325             330             335

Gly Asp Gly Ser Arg Glu Phe Pro Arg Tyr Thr Glu Ala Tyr Ala Gln
            340             345             350

Arg Asn Phe Asp Phe Glu Thr Thr Ser Arg Glu Leu Ala Ala Gly Leu
        355             360             365

Ser Met Leu Leu Thr Gln Arg Gly Gln Lys His Ser Leu Lys Tyr Arg
    370             375             380

Asp Ser Lys Asp Ser Tyr Thr Ile Arg Thr Xaa Ser Thr Tyr Arg Glu
385             390             395             400

Gly Arg Asp Pro Val Leu Thr Glu Val Asp His Asp Gly Tyr Val Tyr
            405             410             415

Asp Leu Ser Val Glu Glu Asn Glu Asn Phe Val Asp Gly Val Gly Gly
            420             425             430

Ile Val Leu His Asn
            435


<210>  173
<211>  437
<212>  PRT
<213>  Artificial sequence

<220>
<223>  modified contiguous intein from Haloferax prahovense DSM 18310

<220>
<221>  misc_feature
<222>  (395)..(395)
<223>  Xaa can be any naturally occurring amino acid

<400>  173

Ser Val Thr Gly Asp Arg Pro Val Val Val Arg Asp Pro Gly Gly Thr
1               5               10              15

Val Arg Ile Leu Pro Ile Glu Asp Leu Phe Ala Arg Gly Thr Thr Glu
            20              25              30
```

```
Ser Glu Val Leu Ile Ala Ala Asp Gly Asp Val Val Ala Ser Ala Thr
        35              40              45

Pro Gly Lys Thr Arg Arg Ala Leu Asp Gly Trp Asp Ala Leu Ser Val
        50              55              60

Asn Glu Asp Gly Glu Ala Glu Trp Gln Pro Ile Ala Gln Ala Ile Arg
65              70              75              80

His Asn Thr Asp Lys Pro Val Val Asn Leu Gln His Lys Phe Gly Glu
            85              90              95

Ser Thr Thr Thr Arg Asp His Ser Tyr Val Val Pro Gly Glu Asp Gly
            100             105             110

Leu Thr Thr Val Ser Pro Asp Asp Val Ala Glu Pro Tyr Arg Val Ser
        115             120             125

Gly Val Pro Asp Val Glu Pro Val Glu Gln Val Asp Val Tyr Glu Val
        130             135             140

Leu Arg Gly Tyr Glu Arg Glu Tyr Glu Asp Gly Arg Ser Val Gly Ser
145             150             155             160

Asp Asn Ser Ile Thr Lys Arg Lys Gln Ile His Ala Asp Asp Glu Tyr
                165             170             175

Val Trp Phe Gly His Glu His His Arg Asp Val Asp Ser Thr Val Lys
            180             185             190

Val Lys Arg Phe Val Asp Ile Asp Ser Glu Asp Gly Ala Ala Leu Ile
        195             200             205

Arg Leu Leu Gly Ala Tyr Val Pro Glu Gly Ser Ala Ser Thr Gly Glu
        210             215             220

Thr Ala Thr Ser Glu Phe Gly Ala Ser Ile Ala Glu Ser Asp Arg Glu
225             230             235             240

Trp Leu Ala Gln Leu Gln Arg Asp Tyr Ser Arg Leu Phe Glu Asn Thr
                245             250             255

Thr Ala Gly Ile Ile Thr Ser Asp Arg Arg Ala Glu Arg Thr Val Glu
            260             265             270

Tyr Gln Thr Asp Thr Gly Gly Ala Ser Val Thr Tyr Asn Asp Glu Thr
```

```
              275                    280                    285


    Leu Lys Leu Gln Met Met Asn Glu Leu Ala Ala Val Phe Phe Arg Glu
        290                    295                    300


    Phe Ala Gly Gln Thr Ser Arg Gly Lys Arg Ile Pro Ser Phe Val Phe
    305                    310                    315                    320


    His Leu Pro Glu Glu Lys Gln Asp Leu Phe Leu Thr Leu Leu Val Glu
                   325                    330                    335


    Gly Asp Gly Ser Arg Glu Phe Pro Arg Tyr Thr Glu Ala Tyr Ala Gln
                   340                    345                    350


    Arg Asn Phe Asp Phe Glu Thr Thr Ser Arg Glu Leu Ala Ala Gly Leu
            355                    360                    365


    Ser Met Leu Leu Thr Gln Arg Gly Gln Lys His Ser Leu Lys Tyr Arg
        370                    375                    380


    Asp Ser Lys Asp Ser Tyr Thr Ile Arg Thr Xaa Ser Thr Tyr Arg Glu
    385                    390                    395                    400


    Gly Arg Asp Pro Val Leu Thr Glu Ala Asp His Asp Gly Tyr Val Tyr
                   405                    410                    415


    Asp Leu Ser Val Glu Glu Asn Glu Asn Phe Val Asp Gly Val Gly Gly
                   420                    425                    430


    Ile Val Leu His Asn
               435


    <210>  174
    <211>  437
    <212>  PRT
    <213>  Artificial sequence

    <220>
    <223>  modified contiguous intein from Natronomonas moolapensis 8.8.11


    <220>
    <221>  misc_feature
    <222>  (204)..(204)
    <223>  Xaa can be any naturally occurring amino acid

    <220>
    <221>  misc_feature
    <222>  (208)..(208)
    <223>  Xaa can be any naturally occurring amino acid

    <220>
```

```
<221>  misc_feature
<222>  (228)..(228)
<223>  Xaa can be any naturally occurring amino acid

<400>  174

Ser Val Ser Gly Asp Arg Pro Val Val Val Arg Asp Pro Asp Gly Thr
1               5                   10                  15

Ile Arg Ile Val Pro Ile Glu Ala Leu Phe Asp Arg Ala Glu Lys Arg
                20                  25                  30

Pro Asp Asp Arg Val Phe Val Ala Ala Asp Gly Asp Thr Gln Ile Asp
            35                  40                  45

Asp Gly Ser Arg Lys Glu Phe Ala Asp Leu Asp Gly Trp Asp Ala Leu
        50                  55                  60

Ser Leu Ser Glu Glu Gly Thr Ala Gly Trp Arg Pro Ile Glu Arg Val
65                  70                  75                  80

Ile Arg His Arg Thr Asp Arg Pro Val Val Asn Leu Gln His Lys Phe
                85                  90                  95

Gly Glu Ser Thr Thr Thr Arg Asn His Ser Tyr Ile Val Asp Asp Asn
            100                 105                 110

Ser Lys Tyr Val Glu Ala Thr Pro Glu Glu Val Asp Glu Pro Leu Arg
        115                 120                 125

Ile Pro Asp Leu Pro Ala Glu Thr Arg Asn Asp Ala Ile Asp Val Tyr
    130                 135                 140

Glu Val Leu Ser Gly Tyr Glu Arg Glu Tyr Glu Asp Gly Arg Gly Thr
145                 150                 155                 160

Gly Gly Thr Thr Ile Lys Thr Lys Arg Val His Ala Asn Asp Glu Tyr
                165                 170                 175

Val Trp Phe Gly His Asp His Tyr Gly Glu Leu Asp Ser Thr Val Lys
            180                 185                 190

Val Lys Arg Tyr Ile Arg Pro Gly Thr Gln Glu Xaa Val Ser Leu Xaa
        195                 200                 205

Arg Leu Leu Ala Ala Tyr Val Thr Glu Gly Ser Ala Thr Thr Lys Glu
    210                 215                 220

Thr Ser Asp Xaa Arg Tyr Gly Ala Ser Ile Ala Glu Ser Arg Arg Arg
```

225                     230                     235                     240

Trp Leu Val Gly Leu Arg Asp Asp Tyr Tyr Arg Leu Phe Glu Asn Thr
            245                 250                 255

Thr Ala Ser Val Ile Asp Asn Asp Ser Ser Asp Glu Arg Thr Ile Glu
            260                 265                 270

Tyr Arg Thr Asp His Gly Asp Thr Ala Val Ser Tyr Asp Asp Gly Thr
            275                 280                 285

Lys Lys Leu Gln Met Met Asn Glu Leu Ala Ala Val Phe Phe Arg Glu
    290                 295                 300

Phe Ala Gly Gln Thr Ser Arg Glu Lys Arg Leu Pro Ser Phe Val Tyr
305                 310                 315                 320

His Leu Gln Asp Asp Glu Gln Asp Leu Phe Leu Glu Thr Leu Ile Glu
            325                 330                 335

Gly Asp Gly Ser Arg Glu Phe Pro Arg Tyr Ser Asp Ala Tyr Ala Glu
            340                 345                 350

Arg Asn Phe Asp Phe Glu Thr Ile Ser Arg Glu Leu Ala Ala Gly Leu
            355                 360                 365

Ser Met Leu Leu Ile Gln Arg Gly Lys Lys His Ser Leu Lys Tyr Arg
    370                 375                 380

Asp Ala Lys Asp Ser Tyr Thr Ile Arg Thr Val Asp Ser Tyr Arg Arg
385                 390                 395                 400

Gly Arg Asp Pro Val Leu Arg Glu Val Asp His Asp Gly Tyr Val Tyr
            405                 410                 415

Asp Leu Ser Val Ala Glu Asn Asp Asn Phe Val Asp Gly Val Gly Gly
            420                 425                 430

Val Val Leu His Asn
        435

<210>  175
<211>  437
<212>  PRT
<213>  Artificial sequence

<220>
<223>  modified contiguous intein from Haloferax volcanii DS2 ATCC29605

<220>
<221>    misc_feature
<222>    (395)..(395)
<223>    Xaa can be any naturally occurring amino acid

<400>    175

Ser Val Thr Gly Asp Arg Pro Val Val Val Arg Asp Pro Gly Gly Thr
1               5                   10                  15

Val Arg Ile Leu Pro Ile Glu Asp Leu Phe Ala Arg Gly Thr Thr Glu
            20                  25                  30

Ser Glu Val Leu Ile Ala Ala Asp Gly Asp Val Val Ala Ser Ala Thr
            35                  40                  45

Pro Gly Lys Thr Arg Arg Ala Leu Asp Gly Trp Asp Ala Leu Ser Val
        50                  55                  60

Asn Glu Asp Gly Glu Ala Glu Trp Gln Pro Ile Ala Gln Ala Ile Arg
65                  70                  75                  80

His Asn Thr Asp Lys Pro Val Val Asn Leu Gln His Lys Phe Gly Glu
                85                  90                  95

Ser Thr Thr Thr Arg Asp His Ser Tyr Val Val Pro Gly Glu Asp Gly
            100                 105                 110

Leu Thr Thr Val Ser Pro Asp Asp Val Ala Glu Pro Tyr Arg Val Ser
            115                 120                 125

Gly Val Pro Asp Val Glu Pro Val Glu Gln Val Asp Val Tyr Glu Val
        130                 135                 140

Leu Arg Gly Tyr Glu Arg Glu Tyr Glu Asp Gly Arg Ser Val Gly Ser
145                 150                 155                 160

Asp Asn Ser Ile Thr Lys Arg Lys Gln Ile His Ala Asp Asp Glu Tyr
                165                 170                 175

Val Trp Phe Gly His Glu His His Arg Asp Val Asp Ser Thr Val Lys
            180                 185                 190

Val Lys Arg Phe Val Asp Ile Asp Ser Glu Asp Gly Ala Ala Leu Ile
            195                 200                 205

Arg Leu Leu Gly Ala Tyr Val Pro Glu Gly Ser Ala Ser Thr Gly Glu
        210                 215                 220

```
Thr Ala Thr Ser Lys Phe Gly Ala Ser Leu Ala Glu Ser Asp Arg Glu
225             230             235             240

Trp Leu Ala Gln Leu Gln Arg Asp Tyr Ser Arg Leu Phe Glu Asn Thr
                245             250             255

Thr Ala Gly Ile Ile Thr Ser Asp Arg Arg Ala Glu Arg Thr Val Glu
            260             265             270

Tyr Gln Thr Asp Thr Gly Gly Ala Ser Val Thr Tyr Asn Asp Glu Thr
        275             280             285

Leu Lys Leu Gln Met Met Asn Glu Leu Ala Ala Val Phe Phe Arg Glu
    290             295             300

Phe Ala Gly Gln Thr Ser Arg Gly Lys Arg Ile Pro Ser Phe Val Phe
305             310             315             320

His Leu Pro Glu Glu Lys Gln Asp Leu Phe Leu Thr Leu Leu Val Glu
                325             330             335

Gly Asp Gly Ser Arg Glu Phe Pro Arg Tyr Thr Glu Ala Tyr Ala Gln
            340             345             350

Arg Asn Phe Asp Phe Glu Thr Thr Ser Arg Glu Leu Ala Ala Gly Leu
        355             360             365

Ser Met Leu Leu Thr Gln Arg Gly Gln Lys His Ser Leu Lys Tyr Arg
    370             375             380

Asp Ser Lys Asp Ser Tyr Thr Ile Arg Thr Xaa Ser Thr Tyr Arg Glu
385             390             395             400

Gly Arg Asp Pro Val Leu Thr Glu Ala Asp His Asp Gly Tyr Val Tyr
                405             410             415

Asp Leu Ser Val Glu Glu Asn Glu Asn Phe Val Asp Gly Val Gly Gly
            420             425             430

Ile Val Leu His Asn
        435
```

```
<210>  176
<211>  431
<212>  PRT
<213>  Artificial sequence

<220>
```

<223> modified contiguous intein from Haloquadratum walsbyi DSM 16790


<220>
<221> misc_feature
<222> (169)..(169)
<223> Xaa can be any naturally occurring amino acid


<220>
<221> misc_feature
<222> (233)..(233)
<223> Xaa can be any naturally occurring amino acid


<220>
<221> misc_feature
<222> (247)..(247)
<223> Xaa can be any naturally occurring amino acid


<220>
<221> misc_feature
<222> (279)..(279)
<223> Xaa can be any naturally occurring amino acid


<220>
<221> misc_feature
<222> (369)..(369)
<223> Xaa can be any naturally occurring amino acid


<220>
<221> misc_feature
<222> (396)..(396)
<223> Xaa can be any naturally occurring amino acid


<400> 176

Ser Val Thr Gly Asp Arg Pro Val Val Val Arg Asp Pro Ser Asp Tyr
1               5                   10                  15


Ile Gln Ile Val Pro Ile Lys Leu Leu Phe Glu Gln Ala Thr Ala Pro
            20                  25                  30


Glu Gln Asn Met Arg Leu Thr Ala Asp Gly Ala Pro Ser Val Asn Ser
            35                  40                  45


Glu Leu Pro Lys Glu Arg Arg His Leu Asp Gln Trp Glu Ala Leu Ser
        50                  55                  60


Leu Ser Asp Thr Gly Glu Thr Glu Trp Gln Pro Ile Asn Gln Ile Ile
65                  70                  75                  80


Arg His Gln Thr Asp Lys Glu Ile Leu Thr Leu Gln His Glu Tyr Gly
                85                  90                  95


Glu Ser Thr Thr Thr Arg Asp His Ser Tyr Ile Thr Ala Asp Asp Gly
                100                 105                 110

```
Glu Tyr Val Glu Thr Ser Pro Glu Asn Val Asp Glu Pro Leu Pro Ile
        115             120             125

Pro Asn Ile Ala Ser Val Lys Thr Ile Glu Thr Ile Asp Ile Tyr Gln
        130             135             140

Thr Leu Thr Thr Asp Thr Gln Ala Gln Ile Gly Asn Asp Thr Glu Pro
145             150             155             160

Asp Lys Trp Leu Pro Ser Ala Asp Xaa Ile His Ala Asn Asp Glu Tyr
            165             170             175

Val Trp Ile Gly Thr Thr Asp Lys Gln Gln Asp Arg Asp Asp Ser Thr
            180             185             190

Pro Ala Ile Pro Arg Tyr Ile Asp Leu Thr Ser Asp Thr Gly His Ala
            195             200             205

Leu Ile Arg Phe Leu Ala Val Tyr Leu Ser Asp Trp Ser Lys Ser Thr
        210             215             220

Ile Thr Thr Thr Glu Arg Gly Gln Xaa Leu His Ile Thr Gly Pro Gln
225             230             235             240

Glu Ser Ala Leu Lys Thr Xaa Ala Ala Asp Ala Asp Gln Leu Phe Thr
            245             250             255

His Ile Thr Pro Ser Ile Ala Val Asp Ala Glu Ser Asn Thr Asn Thr
            260             265             270

Val Asp Ser Gly Phe Arg Xaa His Ile Pro Thr Thr Leu Ala Thr Thr
            275             280             285

Leu Ile Ser Ala Phe Ala Gly His Pro Ala His Thr Lys Gln Ile Pro
        290             295             300

Ser Ile Val Tyr His Leu Pro Ala Ala Glu Gln Ser Leu Phe Ile Arg
305             310             315             320

His Leu Ile Gln Ala Glu Ser Thr Pro Glu Ser Asp Gly Val Ser Gly
            325             330             335

Arg Pro Gln Lys Ser Asp Lys Pro Ile Leu Leu Glu Asn Glu Phe Ile
            340             345             350

Thr Thr Asn Arg Glu Leu Ala Ala Gly Val Ser Met Leu Leu Thr Gln
            355             360             365
```

Xaa Gly Gln Ser Tyr Thr Ile Ser Lys Gln Asp Thr Lys Gly Ala Tyr
    370                 375                 380

Thr Ile His Ile Asn Asn Ser Ser Ser Ser Gly Xaa Thr Pro Thr Leu
    385                 390                 395                 400

Thr Glu Thr Thr His Ser Gly Tyr Val Tyr Asp Leu Ser Val Ala Thr
                405                 410                 415

Asn Gln Asn Phe Val Asp Gly Leu Gly Gly Leu Val Leu His Asn
            420                 425                 430


<210> 177
<211> 431
<212> PRT
<213> Artificial sequence

<220>
<223> modified contiguous intein from Haloquadratum walsbyi DSM 16854


<220>
<221> misc_feature
<222> (169)..(169)
<223> Xaa can be any naturally occurring amino acid

<220>
<221> misc_feature
<222> (233)..(233)
<223> Xaa can be any naturally occurring amino acid

<220>
<221> misc_feature
<222> (247)..(247)
<223> Xaa can be any naturally occurring amino acid

<220>
<221> misc_feature
<222> (279)..(279)
<223> Xaa can be any naturally occurring amino acid

<220>
<221> misc_feature
<222> (369)..(369)
<223> Xaa can be any naturally occurring amino acid

<220>
<221> misc_feature
<222> (396)..(396)
<223> Xaa can be any naturally occurring amino acid

<400> 177

Ser Val Thr Gly Asp Arg Pro Val Val Val Arg Asp Pro Ser Asp Tyr
1                   5                   10                  15

Ile Gln Ile Val Pro Ile Lys Leu Leu Phe Glu Gln Ala Thr Ala Pro

```
                    20                        25                            30

        Glu Gln Asn Ile Arg Leu Thr Ala Asp Gly Ala Pro Ser Gly Asn Ser
                35                    40                    45

        Glu Phe Pro Lys Glu Arg Arg His Leu Asp Gln Trp Glu Ala Leu Ser
                50                    55                    60

        Leu Ser Asp Thr Gly Glu Thr Glu Trp Gln Pro Ile Asp Gln Ile Ile
        65                    70                    75                    80

        Arg His Gln Thr Asp Lys Glu Ile Leu Thr Leu Gln His Glu Tyr Gly
                        85                    90                    95

        Glu Ser Thr Thr Thr Arg Asp His Ser Tyr Ile Thr Ala Asp Asp Gly
                        100                   105                   110

        Gly Tyr Val Glu Thr Ser Pro Glu Asn Val Asp Glu Pro Leu Pro Ile
                        115                   120                   125

        Pro Asn Ile Ala Pro Val Lys Thr Val Glu Thr Ile Asp Ile Tyr Gln
                130                   135                   140

        Thr Leu Thr Thr Asp Thr Gln Ala Gln Ile Gly Ser Asp Thr Glu Pro
        145                   150                   155                   160

        Asp Lys Trp Leu Pro Ser Ala Asp Xaa Ile His Ala Asn Asp Glu Tyr
                        165                   170                   175

        Val Trp Ile Asp Thr Thr Asp Lys Gln Gln His Arg Asp Asp Ser Ile
                        180                   185                   190

        Pro Thr Ile Pro Arg Tyr Ile Asp Leu Ser Ser Asp Thr Gly His Ala
                        195                   200                   205

        Leu Ile Arg Phe Leu Ala Val Tyr Leu Ser Asp Trp Ser Glu Ser Thr
                        210                   215                   220

        Ile Thr Thr Thr Glu Arg Gly Arg Xaa Leu His Ile Thr Gly Pro Gln
        225                   230                   235                   240

        Glu Ser Ala Leu Lys Thr Xaa Ala Ala Asp Ala Asp Gln Leu Phe Thr
                        245                   250                   255

        His Ile Thr Pro Ser Ile Thr Val Asp Ala Glu Ser Asn Thr Asn Thr
                        260                   265                   270
```

```
Val Asp Ser Gly Phe Thr Xaa His Ile Pro Thr Thr Leu Ala Thr Ala
    275                 280             285

Leu Ile Ser Ala Phe Ala Gly His Pro Ala His Thr Lys Gln Ile Pro
    290                 295             300

Ser Ile Val Tyr His Leu Pro Ala Ala Glu Gln Ser Leu Phe Ile Arg
305                 310             315                 320

His Leu Ile Gln Ala Glu Ser Thr Pro Glu Ser Asp Gly Val Ser Gly
                325             330             335

Arg Pro Gln Lys Ser Asn Lys Pro Ile Leu Leu Glu Asn Glu Phe Ile
                340             345             350

Thr Thr Asn Arg Glu Leu Ala Ala Gly Val Ser Met Leu Leu Thr Gln
                355             360             365

Xaa Gly Gln Ser Tyr Thr Ile Ser Lys Gln Asp Thr Lys Gly Ala Tyr
    370                 375             380

Thr Ile His Ile Asn Asn Ser Ser Pro Ser Gly Xaa Thr Pro Thr Leu
385                 390             395                 400

Thr Glu Thr Thr His Ser Gly Tyr Val Tyr Asp Leu Ser Val Ala Thr
                405             410             415

Asn Gln Asn Phe Val Asp Gly Leu Gly Gly Leu Val Leu His Asn
                420             425             430
```

```
<210>  178
<211>  439
<212>  PRT
<213>  Artificial sequence

<220>
<223>  modified contiguous intein from Halorubrum californiensis DSM
       19288


<220>
<221>  misc_feature
<222>  (353)..(353)
<223>  Xaa can be any naturally occurring amino acid

<220>
<221>  misc_feature
<222>  (397)..(397)
<223>  Xaa can be any naturally occurring amino acid

<400>  178
```

```
Ser Val Thr Gly Gly Arg Pro Val Val Val Arg Asp Pro Glu Gly Ile
```

| 1 | | | | 5 | | | | | 10 | | | | | 15 | |
|---|---|---|---|---|---|---|---|---|---|---|---|---|---|---|---|

Val Arg Ile Leu Pro Ile Ala Asp Leu Phe Glu Arg Ser Asp Ala Thr
         20                25              30

Ala Ser Glu Asp Leu Ile Val Thr Ala Asp Gly Gly Pro Val Ala Ser
     35                40            45

Val Ser Ile Asp Lys Glu Arg Arg Ala Gly Gly Trp Glu Ala Leu
     50               55            60

Ser Val Thr Glu Asp Gly Glu Pro Glu Trp Gln Pro Ile Glu Gln Val
65              70            75            80

Ile Arg His Glu Thr Asp Lys Ser Val Val Asn Leu Gln His Lys Phe
         85              90           95

Gly Glu Ser Thr Thr Thr Arg Asp His Ser Tyr Val Val Glu Glu Asp
        100          105        110

Gly Gln Leu Val Glu Thr Lys Pro Glu Asp Val Glu Glu Pro Leu Arg
      115          120          125

Val Pro Gly Leu Pro Glu Val Glu Thr Val Glu Lys Ile Asp Val Tyr
    130            135          140

Asp Val Leu Glu Gly Tyr Thr Arg Glu Tyr Glu Asp Gly Arg Ser Val
145            150          155       160

Gly Ser Glu Asn Ala Glu Thr Lys Val Lys Arg Val His Ala Asn Asp
        165         170        175

Glu Trp Val Trp Phe Gly His Lys His His Asn Ala Ile Glu Arg Ser
       180         185        190

Ile Lys Val Gln Arg Tyr Val Asp Leu Asp Ser Glu Asp Gly Arg Ala
     195          200        205

Leu Val Arg Leu Leu Ala Ala Tyr Val Thr Glu Gly Ser Ala Ser Thr
     210          215        220

Ile Glu Thr Thr Asp Ser Arg Phe Gly Ala Ser Ile Ala Glu Ser Arg
225          230         235      240

Thr Glu Trp Leu Glu Gly Leu Arg Glu Asp Tyr Gln Arg Leu Phe Asp
      245          250        255

```
Gly Ala Thr Ala Ser Val Ile Ala Ser Asp Ala Ser Asp Arg Arg Thr
        260             265             270

Val Glu Tyr Gly Thr Glu Asp Gly Asp Gln Ser Val Thr Tyr Asp Asp
        275             280             285

Gly Thr His Lys Leu Gln Met Met Asn Glu Leu Ser Ala Val Phe Phe
        290             295             300

Arg Glu Phe Ala Gly Gln Thr Ser Arg Gly Lys Arg Ile Pro Gly Ser
305             310             315             320

Val Phe Asn Leu Pro Glu Asp Leu Gln Gln Leu Phe Val Asp Val Leu
        325             330             335

Val Glu Gly Asp Gly Ser Arg Glu Phe Pro Arg Tyr Ser Thr Glu Tyr
        340             345             350

Xaa Glu Arg Asn Phe Asp Phe Glu Thr Thr Ser Arg Glu Leu Ala Ala
        355             360             365

Gly Leu Ser Thr Leu Leu Thr Gln Arg Gly Glu Lys His Ser Leu Lys
        370             375             380

Tyr Arg Glu Ser Lys Gly Ser Tyr Thr Ile Arg Thr Xaa Asp Tyr Tyr
385             390             395             400

Arg Ser Gly Arg Asp Pro Val Val Glu Glu Val Asp His Asp Gly Tyr
            405             410             415

Val Tyr Asp Leu Ser Val Ala Glu Asn Glu Asn Phe Val Asp Gly Val
        420             425             430

Gly Gly Val Val Leu His Asn
        435
```

```
<210>  179
<211>  347
<212>  PRT
<213>  Artificial sequence

<220>
<223>  modified contiguous intein from Cafeteria roenbergensis virus
       BV-PW1


<220>
<221>  misc_feature
<222>  (40)..(40)
<223>  Xaa can be any naturally occurring amino acid
```

```
<220>
<221>  misc_feature
<222>  (136)..(136)
<223>  Xaa can be any naturally occurring amino acid

<220>
<221>  misc_feature
<222>  (145)..(145)
<223>  Xaa can be any naturally occurring amino acid

<220>
<221>  misc_feature
<222>  (152)..(152)
<223>  Xaa can be any naturally occurring amino acid

<220>
<221>  misc_feature
<222>  (158)..(158)
<223>  Xaa can be any naturally occurring amino acid

<220>
<221>  misc_feature
<222>  (176)..(176)
<223>  Xaa can be any naturally occurring amino acid

<220>
<221>  misc_feature
<222>  (231)..(231)
<223>  Xaa can be any naturally occurring amino acid

<220>
<221>  misc_feature
<222>  (263)..(263)
<223>  Xaa can be any naturally occurring amino acid

<400>  179
```

Ser Val Ile Gly Asp Thr Pro Leu Leu Leu Lys Asn Lys Phe Thr Asn
1               5                   10                  15

Glu Ile Leu Ile Asn Lys Ile Lys Asp Leu Ser Ser Asn Trp Ser Asn
            20                  25                  30

Tyr His Asn Gly Lys Glu Ser Xaa Glu Ile Asp Thr Tyr Gln Thr Trp
        35                  40                  45

Thr Glu Thr Gly Trp Thr Asp Ile Lys Arg Val Ile Arg His Lys Leu
        50                  55                  60

Glu Ser Asn Lys Lys Leu Leu Lys Ile Gln Thr His Asn Gly Glu Val
65                  70                  75                  80

Ile Val Thr Asp Glu His Ser Leu Leu Asn Lys Asn Gly Lys Thr Ile
                85                  90                  95

Asn Ala Lys Asn Val Lys Val Gly Asp Asn Ile Leu His Ser Phe Pro
            100                 105                 110

```
Ser Tyr Ile Asn Asn Ile Asp Asn Thr Asn Ser Ile Asn Tyr His Asn
        115                 120             125

Lys Phe Tyr Asn Lys Lys Met Xaa Asn Glu Leu Ala Tyr Ile Leu Gly
        130                 135             140

Xaa Phe Met Lys Tyr Gly Leu Xaa Asp Ser Ser Lys Lys Xaa Phe Thr
145                 150             155                 160

Ile Asn Asn Lys Asp Ile Asn Leu Ile Glu Ser Leu Lys Lys Met Xaa
                165             170                 175

Glu Asn Ile Phe Asp Glu Phe Lys Trp Lys Ile Ser Ser Ser Ser His
            180             185             190

Leu Ser Asp Asn Ile Tyr Lys Leu Val Pro Phe Gln Asn Glu Ile Lys
        195             200             205

Leu Ile Asp Phe Ile Lys Tyr Phe Thr Asn Lys Met Tyr Asn Asn Gly
    210             215             220

Glu Lys Lys Val Pro Gln Xaa Ile Leu Asn Ser Ser Lys Glu Tyr Ile
225             230             235                 240

Lys Ile Phe Leu Ile Gly Leu Tyr Pro Glu Tyr Lys Leu Glu Asn Asn
            245             250             255

Gln Gln Phe Ile Tyr Thr Xaa Lys Asn Asn Glu Phe Ser Leu Gly Ile
            260             265             270

Tyr Tyr Leu Ile Lys Lys Leu Gly Tyr His Val Lys Leu Asn Ser Asn
        275             280             285

Asp Ser Ser Asp Ser Ser Asp Ser Ile Tyr Thr Phe Glu Ile Ser His
        290             295             300

Lys Leu Glu Asn Asn Asn Asn Val Ile Thr Lys Ile Thr Glu Trp Glu
305             310             315             320

His Lys Glu Thr Tyr Val Tyr Asp Leu Thr Thr Glu Asn His His Phe
            325             330             335

His Ala Gly Val Gly Ser Ile Ile Val His Asn
        340             345
```

<210> 180

<211> 257
<212> PRT
<213> Artificial sequence

<220>
<223> modified contiguous intein from unknown phycodnavirus KBvp-2


<220>
<221> misc_feature
<222> (164)..(164)
<223> Xaa can be any naturally occurring amino acid

<220>
<221> misc_feature
<222> (168)..(168)
<223> Xaa can be any naturally occurring amino acid

<220>
<221> misc_feature
<222> (211)..(211)
<223> Xaa can be any naturally occurring amino acid

<400> 180

Ser Val Lys Gly Asp Thr Pro Leu Leu Leu Lys Thr Glu His Gly Val
1               5                   10                  15

Phe Phe Gln Ser Ile Asp Glu Leu Phe Lys Ile Ser Lys Ser Ile Glu
            20                  25                  30

Thr Gly Leu Arg Leu Ala Lys Glu Tyr Ala Asn Ile Glu Asn His Asn
        35                  40                  45

Ile Tyr Val Trp Ser Asp Val Gly Phe Thr Lys Ile Arg Arg Val Met
        50                  55                  60

Arg His Tyr Thr Thr Lys Gly Met Phe Arg Val Thr Thr Lys Thr Gly
65                  70                  75                  80

Tyr Val Asp Val Thr Glu Asp His Ser Leu Leu Leu Glu Asn Gly Phe
                85                  90                  95

Glu Val Arg Pro Ser Asp Thr Thr Val Gly Thr Arg Leu Leu His Lys
            100                 105                 110

Lys Pro Thr Ile Asn Lys Tyr Ile Gln Lys Ser Phe Ser Ser Glu His
            115                 120                 125

Leu Ser Glu Ala Lys Met Met Gly Glu Ser Phe Leu Asp Glu Glu Thr
        130                 135                 140

Ile Pro Ser Phe Val Leu Asn Ser Pro Val Asn Val Leu Arg Lys Tyr
145                 150                 155                 160

Phe Glu Gly Xaa Ile Lys Ala Xaa Gly Val Ile Lys Asn Asp Thr Asn
                165                 170             175

Ile Glu Phe His Phe Ser Lys Ser Lys Gln Gly Val Ala Glu Phe Val
            180                 185                 190

Phe Val Ala Gln Gln Leu Gly Tyr His Val Phe Ile Lys Pro Tyr Gly
        195                 200                 205

Val His Xaa Ser Leu Asp Pro Gln Asp Leu Lys Ile Gln Glu Ile Thr
    210             215                 220

Ala Ile Glu Tyr Met Gly Lys Thr Lys Asp Tyr Val Tyr Asp Leu Glu
225             230                 235                 240

Thr Asp Asn His His Phe His Val Gly Pro Gly Asn Leu Ile Val His
            245             250                 255

Asn

<210> 181
<211> 232
<212> PRT
<213> Artificial sequence

<220>
<223> modified contiguous intein from Heterosigma akashiwo virus HaV01

<220>
<221> misc_feature
<222> (16)..(16)
<223> Xaa can be any naturally occurring amino acid

<220>
<221> misc_feature
<222> (109)..(109)
<223> Xaa can be any naturally occurring amino acid

<220>
<221> misc_feature
<222> (142)..(142)
<223> Xaa can be any naturally occurring amino acid

<220>
<221> misc_feature
<222> (171)..(171)
<223> Xaa can be any naturally occurring amino acid

<400> 181

Ser Val Thr Lys Glu Thr Pro Leu Met Leu Arg Thr Met Glu Thr Xaa
1               5                   10                  15

```
Gly Asn His Lys His Glu Val Ile Ser Ile Glu Asn Val Phe Thr Asp
            20                  25                  30

Asn Met Arg Ser Ile Asp Met Tyr Ser Ile Ile Gly Glu Lys Glu His
            35                  40                  45

Val Met Leu Ser Arg Asn Glu Glu Ile Trp Thr Gly Glu Asn Trp Ser
    50                  55                  60

Arg Ile Ile Arg Val Ile Arg His Lys Thr Gln Lys Lys Ile Tyr Gly
65                  70                  75                  80

Val Leu Thr Glu Asn Gly Tyr Val Glu Val Thr Glu Asp His Ser Leu
                85                  90                  95

Ile Ser Ser Asp Tyr Glu Leu Leu Lys Pro Lys Asn Xaa Ile Val Lys
            100                 105                 110

Glu Thr Gln Leu Leu Gln Ser Phe Pro Asp Ile Val Glu Asn Ser Thr
            115                 120                 125

Ile Glu Asn Asn Met Ile Asp Ile Pro Lys Gly Gln Pro Xaa Arg Leu
    130                 135                 140

Thr Val Phe Gly Gln Val Ser Ala Met Ile Ile Tyr Thr Tyr Leu Lys
145                 150                 155                 160

Arg Lys Asn Tyr Ser Ile Thr Leu Asn Val Xaa Asn Val Asn Ser Asn
                165                 170                 175

Lys Phe Tyr Ile Ser Phe Met Glu Arg Pro Arg Phe Lys Asn Thr Lys
            180                 185                 190

Lys Asn Ile Ile Lys Lys Ile Phe Phe Ile Arg Asn Thr Asp Asn Glu
            195                 200                 205

Glu Tyr Val Tyr Asp Val Glu Thr Glu Asp Gly Ile Phe His Ala Gly
    210                 215                 220

Ile Gly Glu Ile Ile Val Lys Asn
225                 230

<210>   182
<211>   386
<212>   PRT
<213>   Artificial sequence
```

<220>
<223>    modified contiguous intein from Archaeon GW2011_AR16

<220>
<221>    misc_feature
<222>    (204)..(204)
<223>    Xaa can be any naturally occurring amino acid

<220>
<221>    misc_feature
<222>    (239)..(239)
<223>    Xaa can be any naturally occurring amino acid

<400>    182

Ser Val Gly Lys Asp Thr Glu Ile Val Met Asn Glu Asn Gly Thr Ile
1               5               10              15

Arg Phe Val Lys Ile Ser Glu Leu Phe Glu Arg Thr Gln Lys Arg Thr
            20              25              30

Ser Asp Gly Lys Glu Tyr Phe Phe Pro Pro Ser Arg Leu Val Leu Thr
            35              40              45

Leu Asp Ala Gln Gly Lys Ser Val Phe Lys Lys Val Lys Tyr Val Met
        50              55              60

Lys His Arg Val Gln Lys Lys Met Tyr Arg Ile Phe Phe Thr Asn Asp
65              70              75              80

His Tyr Ile Asp Val Thr Glu Asp His Ser Leu Ile Gly Tyr Val Asn
            85              90              95

Lys Gln Lys Asn Asn Gln Leu Ala Asp Leu Asp Arg Leu Ile Glu Val
            100             105             110

Lys Pro Thr Asp Ile Gly Lys Arg Val Arg Thr Ile Ile Thr Ile Lys
            115             120             125

Asn Ile Pro Arg Ser Ser Ile Lys Thr Arg Asn Tyr His Arg Glu Leu
            130             135             140

Tyr Glu Phe Met Gly Leu Phe Ile Gly Asp Gly Ser Phe Asp Arg Gln
145             150             155             160

Lys Lys Gln Asn Tyr Tyr Leu His Leu Ala Gly Gly Leu Asp Ser Trp
            165             170             175

Glu Ile Ile Thr Lys Val Leu Val Pro Leu Lys Glu Lys Glu Tyr Ile
            180             185             190

```
    Lys Asn Tyr Trp Leu Lys Lys Lys Gly Asp Ile Xaa Ile Asn Gly Leu
            195             200             205

    Arg Leu Val Arg Leu Phe Asn Asp Glu Phe Arg Lys Glu Ser Lys Lys
            210             215             220

    Ser Ile Pro Ala Phe Leu Leu Arg Glu Lys Gln Glu Ala Ile Xaa Ser
    225             230             235             240

    Phe Leu Arg Gly Leu Phe Ser Ala Asp Gly Ser Val Leu Phe Arg Asn
                    245             250             255

    Lys Lys Pro Ile Ile Lys Phe Thr Asn Thr Asn Thr Glu Ile Ile Lys
                260             265             270

    Met Thr Ser Arg Leu Leu His Leu Val Gly Ile Ser His Ser Thr Phe
            275             280             285

    Ser Glu Thr Arg Lys Asn Arg Tyr Lys Gly Lys Glu Ser Glu Thr Ile
            290             295             300

    Ser Lys His Ile Tyr Ile Lys Asp Ala Leu Ser Phe Arg Glu Lys Val
    305             310             315             320

    Gly Phe Val Ile Asn Arg Lys Gln Glu Arg Leu Ser Leu Val Ser Lys
                    325             330             335

    Asn Ser Thr His Arg Arg Thr Ile Lys Asn Tyr Asp Phe Asp Leu Ser
                340             345             350

    Lys Val Ile Lys Ile Glu Pro Ile Glu Tyr Arg Gly Asp Val Tyr Asp
            355             360             365

    Leu Glu Ile Glu Asp Thr His Arg Phe Phe Ala Asn Asn Val Leu Val
            370             375             380

    His Asn
    385


    <210>  183
    <211>  184
    <212>  PRT
    <213>  Artificial sequence

    <220>
    <223>  modified contiguous intein from Methanomethylicus oleusabulum
           isolate V3
```

```
<220>
<221>  misc_feature
<222>  (38)..(38)
<223>  Xaa can be any naturally occurring amino acid

<220>
<221>  misc_feature
<222>  (67)..(67)
<223>  Xaa can be any naturally occurring amino acid

<220>
<221>  misc_feature
<222>  (84)..(84)
<223>  Xaa can be any naturally occurring amino acid

<220>
<221>  misc_feature
<222>  (93)..(93)
<223>  Xaa can be any naturally occurring amino acid

<220>
<221>  misc_feature
<222>  (143)..(143)
<223>  Xaa can be any naturally occurring amino acid

<220>
<221>  misc_feature
<222>  (160)..(160)
<223>  Xaa can be any naturally occurring amino acid

<400>  183
```

Ser Val Ala Gly Asp Ser Ile Val Ala Val Asp Asp Gly Lys Gly Arg
1               5               10              15

Ser Glu Val Arg Ile Asp Gly Leu Phe Lys Gly Thr Ser Ala Lys Ala
            20              25              30

Gly Glu Lys Glu Tyr Xaa Arg Pro Arg Ser Leu Arg Thr Ile Ser Leu
        35              40              45

Gly Pro Asp Gly Arg Val Thr Trp Ser Arg Ile Asn Ala Ile Met Arg
    50              55              60

His Arg Xaa Gly Lys Arg Met Phe Arg Val Trp Leu Ser Asp Ser Trp
65              70              75              80

His Val Asp Xaa Thr Glu Asp His Ser Leu Ile Gly Xaa Leu Val Asp
            85              90              95

Gly Asp Gly Lys Val Asp Gly Glu Leu Pro Ala Ala Gly Leu Arg Leu
        100             105             110

Val Asn Leu Lys Pro Thr Glu Ile Gly Lys Val Ala Asn Arg Leu Val
        115             120             125

```
Ala Leu Asp Ala Gln Pro His Ser Asn Gly Gly Ser Ala Gly Xaa Gly
    130                 135                 140

Ala Gly Ile Arg Thr Val Thr Pro Val Arg Val Glu Glu Ile Pro Xaa
    145                 150                 155                 160

Asp Gly Tyr Val Tyr Asp Met Glu Val Asp Gly Thr His Arg Phe Phe
                    165                 170                 175

Ala Asn Arg Val Leu Val His Asn
                    180
```

```
<210> 184
<211> 184
<212> PRT
<213> Artificial sequence

<220>
<223> modified contiguous intein from Verstraetearchaeota species
      UBA156

<220>
<221> misc_feature
<222> (38)..(38)
<223> Xaa can be any naturally occurring amino acid

<220>
<221> misc_feature
<222> (67)..(67)
<223> Xaa can be any naturally occurring amino acid

<220>
<221> misc_feature
<222> (84)..(84)
<223> Xaa can be any naturally occurring amino acid

<220>
<221> misc_feature
<222> (93)..(93)
<223> Xaa can be any naturally occurring amino acid

<220>
<221> misc_feature
<222> (143)..(143)
<223> Xaa can be any naturally occurring amino acid

<220>
<221> misc_feature
<222> (160)..(160)
<223> Xaa can be any naturally occurring amino acid

<400> 184

Ser Val Ala Gly Asp Ser Ile Val Ala Val Asp Asp Gly Lys Gly Arg
1               5                   10                  15
```

Ser Glu Val Arg Ile Asp Gly Leu Phe Lys Gly Thr Ser Ala Lys Ala
                20                  25                  30

Gly Glu Lys Glu Tyr Xaa Arg Pro Arg Ser Leu Arg Thr Ile Ser Leu
        35                  40                  45

Gly Pro Asp Gly Arg Val Thr Trp Ser Arg Ile Asn Ala Ile Met Arg
        50                  55                  60

His Arg Xaa Gly Lys Arg Met Phe Arg Val Trp Leu Ser Asp Ser Trp
65                  70                  75                  80

His Val Asp Xaa Thr Glu Asp His Ser Leu Ile Gly Xaa Leu Val Asp
                85                  90                  95

Gly Asp Gly Lys Val Asp Gly Glu Leu Pro Ala Ala Gly Leu Arg Leu
            100                 105                 110

Val Asn Leu Lys Pro Thr Glu Ile Gly Lys Val Ala Asn Arg Leu Val
        115                 120                 125

Ala Leu Asp Ala Gln Pro His Ser Asn Gly Gly Ser Ala Gly Xaa Gly
        130                 135                 140

Ala Gly Ile Arg Thr Val Thr Pro Val Arg Val Glu Glu Ile Pro Xaa
145                 150                 155                 160

Asp Gly Tyr Val Tyr Asp Met Glu Val Asp Gly Thr His Arg Phe Phe
                165                 170                 175

Ala Asn Arg Val Leu Val His Asn
                180

<210> 185
<211> 179
<212> PRT
<213> Artificial sequence

<220>
<223> modified contiguous intein from Verstraetearchaeota species UBA76

<220>
<221> misc_feature
<222> (38)..(38)
<223> Xaa can be any naturally occurring amino acid

<400> 185

Ser Val Ala Gly Ser Ser Val Val Ser Val Asp Ala Gly Gly Lys Lys
1               5                   10                  15

Ser Asp Val Pro Val Glu Ser Leu Phe Gly Arg Pro Asp Gln Ser Val
20 25 30

Gly Gly Lys Glu Tyr Xaa Tyr Pro Ala Ser Leu Arg Ala Leu Ser Leu
35 40 45

Asp Pro Gly Gly Arg Ala Val Tyr Ser Arg Val Asn Ala Ile Met Arg
50 55 60

His Arg Ser Gly Lys Lys Met Phe Arg Val Arg Leu Ala Asp Ser Trp
65 70 75 80

His Ile Asp Val Thr Glu Asp His Ser Leu Ile Gly Tyr Arg Asp Gly
85 90 95

Gly Gly Ser Gly Ser Ser Gly Ile Asp Gly His Leu Val Asp Val Arg
100 105 110

Pro Ala Glu Ile Gly Lys Ala Val Lys Arg Leu Val Val Leu Lys Lys
115 120 125

Gly Pro Leu Val Ala Gly Arg Arg Ala Pro Ser Ala Asp Phe Asp Thr
130 135 140

Ala Ala Pro Leu Arg Ile Glu Glu Val Pro Tyr Asp Gly Tyr Val Tyr
145 150 155 160

Asp Leu Glu Ile Glu Gly Thr Arg Arg Phe Phe Ala Asn Gly Val Leu
165 170 175

Val His Asn

<210> 186
<211> 179
<212> PRT
<213> Artificial sequence

<220>
<223> modified contiguous intein from Methanomethylicus mesodigestum isolate V2

<220>
<221> misc_feature
<222> (38)..(38)
<223> Xaa can be any naturally occurring amino acid

<400> 186

Ser Val Ala Gly Ser Ser Val Val Ser Val Asp Ala Gly Gly Lys Lys

```
1                  5                      10                     15


Ser Asp Val Pro Val Glu Ser Leu Phe Gly Arg Pro Asp Gln Ser Val
            20              25              30


Gly Gly Lys Glu Tyr Xaa Tyr Pro Ala Ser Leu Arg Ala Leu Ser Leu
            35              40              45


Asp Pro Gly Gly Arg Ala Val Tyr Ser Arg Val Asn Ala Ile Met Arg
        50              55              60


His Arg Ser Gly Lys Lys Met Phe Arg Val Arg Leu Ala Asp Ser Trp
65                  70              75                  80


His Ile Asp Val Thr Glu Asp His Ser Leu Ile Gly Tyr Arg Asp Gly
                85              90              95


Gly Gly Ser Gly Ser Ser Gly Ile Asp Gly His Leu Val Asp Val Arg
            100             105             110


Pro Ala Glu Ile Gly Lys Ala Val Lys Arg Leu Val Val Leu Lys Lys
            115             120             125


Gly Pro Leu Val Ala Gly Arg Arg Ala Pro Ser Ala Asp Phe Asp Thr
            130             135             140


Ala Ala Pro Leu Arg Ile Glu Glu Val Pro Tyr Asp Gly Tyr Val Tyr
145             150             155                 160


Asp Leu Glu Ile Glu Gly Thr Arg Arg Phe Phe Ala Asn Gly Val Leu
                165             170             175


Val His Asn
```

```
<210>  187
<211>  351
<212>  PRT
<213>  Artificial sequence

<220>
<223>  modified contiguous intein from Mimivirus terra2


<220>
<221>  misc_feature
<222>  (88)..(88)
<223>  Xaa can be any naturally occurring amino acid

<220>
<221>  misc_feature
```

```
<222>   (110)..(110)
<223>   Xaa can be any naturally occurring amino acid

<220>
<221>   misc_feature
<222>   (147)..(147)
<223>   Xaa can be any naturally occurring amino acid

<220>
<221>   misc_feature
<222>   (173)..(173)
<223>   Xaa can be any naturally occurring amino acid

<220>
<221>   misc_feature
<222>   (259)..(259)
<223>   Xaa can be any naturally occurring amino acid

<220>
<221>   misc_feature
<222>   (262)..(262)
<223>   Xaa can be any naturally occurring amino acid

<220>
<221>   misc_feature
<222>   (272)..(272)
<223>   Xaa can be any naturally occurring amino acid

<400>   187
```

Ser Val Thr Gly Asp Thr Pro Ile Ile Thr Arg His Gln Asn Gly Asp
1               5                   10                  15

Ile Asn Ile Thr Thr Ile Glu Glu Leu Gly Ser Lys Trp Lys Pro Tyr
            20                  25                  30

Glu Ile Phe Lys Ala His Glu Lys Asn Ser Asn Arg Lys Phe Lys Gln
        35                  40                  45

Gln Ser Gln Tyr Pro Thr Asp Ser Glu Val Trp Thr Ala Lys Gly Trp
    50                  55                  60

Ala Lys Ile Lys Arg Val Ile Arg His Lys Thr Val Lys Lys Ile Tyr
65                  70                  75                  80

Arg Val Leu Thr His Thr Gly Xaa Ile Asp Val Thr Glu Asp His Ser
            85                  90                  95

Leu Leu Asp Pro Asn Gln Asn Ile Ile Lys Pro Ile Asn Xaa Gln Ile
            100                 105                 110

Gly Thr Glu Leu Leu His Gly Phe Pro Glu Ser Asn Asn Val Tyr Asp
            115                 120                 125

Asn Ile Ser Glu Gln Glu Ala Tyr Val Trp Gly Phe Phe Met Gly Asp

226

```
                130                    135                    140


Gly Ser Xaa Gly Ser Tyr Gln Thr Lys Asn Gly Ile Lys Tyr Ser Trp
145                 150                 155                 160


Ala Leu Asn Asn Gln Asp Leu Asp Val Leu Asn Lys Xaa Lys Lys Tyr
                165                 170                 175


Leu Glu Glu Thr Glu Asn Ile Gln Phe Lys Ile Leu Asp Thr Met Lys
            180                 185                 190


Ser Ser Ser Val Tyr Lys Leu Val Pro Ile Gly Lys Ile Lys Tyr Met
        195                 200                 205


Val Asn Lys Tyr Arg Lys Ile Phe Tyr Asp Asn Lys Lys Tyr Lys Leu
    210                 215                 220


Val Pro Lys Glu Ile Leu Asn Ser Thr Lys Asp Ile Lys Asn Ser Phe
225                 230                 235                 240


Leu Glu Gly Tyr Tyr Ala Ala Asp Gly Ser Arg Lys Glu Thr Glu Asn
                245                 250                 255


Met Gly Xaa Arg Arg Xaa Asp Val Lys Gly Lys Ile Ser Ala Gln Xaa
            260                 265                 270


Leu Phe Tyr Leu Leu Lys Ser Leu Gly Tyr Asn Val Ser Ile Asn Ile
        275                 280                 285


Arg Ser Asp Lys Asn Gln Ile Tyr Arg Leu Thr Phe Ser Asn Lys Lys
    290                 295                 300


Gln Arg Lys Asn Pro Ile Ala Ile Lys Lys Ile Gln Leu Met Asn Glu
305                 310                 315                 320


Thr Ser Asn Asp His Asp Gly Asp Tyr Val Tyr Asp Leu Glu Thr Glu
                325                 330                 335


Ser Gly Ser Phe His Ala Gly Val Gly Glu Met Ile Val Lys Asn
            340                 345                 350


<210>   188
<211>   86
<212>   PRT
<213>   Artificial sequence

<220>
<223>   modified contiguous intein (partial) from activated-carbon
        dual-media filters Ann Arbor (MI, USA) drinking water treatment
```

plant metagenome

<220>
<221> misc_feature
<222> (20)..(20)
<223> Xaa can be any naturally occurring amino acid

<220>
<221> misc_feature
<222> (25)..(25)
<223> Xaa can be any naturally occurring amino acid

<220>
<221> misc_feature
<222> (60)..(60)
<223> Xaa can be any naturally occurring amino acid

<220>
<221> misc_feature
<222> (76)..(76)
<223> Xaa can be any naturally occurring amino acid

<220>
<221> misc_feature
<222> (86)..(86)
<223> Xaa can be any naturally occurring amino acid

<400> 188

Ser Val Ala Ala Asp Thr Pro Ile Leu Val Lys Arg Asn Asp Gln Ile
1               5               10              15

Glu Trp Ile Xaa Ile Arg Asp Leu Xaa Gln His Glu Gln Asp Pro Asp
            20              25              30

Lys Lys Thr Glu Leu Asn Thr Glu His Phe Asn Tyr Glu Val Trp Ser
        35              40              45

Asp Ile Gly Trp Thr Lys Ile Lys Arg Leu Ile Xaa His Lys Thr Thr
    50              55              60

Lys Gln Met Tyr Arg Val Leu Thr His Thr Gly Xaa Val Asp Val Thr
65              70              75              80

Glu Asp His Ser Leu Xaa
                85

<210> 189
<211> 282
<212> PRT
<213> Artificial sequence

<220>
<223> modified contiguous intein from Megavirus LBA111

```
<220>
<221>  misc_feature
<222>  (86)..(86)
<223>  Xaa can be any naturally occurring amino acid

<220>
<221>  misc_feature
<222>  (108)..(108)
<223>  Xaa can be any naturally occurring amino acid

<220>
<221>  misc_feature
<222>  (145)..(145)
<223>  Xaa can be any naturally occurring amino acid

<220>
<221>  misc_feature
<222>  (181)..(181)
<223>  Xaa can be any naturally occurring amino acid

<220>
<221>  misc_feature
<222>  (213)..(213)
<223>  Xaa can be any naturally occurring amino acid

<220>
<221>  misc_feature
<222>  (217)..(217)
<223>  Xaa can be any naturally occurring amino acid

<220>
<221>  misc_feature
<222>  (234)..(234)
<223>  Xaa can be any naturally occurring amino acid

<220>
<221>  misc_feature
<222>  (244)..(244)
<223>  Xaa can be any naturally occurring amino acid

<400>  189
```

Ser Val Thr Gly Asp Thr Pro Ile Met Ile Lys Asp Lys Asn Asn Asn
1               5                   10                  15

Ile Asn Ile Val Thr Ile Lys Glu Leu Gly Glu Lys Trp Lys Pro Tyr
            20                  25                  30

Asp Ile Phe Lys Ser His Glu Ile Asn Ser Asn Arg Lys Tyr Lys Gln
            35                  40                  45

Gln Ala Asp Phe Asn Gly Glu Val Trp Thr Ser Asn Gly Trp Ala Lys
            50                  55                  60

Ile Lys Arg Val Ile Arg His Lys Thr Val Lys Lys Leu Tyr Arg Val
65                  70                  75                  80

Leu Thr Asn Thr Gly Xaa Ile Asp Val Thr Glu Asp His Ser Leu Leu

|  |  |  |  | 85 |  |  |  |  | 90 |  |  |  |  | 95 |  |
|---|---|---|---|---|---|---|---|---|---|---|---|---|---|---|---|

```
        Asp Thr Asn Lys Asn Ile Ile Lys Pro Ile Asp Xaa Lys Ile Gly Thr
                    100                 105                 110

        Glu Leu Leu His Gly Phe Pro Glu Ile Asn Asn Asn His Asn Lys Leu
                    115                 120                 125

        Ser Leu Glu Ile Tyr Lys Glu Leu Glu Ile Thr Arg Met Leu Phe Asp
                    130                 135                 140

        Xaa Met Ile Glu Ser Asn Lys Lys Trp Asn Asn Glu Lys Met Lys Ala
        145                 150                 155                 160

        Tyr Phe Ile Gly Ser Glu Tyr Arg Lys Gln Asn Lys Asn Ile Ser Asn
                    165                 170                 175

        Glu Ile Leu Asn Xaa Ser Lys Lys Ile Lys Lys Tyr Phe Leu Leu Gly
                    180                 185                 190

        Tyr Leu Gly Asn Asp Lys Glu Tyr Ile Thr Asn Asn Lys Ile Asn Ala
                    195                 200                 205

        Gln Ile Ile Tyr Xaa Leu Met Lys Xaa Leu Glu Tyr Asn Ile Val Ile
                    210                 215                 220

        Asp Leu Ile Glu Ser Ser Tyr Lys Leu Xaa Ile Ile Asp Asn Ile Asn
        225                 230                 235                 240

        Glu Pro Tyr Xaa Ile Asn Lys Ile Ile Gln Leu Gln Asp Thr Ser Ile
                    245                 250                 255

        Asn Gly Glu Tyr Val Tyr Asp Leu Glu Thr Glu Ser Gly Thr Phe His
                    260                 265                 270

        Ala Gly Ile Gly Glu Leu Ile Val Lys Asn
                    275                 280
```

```
        <210>   190
        <211>   348
        <212>   PRT
        <213>   Artificial sequence

        <220>
        <223>   modified contiguous intein from Picocystis salinarum CCMP1897


        <220>
        <221>   misc_feature
        <222>   (17)..(17)
```

<223> Xaa can be any naturally occurring amino acid

<220>
<221> misc_feature
<222> (73)..(73)
<223> Xaa can be any naturally occurring amino acid

<220>
<221> misc_feature
<222> (148)..(148)
<223> Xaa can be any naturally occurring amino acid

<220>
<221> misc_feature
<222> (153)..(153)
<223> Xaa can be any naturally occurring amino acid

<220>
<221> misc_feature
<222> (217)..(217)
<223> Xaa can be any naturally occurring amino acid

<220>
<221> misc_feature
<222> (251)..(251)
<223> Xaa can be any naturally occurring amino acid

<220>
<221> misc_feature
<222> (260)..(260)
<223> Xaa can be any naturally occurring amino acid

<220>
<221> misc_feature
<222> (322)..(322)
<223> Xaa can be any naturally occurring amino acid

<400> 190

Ser Val Met Pro Tyr Thr Pro Val Leu Val Arg Asn Lys Arg Thr Gln
1               5               10              15

Xaa Ile Ser Ala Val Ala Ile Lys His Leu Ala Gln Asp Trp Met Pro
            20              25              30

Tyr Glu Ala Phe His Glu Gly Asp Pro Arg Arg Phe Glu Lys Glu Gln
        35              40              45

Gly Asn Ala Ala His Met Gln Ala Trp Thr Asp Gln Gly Trp Ala Asp
    50              55              60

Val Leu Arg Val Val Arg His Lys Xaa Ser Lys Lys Ile Tyr Arg Val
65              70              75              80

Val Thr His Thr Gly Leu Val Asp Val Thr Glu Asp His Ser Leu Leu
            85              90              95

Leu Pro Asp Arg Arg Lys Val Lys Pro His Gln Leu Glu Val Gly Thr
        100             105             110

Ala Leu Leu His Ser Phe Pro Gly Leu Glu Leu Trp Pro Asp Arg Leu
        115             120             125

Glu Ala Gly Thr Pro Glu Gln Ala Tyr Met Tyr Gly Val Phe Val Gly
        130             135             140

Asn Gly Ser Xaa Ala Lys Tyr Asp Xaa Pro Ser Gly Arg Lys Tyr Tyr
145             150             155             160

Trp Ala Ile Lys Asn Ser Asp Ile Thr Leu Ile Asn Lys Trp Lys Thr
            165             170             175

Val Leu Glu Met Ile His Lys Arg Pro Phe Lys Ile Val Asp Thr Leu
            180             185             190

Lys His Ser Gly Asp Tyr Lys Leu Val Pro Thr Asp Ser Ser Lys Asp
            195             200             205

Leu Val Ile Leu Tyr Trp Gln Ser Xaa Tyr Asp Asn Thr Gly Ala Lys
        210             215             220

Val Val Pro Tyr Glu Ile Leu Asn Gly Gln Ile Asp His Val Asp Ala
225             230             235             240

Phe Ile Glu Gly Leu Ser Val Ala Asp Gly Xaa Arg Arg Asp Leu Asp
            245             250             255

Thr Thr Gly Xaa Arg Arg Ile Asp Thr Lys Ser Gln Ile Ser Ala Gln
        260             265             270

His Tyr Tyr Val Leu Leu Lys Arg Leu Gly Tyr Arg Val Ser Ile Asn
        275             280             285

Ala Arg Asp Asp Lys Thr Asn Met Phe Arg Leu Thr Trp Thr Met Gly
        290             295             300

Arg Gln Arg Arg Glu Thr Thr Ile Ile Lys Lys Gln His Met Leu His
305             310             315             320

Glu Xaa Tyr Asp Gly Phe Val Tyr Asp Leu Glu Thr Ser Gln Gly Val
        325             330             335

Phe Gln Ala Gly Val Gly Glu Leu Ile Val Lys Asn
        340             345

<210> 191
<211> 144
<212> PRT
<213> Artificial sequence

<220>
<223> modified possibly N-part of a contiguous intein (partial) from bovine gut rumen metagenome

<220>
<221> misc_feature
<222> (49)..(49)
<223> Xaa can be any naturally occurring amino acid

<220>
<221> misc_feature
<222> (144)..(144)
<223> Xaa can be any naturally occurring amino acid

<400> 191

Ser Phe Thr Gly Asp Thr Pro Leu Phe Ile Lys Tyr Asp Asp Gly Asn
1               5                   10                  15

Ile Asp Ile Lys Pro Ile Glu Glu Leu Ile Gly Glu Thr Glu Thr Asp
                20                  25                  30

Ala Leu Gly Arg Glu Tyr Asp Tyr Ser Glu Lys Pro Tyr Ser Val Leu
            35                  40                  45

Xaa Arg Ser Gly Trp Val Lys Pro Lys Tyr Ile Tyr Arg His Lys Thr
    50                  55                  60

Asn Lys Gln Leu Tyr Thr Val Ser Glu Gly Asp Met Ser Ile Thr Val
65                  70                  75                  80

Thr Glu Asp His Ser Leu Phe Asn Asn Glu Lys Lys Lys Ile Lys Pro
                85                  90                  95

Ser Gln Ile Asn Ser Thr Thr Lys Leu Glu Tyr Tyr Thr Lys Asp Ile
            100                 105                 110

Lys Thr Ser Ser Asp Phe Lys Trp Leu Thr Lys Gln Arg Ala Lys Thr
            115                 120                 125

Met Ala Lys Met Ile Ile Asp Gly Thr Ile Asp Arg Val Ser Ile Xaa
            130                 135                 140

<210> 192
<211> 355
<212> PRT
<213> Artificial sequence

<220>
<223> modified continuous intein from sheep gut metagenome


<220>
<221> misc_feature
<222> (23)..(23)
<223> Xaa can be any naturally occurring amino acid

<220>
<221> misc_feature
<222> (47)..(47)
<223> Xaa can be any naturally occurring amino acid

<220>
<221> misc_feature
<222> (82)..(82)
<223> Xaa can be any naturally occurring amino acid

<220>
<221> misc_feature
<222> (142)..(142)
<223> Xaa can be any naturally occurring amino acid

<220>
<221> misc_feature
<222> (249)..(250)
<223> Xaa can be any naturally occurring amino acid

<220>
<221> misc_feature
<222> (261)..(261)
<223> Xaa can be any naturally occurring amino acid

<220>
<221> misc_feature
<222> (288)..(288)
<223> Xaa can be any naturally occurring amino acid

<220>
<221> misc_feature
<222> (325)..(325)
<223> Xaa can be any naturally occurring amino acid

<220>
<221> misc_feature
<222> (353)..(353)
<223> Xaa can be any naturally occurring amino acid

<400> 192

Ser Val Thr Tyr Asp Thr Pro Ile Leu Val Arg Gly Glu Asp Lys Arg
1               5                   10                  15


Ile Asp Ile Val Pro Ile Xaa Asp Ile Phe Asn Asn Asn Glu Ala Ile
            20                  25                  30


Glu Phe Gly Glu Glu Gln Tyr Arg Asp Phe Ser His Lys Asn Xaa Glu
            35                  40                  45

Val Leu Thr Arg Asn Gly Trp Lys Pro Ile Glu Tyr Val Tyr Lys His
    50              55              60

Lys Thr Asn Lys Thr Leu Lys Arg Val Glu Thr Lys Asn Gly Leu Ile
    65              70              75              80

Asp Xaa Thr Glu Asp His Ser Leu Phe Asp Asn Lys Arg Asn Glu Val
            85              90              95

Lys Pro Ser Thr Leu Asn Arg Gly Asp Lys Ile Glu Ile Tyr Thr Lys
            100             105             110

Asp Ile Asp Tyr Tyr Ala Ser Ser Thr Val Thr Asp Arg Glu Ala Trp
        115             120             125

Leu Phe Gly Phe Phe Met Ala Asp Gly Ser Ser Val Tyr Xaa Asp Arg
        130             135             140

Thr Gln Lys Tyr Tyr Ser Lys Arg Lys Gly Glu Trp Val Ile His Asn
145             150             155             160

Gly Lys Arg Ala Asn Trp Lys Ile Ser Asn Lys Ser Ile Asp Arg Leu
                165             170             175

Asn Lys Ala Lys Glu Ile Leu Glu Asp Ser Phe Phe Leu Lys Ala Ser
            180             185             190

Ile Lys Asp His Arg Thr Ser Ser Asn Val Tyr Asp Leu Val Val Glu
        195             200             205

Asn Ala Glu Asn Ala Lys Phe Phe Ser Asn Asn Phe Tyr Thr Ser Tyr
210             215             220

Arg Tyr Lys Lys Val Pro Glu Phe Ile Leu Asn Ala Lys Lys Glu Val
225             230             235             240

Lys Lys Ala Phe Leu Asp Gly Phe Xaa Xaa Gly Asp Gly Gln Asn Asp
            245             250             255

Thr Ile Asp Glu Xaa Ile Glu Phe Gly Gln Lys Ser Lys Val Ala Met
        260             265             270

Ala Gly Leu Tyr Leu Leu Met Lys Glu Leu Gly Tyr Asn Phe Arg Xaa
        275             280             285

His Asn Arg Asn Asp Lys Gln Glu Phe Ile Ser Phe Arg Leu Arg Asn
    290             295             300

**235**

```
His Arg Gly Asn Leu Leu Asn Glu Asn Tyr Ser Glu Arg Lys Glu Asp
305                 310                 315                 320


Glu Val Trp Asn Xaa Gly Asn Ile Thr Ser Lys Ser Glu Tyr Val Tyr
                325                 330                 335


Asp Ile Ser Ala Asp Gly Thr Phe Val Asn Ala Leu Gly Met Ile Val
                340                 345                 350


Xaa His Asn
        355
```

```
<210>  193
<211>  348
<212>  PRT
<213>  Artificial sequence

<220>
<223>  modified contiguous intein from bovine gut rumen metagenome


<220>
<221>  misc_feature
<222>  (23)..(23)
<223>  Xaa can be any naturally occurring amino acid


<220>
<221>  misc_feature
<222>  (82)..(82)
<223>  Xaa can be any naturally occurring amino acid


<220>
<221>  misc_feature
<222>  (142)..(142)
<223>  Xaa can be any naturally occurring amino acid


<220>
<221>  misc_feature
<222>  (181)..(181)
<223>  Xaa can be any naturally occurring amino acid


<220>
<221>  misc_feature
<222>  (242)..(243)
<223>  Xaa can be any naturally occurring amino acid


<220>
<221>  misc_feature
<222>  (254)..(254)
<223>  Xaa can be any naturally occurring amino acid


<220>
<221>  misc_feature
<222>  (281)..(281)
<223>  Xaa can be any naturally occurring amino acid


<220>
```

```
<221>  misc_feature
<222>  (318)..(318)
<223>  Xaa can be any naturally occurring amino acid

<220>
<221>  misc_feature
<222>  (346)..(346)
<223>  Xaa can be any naturally occurring amino acid

<400>  193
```

```
Ser Val Thr Tyr Asp Thr Pro Ile Leu Val Arg Gly Glu Asp Lys Arg
1               5                   10                  15

Ile Asn Ile Ile Pro Ile Xaa Asp Ile Phe Asn Asn Asn Glu Ala Ile
            20                  25                  30

Glu Phe Gly Glu Glu Gln Tyr Arg Asp Phe Ser Arg Lys Asn Tyr Glu
        35                  40                  45

Val Leu Thr Arg Asn Gly Trp Lys Pro Ile Glu Tyr Val Tyr Lys His
    50                  55                  60

Lys Thr Thr Lys Gln Leu Lys Arg Val Glu Thr Lys Asn Gly Val Ile
65                  70                  75                  80

Asp Xaa Thr Glu Asp His Ser Leu Phe Asp Asn Asn Gly Asn Glu Val
        85                  90                  95

Lys Pro Ser Thr Leu Val Arg Gly Asp Lys Ile Glu Ile Tyr Asn Asn
            100                 105                 110

Asp Ile Asp Tyr Phe Ala Ser Ser Thr Val Thr Asp Arg Glu Ala Trp
        115                 120                 125

Leu Phe Gly Phe Phe Met Ala Asp Gly Ser Ser Val Tyr Xaa Asp Arg
    130                 135                 140

Thr Gln Lys Tyr Phe Ser Lys Arg Met Gly Lys Arg Ala Asn Trp Lys
145                 150                 155                 160

Ile Ser Asn Lys Ser Leu Asp Arg Leu Asn Lys Ala Lys Glu Ile Met
            165                 170                 175

Glu Asn Ser Phe Xaa Leu Lys Ala Ser Ile Lys Asp His Arg Ala Ser
            180                 185                 190

Ser Asn Val Tyr Asn Leu Val Val Glu Asn Ala Glu Asn Ala Lys Phe
            195                 200                 205
```

Phe Ser Asn Asn Phe Tyr Thr Ser Tyr Arg Tyr Lys Lys Val Pro Glu
210 215 220

Phe Ile Leu Asn Ala Ser Lys Glu Val Lys Lys Ala Phe Leu Asp Gly
225 230 235 240

Phe Xaa Xaa Gly Asp Gly Gln Asn Asp Thr Ile Asp Glu Xaa Ile Glu
245 250 255

Phe Gly Gln Lys Ser Lys Val Ala Met Ala Gly Leu Tyr Phe Ile Met
260 265 270

Lys Glu Leu Gly Tyr Asn Phe Arg Xaa His Asn Arg Asn Asp Lys Pro
275 280 285

Glu Phe Ile Ser Phe Arg Leu Arg Asn His His Gly Asn Leu Leu Asn
290 295 300

Glu Asn Tyr Ser Glu Arg Lys Glu Asp Glu Ala Trp Leu Xaa Asn Asp
305 310 315 320

Ile Thr Ser Lys Ser Glu Tyr Val Tyr Asp Ile Ser Ala Asp Gly Thr
325 330 335

Phe Val Asn Ala Leu Gly Met Ile Val Xaa His Asn
340 345

<210> 194
<211> 355
<212> PRT
<213> Artificial sequence

<220>
<223> modified contiguous intein from sheep gut metagenome

<220>
<221> misc_feature
<222> (23)..(23)
<223> Xaa can be any naturally occurring amino acid

<220>
<221> misc_feature
<222> (82)..(82)
<223> Xaa can be any naturally occurring amino acid

<220>
<221> misc_feature
<222> (142)..(142)
<223> Xaa can be any naturally occurring amino acid

<220>
<221> misc_feature
<222> (249)..(250)

<223> Xaa can be any naturally occurring amino acid

<220>
<221> misc_feature
<222> (261)..(261)
<223> Xaa can be any naturally occurring amino acid

<220>
<221> misc_feature
<222> (288)..(288)
<223> Xaa can be any naturally occurring amino acid

<220>
<221> misc_feature
<222> (325)..(325)
<223> Xaa can be any naturally occurring amino acid

<220>
<221> misc_feature
<222> (353)..(353)
<223> Xaa can be any naturally occurring amino acid

<400> 194

Ser Val Thr Tyr Asp Thr Pro Ile Ile Val Arg Gly Glu Asp Lys Arg
1               5                   10                  15

Ile Asp Ile Ile Pro Ile Xaa Asp Ile Phe Asn Asn Asp Glu Ala Val
            20                  25                  30

Glu Phe Asp Asn Glu Gln Tyr Arg Asp Phe Ser Arg Lys Asn Tyr Asp
        35                  40                  45

Val Leu Thr Arg Asp Gly Trp Lys Pro Ile Glu Tyr Ile Tyr Lys His
    50                  55                  60

Lys Thr Asn Lys Gln Leu Lys Arg Val Glu Thr Lys Asn Gly Leu Val
65                  70                  75                  80

Asp Xaa Thr Glu Asp His Ser Leu Phe Asp Asn Asn Gly Asn Glu Val
            85                  90                  95

Lys Pro Ser Thr Leu Thr Arg Gly Asn Lys Ile Glu Ile Tyr Thr Lys
            100                 105                 110

Asp Ile Asp Tyr Phe Ala Ser Ser Thr Val Thr Asp Arg Glu Ala Trp
            115                 120                 125

Leu Phe Gly Phe Phe Met Ala Asp Gly Ser Ser Val Tyr Xaa Asp Arg
        130                 135                 140

Thr Gln Lys Tyr Tyr Ser Lys Arg Lys Gly Glu Trp Val Ile His Asn
145                 150                 155                 160

```
      Gly Lys Arg Ala Asn Trp Lys Ile Ser Asn Lys Ser Leu Asp Arg Leu
                      165             170             175

      Asn Lys Ala Lys Glu Ile Leu Glu Asp Ser Phe Phe Leu Lys Ala Ser
                      180             185             190

      Ile Lys Asp His Arg Ala Ser Ser Asn Val Tyr Asn Leu Val Val Glu
                      195             200             205

      Asn Thr Asp Asn Ala Lys Phe Phe Ser Asn Asn Phe Tyr Thr Ser Tyr
          210             215             220

      Arg Tyr Lys Lys Val Pro Glu Phe Ile Leu Asn Ala Arg Lys Glu Val
      225             230             235             240

      Lys Lys Ala Phe Leu Asp Gly Phe Xaa Xaa Gly Asp Gly Gln Asn Asp
                      245             250             255

      Thr Ile Asp Glu Xaa Ile Glu Phe Gly Gln Lys Ser Lys Val Ala Met
                      260             265             270

      Ala Gly Leu Tyr Phe Leu Met Lys Glu Leu Gly Tyr Asn Phe Arg Xaa
                      275             280             285

      His Asn Arg Asn Asp Lys Gln Glu Phe Ile Ser Phe Arg Leu Arg Asn
          290             295             300

      His Arg Gly Ser Leu Leu Asn Glu Asn Tyr Ser Glu Lys Lys Glu Asp
      305             310             315             320

      Glu Val Trp Asn Xaa Glu Asp Ile Thr Ser Lys Ser Glu Tyr Val Tyr
                      325             330             335

      Asp Ile Ser Ala Asp Gly Thr Phe Val Asn Ala Leu Gly Met Ile Val
                      340             345             350

      Xaa His Asn
              355
```

```
<210>  195
<211>  356
<212>  PRT
<213>  Artificial sequence

<220>
<223>  modified contiguous intein from Lake Huron low oxygen high sulfur
       sink hole purple microbial mat bin unclassified.01
```

```
<220>
<221>  misc_feature
<222>  (23)..(23)
<223>  Xaa can be any naturally occurring amino acid

<220>
<221>  misc_feature
<222>  (80)..(81)
<223>  Xaa can be any naturally occurring amino acid

<220>
<221>  misc_feature
<222>  (209)..(209)
<223>  Xaa can be any naturally occurring amino acid

<220>
<221>  misc_feature
<222>  (216)..(216)
<223>  Xaa can be any naturally occurring amino acid

<220>
<221>  misc_feature
<222>  (267)..(267)
<223>  Xaa can be any naturally occurring amino acid

<400>  195
```

Ser Val Glu Tyr Asp Thr Pro Ile Tyr Leu Lys Asp Lys Tyr Asp Asn
1               5                   10                  15

Leu Asn Ile Leu Pro Ile Xaa Asp Leu Phe Asp Asp Asn Ser Asn Phe
            20                  25                  30

Leu Ser Pro Asp Gly Leu Arg Asp Phe Ser Glu Lys Asp Phe Met Val
            35                  40                  45

Leu Thr Lys Asn Gly Trp Lys Asn Ile Asn Tyr Val Tyr Lys His Glu
        50                  55                  60

Thr Asn Lys Pro Ile His Lys Ile Val Thr Lys Asp Arg Leu Val Xaa
65                  70                  75                  80

Xaa Thr Ser Asp His Ser Val Phe Gln Asn Gly Glu Gln Ile Lys Pro
                85                  90                  95

Thr Glu Leu Lys Arg Gly Asp Lys Ile Asp Ile Ile Asp Ile Pro Ile
            100                 105                 110

Leu Lys Ser Leu Asn Val Ile Thr Pro Asn Gln Ala Lys Leu Ile Gly
            115                 120                 125

Phe Phe Ile Gly Asp Gly Ser Ser Ser Tyr Lys Lys Lys Pro Tyr Lys
        130                 135                 140

```
Tyr Asn Ser Val Lys Asn Gly Glu Lys Thr Tyr Gln Val Met Ser Gly
145                 150                 155                 160

Asn Phe Ser Leu Asn Asn Ser Arg Ile Glu Leu Leu Glu Glu Phe Lys
                165                 170                 175

Leu Ile Met Lys Asn Glu Tyr Asn Val Asp Thr Gln Ile Asn Asn Thr
                180                 185                 190

Met Lys Ser Ser Ser Val Tyr Lys Leu Gln Thr Ser Asn Ala Glu Ile
            195                 200                 205

Xaa Lys Trp Phe Ser Lys Asn Xaa Tyr Thr Ser Tyr Arg Gln Lys Met
        210                 215                 220

Ile Pro Tyr Glu Ile Leu Asn Gly Ser Lys Glu Ile Met Lys Ser Phe
225                 230                 235                 240

Met Asp Gly Phe Tyr Leu Ala Asp Gly Trp Gly Asp Asn Phe Asp Gln
                245                 250                 255

Pro Leu Asp Ile Thr Gln Lys Ser Lys Val Xaa Val Ala Gly Leu Thr
                260                 265                 270

His Ile Leu Lys Thr Leu Asp Val Asn Tyr Arg Ile Leu Ile Arg Thr
            275                 280                 285

Asp Lys Pro Asn Ile Gln Ser Leu Thr Leu Gly Ser Phe Asn Asn Lys
            290                 295                 300

Ile Lys Tyr His Pro Leu Asn Asp Glu Lys Ser Lys Arg Lys Thr Asn
305                 310                 315                 320

Glu Val Trp Asn Asn Val Ile Tyr Glu Asn Lys Gln Gln Tyr Val Tyr
                325                 330                 335

Asp Ile Ser Thr Glu Asp Gly Thr Phe Val Gly Gly Ile Gly Gly Val
                340                 345                 350

Leu Leu Lys Asn
            355


<210>  196
<211>  285
<212>  PRT
<213>  Artificial sequence

<220>
<223>  modified contiguous intein from Bedford Basin environmental
```

sampling

```
<220>
<221>  misc_feature
<222>  (78)..(78)
<223>  Xaa can be any naturally occurring amino acid

<220>
<221>  misc_feature
<222>  (114)..(114)
<223>  Xaa can be any naturally occurring amino acid

<220>
<221>  misc_feature
<222>  (136)..(136)
<223>  Xaa can be any naturally occurring amino acid

<220>
<221>  misc_feature
<222>  (170)..(170)
<223>  Xaa can be any naturally occurring amino acid

<220>
<221>  misc_feature
<222>  (196)..(196)
<223>  Xaa can be any naturally occurring amino acid

<220>
<221>  misc_feature
<222>  (203)..(203)
<223>  Xaa can be any naturally occurring amino acid

<400>  196
```

Ser Val Thr Gly Asp Thr Pro Leu Leu Ile Arg Met Arg Asp Gly Ser
1               5                   10                  15

Ile His Thr Lys Arg Ile Asp Glu Leu Thr Asn Glu Tyr His Ala Ser
           20                  25                  30

Asp Gly Gly Lys Glu Ser Phe Pro Gly Asn Tyr Glu Val Trp Thr Glu
           35                  40                  45

Lys Gly Phe Thr Pro Val Glu Arg Val Ile Arg His Lys Thr Met Lys
       50                  55                  60

Lys Met Tyr Arg Val Leu Thr His Thr Gly Val Val Asp Xaa Thr Glu
65              70                  75                  80

Asp His Ser Leu Leu Asp Gln Ala Ala Thr Met Val Lys Pro Thr Asp
               85                  90                  95

Ile Thr Ile Gly Thr Lys Leu Leu His Gly Asn Thr Leu Asp Ala Phe
           100                 105                 110

```
Asp Xaa Ile Asp Met Thr Val Ser Ile Asn Glu Ala Lys Val Met Gly
    115                 120             125

Phe Phe Phe Gly Asp Gly Ser Xaa Gly Gln Tyr Gly Asn Lys Phe Thr
    130             135             140

Trp Ala Leu Asn Asn Ser Asn Pro Asp Tyr Arg Ser Leu Phe Tyr Asn
145             150             155             160

Asp Ala Lys Glu Lys Ile Val Pro Ser Xaa Ile Leu Asn Ala Pro Ile
        165             170             175

Glu Ile Val Gln Ser Phe Ile Asn Gly Tyr Tyr Met Ala Asp Gly Asp
        180             185             190

Lys Asp Ala Xaa Gly Tyr Thr Arg Met Asp Xaa Lys Ser Lys Gln Gly
        195             200             205

Thr Met Gly Leu Gln Leu Leu Gly Arg Arg Leu Gly Tyr Ser Val Ser
    210             215             220

Leu Asn Thr Arg Ser Asp Lys Leu Asn Val Phe Arg Gln Thr Trp Thr
225             230             235             240

Lys Ser Thr Gln Arg Lys Glu Pro Asn Ala Ile Lys Lys Val Leu Tyr
            245             250             255

Leu Gly Glu Thr Glu Gln Tyr Val Tyr Asp Leu Thr Thr Glu Ser His
        260             265             270

His Phe His Val Gly Pro Gly Glu Leu Ile Val His Asn
        275             280             285
```

```
<210>  197
<211>  370
<212>  PRT
<213>  Artificial sequence

<220>
<223>  modified contiguous intein from Chrysochromulina ericina virus
       01B


<220>
<221>  misc_feature
<222>  (20)..(20)
<223>  Xaa can be any naturally occurring amino acid

<220>
<221>  misc_feature
<222>  (42)..(42)
<223>  Xaa can be any naturally occurring amino acid
```

```
<220>
<221>  misc_feature
<222>  (58)..(58)
<223>  Xaa can be any naturally occurring amino acid

<220>
<221>  misc_feature
<222>  (112)..(112)
<223>  Xaa can be any naturally occurring amino acid

<220>
<221>  misc_feature
<222>  (142)..(142)
<223>  Xaa can be any naturally occurring amino acid

<220>
<221>  misc_feature
<222>  (147)..(147)
<223>  Xaa can be any naturally occurring amino acid

<220>
<221>  misc_feature
<222>  (172)..(172)
<223>  Xaa can be any naturally occurring amino acid

<220>
<221>  misc_feature
<222>  (196)..(196)
<223>  Xaa can be any naturally occurring amino acid

<220>
<221>  misc_feature
<222>  (271)..(271)
<223>  Xaa can be any naturally occurring amino acid

<400>  197
```

Ser Val Ala Ser Thr Pro Ile Tyr Val Arg Tyr Asn Lys Ser Ile
1               5                   10                  15

Ile Asp Ile Xaa Ser Val Glu Glu Leu Ala Glu Lys Tyr Gly Asn Gly
                20                  25                  30

Trp His Leu Glu Ser Pro Lys Glu Tyr Xaa Glu Leu Asn Asn Ile Glu
            35                  40                  45

Ser Trp Thr Glu Asn Gly Trp Thr Glu Xaa His Arg Val Ile Arg His
        50                  55                  60

Arg Leu Ala Pro Tyr Lys Lys Met Val Arg Ile Leu Thr His Thr Gly
65                  70                  75                  80

Leu Val Asp Val Thr Asp Asp His Ser Leu Val Lys Asn Thr Gly Glu
                85                  90                  95

Glu Ile Ser Pro Lys Asp Val Ser Ile Gly Thr Lys Leu Leu His Xaa

                100                    105                        110

Thr Met Ser Glu Asn Glu Ser Asn Ile Glu Ser Asp Ile Ser Ile Asp
        115                120                125

Glu Ala Arg Ile Met Gly Phe Phe Phe Gly Asp Gly Ser Xaa Gly Ile
    130                135                140

Tyr Asp Xaa Pro Ser Gly His Lys Ala Ser Trp Ala Leu Asn Asn Ser
145                150                155                    160

Asn Lys Glu Leu Ile Glu Lys Tyr Tyr Asn Leu Xaa Lys Ser Val Tyr
                165                170                    175

Pro Glu Phe Glu Trp Lys Val Tyr Asp Thr Leu Asn Ser Ser Gly Val
            180                185                190

Tyr Lys Ile Xaa Phe Asn Lys Lys Ser Gly Ser Lys Ser Lys Ile Gln
        195                200                205

Phe Ile Glu Lys Tyr Arg Ser Met Leu Tyr Asn Lys Lys Ser Lys Ile
    210                215                220

Ile Pro Ser Glu Ile Ile Asn Gly Ser Ile Glu Leu Arg Lys Ser Phe
225                230                235                    240

Trp Glu Gly Leu Tyr Asp Ala Asp Gly Asp Lys Asp Lys Asn Gly Tyr
                245                250                    255

Thr Arg Ile Asp Gln Lys Ser Gln Ile Ser Ala Ala Tyr Ile Xaa Trp
            260                265                270

Leu Ala Asn Ser Ile Gly Tyr Lys Thr Ser Leu Asn Ile Arg Asp Asp
        275                280                285

Lys Thr Asp Ile Tyr Arg Ile Thr Ala Thr Lys Asn Lys Gln Arg Arg
    290                295                300

Asp Gly Asp Lys Ile Lys Lys Ile Val Asn Ile Gln Asn Ser Ala Asn
305                310                315                    320

Ile Gln Asn Ser Ala Asn Ile Gln Asn Ser Val Asn Ile Gln Asn Ser
            325                330                335

Val Asn Ile Gln Asn Ser Lys Asp Asn Gln Asp Tyr Val Tyr Asp Leu
        340                345                350

```
Thr Thr Glu Asn His His Phe Ala Ala Gly Ile Gly Asn Met Ile Val
        355                 360                 365

His Asn
    370


<210>  198
<211>  110
<212>  PRT
<213>  Artificial sequence

<220>
<223>  modified contiguous intein (partial) from Hyphochytrium
       catenoides ATCC 18719


<220>
<221>  misc_feature
<222>  (36)..(36)
<223>  Xaa can be any naturally occurring amino acid

<220>
<221>  misc_feature
<222>  (63)..(63)
<223>  Xaa can be any naturally occurring amino acid

<220>
<221>  misc_feature
<222>  (110)..(110)
<223>  Xaa can be any naturally occurring amino acid

<400>  198

Ser Val Ala Ser Tyr Thr Pro Ile Met Val Arg Leu Ser Lys Arg Asn
1               5               10                  15


Arg Val Leu Ile Thr Ile Glu Glu Leu Ser Arg Leu Ser Gly Lys Arg
            20                  25                  30


Trp Thr Ser Xaa Gly Asp Pro Gly Arg Asp Asn Lys Glu Phe Ile Asp
        35                  40                  45


Leu Asn Asp Val Glu Thr Trp Ser Asp Lys Gly Trp Thr Pro Xaa His
        50                  55                  60


Arg Ile Ile Arg His Gln Leu Ala Pro Asn Lys Lys Met Ile Arg Val
65                  70                  75                  80


Val Thr Arg Ser Ser Val Val Asp Val Thr Asp Asp His Ser Leu Leu
                85                  90                  95


Arg Pro Asp Gly Thr Met Val Ser Pro Lys Asp Leu Arg Xaa
                100                 105                 110
```

247

<210> 199
<211> 169
<212> PRT
<213> Artificial sequence

<220>
<223> modified contiguous intein (partial) from Hyphochytrium catenoides ATCC 18719

<220>
<221> misc_feature
<222> (1)..(1)
<223> Xaa can be any naturally occurring amino acid

<220>
<221> misc_feature
<222> (7)..(7)
<223> Xaa can be any naturally occurring amino acid

<220>
<221> misc_feature
<222> (14)..(14)
<223> Xaa can be any naturally occurring amino acid

<220>
<221> misc_feature
<222> (39)..(39)
<223> Xaa can be any naturally occurring amino acid

<220>
<221> misc_feature
<222> (123)..(123)
<223> Xaa can be any naturally occurring amino acid

<400> 199

Xaa Gly Glu His Leu Leu Xaa His Ala Leu Ala Pro Arg Xaa Ser Phe
1               5                   10                  15

Asn Asp Thr Phe Pro Val Thr Ser Glu Glu Ala Arg Gln Met Gly Tyr
                20                  25                  30

Phe Phe Gly Asn Gly Thr Xaa Gly Gly Thr Trp Asp Leu Ile Pro Gln
            35                  40                  45

Asp Val Leu Asn Gly Ser Arg Ala Val Arg Gln Ala Phe Trp Asp Ala
        50                  55                  60

Met His Gly Ile Asp Thr Gly Asp Gly His Asp His Gly Arg Asn Met
65                  70                  75                  80

Leu Gln Ile Asp Gln Lys Ser Gln Leu Ser Ile Ala His Ile Asn Leu
                85                  90                  95

Leu Ala Gln Ser Leu Gly Tyr Thr Thr Ser Val Thr Ile Leu Thr Thr
            100                 105                 110

Ser Thr Glu Ser Thr Val Tyr Arg Leu Ile Xaa Thr Ala Ala Thr Asn
          115                 120                 125

Glu Arg Ser Ile Asn Ser Glu Ile Val Glu Ile Tyr Glu Ile Pro Tyr
          130                 135                 140

Asp Gly Tyr Val Tyr Asp Leu Thr Thr Glu Asn His His Phe Ala Ala
145                     150                 155                 160

Gly Ala Gly Asn Ile Ile Val His Asn
                    165

<210> 200
<211> 269
<212> PRT
<213> Artificial sequence

<220>
<223> modified contiguous intein (partial) from Paramoeba atlantica
      CCAP 1560/9 (621/1)


<220>
<221> misc_feature
<222> (1)..(1)
<223> Xaa can be any naturally occurring amino acid

<220>
<221> misc_feature
<222> (69)..(69)
<223> Xaa can be any naturally occurring amino acid

<220>
<221> misc_feature
<222> (72)..(72)
<223> Xaa can be any naturally occurring amino acid

<220>
<221> misc_feature
<222> (74)..(74)
<223> Xaa can be any naturally occurring amino acid

<220>
<221> misc_feature
<222> (171)..(171)
<223> Xaa can be any naturally occurring amino acid

<220>
<221> misc_feature
<222> (208)..(208)
<223> Xaa can be any naturally occurring amino acid

<400> 200

Xaa Ile Tyr Arg Val Thr Ser His Thr Gly Val Val Lys Val Thr Glu
1               5                   10                  15

Asp His Ser Leu Leu Tyr His Asn Gly Thr Glu Val Arg Pro Lys Asp
20 25 30

Val Phe Ala Gly Glu Asn Leu Leu Thr Ser Thr Leu Pro Ser Ile Asp
35 40 45

Gly Thr Val Asp His Ser Asp Ile Ser Trp Val Trp Gly Leu Phe Tyr
50 55 60

Gly Asp Gly Ser Xaa Gly Tyr Xaa Asp Xaa Leu Thr Gly Lys Lys Tyr
65 70 75 80

Thr Trp Ala Ile Asn Asn Gln Asn Gly Glu Tyr Leu Ser Lys Ala Lys
85 90 95

Glu Ile Leu Gln Gly Tyr Tyr Ser Asp Tyr Gly Phe Lys Ile Leu Glu
100 105 110

Thr Met Lys Lys Ser Ser Ser Val Tyr Lys Leu Val Pro Thr Gly Lys
115 120 125

Val Ser Glu Ile Val Glu Glu Trp Arg Ser Leu Phe Tyr Asp Pro Glu
130 135 140

Thr Arg His Lys Met Val Pro Asn Val Leu Trp Ser Thr Thr Leu Lys
145 150 155 160

Thr Arg Glu Glu Phe Phe Glu Gly Tyr Tyr Xaa Ala Gly Gly Glu Lys
165 170 175

Gly Glu Asn Gly Pro Thr Arg Phe Asp Asn Lys Gly Asp Ile Gly Ser
180 185 190

Ala Gly Leu Phe Tyr Leu Gly Ile Ser Leu Gly Tyr Lys Ala Ser Xaa
195 200 205

Asn Thr Arg Lys Asp Asn Leu Asp Ile Thr Arg Ile Thr Leu Thr Lys
210 215 220

Ser Tyr Gln Arg Lys Lys Pro Gly Gly Ile Ile Lys Lys Ile Glu Asp
225 230 235 240

Phe Gly Gln Thr Gly Asp Tyr Val Tyr Asp Leu Glu Thr Glu Asn His
245 250 255

His Phe Ser Ala Gly Ile Gly Glu Leu Val Val His Asn
260 265

<210> 201
<211> 437
<212> PRT
<213> Artificial sequence

<220>
<223> modified contiguous intein from Haloferax lucentense DSM 14919

<220>
<221> misc_feature
<222> (395)..(395)
<223> Xaa can be any naturally occurring amino acid

<400> 201

Ser Val Thr Gly Asp Arg Pro Val Val Val Arg Asp Pro Gly Gly Thr
1               5                   10                  15

Val Arg Ile Leu Pro Ile Glu Asp Leu Phe Ala Arg Gly Thr Thr Glu
            20                  25                  30

Ser Glu Val Leu Ile Ala Ala Asp Gly Asp Val Val Ala Ser Ala Thr
            35                  40                  45

Pro Gly Lys Thr Arg Arg Ala Leu Asp Gly Trp Asp Ala Leu Ser Val
        50                  55                  60

Asn Glu Ala Gly Glu Ala Glu Trp Gln Pro Ile Ala Gln Ala Ile Arg
65                  70                  75                  80

His Lys Thr Asp Lys Pro Val Val Asn Leu Gln His Lys Phe Gly Glu
                85                  90                  95

Ser Thr Thr Thr Arg Asp His Ser Tyr Val Val Pro Gly Glu Asp Gly
                100                 105                 110

Leu Thr Thr Val Ser Pro Asp Asp Val Ala Glu Pro Tyr Arg Val Ser
            115                 120                 125

Gly Val Pro Asp Val Glu Pro Val Glu Gln Val Asp Val Tyr Glu Val
        130                 135                 140

Leu Arg Gly Tyr Glu Arg Glu Tyr Glu Asp Gly Arg Ser Val Gly Ser
145                 150                 155                 160

Asp Asn Ser Ile Thr Lys Arg Lys Gln Ile His Ala Asp Asp Glu Tyr
                165                 170                 175

Val Trp Phe Gly His Glu His His Arg Asp Val Asp Ser Thr Val Lys

|  |  |  | 180 |  |  |  |  | 185 |  |  |  |  | 190 |  |
|---|---|---|---|---|---|---|---|---|---|---|---|---|---|---|

Val Lys Arg Phe Val Asp Ile Asp Ser Glu Asp Gly Ala Ala Leu Ile
    195            200            205

Arg Leu Leu Gly Ala Tyr Val Ser Glu Gly Ser Ala Ser Thr Gly Glu
    210            215            220

Thr Ala Thr Ser Lys Phe Gly Ala Ser Ile Ala Glu Ser Asp Arg Glu
225            230          235            240

Trp Leu Ala Gln Leu Gln Arg Asp Tyr Ser Arg Leu Phe Glu Asn Thr
        245          250          255

Thr Ala Gly Ile Ile Thr Ser Asp Arg Arg Ala Glu Arg Thr Val Glu
        260          265          270

Tyr Gln Thr Asp Thr Gly Gly Ala Ser Val Thr Tyr Asn Asp Glu Thr
    275          280          285

Leu Lys Leu Gln Met Met Asn Glu Leu Ala Ala Val Phe Phe Arg Glu
    290          295          300

Phe Ala Gly Gln Thr Ser Arg Gly Lys Arg Ile Pro Ser Phe Val Phe
305          310          315            320

His Leu Pro Glu Glu Lys Gln Asp Leu Phe Leu Thr Leu Leu Val Glu
        325          330          335

Gly Asp Gly Ser Arg Glu Phe Pro Arg Tyr Thr Glu Ala Tyr Ala Gln
        340          345          350

Arg Asn Phe Asp Phe Glu Thr Thr Ser Arg Glu Leu Ala Ala Gly Leu
        355          360          365

Ser Met Leu Leu Thr Gln Arg Gly Gln Lys His Ser Leu Lys Tyr Arg
    370          375          380

Asp Ser Lys Asp Ser Tyr Thr Ile Arg Thr Xaa Ser Thr Tyr Arg Glu
385          390          395            400

Gly Arg Asp Pro Val Leu Thr Glu Val Asp His Asp Gly Tyr Val Tyr
        405          410          415

Asp Leu Ser Val Glu Glu Asn Glu Asn Phe Val Asp Gly Val Gly Gly
    420          425          430

Ile Val Leu His Asn
435


<210> 202
<211> 437
<212> PRT
<213> Artificial sequence

<220>
<223> modified contiguous intein from Haloferax species Q22


<220>
<221> misc_feature
<222> (395)..(395)
<223> Xaa can be any naturally occurring amino acid

<400> 202

Ser Val Thr Gly Asp Arg Pro Val Val Val Arg Asp Pro Gly Gly Thr
1               5                   10                  15


Val Arg Ile Leu Pro Ile Glu Asp Leu Phe Ala Arg Gly Thr Thr Glu
            20                  25                  30


Ser Glu Val Leu Ile Ala Ala Asp Gly Asp Val Val Ala Ser Ala Thr
            35                  40                  45


Pro Gly Lys Thr Arg Arg Ala Leu Asp Gly Trp Asp Ala Leu Ser Val
        50                  55                  60


Asn Glu Asp Gly Glu Ala Glu Trp Gln Pro Ile Ala Gln Ala Ile Arg
65                  70                  75                  80


His Lys Thr Asp Lys Pro Val Val Asn Leu Gln His Lys Phe Gly Glu
                85                  90                  95


Ser Thr Thr Thr Arg Asp His Ser Tyr Val Val Pro Gly Glu Asp Gly
            100                 105                 110


Leu Thr Thr Val Ser Pro Asp Asp Val Ala Glu Pro Tyr Arg Val Ser
            115                 120                 125


Gly Val Pro Asp Val Glu Pro Val Glu Gln Val Asp Val Tyr Glu Val
        130                 135                 140


Leu Arg Gly Tyr Glu Arg Glu Tyr Glu Asp Gly Arg Ser Val Gly Ser
145                 150                 155                 160


Asp Asn Ser Ile Thr Lys Arg Lys Gln Ile His Ala Asp Asp Glu Tyr
                165                 170                 175

```
Val Trp Phe Gly His Glu His His Arg Asp Val Asp Ser Thr Val Lys
        180             185             190

Val Lys Arg Phe Val Asp Ile Asp Ser Glu Asp Gly Ala Ala Leu Ile
        195             200             205

Arg Leu Leu Gly Ala Tyr Val Pro Glu Gly Ser Ala Ser Thr Gly Glu
        210             215             220

Thr Val Thr Ser Lys Phe Gly Ala Ser Ile Ala Glu Ser Asp Arg Glu
225             230             235             240

Trp Leu Ala Gln Leu Gln Arg Asp Tyr Ser Arg Leu Phe Glu Asn Thr
            245             250             255

Thr Ala Gly Ile Ile Thr Ser Asp Arg Arg Ala Glu Arg Thr Val Glu
        260             265             270

Tyr Gln Thr Asp Thr Gly Gly Ala Ser Val Thr Tyr Asn Asp Glu Thr
        275             280             285

Leu Lys Leu Gln Met Met Asn Glu Leu Ala Ala Val Phe Phe Arg Glu
        290             295             300

Phe Ala Gly Gln Thr Ser Arg Gly Lys Arg Ile Pro Ser Phe Val Phe
305             310             315             320

His Leu Pro Glu Glu Lys Gln Asp Leu Phe Leu Thr Leu Leu Val Glu
            325             330             335

Gly Asp Gly Ser Arg Glu Phe Pro Arg Tyr Thr Glu Ala Tyr Ala Gln
            340             345             350

Arg Asn Phe Asp Phe Glu Thr Thr Ser Arg Glu Leu Ala Ala Gly Leu
        355             360             365

Ser Met Leu Leu Thr Gln Arg Gly Gln Lys His Ser Leu Lys Tyr Arg
        370             375             380

Asp Ser Lys Asp Ser Tyr Thr Ile Arg Thr Xaa Ser Ser Tyr Arg Glu
385             390             395             400

Gly Arg Asp Pro Val Leu Thr Glu Val Asp His Asp Gly Tyr Val Tyr
            405             410             415

Asp Leu Ser Val Glu Glu Asn Glu Asn Phe Val Asp Gly Val Gly Gly
        420             425             430
```

254

```
Ile Val Leu His Asn
        435


<210>  203
<211>  437
<212>  PRT
<213>  Artificial sequence

<220>
<223>  modified contiguous intein from Haloferax massiliensis ArcH


<220>
<221>  misc_feature
<222>  (395)..(395)
<223>  Xaa can be any naturally occurring amino acid

<400>  203

Ser Val Thr Gly Asp Arg Pro Val Val Ala Arg Asp Pro Gly Gly Thr
1               5                   10                  15

Val Arg Ile Leu Pro Ile Glu Asp Leu Phe Ala Arg Gly Thr Thr Glu
            20                  25                  30

Ser Glu Val Leu Ile Ala Ala Asp Gly Asp Val Val Ala Ser Ala Thr
        35                  40                  45

Pro Gly Lys Thr Arg Arg Ala Leu Asp Gly Trp Asp Ala Leu Ser Val
    50                  55                  60

Asn Glu Asp Gly Glu Ala Glu Trp Gln Pro Ile Ala Gln Ala Ile Arg
65                  70                  75                  80

His Lys Thr Asp Lys Pro Val Val Asn Leu Gln His Lys Phe Gly Glu
                85                  90                  95

Ser Thr Thr Thr Arg Asp His Ser Tyr Val Val Pro Gly Glu Asp Gly
            100                 105                 110

Leu Thr Thr Val Ser Pro Asp Val Ala Glu Pro Tyr Arg Val Ser
        115                 120                 125

Gly Val Pro Asp Val Glu Pro Val Glu Arg Val Asp Val Tyr Glu Val
    130                 135                 140

Leu Arg Gly Tyr Glu Arg Glu Tyr Glu Asp Gly Arg Ser Val Gly Ser
145                 150                 155                 160

Asp Asn Ser Ile Thr Lys Arg Lys Gln Ile His Ala Asp Asp Glu Tyr
```

EP 3 750 911 A1

```
                    165                 170                 175

        Val Trp Phe Gly His Glu His His Arg Asp Val Asp Ser Thr Val Lys
                    180                 185                 190

        Val Lys Arg Phe Val Asp Ile Asp Ser Glu Asp Gly Ala Ala Leu Ile
                    195                 200                 205

        Arg Leu Leu Gly Ala Tyr Val Pro Glu Gly Ser Ala Ser Thr Gly Glu
                    210                 215                 220

        Thr Ala Thr Ser Lys Phe Gly Ala Ser Ile Ala Glu Ser Asp Arg Glu
        225                 230                 235                 240

        Trp Leu Ala Gln Leu Gln Arg Asp Tyr Ser Arg Leu Phe Glu Asn Thr
                    245                 250                 255

        Thr Ala Gly Ile Ile Thr Ser Asp Arg Arg Ala Glu Arg Thr Ala Glu
                    260                 265                 270

        Tyr Gln Thr Asp Thr Gly Gly Ala Ser Val Thr Tyr Asn Asp Glu Thr
                    275                 280                 285

        Leu Lys Leu Gln Met Met Asn Glu Leu Ala Ala Val Phe Phe Arg Glu
                    290                 295                 300

        Phe Ala Gly Gln Thr Ser Arg Gly Lys Arg Ile Pro Ser Phe Val Phe
        305                 310                 315                 320

        His Leu Pro Glu Glu Lys Gln Asp Leu Phe Leu Thr Leu Leu Val Glu
                    325                 330                 335

        Gly Asp Gly Ser Arg Glu Phe Pro Arg Tyr Thr Glu Ala Tyr Ala Gln
                    340                 345                 350

        Arg Asn Phe Asp Phe Glu Thr Thr Ser Arg Glu Leu Ala Ala Gly Leu
                    355                 360                 365

        Ser Met Leu Leu Thr Gln Arg Gly Gln Lys His Ser Leu Lys Tyr Arg
                    370                 375                 380

        Asn Ser Lys Asp Ser Tyr Thr Ile Arg Thr Xaa Gly Thr Tyr Arg Lys
        385                 390                 395                 400

        Gly Arg Asp Pro Val Leu Thr Glu Val Asp His Asp Gly Tyr Val Tyr
                    405                 410                 415
```

Asp Leu Ser Val Glu Glu Asn Glu Asn Phe Val Asp Gly Val Gly Gly
                420             425             430

Ile Val Leu His Asn
            435

<210> 204
<211> 437
<212> PRT
<213> Artificial sequence

<220>
<223> modified contiguous intein from Haloferax gibbonsii ATCC 33959

<220>
<221> misc_feature
<222> (395)..(395)
<223> Xaa can be any naturally occurring amino acid

<400> 204

Ser Val Thr Gly Asp Arg Pro Val Val Arg Asp Pro Gly Gly Thr
1               5               10              15

Val Arg Ile Leu Pro Ile Glu Asp Leu Phe Ala Arg Gly Thr Thr Glu
            20              25              30

Ser Glu Val Leu Ile Ala Ala Asp Gly Asp Val Val Ala Ser Ala Thr
            35              40              45

Pro Gly Lys Thr Arg Arg Ala Leu Asp Gly Trp Asp Ala Leu Ser Val
        50              55              60

Asn Glu Asp Gly Glu Ala Glu Trp Gln Pro Ile Ala Gln Ala Ile Arg
65              70              75              80

His Asn Thr Asp Lys Pro Val Val Asn Leu Gln His Lys Phe Gly Glu
            85              90              95

Ser Thr Thr Thr Arg Asp His Ser Tyr Val Val Pro Gly Glu Asp Gly
            100             105             110

Leu Thr Thr Val Ser Pro Asp Asp Val Ala Glu Pro Tyr Arg Val Ser
            115             120             125

Gly Val Pro Asp Val Glu Pro Val Glu Gln Val Asp Val Tyr Glu Val
        130             135             140

Leu Arg Gly Tyr Glu Arg Glu Tyr Glu Asp Gly Arg Ser Val Gly Ser
145             150             155             160

```
Asp Asn Ser Ile Thr Lys Arg Lys Gln Ile His Ala Asp Asp Glu Tyr
                165                 170                 175

Val Trp Phe Gly His Glu His His Arg Asp Val Asp Ser Thr Val Lys
                180                 185                 190

Val Lys Arg Phe Val Asp Ile Asp Ser Glu Asp Gly Ala Ala Leu Ile
                195                 200                 205

Arg Leu Leu Gly Ala Tyr Val Ser Glu Gly Ser Ala Ser Thr Gly Glu
    210                 215                 220

Thr Ala Thr Ser Lys Phe Gly Ala Ser Ile Ala Glu Ser Asp Arg Glu
225                 230                 235                 240

Trp Leu Ala Gln Leu Gln Arg Asp Tyr Ser Arg Leu Phe Glu Asn Thr
                245                 250                 255

Thr Ala Gly Ile Ile Thr Ser Asp Arg Arg Ala Glu Arg Thr Val Glu
                260                 265                 270

Tyr Gln Thr Asp Thr Gly Gly Ala Ser Val Thr Tyr Asn Asp Glu Thr
                275                 280                 285

Leu Lys Leu Gln Met Met Asn Glu Leu Ala Ala Val Phe Phe Arg Glu
    290                 295                 300

Phe Ala Gly Gln Thr Ser Arg Gly Lys Arg Ile Pro Ser Phe Val Phe
305                 310                 315                 320

His Leu Pro Glu Glu Lys Gln Asp Leu Phe Leu Thr Leu Leu Val Glu
                325                 330                 335

Gly Asp Gly Ser Arg Glu Phe Pro Arg Tyr Thr Glu Ala Tyr Ala Gln
                340                 345                 350

Arg Asn Phe Asp Phe Glu Thr Thr Ser Arg Glu Leu Ala Ala Gly Leu
                355                 360                 365

Ser Met Leu Leu Thr Gln Arg Gly Gln Lys His Ser Leu Lys Tyr Arg
    370                 375                 380

Asp Ser Lys Asp Ser Tyr Thr Ile Arg Thr Xaa Ser Ser Tyr Arg Glu
385                 390                 395                 400

Gly Arg Asp Pro Val Leu Thr Glu Val Asp His Asp Gly Tyr Val Tyr
                405                 410                 415
```

258

```
Asp Leu Ser Val Glu Glu Asn Glu Asn Phe Val Asp Gly Val Gly Gly
            420                 425             430

Ile Val Leu His Asn
            435


<210>  205
<211>  437
<212>  PRT
<213>  Artificial sequence

<220>
<223>  modified contiguous intein from Haloferax gibbonsii ARA6


<220>
<221>  misc_feature
<222>  (395)..(395)
<223>  Xaa can be any naturally occurring amino acid

<400>  205

Ser Val Thr Gly Asp Arg Pro Val Val Arg Asp Pro Gly Gly Thr
1               5                 10              15

Val Arg Ile Leu Pro Ile Glu Asp Leu Phe Ala Arg Gly Thr Thr Glu
            20                25              30

Ser Glu Val Leu Ile Ala Ala Asp Gly Asp Val Val Ala Ser Ala Thr
            35                40              45

Pro Gly Lys Thr Arg Arg Ala Leu Asp Gly Trp Glu Ala Leu Ser Val
        50                55              60

Asn Glu Asp Gly Glu Ala Glu Trp Gln Pro Ile Ala Gln Ala Ile Arg
65              70                75              80

His Lys Thr Asp Lys Pro Val Val Asn Leu Gln His Lys Phe Gly Glu
            85                90              95

Ser Thr Thr Thr Arg Asp His Ser Tyr Val Val Pro Gly Glu Gly Gly
            100               105             110

Leu Thr Thr Val Ser Pro Asp Asp Val Ala Glu Pro Tyr Arg Val Ser
            115               120             125

Gly Val Pro Asp Val Glu Pro Val Glu Gln Val Asp Val Tyr Glu Val
        130               135             140

Leu Arg Gly Tyr Glu Arg Glu Tyr Glu Asp Gly Arg Ser Val Gly Ser
```

```
              145                  150                  155                  160


              Asp Asn Ser Ile Thr Lys Arg Lys Gln Ile His Ala Asp Asp Glu Tyr
                          165                  170                  175


              Val Trp Phe Gly His Glu His His Arg Asp Val Asp Ser Thr Val Lys
                          180                  185                  190


              Val Lys Arg Phe Val Asp Ile Asp Ser Glu Asp Gly Ala Ala Leu Ile
                          195                  200                  205


              Arg Leu Leu Gly Ala Tyr Val Pro Glu Gly Ser Ala Ser Thr Gly Glu
                          210                  215                  220


              Thr Ala Ala Ser Lys Phe Gly Ala Ser Ile Ala Glu Ser Asp Arg Glu
              225                  230                  235                  240


              Trp Leu Ala Gln Leu Gln Arg Asp Tyr Ser Arg Leu Phe Glu Asn Thr
                          245                  250                  255


              Thr Ala Gly Ile Ile Thr Ser Asp Arg Arg Ala Glu Arg Thr Val Glu
                          260                  265                  270


              Tyr Gln Thr Asp Thr Gly Gly Ala Ser Val Thr Tyr Asn Asp Glu Thr
                          275                  280                  285


              Leu Lys Leu Gln Met Met Asn Glu Leu Ala Ala Val Phe Phe Arg Glu
                          290                  295                  300


              Phe Ala Gly Gln Thr Ser Arg Gly Lys Arg Ile Pro Ser Phe Val Phe
              305                  310                  315                  320


              His Leu Pro Glu Glu Lys Gln Asp Leu Phe Leu Thr Leu Leu Val Glu
                          325                  330                  335


              Gly Asp Gly Ser Arg Glu Phe Pro Arg Tyr Thr Glu Ala Tyr Ala Gln
                          340                  345                  350


              Arg Asn Phe Asp Phe Glu Thr Thr Ser Arg Glu Leu Ala Ala Gly Leu
                          355                  360                  365


              Ser Met Leu Leu Thr Gln Arg Gly Gln Lys His Ser Leu Lys Tyr Arg
                          370                  375                  380


              Asp Ser Lys Gly Ser Tyr Thr Ile Arg Thr Xaa Ser Ser Tyr Arg Glu
              385                  390                  395                  400
```

```
Gly Arg Asp Pro Val Leu Thr Glu Val Asp His Asp Gly Tyr Val Tyr
            405             410             415

Asp Leu Ser Val Glu Glu Asn Glu Asn Phe Val Asp Gly Val Gly Gly
            420             425             430

Ile Val Leu His Asn
            435


<210> 206
<211> 434
<212> PRT
<213> Artificial sequence

<220>
<223> modified contiguous intein Halarchaeum acidiphilum MH1-52-1


<220>
<221> misc_feature
<222> (61)..(61)
<223> Xaa can be any naturally occurring amino acid

<220>
<221> misc_feature
<222> (392)..(392)
<223> Xaa can be any naturally occurring amino acid

<400> 206

Ser Val Thr Gly Asp Arg Pro Val Val Val Arg Asp Pro Glu Gly Arg
1               5               10              15

Val Arg Ile Thr Pro Ile Ala Glu Leu Phe Glu Arg Ala Ala Arg Ser
            20              25              30

Glu Asn Val Leu Val Thr Ala Asp Gly Gly Pro Val Thr Ser Ala Ser
            35              40              45

Val Gly Lys Asp Arg Arg Thr Leu Asp Gly Trp Asp Xaa Leu Ser Leu
        50              55              60

Asn Asp Asp Gly Glu Thr Glu Trp Lys Pro Ile Glu Gln Ala Ile Arg
65              70              75              80

His Glu Thr Asp Glu Pro Val Val Lys Leu Gln His Glu Phe Gly Glu
            85              90              95

Ser Thr Thr Thr Arg Asp His Ser Tyr Val Val Asp Gly Ala Asp Gly
            100             105             110

Leu Glu Glu Ala Val Pro Ala Asp Val Asp Glu Pro Leu Arg Val Pro
            115             120             125
```

Asp Met Pro Asp Ala Gly Thr Val Thr Glu Ile Asp Val Tyr Glu Val
130                135                140

Leu Arg Gly Tyr Glu Arg Glu Tyr Glu Asp Gly Arg Gly Thr Gly Gly
145                150                155                160

Ser Thr Val Lys Thr Lys Arg Val Tyr Ala Asp Asp Glu Ser Val Trp
                165                170                175

Phe Gly His Glu His Tyr Gly Asp Leu Asp Ser Thr Val Thr Val Gln
                180                185                190

Arg His Ile Asp Leu Ala Ser Glu Asp Gly Ala Ala Leu Val Arg Leu
                195                200                205

Leu Gly Ala Tyr Val Pro Glu Gly Ser Ala Ser Thr Val Glu Thr Ala
210                215                220

Asp Gly Lys Phe Gly Ala Ser Ile Ala Glu Ser Arg Arg Glu Trp Ile
225                230                235                240

Glu Gln Leu Glu Asp Asp Tyr His Arg Leu Phe Glu Asn Ala Glu Ala
                245                250                255

Ser Ile Ile Ala Ser Asp Ser Arg Asp Glu Arg Ala Leu Glu Tyr Glu
                260                265                270

Thr Glu Ser Gly Ala Glu Ser Ala Thr Tyr Asp Asp Arg Thr Leu Lys
                275                280                285

Leu Gln Met Met Asn Glu Leu Ser Ala Val Phe Phe Arg Glu Phe Ala
290                295                300

Gly Gln Thr Ser His Arg Thr Arg Ile Pro Ser Phe Val Tyr His Leu
305                310                315                320

Asp Asp Asp Leu Gln Ala Leu Phe Leu Asp Val Leu Val Glu Gly Asp
                325                330                335

Gly Ser Arg Glu Phe Pro Tyr Ser Glu Gly Tyr Ala Ala Arg Asn Phe
                340                345                350

Asp Phe Glu Thr Thr Ser Arg Glu Leu Ala Ala Gly Leu Ser Met Leu
                355                360                365

Leu Thr Gln Arg Gly Lys Lys His Ser Leu Lys Tyr Arg Asp Gly Lys

```
              370                    375                    380


        Gly Ser Tyr Thr Val Arg Thr Xaa Asp Ser Tyr Arg Gly Gly Arg Asp
        385                    390                    395                    400


        Pro Val Leu Thr Thr Val Glu His Asp Gly Tyr Val Tyr Asp Leu Ser
                    405                    410                    415


        Val Ala Asp Asn Glu Asn Phe Val Asp Ala Leu Gly Gly Ile Val Leu
                    420                    425                    430


        His Asn



        <210>  207
        <211>  420
        <212>  PRT
        <213>  Artificial sequence

        <220>
        <223>  modified contiguous intein from Micrarchaeota species CG1_02_55_2


        <220>
        <221>  misc_feature
        <222>  (7)..(7)
        <223>  Xaa can be any naturally occurring amino acid

        <220>
        <221>  misc_feature
        <222>  (13)..(13)
        <223>  Xaa can be any naturally occurring amino acid

        <220>
        <221>  misc_feature
        <222>  (36)..(36)
        <223>  Xaa can be any naturally occurring amino acid

        <220>
        <221>  misc_feature
        <222>  (105)..(105)
        <223>  Xaa can be any naturally occurring amino acid

        <220>
        <221>  misc_feature
        <222>  (190)..(190)
        <223>  Xaa can be any naturally occurring amino acid

        <220>
        <221>  misc_feature
        <222>  (241)..(241)
        <223>  Xaa can be any naturally occurring amino acid

        <220>
        <221>  misc_feature
        <222>  (271)..(271)
        <223>  Xaa can be any naturally occurring amino acid
```

```
<220>
<221>  misc_feature
<222>  (288)..(288)
<223>  Xaa can be any naturally occurring amino acid

<220>
<221>  misc_feature
<222>  (325)..(325)
<223>  Xaa can be any naturally occurring amino acid

<220>
<221>  misc_feature
<222>  (374)..(374)
<223>  Xaa can be any naturally occurring amino acid

<220>
<221>  misc_feature
<222>  (378)..(378)
<223>  Xaa can be any naturally occurring amino acid

<220>
<221>  misc_feature
<222>  (413)..(413)
<223>  Xaa can be any naturally occurring amino acid

<400>  207
```

Ser Ile Thr Asp Glu Arg Xaa Ile Ala Tyr Leu Asp Xaa Asn Gly Ile
1               5                   10                  15

Leu Arg Leu Ala Pro Ile Ala Glu Ile Phe Asp Arg Tyr Gly Lys Thr
        20                  25                  30

Lys Thr Ala Xaa Gly Asp Lys Glu Val Ile Tyr Ala Pro Gly Ile Arg
        35                  40                  45

Ala Leu Ser Val Asp Pro Lys Thr Met Glu Pro Ile Trp Arg Pro Val
    50                  55                  60

Thr Glu Ile Ile Arg His Arg Asn Thr Lys Arg Val Tyr Arg Val Arg
65                  70                  75                  80

Gln Lys Thr Gly Glu Thr Arg Val Thr Glu Asp His Ser Ile Met Ile
                85                  90                  95

Asp Lys Arg Gly Phe Leu Glu Glu Xaa Lys Pro Ala Asp Ile Gly Lys
                100                 105                 110

Lys Arg Leu Ala Tyr Leu Arg Lys Ile Pro Ala Val Lys Glu Ile Thr
        115                 120                 125

Glu Ile Asn Leu Thr Asp Trp Leu Gly Glu Tyr Glu Asn Lys Val Arg
        130                 135                 140

```
Tyr Lys Gly Arg Ile Lys Thr Arg Ala Ile Lys Val Ala Asp Asp Gly
145                 150                 155                 160

Ser Leu Thr Phe Ser Trp Thr Ala Gln Lys Arg Gln Ile Lys Val Gln
                165                 170                 175

Arg Arg Tyr Pro Val Glu Ser Pro Arg Phe Glu Ala Leu Xaa Arg Leu
            180                 185                 190

Leu Gly Ala Tyr Ile Ala Glu Gly Ser Ala Ser Thr Pro Glu Thr Thr
        195                 200                 205

Gly Thr Arg Met Gly Ala Ser Ile Ala Ser Gly Asn Arg Glu Trp Leu
    210                 215                 220

Glu Ser Leu Lys Met Asp Tyr Glu Ser Leu Phe Thr Asn Ala Arg Ala
225                 230                 235                 240

Xaa Val Ile Arg Ser Asn Ile Lys Thr Arg His Leu Asp Tyr Val Asn
            245                 250                 255

Leu Asp Gly Met Ala His Ser Thr Val Tyr Asp Asp Ala Thr Xaa Lys
            260                 265                 270

Leu Gln Met Met Asn Ala Val Ser Ala Met Val Phe Lys Gln Leu Xaa
        275                 280                 285

Gly Gln Lys Ser Tyr Gly Lys His Leu Pro Glu Phe Ile Tyr His Val
    290                 295                 300

Pro Arg Lys Tyr Lys Leu Leu Met Leu Glu Lys Met Val Glu Gly Asp
305                 310                 315                 320

Gly Ser Arg Ala Xaa Gly Pro Arg Tyr Thr Arg Glu Tyr Arg Asp Arg
            325                 330                 335

Asn Phe Lys Tyr His Thr Ser Ser Leu Arg Leu Ala Ser Gly Leu Ser
        340                 345                 350

Leu Leu Leu Asn Gln Leu Gly Ile Asn His Ser Ile Arg Tyr Tyr Ser
        355                 360                 365

Lys Arg Lys Ser Tyr Xaa Val Thr Thr Xaa Ser Ile Thr Asn Asp Arg
    370                 375                 380

Leu Gly Thr Asp Val Val Glu Glu Pro Tyr Gln Gly Phe Val Tyr Asp
385                 390                 395                 400
```

```
Leu Ser Val Glu Gly Ser Arg Met Phe Thr Asp Ala Xaa Gly Ser Val
            405                 410                 415


Val Leu His Asn
            420
```

<210> 208
<211> 419
<212> PRT
<213> Artificial sequence

<220>
<223> modified contiguous intein from Micrarchaeota archaeon UBA95


<220>
<221> misc_feature
<222> (36)..(36)
<223> Xaa can be any naturally occurring amino acid

<220>
<221> misc_feature
<222> (49)..(49)
<223> Xaa can be any naturally occurring amino acid

<220>
<221> misc_feature
<222> (156)..(156)
<223> Xaa can be any naturally occurring amino acid

<220>
<221> misc_feature
<222> (189)..(189)
<223> Xaa can be any naturally occurring amino acid

<220>
<221> misc_feature
<222> (285)..(285)
<223> Xaa can be any naturally occurring amino acid

<220>
<221> misc_feature
<222> (364)..(364)
<223> Xaa can be any naturally occurring amino acid

<220>
<221> misc_feature
<222> (412)..(412)
<223> Xaa can be any naturally occurring amino acid

<400> 208

```
Ser Val Thr Ser Glu Arg Phe Leu Val Leu Leu Asp Asp Lys Glu Leu
1               5                   10                  15


Val His Val Lys Asn Val Glu Glu Leu Phe Glu Glu Asn Ala Lys His
            20                  25                  30
```

Leu Ile Glu Xaa Gly Glu Lys Gln Val Ile Pro Leu Thr Gly Trp Arg
            35                  40              45

Xaa Leu Ser Val Asn Pro Ala Ser Lys Lys Thr Glu Trp Lys Lys Val
        50              55              60

Thr Glu Leu Ile Arg His Lys Thr Asn Lys Arg Val Tyr Arg Val Asn
65                  70              75              80

Gln Lys Phe Gly Glu Thr Arg Val Thr Glu Asp His Ser Leu Met Ala
                85              90              95

Asp Thr Pro Asn Gly Leu Val Glu Val Lys Pro Val Asn Ala Lys Lys
            100             105             110

His Arg Leu Ala Gln Ala Glu Val Leu Lys Ala Lys Gly Gly Val Glu
        115             120             125

Lys Ile Asp Val Tyr Glu Val Leu Lys Asp Tyr Ser Glu Lys Thr Val
    130             135             140

Tyr Lys Gly Phe Gly Lys Ile Lys Thr Ile Lys Xaa Asn Ser Glu Arg
145             150             155             160

Val Trp Phe Gly Trp Thr Asn Gln Lys Asn Pro Val Lys Val Lys Arg
            165             170             175

Phe Ile Gly Ile Glu Thr Lys Glu Phe Glu Ser Leu Xaa Arg Leu Leu
            180             185             190

Gly Ala Tyr Ala Ala Glu Gly Ser Ser Ser Thr Ile Glu Thr Thr Arg
        195             200             205

Ser Arg Tyr Gly Ala Ser Ile Ala Gly Lys Arg Lys Trp Leu Glu Gly
    210             215             220

Leu Gln Lys Asp Tyr Leu Ala Leu Phe Thr Ala Lys Ala Gly Val Ile
225             230             235             240

Pro Ser Gln Lys Lys Thr Arg His Leu Thr Tyr Arg Thr Gln Lys Gly
            245             250             255

Val Lys Lys Thr Val Val Tyr Lys Asp Asp Thr His Lys Leu Gln Met
        260             265             270

Met Asn Ser Leu Ser Ala Val Phe Phe Lys Met Phe Xaa Gly Gln Lys
        275             280             285

267

```
Ser Ala Gly Lys Lys Leu Pro Asp Phe Ile Tyr Asn Val Pro Lys Lys
    290             295             300

Tyr Gln Leu Ile Phe Leu Lys Lys Leu Leu Glu Gly Asp Gly Ser Arg
305             310             315             320

Ser Val Asn Glu Arg Leu Gly Tyr Ser Ala Glu Tyr Lys Lys Lys Asn
            325             330             335

Phe Lys Tyr Thr Thr Ile Ser Ala Gly Leu Ala Ser Gly Leu Ser Val
            340             345             350

Leu Leu Arg Gln Leu Glu Leu Asn His Ser Ile Xaa Tyr Arg Pro Ser
        355             360             365

Lys Lys Ala Tyr Thr Leu Ser Thr Ser Gly Lys Tyr Asn Lys Arg Ile
        370             375             380

Gln Thr Lys Val Ala Arg Glu Glu Tyr Ser Gly Trp Val Tyr Asp Leu
385             390             395             400

Ser Val Glu Asp Asn His Ala Phe Thr Asp Ala Xaa Gly Gln Ile Val
            405             410             415

Leu His Asn
```

```
<210>  209
<211>  437
<212>  PRT
<213>  Artificial sequence

<220>
<223>  modified contiguous intein from Haloarchaeon species J07HX64

<220>
<221>  misc_feature
<222>  (171)..(171)
<223>  Xaa can be any naturally occurring amino acid

<220>
<221>  misc_feature
<222>  (395)..(395)
<223>  Xaa can be any naturally occurring amino acid

<400>  209
```

```
Ser Val Thr Gly Glu Arg Pro Val Val Val Arg Asp Pro Asp Gly Ile
1               5               10              15

Val Arg Ile Tyr Pro Ile Glu Arg Leu Tyr Gln Arg Ala Thr Gln Ser
```

|  |  | 20 |  |  |  |  | 25 |  |  |  |  | 30 |  |  |
|---|---|---|---|---|---|---|---|---|---|---|---|---|---|---|

Pro Ala Glu Asp Thr Val Ile Thr Val Asp Gly Met Pro Ile Thr Gly
       35             40            45

Ile Glu Ser Ser Lys Glu Tyr Ala Thr Leu Glu Gly Trp Asp Ala Leu
       50             55            60

Ser Val Asp Asp Asp Gly Gln Ser Glu Trp Ser Pro Ile Glu Gln Val
65              70            75            80

Val Arg His Glu Thr Asp Lys Pro Val Ile Lys Leu Gln His Lys Phe
            85            90            95

Gly Glu Ser Val Thr Thr Arg Asp His Ser Tyr Val Val Glu Ser Gly
       100           105          110

Gly Glu Leu Val Glu Ala Pro Pro Asn Glu Val Glu Ser Pro Leu Arg
       115           120          125

Ile Pro Asp Met Pro Glu Thr Asp Glu Ile Glu Thr Ile Asp Val Tyr
   130           135          140

Glu Val Leu Asn Gly Tyr Thr Arg Arg Tyr Glu Asp Gly Arg Gly Ser
145            150          155          160

Gly Gly Val Thr Thr Lys Thr Lys Arg Val Xaa Ala Asp Asp Glu Ala
       165           170          175

Val Trp Phe Gly His Glu His His Arg Gly Leu Asp Lys Thr Val Glu
       180           185          190

Val Gln Arg Tyr Ile Asp Leu Asp Ser Glu Asp Gly Glu Ala Leu Leu
       195           200          205

Arg Leu Leu Gly Ala Tyr Val Pro Glu Gly Ser Ala Ser Thr Val Glu
   210           215          220

Thr Thr Asp Ser Arg Phe Gly Ala Ser Ile Ala Glu Ser Arg Arg Thr
225            230          235          240

Trp Leu Glu Gln Leu Gln Asp Asp Tyr His Arg Leu Phe Glu Asn Thr
       245           250          255

Ser Val Ser Ile Val Ala Ser Asp Thr Arg Asp Glu Arg Thr Val Glu
       260           265          270

His Pro Gly Asp Asp Ser Glu Thr Ala Leu Thr Tyr Asp Asp Gln Thr
        275             280             285

Leu Lys Leu Gln Met Met Asn Glu Leu Ala Ala Val Phe Phe Arg Glu
        290             295             300

Phe Ala Gly Gln Arg Ser Arg Gly Lys Lys Ile Pro Ser Phe Val Phe
305             310             315             320

His Leu Pro Ala Glu Lys Gln Glu Leu Phe Ile Gln Met Leu Val Glu
                325             330             335

Gly Asp Gly Ser Arg Glu Phe Pro Arg Tyr Ser Ala Glu Tyr Ala Glu
                340             345             350

Arg Asn Phe Asp Phe Glu Thr Thr Ser Arg Glu Leu Ala Gly Gly Leu
        355             360             365

Ser Val Leu Leu Thr Gln Arg Gly Thr Lys His Ser Leu Lys Tyr Arg
        370             375             380

Glu Glu Lys Glu Ser Tyr Thr Val Arg Thr Xaa Asp Tyr Tyr Asp Ser
385             390             395             400

Gly Arg Glu Pro Val Leu Thr Glu Val Ala His Asp Gly Tyr Val Tyr
                405             410             415

Asp Leu Ser Val Arg Glu Asn Glu Asn Phe Val Asp Ala Val Gly Gly
        420             425             430

Ile Val Leu His Asn
        435


<210>  210
<211>  403
<212>  PRT
<213>  Artificial sequence

<220>
<223>  modified contiguous intein (partial) from halophilic archaeon
       J07HX5


<220>
<221>  misc_feature
<222>  (315)..(315)
<223>  Xaa can be any naturally occurring amino acid

<220>
<221>  misc_feature
<222>  (403)..(403)
<223>  Xaa can be any naturally occurring amino acid

<400> 210

```
Ser Val Thr Gly Asp Arg Pro Ile Val Ala Arg Thr Pro Asp Gly Leu
1               5                   10                  15

Ile Arg Ile Val Pro Ile Glu Glu Leu Phe Glu Arg Ala Ser Pro Ala
            20                  25                  30

Pro Ser Asp Arg Ala Leu Val Thr Thr Asp Gly Gly Pro Ala Ala Thr
            35                  40                  45

Ala Gly Ser Ala Lys Glu Tyr Arg Ser Leu Asp Gly Trp Asp Ala Leu
        50                  55                  60

Ser Val Asn Asn Arg Gly Met Thr Glu Trp Gln Pro Ile Glu Gln Val
65                  70                  75                  80

Leu Arg His Glu Thr Glu Lys Glu Val Val Arg Leu Gln His Glu Arg
                85                  90                  95

Gly Glu Ser Val Thr Thr Arg Asp His Ser Tyr Val Ile Glu Glu Asn
            100                 105                 110

Gly Glu Leu Ile Glu Ala Pro Pro Glu Asp Val Gly Ser Pro Leu Gln
            115                 120                 125

Ile Pro Asp Val Pro Ala Thr Gln Glu Val Gly Lys Ile Asp Val Tyr
    130                 135                 140

Glu Leu Leu His Gly His Glu Arg Glu Ser Met Asp Arg Gln Gly Thr
145                 150                 155                 160

Asp Ser Thr Thr Ile Val Arg Arg Ile His Ala Asn Asp Asp Arg Val
                165                 170                 175

Trp Phe Gly His Glu His Ala Ser Asn Arg His Glu Gln Thr Val Ser
            180                 185                 190

Val Gln Arg Tyr Ile Asp Leu Asp Ser Glu Ser Gly Asn Ala Leu Val
        195                 200                 205

Arg Leu Leu Gly Ala Tyr Ser Ser Asn Glu Ser Ala Ser Thr Val Glu
    210                 215                 220

Thr Thr Asp Asn Arg Pro Gly Val Ser Ile Ser Ala Ser Asn Arg Arg
225                 230                 235                 240
```

```
Arg Leu Glu Gln Leu Gln Ala Asp Tyr His Arg Leu Phe Glu Asn Thr
            245             250             255

Thr Gly Arg Ile Val Ala Arg Glu Thr Gly Ser Glu Arg Thr Val Ser
            260             265             270

Asp Ala Ser Ser Glu Gly Glu Ala Ala Thr Ala Pro Ala Val Ala Ser
            275             280             285

Val Asp Asp Thr Leu Lys Leu Gln Met Met Asp Glu Leu Ala Ala Val
    290             295             300

Phe Phe Arg Glu Phe Ala Gly Gln Thr Pro Xaa Glu Thr Arg Ile Pro
305             310             315             320

Ser Phe Ile Tyr His Leu Pro Glu Glu Lys Gln Asp Leu Phe Leu Arg
            325             330             335

Met His Leu Gly Asp Asp Gly Ala Arg Thr Phe Pro Gln Ala Ala Glu
            340             345             350

Glu Ser Ala Glu Gln Asp Ile Gln Phe Glu Thr Thr Ser Arg Glu Leu
            355             360             365

Ala Gly Gly Leu Ser Leu Leu Leu Thr Gln Arg Gly Lys Lys His Ser
    370             375             380

Phe Asn Tyr Arg Asn Gln Arg Asn Ser Tyr Thr Ile Gln Thr Gly Glu
385             390             395             400

Tyr Asp Xaa
```

```
<210>  211
<211>  437
<212>  PRT
<213>  Artificial sequence

<220>
<223>  modified contiguous intein from Halorubrum species J07HR59


<220>
<221>  misc_feature
<222>  (298)..(298)
<223>  Xaa can be any naturally occurring amino acid

<400>  211

Ser Val Thr Gly Glu Arg Pro Leu Val Val Arg Asp Gly Asp Gly Arg
1               5               10              15
```

Val Arg Ile Leu Pro Ala Ala Glu Leu Phe Glu Arg Ala Glu Ala Asn
            20                  25                  30

Asp His Ile Ala Ile Ala Ala Asp Gly Gly Pro Ala Gly Ser His Gly
            35                  40                  45

Leu Gly Lys Gly Arg Ala Ser Leu Pro Gly Trp Glu Ala Leu Ser Leu
        50                  55                  60

Ala Ser Asp Gly Thr Ala Glu Trp Gln Pro Ile Glu Glu Val Ile Arg
65                  70                  75                  80

His Asp Thr Asp Gly Thr Val Val His Leu Gln His Gln Leu Gly Glu
                85                  90                  95

Ser Thr Thr Thr Arg Asp His Ser Tyr Val Val Glu Glu Asp Gly Glu
            100                 105                 110

Tyr Ile Glu Arg Ala Pro Glu Asp Val Thr Asp Pro Leu Arg Ile Pro
        115                 120                 125

Asp Val Pro Ser Val Asp Thr Val Asp Ser Ile Asp Val His Glu Ile
    130                 135                 140

Leu Asp Gly Tyr Thr Gly Glu His Ala Asp Arg Thr Thr Pro Arg Gly
145                 150                 155                 160

Glu Thr Gly Ser Gln Lys Arg Pro Arg Val His Thr Asp Gly Glu Ser
                165                 170                 175

Val Trp Val Gly His Ser Arg Glu Gly Glu Ser Glu Asn Thr Thr Lys
            180                 185                 190

Val Gln Arg Glu Ile Asp Leu Thr Gly Pro Thr Gly Arg Ser Leu Val
        195                 200                 205

Arg Leu Leu Ala Ala Tyr Ile Arg Asp Gly Ser Thr Thr Thr Ala Glu
    210                 215                 220

Thr Ala Asp Ser Thr Val Gly Val Ser Ile Ser Glu Ser Arg Pro Glu
225                 230                 235                 240

Trp Leu Glu Thr Ile Ala Thr Asp Ser Glu Arg Leu Phe Glu Asn Ala
            245                 250                 255

Gln Val Ser Val Asn Ala Gly Gly Thr Asp Asp Glu Gln Thr Leu Val
        260                 265                 270

273

```
        Ser Glu Thr Arg Thr Asp Glu Thr Val Ser Ser Asn Gly Gly Gly Thr
            275                 280                 285

        Gly Glu Leu Arg Met Met Asp Glu Leu Xaa Ala Val Phe Phe Thr Glu
            290                 295                 300

        Phe Val Gly His Thr Ala Pro Glu Lys His Ile Pro Ser Phe Val Tyr
        305                 310                 315                 320

        His Leu Asn Asp Glu Leu Gln Arg Val Phe Thr Glu Thr Leu Val Ala
                        325                 330                 335

        Val Asp Asp Leu Ala Glu Leu Ala Ser His Thr Glu Ala His Ala Gln
                        340                 345                 350

        Arg Gln Met Asn Phe Glu Ala Thr Ser Arg Glu Leu Ala Ala Gly Val
                355                 360                 365

        Ser Met Leu Leu Thr Gln Gln Asp Arg Thr His Ser Leu His Tyr Arg
                370                 375                 380

        Asp Glu Thr Asn Arg Tyr Thr Ile Thr Pro Asp Glu Ser Asp Gln Ser
        385                 390                 395                 400

        Ala Arg Asp Pro Val Val Thr Glu Gln Glu His Asp Gly Tyr Val Tyr
                        405                 410                 415

        Asp Leu Ser Val Ala Glu Asn Glu Asn Phe Val Asp Ala Val Gly Gly
                        420                 425                 430

        Ile Val Leu His Asn
                        435


        <210>  212
        <211>  437
        <212>  PRT
        <213>  Artificial sequence

        <220>
        <223>  modified contiguous intein from Archaeon species A07HR60


        <220>
        <221>  misc_feature
        <222>  (298)..(298)
        <223>  Xaa can be any naturally occurring amino acid

        <400>  212

        Ser Val Thr Gly Glu Arg Pro Leu Val Val Arg Asp Gly Asp Gly Arg
```

```
1               5                       10                      15

Val Arg Ile Leu Pro Ala Ala Glu Leu Phe Glu Arg Ala Glu Ala Asn
            20                      25                  30

Asp His Ile Ala Ile Ala Ala Asp Gly Gly Pro Ala Gly Ser His Gly
            35                      40                  45

Leu Gly Lys Gly Arg Ala Ser Leu Pro Gly Trp Glu Ala Leu Ser Leu
            50                      55                  60

Ala Ser Asp Gly Thr Ala Glu Trp Gln Pro Ile Glu Glu Val Ile Arg
65                      70                  75                  80

His Asp Thr Asp Gly Thr Val Val His Leu Gln His Gln Leu Gly Glu
            85                      90                  95

Ser Thr Thr Thr Arg Asp His Ser Tyr Val Val Glu Glu Asp Gly Glu
            100                     105                 110

Tyr Leu Glu Arg Ala Pro Glu Asp Val Thr Asp Pro Leu Arg Ile Pro
            115                     120                 125

Asp Val Pro Ser Val Asp Thr Val Asp Ser Ile Asp Val His Glu Ile
            130                     135                 140

Leu Asp Gly Tyr Thr Gly Glu His Ala Asp Arg Thr Thr Pro Arg Gly
145                     150                 155                 160

Glu Thr Gly Ser Gln Lys Arg Pro Arg Val His Thr Asp Gly Glu Ser
            165                     170                 175

Val Trp Val Gly His Ser Arg Glu Gly Glu Ser Glu Asn Thr Thr Lys
            180                     185                 190

Val Gln Arg Glu Ile Asp Leu Thr Gly Pro Thr Gly Arg Ser Leu Val
            195                     200                 205

Arg Leu Leu Ala Ala Tyr Ile Arg Asp Gly Ser Thr Thr Thr Ala Glu
            210                     215                 220

Thr Ala Asp Ser Thr Val Gly Val Ser Ile Ser Glu Ser Arg Pro Glu
225                     230                 235                 240

Trp Leu Glu Ala Ile Ala Thr Asp Ser Glu Arg Leu Phe Glu Asn Ala
            245                     250                 255
```

275

```
Gln Val Ser Val Asn Ala Gly Gly Thr Asn Asp Glu Gln Thr Leu Val
            260                 265                 270

Ser Glu Thr Arg Thr Asp Glu Thr Val Ser Ser Asn Gly Gly Gly Thr
            275                 280                 285

Gly Glu Leu Arg Met Met Asp Glu Leu Xaa Ala Val Phe Phe Thr Glu
        290                 295                 300

Phe Val Gly His Thr Ala Pro Glu Lys His Ile Pro Ser Phe Val Tyr
305                 310                 315                 320

His Leu Asn Asp Glu Leu Gln Arg Val Phe Thr Glu Thr Leu Val Ala
                325                 330                 335

Val Asp Asp Leu Ala Glu Leu Ala Ser His Thr Glu Ala His Ala Gln
            340                 345                 350

Arg Gln Met Asn Phe Glu Ala Thr Ser Arg Glu Leu Ala Ala Gly Val
            355                 360                 365

Ser Met Leu Leu Thr Gln Gln Asp Arg Thr His Ser Leu His Tyr Arg
            370                 375                 380

Asp Glu Thr Asn Arg Tyr Thr Ile Thr Pro Asp Glu Ser Asp Gln Ser
385                 390                 395                 400

Ala Arg Asp Pro Val Val Thr Glu Gln Glu His Asp Gly Tyr Val Tyr
                405                 410                 415

Asp Leu Ser Val Ala Glu Asn Glu Asn Phe Val Asp Ala Val Gly Gly
            420                 425                 430

Ile Val Leu His Asn
            435


<210>  213
<211>  439
<212>  PRT
<213>  Artificial sequence

<220>
<223>  modified contiguous intein from Halonotius species J07HN6


<220>
<221>  misc_feature
<222>  (397)..(397)
<223>  Xaa can be any naturally occurring amino acid

<400>  213
```

```
Ser Val Thr Gly Asp Arg Pro Val Val Val Arg Asp Pro Asp Gly Ile
1               5                   10                  15

Ile Arg Val Leu Pro Ile Glu Asn Leu Phe Glu Arg Ala Thr Ser Ser
            20                  25                  30

Thr Ser Asp Thr Val Val Ile Thr Ala Asp Gly Gly Ala Val Gly Ser
        35                  40                  45

Ala Ser Ala Gly Lys Asp Arg Arg Arg Leu Asp Gly Trp Glu Ala Leu
    50                  55                  60

Ser Leu Ala Ala Asp Gly Glu Pro Glu Trp Gln Pro Ile Gln Gln Ala
65                  70                  75                  80

Ile Arg His Asp Thr Asp Lys Pro Val Val Asn Leu Gln His Lys Phe
            85                  90                  95

Gly Glu Ser Thr Thr Thr Arg Asp His Ser Tyr Val Val Gly Asp Asp
            100                 105                 110

Gly Lys Phe Ala Glu Ala Thr Pro Asp Asp Val Asp Glu Pro Leu Arg
        115                 120                 125

Ile Pro Gly Val Pro Ala Val Asp Thr Ile Glu Arg Ile Asp Val Tyr
    130                 135                 140

Glu Val Leu Asp Gly Tyr Thr Arg Glu Tyr Glu Asp Gly Arg Ser Val
145                 150                 155                 160

Gly Ser Ala Asn Ala Thr Ser Lys Thr Lys Arg Val His Ala Asn Asp
            165                 170                 175

Glu Trp Val Trp Phe Gly His Asp His His Asn Glu Leu Ser Lys Pro
            180                 185                 190

Val Lys Val Gln Arg Tyr Ile Asp Ile Asp Ser Glu Asp Gly Ala Ala
        195                 200                 205

Leu Leu Arg Leu Leu Ala Ala Tyr Ile Thr Glu Gly Ser Ala Ser Thr
    210                 215                 220

Ile Glu Thr Thr Glu Ser Arg Phe Gly Ala Ser Ile Ser Glu Ser Arg
225                 230                 235                 240

Glu Glu Trp Leu Asp Gly Leu Gln Ser Asp Tyr Tyr Arg Leu Phe Glu
            245                 250                 255
```

```
Asn Thr Thr Ala Ser Val Ile Ala Ser Asp Ser Ser Gly Asp Arg Thr
            260                 265             270

Val Glu Tyr Asp Thr Ser Asp Gly Ala Gln Ser Val Thr Tyr Asp Asp
            275                 280             285

Thr Thr His Lys Leu Gln Leu Met Asn Glu Leu Ser Ala Val Phe Phe
            290                 295             300

Arg Glu Phe Ala Gly Gln Thr Ser Arg Gly Lys Arg Ile Pro Gly Leu
305             310             315             320

Val Phe Asn Leu Pro Ala Asp Ala Gln Asp Leu Phe Leu Asp Thr Leu
            325                 330             335

Ile Glu Gly Asp Gly Ser Arg Glu Phe Pro Arg Tyr Thr Asp Ala Tyr
            340                 345             350

Ser Glu Arg His Phe Asp Phe Glu Thr Thr Ser Arg Glu Leu Ala Ala
            355                 360             365

Gly Leu Ser Met Leu Leu Thr Gln Arg Glu Gln Lys His Ser Leu Lys
            370                 375             380

Tyr Arg Asp Thr Lys Asn Ser Tyr Thr Ile Arg Thr Xaa Asp Ser Tyr
385             390             395             400

Arg Ser Gly Arg Asp Pro Val Leu Thr Glu Val Asp His Asp Gly Tyr
            405                 410             415

Val Tyr Asp Leu Ser Val Ala Asp Asn Asp Asn Phe Val Asp Ala Val
            420                 425             430

Gly Gly Val Val Leu His Asn
            435
```

<210> 214
<211> 282
<212> PRT
<213> Artificial sequence

<220>
<223> modified contiguous intein (partial) from Halonotius species J07HN4

<220>
<221> misc_feature
<222> (237)..(237)

<223>   Xaa can be any naturally occurring amino acid

<220>
<221>   misc_feature
<222>   (282)..(282)
<223>   Xaa can be any naturally occurring amino acid

<400>   214

Ser Val Thr Gly Asp Arg Pro Ile Val Val Arg Asp Pro Asp Gly Ile
1               5                   10                  15

Val Arg Val Leu Pro Ile Glu Asn Leu Phe Glu Arg Ala Thr Ser Ser
            20                  25                  30

Thr Gly Asp Thr Val Val Ile Thr Ala Asp Gly Gly Ala Val Gly Ser
        35                  40                  45

Thr Thr Thr Gly Lys Asp Arg Arg Gln Leu Ala Asp Trp Glu Ala Leu
    50                  55                  60

Ser Leu Ser Ala Asp Gly Thr Pro Glu Trp Gln Pro Ile Gln Gln Ala
65                  70                  75                  80

Ile Arg His Glu Val Asp Lys Pro Val Ile Asn Leu Gln His Lys Phe
                85                  90                  95

Gly Glu Ser Thr Thr Thr Arg Asp His Ser Tyr Val Val Gly Asp Asp
            100                 105                 110

Gly Glu Leu Val Glu Ala Thr Pro Asp Asp Val Asp Glu Pro Leu Arg
        115                 120                 125

Ile Pro Gly Met Pro Ala Val Asp Thr Val Glu Thr Ile Asp Val Tyr
    130                 135                 140

Glu Ile Leu Asp Gly Tyr Thr Arg Glu Tyr Glu Asp Gly Arg Ser Val
145                 150                 155                 160

Gly Ser Glu Asp Ala Ala Thr Lys Thr Lys Arg Val His Ala Asn Asn
                165                 170                 175

Glu Trp Val Trp Phe Gly His Asp His His Asn Glu Leu Ser Lys Pro
            180                 185                 190

Val Lys Val Gln Arg Tyr Ile Asp Ile Asp Ser Glu Asp Gly Ala Ala
        195                 200                 205

Leu Leu Arg Leu Leu Ala Ala Tyr Ile Thr Glu Gly Ser Ala Ser Thr
    210                 215                 220

```
Ile Glu Thr Thr Glu Ser Arg Phe Gly Ala Ser Ile Xaa Glu Ser Arg
225                 230                 235                 240


Glu Glu Trp Leu His Gly Leu Gln Ser Asn Tyr Tyr Arg Leu Phe Glu
                245                 250                 255


Asn Thr Thr Ala Ser Val Ile Ala Ser Asp Ser Ser Gly Glu Arg Thr
                260                 265                 270


Val Glu Tyr Glu Thr Asn Asn Gly Met Xaa
            275                 280
```

```
<210>  215
<211>  435
<212>  PRT
<213>  Artificial sequence

<220>
<223>  modified contiguous intein from Haloquadratum walsbyi J07HQW1


<220>
<221>  misc_feature
<222>  (250)..(250)
<223>  Xaa can be any naturally occurring amino acid

<220>
<221>  misc_feature
<222>  (282)..(282)
<223>  Xaa can be any naturally occurring amino acid

<220>
<221>  misc_feature
<222>  (302)..(302)
<223>  Xaa can be any naturally occurring amino acid

<220>
<221>  misc_feature
<222>  (324)..(324)
<223>  Xaa can be any naturally occurring amino acid

<220>
<221>  misc_feature
<222>  (400)..(400)
<223>  Xaa can be any naturally occurring amino acid

<400>  215
```

```
Ser Val Thr Gly Asp Arg Pro Val Val Val Arg Asp Pro Arg Gly Tyr
1               5                   10                  15


Leu Arg Ile Ile Pro Ile Gly Thr Leu Phe Glu Gln Ala Thr Val Pro
                20                  25                  30


Glu Gln Asn Ala Arg Leu Thr Ala Asp Gly Ala Pro Thr Gly Ser Ser
```

                    35                          40                          45

Gly Phe Pro Lys Glu Arg Arg His Leu Ser Gln Trp Glu Ala Leu Ser
        50              55              60

Leu Thr Asp Ala Gly Glu Thr Glu Trp Gln Pro Ile Lys Gln Ile Ile
65              70              75              80

Arg His Gln Thr Glu Lys Glu Val Ile Thr Leu Gln His Glu His Gly
            85              90              95

Glu Ser Thr Thr Thr Arg Asp His Ser Tyr Ile Thr Asp Asp Asn Gly
        100             105             110

Glu Tyr Val Glu Thr Pro Pro Glu Asp Val Asp Glu Pro Leu Pro Ile
        115             120             125

Pro Glu Ile Ala Pro Ile Lys Thr Ile Glu Thr Ile Asp Ile Tyr Gln
    130             135             140

Thr Leu Thr Thr Ala Ala His Thr His Thr Gly Ser Asp Ile Glu Thr
145             150             155             160

Ser Glu Arg Leu Pro Ser Thr Asp His Ile His Ala Thr Asp Glu Tyr
            165             170             175

Val Trp Ile Asp Thr Thr Pro Glu Gly Gln Asp Lys His Asp Ser Ile
        180             185             190

Pro Ala Ile Pro Arg Tyr Ile Asp Leu Thr Ser Asp Thr Gly His Ala
        195             200             205

Leu Ile Arg Phe Leu Gly Val Tyr Leu Ser Asp Trp Ser Glu Ser Thr
    210             215             220

Val Thr Ile Ser Glu Gln Glu Gln Gln Ala Gln Gln Leu Asp Ile Thr
225             230             235             240

Gly Pro His Glu Ser Ala Leu Arg Thr Xaa Thr Ala Asp Ala Asp Gln
            245             250             255

Leu Phe Ile Asn Val Thr Pro Thr Val Thr Ala Asn Ala Glu Asn Asn
        260             265             270

Ala Asn Ala Val Asp Gly Glu Tyr Ser Xaa His Ile Pro Thr Thr Leu
        275             280             285

```
Ala Thr Thr Leu Val Ser Ala Leu Ala Gly His Pro Ala Xaa Val Lys
    290                 295             300

Gln Val Pro Ser Val Ile Tyr His Leu Pro Asp Ala Glu Gln Ser Arg
305                 310             315                 320

Phe Val Gln Xaa Leu Ile Gly Ala Glu Ser Lys Leu Thr Ser Gly Thr
                325             330             335

Leu Leu Asn Asp Ser Gln Asn Ile Asn Asp Pro Ile Ser Ser Thr Gly
            340             345             350

Glu Phe Thr Ala Asn Arg Glu Leu Ala Ala Gly Val Ser Met Leu Leu
        355             360             365

Thr Gln Arg Glu Gln Ser Tyr Ser Ile Val Gln Gln Asp Ala Glu Asp
    370             375             380

Thr Tyr Thr Ile Arg Ile Asn Asp Thr Glu Ser Ser Ser Ser Glu Xaa
385             390             395                 400

His Pro Thr Leu Thr Glu Thr Ala His Ser Gly Tyr Val Tyr Asp Leu
            405             410             415

Ser Val Glu Ala Asn Gln Asn Phe Val Asp Gly Leu Gly Gly Leu Val
            420             425             430

Leu His Asn
        435
```

```
<210>  216
<211>  425
<212>  PRT
<213>  Artificial sequence

<220>
<223>  modified contiguous intein from Haloquadratum walsbyi J07HQW2

<220>
<221>  misc_feature
<222>  (169)..(169)
<223>  Xaa can be any naturally occurring amino acid

<220>
<221>  misc_feature
<222>  (176)..(176)
<223>  Xaa can be any naturally occurring amino acid

<220>
<221>  misc_feature
<222>  (214)..(214)
<223>  Xaa can be any naturally occurring amino acid
```

```
<220>
<221>  misc_feature
<222>  (245)..(245)
<223>  Xaa can be any naturally occurring amino acid

<220>
<221>  misc_feature
<222>  (277)..(277)
<223>  Xaa can be any naturally occurring amino acid

<220>
<221>  misc_feature
<222>  (331)..(331)
<223>  Xaa can be any naturally occurring amino acid

<220>
<221>  misc_feature
<222>  (363)..(363)
<223>  Xaa can be any naturally occurring amino acid

<220>
<221>  misc_feature
<222>  (390)..(390)
<223>  Xaa can be any naturally occurring amino acid

<400>  216
```

Ser Val Thr Gly Asp Arg Pro Val Val Val Arg Asp Pro Thr Gly Arg
1               5               10                  15

Ile Gln Ile Val Pro Ile Glu Thr Leu Phe Glu Leu Ala Thr Ala Pro
            20              25                  30

Lys Arg Asn Thr Arg Ile Thr Ala Asp Gly Ala Pro Thr Gly Ser Ser
        35                  40                  45

Gly Leu Pro Lys Glu Arg Arg His Leu Asp Gln Trp Glu Ala Leu Ser
    50                  55                  60

Leu Ser Asp Thr Gly Glu Thr Glu Trp Gln Ser Ile Lys Gln Ile Ile
65                  70                  75                  80

Arg His Gln Thr Asp Lys Glu Ile Leu Thr Leu Gln His Glu Asp Gly
                85                  90                  95

Glu Ser Thr Thr Thr Arg Asp His Ser Tyr Ile Thr Ala Asp Asp Gly
            100                 105                 110

Glu Tyr Val Glu Thr Ser Pro Lys Asn Val Asp Glu Pro Leu Ser Ile
        115                 120                 125

Pro Glu Ile Ala Pro Val Lys Thr Ile Glu Thr Ile Asp Ile Tyr Gln
    130                 135                 140

```
Ile Leu Thr Ala Asn Thr Gln Thr His Ala Gly Ser Asn Ile Asp Pro
145                 150                 155                 160

Gly Glu Trp Leu Pro Ser Thr Asp Xaa Ile His Ala Asn Asp Glu Xaa
                165                 170                 175

Val Trp Ile Asn Pro Thr Gly Glu Glu Arg Glu Asp Ser Thr Pro Thr
                180                 185                 190

Ile Gln Arg His Ile Asp Leu Thr Ser Asp Ala Gly His Ala Leu Ile
            195                 200                 205

Arg Phe Leu Ala Val Xaa Leu Ser Ser Arg Ser Lys Ser Thr Val Arg
            210                 215                 220

Thr Ile Glu Ser Lys Gln Tyr Leu Gln Ile Ile Gly Pro His Lys Ser
225                 230                 235                 240

Glu Ile Lys Thr Xaa Lys Val Ala Ile Asp Gln Leu Phe Thr Asn Val
                245                 250                 255

Thr Thr Arg Ile Ala Gly Asp Ala Glu Asn Asn Thr Asn Thr Gly Asp
                260                 265                 270

Ser Thr Phe Arg Xaa His Ile Ser Thr Thr Leu Ala Ala Thr Val Met
            275                 280                 285

Thr Ala Phe Val Gly His Pro Ala Glu Asn Asn Gln Leu Pro Ser Val
        290                 295                 300

Val Tyr His Leu Pro Asp Ala Glu Gln Ser Tyr Phe Ile Gln Gln Leu
305                 310                 315                 320

Ile Arg Pro Lys Ser Asn Glu Leu Leu Ser Xaa Pro Gln Asn Ile Asp
                325                 330                 335

Asp Ser Ile Ser Ile Glu Ser Asp Phe Thr Thr Ser Ser Arg Glu Leu
            340                 345                 350

Ala Ala Gly Val Ser Met Leu Leu Thr Gln Xaa Gly Gln Ser Tyr Ser
            355                 360                 365

Ile Leu Gln Arg Gly Ser Glu Asp Val Tyr Thr Ile Gln Val Gly Asp
        370                 375                 380

Pro Pro Ser Ser Glu Xaa Glu Pro Met Leu Ile Glu Thr Ala Asp Ser
385                 390                 395                 400
```

Gly Tyr Val Tyr Asp Leu Ser Val Glu Ala Asn Gln Asn Phe Val Asp
                405                 410                 415

Gly Leu Gly Gly Leu Val Leu His Asn
            420                 425

<210>  217
<211>  439
<212>  PRT
<213>  Artificial sequence

<220>
<223>  modified contiguous intein from Halopenitus species DYS4

<220>
<221>  misc_feature
<222>  (353)..(353)
<223>  Xaa can be any naturally occurring amino acid

<220>
<221>  misc_feature
<222>  (397)..(397)
<223>  Xaa can be any naturally occurring amino acid

<400>  217

Ser Val Thr Gly Asp Arg Pro Val Val Arg Asp Pro Asp Gly Val
1               5                   10                  15

Val Arg Val Val Pro Ile Glu Arg Leu Phe Glu Arg Ala Glu Met Glu
            20                  25                  30

Arg Gly Glu Glu Leu Leu Val Thr Ala Asp Gly Gly Pro Val Ala Ser
            35                  40                  45

Val Ala Ala Gly Lys Glu Arg Arg Asn Leu Ser Glu Trp Glu Ala Leu
        50                  55                  60

Ser Val Ser Glu Gly Gly Val Pro Glu Trp Gln Pro Ile Glu Glu Ile
65                  70                  75                  80

Val Arg His Glu Thr Asp Lys Asp Ile Val Asn Leu Gln His Lys Phe
                85                  90                  95

Gly Glu Ser Thr Thr Thr Thr Asp His Ser Tyr Val Val Glu Asp Gly
            100                 105                 110

Asp Asp Leu Val Glu Thr Lys Pro Glu Asp Val Thr Ala Pro Leu Arg
            115                 120                 125

```
Ile Pro Gly Leu Pro Glu Val Asp Thr Val Asp Arg Ile Asp Val Tyr
    130                 135                 140

Glu Val Leu Asp Gly Tyr Thr Arg Ser Tyr Glu Asp Gly Arg Ser Val
145                 150                 155                 160

Gly Ser Asp Asn Ala Glu Thr Lys Ile Asn Arg Val His Ala Asn Glu
                165                 170                 175

Glu Tyr Val Trp Phe Gly His Glu His Gln Ala Asp Gln Ser Arg Thr
            180                 185                 190

Val Lys Val Lys Arg His Ile Asp Leu Glu Gly Ala Asp Gly Glu Ala
            195                 200                 205

Leu Val Arg Leu Leu Ala Ala Tyr Val Thr Glu Gly Ser Ala Ser Thr
    210                 215                 220

Ile Glu Thr Thr Asp Ser Arg Phe Gly Ala Ser Ile Ala Glu Ala Arg
225                 230                 235                 240

Thr Asp Trp Leu Asp Gly Leu Lys Glu Asp Tyr Asp Arg Leu Phe Glu
            245                 250                 255

Gly Val Thr Ala Thr Val Ile Ala Ser Asp Thr Ser Ala Glu Arg Thr
            260                 265                 270

Val Glu Tyr Glu Thr Asp Glu Gly Pro Ser Ser Thr Thr Tyr Asn Asp
    275                 280                 285

Gly Thr His Lys Leu Gln Met Met Asn Glu Leu Thr Ala Val Phe Phe
    290                 295                 300

Arg Glu Phe Ala Gly Gln Thr Ser Arg Gly Lys Arg Ile Pro Gly Phe
305                 310                 315                 320

Val Phe Asn Leu Asp Glu Asp Leu Gln Asn Leu Phe Leu Asp Val Leu
            325                 330                 335

Ile Glu Gly Asp Gly Ser Arg Glu Phe Pro Arg Tyr Ser Glu Glu Tyr
            340                 345                 350

Xaa Glu Arg Asn Phe Asp Phe Glu Thr Thr Ser Arg Glu Leu Ala Ala
    355                 360                 365

Gly Leu Ser Thr Leu Leu Thr Gln Arg Gly Lys Lys His Ser Leu Lys
    370                 375                 380
```

```
Tyr Arg Asp Ser Lys Gly Ser Tyr Thr Val Arg Thr Xaa Glu Phe Tyr
385             390             395             400


Arg Gly Gly Arg Asp Pro Val Ile Lys Asp Val Asp His Asp Gly Tyr
            405             410             415


Val Tyr Asp Leu Ser Val Ala Glu Asn Glu Asn Phe Val Asp Gly Val
        420             425             430


Gly Gly Ile Val Leu His Asn
        435
```

```
<210>  218
<211>  402
<212>  PRT
<213>  Artificial sequence

<220>
<223>  modified contiguous intein from Pandoravirus salinus


<220>
<221>  misc_feature
<222>  (52)..(52)
<223>  Xaa can be any naturally occurring amino acid

<220>
<221>  misc_feature
<222>  (89)..(89)
<223>  Xaa can be any naturally occurring amino acid

<220>
<221>  misc_feature
<222>  (127)..(127)
<223>  Xaa can be any naturally occurring amino acid

<220>
<221>  misc_feature
<222>  (189)..(189)
<223>  Xaa can be any naturally occurring amino acid

<220>
<221>  misc_feature
<222>  (197)..(197)
<223>  Xaa can be any naturally occurring amino acid

<220>
<221>  misc_feature
<222>  (201)..(201)
<223>  Xaa can be any naturally occurring amino acid

<220>
<221>  misc_feature
<222>  (234)..(234)
<223>  Xaa can be any naturally occurring amino acid

<220>
<221>  misc_feature
```

```
<222>   (305)..(305)
<223>   Xaa can be any naturally occurring amino acid

<220>
<221>   misc_feature
<222>   (309)..(309)
<223>   Xaa can be any naturally occurring amino acid

<220>
<221>   misc_feature
<222>   (327)..(327)
<223>   Xaa can be any naturally occurring amino acid

<220>
<221>   misc_feature
<222>   (336)..(336)
<223>   Xaa can be any naturally occurring amino acid

<220>
<221>   misc_feature
<222>   (349)..(349)
<223>   Xaa can be any naturally occurring amino acid

<400>   218
```

```
Ser Val Gly Ala Asp Thr Pro Leu Leu Leu Arg Phe Asp Gly Lys His
1               5               10                  15


Val Asp Tyr Val Arg Ala Asp Gln Val Asp Gly Ile Leu Ala Arg Gly
            20                  25                  30


Asp Thr Ser Ala Ala Ala Ser Leu Trp Ser Ala Tyr Gln Gly Asp Lys
            35                  40                  45


Glu Ala Phe Xaa Leu Ala Arg Pro Val Glu Val Trp Thr Glu Arg Gly
        50                  55                  60


Trp Thr Ala Val Asn Arg Val Ile Arg His Arg Ala Gly Lys Lys Met
65                  70                  75                  80


Phe Arg Val Leu Thr His Thr Gly Xaa Val Asp Val Thr Glu Asp His
                85                  90                  95


Ser Leu Leu Asp Pro Asn Ala Glu Lys Val Lys Pro Thr Glu Val Ser
            100                 105                 110


Val Gly Ser Ala Leu Leu His Ala Asp Leu Pro Thr His Glu Xaa Ala
            115                 120                 125


Thr Ala Ser Leu Lys Ser Arg Val Pro Met Ser Thr Gln Asp Ile Thr
            130                 135                 140


Asp Asp Asp Gly Asp Asp Asp Ser Ala Lys Val Ser Ala Ala Ser Ser
145                 150                 155                 160
```

Thr Ser Ala Asp Gly Ser Val Leu Val Ser Ala Ala Asp Lys Ala His
165             170             175

Ala Trp Ala Met Gly Met Phe Phe Ala Glu Gly Ser Xaa Asn Glu His
180             185             190

Pro Arg Asp Asn Xaa Thr Gln Tyr Xaa Trp Arg Ile Ala Asn Lys Asp
195             200             205

Met Ala Leu Val Arg Met Ala Phe Asp Gly Leu Glu Gly Arg Tyr Pro
210             215             220

Gly Val Thr Phe Ser Ile Ser Gly Pro Xaa Thr Asp Gly Met Ala Tyr
225             230             235             240

Val Val Ala Asn Gly Pro Gly Lys Met Gly Leu Val Ala Asn Tyr Arg
245             250             255

Thr Ala Phe Tyr Asp Pro Val Tyr Ala Leu Lys Arg Val Pro Thr Glu
260             265             270

Ile Leu Asn Ala His Val Glu Ile Lys Arg Ala Phe Ile Arg Gly Tyr
275             280             285

Phe Ala Gly Asp Gly Asn Lys Lys Leu Tyr Gly Ala Asp Gly Thr Tyr
290             295             300

Xaa Gly Ser Arg Xaa Gly Gly Lys Gly Lys Ile Gly Met Ala Gly Ile
305             310             315             320

Tyr Tyr Leu Leu Ser Ala Xaa Gly Tyr Leu Ala Ser Val Asp Thr Xaa
325             330             335

Gly Gly Pro Glu Arg Asp Thr Tyr Arg Ile Asn Phe Xaa Asp Ala Ala
340             345             350

Thr Ala Arg Arg Thr Gln Arg Lys Pro Ala Asp Thr Val Lys Lys Ile
355             360             365

Ile Pro Leu Asp Ser Ala Ala Phe Asp Gly Ala Tyr Val Tyr Asp Leu
370             375             380

Glu Thr Ala Asn His His Phe Ala Ala Gly Ile Gly Arg Leu Val Val
385             390             395             400

His Asn

```
<210>  219
<211>  395
<212>  PRT
<213>  Artificial sequence

<220>
<223>  modified contiguous intein from Pandoravirus inopinatum KlaHel


<220>
<221>  misc_feature
<222>  (52)..(52)
<223>  Xaa can be any naturally occurring amino acid

<220>
<221>  misc_feature
<222>  (89)..(89)
<223>  Xaa can be any naturally occurring amino acid

<220>
<221>  misc_feature
<222>  (127)..(127)
<223>  Xaa can be any naturally occurring amino acid

<220>
<221>  misc_feature
<222>  (189)..(189)
<223>  Xaa can be any naturally occurring amino acid

<220>
<221>  misc_feature
<222>  (201)..(201)
<223>  Xaa can be any naturally occurring amino acid

<220>
<221>  misc_feature
<222>  (298)..(298)
<223>  Xaa can be any naturally occurring amino acid

<220>
<221>  misc_feature
<222>  (302)..(302)
<223>  Xaa can be any naturally occurring amino acid

<220>
<221>  misc_feature
<222>  (320)..(320)
<223>  Xaa can be any naturally occurring amino acid

<220>
<221>  misc_feature
<222>  (329)..(329)
<223>  Xaa can be any naturally occurring amino acid

<220>
<221>  misc_feature
<222>  (342)..(342)
<223>  Xaa can be any naturally occurring amino acid

<400>  219
```

```
Ser Val Gly Ala Asp Thr Pro Leu Leu Leu Arg Phe Asp Gly Lys His
1               5                   10                  15


Ile Asp Tyr Val Arg Ala Asp Gln Val Asp Asp Val Leu Ala Arg Gly
            20                  25                  30


Asp Ala Ser Ala Ala Ala Arg Leu Trp Ser Ala Tyr Gln Gly Asp Lys
        35                  40                  45


Glu Ala Phe Xaa Pro Ala Arg Pro Val Glu Val Trp Thr Glu Arg Gly
    50                  55                  60


Trp Thr Ala Val Asn Arg Val Ile Arg His Arg Ala Gly Lys Lys Met
65                  70                  75                  80


Phe Arg Val Leu Thr His Thr Gly Xaa Val Asp Val Thr Glu Asp His
            85                  90                  95


Ser Leu Leu Asp Pro Asn Ala Glu Lys Val Lys Pro Thr Glu Val Thr
        100                 105                 110


Val Gly Ser Ala Leu Leu His Ala Asp Leu Pro Ala Tyr Glu Xaa Ile
        115                 120                 125


Thr Ala Ser Leu Lys Ser His Val Pro Ala Leu Thr Gln Asp Lys Thr
    130                 135                 140


Asp Asp Asn Gly Asp Asp Asp Gly Thr Thr Arg Val Ser Thr Thr Leu
145                 150                 155                 160


Ser Ala Thr Ala Asp Ser Ile Leu Val Thr Ala Ala Asp Lys Ala His
            165                 170                 175


Ala Trp Ala Met Gly Met Phe Phe Ala Glu Gly Ser Xaa Asn Gly Tyr
            180                 185                 190


Pro Arg Gly Asn Leu Gln Pro Tyr Xaa Trp Arg Ile Ala Asn Lys Asp
        195                 200                 205


Met Val Leu Met Arg Met Ala Leu Asp Gly Leu Glu Gly Arg Tyr Pro
    210                 215                 220


Asp Val Thr Phe Ser Ile Asn Gly Pro Tyr Ala Asp Gly Met Ala Tyr
225                 230                 235                 240


Val Val Ala Asn Gly Thr Gly Lys Met Gly Leu Val Ala Asn Tyr Arg
            245                 250                 255
```

```
Ala Ala Phe Tyr Asp Pro Val His Ala Leu Lys Arg Val Pro Thr Glu
        260             265             270

Ile Leu Asn Ala His Val Glu Ile Lys Arg Ala Phe Ile Arg Gly Tyr
        275             280             285

Phe Ala Gly Asp Gly Asn Lys Thr Asp Xaa Gly Ser Arg Xaa Asp Gly
        290             295             300

Arg Gly Lys Ile Gly Met Ala Gly Ile Tyr Tyr Leu Leu Ser Ala Xaa
305             310             315             320

Gly Tyr Leu Ala Ser Ile Asn Thr Xaa Gly Gly Pro Glu Arg Asp Thr
        325             330             335

Tyr Arg Ile Asn Phe Xaa Asp Ala Ala Thr Ala Lys Arg Thr Gln Arg
        340             345             350

Lys Pro Arg Asp Ala Ile Lys Lys Ile Ile Pro Leu Asp Ala Glu Ala
        355             360             365

Phe Asp Gly Ala Tyr Val Tyr Asp Leu Glu Thr Ala Asn His His Phe
    370             375             380

Ala Ala Gly Val Gly Arg Leu Val Val His Asn
385             390             395
```

```
<210>  220
<211>  384
<212>  PRT
<213>  Artificial sequence

<220>
<223>  modified contiguous intein from Pandoravirus macleodensis
       macleodensis


<220>
<221>  misc_feature
<222>  (52)..(52)
<223>  Xaa can be any naturally occurring amino acid

<220>
<221>  misc_feature
<222>  (89)..(89)
<223>  Xaa can be any naturally occurring amino acid

<220>
<221>  misc_feature
<222>  (127)..(127)
<223>  Xaa can be any naturally occurring amino acid
```

```
<220>
<221>  misc_feature
<222>  (177)..(177)
<223>  Xaa can be any naturally occurring amino acid

<220>
<221>  misc_feature
<222>  (189)..(189)
<223>  Xaa can be any naturally occurring amino acid

<220>
<221>  misc_feature
<222>  (286)..(286)
<223>  Xaa can be any naturally occurring amino acid

<220>
<221>  misc_feature
<222>  (290)..(290)
<223>  Xaa can be any naturally occurring amino acid

<220>
<221>  misc_feature
<222>  (308)..(308)
<223>  Xaa can be any naturally occurring amino acid

<220>
<221>  misc_feature
<222>  (317)..(317)
<223>  Xaa can be any naturally occurring amino acid

<220>
<221>  misc_feature
<222>  (330)..(330)
<223>  Xaa can be any naturally occurring amino acid

<400>  220
```

Ser Val Gly Ala Asp Thr Pro Leu Leu Leu Arg Phe Asp Asn Lys Tyr
1               5                   10                  15

Ile Asp Tyr Val Arg Ala Asp Gln Val Asp Glu Ser Leu Ala Gly Gly
            20                  25                  30

Asp Ala Ser Ala Ala Ala Arg Met Trp Ser Ala Tyr Gln Gly Asp Lys
            35                  40                  45

Glu Ala Phe Xaa Pro Pro Arg Ser Ile Glu Val Trp Thr Glu Arg Gly
            50                  55                  60

Trp Thr Ala Val Asn Arg Val Ile Arg His Lys Ala Gly Lys Lys Met
65                  70                  75                  80

Tyr Arg Val Leu Thr His Thr Gly Xaa Val Asp Val Thr Glu Asp His
                85                  90                  95

Ser Leu Leu Asp Arg His Ala Arg Lys Ile Lys Pro Thr Asp Val Ala
            100                 105                 110

```
Val Gly Ser Ala Leu Leu His Thr Asp Leu Pro Pro His Glu Xaa Pro
        115                 120             125

Ser Pro Ser Leu Gln Arg His Thr Asn Ala Ser Ala Asp Pro Val Thr
        130                 135             140

Gly Asp Ala Lys Leu Leu Ala Ser Ser Thr Ala Gln Asn Met Thr Asp
145                 150                 155                 160

Asp Thr Ala His Ala Trp Ala Leu Gly Met Phe Phe Ala Glu Gly Ser
                165                 170                 175

Xaa Asn Glu Tyr Val Arg Arg Asp Arg Asp Gln Tyr Xaa Trp Arg Ile
                180                 185                 190

Ala Asn Lys Asp Met Val Leu Leu Arg Met Ala Leu Asp Gly Leu Asp
        195                 200                 205

Gly Arg Tyr Pro Gly Ile Thr Phe Ser Ile Asn Gly Pro Tyr Lys Asp
        210                 215                 220

Gly Met Ala Tyr Val Val Ala Asn Gly Pro Ser Lys Met Gly Leu Val
225                 230                 235                 240

Ala Asn Tyr Arg Ala Ala Phe Tyr Asp Pro Ala Tyr Ala Leu Lys Arg
                245                 250                 255

Val Pro Thr Glu Ile Leu Asn Ala Asn Thr Lys Ile Lys Arg Ala Phe
                260                 265                 270

Ile Arg Gly Tyr Phe Ala Gly Asp Gly Asn Lys Thr Asp Xaa Gly Ser
        275                 280                 285

Arg Xaa Asp Gly Arg Gly Lys Ile Gly Met Ala Gly Ile Tyr Tyr Leu
        290                 295                 300

Leu Ser Thr Xaa Gly Tyr Ser Val Ser Ile Asn Thr Xaa Gly Gly Pro
305                 310                 315                 320

Glu Arg Asp Thr Tyr Arg Val Asn Phe Xaa Asp Ala Ser Ala Pro Lys
                325                 330                 335

Arg Thr Gln Arg Lys Ala Pro Asp Thr Ile Lys Lys Ile Ile Pro Leu
        340                 345                 350

Asp Asp Lys Ser Phe Gly Val Asn Ala Tyr Val Tyr Asp Leu Glu Thr
```

                        355                    360                        365


          Ala Asn His His Phe Ala Ala Gly Ile Gly Arg Leu Val Val His Asn
               370                    375                    380


          <210> 221
          <211> 389
          <212> PRT
          <213> Artificial sequence

          <220>
          <223> modified contiguous intein from Pandoravirus neocaledonia
                neocaledonia


          <220>
          <221> misc_feature
          <222> (52)..(52)
          <223> Xaa can be any naturally occurring amino acid

          <220>
          <221> misc_feature
          <222> (89)..(89)
          <223> Xaa can be any naturally occurring amino acid

          <220>
          <221> misc_feature
          <222> (127)..(127)
          <223> Xaa can be any naturally occurring amino acid

          <220>
          <221> misc_feature
          <222> (134)..(134)
          <223> Xaa can be any naturally occurring amino acid

          <220>
          <221> misc_feature
          <222> (181)..(181)
          <223> Xaa can be any naturally occurring amino acid

          <220>
          <221> misc_feature
          <222> (193)..(193)
          <223> Xaa can be any naturally occurring amino acid

          <220>
          <221> misc_feature
          <222> (290)..(290)
          <223> Xaa can be any naturally occurring amino acid

          <220>
          <221> misc_feature
          <222> (294)..(294)
          <223> Xaa can be any naturally occurring amino acid

          <220>
          <221> misc_feature
          <222> (312)..(312)
          <223> Xaa can be any naturally occurring amino acid

          <220>

```
<221>   misc_feature
<222>   (321)..(321)
<223>   Xaa can be any naturally occurring amino acid

<220>
<221>   misc_feature
<222>   (334)..(334)
<223>   Xaa can be any naturally occurring amino acid

<220>
<221>   misc_feature
<222>   (358)..(358)
<223>   Xaa can be any naturally occurring amino acid

<400>   221
```

Ser Val Gly Ala Asp Thr Pro Leu Leu Leu Arg Phe Asp Asn Lys Tyr
1               5               10              15

Ile Asp Tyr Val Arg Ala Asp Gln Val Asp Glu Ser Leu Ala Gly Gly
            20              25              30

Asp Ala Ser Ala Ala Ala Arg Met Trp Gly Ala Tyr Gln Gly Asp Lys
            35              40              45

Glu Ala Phe Xaa Pro Pro Arg Ser Ile Glu Val Trp Thr Glu Arg Gly
        50              55              60

Trp Thr Ala Val Asn Arg Val Ile Arg His Lys Ala Gly Lys Lys Met
65              70              75              80

Tyr Arg Val Leu Thr His Thr Gly Xaa Val Asp Val Thr Glu Asp His
                85              90              95

Ser Leu Leu Asp Arg His Ala Asn Lys Ile Lys Pro Thr Asp Val Ala
            100             105             110

Val Gly Ser Ala Leu Leu His Ala Asp Leu Pro Pro Tyr Glu Xaa Pro
            115             120             125

Ser Pro Ile Phe Glu Xaa Arg Ala Asp Ala Ser Ala Lys Ser Ala Thr
        130             135             140

Gly Ser Ala Asn Thr Val Val Ala Ser Ser Val Ala Asp Asn Gly Ala
145             150             155             160

Ala Val Ala Asp Asp Ala Ala His Ala Trp Ala Leu Gly Met Phe Phe
                165             170             175

Ala Glu Gly Ser Xaa Asn Glu Tyr Val Arg His Asp Arg Asp Gln Tyr
            180             185             190

```
Xaa Trp Arg Ile Ala Asn Lys Asp Met Ala Leu Leu Arg Ile Ala Leu
    195             200             205

Ala Gly Leu Gly Gly Arg Tyr Pro Asp Ile Ser Phe Ser Ile Val Gly
    210             215             220

Pro Tyr Lys Asp Gly Met Ala Tyr Val Val Ala Asn Gly Pro Gly Lys
225             230             235             240

Met Gly Leu Val Ala Asn Tyr Arg Ala Ala Phe Tyr Asp Pro Val His
            245             250             255

Ala Leu Lys Arg Val Pro Thr Glu Ile Leu Asn Ala Asn Ala Lys Ile
            260             265             270

Lys Arg Ala Phe Ile Arg Gly Tyr Phe Ala Gly Asp Gly Asn Lys Thr
    275             280             285

Asp Xaa Gly Ser Arg Xaa Asp Gly Arg Gly Lys Ile Gly Met Ala Gly
    290             295             300

Ile Tyr Tyr Leu Leu Ser Thr Xaa Gly Tyr Ser Val Ser Ile Asn Thr
305             310             315             320

Xaa Gly Gly Pro Glu Arg Asp Thr Tyr Arg Val Asn Phe Xaa Asp Ala
            325             330             335

Ala Thr Pro Lys Arg Thr Gln Arg Lys Ala Pro Asp Thr Ile Lys Lys
            340             345             350

Ile Ile Pro Leu Asp Xaa Asp Lys Ser Leu Gly Gly Asn Val Tyr Val
            355             360             365

Tyr Asp Leu Glu Thr Ala Asn His His Phe Ala Ala Gly Ile Gly Arg
370             375             380

Leu Val Val His Asn
385
```

```
<210>  222
<211>  389
<212>  PRT
<213>  Artificial sequence

<220>
<223>  modified contiguous intein from Pandoravirus pampulha strain 8.5

<220>
```

```
<221>  misc_feature
<222>  (52)..(52)
<223>  Xaa can be any naturally occurring amino acid

<220>
<221>  misc_feature
<222>  (89)..(89)
<223>  Xaa can be any naturally occurring amino acid

<220>
<221>  misc_feature
<222>  (127)..(127)
<223>  Xaa can be any naturally occurring amino acid

<220>
<221>  misc_feature
<222>  (183)..(183)
<223>  Xaa can be any naturally occurring amino acid

<220>
<221>  misc_feature
<222>  (188)..(188)
<223>  Xaa can be any naturally occurring amino acid

<220>
<221>  misc_feature
<222>  (195)..(195)
<223>  Xaa can be any naturally occurring amino acid

<220>
<221>  misc_feature
<222>  (292)..(292)
<223>  Xaa can be any naturally occurring amino acid

<220>
<221>  misc_feature
<222>  (296)..(296)
<223>  Xaa can be any naturally occurring amino acid

<220>
<221>  misc_feature
<222>  (314)..(314)
<223>  Xaa can be any naturally occurring amino acid

<220>
<221>  misc_feature
<222>  (323)..(323)
<223>  Xaa can be any naturally occurring amino acid

<220>
<221>  misc_feature
<222>  (336)..(336)
<223>  Xaa can be any naturally occurring amino acid

<400>  222

Ser Val Gly Ala Asp Thr Pro Leu Leu Leu Arg Phe Glu Gly Lys Tyr
1                5                10                15

Val Asp Tyr Val Arg Ala Asp Gln Val Ala Glu Thr Leu Ala Ala Gly
            20                25                30
```

298

```
Asp Gly Val Ala Ala Ala Gln Leu Trp Ser Ala Tyr Gln Gly Asp Lys
        35                  40                  45

Glu Ala Phe Xaa Pro Arg Arg Pro Ile Glu Val Trp Thr Glu Arg Gly
        50                  55                  60

Trp Thr Val Val Asn Arg Val Ile Arg His Arg Ala Gly Lys Lys Met
65                  70                  75                  80

Phe Arg Val Leu Thr His Thr Gly Xaa Val Asp Val Thr Glu Asp His
                85                  90                  95

Ser Leu Leu Asp Pro Asn Ala Glu Lys Val Lys Pro Thr Glu Ile Ser
            100                 105                 110

Ile Gly Ser Ala Leu Leu His Ala Asp Leu Pro Ala His Glu Xaa Ala
            115                 120                 125

Ala Val Ser Leu Lys Ser Arg Ala Ile Arg Pro Ala Asp Thr Thr Asn
        130                 135                 140

Pro Ala Ile Leu Ala Ala Pro Thr Ala Val Asp Ser Asp Gly Asp Val
145                 150                 155                 160

Ala Ile Asp Asp Ala Asn Asp Thr Ala His Ala Trp Ala Met Gly Met
                165                 170                 175

Phe Phe Ala Glu Gly Ser Xaa Ser Glu Tyr Leu Xaa Gly Asn Ala Gln
            180                 185                 190

Gln Tyr Xaa Trp Arg Ile Ala Asn Lys Asp Met Val Leu Val Arg Met
        195                 200                 205

Thr Leu Asp Gly Leu Lys Gly Arg Tyr Pro Asp Val Asp Phe Ser Ile
    210                 215                 220

Ile Gly Pro Tyr Ala Asp Gly Met Ala Tyr Val Val Ala Asn Gly Pro
225                 230                 235                 240

Ala Lys Met Gly Leu Val Ala Asn Tyr Arg Ala Ala Phe Tyr Asp Pro
            245                 250                 255

Val His Ala Leu Lys Arg Val Pro Thr Glu Ile Leu Asn Ala Asn Val
            260                 265                 270

Glu Ile Lys Arg Ala Phe Ile Arg Gly Tyr Phe Ala Gly Asp Gly Asn
        275                 280                 285
```

```
Lys Thr Asp Xaa Gly Ser Arg Xaa Asp Gly Arg Gly Lys Ile Gly Met
    290             295             300

Ala Gly Ile Tyr Tyr Leu Leu Ser Ala Xaa Gly Tyr Leu Gly Ser Ile
    305             310             315             320

Asn Thr Xaa Gly Gly Pro Glu Arg Asp Thr Tyr Arg Ile Asn Phe Xaa
            325             330             335

Asp Ala Ala Ala Pro Lys Arg Thr Gln Arg Lys Pro Pro Asp Asn Ile
        340             345             350

Lys Lys Ile Ile Pro Leu Asp Ala Lys Ala Phe Asp Gly Ala Tyr Val
        355             360             365

Tyr Asp Leu Glu Thr Ala Asn His His Phe Ala Ala Gly Ile Gly Arg
    370             375             380

Leu Val Val His Asn
385


<210>  223
<211>  387
<212>  PRT
<213>  Artificial sequence

<220>
<223>  modified contiguous intein from Pandoravirus braziliensis strain
       SL2


<220>
<221>  misc_feature
<222>  (52)..(52)
<223>  Xaa can be any naturally occurring amino acid

<220>
<221>  misc_feature
<222>  (89)..(89)
<223>  Xaa can be any naturally occurring amino acid

<220>
<221>  misc_feature
<222>  (127)..(127)
<223>  Xaa can be any naturally occurring amino acid

<220>
<221>  misc_feature
<222>  (180)..(180)
<223>  Xaa can be any naturally occurring amino acid

<220>
<221>  misc_feature
<222>  (192)..(192)
```

<223> Xaa can be any naturally occurring amino acid

<220>
<221> misc_feature
<222> (279)..(279)
<223> Xaa can be any naturally occurring amino acid

<220>
<221> misc_feature
<222> (289)..(289)
<223> Xaa can be any naturally occurring amino acid

<220>
<221> misc_feature
<222> (293)..(293)
<223> Xaa can be any naturally occurring amino acid

<220>
<221> misc_feature
<222> (311)..(311)
<223> Xaa can be any naturally occurring amino acid

<220>
<221> misc_feature
<222> (320)..(320)
<223> Xaa can be any naturally occurring amino acid

<220>
<221> misc_feature
<222> (333)..(333)
<223> Xaa can be any naturally occurring amino acid

<400> 223

Ser Val Gly Ala Asp Thr Pro Leu Leu Leu Arg Phe Asp Asn Lys Tyr
1               5                   10                  15

Ile Asp Tyr Val Arg Ala Asp Gln Val Asp Glu Ser Leu Ser Gly Gly
            20                  25                  30

Asp Ala Ala Ala Ala Ala Arg Met Trp Ser Ala Tyr Gln Gly Asp Lys
            35                  40                  45

Glu Ala Phe Xaa Pro Pro Arg Ser Ile Glu Val Trp Thr Glu Arg Gly
        50                  55                  60

Trp Thr Ala Val Asn Arg Val Ile Arg His Arg Ala Gly Lys Arg Met
65                  70                  75                  80

Tyr Arg Val Leu Thr His Thr Gly Xaa Val Asp Val Thr Lys Asp His
                85                  90                  95

Ser Leu Leu Asp Pro Gln Ala Asn Lys Ile Lys Pro Thr Asp Val Ala
                100                 105                 110

Val Gly Ser Ala Leu Leu His Ala Asp Leu Pro Pro Tyr Glu Xaa Pro

115                        120                        125

Ser Ser Ser Leu Gln Arg Arg Ala Ala Ala Ser Asp Asp Pro Val Ile
    130                 135                 140

Asp Ser Ala Lys Ser Leu Ala Ser Ser Ala Gly Glu Ser Thr Ala Val
145                 150                 155                 160

Ser Thr Asn Asp Thr Ala His Ala Trp Ala Leu Gly Met Phe Phe Ala
                165                 170                 175

Glu Gly Ser Xaa Asn Glu Tyr Val Arg Gly Asp Arg Asp Gln Tyr Xaa
                180                 185                 190

Trp Arg Ile Ala Asp Lys Asp Met Val Leu Leu Arg Thr Ala Leu Asp
        195                 200                 205

Gly Leu Asp Gly Arg Tyr Pro Gly Val Thr Phe Ser Ile Asn Gly Pro
    210                 215                 220

Tyr Lys Asp Gly Met Ala Phe Val Val Ala Asn Gly Pro Gly Lys Met
225                 230                 235                 240

Gly Leu Val Ala Asn Tyr Arg Ala Ala Phe Tyr Asp Pro Val His Ala
                245                 250                 255

Leu Lys Arg Val Pro Thr Glu Ile Leu Asn Ala Asn Thr Glu Ile Lys
        260                 265                 270

Arg Ala Phe Ile Arg Gly Xaa Phe Ala Gly Asp Gly Asn Lys Thr Asp
        275                 280                 285

Xaa Gly Ser Arg Xaa Asp Gly Leu Gly Lys Ile Gly Met Ala Gly Ile
    290                 295                 300

Tyr Tyr Leu Leu Ser Thr Xaa Gly Tyr Ser Ala Ser Val Asn Thr Xaa
305                 310                 315                 320

Gly Gly Pro Glu Arg Asp Thr Tyr Arg Ile Asn Phe Xaa Asp Ala Ala
                325                 330                 335

Thr Pro Lys Arg Thr Gln Arg Lys Ala Pro Asn Ile Ile Lys Lys Ile
        340                 345                 350

Ile Pro Leu Asp Gly Glu Ala Phe Gly Gly Asn Ala Tyr Val Tyr Asp
    355                 360                 365

Leu Glu Thr Ala Asn His His Phe Ala Ala Gly Ile Gly Arg Leu Ile
    370                  375                  380

Val His Asn
  385


<210> 224
<211> 308
<212> PRT
<213> Artificial sequence

<220>
<223> modified contiguous intein from Labyrinthulid quahog parasite QPX
     NY07348D


<220>
<221> misc_feature
<222> (17)..(17)
<223> Xaa can be any naturally occurring amino acid

<220>
<221> misc_feature
<222> (65)..(65)
<223> Xaa can be any naturally occurring amino acid

<220>
<221> misc_feature
<222> (71)..(71)
<223> Xaa can be any naturally occurring amino acid

<220>
<221> misc_feature
<222> (87)..(87)
<223> Xaa can be any naturally occurring amino acid

<220>
<221> misc_feature
<222> (100)..(100)
<223> Xaa can be any naturally occurring amino acid

<220>
<221> misc_feature
<222> (104)..(104)
<223> Xaa can be any naturally occurring amino acid

<220>
<221> misc_feature
<222> (120)..(120)
<223> Xaa can be any naturally occurring amino acid

<220>
<221> misc_feature
<222> (137)..(137)
<223> Xaa can be any naturally occurring amino acid

<220>
<221> misc_feature
<222> (164)..(164)
<223> Xaa can be any naturally occurring amino acid

<220>
<221> misc_feature
<222> (220)..(220)
<223> Xaa can be any naturally occurring amino acid

<400> 224

Ser Val Val Gly Ser Thr Gln Leu Leu Leu Lys Val Asn Gly Met Leu
1               5               10                  15

Xaa Val Ser Arg Ile Ser Glu Met Val Tyr Leu Leu His Ser Ser Arg
            20              25                  30

Trp Thr Thr Trp Arg His Glu Lys Gln Ala Val Gln Ile Asp Pro Arg
            35              40                  45

Lys Asp Glu Ile Phe Thr Trp Thr Asp Thr Gly Trp Ser Arg Val Arg
        50              55                  60

Xaa Ile Ile Arg His Arg Xaa Thr Lys Ser Leu Phe Lys Ile Lys Thr
65              70                  75                  80

Thr His Gly Asp Val Thr Xaa Thr Asn Asp His Ser Leu Leu Gln Pro
                85                  90                  95

Asp Gly Ser Xaa Ile Ser Pro Xaa Ala Leu Ser Ile Gly Lys Thr Arg
            100             105                 110

Leu Leu Ser Ser Phe Pro Asn Xaa Arg Asp Phe Pro Gln Glu Asp Ser
            115             120                 125

Pro Glu Leu Leu Val Pro Gly Pro Xaa Val Pro Gln Ser Phe Val Leu
        130             135                 140

Asp Ala Met Ile Ala Glu Leu Ala Gly Leu Tyr Ile Ser Gln Gly Arg
145             150                 155                 160

Asp Asp Leu Xaa Val Val Val Pro Asn Pro Val Arg Arg Gln Ala Phe
                165             170                 175

Leu His Thr Leu Gln His Arg Leu Gly His Leu Asn Trp Ala Ile Val
            180             185                 190

Asn Asp Leu Lys Ile Val Pro Val Asp Leu Gly Asp Ala Lys Ala Leu
            195             200                 205

Lys Glu Thr Arg Arg Phe Trp Asn His Asp Val Xaa Asn Asp Gly Leu
        210             215                 220

```
Gly Val Pro Asn Trp Val Leu Asn Ser Asn Arg Arg Ile Arg Val Ala
225                 230             235                 240


Phe Ser Lys Gly Phe Gly Ala Gly Asp Leu Glu Leu Gly Gly Phe Thr
                245             250                 255


Leu Glu Ala Ser Leu Thr Leu Asn His Asp Arg Lys Leu Glu Glu Ala
                260             265                 270


Leu Val Leu Asp Ile Leu Pro Val Glu Asn His Gly Glu Phe Val Tyr
            275             280             285


Asp Leu Thr Thr Glu Asn His His Phe Gln Ala Gly Ser Gly Ser Leu
        290             295                 300


Ile Val His Asn
305


<210>  225
<211>  308
<212>  PRT
<213>  Artificial sequence

<220>
<223>  modified contiguous intein from Labyrinthulid quahog parasite QPX
       NY0313808BC1


<220>
<221>  misc_feature
<222>  (17)..(17)
<223>  Xaa can be any naturally occurring amino acid

<220>
<221>  misc_feature
<222>  (65)..(65)
<223>  Xaa can be any naturally occurring amino acid

<220>
<221>  misc_feature
<222>  (71)..(71)
<223>  Xaa can be any naturally occurring amino acid

<220>
<221>  misc_feature
<222>  (87)..(87)
<223>  Xaa can be any naturally occurring amino acid

<220>
<221>  misc_feature
<222>  (100)..(100)
<223>  Xaa can be any naturally occurring amino acid

<220>
<221>  misc_feature
<222>  (104)..(104)
<223>  Xaa can be any naturally occurring amino acid
```

```
<220>
<221>  misc_feature
<222>  (120)..(120)
<223>  Xaa can be any naturally occurring amino acid

<220>
<221>  misc_feature
<222>  (137)..(137)
<223>  Xaa can be any naturally occurring amino acid

<220>
<221>  misc_feature
<222>  (164)..(164)
<223>  Xaa can be any naturally occurring amino acid

<220>
<221>  misc_feature
<222>  (220)..(220)
<223>  Xaa can be any naturally occurring amino acid

<400>  225
```

Ser Val Val Gly Ser Thr Gln Leu Leu Leu Lys Val Asn Gly Met Leu
1                   5                   10                  15

Xaa Val Ser Arg Ile Ser Glu Met Val Tyr Leu Leu His Ser Ser Arg
            20                  25                  30

Trp Thr Thr Trp Arg His Glu Lys Gln Ala Val Gln Ile Asp Pro Arg
            35                  40                  45

Lys Asp Glu Ile Phe Thr Trp Thr Asp Thr Gly Trp Ser Arg Val Arg
            50                  55                  60

Xaa Ile Ile Arg His Arg Xaa Thr Lys Ser Leu Phe Lys Ile Lys Thr
65                  70                  75                  80

Thr His Gly Asp Val Thr Xaa Thr Asn Asp His Ser Leu Leu Gln Pro
                85                  90                  95

Asp Gly Ser Xaa Ile Ser Pro Xaa Ala Leu Ser Ile Gly Lys Thr Arg
                100                 105                 110

Leu Leu Ser Ser Phe Pro Asn Xaa Arg Asp Phe Pro Gln Glu Asp Ser
                115                 120                 125

Pro Glu Leu Leu Val Pro Gly Pro Xaa Val Pro Gln Ser Phe Val Leu
        130                 135                 140

Asp Ala Met Ile Ala Glu Leu Ala Gly Leu Tyr Ile Ser Gln Gly Arg
145                 150                 155                 160

```
Asp Asp Leu Xaa Val Val Val Pro Asn Pro Val Arg Arg Gln Ala Phe
            165             170             175

Leu His Thr Leu Gln His Arg Leu Gly His Leu Asn Trp Ala Ile Val
            180             185             190

Asn Asp Leu Lys Ile Val Pro Val Asp Leu Gly Asp Ala Lys Ala Leu
            195             200             205

Lys Glu Thr Arg Arg Phe Trp Asn His Asp Val Xaa Asn Asp Gly Leu
    210             215             220

Gly Val Pro Asn Trp Val Leu Asn Ser Asn Arg Arg Ile Arg Val Ala
225             230             235             240

Phe Ser Lys Gly Phe Gly Ala Gly Asp Leu Glu Leu Gly Gly Phe Thr
            245             250             255

Leu Glu Ala Ser Leu Thr Leu Asn His Asp Arg Lys Leu Glu Glu Ala
            260             265             270

Leu Val Leu Asp Ile Leu Pro Val Glu Asn His Gly Glu Phe Val Tyr
    275             280             285

Asp Leu Thr Thr Glu Asn His His Phe Gln Ala Gly Ser Gly Ser Leu
    290             295             300

Ile Val His Asn
305
```

```
<210>  226
<211>  229
<212>  PRT
<213>  Artificial sequence

<220>
<223>  modified contiguous intein from Russia Kulunda-steppe soda lake
       Tanatar-5 brine environmental genomics

<220>
<221>  misc_feature
<222>  (82)..(82)
<223>  Xaa can be any naturally occurring amino acid

<220>
<221>  misc_feature
<222>  (117)..(117)
<223>  Xaa can be any naturally occurring amino acid

<400>  226

Ser Val Leu Lys Asp Thr Pro Ile Ile Val Tyr Asp Ile Ile Glu Lys
```

307

1                    5                              10                             15

Glu Ile Lys Ile Lys Thr Ile Glu Glu Leu Gly Gly Lys Glu Pro Arg
        20                      25                      30

Ile Trp His Ser Tyr Lys Asn Phe Lys Val Leu Asp Ser Tyr Leu Ser
        35                      40                      45

Asn Arg Arg Ser Lys Lys Gln Ser Phe Ile Ser Asn Glu Arg Tyr Leu
        50                      55                      60

Val Trp Thr Ala Ser Gly Trp Ser Ser Ile Asn Arg Val Ile Lys His
65                      70                      75                      80

Lys Xaa Asn Lys Lys Ile Tyr Arg Ile Gln Thr Ser His Ser Ile Val
                85                      90                      95

Asp Val Thr Glu Asp His Ser Leu Ile Asn Glu Lys Gly Asn Lys Ile
        100                     105                     110

Lys Pro Ser Glu Xaa Thr Ile Gly Thr Arg Leu Leu Tyr His Pro Leu
        115                     120                     125

Leu Met Asn Leu Ile Asp Lys Glu Glu Tyr Arg Ile Lys Tyr Lys Lys
        130                     135                     140

Val Ser Asp Lys Arg His Phe Thr Ser Lys Lys Thr Leu Ser Lys Tyr
145                     150                     155                     160

Ile Leu Lys Ser Lys Tyr Pro Met Asn Val Asn Val Ser Arg Asp Thr
                165                     170                     175

Gln Gly Asn Glu Ile Tyr Ser Ala Ser Val His Ile Gly Leu Tyr Asp
                180                     185                     190

Asn Arg Ile Thr Lys Ile Glu Leu Leu His Asp Lys Val Val Glu Tyr
                195                     200                     205

Val Tyr Asp Ile Glu Thr Gln Asp Gly Thr Phe Asn Val Gly Phe Pro
        210                     215                     220

Leu Ile Val Lys Asn
225

<210>   227
<211>   229
<212>   PRT
<213>   Artificial sequence

<220>
<223> modified contiguous intein from Russia Kulunda-steppe soda lake
Tanatar-5 brine environmental genomics


<220>
<221> misc_feature
<222> (82)..(82)
<223> Xaa can be any naturally occurring amino acid

<220>
<221> misc_feature
<222> (117)..(117)
<223> Xaa can be any naturally occurring amino acid

<400> 227

Ser Val Leu Lys Asp Thr Pro Ile Ile Val Tyr Asp Val Ile Lys Arg
1               5                   10                  15


Lys Ile Asn Ile Lys Thr Ile Glu Glu Leu Gly Gly Ile Asp Glu Tyr
            20                  25                  30


Val Trp His Ser Tyr Asn Asn Phe Lys Val Phe Asp Ser Tyr Leu Ser
        35                  40                  45


Asn Arg Arg Phe Lys Lys Gln Ser Phe Ile Ser Asn Glu Arg Tyr Leu
    50                  55                  60


Val Trp Thr Ser Ser Gly Trp Ser Ser Ile His Arg Ile Ile Lys His
65                  70                  75                  80


Lys Xaa Asn Lys Lys Ile Tyr Arg Ile Gln Thr Asn His Ser Ile Val
                85                  90                  95


Asp Val Thr Glu Asp His Ser Leu Ile Asp Glu Thr Gly Lys Lys Ile
            100                 105                 110


Lys Pro Ser Gln Xaa Ser Ile Gly Thr Arg Leu Leu Tyr Asn Pro Leu
        115                 120                 125


Leu Phe Gly Leu Glu Asn Lys Asp Asn Tyr Glu Met Lys Tyr Arg Lys
        130                 135                 140


Val Ser Asp Ile Lys Tyr Tyr Glu Thr Lys Lys Glu Leu Ser Lys Tyr
145                 150                 155                 160


Val Leu Gln Ser Lys Tyr Pro Met Lys Ile Glu Ser Ser Arg Asp Ser
                165                 170                 175


Gln Gly Asn Glu Ile Tyr Ser Ala Ser Val His Ile Gly Leu Tyr Asp

```
                      180                  185                      190


     Asn Arg Ile Lys Lys Ile Glu Leu Leu His Asn Arg Val Gly Asp Tyr
             195                 200                 205


     Val Tyr Asp Ile Glu Thr Thr Asp Gly Thr Phe Asn Val Gly Phe Pro
             210                 215                 220


     Leu Ile Val Lys Asn
     225


     <210>  228
     <211>  228
     <212>  PRT
     <213>  Artificial sequence

     <220>
     <223>  modified contiguous intein from Russia Kulunda-steppe soda lake
            Tanatar-5 brine environmental genomics


     <220>
     <221>  misc_feature
     <222>  (19)..(19)
     <223>  Xaa can be any naturally occurring amino acid


     <220>
     <221>  misc_feature
     <222>  (82)..(82)
     <223>  Xaa can be any naturally occurring amino acid


     <220>
     <221>  misc_feature
     <222>  (117)..(117)
     <223>  Xaa can be any naturally occurring amino acid

     <400>  228

     Ser Val Leu Gly Asp Thr Pro Ile Ile Val Tyr Asp Leu Ile Glu His
     1               5                   10                  15


     Lys Val Xaa Val Lys Thr Ile Lys Glu Leu Gly Asp Gly Arg Ile His
                 20                  25                  30


     Gln Trp Thr Ser Tyr Lys Asn Phe Lys Val Phe Asp Ser Tyr Leu Ser
             35                  40                  45


     Asn Arg Arg Phe Lys Lys Gln Ser Tyr Asn Ala Asn Glu Arg Tyr Leu
             50                  55                  60


     Val Trp Ser Ala Ser Gly Trp Ser Ser Ile Arg Arg Ile Ile Lys His
     65                  70                  75                  80


     Lys Xaa Asn Lys Lys Ile Tyr Arg Val Gln Thr Pro His Ser Ile Val
```

...

```
                    85                    90                    95


        Asp Val Thr Glu Asp His Ser Leu Leu Asp Tyr Lys Gly Arg Lys Leu
                    100                   105                   110


        Lys Pro Ser Glu Xaa Val Val Gly Thr Lys Leu Leu Tyr Asn Pro Ile
                    115                   120                   125


        Leu Phe Gly Leu Pro Asp Lys Glu Phe Tyr Gln Leu Lys Tyr His Lys
                    130                   135                   140


        Asp Thr Gln Glu Arg Met Ser Phe Lys Thr Lys Lys Glu Leu Ala Thr
        145                   150                   155                   160


        Phe Ile Leu Arg Ser Lys Tyr Pro Met Lys Val Glu Tyr Arg Lys Gly
                    165                   170                   175


        Asp Glu Lys Pro Tyr Ser Ala Thr Thr His Ile Gly Leu Tyr Asp Asn
                    180                   185                   190


        Arg Ile Thr His Met Glu Val Leu His Ser Arg Ile Met Glu Tyr Val
                    195                   200                   205


        Tyr Asp Ile Glu Thr Ser Asp Gly Thr Phe Asn Val Gly Phe Pro Leu
                    210                   215                   220


        Ile Val Lys Asn
        225


        <210>  229
        <211>  149
        <212>  PRT
        <213>  Artificial sequence

        <220>
        <223>  modified contiguous intein (partial) from Gujarat India activated
               biomass of a wastewater treatment plant treating hydrocarbon
               contaminated wastewater at high TDS (total dissolved solids)


        <220>
        <221>  misc_feature
        <222>  (1)..(1)
        <223>  Xaa can be any naturally occurring amino acid

        <220>
        <221>  misc_feature
        <222>  (15)..(15)
        <223>  Xaa can be any naturally occurring amino acid

        <220>
        <221>  misc_feature
        <222>  (29)..(29)
```

<223> Xaa can be any naturally occurring amino acid

<220>
<221> misc_feature
<222> (103)..(103)
<223> Xaa can be any naturally occurring amino acid

<400> 229

Xaa Ser Ile Gly Asp Tyr Thr Ile Glu Asp Leu Tyr Asn Gln Xaa Thr
1               5                   10                  15

Asp Phe Tyr Ser Lys Gly Ser Lys Glu Tyr Gly Lys Xaa Ser Leu Leu
            20                  25                  30

Val Leu Gly Tyr Asp His Ser Ser Gln Thr Ala Asp Phe Lys Ser Val
            35                  40                  45

Asn Tyr Val Met Arg His Lys Thr Thr Lys Asp Ile Tyr Lys Leu Thr
        50                  55                  60

Leu Gln Asp Gly Lys Thr Val Lys Thr Thr His Asp His Ser Ala Met
65                  70                  75                  80

Val Ile Arg Asp Gly Thr Leu Ile Glu Val Lys Pro Phe Glu Ile Asn
                85                  90                  95

Asn Thr Asp Met Phe Ile Xaa Ile Asp Asp Thr Asn Lys Val Tyr Asn
            100                 105                 110

Thr Ala Ile Asp Lys Val Glu Leu Leu Gly Thr Phe Asp Asp Tyr Val
            115                 120                 125

Tyr Asp Val Ser Ile Asn Asp Asn Ser His Tyr Phe Phe Gly Asn Asp
        130                 135                 140

Ile Leu Leu His Asn
145

<210> 230
<211> 164
<212> PRT
<213> Artificial sequence

<220>
<223> modified contiguous intein from sheep gut metagenome

<220>
<221> misc_feature
<222> (25)..(25)
<223> Xaa can be any naturally occurring amino acid

```
<220>
<221>  misc_feature
<222>  (88)..(88)
<223>  Xaa can be any naturally occurring amino acid

<220>
<221>  misc_feature
<222>  (96)..(96)
<223>  Xaa can be any naturally occurring amino acid

<220>
<221>  misc_feature
<222>  (161)..(161)
<223>  Xaa can be any naturally occurring amino acid

<400>  230

Ser Val Val Gly Ser Ser Glu Val Met Thr Asp Ser Gly Lys Met Thr
1               5                   10                  15


Ile Glu Asp Met Phe Asp Glu Ile Xaa Asp Ser Asp Ser Asp Ser Lys
            20                  25                  30


Ile Arg Val Arg Ser Asn Arg Lys Val Tyr Val Leu Ala Asp Gly Val
        35                  40                  45


Pro Glu Leu Arg Glu Ile Asn Tyr Ile Met Arg His Lys Thr Glu Lys
        50                  55                  60


Lys Ile Tyr Arg Ile Glu Ser Glu Asn Gly Lys Ile Val His Ala Thr
65                  70                  75                  80


Ser Asp His Ser Ile Ile Val Xaa Arg Asp Gly Ile Ile Ser Arg Xaa
                85                  90                  95


Lys Pro Glu Glu Leu Thr Ser Ser Asp Glu Leu Leu Thr Ile Asn Asp
            100                 105                 110


Tyr Pro Asp Leu Gly Phe Ile Pro Ser Lys Val Lys Ser Val Thr Val
        115                 120                 125


Ser Glu Tyr Asn Trp Glu Asp Asn Tyr Val Tyr Asp Ile Ser Val Lys
        130                 135                 140


Val Glu His Glu Asp Asp Leu Glu His Thr Phe Phe Ala Asn Gly Ile
145                 150                 155                 160


Xaa Val His Asn


<210>  231
<211>  159
```

<212>  PRT
<213>  Artificial sequence

<220>
<223>  modified contiguous intein from human gut metagenome Denmark
       Roux-en-Y gastric bypass surgery of morbidly obese patient


<220>
<221>  misc_feature
<222>  (26)..(26)
<223>  Xaa can be any naturally occurring amino acid

<220>
<221>  misc_feature
<222>  (38)..(38)
<223>  Xaa can be any naturally occurring amino acid

<220>
<221>  misc_feature
<222>  (45)..(45)
<223>  Xaa can be any naturally occurring amino acid

<220>
<221>  misc_feature
<222>  (52)..(52)
<223>  Xaa can be any naturally occurring amino acid

<220>
<221>  misc_feature
<222>  (95)..(95)
<223>  Xaa can be any naturally occurring amino acid

<400>  231

Ser Ile Thr Gly Asp Ser Ala Ile Arg Met Glu Asp Gly Ser Tyr Glu
1                5                   10                  15

Thr Ile Glu Asn Leu Phe Asn Ala Tyr Xaa Asp Thr Asp Ser Asp Asp
            20                  25                  30

Lys Ile Arg Val Ser Xaa Asp Lys Arg Val Tyr Gly Xaa Ser Ser Asp
            35                  40                  45

Phe Asn Pro Xaa Thr Tyr Pro Ile Lys Tyr Ile Met Arg His Lys Thr
        50                  55                  60

Asn Lys Thr Ile Trp Arg Val Thr Ser Thr Gln Lys Thr Ile Asn Val
65                  70                  75                  80

Thr Glu Asp His Ser Ile Val Ile Val Arg Asn Asn Ile Met Xaa Asn
                85                  90                  95

Ile Lys Pro Asn Asp Ile Asp Ile Asp Thr Asp Lys Leu Ile Ile Tyr
            100                 105                 110

```
Asp Asn Asp Lys Ile Val Leu Ala Asp Ile Gln Ser Asn Thr Ile Thr
        115                 120                 125


Asn Leu Ser Asp Glu Tyr Val Tyr Asp Ile Glu Ile Asp Ser Asp Asp
        130                 135                 140


Ala Glu Lys His Val Phe Phe Ala Asn Asp Ile Leu Val His Asn
145                 150                 155
```

```
<210>   232
<211>   166
<212>   PRT
<213>   Artificial sequence

<220>
<223>   modified contiguous intein from sheep gut metagenome


<220>
<221>   misc_feature
<222>   (29)..(29)
<223>   Xaa can be any naturally occurring amino acid

<220>
<221>   misc_feature
<222>   (78)..(78)
<223>   Xaa can be any naturally occurring amino acid

<220>
<221>   misc_feature
<222>   (137)..(137)
<223>   Xaa can be any naturally occurring amino acid

<220>
<221>   misc_feature
<222>   (146)..(146)
<223>   Xaa can be any naturally occurring amino acid

<400>   232
```

```
Ser Val Ala Gly Asp Ser Arg Ile Leu Leu Lys Tyr Gly Asp Asn Tyr
1               5                 10                  15


Phe Thr Lys Glu Ile Gln Glu Leu Phe Asn Glu Phe Xaa Asp Ser Asp
        20                  25                  30


Ser Gly Asp Lys Ile Arg Val Lys Val Gly Ser Lys Tyr Leu Val Gly
        35                  40                  45


Thr Tyr Asp Pro Glu Thr Asp Lys Pro Ile Tyr Arg Gly Ile Asp Tyr
        50                  55                  60


Val Met Arg His Lys Thr Asn Lys Arg Arg Phe Arg Ile Xaa Leu Asp
65                  70                  75                  80
```

Gly Gly Asn Ser Val Val Val Thr Glu Asp His Ser Ile Met Val Leu
                    85              90              95

Lys Asp Asp Asn Leu Val Glu Ala Ala Val Lys Asp Leu His Ile Gly
                100             105             110

Asp Lys Leu Ile Val Tyr Ala Asn Arg Glu Ser Val Val Gln Lys Asp
            115             120             125

Ile His Ser Ile Ile Tyr Val Gly Xaa Asp Asp Glu Tyr Val Tyr Asp
        130             135             140

Leu Xaa Val Phe Ala Asp Lys Asp Ile Gln His Thr Phe Phe Ala Asp
145             150             155             160

Asn Ile Leu Val His Asn
                165

<210>  233
<211>  253
<212>  PRT
<213>  Artificial sequence

<220>
<223>  modified contiguous intein from uncultured linear-genome virus ML
       43 kmer contig 2588 from Midtre Lovenbreen (Svalbard) glacier
       cryoconite holes sediment


<220>
<221>  misc_feature
<222>  (88)..(88)
<223>  Xaa can be any naturally occurring amino acid

<220>
<221>  misc_feature
<222>  (142)..(142)
<223>  Xaa can be any naturally occurring amino acid

<220>
<221>  misc_feature
<222>  (171)..(171)
<223>  Xaa can be any naturally occurring amino acid

<220>
<221>  misc_feature
<222>  (197)..(197)
<223>  Xaa can be any naturally occurring amino acid

<220>
<221>  misc_feature
<222>  (212)..(212)
<223>  Xaa can be any naturally occurring amino acid

<220>
<221>  misc_feature
<222>  (239)..(239)

<223> Xaa can be any naturally occurring amino acid

<220>
<221> misc_feature
<222> (243)..(243)
<223> Xaa can be any naturally occurring amino acid

<400> 233

Ser Val Leu Gly Ser Thr Val Leu Val Leu Arg Asp Pro Lys Thr Asn
1               5                   10                  15

Trp Ile Ser Ile Lys Thr Ile Glu Gln Leu Ser Ile Gly Thr Asn Ser
            20                  25                  30

Tyr Ser Tyr Asp Asn Phe Lys Leu Asp Gly Thr Leu Arg Ser His Lys
        35                  40                  45

Ser Tyr Thr Leu Thr Asn Tyr Gln Ile Trp Thr Asp Val Gly Trp Ser
    50                  55                  60

Asn Ile Lys Arg Val Ile Lys His Lys Thr Asn Lys Lys Ile Phe Arg
65                  70                  75                  80

Val Val Asn Asn Asn Gly Trp Xaa Asp Val Thr Glu Asp His Ser Leu
                85                  90                  95

Leu Asp Ser Gln Leu Asn Pro Ile Lys Pro Glu Gln Ile Asn Gly Asp
            100                 105                 110

Thr Val Leu Ala Asn Thr Phe Ile Asn Glu Phe Asn Lys Asp Lys Thr
        115                 120                 125

Asn Ile Ser Ser Ser Leu Ala Tyr Lys Met Gly Tyr Lys Xaa Ile Leu
        130                 135                 140

Asp Pro Val Ile Asn Gly Ser Ile Ile Asn Ser Pro Lys Asn Ile Arg
145                 150                 155                 160

Ala Ser Phe Tyr Lys Gly Tyr Leu Lys Asn Xaa Asn Ile Lys Gln Val
                165                 170                 175

Lys Ser Gln Val Trp Trp Gln Gly Ile Tyr Tyr Ile Gln Lys Ser Leu
            180                 185                 190

Gly Phe Asn Asn Xaa Ile Ser Lys Ser Asp Ser Pro Phe Val Asn Thr
        195                 200                 205

Asn Leu Lys Xaa Ser Asn Asp Glu Thr Ser Ile Gln Glu Leu Glu Met
        210                 215                 220

Ser Ser Ile Asp Gly Asp Phe Val Tyr Asp Leu Glu Thr Glu Xaa Gly
225                 230                 235                 240

Arg Phe Xaa Ala Gly Val Gly Ser Leu Leu Leu Lys Asn
                245                 250

<210> 234
<211> 253
<212> PRT
<213> Artificial sequence

<220>
<223> modified contiguous intein from uncultured linear-genome virus AB
      43 kmer contig 3071 from Austre Broggerbreen (Svalbard) glacier
      cryoconite holes sediment

<220>
<221> misc_feature
<222> (88)..(88)
<223> Xaa can be any naturally occurring amino acid

<220>
<221> misc_feature
<222> (142)..(142)
<223> Xaa can be any naturally occurring amino acid

<220>
<221> misc_feature
<222> (171)..(171)
<223> Xaa can be any naturally occurring amino acid

<220>
<221> misc_feature
<222> (197)..(197)
<223> Xaa can be any naturally occurring amino acid

<220>
<221> misc_feature
<222> (212)..(212)
<223> Xaa can be any naturally occurring amino acid

<220>
<221> misc_feature
<222> (239)..(239)
<223> Xaa can be any naturally occurring amino acid

<220>
<221> misc_feature
<222> (243)..(243)
<223> Xaa can be any naturally occurring amino acid

<400> 234

Ser Val Leu Gly Ser Thr Val Leu Val Leu Arg Asp Pro Lys Thr Asn
1               5                   10                  15

Trp Ile Ser Ile Lys Thr Ile Glu Gln Leu Ser Ile Gly Thr Asn Ser

318

```
              20                      25                      30

      Tyr Ser Tyr Asp Asn Phe Lys Leu Asp Gly Thr Leu Arg Ser His Lys
              35                  40                  45

      Ser Tyr Thr Leu Thr Asn Tyr Gln Ile Trp Thr Asp Val Gly Trp Ser
              50                  55                  60

      Asn Ile Lys Arg Val Ile Lys His Lys Thr Asn Lys Lys Ile Phe Arg
      65                  70                  75                  80

      Val Val Asn Asn Asn Gly Trp Xaa Asp Val Thr Glu Asp His Ser Leu
                      85                  90                  95

      Leu Asp Ser Gln Leu Asn Pro Ile Lys Pro Glu Gln Ile Asn Gly Asp
              100                 105                 110

      Thr Val Leu Ala Asn Thr Phe Ile Asn Glu Phe Asn Lys Asp Lys Thr
              115                 120                 125

      Asn Ile Ser Ser Ser Leu Ala Tyr Lys Met Gly Tyr Lys Xaa Ile Leu
              130                 135                 140

      Asp Pro Val Ile Asn Gly Ser Ile Ile Asn Ser Pro Lys Asn Ile Arg
      145                 150                 155                 160

      Ala Ser Phe Tyr Lys Gly Tyr Leu Lys Asn Xaa Asn Ile Lys Gln Val
                      165                 170                 175

      Lys Ser Gln Val Trp Trp Gln Gly Ile Tyr Tyr Ile Gln Lys Ser Leu
              180                 185                 190

      Gly Phe Asn Asn Xaa Ile Ser Lys Ser Asp Ser Pro Phe Val Asn Thr
              195                 200                 205

      Asn Leu Lys Xaa Ser Asn Asp Glu Thr Ser Ile Gln Glu Leu Glu Met
              210                 215                 220

      Ser Ser Ile Asp Gly Asp Phe Val Tyr Asp Leu Glu Thr Glu Xaa Gly
      225                 230                 235                 240

      Arg Phe Xaa Ala Gly Val Gly Ser Leu Leu Leu Lys Asn
                      245                 250


      <210>   235
      <211>   505
      <212>   PRT
      <213>   Artificial sequence
```

<220>
<223> modified contiguous intein from Klosneuvirus-KNV1


<220>
<221> misc_feature
<222> (26)..(26)
<223> Xaa can be any naturally occurring amino acid


<220>
<221> misc_feature
<222> (163)..(163)
<223> Xaa can be any naturally occurring amino acid


<220>
<221> misc_feature
<222> (177)..(177)
<223> Xaa can be any naturally occurring amino acid


<220>
<221> misc_feature
<222> (393)..(393)
<223> Xaa can be any naturally occurring amino acid


<220>
<221> misc_feature
<222> (432)..(432)
<223> Xaa can be any naturally occurring amino acid


<220>
<221> misc_feature
<222> (437)..(437)
<223> Xaa can be any naturally occurring amino acid


<400> 235

Ser Ile Leu Gly Asn Glu Val Leu Thr Leu Leu Asn Asp Lys Asn Asp
1               5                   10                  15

Ile Glu Phe His Arg Ile Asp Lys Leu Xaa Asp Lys Trp Glu Ser Tyr
            20                  25                  30

Asp Asn Phe Lys Thr Asp Glu Tyr Asn Glu Tyr Phe Thr Asn Ile Leu
            35                  40                  45

Asn Lys Leu Phe Lys Asp Lys Thr Asp Ser Asn Asn Ser Ile Leu Met
        50                  55                  60

Asn Glu Thr Asp Tyr Ile Asn Gly Lys Ile Met Gly Ser Ile Tyr Thr
65                  70                  75                  80

Asn Asn Ile Arg Lys Asn His Glu Val Ser Ile Gly Ala Leu Gly Lys
                85                  90                  95

Ile Asp Gly Lys Arg Thr Lys Lys Gln Phe Tyr Phe Ser Met Tyr Gly
            100                 105                 110

```
Asn Asp Thr Lys Ala Leu His Glu Ala Leu Lys Tyr Arg Leu Lys Leu
    115                 120             125

Asn Glu Glu Leu Asp Leu Ile Lys Asn Lys Tyr Arg Tyr Met Ile Asp
    130                 135             140

Leu Asp Ser Asn Arg Tyr Ile Glu Val Gln Val Asn Asn Asn Lys Ile
145                 150             155             160

Met Leu Xaa Asp Ile Asp Asp Ile Asp Ile Ile Glu Lys Tyr Thr Trp
            165             170             175

Xaa Ile Asn Glu Lys Asn Tyr Val Val Ser Asn Asn Val Asn Ser Glu
            180             185             190

Ser Trp Gln Tyr His Lys Leu Val Leu Asn Lys Ile Leu Asn Lys Leu
        195             200             205

Pro Asn Asn Ile Arg Asn Gln Ile Lys Asp Leu Thr Val Asp His Met
    210             215             220

Asn Arg Asn Thr Leu Asp Asn Arg Lys Gln Asn Leu Arg Leu Val Asn
225             230             235             240

Ser Lys Glu Gln Gln Trp Asn Gln Asp Ile Phe Lys Thr Asn Thr Ser
            245             250             255

Gly Thr Arg Gly Val Tyr Tyr Arg Thr Asn Arg Lys Ala Trp Ile Ala
        260             265             270

Asn Trp Leu Asn Leu Asp Gly Lys Arg Glu Ser Lys Tyr Phe Lys Asn
    275             280             285

Lys Gln Asp Ala Ile Asn Glu Arg Lys Thr Gln Glu Gln Ile Met Glu
    290             295             300

Thr Tyr Phe Asn Asp Glu Arg Lys Lys Leu Ile Asp Lys Leu Lys Gln
305             310             315             320

Leu Leu Ile Asn Asp Gln Lys Asp Arg Tyr Asp Lys Glu Gln Ser Ser
            325             330             335

Ala Asn Tyr Lys Val Trp Thr Asp Lys Gly Trp Ser Asn Ile Asn Arg
        340             345             350

Val Ile Arg His Lys Thr Tyr Lys Arg Ile Phe Arg Ile Val Thr Lys
```

355                360                365

```
Thr Ser Ile Ile Asp Val Thr Glu Asp His Ser Leu Ile Asp Lys Asn
    370             375             380

Gly Asn Tyr Ile Thr Pro Lys Thr Xaa Thr Ile Gly Thr Glu Leu Met
385             390             395             400

Tyr Gly Ile Asn Asn Phe Asp Thr Ile Glu Phe Val Asp Ile Lys Ser
            405             410             415

Asn Lys Tyr Asp Ile Leu Ala Phe Ser Ser Asp Asp Lys Val Glu Xaa
            420             425             430

Met Arg Tyr Tyr Xaa Tyr Asn Lys Lys Leu Gly Tyr Asn Ile Leu Ile
            435             440             445

Asp Val Asn Asn Asp Lys Phe Val Leu His Arg Thr Asn Asp Val Ile
        450             455             460

Glu Asn Pro Asn Lys Ile Ile Asn Ile Gln Gln Leu Asn Asp Val Val
465             470             475             480

Asp Asp Tyr Val Tyr Asp Leu Glu Thr Glu Val Gly His Phe His Ala
            485             490             495

Gly Ile Gly Glu Ile Ile Val Lys Asn
        500             505
```

```
<210>   236
<211>   355
<212>   PRT
<213>   Artificial sequence

<220>
<223>   modified contiguous intein from Catovirus CTV1932-1286


<220>
<221>   misc_feature
<222>   (104)..(104)
<223>   Xaa can be any naturally occurring amino acid

<220>
<221>   misc_feature
<222>   (114)..(114)
<223>   Xaa can be any naturally occurring amino acid

<220>
<221>   misc_feature
<222>   (157)..(157)
<223>   Xaa can be any naturally occurring amino acid
```

```
<220>
<221>  misc_feature
<222>  (229)..(229)
<223>  Xaa can be any naturally occurring amino acid

<220>
<221>  misc_feature
<222>  (246)..(246)
<223>  Xaa can be any naturally occurring amino acid

<220>
<221>  misc_feature
<222>  (281)..(281)
<223>  Xaa can be any naturally occurring amino acid

<400>  236
```

Ser Val Thr Gly Glu Thr Pro Ile Leu Leu Lys Asn Pro Thr Thr Asn
1               5                   10                  15

Lys Ile Val Ile Lys Arg Ile Asp Glu Leu Gly Thr Glu Trp Lys Asn
                20                  25                  30

Tyr Asn Asn Tyr Lys Ser Ser Asp Ser Asn Arg Tyr Phe Arg Glu Leu
        35                  40                  45

Leu Thr Ile Leu Phe Lys Asn Lys Asn Val Glu Lys Lys Arg Glu Asn
    50                  55                  60

Phe Val Pro Val Ser Gln Trp Met Tyr Ile Lys Asn Asn Lys Tyr Arg
65                  70                  75                  80

Tyr Ile Asn Asp Ala Tyr Ser Asn Asn Thr Tyr Leu Gln Val Asn Leu
            85                  90                  95

Asn Asn Asn Lys Asn Met Ile Xaa Asp Val Glu Asp Ile Asp Ile Ile
            100                 105                 110

Asn Xaa Glu Leu Lys Asn Ile Ser Lys Met Ile Leu Asn Lys Ile Ile
        115                 120                 125

Lys Leu Phe Pro Lys Gln Ile Gln Ala Lys Leu Gln Lys Tyr Glu Val
    130                 135                 140

Asn Tyr Ile Asn Glu Asn Ser Leu Asp Asn Arg Lys Xaa Asn Leu Ser
145                 150                 155                 160

Ile Asp Val Asp Asn Gln Thr Ile Asp Lys Ile Ile Glu Asp Ile Glu
            165                 170                 175

Asn Asp Gln Lys Asp Arg Tyr Ser Lys Glu Gln Ser Thr Thr Asn Tyr
            180                 185                 190

```
Leu Ala Tyr Thr Asp Lys Gly Trp Ala Lys Ile Asn Lys Ile Ile Arg
        195                 200                 205

His Lys Thr Thr Lys Lys Ile Tyr Arg Ile Leu Thr Thr Asn Ser Ile
        210                 215                 220

Val Glu Val Thr Xaa Asp His Ser Leu Ile Asp Glu Asn Gly Asn Tyr
225                 230                 235                 240

Ile Met Pro Lys Asp Xaa Val Ile Gly Lys Gly Leu Met Gln Ser Tyr
                245                 250                 255

Pro His Thr Asn Lys Leu Lys Tyr Asn Asp Lys Tyr Asp Lys Ser Ile
            260                 265                 270

Phe Thr Asn Lys Asn Tyr Lys Glu Xaa Met Lys Tyr Tyr His Tyr Asn
        275                 280                 285

Lys Ser His Gly Phe Asn Ile Thr Ile Asp Glu Asn Asn Gly Ile Ile
    290                 295                 300

Thr Leu Lys Arg Thr Lys Asp Asn Ile Asn Asn Ile Asn Lys Ile Val
305                 310                 315                 320

Lys Ile Phe Asp Leu Gly Asp Ile Ser Thr Asp Arg Tyr Val Tyr Asp
                325                 330                 335

Leu Glu Thr Glu His Gly Arg Phe His Ala Gly Val Gly Glu Leu Ile
            340                 345                 350

Val Thr Asn
        355
```

```
<210>  237
<211>  444
<212>  PRT
<213>  Artificial sequence

<220>
<223>  modified contiguous intein from coal oil point, Santa-Barbara
       California, crude oil metagenome 3 and 7

<220>
<221>  misc_feature
<222>  (28)..(28)
<223>  Xaa can be any naturally occurring amino acid

<220>
<221>  misc_feature
```

```
<222>  (48)..(48)
<223>  Xaa can be any naturally occurring amino acid


<220>
<221>  misc_feature
<222>  (57)..(57)
<223>  Xaa can be any naturally occurring amino acid


<220>
<221>  misc_feature
<222>  (104)..(104)
<223>  Xaa can be any naturally occurring amino acid


<220>
<221>  misc_feature
<222>  (171)..(171)
<223>  Xaa can be any naturally occurring amino acid


<220>
<221>  misc_feature
<222>  (252)..(252)
<223>  Xaa can be any naturally occurring amino acid


<220>
<221>  misc_feature
<222>  (278)..(278)
<223>  Xaa can be any naturally occurring amino acid


<220>
<221>  misc_feature
<222>  (341)..(341)
<223>  Xaa can be any naturally occurring amino acid


<220>
<221>  misc_feature
<222>  (353)..(353)
<223>  Xaa can be any naturally occurring amino acid


<220>
<221>  misc_feature
<222>  (373)..(373)
<223>  Xaa can be any naturally occurring amino acid


<220>
<221>  misc_feature
<222>  (435)..(435)
<223>  Xaa can be any naturally occurring amino acid


<400>  237

Ser Val Asp Ala Gly Thr Asp Ile Ile Ile Arg Glu Gly Lys Leu Val
1               5                   10                  15


Lys Ile Val Asn Ile Lys Asp Leu Phe Glu Ser Xaa Asp Ser Glu Ile
            20                  25                  30


Tyr Arg Lys Gly Asp His Glu Tyr Lys Ala Pro Glu Lys Ile Glu Xaa
        35                  40                  45


Leu Ser Val Ser Lys Glu Gly Ile Xaa Glu Trp Lys Lys Ala Asn Ser
```

50        55        60

Ile Lys Arg His Pro Tyr Glu Gly Lys Ile Leu Asn Ile Gln Thr Arg
65        70        75        80

Arg Gly Ser Ile Ser Val Thr Lys Asn His Ser Leu Tyr Ala Leu Ser
       85        90        95

Ser Gly Leu Lys Glu Ile Leu Xaa Arg Asp Leu Asn Lys Asn Thr Pro
       100        105        110

Leu Ala His Ile Ser Lys Tyr Ser Gln Ala Glu Lys Lys Leu Lys Ile
       115        120        125

Asn Ala Leu Glu Pro Leu Arg Lys Phe Ser Asn Glu Leu Asn Ile Trp
       130        135        140

Leu Ser Val Pro Ile Asn Asp Leu Thr Lys Arg Leu Leu Lys Tyr His
145        150        155        160

Gln Pro Arg Asn Asn Phe Lys Gly Gly Arg Xaa Lys Lys Glu Phe Ile
       165        170        175

Arg Ile Pro Ile Pro Thr Ala Ile Glu Leu Tyr Asn Lys Lys Val Ile
       180        185        190

Glu Asp Lys Asp Ile Gln Asn Ala Phe Ile Ser Ser Tyr Gly Gly Lys
       195        200        205

Gly Lys Ile Pro Val Ile Tyr Glu Leu Asn Lys Asp Phe Ala Arg Ile
       210        215        220

Leu Gly Ala Tyr Ala Ala Glu Gly Ser Leu His Ile Arg Lys Arg Lys
225        230        235        240

Gly Ile Ser Lys Glu Gly Ala Tyr Ile Phe Ala Xaa Gly His Asp Ile
       245        250        255

Gln Ser Leu Glu Glu Leu Lys Lys Ile Leu Ala Arg Ile Phe Arg Arg
       260        265        270

Asn Phe Asn Val Thr Xaa Ser Gly Val Asp Lys Asn Gly Arg Asn Phe
       275        280        285

Arg Ile Lys Ser Asn Ser Ala Val Ala Tyr Leu Phe Lys Phe Val Leu
       290        295        300

```
Asp Val Gly Gln Gly Ser Gln Gly Lys Glu Val Ser Pro Tyr Ile Leu
305             310             315                     320


Ser Ser Ser Lys Ser Ile Gln Arg Ala Phe Phe Asp Glu Tyr Thr Lys
                325             330                     335


Gly Glu Gly Tyr Xaa Asp Lys Arg Arg Arg Val Asn Pro Leu Leu Glu
            340             345             350


Xaa Thr Thr Lys Ser Lys Lys Leu Ala Glu Gly Leu Ser Leu Met Ala
            355             360             365


Ile Asn Leu Asn Xaa Gly Leu Pro Ser Ile Arg Phe Arg Lys Glu Asn
    370             375             380


Ser Ser Tyr Gln Leu Arg Phe Val Gln Tyr Asp Leu Asn Ser Val Lys
385             390             395                     400


Tyr Arg Asp Leu Ser Gly Leu Leu Pro Lys Glu Ile Lys Glu Val Lys
            405             410             415


Pro Thr Asp Gly Tyr Val Tyr Asp Val Gly Val Glu Gly Asn Asn Asn
            420             425             430


Phe Val Xaa Ala Lys Gly Leu Ile Leu Ala His Asn
            435             440
```

```
<210>  238
<211>  186
<212>  PRT
<213>  Artificial sequence

<220>
<223>  modified contiguous intein (partial) from Euryarchaeon species
       RBG_13_31_8


<220>
<221>  misc_feature
<222>  (48)..(48)
<223>  Xaa can be any naturally occurring amino acid

<220>
<221>  misc_feature
<222>  (57)..(57)
<223>  Xaa can be any naturally occurring amino acid

<220>
<221>  misc_feature
<222>  (104)..(104)
<223>  Xaa can be any naturally occurring amino acid

<220>
<221>  misc_feature
```

```
<222>  (109)..(109)
<223>  Xaa can be any naturally occurring amino acid

<220>
<221>  misc_feature
<222>  (186)..(186)
<223>  Xaa can be any naturally occurring amino acid

<400>  238
```

Ser Val Asp Ser Glu Thr Asp Ile Ile Ile Lys Gly Asn Asn Leu Ile
1               5                   10                  15

Lys Ile Thr Asn Ile Lys Asp Leu Phe Glu Lys Gln Ser Ser His Ile
            20                  25                  30

Tyr Lys Lys Glu Asn His Glu Tyr Lys Lys Leu Lys Asp Ile Glu Xaa
        35                  40                  45

Leu Ser Val Asn Asn Asn Gly Ile Xaa Glu Trp Lys Lys Ala Asn Phe
    50                  55                  60

Ile Lys Arg His Pro Tyr Lys Asn Asp Ile Ile Lys Val Lys Thr Gln
65                  70                  75                  80

Arg Gly Met Ile Ser Val Thr Lys Asn His Ser Leu Tyr Thr Ile Ser
            85                  90                  95

Lys Asn Ile Lys Glu Ile Tyr Xaa Lys Gln Leu Asn Xaa Lys Asp Thr
            100                 105                 110

Asn Ile Val His Ile Ser Asn Phe Asn Glu Lys Gly Lys Lys Ile Ile
        115                 120                 125

Ile Asn Ala Leu Asn Pro Leu Val Glu Phe Asn Asn Glu Leu Asn Ile
    130                 135                 140

Val Phe Asn Ile Pro Leu Lys Asn Ser Thr Lys Asn Leu Leu Lys Tyr
145                 150                 155                 160

Tyr Gln His Arg Lys Asn Tyr Lys Gly Lys Val Thr Leu Asn Lys Lys
            165                 170                 175

Tyr Ile Arg Ile Lys Leu Ile Asp Ala Xaa
            180                 185

```
<210>  239
<211>  393
<212>  PRT
<213>  Artificial sequence
```

<220>
<223>  modified contiguous intein from coal oil point, Santa-Barbara
       California, crude oil metagenome 7


<220>
<221>  misc_feature
<222>  (7)..(7)
<223>  Xaa can be any naturally occurring amino acid

<220>
<221>  misc_feature
<222>  (28)..(29)
<223>  Xaa can be any naturally occurring amino acid

<220>
<221>  misc_feature
<222>  (82)..(82)
<223>  Xaa can be any naturally occurring amino acid

<220>
<221>  misc_feature
<222>  (171)..(171)
<223>  Xaa can be any naturally occurring amino acid

<220>
<221>  misc_feature
<222>  (177)..(177)
<223>  Xaa can be any naturally occurring amino acid

<220>
<221>  misc_feature
<222>  (267)..(267)
<223>  Xaa can be any naturally occurring amino acid

<220>
<221>  misc_feature
<222>  (321)..(321)
<223>  Xaa can be any naturally occurring amino acid

<220>
<221>  misc_feature
<222>  (386)..(386)
<223>  Xaa can be any naturally occurring amino acid

<400>  239

Ser Ile Val Gly Ala Arg Xaa Ile Ala Ile Lys Asp Asp Asn Leu Ile
1               5                   10                  15


Asn Ile Ile Pro Ile Glu Glu Leu Trp Lys Lys Xaa Xaa Glu Pro Met
            20                  25                  30


Glu Lys Ile Asn Gly Lys Glu Ile Lys Thr Pro Gln Ser Val Phe Thr
        35                  40                  45


Leu Ser Lys Asn Gly Glu Trp Gln Lys Ile Lys Lys Ile Ile Arg His
    50                  55                  60

```
Lys Thr Asn Lys Ile Ile Phe Arg Ile Asn Gln Lys Asn Gly Glu Thr
65              70              75                  80


Ile Xaa Thr Glu Asp His Ser Leu Ile Thr Glu Asn Tyr Lys Pro Ile
        85                  90                  95


Lys Pro Lys Glu Leu Gly Asp Lys Lys Ile Leu Phe Leu Lys Lys Val
        100                 105                 110


Pro His Glu Pro Thr Ile Phe Asp Arg Lys Ile Asp Leu Tyr Pro Leu
        115                 120                 125


Val Ser His Tyr Ser Phe Glu Thr Glu Tyr Lys Glu Arg Lys Lys Gln
        130                 135                 140


Asn Ala Trp Lys Ala Asp Asn Lys Phe Leu Trp Phe Gly Trp Thr Ser
145                 150                 155                 160


Arg Glu Asn Gln His Lys Ile Asn Arg Phe Xaa Asn Leu Ser Asp Leu
            165                 170                 175


Xaa Lys Leu Leu Gly Ile Tyr Ile Ala Asp Gly His Ser Ser Leu His
            180                 185                 190


Lys Gly Lys Tyr Gly Ile Lys Ala Thr Ala Gly Ile Ser Ser Lys Asp
            195                 200                 205


Thr Leu Phe Leu Asn Glu Leu Lys Glu Ile Met Lys Arg Ile Asp Tyr
    210                 215                 220


Asn His Gln Ile Ser Ile Leu Arg Val Ser Lys Gly Glu Arg Val Ile
225                 230                 235                 240


Gln Gly Tyr Lys Tyr Glu Asp Arg Thr Tyr Arg Leu Gln Thr Asn Ser
            245                 250                 255


Thr Thr Trp Thr Ala Phe Phe Ser Ser Leu Xaa Gly Ser Gly Ser Glu
            260                 265                 270


Asn Lys His Leu Pro Gln Phe Ile Phe Asn Val Glu Lys Lys Tyr Gln
            275                 280                 285


Glu Leu Leu Tyr Lys Tyr Tyr Leu Leu Gly Asp Gly Ser Val Glu Gln
            290                 295                 300


Gly Lys Asn Thr Phe Thr Ser Lys Ser Leu Gln Leu Val Ser Gly Leu
305                 310                 315                 320
```

```
Xaa Phe Leu Leu Lys Ser Trp Asn Ile Asp Thr Ser Ile Tyr Tyr His
            325             330             335


Glu Lys Arg Asp Val Tyr Arg Val Arg Glu Arg Glu Arg Ala Val Asp
            340             345             350


Ser Met His Pro Ile Lys Thr Lys Leu Ile Glu Leu Pro Lys Val Glu
            355             360             365


Arg Tyr Val Tyr Asp Leu Ser Val Glu Asn Thr Glu Met Phe Val Asp
     370             375             380


Ala Xaa Gly Met Leu Leu Leu His Asn
385             390
```

```
<210>  240
<211>  359
<212>  PRT
<213>  Artificial sequence

<220>
<223>  modified contiguous intein from Chlamydomonas sphaeroides
       NIES-2242


<220>
<221>  misc_feature
<222>  (19)..(19)
<223>  Xaa can be any naturally occurring amino acid

<220>
<221>  misc_feature
<222>  (156)..(156)
<223>  Xaa can be any naturally occurring amino acid

<220>
<221>  misc_feature
<222>  (182)..(182)
<223>  Xaa can be any naturally occurring amino acid

<220>
<221>  misc_feature
<222>  (222)..(224)
<223>  Xaa can be any naturally occurring amino acid

<220>
<221>  misc_feature
<222>  (239)..(239)
<223>  Xaa can be any naturally occurring amino acid

<220>
<221>  misc_feature
<222>  (260)..(260)
<223>  Xaa can be any naturally occurring amino acid

<400>  240
```

```
Ser Val Thr Gly Tyr Thr Pro Val Leu Val Arg Ile Gln His Lys Val
1               5                   10              15

Arg Tyr Xaa Thr Ile Glu Lys Leu Pro Gly Leu Leu Gly Gly Gly Glu
        20                  25                  30

Trp Val Pro Val Val Pro Ala Glu Pro Ala Ala His His Glu Lys Pro
        35              40                  45

Lys Glu Ala Leu Asp Leu Thr His His His Val Glu Thr Trp Thr Glu
        50              55                  60

Ser Gly Trp Thr Arg Leu Arg Arg Ile Ile Arg His Ala Leu Pro Pro
65                  70                  75                  80

Gly Lys Arg Ile Leu Arg Ile Thr Thr Pro Thr Gly Val Val Asp Ala
            85                  90                  95

Thr Asp Asp His Ser Leu Leu Ala Pro Asn Gly Asp Pro Ile Thr Pro
            100                 105                 110

Arg Asn Leu His Ile Gly Thr Pro Leu Met His Ala Pro Leu Pro Leu
        115                 120                 125

Asp Glu Trp Arg Arg Met Ala Gln Thr Ala Ala Arg Thr Thr Thr Pro
        130             135                 140

Glu Arg Ala Arg Ile Leu Gly Ile Phe Val Ala Xaa Arg Ala Met Ala
145                 150                 155                 160

Phe Ser Gln Arg Gly Phe Ile Trp Thr Ala Ser Lys Ser Ile Gly Ser
            165                 170                 175

Tyr Phe Met Asp Leu Xaa Asn Lys Glu Phe Gly Gly Phe Thr Trp Thr
            180                 185                 190

Met Thr Thr Ser Pro Glu Asp Gly Ile Thr Val Val Lys Pro Glu Gly
        195                 200                 205

Val Pro Gly His Ile Leu Tyr Ser Thr Leu Leu Gln Thr Xaa Xaa Xaa
        210                 215                 220

Gly Gly Ser Ala Asp Glu Pro Met Val Pro Asp Asp Val Leu Xaa Ser
225                 230                 235                 240

Asn Ser Ile His Val His Met Ala Phe Ile Glu Gly Phe Arg Met Gly
            245                 250                 255
```

```
Asn Pro Met Xaa Leu Ile Phe Thr His Leu Leu Gly Ala Thr Leu Phe
            260             265             270

Val Leu Leu Gln Phe Met Ile Glu Ala His Gly Asp Ser Gly Ala Thr
            275             280             285

Asp Val Arg Val Ala Gly Ser Met Pro Asn Ile Asn Leu Val Thr Ser
    290             295             300

Pro Ser Ser Thr Pro Ser Thr Glu Asp Ser Thr Ser Val Met Ser Met
305             310             315             320

Val Asp Ile Thr Asp Ala Leu Arg Ser Lys Gly Asp Pro Thr Leu Leu
            325             330             335

Met Val Tyr Asp Leu Thr Thr Asp Asn His His Phe Ala Ala Gly Val
            340             345             350

Gly Gln Met Val Val His Asn
            355
```

```
<210>  241
<211>  337
<212>  PRT
<213>  Artificial sequence

<220>
<223>  modified contiguous intein from Marine sediment metagenome LCGC14


<220>
<221>  misc_feature
<222>  (143)..(143)
<223>  Xaa can be any naturally occurring amino acid

<220>
<221>  misc_feature
<222>  (335)..(335)
<223>  Xaa can be any naturally occurring amino acid

<400>  241
```

```
Ser Ile Met Gly Thr Glu Ala Val Val Val Arg Phe Ala Asp Asp Glu
1               5               10              15

Pro Pro Met Ile Leu Ser Ile Gln Glu Leu Trp Asp Met Ala His Glu
            20              25              30

Lys Gly Tyr Gln Ile Val Tyr Arg Ala Asp Gly Lys Glu Gln Leu Val
            35              40              45

Val Gln Gly Leu Arg Val Trp Asp Gly Lys Gly Trp Asn Arg Val Tyr
```

50                         55                         60

```
Lys Leu Ile Arg His Phe Thr Ile Lys Thr Ile Tyr Arg Thr Ala Thr
65              70              75                      80

Gly Lys Gly Ala Val Asp Thr Thr Glu Asp His Ser Leu Val Asp Asp
                85              90                      95

Glu Gly His Glu Phe Lys Pro Glu His Ile Ser Ser Arg Gly Lys Glu
                100             105             110

Pro Val Ser Ser Gln Phe Ile Leu Pro Ser Lys Arg Leu Ser Tyr Thr
                115             120             125

Asp Asp Ile Ser Ala Thr Leu Leu Leu Phe Leu Phe Ser Met Xaa Gly
                130             135             140

Thr Ala Ser Gly Thr Tyr Thr Gly Tyr Pro Ser Gln Arg Thr Val Ala
145             150             155                     160

Leu His Phe Ser Pro Lys Ile Lys Asp Ile Ala Gln Lys Leu Trp Trp
                165             170             175

Glu Ala Lys Pro Ala Ala Ala Ala Phe Gly Ala Lys His Lys Leu Tyr
                180             185             190

Ser Thr Lys Lys Glu Lys Leu Val Ile Lys Phe Ser Asn His Thr Gln
                195             200             205

Leu Trp Ala Phe Val Arg Arg Asn Leu Tyr Ile Lys Asn Gln Lys Ile
                210             215             220

Lys Pro Leu Pro Ser Val Leu Ser Leu Pro Glu His Tyr Leu Glu Thr
225             230             235                     240

Ile Tyr Gly Met Leu Lys Asp Tyr Tyr Asn Phe Asp Glu Lys Thr
                245             250             255

Lys Gln Asp Arg Trp Thr Ile Lys Ser Ser Ser Glu Met Val Leu Phe
                260             265             270

Thr Ala Leu Ala Asn Arg Phe Lys Glu Glu Met Thr Val Val Pro Gln
                275             280             285

Pro Lys Gly Lys Ile Tyr Gln Ile Lys Arg Thr Ser Asp Met Lys Arg
                290             295             300
```

```
Arg Ala Thr Val Ser Ile Thr Arg Ile Leu Pro Asp Tyr Val Tyr Asp
305                 310             315                 320


Met Glu Thr Glu Asp Gly Thr Phe Met Ala Gly Asn Ile Leu Xaa His
                325                 330                 335


Asn



<210>  242
<211>  309
<212>  PRT
<213>  Artificial sequence

<220>
<223>  modified contiguous intein from Russia Kulunda-steppe soda lake
       Tanatar-5 brine environmental genomics


<220>
<221>  misc_feature
<222>  (37)..(37)
<223>  Xaa can be any naturally occurring amino acid

<220>
<221>  misc_feature
<222>  (48)..(48)
<223>  Xaa can be any naturally occurring amino acid

<220>
<221>  misc_feature
<222>  (123)..(123)
<223>  Xaa can be any naturally occurring amino acid

<220>
<221>  misc_feature
<222>  (173)..(173)
<223>  Xaa can be any naturally occurring amino acid

<220>
<221>  misc_feature
<222>  (212)..(212)
<223>  Xaa can be any naturally occurring amino acid

<400>  242

Ser Val Thr Gly Tyr Thr Pro Val Tyr Val Lys Asp Gln Asn Gly Ser
1               5                   10                  15


Ile Met Leu Val Thr Ile Glu Ser Leu Ala Glu Lys Tyr Gly Asp Ser
                20                  25                  30


Thr Trp Ile Pro Xaa Val Glu Asn Gly Arg Gln Thr Lys Glu Ala Xaa
                35                  40                  45


Glu Leu Val Gly Leu Glu Thr Trp Thr Glu Lys Gly Trp Thr Pro Leu
        50                  55                  60
```

Lys Arg Val Ile Arg His Ile Leu Ala Pro His Lys Lys Ile Ile Arg
65                  70              75                  80

Ile Val Thr His Thr Gly Ile Val Asp Val Thr Asp Asp His Ser Leu
                85              90                  95

Leu Asp Ala Glu Gly Asn Pro Val Thr Ser Lys Asp Ile Ser Met Gly
            100             105             110

Thr Pro Leu Leu His His Val Leu Pro Pro Xaa Glu Glu Glu Glu Glu
        115             120             125

Glu Glu Gly Lys Glu Glu Glu Lys Glu Ala Glu Val Met Gly Phe Ser
    130             135             140

Tyr Gly Glu Gly Glu His Asp Glu Ile Pro Arg His Ile Phe Asp Ala
145             150             155             160

Pro Leu His Ile Lys Gln Ala Phe Trp Lys Gly Leu Xaa Asp Ala Lys
            165             170             175

Arg Gly Asp Glu Lys Asp Val Glu Gly Asn Thr Thr Phe Asp His Arg
            180             185             190

Ser Met Leu Gly Ala Ser His Ile Ala Arg Leu Ala Thr Tyr Leu Gly
        195             200             205

Tyr Ser Tyr Xaa Leu Ser Ile Asp Ser Met Asp Ser Met Asp Ser Met
    210             215             220

Asp Ser Met Lys Gln Asp Lys Phe Arg Ser Ala Gly Ala Lys Phe Arg
225             230             235             240

Ser Ala Gly Thr Lys Phe Arg Ile Thr Leu Lys Ser Thr Thr Ser His
            245             250             255

Glu Thr Arg Gly His Thr Asp His Thr Asp His Thr Asp Asn Ser Asp
            260             265             270

Arg Thr Thr Ile Ser Lys Met Tyr Glu Ile Pro Tyr Glu Gly Tyr Val
        275             280             285

Tyr Asp Leu Thr Thr Glu Asn His His Phe Ala Gly Gly Val Gly Gln
    290             295             300

Leu Ile Leu His Asn

305

<210> 243
<211> 303
<212> PRT
<213> Artificial sequence

<220>
<223> modified contiguous intein from Chlamydomonas asymmetrica
      NIES-2207


<220>
<221> misc_feature
<222> (5)..(5)
<223> Xaa can be any naturally occurring amino acid

<220>
<221> misc_feature
<222> (39)..(39)
<223> Xaa can be any naturally occurring amino acid

<220>
<221> misc_feature
<222> (57)..(57)
<223> Xaa can be any naturally occurring amino acid

<220>
<221> misc_feature
<222> (95)..(95)
<223> Xaa can be any naturally occurring amino acid

<220>
<221> misc_feature
<222> (141)..(141)
<223> Xaa can be any naturally occurring amino acid

<220>
<221> misc_feature
<222> (156)..(156)
<223> Xaa can be any naturally occurring amino acid

<220>
<221> misc_feature
<222> (262)..(262)
<223> Xaa can be any naturally occurring amino acid

<400> 243

Ser Val Ala Ala Xaa Thr Pro Val Ile Val Arg Ser Ser Ala Asp Ala
1               5                   10                  15


Asp Asp Trp Asp Ile Leu Pro Ile Glu Leu Val Pro Tyr Ala Tyr Gly
            20                  25                  30


Asp Ala His Trp Val Arg Xaa His Asp Glu Ser Gly Asp Asp Asn Ser
        35                  40                  45


Ser Asp Val Asp Ala Lys Glu Ala Xaa Glu Leu Tyr Asp Arg Val Glu

```
            50                      55                      60

        Ala Trp Thr Glu Asp Gly Trp Thr Pro Leu His Arg Val Ile Arg His
        65                  70                  75                  80

        Val Leu Pro Gln His Thr Ser Leu Thr Arg Val Val Thr Pro Xaa Gly
                        85                  90                  95

        Leu Val Asp Val Thr Asn Asp His Ser Leu Leu Thr Ala Pro Pro Glu
                        100                 105                 110

        Pro Val Pro Val Ser Pro Arg Asp Val Val Ala Gly Ser Thr Arg Leu
                        115                 120                 125

        Leu His His Ala Ala Tyr Pro Pro Ala Pro Thr Ser Xaa Ser Ser Ile
                        130                 135                 140

        Asn Glu Asp Trp Ala Gln Arg Ala Ala Val Glu Xaa Val Ile Thr Ala
        145                 150                 155                 160

        Thr Phe Asn Gly Ser Ala Ala Leu Pro Trp Gln Leu Lys Gln Val Ala
                        165                 170                 175

        Met Ala Pro Gly Arg Ile Val Arg Glu Phe Trp Lys Ala Met Leu Gly
                        180                 185                 190

        Thr Met Lys Ala Leu Arg Ala Ser Pro Gly Ala Asp Ile Leu Ser Leu
                        195                 200                 205

        Arg Met Ser Gln Ala Ser Ala Ala Trp Leu Leu Val Val Ala Ala Arg
                        210                 215                 220

        Ile Gln Glu Arg Ala Thr Val His Asp Thr Thr Ser His Asp Thr Trp
        225                 230                 235                 240

        Met Gly Asp Asp Val Val Val Val Ser Phe Ser Ala Thr Pro Ser Ser
                        245                 250                 255

        Ser Leu Gln Ser His Xaa Asp Asp His Val Val Arg Thr Val Arg Ala
                        260                 265                 270

        Leu Pro Thr Ser Ser Ile Pro Arg Tyr Val Tyr Asp Leu Thr Thr Asp
                        275                 280                 285

        Asn His His Phe Ala Ala Gly Pro Gly Arg Met Val Val His Asn
                        290                 295                 300
```

```
<210>   244
<211>   210
<212>   PRT
<213>   Artificial sequence

<220>
<223>   modified contiguous intein from Gulf of Maine environmental
        sampling


<220>
<221>   misc_feature
<222>   (28)..(28)
<223>   Xaa can be any naturally occurring amino acid

<400>   244

Ser Val Met Gly Tyr Thr Pro Ile Val Leu Lys Asn Asp Lys Asp Ile
1               5                   10                  15


Ile Asn Ile Ile Ser Phe Asp Asp Leu Glu Tyr Xaa Lys Trp Leu Ser
            20                  25                  30


Tyr Asn Asn Leu Asp Lys Asn Gly Ser Asn Lys Glu Gln Leu Phe Asn
        35                  40                  45


Pro Gly Tyr Phe Ile Trp Thr His Asn Gly Trp Ser Pro Ile Ile Arg
    50                  55                  60


Phe Ile Arg His Lys Thr Ile Lys Gln Ile Tyr Arg Ile Ile Thr Asn
65                  70                  75                  80


Ser Gly Ile Ile Asp Val Thr Glu Asp His Ser Leu Leu Asp Leu Asn
                85                  90                  95


Val Lys Glu Ile Lys Pro Asn Gln Leu Lys Ile Asn Asp Asn Leu Leu
            100                 105                 110


His Ser Lys Ile Lys Thr Lys Lys Ile Asn Lys Glu Ser Ser Lys Phe
        115                 120                 125


Phe Asn Ile Asn Asn Lys Leu Ile Tyr Lys Asp Leu Gln Asp Phe Val
        130                 135                 140


Leu Arg Ser Lys Asn Ile Lys Ile Glu Lys Ile Asn Asn Asn Asp Tyr
145                 150                 155                 160


Met Ile Val Asn Val Asp Tyr Lys Tyr Arg Asp Glu Asn Lys Ile Ile
                165                 170                 175


Glu Ile Ile Lys Leu His Glu Lys Tyr Glu Gly Tyr Val Tyr Asp Ile
                180                 185                 190
```

```
Glu Thr Lys Glu Gly Val Phe Asn Ala Gly Ile Gly Asn Leu Val Val
        195                 200                 205

Lys Asn
    210
```

<210> 245
<211> 211
<212> PRT
<213> Artificial sequence

<220>
<223> modified contiguous intein (partial) from Block Island
      environmental sampling

<220>
<221> misc_feature
<222> (105)..(105)
<223> Xaa can be any naturally occurring amino acid

<220>
<221> misc_feature
<222> (112)..(112)
<223> Xaa can be any naturally occurring amino acid

<220>
<221> misc_feature
<222> (171)..(171)
<223> Xaa can be any naturally occurring amino acid

<220>
<221> misc_feature
<222> (211)..(211)
<223> Xaa can be any naturally occurring amino acid

<400> 245

```
Ser Val Met Pro Tyr Thr Pro Leu Thr Ile Leu Arg Asp Asp Gly Val
1               5               10                  15

Val Glu Val Thr Thr Phe Asn Asp Phe Asn Asp Leu Glu Trp Thr Ser
            20                  25                  30

Tyr Ser Glu Phe Asn Lys Val Gly Thr His Lys Glu Gln Ile Phe Asn
        35                  40                  45

Pro Gly Phe Lys Val Trp Thr Asn Asn Gly Trp Ser Ser Val Val Arg
    50                  55                  60

Leu Ile Arg His Lys Thr Val Lys Lys Ile Tyr Arg Val Leu Thr Asn
65                  70                  75                  80

Ser Gly Leu Val Asp Val Thr Glu Asp His Arg Leu Leu Asp Lys Asp
```

```
                    85                    90                    95


      Leu Asn Ile Ile Lys Pro Ser Gln Xaa Glu Lys Gly Gln Glu Leu Xaa
              100                 105                 110


      His Ser Lys Ile Lys Ile Gly Lys His Ile Leu Lys Tyr Arg Lys Gln
              115                 120                 125


      Asp Glu Ile Tyr Ile Glu Lys Tyr Gly Lys Ile Tyr Leu Asn Asp Asp
              130                 135                 140


      Gln Gln Asn Glu Ala Gln Tyr Ile Tyr Ile Leu Ser Gln His Phe Asn
      145                 150                 155                 160


      Asp Asp Asn Ile Thr Ile Ser Ile Glu Asn Xaa Lys Ile Val Leu Ser
              165                 170                 175


      Tyr Glu Glu Arg Glu Ile Met Lys Glu Asp Lys Ser Lys Thr Arg Ile
              180                 185                 190


      Gln Ser Ile Tyr Val Leu Tyr Glu Tyr Tyr Gly Asp Thr Thr Pro Thr
              195                 200                 205


      Pro Tyr Xaa
              210


      <210>  246
      <211>  184
      <212>  PRT
      <213>  Artificial sequence

      <220>
      <223>  modified contiguous intein from Aureococcus anophagefferens virus
             BtV-01


      <220>
      <221>  misc_feature
      <222>  (36)..(36)
      <223>  Xaa can be any naturally occurring amino acid

      <220>
      <221>  misc_feature
      <222>  (47)..(47)
      <223>  Xaa can be any naturally occurring amino acid

      <220>
      <221>  misc_feature
      <222>  (52)..(52)
      <223>  Xaa can be any naturally occurring amino acid

      <220>
      <221>  misc_feature
      <222>  (85)..(86)
```

<223> Xaa can be any naturally occurring amino acid

<400> 246

```
Ser Val Thr Gly Tyr Thr Pro Ile Thr Ile Lys Tyr Lys Glu Gln Ile
1               5               10              15

Phe Ile Glu Lys Ile Glu Asn Val Ala Lys Ile Phe Gly Glu Asp Lys
            20              25              30

Trp Gln Lys Xaa Ile Asp Pro Gly Lys Gln Glu Lys Glu Ala Xaa Glu
            35              40              45

Leu Asn Glu Xaa Phe Thr Trp Thr Ser Lys Gly Trp Thr Lys Leu His
        50              55              60

Arg Val Ile Arg His Ile Leu Val Lys Glu Lys Lys Ile Ile Arg Val
65              70              75              80

Leu Thr His Thr Xaa Xaa Val Asp Val Thr Asp Asp His Ser Leu Ile
            85              90              95

Leu Lys Ser Gly Glu Glu Ile Ser Pro Lys Asp Leu Lys Ile Gly Asp
            100             105             110

Glu Leu Leu Gln Arg Asp Ile Glu Phe Asp Glu Ile Ile Glu Tyr Ser
        115             120             125

Asn Asp Glu Tyr Val Lys Lys Gln Ile Lys Tyr Leu Lys Glu Asn Met
    130             135             140

Lys Ser Glu Asn Glu Ser Ile Leu Ser Leu Asn Glu Ile Asp Tyr Lys
145             150             155             160

Gly Tyr Val Tyr Asp Leu Thr Thr Asp Asn His Glu Phe Gln Ala Gly
                165             170             175

Ile Gly Asn Ile Ile Val His Asn
                180
```

<210> 247
<211> 225
<212> PRT
<213> Artificial sequence

<220>
<223> modified contiguous intein from Browns Bank environmental
      sampling

<220>

<221> misc_feature
<222> (36)..(36)
<223> Xaa can be any naturally occurring amino acid

<220>
<221> misc_feature
<222> (88)..(88)
<223> Xaa can be any naturally occurring amino acid

<220>
<221> misc_feature
<222> (110)..(110)
<223> Xaa can be any naturally occurring amino acid

<220>
<221> misc_feature
<222> (121)..(121)
<223> Xaa can be any naturally occurring amino acid

<220>
<221> misc_feature
<222> (183)..(183)
<223> Xaa can be any naturally occurring amino acid

<400> 247

Ser Val Ala Asn Tyr Thr Pro Ile Tyr Val Lys Ile Asp Gly Lys Phe
1               5                   10                  15

Glu Ile Ile Gln Ile Asp Glu Leu Gly Lys Lys Tyr Gly Asn Asp Asn
            20                  25                  30

Trp Lys Gln Xaa Val Glu Pro Gly Lys Gln Thr Lys Glu Tyr Ile Glu
        35                  40                  45

Leu Thr Asp Lys Asn Ile Tyr Thr Trp Thr Glu Asn Ser Trp Thr Gln
        50                  55                  60

Leu Lys Thr Ile Ile Arg His Lys Leu Ala Ser Glu Lys Lys Met Met
65                  70                  75                  80

Arg Ile Leu Thr His Thr Gly Xaa Val Asp Val Thr Asp Asp His Ser
                85                  90                  95

Leu Ile Arg Asn Asp Gly Val Glu Ile Ser Pro Lys Asp Xaa Glu Ile
                100                 105                 110

Gly Thr Glu Leu Leu His His Pro Xaa Leu Ser Asp Asn Ile Arg Asn
        115                 120                 125

Asp Ala Ser Asn Ser Thr Phe Val Gln Asp Glu Phe Asp Leu Ser Ile
        130                 135                 140

Pro Ser Asn His Ile Leu Met Ala Lys Tyr Ile His His Lys Tyr Asn

```
        145               150               155               160

Ala Thr Asp Thr Lys Tyr Lys Leu Val Ser His Asp Ser Asn Asp Ser
            165               170               175

Phe Met Leu Ser Ser Glu Xaa Ser Glu Thr Thr Gly His Lys Ile Lys
            180               185               190

Lys Ile Met Thr Leu Asp Asp Tyr Asp Asp Tyr Val Tyr Asp Leu Thr
            195               200               205

Thr Asp Asn His His Phe Ala Ala Gly Ile Gly Asn Met Ile Val His
    210               215               220

Asn
225


<210>  248
<211>  163
<212>  PRT
<213>  Artificial sequence

<220>
<223>  modified contiguous intein from Salicola phage SCTP-2


<220>
<221>  misc_feature
<222>  (119)..(119)
<223>  Xaa can be any naturally occurring amino acid

<220>
<221>  misc_feature
<222>  (125)..(125)
<223>  Xaa can be any naturally occurring amino acid

<400>  248

Ser Val Thr His Asp Thr Ile Ile Ser Thr Asn Asn Gly Glu Tyr Thr
1               5               10              15

Ile Glu Lys Leu Phe Val Asn Ser Ser Arg Phe Trp Glu Asp Ser Glu
            20              25              30

Thr Gly Lys Glu Tyr Ser Tyr Asp Pro Ser Ile Lys Val Lys Thr Tyr
            35              40              45

Asp Pro Gln Asn Gly Thr Ser Tyr Tyr Gly Glu Ile Asn Tyr Ile Tyr
        50              55              60

Arg His Lys Thr Ser Lys Ser Lys Trp Leu Ile Arg Asp Ser Asn Asn
65              70              75              80
```

344

```
Asn Glu Val Ile Val Thr Glu Asp His Ser Ile Met Ile Glu Asn Asp
                85                  90                  95

Asp Gly Met Ile Lys Ile Ser Pro Lys Asp Ile Val Lys Gly Glu Asp
            100                 105                 110

Val Leu Ile Thr Val Lys Xaa Gly Glu Val Leu Lys Xaa Ser Ile Glu
            115                 120                 125

Asp Ile Val Trp Leu Gly Tyr Phe Asp Asn Glu Tyr Val Tyr Asp Val
        130                 135                 140

Gly Met Lys Asn Ala Glu His Asn Trp Phe Phe Gly Asn Asn Ile Leu
145                 150                 155                 160

Leu Lys Asn
```

```
<210>  249
<211>  162
<212>  PRT
<213>  Artificial sequence

<220>
<223>  modified contiguous intein from sheep gut metagenome

<220>
<221>  misc_feature
<222>  (46)..(46)
<223>  Xaa can be any naturally occurring amino acid

<220>
<221>  misc_feature
<222>  (83)..(83)
<223>  Xaa can be any naturally occurring amino acid

<220>
<221>  misc_feature
<222>  (121)..(121)
<223>  Xaa can be any naturally occurring amino acid

<220>
<221>  misc_feature
<222>  (129)..(129)
<223>  Xaa can be any naturally occurring amino acid

<400>  249
```

```
Ser Val Val Gly Asp Ser Trp Ile Thr Leu Ser Asp Asp Ser Phe Met
1               5                   10                  15

Met Ile Ser Asp Leu Phe Asn Glu Gly Thr Asp Thr Glu Ile Asp Gln
            20                  25                  30
```

Phe Gly Lys Glu Arg Val Lys Ser Asn Arg Ser Ile Tyr Xaa Val Asp
35              40              45

Leu Thr Thr Gly Lys Ile Asp Lys Lys Pro Ile Lys Tyr Val Met Arg
50              55              60

His Lys Val Asn Lys Arg Leu Tyr Lys Val Phe Asn Asp Tyr Thr Tyr
65              70              75              80

Val Val Xaa Thr Glu Asp His Ser Leu Leu Lys Tyr Tyr Asp Thr Asn
85              90              95

Phe Lys Glu Val Lys Pro Asp Asp Ile Asp Lys Asp Thr Phe Leu Phe
100             105             110

Leu Lys Gly Glu Asn Asp Leu Leu Xaa Val Ser Gly Phe Ser Val Glu
115             120             125

Xaa Val Ser Asn Arg Asn Val Ala Tyr Val Tyr Asp Ile Glu Val Asp
130             135             140

Ser Asp Thr Asp Asn Tyr His Asn Phe Phe Ala Asn Gly Ile Leu Val
145             150             155             160

His Asn


<210>  250
<211>  164
<212>  PRT
<213>  Artificial sequence

<220>
<223>  modified contiguous intein from sheep gut metagenome


<220>
<221>  misc_feature
<222>  (46)..(46)
<223>  Xaa can be any naturally occurring amino acid

<220>
<221>  misc_feature
<222>  (53)..(53)
<223>  Xaa can be any naturally occurring amino acid

<220>
<221>  misc_feature
<222>  (83)..(83)
<223>  Xaa can be any naturally occurring amino acid

<220>
<221>  misc_feature

<222>  (129)..(129)
<223>  Xaa can be any naturally occurring amino acid

<400>  250

Ser Val Ile Gly Ser Ser Met Ile Ser Leu Ser Asp Asn Ser Leu Lys
1               5                   10                  15

Arg Ile Ser Thr Leu Phe Asn Glu Gly Asp Asn Ile Glu Thr Asp Val
            20                  25                  30

Phe Gly Lys Glu Arg Val Ser Ser Asp Lys Ser Val Tyr Xaa Leu Asn
            35                  40                  45

Thr Val Thr Gly Xaa Val Glu Ile Lys Lys Ile Lys Tyr Val Met Arg
        50                  55                  60

His Lys Leu Thr Lys Arg Leu Tyr Lys Val Ser Asn Gly Asp Tyr Glu
65                  70                  75                  80

Ile Ile Xaa Thr Glu Asp His Ser Ile Met Lys Leu Asp Asp Lys Ser
            85                  90                  95

Asn Lys Ile Ile Glu Val Arg Pro Ile Glu Ile Thr Lys Asn Asp Asn
            100                 105                 110

Leu Ile Val Arg Tyr Thr Asp Arg Thr Ile Tyr Val Ser Gly Phe Glu
            115                 120                 125

Xaa Thr Pro Ile Lys Asp Lys Arg Ile Lys Tyr Val Tyr Asp Ile Glu
    130                 135                 140

Val Asp Ser Asp Thr Asp Glu Tyr His Asn Phe Phe Ala Asn Gly Leu
145                 150                 155                 160

Leu Val His Asn

<210>  251
<211>  524
<212>  PRT
<213>  Artificial sequence

<220>
<223>  MBP-AesN-H6 (1)

<400>  251

Met Lys Thr Glu Glu Gly Lys Leu Val Ile Trp Ile Asn Gly Asp Lys
1               5                   10                  15

Gly Tyr Asn Gly Leu Ala Glu Val Gly Lys Lys Phe Glu Lys Asp Thr
20 25 30

Gly Ile Lys Val Thr Val Glu His Pro Asp Lys Leu Glu Glu Lys Phe
35 40 45

Pro Gln Val Ala Ala Thr Gly Asp Gly Pro Asp Ile Ile Phe Trp Ala
50 55 60

His Asp Arg Phe Gly Gly Tyr Ala Gln Ser Gly Leu Leu Ala Glu Ile
65 70 75 80

Thr Pro Asp Lys Ala Phe Gln Asp Lys Leu Tyr Pro Phe Thr Trp Asp
85 90 95

Ala Val Arg Tyr Asn Gly Lys Leu Ile Ala Tyr Pro Ile Ala Val Glu
100 105 110

Ala Leu Ser Leu Ile Tyr Asn Lys Asp Leu Leu Pro Asn Pro Pro Lys
115 120 125

Thr Trp Glu Glu Ile Pro Ala Leu Asp Lys Glu Leu Lys Ala Lys Gly
130 135 140

Lys Ser Ala Leu Met Phe Asn Leu Gln Glu Pro Tyr Phe Thr Trp Pro
145 150 155 160

Leu Ile Ala Ala Asp Gly Gly Tyr Ala Phe Lys Tyr Glu Asn Gly Lys
165 170 175

Tyr Asp Ile Lys Asp Val Gly Val Asp Asn Ala Gly Ala Lys Ala Gly
180 185 190

Leu Thr Phe Leu Val Asp Leu Ile Lys Asn Lys His Met Asn Ala Asp
195 200 205

Thr Asp Tyr Ser Ile Ala Glu Ala Ala Phe Asn Lys Gly Glu Thr Ala
210 215 220

Met Thr Ile Asn Gly Pro Trp Ala Trp Ser Asn Ile Asp Thr Ser Lys
225 230 235 240

Val Asn Tyr Gly Val Thr Val Leu Pro Thr Phe Lys Gly Gln Pro Ser
245 250 255

Lys Pro Phe Val Gly Val Leu Ser Ala Gly Ile Asn Ala Ala Ser Pro
260 265 270

```
Asn Lys Glu Leu Ala Lys Glu Phe Leu Glu Asn Tyr Leu Leu Thr Asp
        275             280             285

Glu Gly Leu Glu Ala Val Asn Lys Asp Lys Pro Leu Gly Ala Val Ala
        290             295             300

Leu Lys Ser Tyr Glu Glu Glu Leu Ala Lys Asp Pro Arg Ile Ala Ala
305             310             315             320

Thr Met Glu Asn Ala Gln Lys Gly Glu Ile Met Pro Asn Ile Pro Gln
        325             330             335

Met Ser Ala Phe Trp Tyr Ala Val Arg Thr Ala Val Ile Asn Ala Ala
        340             345             350

Ser Gly Arg Gln Thr Val Asp Glu Ala Leu Lys Asp Ala Gln Thr Asn
        355             360             365

Ser Ser Ser Asn Asn Asn Asn Asn Asn Asn Asn Asn Leu Gly Ile
        370             375             380

Glu Gly Arg Ile Ser Glu Phe Tyr Ile Asp Thr Asp Ser Val Val Gly
385             390             395             400

Asp Thr Ile Ile Asp Val Ser Gly Lys Lys Met Thr Ile Ala Glu Phe
            405             410             415

Tyr Asp Ser Thr Pro Asp Val Phe Met Arg Arg Asn Asp Glu Ala Arg
        420             425             430

Asp Trp Val Lys Arg Val Gly Gly Lys Thr Ser Leu Ser Val Asn Thr
        435             440             445

Tyr Ser Gly Glu Val Glu Arg Lys Asn Ile Asn Tyr Ile Met Lys His
        450             455             460

Thr Val Lys Lys Arg Met Phe Lys Ile Lys Ala Gly Gly Lys Glu Val
465             470             475             480

Ile Val Thr Ala Asp His Ser Val Met Val Lys Arg Asp Gly Lys Ile
            485             490             495

Ile Asp Val Lys Pro Thr Glu Met Lys Gln Thr Asp Arg Val Val Lys
        500             505             510

Trp Met Leu Thr Gly Ser His His His His His
        515             520
```

349

```
<210>    252
<211>    132
<212>    PRT
<213>    Artificial sequence

<220>
<223>    SBP-AesC-SBP (2)

<400>    252

Met Asp Glu Lys Thr Thr Gly Trp Arg Gly Gly His Val Val Glu Gly
1               5                   10                  15


Leu Ala Gly Glu Leu Glu Gln Leu Arg Ala Arg Leu Glu His His Pro
            20                  25                  30


Gln Gly Gln Arg Glu Pro Gly Ala Ser Gly Gly Gly Gly Ser Ser Ser
        35                  40                  45


Met Ile Glu Phe Ile Glu Phe Glu Ile Glu Asp Leu Gly Val Met Glu
        50                  55                  60


Ile Asp Val Tyr Asp Ile Glu Val Asp Gly Asn His Asn Phe Phe Gly
65                  70                  75                  80


Asn Asp Ile Leu Val His Asn Ser Val Tyr Leu Asn Gly Thr Met Asp
                85                  90                  95


Glu Lys Thr Thr Gly Trp Arg Gly Gly His Val Val Glu Gly Leu Ala
            100                 105                 110


Gly Glu Leu Glu Gln Leu Arg Ala Arg Leu Glu His His Pro Gln Gly
        115                 120                 125


Gln Arg Glu Pro
        130


<210>    253
<211>    430
<212>    PRT
<213>    Artificial sequence

<220>
<223>    MBP-CLN-H6 (3)

<400>    253

Met Lys Thr Glu Glu Gly Lys Leu Val Ile Trp Ile Asn Gly Asp Lys
1               5                   10                  15


Gly Tyr Asn Gly Leu Ala Glu Val Gly Lys Lys Phe Glu Lys Asp Thr
```

|     |     | 20  |     |     |     |     | 25  |     |     |     |     | 30  |     |     |
|-----|-----|-----|-----|-----|-----|-----|-----|-----|-----|-----|-----|-----|-----|-----|

Gly Ile Lys Val Thr Val Glu His Pro Asp Lys Leu Glu Glu Lys Phe
        35                    40                    45

Pro Gln Val Ala Ala Thr Gly Asp Gly Pro Asp Ile Ile Phe Trp Ala
        50                    55                    60

His Asp Arg Phe Gly Gly Tyr Ala Gln Ser Gly Leu Leu Ala Glu Ile
65                      70                    75                    80

Thr Pro Asp Lys Ala Phe Gln Asp Lys Leu Tyr Pro Phe Thr Trp Asp
                    85                    90                    95

Ala Val Arg Tyr Asn Gly Lys Leu Ile Ala Tyr Pro Ile Ala Val Glu
                100                    105                    110

Ala Leu Ser Leu Ile Tyr Asn Lys Asp Leu Leu Pro Asn Pro Pro Lys
        115                    120                    125

Thr Trp Glu Glu Ile Pro Ala Leu Asp Lys Glu Leu Lys Ala Lys Gly
        130                    135                    140

Lys Ser Ala Leu Met Phe Asn Leu Gln Glu Pro Tyr Phe Thr Trp Pro
145                    150                    155                    160

Leu Ile Ala Ala Asp Gly Gly Tyr Ala Phe Lys Tyr Glu Asn Gly Lys
                165                    170                    175

Tyr Asp Ile Lys Asp Val Gly Val Asp Asn Ala Gly Ala Lys Ala Gly
            180                    185                    190

Leu Thr Phe Leu Val Asp Leu Ile Lys Asn Lys His Met Asn Ala Asp
        195                    200                    205

Thr Asp Tyr Ser Ile Ala Glu Ala Ala Phe Asn Lys Gly Glu Thr Ala
        210                    215                    220

Met Thr Ile Asn Gly Pro Trp Ala Trp Ser Asn Ile Asp Thr Ser Lys
225                    230                    235                    240

Val Asn Tyr Gly Val Thr Val Leu Pro Thr Phe Lys Gly Gln Pro Ser
            245                    250                    255

Lys Pro Phe Val Gly Val Leu Ser Ala Gly Ile Asn Ala Ala Ser Pro
            260                    265                    270

```
Asn Lys Glu Leu Ala Lys Glu Phe Leu Glu Asn Tyr Leu Leu Thr Asp
        275             280             285

Glu Gly Leu Glu Ala Val Asn Lys Asp Lys Pro Leu Gly Ala Val Ala
        290             295             300

Leu Lys Ser Tyr Glu Glu Glu Leu Ala Lys Asp Pro Arg Ile Ala Ala
305             310             315             320

Thr Met Glu Asn Ala Gln Lys Gly Glu Ile Met Pro Asn Ile Pro Gln
        325             330             335

Met Ser Ala Phe Trp Tyr Ala Val Arg Thr Ala Val Ile Asn Ala Ala
        340             345             350

Ser Gly Arg Gln Thr Val Asp Glu Ala Leu Lys Asp Ala Gln Thr Asn
        355             360             365

Ser Ser Ser Asn Asn Asn Asn Asn Asn Asn Asn Asn Asn Leu Gly Ile
        370             375             380

Glu Gly Arg Ile Ser Glu Phe Tyr Ile Asp Thr Asp Ser Val Val Gly
385             390             395             400

Asp Thr Ile Ile Asp Val Ser Gly Lys Lys Met Thr Ile Ala Glu Phe
        405             410             415

Tyr Asp Ser Thr Pro Asp Gly Ser His His His His His
        420             425             430
```

```
<210>   254
<211>   305
<212>   PRT
<213>   Artificial sequence

<220>
<223>   SBP-CLC-Trx-H6 (4)

<400>   254
```

```
Met Asp Glu Lys Thr Thr Gly Trp Arg Gly Gly His Val Val Glu Gly
1               5               10              15

Leu Ala Gly Glu Leu Glu Gln Leu Arg Ala Arg Leu Glu His His Pro
            20              25              30

Gln Gly Gln Arg Glu Pro Gly Ala Ser Gly Gly Gly Gly Ser Ser Ser
        35              40              45

Glu Ala Arg Asp Trp Val Lys Arg Val Gly Gly Lys Thr Ser Leu Ser
```

```
                50                      55                      60

        Val Asn Thr Tyr Ser Gly Glu Val Glu Arg Lys Asn Ile Asn Tyr Ile
        65              70              75              80

        Met Lys His Thr Val Lys Lys Arg Met Phe Lys Ile Lys Ala Gly Gly
                        85              90              95

        Lys Glu Val Ile Val Thr Ala Asp His Ser Val Met Val Lys Arg Asp
                        100             105             110

        Gly Lys Ile Ile Asp Val Lys Pro Thr Glu Met Lys Gln Thr Asp Arg
                115             120             125

        Val Val Lys Trp Met Leu Thr Gly Ser His Met Ile Glu Phe Ile Glu
                130             135             140

        Phe Glu Ile Glu Asp Leu Gly Val Met Glu Ile Asp Val Tyr Asp Ile
        145             150             155             160

        Glu Val Asp Gly Asn His Asn Phe Phe Gly Asn Asp Ile Leu Val His
                        165             170             175

        Asn Ser Val Tyr Leu Asn Gly Thr Gly Ser Asp Lys Ile Ile His Leu
                        180             185             190

        Thr Asp Asp Ser Phe Asp Thr Asp Val Leu Lys Ala Asp Gly Ala Ile
                        195             200             205

        Leu Val Asp Phe Trp Ala His Trp Cys Gly Pro Cys Lys Met Ile Ala
                        210             215             220

        Pro Ile Leu Asp Glu Ile Ala Asp Glu Tyr Gln Gly Lys Leu Thr Val
        225             230             235             240

        Ala Lys Leu Asn Ile Asp His Asn Pro Gly Thr Ala Pro Lys Tyr Gly
                        245             250             255

        Ile Arg Gly Ile Pro Thr Leu Leu Leu Phe Lys Asn Gly Glu Val Ala
                        260             265             270

        Ala Thr Lys Val Gly Ala Leu Ser Lys Gly Gln Leu Lys Glu Phe Leu
                        275             280             285

        Asp Ala Asn Leu Ala Gly Ser Glu Phe Arg Ser His His His His His
                290             295             300
```

His
305

<210> 255
<211> 430
<212> PRT
<213> Artificial sequence

<220>
<223> MBP-CLN(S1C)-H6 (3a)

<400> 255

Met Lys Thr Glu Glu Gly Lys Leu Val Ile Trp Ile Asn Gly Asp Lys
1               5                   10                  15

Gly Tyr Asn Gly Leu Ala Glu Val Gly Lys Lys Phe Glu Lys Asp Thr
            20                  25                  30

Gly Ile Lys Val Thr Val Glu His Pro Asp Lys Leu Glu Glu Lys Phe
        35                  40                  45

Pro Gln Val Ala Ala Thr Gly Asp Gly Pro Asp Ile Ile Phe Trp Ala
        50                  55                  60

His Asp Arg Phe Gly Gly Tyr Ala Gln Ser Gly Leu Leu Ala Glu Ile
65                  70                  75                  80

Thr Pro Asp Lys Ala Phe Gln Asp Lys Leu Tyr Pro Phe Thr Trp Asp
                85                  90                  95

Ala Val Arg Tyr Asn Gly Lys Leu Ile Ala Tyr Pro Ile Ala Val Glu
            100                 105                 110

Ala Leu Ser Leu Ile Tyr Asn Lys Asp Leu Leu Pro Asn Pro Pro Lys
            115                 120                 125

Thr Trp Glu Glu Ile Pro Ala Leu Asp Lys Glu Leu Lys Ala Lys Gly
            130                 135                 140

Lys Ser Ala Leu Met Phe Asn Leu Gln Glu Pro Tyr Phe Thr Trp Pro
145                 150                 155                 160

Leu Ile Ala Ala Asp Gly Gly Tyr Ala Phe Lys Tyr Glu Asn Gly Lys
                165                 170                 175

Tyr Asp Ile Lys Asp Val Gly Val Asp Asn Ala Gly Ala Lys Ala Gly
            180                 185                 190

Leu Thr Phe Leu Val Asp Leu Ile Lys Asn Lys His Met Asn Ala Asp

```
              195                     200                     205

      Thr Asp Tyr Ser Ile Ala Glu Ala Ala Phe Asn Lys Gly Glu Thr Ala
          210             215             220

      Met Thr Ile Asn Gly Pro Trp Ala Trp Ser Asn Ile Asp Thr Ser Lys
      225             230             235             240

      Val Asn Tyr Gly Val Thr Val Leu Pro Thr Phe Lys Gly Gln Pro Ser
                  245             250             255

      Lys Pro Phe Val Gly Val Leu Ser Ala Gly Ile Asn Ala Ala Ser Pro
                  260             265             270

      Asn Lys Glu Leu Ala Lys Glu Phe Leu Glu Asn Tyr Leu Leu Thr Asp
          275             280             285

      Glu Gly Leu Glu Ala Val Asn Lys Asp Lys Pro Leu Gly Ala Val Ala
          290             295             300

      Leu Lys Ser Tyr Glu Glu Glu Leu Ala Lys Asp Pro Arg Ile Ala Ala
      305             310             315             320

      Thr Met Glu Asn Ala Gln Lys Gly Glu Ile Met Pro Asn Ile Pro Gln
                  325             330             335

      Met Ser Ala Phe Trp Tyr Ala Val Arg Thr Ala Val Ile Asn Ala Ala
                  340             345             350

      Ser Gly Arg Gln Thr Val Asp Glu Ala Leu Lys Asp Ala Gln Thr Asn
          355             360             365

      Ser Ser Ser Asn Asn Asn Asn Asn Asn Asn Asn Asn Asn Leu Gly Ile
          370             375             380

      Glu Gly Arg Ile Ser Glu Phe Tyr Ile Asp Thr Asp Cys Val Val Gly
      385             390             395             400

      Asp Thr Ile Ile Asp Val Ser Gly Lys Lys Met Thr Ile Ala Glu Phe
                  405             410             415

      Tyr Asp Ser Thr Pro Asp Gly Ser His His His His His His
                  420             425             430
```

```
<210>   256
<211>   305
<212>   PRT
<213>   Artificial sequence
```

<220>
<223>  SBP-CLC(S+1C)-Trx-H6 (4a)

<400>  256

Met Asp Glu Lys Thr Thr Gly Trp Arg Gly Gly His Val Val Glu Gly
1               5                   10                  15

Leu Ala Gly Glu Leu Glu Gln Leu Arg Ala Arg Leu Glu His His Pro
            20                  25                  30

Gln Gly Gln Arg Glu Pro Gly Ala Ser Gly Gly Gly Gly Ser Ser Ser
        35                  40                  45

Glu Ala Arg Asp Trp Val Lys Arg Val Gly Gly Lys Thr Ser Leu Ser
    50                  55                  60

Val Asn Thr Tyr Ser Gly Glu Val Glu Arg Lys Asn Ile Asn Tyr Ile
65                  70                  75                  80

Met Lys His Thr Val Lys Lys Arg Met Phe Lys Ile Lys Ala Gly Gly
            85                  90                  95

Lys Glu Val Ile Val Thr Ala Asp His Ser Val Met Val Lys Arg Asp
            100                 105                 110

Gly Lys Ile Ile Asp Val Lys Pro Thr Glu Met Lys Gln Thr Asp Arg
            115                 120                 125

Val Val Lys Trp Met Leu Thr Gly Ser His Met Ile Glu Phe Ile Glu
    130                 135                 140

Phe Glu Ile Glu Asp Leu Gly Val Met Glu Ile Asp Val Tyr Asp Ile
145                 150                 155                 160

Glu Val Asp Gly Asn His Asn Phe Phe Gly Asn Asp Ile Leu Val His
                165                 170                 175

Asn Cys Val Tyr Leu Asn Gly Thr Gly Ser Asp Lys Ile Ile His Leu
            180                 185                 190

Thr Asp Asp Ser Phe Asp Thr Asp Val Leu Lys Ala Asp Gly Ala Ile
            195                 200                 205

Leu Val Asp Phe Trp Ala His Trp Cys Gly Pro Cys Lys Met Ile Ala
    210                 215                 220

Pro Ile Leu Asp Glu Ile Ala Asp Glu Tyr Gln Gly Lys Leu Thr Val

356

225                     230                     235                     240


Ala Lys Leu Asn Ile Asp His Asn Pro Gly Thr Ala Pro Lys Tyr Gly
                245                 250                 255


Ile Arg Gly Ile Pro Thr Leu Leu Leu Phe Lys Asn Gly Glu Val Ala
                260                 265                 270


Ala Thr Lys Val Gly Ala Leu Ser Lys Gly Gln Leu Lys Glu Phe Leu
                275                 280                 285


Asp Ala Asn Leu Ala Gly Ser Glu Phe Arg Ser His His His His His
                290                 295                 300


His
305


<210> 257
<211> 294
<212> PRT
<213> Artificial sequence

<220>
<223> eGFP-CLN(S1A)-H6 (5)

<400> 257

Met Ala Ser Trp Ser His Pro Gln Phe Glu Lys Ala Ser Gly Thr Val
1               5                   10                  15


Ser Lys Gly Glu Glu Leu Phe Thr Gly Val Val Pro Ile Leu Val Glu
                20                  25                  30


Leu Asp Gly Asp Val Asn Gly His Lys Phe Ser Val Ser Gly Glu Gly
                35                  40                  45


Glu Gly Asp Ala Thr Tyr Gly Lys Leu Thr Leu Lys Phe Ile Cys Thr
                50                  55                  60


Thr Gly Lys Leu Pro Val Pro Trp Pro Thr Leu Val Thr Thr Leu Thr
65                  70                  75                  80


Tyr Gly Val Gln Cys Phe Ser Arg Tyr Pro Asp His Met Lys Gln His
                85                  90                  95


Asp Phe Phe Lys Ser Ala Met Pro Glu Gly Tyr Val Gln Glu Arg Thr
                100                 105                 110


Ile Phe Phe Lys Asp Asp Gly Asn Tyr Lys Thr Arg Ala Glu Val Lys
                115                 120                 125

Phe Glu Gly Asp Thr Leu Val Asn Arg Ile Glu Leu Lys Gly Ile Asp
    130             135             140

Phe Lys Glu Asp Gly Asn Ile Leu Gly His Lys Leu Glu Tyr Asn Tyr
    145             150             155             160

Asn Ser His Asn Val Tyr Ile Met Ala Asp Lys Gln Lys Asn Gly Ile
            165             170             175

Lys Val Asn Phe Lys Ile Arg His Asn Ile Glu Asp Gly Ser Val Gln
            180             185             190

Leu Ala Asp His Tyr Gln Gln Asn Thr Pro Ile Gly Asp Gly Pro Val
        195             200             205

Leu Leu Pro Asp Asn His Tyr Leu Ser Thr Gln Ser Ala Leu Ser Lys
    210             215             220

Asp Pro Asn Glu Lys Arg Asp His Met Val Leu Leu Glu Phe Val Thr
225             230             235             240

Ala Ala Gly Ile Thr Leu Gly Met Asp Glu Leu Tyr Lys Gly Ser Tyr
            245             250             255

Ile Asp Thr Asp Ala Val Val Gly Asp Thr Ile Ile Asp Val Ser Gly
        260             265             270

Lys Lys Met Thr Ile Ala Glu Phe Tyr Asp Ser Thr Pro Asp Gly Ser
    275             280             285

His His His His His His
    290

<210> 258
<211> 305
<212> PRT
<213> Artificial sequence

<220>
<223> SBP-CLC(N159'A)-Trx-H6 (6)

<400> 258

Met Asp Glu Lys Thr Thr Gly Trp Arg Gly Gly His Val Val Glu Gly
1               5               10              15

Leu Ala Gly Glu Leu Glu Gln Leu Arg Ala Arg Leu Glu His His Pro
            20              25              30

Gln Gly Gln Arg Glu Pro Gly Ala Ser Gly Gly Gly Gly Ser Ser Ser
        35                  40                  45

Glu Ala Arg Asp Trp Val Lys Arg Val Gly Gly Lys Thr Ser Leu Ser
        50                  55                  60

Val Asn Thr Tyr Ser Gly Glu Val Glu Arg Lys Asn Ile Asn Tyr Ile
65                  70                  75                  80

Met Lys His Thr Val Lys Lys Arg Met Phe Lys Ile Lys Ala Gly Gly
                85                  90                  95

Lys Glu Val Ile Val Thr Ala Asp His Ser Val Met Val Lys Arg Asp
                100                 105                 110

Gly Lys Ile Ile Asp Val Lys Pro Thr Glu Met Lys Gln Thr Asp Arg
        115                 120                 125

Val Val Lys Trp Met Leu Thr Gly Ser His Met Ile Glu Phe Ile Glu
        130                 135                 140

Phe Glu Ile Glu Asp Leu Gly Val Met Glu Ile Asp Val Tyr Asp Ile
145                 150                 155                 160

Glu Val Asp Gly Asn His Asn Phe Phe Gly Asn Asp Ile Leu Val His
                165                 170                 175

Ala Ser Val Tyr Leu Asn Gly Thr Gly Ser Asp Lys Ile Ile His Leu
                180                 185                 190

Thr Asp Asp Ser Phe Asp Thr Asp Val Leu Lys Ala Asp Gly Ala Ile
        195                 200                 205

Leu Val Asp Phe Trp Ala His Trp Cys Gly Pro Cys Lys Met Ile Ala
        210                 215                 220

Pro Ile Leu Asp Glu Ile Ala Asp Glu Tyr Gln Gly Lys Leu Thr Val
225                 230                 235                 240

Ala Lys Leu Asn Ile Asp His Asn Pro Gly Thr Ala Pro Lys Tyr Gly
                245                 250                 255

Ile Arg Gly Ile Pro Thr Leu Leu Leu Phe Lys Asn Gly Glu Val Ala
        260                 265                 270

Ala Thr Lys Val Gly Ala Leu Ser Lys Gly Gln Leu Lys Glu Phe Leu
        275                 280                 285

Asp Ala Asn Leu Ala Gly Ser Glu Phe Arg Ser His His His His His
   290             295             300

His
305


```
<210>  259
<211>  87
<212>  PRT
<213>  Artificial sequence

<220>
<223>  CysTag-CLN-SBP (7)

<400>  259
```

Met Gly Cys Asp Thr Asp Ser Val Val Gly Asp Thr Ile Ile Asp Val
1            5             10            15

Ser Gly Lys Lys Met Thr Ile Ala Glu Phe Tyr Asp Ser Thr Pro Asp
       20           25            30

Ser Gly Gly Ser Pro Arg Lys Val Ile Lys Met Glu Ser Glu Glu Arg
       35           40            45

Ser Met Asp Glu Lys Thr Thr Gly Trp Arg Gly Gly His Val Val Glu
     50           55           60

Gly Leu Ala Gly Glu Leu Glu Gln Leu Arg Ala Arg Leu Glu His His
65             70           75           80

Pro Gln Gly Gln Arg Glu Pro
              85


```
<210>  260
<211>  310
<212>  PRT
<213>  Artificial sequence

<220>
<223>  SBP-CLC-VHHGFP-H6 (8)

<400>  260
```

Met Asp Glu Lys Thr Thr Gly Trp Arg Gly Gly His Val Val Glu Gly
1            5             10            15

Leu Ala Gly Glu Leu Glu Gln Leu Arg Ala Arg Leu Glu His His Pro
       20           25            30

Gln Gly Gln Arg Glu Pro Gly Ala Ser Gly Gly Gly Gly Ser Ser Ser

360

                35                        40                        45

Glu Ala Arg Asp Trp Val Lys Arg Val Gly Gly Lys Thr Ser Leu Ser
    50              55              60

Val Asn Thr Tyr Ser Gly Glu Val Glu Arg Lys Asn Ile Asn Tyr Ile
65              70              75              80

Met Lys His Thr Val Lys Lys Arg Met Phe Lys Ile Lys Ala Gly Gly
            85              90              95

Lys Glu Val Ile Val Thr Ala Asp His Ser Val Met Val Lys Arg Asp
            100             105             110

Gly Lys Ile Ile Asp Val Lys Pro Thr Glu Met Lys Gln Thr Asp Arg
        115             120             125

Val Val Lys Trp Met Leu Thr Gly Ser His Met Ile Glu Phe Ile Glu
    130             135             140

Phe Glu Ile Glu Asp Leu Gly Val Met Glu Ile Asp Val Tyr Asp Ile
145             150             155             160

Glu Val Asp Gly Asn His Asn Phe Phe Gly Asn Asp Ile Leu Val His
            165             170             175

Asn Ser Val Tyr Leu Asn Gly Met Ala Gln Val Gln Leu Val Glu Ser
        180             185             190

Gly Gly Ala Leu Val Gln Pro Gly Gly Ser Leu Arg Leu Ser Cys Ala
    195             200             205

Ala Ser Gly Phe Pro Val Asn Arg Tyr Ser Met Arg Trp Tyr Arg Gln
    210             215             220

Ala Pro Gly Lys Glu Arg Glu Trp Val Ala Gly Met Ser Ser Ala Gly
225             230             235             240

Asp Arg Ser Ser Tyr Glu Asp Ser Val Lys Gly Arg Phe Thr Ile Ser
            245             250             255

Arg Asp Asp Ala Arg Asn Thr Val Tyr Leu Gln Met Asn Ser Leu Lys
        260             265             270

Pro Glu Asp Thr Ala Val Tyr Tyr Cys Asn Val Asn Val Gly Phe Glu
        275             280             285

```
Tyr Trp Gly Gln Gly Thr Gln Val Thr Val Ser Ser Pro Asp Arg Ser
    290                 295                 300
```

```
His His His His His His
305                 310
```

```
<210>  261
<211>  19
<212>  PRT
<213>  Artificial sequence

<220>
<223>  First amino acid sequence from Fluorescein-CLN (9)

<400>  261
```

```
Tyr Ile Asp Thr Asp Ser Val Val Gly Asp Thr Ile Ile Asp Val Ser
1                 5                 10                  15
```

```
Gly Lys Lys
```

```
<210>  262
<211>  11
<212>  PRT
<213>  Artificial sequence

<220>
<223>  Second amino acid sequence from Fluorescein-CLN (9)

<400>  262
```

```
Thr Ile Ala Glu Phe Tyr Asp Ser Thr Pro Asp
1                 5                 10
```

```
<210>  263
<211>  197
<212>  PRT
<213>  Artificial sequence

<220>
<223>  SBP-CLC-CysTag-H6 (11)

<400>  263
```

```
Met Asp Glu Lys Thr Thr Gly Trp Arg Gly Gly His Val Val Glu Gly
1                 5                 10                  15
```

```
Leu Ala Gly Glu Leu Glu Gln Leu Arg Ala Arg Leu Glu His His Pro
                20                  25                  30
```

```
Gln Gly Gln Arg Glu Pro Gly Ala Ser Gly Gly Gly Gly Ser Ser Ser
            35                  40                  45
```

```
Glu Ala Arg Asp Trp Val Lys Arg Val Gly Gly Lys Thr Ser Leu Ser
```

```
            50                     55                     60

        Val Asn Thr Tyr Ser Gly Glu Val Glu Arg Lys Asn Ile Asn Tyr Ile
        65                  70                  75                  80

        Met Lys His Thr Val Lys Lys Arg Met Phe Lys Ile Lys Ala Gly Gly
                        85                  90                  95

        Lys Glu Val Ile Val Thr Ala Asp His Ser Val Met Val Lys Arg Asp
                    100                 105                 110

        Gly Lys Ile Ile Asp Val Lys Pro Thr Glu Met Lys Gln Thr Asp Arg
                    115                 120                 125

        Val Val Lys Trp Met Leu Thr Gly Ser His Met Ile Glu Phe Ile Glu
                130                 135                 140

        Phe Glu Ile Glu Asp Leu Gly Val Met Glu Ile Asp Val Tyr Asp Ile
        145                 150                 155                 160

        Glu Val Asp Gly Asn His Asn Phe Phe Gly Asn Asp Ile Leu Val His
                        165                 170                 175

        Asn Ser Val Tyr Ala Ser Pro Ala Ala Pro Ala Pro Ala Ser Cys His
                        180                 185                 190

        His His His His His
                    195


        <210>   264
        <211>   310
        <212>   PRT
        <213>   Artificial sequence

        <220>
        <223>   VHHEGFR-CLN-SBP (10)

        <400>   264

        Met Asp Glu Lys Thr Thr Gly Trp Arg Gly Gly His Val Val Glu Gly
        1                   5                   10                  15

        Leu Ala Gly Glu Leu Glu Gln Leu Arg Ala Arg Leu Glu His His Pro
                        20                  25                  30

        Gln Gly Gln Arg Glu Pro Gly Ala Ser Gly Gly Gly Ser Ser Ser
                    35                  40                  45

        Glu Ala Arg Asp Trp Val Lys Arg Val Gly Gly Lys Thr Ser Leu Ser
                50                  55                  60
```

Val Asn Thr Tyr Ser Gly Glu Val Glu Arg Lys Asn Ile Asn Tyr Ile
65                  70                  75                  80

Met Lys His Thr Val Lys Lys Arg Met Phe Lys Ile Lys Ala Gly Gly
                85                  90                  95

Lys Glu Val Ile Val Thr Ala Asp His Ser Val Met Val Lys Arg Asp
            100                 105                 110

Gly Lys Ile Ile Asp Val Lys Pro Thr Glu Met Lys Gln Thr Asp Arg
        115                 120                 125

Val Val Lys Trp Met Leu Thr Gly Ser His Met Ile Glu Phe Ile Glu
    130                 135                 140

Phe Glu Ile Glu Asp Leu Gly Val Met Glu Ile Asp Val Tyr Asp Ile
145                 150                 155                 160

Glu Val Asp Gly Asn His Asn Phe Phe Gly Asn Asp Ile Leu Val His
                165                 170                 175

Asn Ser Val Tyr Leu Asn Gly Met Ala Gln Val Gln Leu Val Glu Ser
            180                 185                 190

Gly Gly Ala Leu Val Gln Pro Gly Gly Ser Leu Arg Leu Ser Cys Ala
        195                 200                 205

Ala Ser Gly Phe Pro Val Asn Arg Tyr Ser Met Arg Trp Tyr Arg Gln
    210                 215                 220

Ala Pro Gly Lys Glu Arg Glu Trp Val Ala Gly Met Ser Ser Ala Gly
225                 230                 235                 240

Asp Arg Ser Ser Tyr Glu Asp Ser Val Lys Gly Arg Phe Thr Ile Ser
                245                 250                 255

Arg Asp Asp Ala Arg Asn Thr Val Tyr Leu Gln Met Asn Ser Leu Lys
            260                 265                 270

Pro Glu Asp Thr Ala Val Tyr Tyr Cys Asn Val Asn Val Gly Phe Glu
            275                 280                 285

Tyr Trp Gly Gln Gly Thr Gln Val Thr Val Ser Ser Pro Asp Arg Ser
    290                 295                 300

His His His His His His

305                    310

```
<210>  265
<211>  603
<212>  PRT
<213>  Artificial sequence

<220>
<223>  HA-CLC-Trx-TMD-mCherry (13)

<400>  265
```

Met Glu Thr Asp Thr Leu Leu Leu Trp Val Leu Leu Leu Trp Val Pro
1               5                   10                  15


Gly Ser Thr Gly Asp Tyr Pro Tyr Asp Val Pro Asp Tyr Ala Gly Ala
            20                  25                  30


Gln Ser Gly Gly Gly Gly Ser Ser Ser Glu Ala Arg Asp Trp Val Lys
        35                  40                  45


Arg Val Gly Gly Lys Thr Ser Leu Ser Val Asn Thr Tyr Ser Gly Glu
        50                  55                  60


Val Glu Arg Lys Asn Ile Asn Tyr Ile Met Lys His Thr Val Lys Lys
65                  70                  75                  80


Arg Met Phe Lys Ile Lys Ala Gly Gly Lys Glu Val Ile Val Thr Ala
                85                  90                  95


Asp His Ser Val Met Val Lys Arg Asp Gly Lys Ile Ile Asp Val Lys
            100                 105                 110


Pro Thr Glu Met Lys Gln Thr Asp Arg Val Val Lys Trp Met Leu Thr
            115                 120                 125


Gly Ser His Met Ile Glu Phe Ile Glu Phe Glu Ile Glu Asp Leu Gly
        130                 135                 140


Val Met Glu Ile Asp Val Tyr Asp Ile Glu Val Asp Gly Asn His Asn
145                 150                 155                 160


Phe Phe Gly Asn Asp Ile Leu Val His Asn Ser Val Tyr Leu Asn Ser
            165                 170                 175


Gly Gly Ser Gly Thr Gly Ser Asp Lys Ile Ile His Leu Thr Asp Asp
            180                 185                 190


Ser Phe Asp Thr Asp Val Leu Lys Ala Asp Gly Ala Ile Leu Val Asp
            195                 200                 205

Phe Trp Ala His Trp Cys Gly Pro Cys Lys Met Ile Ala Pro Ile Leu
210                215                220

Asp Glu Ile Ala Asp Glu Tyr Gln Gly Lys Leu Thr Val Ala Lys Leu
225                230                235                240

Asn Ile Asp His Asn Pro Gly Thr Ala Pro Lys Tyr Gly Ile Arg Gly
245                250                255

Ile Pro Thr Leu Leu Leu Phe Lys Asn Gly Glu Val Ala Ala Thr Lys
260                265                270

Val Gly Ala Leu Ser Lys Gly Gln Leu Lys Glu Phe Leu Asp Ala Asn
275                280                285

Leu Ala Gly Ser Glu Phe Arg Ser His His His His His Tyr Val
290                295                300

Asp Glu Gln Lys Leu Ile Ser Glu Glu Asp Leu Asn Ala Val Gly Gln
305                310                315                320

Asp Thr Gln Glu Val Ile Val Val Pro His Ser Leu Pro Phe Lys Val
325                330                335

Val Val Ile Ser Ala Ile Leu Ala Leu Val Val Leu Thr Ile Ile Ser
340                345                350

Leu Ile Ile Leu Ile Met Leu Trp Gln Lys Lys Pro Arg Ala Ser Met
355                360                365

Val Ser Lys Gly Glu Glu Asp Asn Met Ala Ile Ile Lys Glu Phe Met
370                375                380

Arg Phe Lys Val His Met Glu Gly Ser Val Asn Gly His Glu Phe Glu
385                390                395                400

Ile Glu Gly Glu Gly Glu Gly Arg Pro Tyr Glu Gly Thr Gln Thr Ala
405                410                415

Lys Leu Lys Val Thr Lys Gly Gly Pro Leu Pro Phe Ala Trp Asp Ile
420                425                430

Leu Ser Pro Gln Phe Met Tyr Gly Ser Lys Ala Tyr Val Lys His Pro
435                440                445

Ala Asp Ile Pro Asp Tyr Leu Lys Leu Ser Phe Pro Glu Gly Phe Lys

```
                    450                     455                      460


    Trp Glu Arg Val Met Asn Phe Glu Asp Gly Gly Val Val Thr Val Thr
    465                     470                 475                     480


    Gln Asp Ser Ser Leu Gln Asp Gly Glu Phe Ile Tyr Lys Val Lys Leu
                        485                     490                 495


    Arg Gly Thr Asn Phe Pro Ser Asp Gly Pro Val Met Gln Lys Lys Thr
                    500                     505                 510


    Met Gly Trp Glu Ala Ser Ser Glu Arg Met Tyr Pro Glu Asp Gly Ala
                    515                     520                 525


    Leu Lys Gly Glu Ile Lys Gln Arg Leu Lys Leu Lys Asp Gly Gly His
                    530                     535                 540


    Tyr Asp Ala Glu Val Lys Thr Thr Tyr Lys Ala Lys Lys Pro Val Gln
    545                     550                     555                 560


    Leu Pro Gly Ala Tyr Asn Val Asn Ile Lys Leu Asp Ile Thr Ser His
                        565                     570                 575


    Asn Glu Asp Tyr Thr Ile Val Glu Gln Tyr Glu Arg Ala Glu Gly Arg
                        580                     585                 590


    His Ser Thr Gly Gly Met Asp Glu Leu Tyr Lys
                    595                     600


    <210>   266
    <211>   699
    <212>   PRT
    <213>   Artificial sequence

    <220>
    <223>   MBP-Fc-CLN-SBP (12)

    <400>   266

    Met Lys Thr Glu Glu Gly Lys Leu Val Ile Trp Ile Asn Gly Asp Lys
    1                   5                   10                  15


    Gly Tyr Asn Gly Leu Ala Glu Val Gly Lys Lys Phe Glu Lys Asp Thr
                        20                      25                  30


    Gly Ile Lys Val Thr Val Glu His Pro Asp Lys Leu Glu Glu Lys Phe
                        35                      40                  45


    Pro Gln Val Ala Ala Thr Gly Asp Gly Pro Asp Ile Ile Phe Trp Ala
                    50                      55                  60
```

His Asp Arg Phe Gly Gly Tyr Ala Gln Ser Gly Leu Leu Ala Glu Ile
65                    70              75                    80

Thr Pro Asp Lys Ala Phe Gln Asp Lys Leu Tyr Pro Phe Thr Trp Asp
              85                    90                    95

Ala Val Arg Tyr Asn Gly Lys Leu Ile Ala Tyr Pro Ile Ala Val Glu
              100                   105                   110

Ala Leu Ser Leu Ile Tyr Asn Lys Asp Leu Leu Pro Asn Pro Pro Lys
              115                   120                   125

Thr Trp Glu Glu Ile Pro Ala Leu Asp Lys Glu Leu Lys Ala Lys Gly
              130                   135                   140

Lys Ser Ala Leu Met Phe Asn Leu Gln Glu Pro Tyr Phe Thr Trp Pro
145                   150                   155                   160

Leu Ile Ala Ala Asp Gly Gly Tyr Ala Phe Lys Tyr Glu Asn Gly Lys
              165                   170                   175

Tyr Asp Ile Lys Asp Val Gly Val Asp Asn Ala Gly Ala Lys Ala Gly
              180                   185                   190

Leu Thr Phe Leu Val Asp Leu Ile Lys Asn Lys His Met Asn Ala Asp
              195                   200                   205

Thr Asp Tyr Ser Ile Ala Glu Ala Ala Phe Asn Lys Gly Glu Thr Ala
              210                   215                   220

Met Thr Ile Asn Gly Pro Trp Ala Trp Ser Asn Ile Asp Thr Ser Lys
225                   230                   235                   240

Val Asn Tyr Gly Val Thr Val Leu Pro Thr Phe Lys Gly Gln Pro Ser
              245                   250                   255

Lys Pro Phe Val Gly Val Leu Ser Ala Gly Ile Asn Ala Ala Ser Pro
              260                   265                   270

Asn Lys Glu Leu Ala Lys Glu Phe Leu Glu Asn Tyr Leu Leu Thr Asp
              275                   280                   285

Glu Gly Leu Glu Ala Val Asn Lys Asp Lys Pro Leu Gly Ala Val Ala
              290                   295                   300

Leu Lys Ser Tyr Glu Glu Glu Leu Ala Lys Asp Pro Arg Ile Ala Ala

305              310               315               320

Thr Met Glu Asn Ala Gln Lys Gly Glu Ile Met Pro Asn Ile Pro Gln
              325                330                335

Met Ser Ala Phe Trp Tyr Ala Val Arg Thr Ala Val Ile Asn Ala Ala
              340                345                350

Ser Gly Arg Gln Thr Val Asp Glu Ala Leu Lys Asp Ala Gln Thr Asn
              355                360                365

Ser Ser Ser Asn Asn Asn Asn Asn Asn Asn Asn Asn Leu Gly Ile
              370                375                380

Glu Gly Arg Ile Ser Glu Phe Asp Lys Thr His Thr Cys Pro Pro Cys
385               390                395                400

Pro Ala Pro Glu Ala Glu Gly Ala Pro Ser Val Phe Leu Phe Pro Pro
              405                410                415

Lys Pro Lys Asp Thr Leu Met Ile Ser Arg Thr Pro Glu Val Thr Cys
              420                425                430

Val Val Val Asp Val Ser His Glu Asp Pro Glu Val Lys Phe Asn Trp
              435                440                445

Tyr Val Asp Gly Val Glu Val His Asn Ala Lys Thr Lys Pro Arg Glu
              450                455                460

Glu Gln Tyr Asn Ser Thr Tyr Arg Val Val Ser Val Leu Thr Val Leu
465               470                475                480

His Gln Asp Trp Leu Asn Gly Lys Glu Tyr Lys Cys Lys Val Ser Asn
              485                490                495

Lys Ala Leu Pro Ala Pro Ile Glu Lys Thr Ile Ser Lys Ala Lys Gly
              500                505                510

Gln Pro Arg Glu Pro Gln Val Tyr Thr Leu Pro Pro Ser Arg Glu Glu
              515                520                525

Met Thr Lys Asn Gln Val Ser Leu Thr Cys Leu Val Lys Gly Phe Tyr
              530                535                540

Pro Ser Asp Ile Ala Val Glu Trp Glu Ser Asn Gly Gln Pro Glu Asn
545               550                555                560

```
Asn Tyr Lys Thr Thr Pro Pro Val Leu Asp Ser Asp Gly Ser Phe Phe
            565             570             575

Leu Tyr Ser Lys Leu Thr Val Asp Lys Ser Arg Trp Gln Gln Gly Asn
            580             585             590

Val Phe Ser Cys Ser Val Met His Glu Ala Leu His Asn His Tyr Thr
            595             600             605

Gln Lys Ser Leu Ser Leu Ser Pro Gly Lys Gly Ser Glu Phe Tyr Ile
    610             615             620

Asp Thr Asp Ser Val Val Gly Asp Thr Ile Ile Asp Val Ser Gly Lys
625             630             635             640

Lys Met Thr Ile Ala Glu Phe Tyr Asp Ser Thr Pro Asp Gly Ser Gly
            645             650             655

Ser Gly Ser Ser Arg Met Asp Glu Lys Thr Thr Gly Trp Arg Gly Gly
            660             665             670

His Val Val Glu Gly Leu Ala Gly Glu Leu Glu Gln Leu Arg Ala Arg
    675             680             685

Leu Glu His His Pro Gln Gly Gln Arg Glu Pro
    690             695
```

```
<210>   267
<211>   727
<212>   PRT
<213>   Artificial sequence

<220>
<223>   HA-VHHGFP-CLC-Trx-TMD-mCherry (15)

<400>   267
```

```
Met Glu Thr Asp Thr Leu Leu Leu Trp Val Leu Leu Leu Trp Val Pro
1               5               10              15

Gly Ser Thr Gly Asp Tyr Pro Tyr Asp Val Pro Asp Tyr Ala Gly Ala
            20              25              30

His Ser Arg Met Ala Gln Val Gln Leu Val Glu Ser Gly Gly Ala Leu
            35              40              45

Val Gln Pro Gly Gly Ser Leu Arg Leu Ser Cys Ala Ala Ser Gly Phe
    50              55              60

Pro Val Asn Arg Tyr Ser Met Arg Trp Tyr Arg Gln Ala Pro Gly Lys
```

65           70           75           80

```
Glu Arg Glu Trp Val Ala Gly Met Ser Ser Ala Gly Asp Arg Ser Ser
                85                    90                    95

Tyr Glu Asp Ser Val Lys Gly Arg Phe Thr Ile Ser Arg Asp Asp Ala
                100                   105                   110

Arg Asn Thr Val Tyr Leu Gln Met Asn Ser Leu Lys Pro Glu Asp Thr
        115                   120                   125

Ala Val Tyr Tyr Cys Asn Val Asn Val Gly Phe Glu Tyr Trp Gly Gln
        130                   135                   140

Gly Thr Gln Val Thr Val Ser Ser Pro Asp Trp Ala Gln Ser Gly Gly
145                   150                   155                   160

Gly Gly Ser Ser Ser Glu Ala Arg Asp Trp Val Lys Arg Val Gly Gly
                165                   170                   175

Lys Thr Ser Leu Ser Val Asn Thr Tyr Ser Gly Glu Val Glu Arg Lys
                180                   185                   190

Asn Ile Asn Tyr Ile Met Lys His Thr Val Lys Lys Arg Met Phe Lys
                195                   200                   205

Ile Lys Ala Gly Gly Lys Glu Val Ile Val Thr Ala Asp His Ser Val
        210                   215                   220

Met Val Lys Arg Asp Gly Lys Ile Ile Asp Val Lys Pro Thr Glu Met
225                   230                   235                   240

Lys Gln Thr Asp Arg Val Val Lys Trp Met Leu Thr Gly Ser His Met
                245                   250                   255

Ile Glu Phe Ile Glu Phe Glu Ile Glu Asp Leu Gly Val Met Glu Ile
                260                   265                   270

Asp Val Tyr Asp Ile Glu Val Asp Gly Asn His Asn Phe Phe Gly Asn
        275                   280                   285

Asp Ile Leu Val His Asn Ser Val Tyr Leu Asn Ser Gly Gly Ser Gly
        290                   295                   300

Thr Gly Ser Asp Lys Ile Ile His Leu Thr Asp Asp Ser Phe Asp Thr
305                   310                   315                   320
```

```
Asp Val Leu Lys Ala Asp Gly Ala Ile Leu Val Asp Phe Trp Ala His
            325             330             335

Trp Cys Gly Pro Cys Lys Met Ile Ala Pro Ile Leu Asp Glu Ile Ala
            340             345             350

Asp Glu Tyr Gln Gly Lys Leu Thr Val Ala Lys Leu Asn Ile Asp His
            355             360             365

Asn Pro Gly Thr Ala Pro Lys Tyr Gly Ile Arg Gly Ile Pro Thr Leu
    370             375             380

Leu Leu Phe Lys Asn Gly Glu Val Ala Ala Thr Lys Val Gly Ala Leu
385             390             395             400

Ser Lys Gly Gln Leu Lys Glu Phe Leu Asp Ala Asn Leu Ala Gly Ser
            405             410             415

Glu Phe Arg Ser His His His His His His Tyr Val Asp Glu Gln Lys
            420             425             430

Leu Ile Ser Glu Glu Asp Leu Asn Ala Val Gly Gln Asp Thr Gln Glu
            435             440             445

Val Ile Val Val Pro His Ser Leu Pro Phe Lys Val Val Val Ile Ser
    450             455             460

Ala Ile Leu Ala Leu Val Val Leu Thr Ile Ile Ser Leu Ile Ile Leu
465             470             475             480

Ile Met Leu Trp Gln Lys Lys Pro Arg Ala Ser Met Val Ser Lys Gly
            485             490             495

Glu Glu Asp Asn Met Ala Ile Ile Lys Glu Phe Met Arg Phe Lys Val
            500             505             510

His Met Glu Gly Ser Val Asn Gly His Glu Phe Glu Ile Glu Gly Glu
            515             520             525

Gly Glu Gly Arg Pro Tyr Glu Gly Thr Gln Thr Ala Lys Leu Lys Val
    530             535             540

Thr Lys Gly Gly Pro Leu Pro Phe Ala Trp Asp Ile Leu Ser Pro Gln
545             550             555             560

Phe Met Tyr Gly Ser Lys Ala Tyr Val Lys His Pro Ala Asp Ile Pro
            565             570             575
```

Asp Tyr Leu Lys Leu Ser Phe Pro Glu Gly Phe Lys Trp Glu Arg Val
                580                 585                 590

Met Asn Phe Glu Asp Gly Gly Val Val Thr Val Thr Gln Asp Ser Ser
                595                 600                 605

Leu Gln Asp Gly Glu Phe Ile Tyr Lys Val Lys Leu Arg Gly Thr Asn
                610                 615                 620

Phe Pro Ser Asp Gly Pro Val Met Gln Lys Lys Thr Met Gly Trp Glu
625                 630                 635                 640

Ala Ser Ser Glu Arg Met Tyr Pro Glu Asp Gly Ala Leu Lys Gly Glu
                645                 650                 655

Ile Lys Gln Arg Leu Lys Leu Lys Asp Gly Gly His Tyr Asp Ala Glu
                660                 665                 670

Val Lys Thr Thr Tyr Lys Ala Lys Lys Pro Val Gln Leu Pro Gly Ala
                675                 680                 685

Tyr Asn Val Asn Ile Lys Leu Asp Ile Thr Ser His Asn Glu Asp Tyr
                690                 695                 700

Thr Ile Val Glu Gln Tyr Glu Arg Ala Glu Gly Arg His Ser Thr Gly
705                 710                 715                 720

Gly Met Asp Glu Leu Tyr Lys
                725

<210> 268
<211> 294
<212> PRT
<213> Artificial sequence

<220>
<223> eGFP-CLN-H6 (14)

<400> 268

Met Ala Ser Trp Ser His Pro Gln Phe Glu Lys Ala Ser Gly Thr Val
1               5                   10                  15

Ser Lys Gly Glu Glu Leu Phe Thr Gly Val Val Pro Ile Leu Val Glu
                20                  25                  30

Leu Asp Gly Asp Val Asn Gly His Lys Phe Ser Val Ser Gly Glu Gly
                35                  40                  45

Glu Gly Asp Ala Thr Tyr Gly Lys Leu Thr Leu Lys Phe Ile Cys Thr
        50                  55                  60

Thr Gly Lys Leu Pro Val Pro Trp Pro Thr Leu Val Thr Thr Leu Thr
65                  70                  75                  80

Tyr Gly Val Gln Cys Phe Ser Arg Tyr Pro Asp His Met Lys Gln His
                85                  90                  95

Asp Phe Phe Lys Ser Ala Met Pro Glu Gly Tyr Val Gln Glu Arg Thr
            100                 105                 110

Ile Phe Phe Lys Asp Asp Gly Asn Tyr Lys Thr Arg Ala Glu Val Lys
            115                 120                 125

Phe Glu Gly Asp Thr Leu Val Asn Arg Ile Glu Leu Lys Gly Ile Asp
    130                 135                 140

Phe Lys Glu Asp Gly Asn Ile Leu Gly His Lys Leu Glu Tyr Asn Tyr
145                 150                 155                 160

Asn Ser His Asn Val Tyr Ile Met Ala Asp Lys Gln Lys Asn Gly Ile
            165                 170                 175

Lys Val Asn Phe Lys Ile Arg His Asn Ile Glu Asp Gly Ser Val Gln
            180                 185                 190

Leu Ala Asp His Tyr Gln Gln Asn Thr Pro Ile Gly Asp Gly Pro Val
    195                 200                 205

Leu Leu Pro Asp Asn His Tyr Leu Ser Thr Gln Ser Ala Leu Ser Lys
    210                 215                 220

Asp Pro Asn Glu Lys Arg Asp His Met Val Leu Leu Glu Phe Val Thr
225                 230                 235                 240

Ala Ala Gly Ile Thr Leu Gly Met Asp Glu Leu Tyr Lys Gly Ser Tyr
            245                 250                 255

Ile Asp Thr Asp Ser Val Val Gly Asp Thr Ile Ile Asp Val Ser Gly
            260                 265                 270

Lys Lys Met Thr Ile Ala Glu Phe Tyr Asp Ser Thr Pro Asp Gly Ser
    275                 280                 285

His His His His His His
    290

374

<210> 269
<211> 288
<212> PRT
<213> Artificial sequence

<220>
<223> CysTag-H8-CLN-eGFP(C49S) (16)

<400> 269

Met Gly Cys His His His His His His His Tyr Ile Asp Thr Asp
1               5                   10                  15

Ser Val Val Gly Asp Thr Ile Ile Asp Val Ser Gly Lys Lys Met Thr
            20                  25                  30

Ile Ala Glu Phe Tyr Asp Ser Thr Pro Asp Ser Gly Gly Ser Gly Gly
        35                  40                  45

Ser Met Val Ser Lys Gly Glu Glu Leu Phe Thr Gly Val Val Pro Ile
    50                  55                  60

Leu Val Glu Leu Asp Gly Asp Val Asn Gly His Lys Phe Ser Val Ser
65                  70                  75                  80

Gly Glu Gly Glu Gly Asp Ala Thr Tyr Gly Lys Leu Thr Leu Lys Phe
                85                  90                  95

Ile Ser Thr Thr Gly Lys Leu Pro Val Pro Trp Pro Thr Leu Val Thr
            100                 105                 110

Thr Leu Thr Tyr Gly Val Gln Cys Phe Ser Arg Tyr Pro Asp His Met
        115                 120                 125

Lys Gln His Asp Phe Phe Lys Ser Ala Met Pro Glu Gly Tyr Val Gln
    130                 135                 140

Glu Arg Thr Ile Phe Phe Lys Asp Asp Gly Asn Tyr Lys Thr Arg Ala
145                 150                 155                 160

Glu Val Lys Phe Glu Gly Asp Thr Leu Val Asn Arg Ile Glu Leu Lys
                165                 170                 175

Gly Ile Asp Phe Lys Glu Asp Gly Asn Ile Leu Gly His Lys Leu Glu
                180                 185                 190

Tyr Asn Tyr Asn Ser His Asn Val Tyr Ile Met Ala Asp Lys Gln Lys
            195                 200                 205

375

```
Asn Gly Ile Lys Val Asn Phe Lys Ile Arg His Asn Ile Glu Asp Gly
    210                 215             220

Ser Val Gln Leu Ala Asp His Tyr Gln Gln Asn Thr Pro Ile Gly Asp
225             230                 235                     240

Gly Pro Val Leu Leu Pro Asp Asn His Tyr Leu Ser Thr Gln Ser Ala
            245                 250                     255

Leu Ser Lys Asp Pro Asn Glu Lys Arg Asp His Met Val Leu Leu Glu
            260             265                 270

Phe Val Thr Ala Ala Gly Ile Thr Leu Gly Met Asp Glu Leu Tyr Lys
        275                 280                 285
```

```
<210>  270
<211>  727
<212>  PRT
<213>  Artificial sequence

<220>
<223>  HA-VHHGFP-CLC(N159'A)-Trx-TMD-mCherry (15a)

<400>  270
```

```
Met Glu Thr Asp Thr Leu Leu Leu Trp Val Leu Leu Leu Trp Val Pro
1               5               10                  15

Gly Ser Thr Gly Asp Tyr Pro Tyr Asp Val Pro Asp Tyr Ala Gly Ala
            20                  25                  30

His Ser Arg Met Ala Gln Val Gln Leu Val Glu Ser Gly Gly Ala Leu
            35                  40                  45

Val Gln Pro Gly Gly Ser Leu Arg Leu Ser Cys Ala Ala Ser Gly Phe
        50                  55                  60

Pro Val Asn Arg Tyr Ser Met Arg Trp Tyr Arg Gln Ala Pro Gly Lys
65                  70                  75                      80

Glu Arg Glu Trp Val Ala Gly Met Ser Ser Ala Gly Asp Arg Ser Ser
                85                  90                  95

Tyr Glu Asp Ser Val Lys Gly Arg Phe Thr Ile Ser Arg Asp Asp Ala
            100                 105                 110

Arg Asn Thr Val Tyr Leu Gln Met Asn Ser Leu Lys Pro Glu Asp Thr
            115                 120                 125

Ala Val Tyr Tyr Cys Asn Val Asn Val Gly Phe Glu Tyr Trp Gly Gln
```

130            135           140

Gly Thr Gln Val Thr Val Ser Ser Pro Asp Trp Ala Gln Ser Gly Gly
145             150            155            160

Gly Gly Ser Ser Ser Glu Ala Arg Asp Trp Val Lys Arg Val Gly Gly
            165           170            175

Lys Thr Ser Leu Ser Val Asn Thr Tyr Ser Gly Glu Val Glu Arg Lys
       180          185           190

Asn Ile Asn Tyr Ile Met Lys His Thr Val Lys Lys Arg Met Phe Lys
       195          200           205

Ile Lys Ala Gly Gly Lys Glu Val Ile Val Thr Ala Asp His Ser Val
     210          215           220

Met Val Lys Arg Asp Gly Lys Ile Ile Asp Val Lys Pro Thr Glu Met
225            230          235           240

Lys Gln Thr Asp Arg Val Val Lys Trp Met Leu Thr Gly Ser His Met
            245          250           255

Ile Glu Phe Ile Glu Phe Glu Ile Glu Asp Leu Gly Val Met Glu Ile
       260          265          270

Asp Val Tyr Asp Ile Glu Val Asp Gly Asn His Asn Phe Phe Gly Asn
       275          280          285

Asp Ile Leu Val His Asn Ser Val Tyr Leu Asn Ser Gly Gly Ser Gly
     290          295          300

Thr Gly Ser Asp Lys Ile Ile His Leu Thr Asp Asp Ser Phe Asp Thr
305           310          315          320

Asp Val Leu Lys Ala Asp Gly Ala Ile Leu Val Asp Phe Trp Ala His
            325          330          335

Trp Cys Gly Pro Cys Lys Met Ile Ala Pro Ile Leu Asp Glu Ile Ala
            340          345          350

Asp Glu Tyr Gln Gly Lys Leu Thr Val Ala Lys Leu Asn Ile Asp His
            355          360          365

Asn Pro Gly Thr Ala Pro Lys Tyr Gly Ile Arg Gly Ile Pro Thr Leu
       370          375          380

```
Leu Leu Phe Lys Asn Gly Glu Val Ala Ala Thr Lys Val Gly Ala Leu
385                 390             395                     400

Ser Lys Gly Gln Leu Lys Glu Phe Leu Asp Ala Asn Leu Ala Gly Ser
                405             410                 415

Glu Phe Arg Ser His His His His His His Tyr Val Asp Glu Gln Lys
            420             425                 430

Leu Ile Ser Glu Glu Asp Leu Asn Ala Val Gly Gln Asp Thr Gln Glu
            435             440                 445

Val Ile Val Val Pro His Ser Leu Pro Phe Lys Val Val Val Ile Ser
        450             455                 460

Ala Ile Leu Ala Leu Val Val Leu Thr Ile Ile Ser Leu Ile Ile Leu
465                 470             475                     480

Ile Met Leu Trp Gln Lys Lys Pro Arg Ala Ser Met Val Ser Lys Gly
                485             490                 495

Glu Glu Asp Asn Met Ala Ile Ile Lys Glu Phe Met Arg Phe Lys Val
            500             505                 510

His Met Glu Gly Ser Val Asn Gly His Glu Phe Glu Ile Glu Gly Glu
        515             520                 525

Gly Glu Gly Arg Pro Tyr Glu Gly Thr Gln Thr Ala Lys Leu Lys Val
    530             535                 540

Thr Lys Gly Gly Pro Leu Pro Phe Ala Trp Asp Ile Leu Ser Pro Gln
545             550                 555                     560

Phe Met Tyr Gly Ser Lys Ala Tyr Val Lys His Pro Ala Asp Ile Pro
            565             570                 575

Asp Tyr Leu Lys Leu Ser Phe Pro Glu Gly Phe Lys Trp Glu Arg Val
            580             585                 590

Met Asn Phe Glu Asp Gly Gly Val Val Thr Val Thr Gln Asp Ser Ser
            595             600                 605

Leu Gln Asp Gly Glu Phe Ile Tyr Lys Val Lys Leu Arg Gly Thr Asn
    610             615                 620

Phe Pro Ser Asp Gly Pro Val Met Gln Lys Lys Thr Met Gly Trp Glu
625             630                 635                     640
```

```
Ala Ser Ser Glu Arg Met Tyr Pro Glu Asp Gly Ala Leu Lys Gly Glu
             645             650             655

Ile Lys Gln Arg Leu Lys Leu Lys Asp Gly Gly His Tyr Asp Ala Glu
             660             665             670

Val Lys Thr Thr Tyr Lys Ala Lys Lys Pro Val Gln Leu Pro Gly Ala
             675             680             685

Tyr Asn Val Asn Ile Lys Leu Asp Ile Thr Ser His Asn Glu Asp Tyr
         690             695             700

Thr Ile Val Glu Gln Tyr Glu Arg Ala Glu Gly Arg His Ser Thr Gly
705             710             715             720

Gly Met Asp Glu Leu Tyr Lys
                725
```

```
<210>  271
<211>  1076
<212>  PRT
<213>  Artificial sequence

<220>
<223>  HA-VHHGFP-CLC-IFNAR1-mCherry (17)

<400>  271
```

```
Met Glu Thr Asp Thr Leu Leu Leu Trp Val Leu Leu Leu Trp Val Pro
1               5                 10                15

Gly Ser Thr Gly Asp Tyr Pro Tyr Asp Val Pro Asp Tyr Ala Gly Ala
             20              25              30

His Ser Arg Met Ala Gln Val Gln Leu Val Glu Ser Gly Gly Ala Leu
         35              40              45

Val Gln Pro Gly Gly Ser Leu Arg Leu Ser Cys Ala Ala Ser Gly Phe
     50              55              60

Pro Val Asn Arg Tyr Ser Met Arg Trp Tyr Arg Gln Ala Pro Gly Lys
65              70              75              80

Glu Arg Glu Trp Val Ala Gly Met Ser Ser Ala Gly Asp Arg Ser Ser
             85              90              95

Tyr Glu Asp Ser Val Lys Gly Arg Phe Thr Ile Ser Arg Asp Asp Ala
             100             105             110
```

Arg Asn Thr Val Tyr Leu Gln Met Asn Ser Leu Lys Pro Glu Asp Thr
115 120 125

Ala Val Tyr Tyr Cys Asn Val Asn Val Gly Phe Glu Tyr Trp Gly Gln
130 135 140

Gly Thr Gln Val Thr Val Ser Ser Pro Asp Trp Ala Gln Ser Gly Gly
145 150 155 160

Gly Gly Ser Ser Ser Glu Ala Arg Asp Trp Val Lys Arg Val Gly Gly
165 170 175

Lys Thr Ser Leu Ser Val Asn Thr Tyr Ser Gly Glu Val Glu Arg Lys
180 185 190

Asn Ile Asn Tyr Ile Met Lys His Thr Val Lys Lys Arg Met Phe Lys
195 200 205

Ile Lys Ala Gly Gly Lys Glu Val Ile Val Thr Ala Asp His Ser Val
210 215 220

Met Val Lys Arg Asp Gly Lys Ile Ile Asp Val Lys Pro Thr Glu Met
225 230 235 240

Lys Gln Thr Asp Arg Val Val Lys Trp Met Leu Thr Gly Ser His Met
245 250 255

Ile Glu Phe Ile Glu Phe Glu Ile Glu Asp Leu Gly Val Met Glu Ile
260 265 270

Asp Val Tyr Asp Ile Glu Val Asp Gly Asn His Asn Phe Phe Gly Asn
275 280 285

Asp Ile Leu Val His Asn Ser Val Tyr Leu Asn Ser Gln Pro Ala Arg
290 295 300

Ser Lys Asn Leu Lys Ser Pro Gln Lys Val Glu Val Asp Ile Ile Asp
305 310 315 320

Asp Asn Phe Ile Leu Arg Trp Asn Arg Ser Asp Glu Ser Val Gly Asn
325 330 335

Val Thr Phe Ser Phe Asp Tyr Gln Lys Thr Gly Met Asp Asn Trp Ile
340 345 350

Lys Leu Ser Gly Cys Gln Asn Ile Thr Ser Thr Lys Cys Asn Phe Ser
355 360 365

380

Ser Leu Lys Leu Asn Val Tyr Glu Glu Ile Lys Leu Arg Ile Arg Ala
370            375            380

Glu Lys Glu Asn Thr Ser Ser Trp Tyr Glu Val Asp Ser Phe Thr Pro
385            390            395            400

Phe Arg Lys Ala Gln Ile Gly Pro Pro Glu Val His Leu Glu Ala Glu
405            410            415

Asp Lys Ala Ile Val Ile His Ile Ser Pro Gly Thr Lys Asp Ser Val
420            425            430

Met Trp Ala Leu Asp Gly Leu Ser Phe Thr Tyr Ser Leu Leu Ile Trp
435            440            445

Lys Asn Ser Ser Gly Val Glu Glu Arg Ile Glu Asn Ile Tyr Ser Arg
450            455            460

His Lys Ile Tyr Lys Leu Ser Pro Glu Thr Thr Tyr Cys Leu Lys Val
465            470            475            480

Lys Ala Ala Leu Leu Thr Ser Trp Lys Ile Gly Val Tyr Ser Pro Val
485            490            495

His Cys Ile Lys Thr Thr Val Glu Asn Glu Leu Pro Pro Pro Glu Asn
500            505            510

Ile Glu Val Ser Val Gln Asn Gln Asn Tyr Val Leu Lys Trp Asp Tyr
515            520            525

Thr Tyr Ala Asn Met Thr Phe Gln Val Gln Trp Leu His Ala Phe Leu
530            535            540

Lys Arg Asn Pro Gly Asn His Leu Tyr Lys Trp Lys Gln Ile Pro Asp
545            550            555            560

Cys Glu Asn Val Lys Thr Thr Gln Cys Val Phe Pro Gln Asn Val Phe
565            570            575

Gln Lys Gly Ile Tyr Leu Leu Arg Val Gln Ala Ser Asp Gly Asn Asn
580            585            590

Thr Ser Phe Trp Ser Glu Glu Ile Lys Phe Asp Thr Glu Ile Gln Ala
595            600            605

Phe Leu Leu Pro Pro Val Phe Asn Ile Arg Ser Leu Ser Asp Ser Phe
610            615            620

381

His Ile Tyr Ile Gly Ala Pro Lys Gln Ser Gly Asn Thr Pro Val Ile
625                 630             635             640

Gln Asp Tyr Pro Leu Ile Tyr Glu Ile Ile Phe Trp Glu Asn Thr Ser
                645             650             655

Asn Ala Glu Arg Lys Ile Ile Glu Lys Lys Thr Asp Val Thr Val Pro
            660             665             670

Asn Leu Lys Pro Leu Thr Val Tyr Cys Val Lys Ala Arg Ala His Thr
            675             680             685

Met Asp Glu Lys Leu Asn Lys Ser Ser Val Phe Ser Asp Ala Val Cys
            690             695             700

Glu Lys Thr Lys Pro Gly Asn Thr Ser Lys Ile Trp Leu Ile Val Gly
705             710             715             720

Ile Cys Ile Ala Leu Phe Ala Leu Pro Phe Val Ile Tyr Ala Ala Lys
                725             730             735

Val Phe Leu Arg Cys Ile Asn Tyr Val Phe Phe Pro Ser Leu Lys Pro
            740             745             750

Ser Ser Ser Ile Asp Glu Tyr Phe Ser Glu Gln Pro Leu Lys Asn Leu
            755             760             765

Leu Leu Ser Thr Ser Glu Glu Gln Ile Glu Lys Cys Phe Ile Ile Glu
            770             775             780

Asn Ile Ser Thr Ile Ala Thr Val Glu Glu Thr Asn Gln Thr Asp Glu
785             790             795             800

Asp His Lys Lys Tyr Ser Ser Gln Thr Ser Gln Asp Ser Gly Asn Tyr
                805             810             815

Ser Asn Glu Asp Glu Ser Glu Ser Lys Thr Ser Glu Glu Leu Gln Gln
            820             825             830

Asp Phe Val Leu Ala Glu Ala Ser Met Val Ser Lys Gly Glu Glu Asp
            835             840             845

Asn Met Ala Ile Ile Lys Glu Phe Met Arg Phe Lys Val His Met Glu
            850             855             860

Gly Ser Val Asn Gly His Glu Phe Glu Ile Glu Gly Glu Gly Glu Gly

382

865                     870                     875                     880

Arg Pro Tyr Glu Gly Thr Gln Thr Ala Lys Leu Lys Val Thr Lys Gly
                885                     890                     895

Gly Pro Leu Pro Phe Ala Trp Asp Ile Leu Ser Pro Gln Phe Met Tyr
                900                     905                     910

Gly Ser Lys Ala Tyr Val Lys His Pro Ala Asp Ile Pro Asp Tyr Leu
                915                     920                     925

Lys Leu Ser Phe Pro Glu Gly Phe Lys Trp Glu Arg Val Met Asn Phe
        930                     935                     940

Glu Asp Gly Gly Val Val Thr Val Thr Gln Asp Ser Ser Leu Gln Asp
945                     950                     955                     960

Gly Glu Phe Ile Tyr Lys Val Lys Leu Arg Gly Thr Asn Phe Pro Ser
                965                     970                     975

Asp Gly Pro Val Met Gln Lys Lys Thr Met Gly Trp Glu Ala Ser Ser
                980                     985                     990

Glu Arg Met Tyr Pro Glu Asp Gly  Ala Leu Lys Gly Glu  Ile Lys Gln
            995                     1000                    1005

Arg Leu  Lys Leu Lys Asp Gly  Gly His Tyr Asp Ala  Glu Val Lys
    1010                    1015                    1020

Thr Thr  Tyr Lys Ala Lys Lys  Pro Val Gln Leu Pro  Gly Ala Tyr
    1025                    1030                    1035

Asn Val  Asn Ile Lys Leu Asp  Ile Thr Ser His Asn  Glu Asp Tyr
    1040                    1045                    1050

Thr Ile  Val Glu Gln Tyr Glu  Arg Ala Glu Gly Arg  His Ser Thr
    1055                    1060                    1065

Gly Gly  Met Asp Glu Leu Tyr  Lys
    1070                    1075

<210>  272
<211>  1076
<212>  PRT
<213>  Artificial sequence

<220>
<223>  HA-VHHGFP-CLC(N159'A)-IFNAR1-mCherry (17a)

<400> 272

| Met | Glu | Thr | Asp | Thr | Leu | Leu | Leu | Trp | Val | Leu | Leu | Leu | Trp | Val | Pro |
| 1 | | | | 5 | | | | | 10 | | | | | 15 | |

Gly Ser Thr Gly Asp Tyr Pro Tyr Asp Val Pro Asp Tyr Ala Gly Ala
    20              25              30

His Ser Arg Met Ala Gln Val Gln Leu Val Glu Ser Gly Gly Ala Leu
    35              40              45

Val Gln Pro Gly Gly Ser Leu Arg Leu Ser Cys Ala Ala Ser Gly Phe
    50              55              60

Pro Val Asn Arg Tyr Ser Met Arg Trp Tyr Arg Gln Ala Pro Gly Lys
65              70              75              80

Glu Arg Glu Trp Val Ala Gly Met Ser Ser Ala Gly Asp Arg Ser Ser
                85              90              95

Tyr Glu Asp Ser Val Lys Gly Arg Phe Thr Ile Ser Arg Asp Asp Ala
        100             105             110

Arg Asn Thr Val Tyr Leu Gln Met Asn Ser Leu Lys Pro Glu Asp Thr
        115             120             125

Ala Val Tyr Tyr Cys Asn Val Asn Val Gly Phe Glu Tyr Trp Gly Gln
    130             135             140

Gly Thr Gln Val Thr Val Ser Ser Pro Asp Trp Ala Gln Ser Gly Gly
145             150             155             160

Gly Gly Ser Ser Ser Glu Ala Arg Asp Trp Val Lys Arg Val Gly Gly
            165             170             175

Lys Thr Ser Leu Ser Val Asn Thr Tyr Ser Gly Glu Val Glu Arg Lys
        180             185             190

Asn Ile Asn Tyr Ile Met Lys His Thr Val Lys Lys Arg Met Phe Lys
        195             200             205

Ile Lys Ala Gly Gly Lys Glu Val Ile Val Thr Ala Asp His Ser Val
    210             215             220

Met Val Lys Arg Asp Gly Lys Ile Ile Asp Val Lys Pro Thr Glu Met
225             230             235             240

Lys Gln Thr Asp Arg Val Val Lys Trp Met Leu Thr Gly Ser His Met

                    245                      250                              255


        Ile Glu Phe Ile Glu Phe Glu Ile Glu Asp Leu Gly Val Met Glu Ile
                    260                      265                      270


        Asp Val Tyr Asp Ile Glu Val Asp Gly Asn His Asn Phe Phe Gly Asn
                    275                      280                      285


        Asp Ile Leu Val His Ala Ser Val Tyr Leu Asn Ser Gln Pro Ala Arg
            290                      295                      300


        Ser Lys Asn Leu Lys Ser Pro Gln Lys Val Glu Val Asp Ile Ile Asp
        305                      310                      315                      320


        Asp Asn Phe Ile Leu Arg Trp Asn Arg Ser Asp Glu Ser Val Gly Asn
                    325                      330                      335


        Val Thr Phe Ser Phe Asp Tyr Gln Lys Thr Gly Met Asp Asn Trp Ile
                    340                      345                      350


        Lys Leu Ser Gly Cys Gln Asn Ile Thr Ser Thr Lys Cys Asn Phe Ser
                    355                      360                      365


        Ser Leu Lys Leu Asn Val Tyr Glu Glu Ile Lys Leu Arg Ile Arg Ala
            370                      375                      380


        Glu Lys Glu Asn Thr Ser Ser Trp Tyr Glu Val Asp Ser Phe Thr Pro
        385                      390                      395                      400


        Phe Arg Lys Ala Gln Ile Gly Pro Pro Glu Val His Leu Glu Ala Glu
                    405                      410                      415


        Asp Lys Ala Ile Val Ile His Ile Ser Pro Gly Thr Lys Asp Ser Val
                    420                      425                      430


        Met Trp Ala Leu Asp Gly Leu Ser Phe Thr Tyr Ser Leu Leu Ile Trp
                    435                      440                      445


        Lys Asn Ser Ser Gly Val Glu Glu Arg Ile Glu Asn Ile Tyr Ser Arg
                    450                      455                      460


        His Lys Ile Tyr Lys Leu Ser Pro Glu Thr Thr Tyr Cys Leu Lys Val
        465                      470                      475                      480


        Lys Ala Ala Leu Leu Thr Ser Trp Lys Ile Gly Val Tyr Ser Pro Val
                    485                      490                      495

```
His Cys Ile Lys Thr Thr Val Glu Asn Glu Leu Pro Pro Pro Glu Asn
            500             505             510

Ile Glu Val Ser Val Gln Asn Gln Asn Tyr Val Leu Lys Trp Asp Tyr
            515             520             525

Thr Tyr Ala Asn Met Thr Phe Gln Val Gln Trp Leu His Ala Phe Leu
            530             535             540

Lys Arg Asn Pro Gly Asn His Leu Tyr Lys Trp Lys Gln Ile Pro Asp
545             550             555             560

Cys Glu Asn Val Lys Thr Thr Gln Cys Val Phe Pro Gln Asn Val Phe
            565             570             575

Gln Lys Gly Ile Tyr Leu Leu Arg Val Gln Ala Ser Asp Gly Asn Asn
            580             585             590

Thr Ser Phe Trp Ser Glu Glu Ile Lys Phe Asp Thr Glu Ile Gln Ala
            595             600             605

Phe Leu Leu Pro Pro Val Phe Asn Ile Arg Ser Leu Ser Asp Ser Phe
    610             615             620

His Ile Tyr Ile Gly Ala Pro Lys Gln Ser Gly Asn Thr Pro Val Ile
625             630             635             640

Gln Asp Tyr Pro Leu Ile Tyr Glu Ile Ile Phe Trp Glu Asn Thr Ser
            645             650             655

Asn Ala Glu Arg Lys Ile Ile Glu Lys Lys Thr Asp Val Thr Val Pro
            660             665             670

Asn Leu Lys Pro Leu Thr Val Tyr Cys Val Lys Ala Arg Ala His Thr
            675             680             685

Met Asp Glu Lys Leu Asn Lys Ser Ser Val Phe Ser Asp Ala Val Cys
            690             695             700

Glu Lys Thr Lys Pro Gly Asn Thr Ser Lys Ile Trp Leu Ile Val Gly
705             710             715             720

Ile Cys Ile Ala Leu Phe Ala Leu Pro Phe Val Ile Tyr Ala Ala Lys
            725             730             735

Val Phe Leu Arg Cys Ile Asn Tyr Val Phe Phe Pro Ser Leu Lys Pro
            740             745             750
```

386

```
Ser Ser Ser Ile Asp Glu Tyr Phe Ser Glu Gln Pro Leu Lys Asn Leu
        755             760             765

Leu Leu Ser Thr Ser Glu Glu Gln Ile Glu Lys Cys Phe Ile Ile Glu
    770             775             780

Asn Ile Ser Thr Ile Ala Thr Val Glu Glu Thr Asn Gln Thr Asp Glu
785             790             795             800

Asp His Lys Lys Tyr Ser Ser Gln Thr Ser Gln Asp Ser Gly Asn Tyr
            805             810             815

Ser Asn Glu Asp Glu Ser Glu Ser Lys Thr Ser Glu Glu Leu Gln Gln
            820             825             830

Asp Phe Val Leu Ala Glu Ala Ser Met Val Ser Lys Gly Glu Glu Asp
        835             840             845

Asn Met Ala Ile Ile Lys Glu Phe Met Arg Phe Lys Val His Met Glu
    850             855             860

Gly Ser Val Asn Gly His Glu Phe Glu Ile Glu Gly Glu Gly Glu Gly
865             870             875             880

Arg Pro Tyr Glu Gly Thr Gln Thr Ala Lys Leu Lys Val Thr Lys Gly
            885             890             895

Gly Pro Leu Pro Phe Ala Trp Asp Ile Leu Ser Pro Gln Phe Met Tyr
        900             905             910

Gly Ser Lys Ala Tyr Val Lys His Pro Ala Asp Ile Pro Asp Tyr Leu
        915             920             925

Lys Leu Ser Phe Pro Glu Gly Phe Lys Trp Glu Arg Val Met Asn Phe
    930             935             940

Glu Asp Gly Gly Val Val Thr Val Thr Gln Asp Ser Ser Leu Gln Asp
945             950             955             960

Gly Glu Phe Ile Tyr Lys Val Lys Leu Arg Gly Thr Asn Phe Pro Ser
            965             970             975

Asp Gly Pro Val Met Gln Lys Lys Thr Met Gly Trp Glu Ala Ser Ser
        980             985             990

Glu Arg Met Tyr Pro Glu Asp Gly  Ala Leu Lys Gly Glu  Ile Lys Gln
    995             1000            1005
```

387

```
Arg Leu  Lys Leu Lys Asp Gly  Gly His Tyr Asp Ala  Glu Val Lys
    1010            1015           1020


Thr Thr  Tyr Lys Ala Lys Lys  Pro Val Gln Leu Pro  Gly Ala Tyr
    1025            1030           1035


Asn Val  Asn Ile Lys Leu Asp  Ile Thr Ser His Asn  Glu Asp Tyr
    1040            1045           1050


Thr Ile  Val Glu Gln Tyr Glu  Arg Ala Glu Gly Arg  His Ser Thr
    1055            1060           1065


Gly Gly  Met Asp Glu Leu Tyr  Lys
    1070            1075


<210>  273
<211>  700
<212>  PRT
<213>  Artificial sequence

<220>
<223>  HA-eGFP-Trx-TMD-mCherry

<400>  273

Met Glu Thr Asp Thr Leu Leu Leu Trp Val Leu Leu Leu Trp Val Pro
1            5                10                15


Gly Ser Thr Gly Asp Tyr Pro Tyr Asp Val Pro Asp Tyr Ala Gly Ala
            20                25                30


His Ser Arg Val Ser Lys Gly Glu Glu Leu Phe Thr Gly Val Val Pro
        35                40                45


Ile Leu Val Glu Leu Asp Gly Asp Val Asn Gly His Lys Phe Ser Val
    50                55                60


Ser Gly Glu Gly Glu Gly Asp Ala Thr Tyr Gly Lys Leu Thr Leu Lys
65                70                75                80


Phe Ile Cys Thr Thr Gly Lys Leu Pro Val Pro Trp Pro Thr Leu Val
                85                90                95


Thr Thr Leu Thr Tyr Gly Val Gln Cys Phe Ser Arg Tyr Pro Asp His
            100               105               110


Met Lys Gln His Asp Phe Phe Lys Ser Ala Met Pro Glu Gly Tyr Val
            115               120               125
```

Gln Glu Arg Thr Ile Phe Phe Lys Asp Asp Gly Asn Tyr Lys Thr Arg
130                     135                 140

Ala Glu Val Lys Phe Glu Gly Asp Thr Leu Val Asn Arg Ile Glu Leu
145                 150                 155                 160

Lys Gly Ile Asp Phe Lys Glu Asp Gly Asn Ile Leu Gly His Lys Leu
                165                 170                 175

Glu Tyr Asn Tyr Asn Ser His Asn Val Tyr Ile Met Ala Asp Lys Gln
            180                 185                 190

Lys Asn Gly Ile Lys Val Asn Phe Lys Ile Arg His Asn Ile Glu Asp
            195                 200                 205

Gly Ser Val Gln Leu Ala Asp His Tyr Gln Gln Asn Thr Pro Ile Gly
    210                 215                 220

Asp Gly Pro Val Leu Leu Pro Asp Asn His Tyr Leu Ser Thr Gln Ser
225                 230                 235                 240

Ala Leu Ser Lys Asp Pro Asn Glu Lys Arg Asp His Met Val Leu Leu
            245                 250                 255

Glu Phe Val Thr Ala Ala Gly Ile Thr Leu Gly Met Asp Glu Leu Tyr
        260                 265                 270

Lys Ser Ser Ser Gly Thr Gly Ser Asp Lys Ile Ile His Leu Thr Asp
    275                 280                 285

Asp Ser Phe Asp Thr Asp Val Leu Lys Ala Asp Gly Ala Ile Leu Val
    290                 295                 300

Asp Phe Trp Ala His Trp Cys Gly Pro Cys Lys Met Ile Ala Pro Ile
305                 310                 315                 320

Leu Asp Glu Ile Ala Asp Glu Tyr Gln Gly Lys Leu Thr Val Ala Lys
            325                 330                 335

Leu Asn Ile Asp His Asn Pro Gly Thr Ala Pro Lys Tyr Gly Ile Arg
            340                 345                 350

Gly Ile Pro Thr Leu Leu Leu Phe Lys Asn Gly Glu Val Ala Ala Thr
    355                 360                 365

Lys Val Gly Ala Leu Ser Lys Gly Gln Leu Lys Glu Phe Leu Asp Ala
    370                 375                 380

```
Asn Leu Ala Gly Ser Glu Phe Arg Ser His His His His His His Tyr
385             390             395                     400

Val Asp Glu Gln Lys Leu Ile Ser Glu Glu Asp Leu Asn Ala Val Gly
                405             410                     415

Gln Asp Thr Gln Glu Val Ile Val Val Pro His Ser Leu Pro Phe Lys
            420             425                 430

Val Val Val Ile Ser Ala Ile Leu Ala Leu Val Val Leu Thr Ile Ile
            435             440                 445

Ser Leu Ile Ile Leu Ile Met Leu Trp Gln Lys Lys Pro Arg Ala Ser
        450             455             460

Met Val Ser Lys Gly Glu Glu Asp Asn Met Ala Ile Ile Lys Glu Phe
465             470             475                     480

Met Arg Phe Lys Val His Met Glu Gly Ser Val Asn Gly His Glu Phe
            485             490                 495

Glu Ile Glu Gly Glu Gly Glu Gly Arg Pro Tyr Glu Gly Thr Gln Thr
            500             505                 510

Ala Lys Leu Lys Val Thr Lys Gly Gly Pro Leu Pro Phe Ala Trp Asp
        515             520             525

Ile Leu Ser Pro Gln Phe Met Tyr Gly Ser Lys Ala Tyr Val Lys His
        530             535             540

Pro Ala Asp Ile Pro Asp Tyr Leu Lys Leu Ser Phe Pro Glu Gly Phe
545             550             555                     560

Lys Trp Glu Arg Val Met Asn Phe Glu Asp Gly Gly Val Val Thr Val
            565             570                 575

Thr Gln Asp Ser Ser Leu Gln Asp Gly Glu Phe Ile Tyr Lys Val Lys
            580             585                 590

Leu Arg Gly Thr Asn Phe Pro Ser Asp Gly Pro Val Met Gln Lys Lys
            595             600                 605

Thr Met Gly Trp Glu Ala Ser Ser Glu Arg Met Tyr Pro Glu Asp Gly
            610             615                 620

Ala Leu Lys Gly Glu Ile Lys Gln Arg Leu Lys Leu Lys Asp Gly Gly
```

```
        625                    630                    635                    640


        His Tyr Asp Ala Glu Val Lys Thr Thr Tyr Lys Ala Lys Lys Pro Val
                        645                650                655


        Gln Leu Pro Gly Ala Tyr Asn Val Asn Ile Lys Leu Asp Ile Thr Ser
                        660                665                670


        His Asn Glu Asp Tyr Thr Ile Val Glu Gln Tyr Glu Arg Ala Glu Gly
                        675                680                685


        Arg His Ser Thr Gly Gly Met Asp Glu Leu Tyr Lys
                        690                695                700


        <210>  274
        <211>  935
        <212>  PRT
        <213>  Artificial sequence

        <220>
        <223>  HA-EGFR-mCherry

        <400>  274

        Met Glu Thr Asp Thr Leu Leu Leu Trp Val Leu Leu Leu Trp Val Pro
        1               5                   10                  15


        Gly Ser Thr Gly Asp Tyr Pro Tyr Asp Val Pro Asp Tyr Ala Gly Ala
                        20                  25                  30


        His Ser Arg Glu Leu Glu Glu Lys Lys Val Cys Gln Gly Thr Ser Asn
                35                  40                  45


        Lys Leu Thr Gln Leu Gly Thr Phe Glu Asp His Phe Leu Ser Leu Gln
                50                  55                  60


        Arg Met Phe Asn Asn Cys Glu Val Val Leu Gly Asn Leu Glu Ile Thr
        65                  70                  75                  80


        Tyr Val Gln Arg Asn Tyr Asp Leu Ser Phe Leu Lys Thr Ile Gln Glu
                        85                  90                  95


        Val Ala Gly Tyr Val Leu Ile Ala Leu Asn Thr Val Glu Arg Ile Pro
                        100                 105                 110


        Leu Glu Asn Leu Gln Ile Ile Arg Gly Asn Met Tyr Tyr Glu Asn Ser
                        115                 120                 125


        Tyr Ala Leu Ala Val Leu Ser Asn Tyr Asp Ala Asn Lys Thr Gly Leu
                        130                 135                 140
```

Lys Glu Leu Pro Met Arg Asn Leu Gln Glu Ile Leu His Gly Ala Val
145                 150                 155                 160

Arg Phe Ser Asn Asn Pro Ala Leu Cys Asn Val Glu Ser Ile Gln Trp
                165                 170                 175

Arg Asp Ile Val Ser Ser Asp Phe Leu Ser Asn Met Ser Met Asp Phe
                180                 185                 190

Gln Asn His Leu Gly Ser Cys Gln Lys Cys Asp Pro Ser Cys Pro Asn
                195                 200                 205

Gly Ser Cys Trp Gly Ala Gly Glu Glu Asn Cys Gln Lys Leu Thr Lys
            210                 215                 220

Ile Ile Cys Ala Gln Gln Cys Ser Gly Arg Cys Arg Gly Lys Ser Pro
225                 230                 235                 240

Ser Asp Cys Cys His Asn Gln Cys Ala Ala Gly Cys Thr Gly Pro Arg
                245                 250                 255

Glu Ser Asp Cys Leu Val Cys Arg Lys Phe Arg Asp Glu Ala Thr Cys
                260                 265                 270

Lys Asp Thr Cys Pro Pro Leu Met Leu Tyr Asn Pro Thr Thr Tyr Gln
                275                 280                 285

Met Asp Val Asn Pro Glu Gly Lys Tyr Ser Phe Gly Ala Thr Cys Val
            290                 295                 300

Lys Lys Cys Pro Arg Asn Tyr Val Val Thr Asp His Gly Ser Cys Val
305                 310                 315                 320

Arg Ala Cys Gly Ala Asp Ser Tyr Glu Met Glu Glu Asp Gly Val Arg
                325                 330                 335

Lys Cys Lys Lys Cys Glu Gly Pro Cys Arg Lys Val Cys Asn Gly Ile
                340                 345                 350

Gly Ile Gly Glu Phe Lys Asp Ser Leu Ser Ile Asn Ala Thr Asn Ile
            355                 360                 365

Lys His Phe Lys Asn Cys Thr Ser Ile Ser Gly Asp Leu His Ile Leu
            370                 375                 380

Pro Val Ala Phe Arg Gly Asp Ser Phe Thr His Thr Pro Pro Leu Asp

385                     390                     395                     400

Pro Gln Glu Leu Asp Ile Leu Lys Thr Val Lys Glu Ile Thr Gly Phe
                405                     410                     415

Leu Leu Ile Gln Ala Trp Pro Glu Asn Arg Thr Asp Leu His Ala Phe
                420                     425                     430

Glu Asn Leu Glu Ile Ile Arg Gly Arg Thr Lys Gln His Gly Gln Phe
                435                     440                     445

Ser Leu Ala Val Val Ser Leu Asn Ile Thr Ser Leu Gly Leu Arg Ser
    450                     455                     460

Leu Lys Glu Ile Ser Asp Gly Asp Val Ile Ile Ser Gly Asn Lys Asn
465                     470                     475                     480

Leu Cys Tyr Ala Asn Thr Ile Asn Trp Lys Lys Leu Phe Gly Thr Ser
                485                     490                     495

Gly Gln Lys Thr Lys Ile Ile Ser Asn Arg Gly Glu Asn Ser Cys Lys
                500                     505                     510

Ala Thr Gly Gln Val Cys His Ala Leu Cys Ser Pro Glu Gly Cys Trp
                515                     520                     525

Gly Pro Glu Pro Arg Asp Cys Val Ser Cys Arg Asn Val Ser Arg Gly
        530                     535                     540

Arg Glu Cys Val Asp Lys Cys Asn Leu Leu Glu Gly Glu Pro Arg Glu
545                     550                     555                     560

Phe Val Glu Asn Ser Glu Cys Ile Gln Cys His Pro Glu Cys Leu Pro
                565                     570                     575

Gln Ala Met Asn Ile Thr Cys Thr Gly Arg Gly Pro Asp Asn Cys Ile
                580                     585                     590

Gln Cys Ala His Tyr Ile Asp Gly Pro His Cys Val Lys Thr Cys Pro
        595                     600                     605

Ala Gly Val Met Gly Glu Asn Asn Thr Leu Val Trp Lys Tyr Ala Asp
        610                     615                     620

Ala Gly His Val Cys His Leu Cys His Pro Asn Cys Thr Tyr Gly Cys
625                     630                     635                     640

393

```
Thr Gly Pro Gly Leu Glu Gly Cys Pro Thr Asn Gly Pro Lys Ile Pro
            645             650             655

Ser Ile Ala Thr Gly Met Val Gly Ala Leu Leu Leu Leu Leu Val Val
            660             665             670

Ala Leu Gly Ile Gly Leu Phe Met Arg Arg Arg His Ile Val Arg Lys
            675             680             685

Arg Thr Leu Arg Arg Gly Ser Gly Ser Ala Ser Met Val Ser Lys Gly
            690             695             700

Glu Glu Asp Asn Met Ala Ile Ile Lys Glu Phe Met Arg Phe Lys Val
705             710             715             720

His Met Glu Gly Ser Val Asn Gly His Glu Phe Glu Ile Glu Gly Glu
            725             730             735

Gly Glu Gly Arg Pro Tyr Glu Gly Thr Gln Thr Ala Lys Leu Lys Val
            740             745             750

Thr Lys Gly Gly Pro Leu Pro Phe Ala Trp Asp Ile Leu Ser Pro Gln
            755             760             765

Phe Met Tyr Gly Ser Lys Ala Tyr Val Lys His Pro Ala Asp Ile Pro
    770             775             780

Asp Tyr Leu Lys Leu Ser Phe Pro Glu Gly Phe Lys Trp Glu Arg Val
785             790             795             800

Met Asn Phe Glu Asp Gly Gly Val Val Thr Val Thr Gln Asp Ser Ser
            805             810             815

Leu Gln Asp Gly Glu Phe Ile Tyr Lys Val Lys Leu Arg Gly Thr Asn
            820             825             830

Phe Pro Ser Asp Gly Pro Val Met Gln Lys Lys Thr Met Gly Trp Glu
            835             840             845

Ala Ser Ser Glu Arg Met Tyr Pro Glu Asp Gly Ala Leu Lys Gly Glu
            850             855             860

Ile Lys Gln Arg Leu Lys Leu Lys Asp Gly Gly His Tyr Asp Ala Glu
865             870             875             880

Val Lys Thr Thr Tyr Lys Ala Lys Lys Pro Val Gln Leu Pro Gly Ala
            885             890             895
```

394

```
Tyr Asn Val Asn Ile Lys Leu Asp Ile Thr Ser His Asn Glu Asp Tyr
        900                 905             910

Thr Ile Val Glu Gln Tyr Glu Arg Ala Glu Gly Arg His Ser Thr Gly
        915                 920             925

Gly Met Asp Glu Leu Tyr Lys
        930             935
```

<210> 275
<211> 259
<212> PRT
<213> Artificial sequence

<220>
<223> Amino acid sequence of CL-intein construct IntC-Trx-H6

<400> 275

```
Met Gly Glu Ala Arg Asp Trp Val Lys Arg Val Gly Gly Lys Thr Ser
1               5               10              15

Leu Ser Val Asn Thr Tyr Ser Gly Glu Val Glu Arg Lys Asn Ile Asn
        20                  25              30

Tyr Ile Met Lys His Thr Val Lys Lys Arg Met Phe Lys Ile Lys Ala
        35                  40              45

Gly Gly Lys Glu Val Ile Val Thr Ala Asp His Ser Val Met Val Lys
        50                  55              60

Arg Asp Gly Lys Ile Ile Asp Val Lys Pro Thr Glu Met Lys Gln Thr
65                  70              75              80

Asp Arg Val Val Lys Trp Met Leu Thr Gly Ser His Met Ile Glu Phe
        85                  90              95

Ile Glu Phe Glu Ile Glu Asp Leu Gly Val Met Glu Ile Asp Val Tyr
        100                 105             110

Asp Ile Glu Val Asp Gly Asn His Asn Phe Phe Gly Asn Asp Ile Leu
        115                 120             125

Val His Asn Ser Val Tyr Leu Asn Gly Thr Gly Ser Asp Lys Ile Ile
        130                 135             140

His Leu Thr Asp Asp Ser Phe Asp Thr Asp Val Leu Lys Ala Asp Gly
145                 150             155             160
```

```
Ala Ile Leu Val Asp Phe Trp Ala His Trp Cys Gly Pro Cys Lys Met
                165             170             175

Ile Ala Pro Ile Leu Asp Glu Ile Ala Asp Glu Tyr Gln Gly Lys Leu
                180             185             190

Thr Val Ala Lys Leu Asn Ile Asp His Asn Pro Gly Thr Ala Pro Lys
                195             200             205

Tyr Gly Ile Arg Gly Ile Pro Thr Leu Leu Leu Phe Lys Asn Gly Glu
    210             215             220

Val Ala Ala Thr Lys Val Gly Ala Leu Ser Lys Gly Gln Leu Lys Glu
225             230             235             240

Phe Leu Asp Ala Asn Leu Ala Gly Ser Glu Phe Arg Ser His His His
                245             250             255

His His His
```

<210> 276
<211> 222
<212> PRT
<213> Artificial sequence

<220>
<223> Amino acid sequence of CL-intein construct SBP-IntC-SBP

<400> 276

```
Met Asp Glu Lys Thr Thr Gly Trp Arg Gly Gly His Val Val Glu Gly
1               5               10              15

Leu Ala Gly Glu Leu Glu Gln Leu Arg Ala Arg Leu Glu His His Pro
                20              25              30

Gln Gly Gln Arg Glu Pro Gly Ala Ser Gly Gly Gly Gly Ser Ser Ser
                35              40              45

Glu Ala Arg Asp Trp Val Lys Arg Val Gly Gly Lys Thr Ser Leu Ser
    50              55              60

Val Asn Thr Tyr Ser Gly Glu Val Glu Arg Lys Asn Ile Asn Tyr Ile
65              70              75              80

Met Lys His Thr Val Lys Lys Arg Met Phe Lys Ile Lys Ala Gly Gly
                85              90              95

Lys Glu Val Ile Val Thr Ala Asp His Ser Val Met Val Lys Arg Asp
```

```
                     100                   105                   110

        Gly Lys Ile Ile Asp Val Lys Pro Thr Glu Met Lys Gln Thr Asp Arg
                115                   120                   125


        Val Val Lys Trp Met Leu Thr Gly Ser His Met Ile Glu Phe Ile Glu
            130                   135                   140


        Phe Glu Ile Glu Asp Leu Gly Val Met Glu Ile Asp Val Tyr Asp Ile
        145                   150                   155                   160


        Glu Val Asp Gly Asn His Asn Phe Phe Gly Asn Asp Ile Leu Val His
                        165                   170                   175


        Asn Ser Val Tyr Leu Asn Gly Thr Met Asp Glu Lys Thr Thr Gly Trp
                    180                   185                   190


        Arg Gly Gly His Val Val Glu Gly Leu Ala Gly Glu Leu Glu Gln Leu
                195                   200                   205


        Arg Ala Arg Leu Glu His His Pro Gln Gly Gln Arg Glu Pro
            210                   215                   220
```

```
<210>  277
<211>  419
<212>  PRT
<213>  Artificial sequence

<220>
<223>  Amino acid sequence of PolB-16_OarG-intein construct MBP-IntN-H6

<400>  277
```

```
        Met Lys Thr Glu Glu Gly Lys Leu Val Ile Trp Ile Asn Gly Asp Lys
        1                   5                   10                  15


        Gly Tyr Asn Gly Leu Ala Glu Val Gly Lys Lys Phe Glu Lys Asp Thr
                    20                  25                  30


        Gly Ile Lys Val Thr Val Glu His Pro Asp Lys Leu Glu Glu Lys Phe
                35                  40                  45


        Pro Gln Val Ala Ala Thr Gly Asp Gly Pro Asp Ile Ile Phe Trp Ala
            50                  55                  60


        His Asp Arg Phe Gly Gly Tyr Ala Gln Ser Gly Leu Leu Ala Glu Ile
        65                  70                  75                  80


        Thr Pro Asp Lys Ala Phe Gln Asp Lys Leu Tyr Pro Phe Thr Trp Asp
                    85                  90                  95
```

397

Ala Val Arg Tyr Asn Gly Lys Leu Ile Ala Tyr Pro Ile Ala Val Glu
          100                105              110

Ala Leu Ser Leu Ile Tyr Asn Lys Asp Leu Leu Pro Asn Pro Pro Lys
          115                120              125

Thr Trp Glu Glu Ile Pro Ala Leu Asp Lys Glu Leu Lys Ala Lys Gly
          130                135              140

Lys Ser Ala Leu Met Phe Asn Leu Gln Glu Pro Tyr Phe Thr Trp Pro
145                150              155              160

Leu Ile Ala Ala Asp Gly Gly Tyr Ala Phe Lys Tyr Glu Asn Gly Lys
                165              170              175

Tyr Asp Ile Lys Asp Val Gly Val Asp Asn Ala Gly Ala Lys Ala Gly
          180                185              190

Leu Thr Phe Leu Val Asp Leu Ile Lys Asn Lys His Met Asn Ala Asp
          195              200              205

Thr Asp Tyr Ser Ile Ala Glu Ala Ala Phe Asn Lys Gly Glu Thr Ala
          210              215              220

Met Thr Ile Asn Gly Pro Trp Ala Trp Ser Asn Ile Asp Thr Ser Lys
225              230              235              240

Val Asn Tyr Gly Val Thr Val Leu Pro Thr Phe Lys Gly Gln Pro Ser
          245              250              255

Lys Pro Phe Val Gly Val Leu Ser Ala Gly Ile Asn Ala Ala Ser Pro
          260              265              270

Asn Lys Glu Leu Ala Lys Glu Phe Leu Glu Asn Tyr Leu Leu Thr Asp
          275              280              285

Glu Gly Leu Glu Ala Val Asn Lys Asp Lys Pro Leu Gly Ala Val Ala
          290              295              300

Leu Lys Ser Tyr Glu Glu Glu Leu Ala Lys Asp Pro Arg Ile Ala Ala
305              310              315              320

Thr Met Glu Asn Ala Gln Lys Gly Glu Ile Met Pro Asn Ile Pro Gln
          325              330              335

Met Ser Ala Phe Trp Tyr Ala Val Arg Thr Ala Val Ile Asn Ala Ala

340                    345                        350

Ser Gly Arg Gln Thr Val Asp Glu Ala Leu Lys Asp Ala Gln Thr Asn
     355                    360                365

Ser Ser Ser Asn Asn Asn Asn Asn Asn Asn Asn Asn Asn Leu Gly Ile
     370                    375                380

Glu Gly Arg Ile Ser Glu Phe Ser Gly Asp Thr Asp Ser Val His Gly
385                    390                    395                400

Lys Thr His Val Phe Ile Arg Ser Ile Lys Asn Gly Ser His His His
               405                    410                415

His His His

<210> 278
<211> 296
<212> PRT
<213> Artificial sequence

<220>
<223> Amino acid sequence of PolB-16_OarG-intein construct IntC-Trx-H6

<400> 278

Met Gln Glu Ala Lys Ile Asp Ile Lys Ser Leu Tyr Asp Ser Leu Ala
1                  5                    10                   15

Lys Lys Tyr Asp Val Gln His Lys Asn Ser Tyr Glu Val Ile Tyr Pro
               20                   25                   30

Lys Gly Tyr Glu Ile Lys Val Leu Gly Asn Lys Tyr Val Lys Leu Val
          35                   40                   45

Ala Met Ser Arg His Lys Thr Gln Lys His Leu Val Lys Ile Val Val
          50                   55                   60

Lys Ser Glu Lys Thr Ile Asp Ser Leu Asp Pro Ile Arg Gln Lys Ser
65                   70                   75                   80

Leu Leu Lys Lys Gln Asp Glu Val Val Val Thr Thr Asp His Ile Cys
               85                   90                   95

Met Val Tyr Asn Asp Asp His Phe Phe Glu Asn Val Asn Ala Lys Asn
               100                  105                  110

Leu Lys Val Gly Asn Tyr Val Ser Val Tyr Asp Glu Ala Ser Asp Lys
          115                  120                  125

399

```
Glu Val Ile Gly Glu Ile Ala Ser Ile Glu Asp Leu Gly Met Thr Asp
    130             135             140

Asp Tyr Val Tyr Asp Cys Glu Val Asp Asp Asp Ser His Ala Phe Tyr
145             150             155                 160

Ala Ser Asn Ile Leu Val His Asn Ser Gln Phe Cys Asn Gly Thr Gly
            165             170             175

Ser Asp Lys Ile Ile His Leu Thr Asp Asp Ser Phe Asp Thr Asp Val
            180             185             190

Leu Lys Ala Asp Gly Ala Ile Leu Val Asp Phe Trp Ala His Trp Cys
        195             200             205

Gly Pro Cys Lys Met Ile Ala Pro Ile Leu Asp Glu Ile Ala Asp Glu
    210             215             220

Tyr Gln Gly Lys Leu Thr Val Ala Lys Leu Asn Ile Asp His Asn Pro
225             230             235             240

Gly Thr Ala Pro Lys Tyr Gly Ile Arg Gly Ile Pro Thr Leu Leu Leu
            245             250             255

Phe Lys Asn Gly Glu Val Ala Ala Thr Lys Val Gly Ala Leu Ser Lys
            260             265             270

Gly Gln Leu Lys Glu Phe Leu Asp Ala Asn Leu Ala Gly Ser Glu Phe
        275             280             285

Arg Ser His His His His His His
    290             295
```

<210> 279
<211> 296
<212> PRT
<213> Artificial sequence

<220>
<223> Amino acid sequence of PolB-16_OarG-intein construct IntC(C96A, C150A)-Trx-H6

<400> 279

```
Met Gln Glu Ala Lys Ile Asp Ile Lys Ser Leu Tyr Asp Ser Leu Ala
1               5               10              15

Lys Lys Tyr Asp Val Gln His Lys Asn Ser Tyr Glu Val Ile Tyr Pro
        20              25              30
```

```
Lys Gly Tyr Glu Ile Lys Val Leu Gly Asn Lys Tyr Val Lys Leu Val
        35              40              45

Ala Met Ser Arg His Lys Thr Gln Lys His Leu Val Lys Ile Val Val
        50              55              60

Lys Ser Glu Lys Thr Ile Asp Ser Leu Asp Pro Ile Arg Gln Lys Ser
65              70              75              80

Leu Leu Lys Lys Gln Asp Glu Val Val Val Thr Thr Asp His Ile Ala
            85              90              95

Met Val Tyr Asn Asp Asp His Phe Phe Glu Asn Val Asn Ala Lys Asn
            100             105             110

Leu Lys Val Gly Asn Tyr Val Ser Val Tyr Asp Glu Ala Ser Asp Lys
        115             120             125

Glu Val Ile Gly Glu Ile Ala Ser Ile Glu Asp Leu Gly Met Thr Asp
        130             135             140

Asp Tyr Val Tyr Asp Ala Glu Val Asp Asp Asp Ser His Ala Phe Tyr
145             150             155             160

Ala Ser Asn Ile Leu Val His Asn Ser Gln Phe Cys Asn Gly Thr Gly
            165             170             175

Ser Asp Lys Ile Ile His Leu Thr Asp Asp Ser Phe Asp Thr Asp Val
        180             185             190

Leu Lys Ala Asp Gly Ala Ile Leu Val Asp Phe Trp Ala His Trp Cys
        195             200             205

Gly Pro Cys Lys Met Ile Ala Pro Ile Leu Asp Glu Ile Ala Asp Glu
    210             215             220

Tyr Gln Gly Lys Leu Thr Val Ala Lys Leu Asn Ile Asp His Asn Pro
225             230             235             240

Gly Thr Ala Pro Lys Tyr Gly Ile Arg Gly Ile Pro Thr Leu Leu Leu
            245             250             255

Phe Lys Asn Gly Glu Val Ala Ala Thr Lys Val Gly Ala Leu Ser Lys
            260             265             270

Gly Gln Leu Lys Glu Phe Leu Asp Ala Asn Leu Ala Gly Ser Glu Phe
```

401

                    275                    280                    285


        Arg Ser His His His His His His
            290                    295


        <210>  280
        <211>  6
        <212>  PRT
        <213>  Artificial sequence

        <220>
        <223>  Cys tag

        <400>  280

        Met Gly Cys Asp Thr Asp
        1                   5


        <210>  281
        <211>  14
        <212>  PRT
        <213>  Artificial sequence

        <220>
        <223>  Cys tag

        <400>  281

        Ser Val Tyr Ala Ser Pro Ala Ala Pro Ala Pro Ala Ser Cys
        1                   5                       10


        <210>  282
        <211>  7
        <212>  PRT
        <213>  Artificial sequence

        <220>
        <223>  linker sequence

        <400>  282

        Met Gly Ser Gly Gly Ser Gly
        1                   5


        <210>  283
        <211>  5
        <212>  PRT
        <213>  Artificial sequence

        <220>
        <223>  flanking amino acid sequence

        <400>  283

        Tyr Ile Asp Thr Asp
        1                   5

```
<210>  284
<211>  5
<212>  PRT
<213>  Artificial sequence

<220>
<223>  flanking amino acid sequence

<400>  284

Ser Val Tyr Leu Asn
1                   5


<210>  285
<211>  4
<212>  PRT
<213>  Artificial sequence

<220>
<223>  flanking amino acid sequence

<400>  285

Ile Asp Thr Asp
1


<210>  286
<211>  4
<212>  PRT
<213>  Artificial sequence

<220>
<223>  flanking amino acid sequence

<400>  286

Ser Val Tyr Leu
1
```

**Description**

**FIELD OF THE INVENTION**

The present invention relates to an isolated polypeptide or to a composition of isolated polypeptides comprising (a) at least one first intein amino acid sequence (a1) and at least one first extein amino acid sequence (a2); wherein the at least one first intein amino acid sequence is an N-terminal sequence of a split intein or fragment thereof; wherein the at least one first extein sequence is fused to the N-terminus of the at least one first intein sequence and has a length of at least 1; and (b) at least one second intein amino acid sequence (b1) and at least one second extein sequence (b2); wherein the at least one second intein sequence is a C-terminal sequence of a split intein or fragment thereof; wherein the at least one second extein sequence is fused to the C-terminus of the at least one second intein sequence and has a length of at least 1; wherein the catalytic motifs of the at least one first intein amino acid sequence and/or of the at least one second intein amino acid sequence are free of cysteine residues; wherein the first and second intein amino acid sequence specifically interact with each other. Additionally, the invention relates to at least one isolated nucleic acid molecule comprising at least one nucleotide sequence encoding an isolated polypeptide according to the invention or a homolog, variant or complement thereof. Finally, the invention pertains to a method for modifying amino acid sequences or to a use of said isolated polypeptide(s) or said at least one isolated nucleic acid molecule.

**BACKGROUND OF THE INVENTION**

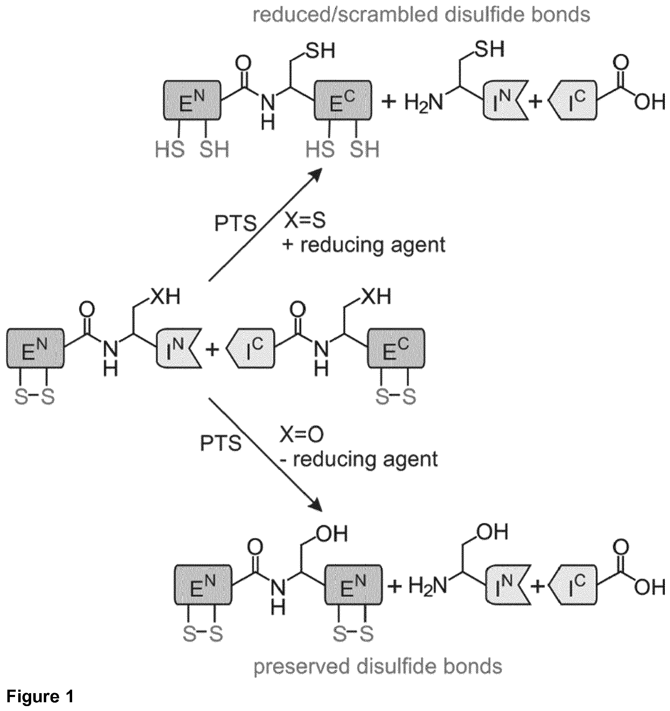

Inteins are intervening protein sequences that auto-catalyze a protein splicing post-translational reaction, whereby the intein excises itself out of the precursor polypeptide and ligates the two flanking N- and C-terminal fragments (exteins also referred to as $Ex^N$ and $Ex^C$) via a native peptide bond (1-4). A variety of applications for protein modification and purification have been developed based on this unique protein backbone alteration and its mechanism, involving peptide bond rearrangements in labile (thio)esters and cleavage reactions (5-8). Some naturally occurring inteins exist in a split form, also referred to as trans-inteins, where the two intein fragments (also referred to as N- and C-terminal intein fragments or $Int^N$ and $Int^C$) are expressed as two separate proteins, which first associate and then carry out protein trans-splicing (Figure 1) (9, 10). Artificially split inteins can be engineered from contiguous inteins (or cis-inteins) (11, 12). Protein trans-splicing is of particular interest to facilitate the reconstitution of a protein of interest (POI) from two fragments or to modify a POI by transfer of a synthetic, chemically or isotopically labeled part. In both these cases, the split intein precursor proteins can be generated and modified individually before being combined to carry out the protein trans-splicing reaction.

A major limitation of established split inteins is their inclusion of at least one mechanistically critical cysteine residue at the positions 1 or +1 at the N-terminal or the C-terminal splice junctions, respectively, that needs to be in a reduced state for the splicing reaction to take place. Intein precursors must thus be generated in a reducing environment or be treated with reducing agents. Partially oxidized cysteines at the splice junctions result in a proportional loss of the ligation yield. Some inteins have even evolved a redox-switch consisting of a disulfide bond of one of these critical cysteines with another cysteine to hold the intein in an inactive form (13, 14). The required in vitro addition of reducing agents like DTT or TCEP, however, has detrimental effects on intein-fused POIs that are sensitive to reduction, for example by breaking structurally critical disulfide bonds, such as in antibodies (15-17). Moreover, such cysteine-dependent inteins will at best be only partially active in oxidizing environments like the endoplasmic reticulum, bacterial periplasmic space or the in vivo extracellular milieu (18-20). Collateral damage by the reducing agents will also occur on other proteins when performing the trans-splicing reaction on an extracellular domain of a cell surface protein in a whole cell context (21, 22). Virtually all characterized inteins face this limitation, including the most widely used split Npu DnaE intein and its engineered variants (17, 23, 24), as well as the split Gp41-1 intein (25, 26), the fastest splicing intein known to date. Cysteine-less split inteins with suitable splicing properties at ambient temperatures have not been described so far. In general, inteins employing only serine or threonine and no cysteine residues at the catalytic 1 and +1 positions are very rare (27, 28). As cysteine residues may also be present in other conserved motifs or unconserved regions of inteins, completely cysteine-less inteins are very rare and naturally split inteins are even rarer, with the Neq Pol and the Psp-GBD Pol inteins being the only two reported cases of cysteine-less split inteins. These are, however, not suitable as general tools for protein manipulation owing to their extremely poor splicing properties under native and ambient conditions.

The naturally split Neq Pol intein derived from the hyperthermophile Nanoarchaeum equitans splices only at temperatures above 50 °C, with an optimum at 70 °C, prohibiting applications with less thermostable proteins and mammalian cells (29). The Psp-GBD Pol intein was artificially split in its internal homing endonuclease domain and the only cysteine, located in the homing endonuclease domain, was removed by mutation (30, 31). However, this intein requires a denaturation step to induce assembly of the two intein fragments, which significantly restricts its scope of application to proteins that can be refolded and prohibits its use on living cells. Notably, also native chemical ligation (NCL) requires reducing conditions (32). This reaction can be regarded as the chemical equivalent of protein *trans*-splicing to link two polypeptide chains and has been extensively used in biotechnological applications. Together, these considerations underline the challenge and the urgent need to identify a cysteine-less split intein that overcomes or mitigates the drawbacks of known split inteins.

Despite tremendous developments in recent years in the field of protein chemistry (33, 34), classical cysteine bioconjugation still is the unsurpassed technique for simple, efficient and chemoselective covalent protein modification. Cysteine is a much less frequently occurring amino acid compared to lysine and the thiol side chain displays a unique reactivity towards reagents with maleimides and iodoacetamides as the preferred functional groups (35). Cysteine bioconjugation is also highly attractive because of the much larger variety of commercially available and affordable reagents compared to other technologies like modern bioorthogonal click reactions. However, in general it is difficult or impossible to restrict cysteine bioconjugation to a desired cysteine when multiple cysteines are present in the target protein. Previous attempts to circumvent this regioselectivity problem included introducing an additional unpaired cysteine into proteins that contain only disulfide-paired cysteines, such as antibodies, antibody fragments and single-domain antibodies, also referred to as nanobodies (36, 37). However, these strategies cannot fully prevent the formation of scrambled disulfides and the resulting loss of sample homogeneity (38, 39), which can promote aggregation as well as the subsequent partial labeling of undesired cysteines. They typically necessitate an optimized mild reduction step as well as an additional purification step to remove the undesired mixed disulfide by-products (36). The so-called CysTag approach to achieve (regioselective) modification of a single cysteine in the presence of additional cysteines in the POI has been reported previously. The CysTag approach exploits the assembly of the POI with a short, pre-labeled cysteine tag (CysTag) using protein *trans*-splicing and thereby circumvents the exposure of the cysteines in the POI to the thiol-reactive reagent (16, 40-42). However, as outlined above, previously used split inteins require reducing conditions for *trans*-splicing, precluding the CysTag protocol with currently available split inteins for disulfide containing proteins and other experimental challenges that require the avoidance of reducing agents.

Hence, new methods that enable protein reconstitution from two fragments and preferably protein chemical labeling would be of great utility.

## SUMMARY OF THE INVENTION

The inventors have found/designed novel split inteins with catalytic motifs that are free of cysteine residues and which are redox-insensitive. These inteins (e.g. Aes intein) and certain engineered intein variants, as described herein, are capable of (trans-)splicing at ambient temperatures and without the requirement of any chemical reducing step or denaturation step.

In a first aspect, the invention relates to an isolated polypeptide comprising

(a) at least one first intein amino acid sequence (a1) and at least one first extein amino acid sequence (a2); wherein the at least one first intein amino acid sequence is an N-terminal sequence of a split intein or fragment thereof; wherein the at least one first extein sequence is fused to the N-terminus of the at least one first intein sequence and has a length of at least 1, preferably at least 5 amino acids; and

(b) at least one second intein amino acid sequence (b1) and at least one second extein sequence (b2); wherein the at least one second intein sequence is a C-terminal sequence of a split intein or fragment thereof; wherein the at least one second extein sequence is fused to the C-terminus of the at least one second intein sequence and has a length of at least 1, preferably at least 5 amino acids;

wherein the catalytic motifs of the at least one first intein amino acid sequence and/or of the at least one second intein amino acid sequence are free of cysteine residues and wherein the first N-terminal amino acid of the second extein sequence (+1 position) is not a cysteine residue;

wherein the first and second intein amino acid sequence interact with each other to catalyze the cleavage of the peptide bonds between the C-terminal amino acid of the first extein sequence and the N-terminal amino acid of the first intein sequence and the N-terminal amino acid of the second extein sequence and the C-terminal amino acid of the second intein sequence and to ligate the C-terminal amino acid of the first extein sequence to the N-terminal amino acid of the second extein sequence by a peptide bond.

In a second aspect, the invention relates to a composition comprising

(a) a first isolated polypeptide comprising at least one first intein amino acid sequence (a1) and at least one first extein amino acid sequence (a2); wherein the at least one first intein amino acid sequence is an N-terminal sequence of a split intein or fragment thereof; wherein the at least one first extein sequence is fused to the N-terminus of the at least one first intein sequence and has a length of at least 1, preferably at least 5 amino acids; and

(b) a second isolated polypeptide comprising at least one second intein amino acid sequence (b1), wherein the at least one second intein sequence is a C-terminal sequence of a split intein or fragment thereof and at least one second extein sequence (b2), wherein the at least one second extein sequence is fused to the C-terminus of the at least one second intein sequence and has a length of at least 1, preferably at least 5 amino acids;

wherein the catalytic motifs of the at least one first intein amino acid sequence and/or of the at least one second intein amino acid sequence are free of cysteine residues and wherein the first N-terminal amino acid of the second extein sequence is not a cysteine residue;

wherein the first and second intein amino acid sequence interact with each other to catalyze the cleavage of the peptide bonds between the C-terminal amino acid of the first extein sequence and the N-terminal amino acid of the first intein sequence and the N-terminal amino acid of the second extein sequence and the C-terminal amino acid of the second intein sequence and to ligate the C-terminal amino acid of the first extein sequence to the N-terminal amino acid of the second extein sequence by a peptide bond.

In various non-limiting embodiments, the catalytic motif(s) of the at least one first intein amino acid sequence comprise(s) the amino acid sequence (A)

$$X_1X_2X_3X_4X_5X_6X_7X_8X_9X_{10}X_{11} \qquad (A)$$

wherein

$X_1$ is S or T, most preferably S;

$X_2$ is V, C, F, Q, I, T, S, L, G or A, preferably V, C, F, Q, I or T, more preferably V, F, Q, I or T, most preferably V;

$X_3$ is V, T, L, S, I, N, D, A, Q, R, H, F, E, Y, C, K or P, preferably V, D, S, T, L or A, more preferably V, D or S, most preferably V or D;

$X_4$ is any naturally occuring amino acid or not present, preferably G, A, W, E, S, T, K, N, F, H, Y, R, C, Q, M or not present, more preferably G, A, W, S, F, M or K, more preferably G, A, S, F or K, most preferably G;

$X_5$ is D, S, K, N, L, E, C, T, A, Q or I, preferably D, S, K, N, Q or L, most preferably D or N;

$X_6$ is T, S, V, A, C, M or E, preferably T, S, E or C, more preferably T or S, most preferably T;

$X_7$ is I, Q, S, K, E, T, L, V, H, C, F, A, M, N, Y or P, preferably I, L, Q, K or P, more preferably I, L, Q or K, most preferably I or L;

$X_8$ is any naturally occuring amino acid or not present, preferably I, V, F, L, N, M or not present, more preferably I, V, F, L or M, more preferably I, V, F or L, most preferably I or L;

$X_9$ is D, N, V, R, F, Y, Q, E, K, C, S, I, M, H, W or L, preferably D, Y, K, R, N, L or V, most preferably D or Y;

$X_{10}$ is any naturally occuring amino acid or not present, preferably V, I, L, Y, T, A, C, W or not present, more preferably V, I, L, T, C or W, more preferably V, I, L or T, more preferably V, I or T, most preferably V or I; and

$X_{11}$ is S, N, D, R, K, E, T, M, G, I or H, preferably S, N, D, R, K, E or H, preferably S, N, D, R or K, more preferably S, N or D, most preferably S or N;

wherein the amino acid sequence (A) comprises or consists of at least 8 amino acids ($X_1$-$X_3$, $X_5$-$X_7$, $X_9$, $X_{11}$), preferably at least 9 amino acids ($X_1$-$X_3$, $X_5$-$X_7$, $X_9$-$X_{11}$) or 10 amino acids ($X_1$-$X_3$, $X_5$-$X_{11}$ or $X_1$-$X_9$, $X_{11}$), most preferably it comprises or consists of 11 amino acids ($X_1$-$X_{11}$).

In various other non-limiting embodiments, the catalytic motif(s) of the at least one first intein amino acid sequence comprise(s) the amino acid sequence (A')

$$X_1X_2X_3X_4X_5X_6X_7X_8X_9X_{10}X_{11} \qquad (A')$$

wherein

$X_1$ is S;

$X_2$ is V, F, I or L, preferably V, F or I, most preferably V;

$X_3$ is G, A, T, M, L, E, S, V, D, N, I or K, preferably A, T, M, L or V, most preferably T;

$X_4$ is K, G, A, P, S, E, Y, H, D or N, preferably K, G, A, P, Y or H, most preferably G;

$X_5$ is D, S, Y, E, G, N, A, T or C, preferably D, S, Y, E or C, more preferably D, S, Y or E, most preferably D;

$X_6$ is T, S, E or R, preferably T, S or R, more preferably T or S, most preferably T;

$X_7$ is E, I, V, P, Q, L, A, R, D, C, F, W or M, preferably I, V, P, Q, L, C, W or M, more preferably I, V, P, Q, L, W or M, more preferably I, V, P or L, most preferably P;

$X_8$ is I, V, L or M, preferably I, V or L, most preferably I;

$X_9$ is V, A, S, I, M, L, F, Y, T or R, preferably V, I, M, L, F, Y or T, more preferably V, I, M, L or Y, most preferably V, I or L;

$X_{10}$ is M, V, T, I, L, A or Y, preferably M, V, T, I or L, more preferably V, I or L, most preferably V; and $X_{11}$ is any

naturally occuring amino acid, preferably N, R, K, Y, S, G, D, E, L or not present, preferably R, K, Y, D or L, more preferably R or K, most preferably R;
wherein the amino acid sequence (A') comprises or consists of at least 10 amino acids ($X_1$-$X_{10}$), preferably it comprises or consists of 11 amino acids ($X_1$-$X_{11}$).

Preferably, the amino acid sequence (A) or (A') forms the N-terminus of the first intein amino acid sequence. In various embodiments, the at least one first intein sequence comprises one catalytic motif comprising the amino acid sequence (A) or (A').

In various non-limiting embodiments, the catalytic motif(s) of the at least one second intein amino acid sequence comprise(s) the amino acid sequence (B1)

$$X_{45}X_{46}X_{47}X_{48}X_{49}X_{50}X_{51}X_{52}X_{53}X_{54}X_{55}X_{56}X_{57} \qquad \text{(B1)}$$

wherein

$X_{45}$ is N, C, Y, T, D, P, S, H, A, G, M or R, preferably N, C, T, S, or H, more preferably N, T or S, most preferably T or N;
$X_{46}$ is H, P, S or F, preferably H, F or P, most preferably H;
$X_{47}$ is N, T, C, Y, M, V, A, S or H, preferably N, T, Y, M, A or H, most preferably N or T;
$X_{48}$ is F or A, preferably F;
$X_{49}$ is F, V, Y, I or G, preferably F, V, Y or I, most preferably F;
$X_{50}$ is G, A, C or I, preferably G or A;
$X_{51}$ is N, S, D or G, preferably N or G, most preferably N;
$X_{52}$ is D, G, N, E, T, S or R, preferably D, N, G or R, more preferably D, N or G, more preferably D or N, most preferably D;
$X_{53}$ is I, V, M or T, preferably I, M or V, more preferably I or V, most preferably I;
$X_{54}$ is L, C, Y, I or V, preferably L;
$X_{55}$ is V, A, I or L, preferably V;
$X_{56}$ is H; and
$X_{57}$ is N;
wherein the amino acid sequence (B1) comprises or consists of at least 13 amino acids ($X_{45}$-$X_{57}$).

In various other non-limiting embodiments, the catalytic motif(s) of the at least one second intein amino acid sequence comprise(s) the amino acid sequence (B1')

$$X_{43}X_{44}X_{45}X_{46}X_{47}X_{48}X_{49}X_{50}X_{51}X_{52}X_{53}X_{54} \qquad \text{(B1')}$$

wherein

$X_{43}$ is any naturally occuring amino acid or not present, preferably R, C, F, V, L, P, H, N or not present, more preferably C, F, V or H, more preferably F, V or H, most preferably F;
$X_{44}$ is any naturally occuring amino acid or not present, preferably F, H, Q, V, I, S, A, N, Y, C, T, W or not present, more preferably F, H, V, A, N, C, T or W, more preferably F, H, V, N or A, most preferably F, H, A or V;
$X_{45}$ is F, Y, A, V, N, G, S, I, C or D, preferably F, A, N, C or D, most preferably F, A, N or D;
$X_{46}$ is any naturally occuring amino acid or not present, preferably G, A, R, F, M or not present, preferably G, A or F, most preferably G;
$X_{47}$ is A, V, I, P, L, S, F, G, K or C, preferably A, V, I, P, L or C, more preferably A, V, I, L or C, most preferably A, V, I or L;
$X_{48}$ is any naturally occuring amino acid or not present, preferably N, G, D or not present, most preferably N or G;
$X_{49}$ is N, D, R, G, E, L, M, S, P or Q, preferably N, D, G, E or M, most preferably N, G, E or M;
$X_{50}$ is V, I, M, L or N, preferably I, M or L, most preferably I or L;
$X_{51}$ is L, I, V, D or C, preferably L, I, V or C, most preferably L, I or V;
$X_{52}$ is V, I, N, L, C or A, preferably V, L or C, most preferably V or L;
$X_{53}$ is H, K, S or T, preferably H or K, most preferably H; and
$X_{54}$ is N or Q, preferably N;
wherein the amino acid sequence (B1') comprises or consists of at least 8 amino acids ($X_{45}$, $X_{49}$-$X_{54}$), preferably at least 10 amino acids ($X_{45}$-$X_{54}$) or at least 11 amino acids ($X_{43}$-$X_{45}$, $X_{47}$-$X_{54}$, or $X_{43}$-$X_{47}$, $X_{49}$-$X_{54}$), most preferably it comprises or consists of 12 amino acids ($X_{43}$-$X_{54}$).

Preferably, the amino acid sequence (B1) or (B1') forms the C-terminus of the second intein amino acid sequence. In various embodiments, the at least one second intein sequence comprises one catalytic motif comprising the amino acid sequence (B1) or (B1').

In various non-limiting embodiments, the catalytic motif(s) of the at least one second intein amino acid sequence comprise(s) the amino acid sequence (B2)

$$X_{28}X_{29}X_{30}X_{31}X_{32}X_{33}X_{34}X_{35}X_{36}X_{37} \qquad (B2)$$

wherein

$X_{28}$ is I, L, E, V, Q, D or N, preferably I, L, E, Q, D or N, more preferably I, E or D, most preferably I or E;
$X_{29}$ is D, Y, W, F, P, I or M, preferably D, Y or W, most preferably D or Y;
$X_{30}$ is V or A, preferably V;
$X_{31}$ is Y or F, preferably Y;
$X_{32}$ is D;
$X_{33}$ is I, V, L, C, A or M, preferably I, V, L or C, more preferably I, V or L, most preferably I;
$X_{34}$ is E, S, G or V, preferably E;
$X_{35}$ is any naturally occuring amino acid or not present, preferably V, M, I, T or not present, more preferably V or M, most preferably V;
$X_{36}$ is any naturally occuring amino acid or not present, preferably D, E, Q, P, K, F, A, N, G or not present, more preferably D or E, most preferably D; and
$X_{37}$ is any naturally occuring amino acid or not present, preferably G, E, D, K, T, N, S or not present, more preferably G, E, D or N, more preferably G, E or D, most preferably G or D;
wherein the amino acid sequence (B2) comprises or consists of at least 7 amino acids ($X_{28}$-$X_{34}$), preferably at least 8 amino acids ($X_{28}$-$X_{34}$, $X_{37}$ or $X_{28}$-$X_{35}$) or at least 9 amino acids ($X_{28}$-$X_{35}$, $X_{37}$ or $X_{28}$-X36), most preferably at least 10 amino acids ($X_{28}$-$X_{37}$).

In various other non-limiting embodiments, the catalytic motif(s) of the at least one second intein amino acid sequence comprise(s) the amino acid sequence (B2')

$$X_{27}X_{28}X_{29}X_{30}X_{31}X_{32}X_{33}X_{34}X_{35}X_{36}X_{37} \qquad (B2')$$

wherein

$X_{27}$ is D, E, Y, F, V, T, M or W, preferably Y, F, M or W, most preferably Y;
$X_{28}$ is V;
$X_{29}$ is Y;
$X_{30}$ is D;
$X_{31}$ is L, I, M or V, preferably L or I, most preferably L;
$X_{32}$ is E, T, S, Q, C or G, preferably E, T, S or C, more preferably E, T or S, most preferably E or S; $X_{33}$ is I, L, M, V, T, C or A, preferably I, L, M, V, T or C, more preferably I, L, M, V or T, most preferably VorT;
$X_{34}$ is any naturally occuring amino acid or not present, preferably E, P, D, G, S, A, Q, T, V, K, N, F, G, R or not present, more preferably E, D, A or K, most preferably E;
$X_{35}$ is any naturally occuring amino acid or not present, preferably V, D, S, Q, N, R, A, C, H, G, E, T or not present, more preferably D, S, N, C or E, more preferably D, S, N or E, most preferably D or N, $X_{36}$ is any naturally occuring amino acid or not present, preferably D, G, H, Q, N, E, T, S, A or not present, more preferably D, G, H, Q or N, more preferably G, H or N, most preferably G or H,
$X_{37}$ is any naturally occuring amino acid or not present, preferably T, D, S, H, V, E, R, Q, K, N, I or not present, more preferably T, D, H or E, most preferably T or H;
wherein the amino acid sequence (B2') comprises or consists of at least 7 amino acids ($X_{27}$-$X_{33}$), preferably at least 9 amino acids ($X_{27}$-$X_{35}$) or at least 10 amino acids ($X_{27}$-$X_{33}$, $X_{35}$-$X_{37}$ or $X_{27}$-$X_{35}$, $X_{37}$ or $X_{27}$-$X_{34}$, $X_{36}$-$X_{37}$), most preferably at least 11 amino acids ($X_{27}$-$X_{37}$).

In various embodiments, the at least one second intein amino acid sequence comprises a catalytic motif having both amino acid sequences (B1) and (B2) or (B1') and (B2'). In such embodiments, the amino acid sequence (B2) or (B2') is located N-terminally to the amino acid sequence (B1) or (B1') in the second intein amino acid sequence. In some embodiments, amino acids $X_{37}$ of (B2) and $X_{45}$ of (B1) or amino acids $X_{37}$ of (B2') and $X_{43}$ of (B1') are linked to each other by a peptide bond. In these embodiments the catalytic motif of the second intein sequence comprises (B2)-(B1) or (B2')-(B1') with the amino acids of the respective motifs being as defined above. In various other embodiments, B2 and B1 or B2' and B1' are linked as described above, but there is a linker amino acid sequence between the two motifs. This is as defined below and is, for example, up to 10 amino acids long, for example 5 or 7 amino acids long, with these amino acids being selected from any amino acid, as defined herein. In various embodiments, the second intein sequence comprises only one catalytic motif comprising one copy of the amino acid sequence (B1) and (B2) or (B1') and (B2').

In various non-limiting embodiments, the catalytic motif(s) of the first or the second intein amino acid sequence further comprise(s) the amino acid sequence (C)

$$X_{12}X_{13}X_{14}X_{15}X_{16}X_{17}X_{18}X_{19}X_{20}X_{21}X_{22}X_{23} \qquad (C)$$

wherein

$X_{12}$ is any naturally occuring amino acid or not present, preferably E, K, T, M, S, P, F, D, V, C, Q, A, Y or not present, more preferably E, C, S, V or P, more preferably E, S, V or P, most preferably E;

$X_{13}$ is V, L, T or I, most preferably V or I;

$X_{14}$ is I, T, E, V, L, F, K, D, S, Y, M or H, preferably I, T, E, V, Y or H, more preferably I, T, E or V, most preferably I;

$X_{15}$ is V, M, I, L, T, C or A, preferably V, T, C or I, more preferably V or I, most preferably V;

$X_{16}$ is T or S, preferably T;

$X_{17}$ is A, E, C, G, N, Q, D, T or S, preferably A, E, C, G, N or T, more preferably A, E, G, N or T, most preferably A, E or G;

$X_{18}$ is D, E or N, preferably D;

$X_{19}$ is H;

$X_{20}$ is S, C, G, I, V or N, preferably S, C, V or N, more preferably S or V, most preferably S;

$X_{21}$ is V, I, L, M, C, or F, preferably V, I, L, M or C, more preferably V, I, C or L, more preferably V, I or L, most preferably V;

$X_{22}$ is any naturally occuring amino acid or not present, preferably M, I, K, V, L or not present, more preferably M, I or V, most preferably M; and

$X_{23}$ is any naturally occuring amino acid or not present, preferably V, I, K, R, A, D or not present, more preferably, V, I, D, R or A, more preferably V, I or A, most preferably V,

wherein the amino acid sequence (C) comprises or consists of at least 9 amino acids ($X_{13}$-$X_{21}$), preferably at least 10 amino acids ($X_{12}$-$X_{21}$) or at least 11 amino acids ($X_{13}$-$X_{23}$), most preferably 12 amino acids ($X_{12}$-$X_{23}$).

In various other non-limiting embodiments, the catalytic motif(s) of the first or the second intein amino acid sequence further comprise(s) the amino acid sequence (C')

$$X_{12}X_{13}X_{14}X_{15}X_{16}X_{17}X_{18}X_{19}X_{20}X_{21}X_{22}X_{23} \qquad (C')$$

wherein

$X_{12}$ is Y, H, C, V, I, L, M, S, T, K, E, D, Q, W, R or A, preferably Y, C, I, L, K or E, more preferably Y, C or E, more preferably Y or E, most preferably E;

$X_{13}$ is I, V, C, T or S, preferably I, V, C or S, more preferably I, V or S, most preferably V;

$X_{14}$ is D, T, M, E, C, K, H, N, V, S, I or R, preferably D, T, E, C, H, V or I, more preferably D, T or H, more preferably D or T, most preferably D;

$X_{15}$ is V, C, A, I or T, preferably V, C or T, most preferably V;

$X_{16}$ is T;

$X_{17}$ is E, R, A, S, D, K, N, H, C, P or T, preferably E, R, D, H or C, more preferably E, R, D or H, more preferably E or R, most preferably E;

$X_{18}$ is D, N, Q or E, preferably D;

$X_{19}$ is H;

$X_{20}$ is S or R, preferably S;

$X_{21}$ is L, V, A, I or Y, preferably L, I or Y, most preferably L;

$X_{22}$ is I, L, F, V, M or Y, preferably I, L, F, V or M, most preferably I, L, F or V; and

$X_{23}$ is G, D, N, L, Q, K, R, Y, V, I, A, T, S or K, preferably G, D, N, V, I or T, more preferably D or V; wherein the amino acid sequence (C') comprises or consists of 12 amino acids ($X_{12}$-$X_{23}$).

The amino acid sequence (C) or (C'), if present in the first intein amino acid sequence, is located C-terminally to amino acid sequence (A) or (A'), or, if present in the second intein amino acid sequence, is located N-terminally to amino acid sequence (B2) or (B2'). In various embodiments only the first or the second intein sequence but not both comprise the amino acid sequence (C)/(C'). It is further preferred that the respective intein sequence comprises the motif (C) or (C') only once.

In various embodiments of the intein sequences of the invention (A) is paired with (B1), (B2) and (C) and (A') is paired with (B1'), (B2') and (C').

In various non-limiting embodiments, the amino acid sequence (A) or (A'), the amino acid sequence (B1) or (B1'), the amino acid sequence (B2) or (B2') and/or the amino acid sequence (C) or (C'), preferably at least two, more preferably at least three, most preferably all four amino acid sequences are free of cysteine residues.

In various embodiments, (i) the at least one first intein amino acid sequence, i.e. the complete sequence and not only the catalytic motifs as defined above, is free of cysteine residues, and/or (ii) the at least one second intein amino acid sequence, i.e. the complete sequence and not only the catalytic motifs as defined above, is free of cysteine residues. This may mean that the complete intein construct comprising the first and second intein sequence does not contain any cysteine residue. In various embodiments, this can be achieved by substituting naturally occurring cysteine residues by other amino acid residues.

In addition to the catalytic motifs of the first and/or second intein sequence being free of cysteine residues, the N-terminal amino acid of the second extein, i.e. the sequence fused to the C-terminus of the second intein sequence, is also not cysteine. In various embodiments, the second extein sequence comprises an N-terminal serine or threonine residue that is fused to the C-terminal amino acid of the second intein sequence by a peptide bond.

Furthermore, in various embodiments the first intein amino acid sequence and the second intein amino acid sequence are not derived from a thermophilic organism.

In various non-limiting embodiments, the first and second intein amino acid sequence interact with each other to catalyze the cleavage of the peptide bonds between the C-terminal amino acid of the first extein sequence and the N-terminal amino acid of the first intein sequence and the N-terminal amino acid of the second extein sequence and the C-terminal amino acid of the second intein sequence and to ligate the C-terminal amino acid of the first extein sequence to the N-terminal amino acid of the second extein sequence by a peptide bond

(a) at a temperature of ≤ 40 °C, preferably less than 40 °C, more preferably 0 to 37 °C, more preferably 8 °C or 37 °C; and/or
(b) under oxidizing reaction conditions, preferably under atmospheric oxygen.

The above-mentioned conditions mean that the intein functions under these conditions at least at about 60 % efficiency, preferably at least 80 % efficiency, relative to its efficiency under optimal conditions.

In various non-limiting embodiments

(i) said at least one first intein amino acid sequence has at least 70 %, at least 80 %, at least 85 %, at least 90 %, at least 95 %, at least 99 % or 100 % sequence identity with any one of the amino acid sequences set forth in SEQ ID Nos. 1, 3, 5, 7, 9, 11, 13, 15, 17, 19, 21, 23, 25, 27, 29, 31, 33, 35, 37, 39, 41, 43, 45, 48, 51, 53, 55, 58, 61, 63, 65, 67, 70, 74, 76, 78, 80, 82, 85, 87, 89, 92, 97, 100, 102, 106, 108, 111, 114, 117, 120, 122, 124, 126, 128, 130, 132, 134, 136, 138, 140, 142, 144, 146 and 147, preferably SEQ ID Nos. 1, 3, 5, 7, 9, 11, 13, 15, 17, 19, 21, 23, 25, 27, 29, 31, 33, 35, 37, 39, 41, 43 and 45, more preferably SEQ ID NO:1 or 3, more preferably SEQ ID NO:3; and/or

(ii) said at least one second intein amino acid sequence has at least 70 %, at least 80 %, at least 85 %, at least 90 %, at least 95 %, at least 99 % or 100 % sequence identity with any one of the amino acid sequences set forth in SEQ ID Nos. 2, 4, 6, 8, 10, 12, 14, 16, 18, 20, 22, 24, 26, 28, 30, 32, 34, 36, 38, 40, 42, 44, 46, 49, 52, 54, 56, 59, 62, 64, 66, 68, 71, 75, 77, 79, 81, 83, 86, 88, 90, 93, 98, 101, 103, 107, 109, 112, 115, 118, 121, 123, 125, 127, 129, 131, 133, 135, 137, 139, 141, 143, 145, 148, 149, 150, 151 and 152, preferably SEQ ID Nos. 2, 4, 6, 8, 10, 12, 14, 16, 18, 20, 22, 24, 26, 28, 30, 32, 34, 36, 38, 40, 42, 44 and 46, more preferably SEQ ID NO:2 or 4, more preferably SEQ ID NO:4; or

(iii) at least one intein amino acid sequence comprises the first intein amino acid sequence and the second intein amino acid sequence in one contiguous amino acid sequence; wherein the C-terminus of the first intein sequence is ligated to the N-terminus of the second intein sequence, and wherein the contiguous amino acid sequence has at least 70 %, at least 80 %, at least 85 %, at least 90 %, at least 95 %, at least 99 % or 100 % sequence identity with any one of the amino acid sequences set forth in SEQ ID Nos. 47, 50, 57, 60, 69, 72, 73, 84, 91, 94, 95, 96, 99, 104, 105, 110, 113, 116, 119, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182,183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 and 250.

Preferably, (a) said at least one first intein amino acid sequence has at least 70 %, at least 80 %, at least 85 %, at least 90 %, at least 95 %, at least 99 % or 100 % sequence identity with the amino acid sequence set forth in SEQ ID NO:1 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:2; or

(b) said at least one first intein amino acid sequence has at least 70 %, at least 80 %, at least 85 %, at least 90 %, at least 95 %, at least 99 % or 100 % sequence identity with the amino acid sequence set forth in SEQ ID NO:3 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:4; or

(c) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:5 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:6; or

(d) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:7 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:8; or

(e) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:9 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:10; or

(f) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:11 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:12; or

(g) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:13 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:14; or

(h) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:15 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:16; or

(i) wherein said at least one firstl intein amino acid sequence has at least 70 %, at least 80 %, at least 85 %, at least 90 %, at least 95 %, at least 99 % or 100 % sequence identity with the amino acid sequence set forth in SEQ ID NO:17 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:18; or

(j) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:19 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:20; or

(k) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:21 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:22; or

(I) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:23 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:24; or

(m) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:25 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:26; or

(n) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:27 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:28; or

(o) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:29 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:30; or

(p) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:31 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:32; or

(q) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:33 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:34; or

(r) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:35 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:36; or

(s) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:37 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:38; or

(t) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:39 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:40; or

(u) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:41 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:42; or

(v) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:43 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:44; or

(w) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:45 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:46.

Preferably,

(i) the at least one first intein amino acid sequence is at least 10 amino acids, more preferably at least 11 amino acids, more preferably at least 12 amino acids, more preferably 26 or 120 amino acids in length; and/or

(ii) the at least one second intein amino acid sequence is at least 6 amino acids, more preferably at least 24 amino acids, more preferably at least 30 amino acids, more preferably 39 or 129 amino acids in length; and/or

(iii) the split site between the N-terminal and C-terminal amino acid sequence of the split intein is between residues 10 to 200, more preferably between 11 to 200, more preferably between 12 to 200, more preferably between 25 to 200, more preferably after amino acid 10 or 11 or 12 or 26 or 120, preferably after amino acids 26 or 120 as calculated from the intein's N-terminal end.

In various non-limiting embodiments, the last amino acid at the C-terminal end of the first extein amino acid sequence is D,

preferably the last two amino acids at the C-terminal end of the first extein amino acid sequence are TD, more preferably the last three amino acids at the C-terminal end of the first extein amino acid sequence are DTD,

more preferably the last four amino acids at the C-terminal end of the first extein amino acid sequence are IDTD,

most preferably the last five amino acids at the C-terminal end of the first extein amino acid sequence are YIDTD; and/or

the first amino acid at the N-terminal end of the second extein amino acid sequence is S,

preferably the first two amino acids at the N-terminal end of the second extein amino acid sequence are SV,

more preferably the first three amino acids at the N-terminal end of the second extein amino acid sequence are SVY,

more preferably the first four amino acids at the N-terminal end of the second extein amino acid sequence are SVYL,

most preferably the first five amino acids at the N-terminal end of the second extein amino acid sequence are SVYLN.

In various non-limiting embodiments, the isolated polypeptide(s), preferably the first and/or second extein amino acid sequence(s) of the polypeptide(s), comprise(s) the amino acid sequence of a recombinant protein, an antibody, a nanobody, a protein hormone, or a fragment or homolog thereof. These sequences may be split, such that upon the intein mediated splicing the full sequences is obtained or may be completely contained in either the first or second extein sequence.

In various non-limiting embodiments, the isolated polypeptide(s), preferably the first and/or second extein amino acid sequence(s) of the polypeptide(s), further comprise(s) a cysteine tag (Cys tag), wherein the cysteine tag comprises a single cysteine residue in its amino acid sequence.

In various non-limiting embodiments, the isolated polypeptide(s), preferably the first and/or second extein amino acid sequence(s) of the isolated polypeptide(s), further comprise(s) at least one component selected from a solubility factor, a marker, a linker, an epitope, an affinity tag, a fluorophore or a fluorescent protein, a toxic compound or protein and a small-molecule or a small-molecule binding protein.

The first and second extein sequence may thus be used to label a given protein, such as those listed above, with a tag, such as those listed above.

Preferably, the isolated polypeptide(s) comprise(s) or consist(s) of an amino acid sequence having at least 70 %, at least 80 %, at least 85 %, at least 90 %, at least 95 %, at least 99 % or 100 % sequence identity with any one of the sequences set forth in SEQ ID Nos. 251 to 272 and 275 to 279.

In a third aspect, the invention relates to at least one isolated nucleic acid molecule comprising at least one nucleotide sequence encoding an isolated polypeptide according to the invention or a homolog, variant or complement thereof.

In a fourth aspect, the invention relates to a method for modifying amino acid sequences, preferably ligating two amino acid sequences to each other, using an isolated polypeptide according to the invention or a composition according to the invention or at least one isolated nucleic acid molecule according to the invention, wherein the modification is preferably selected from the group consisting of modification of a protein, protein lipidation, protein immobilization, protein backbone ligation, protein backbone semi-synthesis, protein or peptide cyclization, protein modification with a chemically modified tag sequence, preferably with a cysteine tag, and artificial control of protein splicing by light.

Preferably, in the method according to the invention

(i) the isolated polypeptide or the composition according to the invention is exposed to oxidizing conditions, preferably to atmospheric oxygen, for example in that the method is performed under oxidizing coditions or in the presence of atmospheric oxygen; and/or

(ii) the isolated polypeptide or the composition according to the invention is exposed to a temperature of ≤ 40 °C, preferably less than 40 °C, more preferably 0 to 37 °C, more preferably 8 °C or 37 °C, for example in that the method is carried out at these temperatures; and/or

(iii) the method is essentially free of a reducing step or a reducing agent; and/or

(iv) the method is free of a denaturation step.

In various non-limiting embodiments, the method comprises the step(s) of

(i) protein preparation, preferably recombinant protein expression or *in vitro* translation; and/or
(ii) protein purification; and/or
(iii) dialysis of produced/purified protein.

In a fifth aspect, the invention relates to a use of an isolated polypeptide according to the invention or a composition according to the invention or at least one nucleic acid molecule according to the invention, wherein the polypeptide or the composition or the nucleic acid molecule is preferably used for modification of a protein, protein lipidation, protein immobilization, protein backbone ligation, protein backbone semi-synthesis, protein or peptide cyclization, protein modification with a (chemically modified) tag sequence, preferably with a cysteine tag, and as molecular switch.

**BRIEF DESCRIPTION OF FIGURES**

The invention will be better understood with reference to the detailed description when considered in conjunction with the non-limiting examples and the accompanying figures.

**Figure 1. Protein *trans*-splicing (PTS) using a redox-insensitive intein.** Schematic representation of a *trans*-splicing reaction. Inteins containing at least one catalytically active cysteine residue at the upstream or downstream splice junction (X= S) require the presence of reducing agents for quantitative *trans*-splicing to occur. In contrast, a cysteine-free intein can carry out *trans*-splicing in the absence of any reducing agent, thus preserving disulfide bonds and avoiding other potentially deleterious effects.

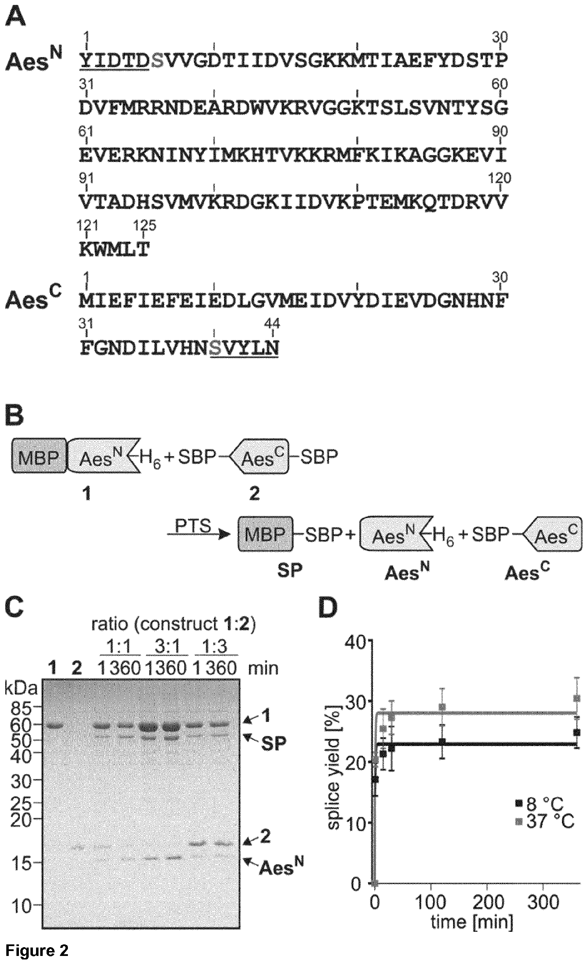

**Figure 2. Sequence and biochemical characterization of the split Aes123 PolB1 intein.** (A) Amino acid sequences of the $Aes^N$ and $Aes^C$ split intein fragments, each with five residues of extein sequence (underlined). The active serine residues are shown as blue characters. (B) Scheme of the constructs used for protein *trans*-splicing. (C) Analysis of the protein *trans*-splicing reaction carried out at 37 °C using different molar ratios of the precursors (either 10 $\mu$M or 30 $\mu$M). Shown is a representative Coomassie-stained SDS-PAGE gel. (D) Time course of the splicing reactions carried out at 37 °C and 8 °C under pseudo-unimolecular conditions (**1**: 10 $\mu$M and **2**: 30 $\mu$M). The data points represent averages and standard deviations obtained from three experiments. SP = splice product. Calculated molecular weights: **1**: 58.2 kDa, **2**: 14.6 kDa, **SP**: 48.6 kDa, $Aes^N$: 14.7 kDa, $Aes^C$: 9.6 kDa.

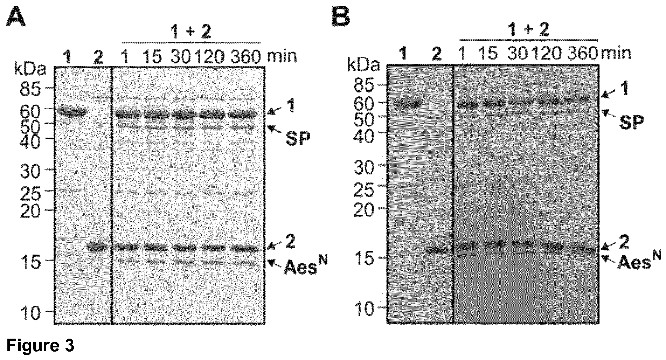

**Figure 3. Splicing kinetics of the Aes123 PolB1 intein at different temperatures.** Representative gels showing PAGE analysis of the time-course of splicing at 8 °C (A) and 37 °C (B). The precursor proteins and splicing products are the same as shown in Figure 2B. Calculated molecular weights: **1**: 58.2 kDa, **2**: 14.6 kDa, **SP**: 48.6 kDa, $Aes^N$: 14.7 kDa, $Aes^C$: 9.6 kDa.

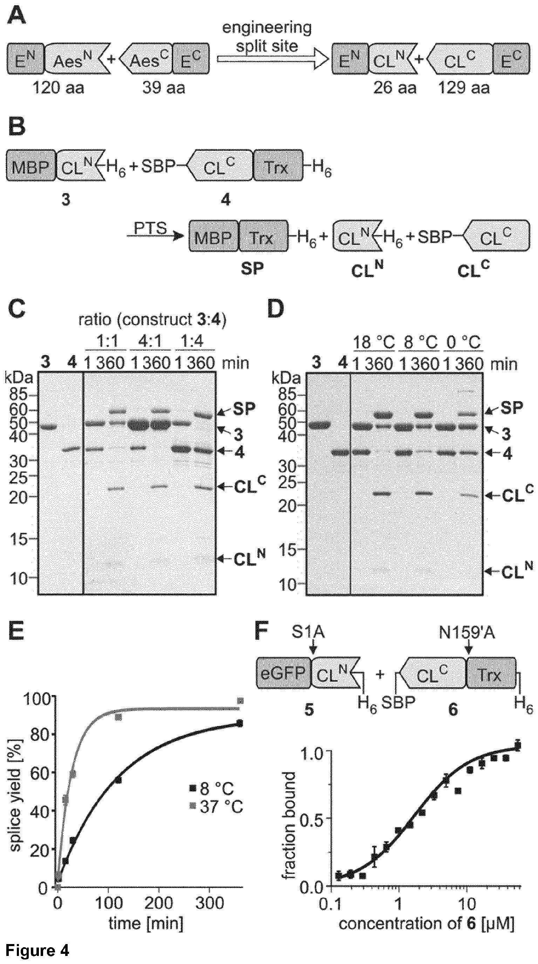

**Figure 4. N-terminal labeling of target proteins using the CL intein under oxidizing conditions.** (A) Reaction scheme. (B) SDS-PAGE analysis of the labeling and purification steps for the generation of Alexa647-labeled $V_HH^{GFP}$ nanobody (**SP**). FT and E represent the flow through and elution fractions from Streptactin resin, respectively. The arrows mark the purified splice product (**SP**) that was used for further experiments. (C) Confocal microscopy images of HeLa cells transfected with HA-eGFP-TMD-mCherry (HA= hemagglutinin A tag, TMD= transmembrane domain of the PDGF receptor) that were treated with 1 nM of Alexa647-labeled $V_HH^{GFP}$ nanobody (**SP**). Scale bars represent 20 $\mu$m. (D) Sequence of the fluorescein labeled $CL^N$ peptide (**9**) synthesized via SPPS. M15 was replaced with norleucine (NorL). (E) Mass spectrometric analysis of the purified peptide **9**. M(calc) 3736.69 Da, M(obs) 3736.03 Da. (F) Time-course of the *trans*-splicing reaction between **9** (40 $\mu$M) and **4** (10 $\mu$M). SP = splice product. Calculated molecular weights: **7**: 9.6 kDa, **7a**: 10.5 kDa, **8**: 35.0 kDa, **SP**: 16.4 kDa, $CL^N$: 8.9 kDa, $CL^C$: 19.8 kDa.

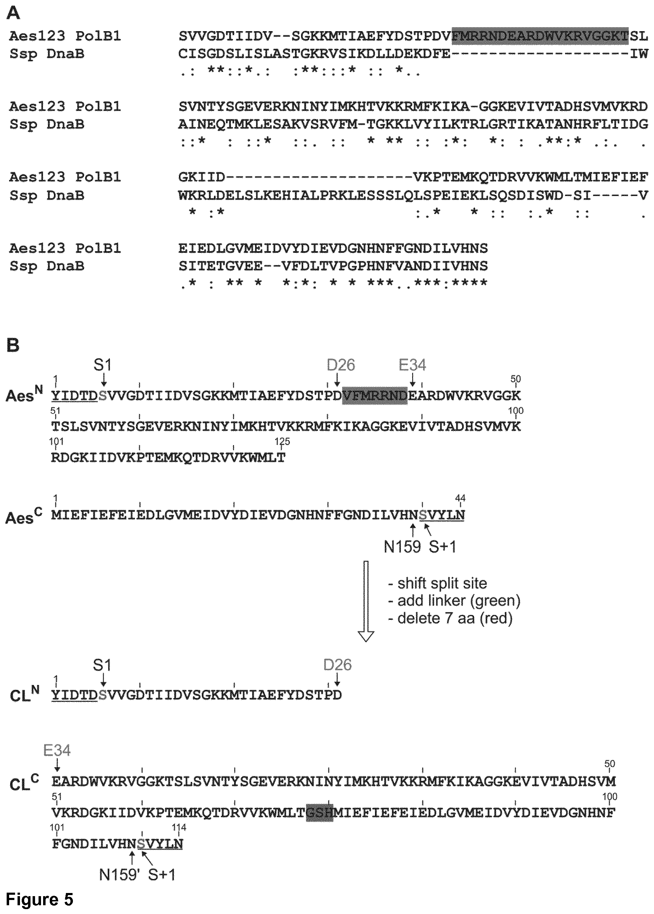

**Figure 5. Engineering the CL intein.** (A) Sequence alignment of the concatenated $Aes^N$ and $Aes^C$ sequences with the sequence of the Ssp DnaB intein showing the presence of an extra stretch of residues from Phe28 to Thr46 (highlighted). (B) Sequences of the fragments corresponding to the original Aes123 PolB1 intein and the engineered CL-intein, together with the steps carried out to generate the engineered intein. The catalytic serines are shown as shaded residues, while the native extein residues are underlined. The residues deleted and inserted are highlighted . The catalytic residues that were mutated in different experiments are denoted by arrows and black alphabets, while the residues where the original intein was artificially split are denoted by arrows and red alphabets. The last residue of the CL intein is still denoted as N159'.

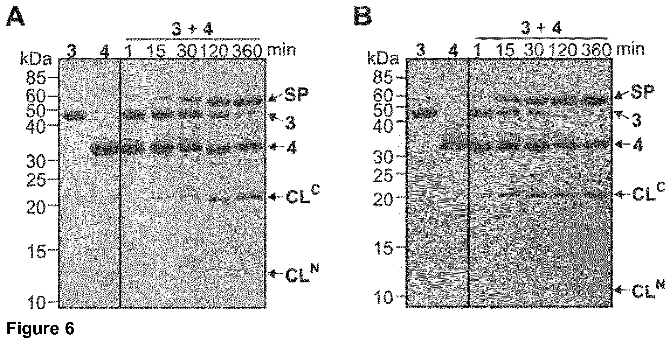

**Figure 6. Splicing kinetics of the CL intein at different temperatures.** Representative gels showing PAGE analysis of the time-course of splicing at 8 °C (A) and 37 °C (B). The precursor proteins and splicing products are the same as shown in Figure 4B. Calculated molecular weights: **3**: 47.3 kDa, **4**: 33.8 kDa, **SP**: 57.5 kDa, **CL$^N$**: 3.8 kDa, **CL$^C$**: 19.8 kDa.

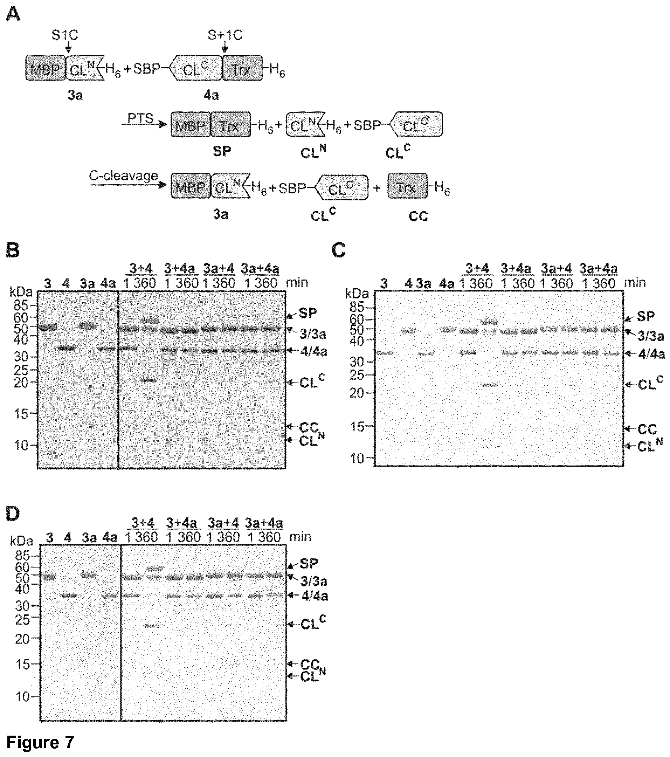

**Figure 7. Splicing activity of the CL intein with cysteines at key catalytic positions.** (A) Schematic representation of the fusion proteins. The Ser1 and Ser+1 residues were mutated into Cys. (B-D) PAGE analysis of splicing reactions carried out with the non-mutated and mutated fragments of the CL-intein in different combinations either in the absence of any reducing agents (B), with 1 mM TCEP (C), or with 50 mM DTT (D). 10 $\mu$M of each precursor protein were used. The splice product (**SP**) is only formed when both fragments do not contain Cys (but contain Ser) as catalytic residues. Calculated molecular weights: **3/3a**: 47.3 kDa, **4/4a**: 33.8 kDa, **SP**: 57.5 kDa, **CL$^N$**: 3.8 kDa, **CL$^C$**: 19.8 kDa, **CC**: 13.9 kDa.

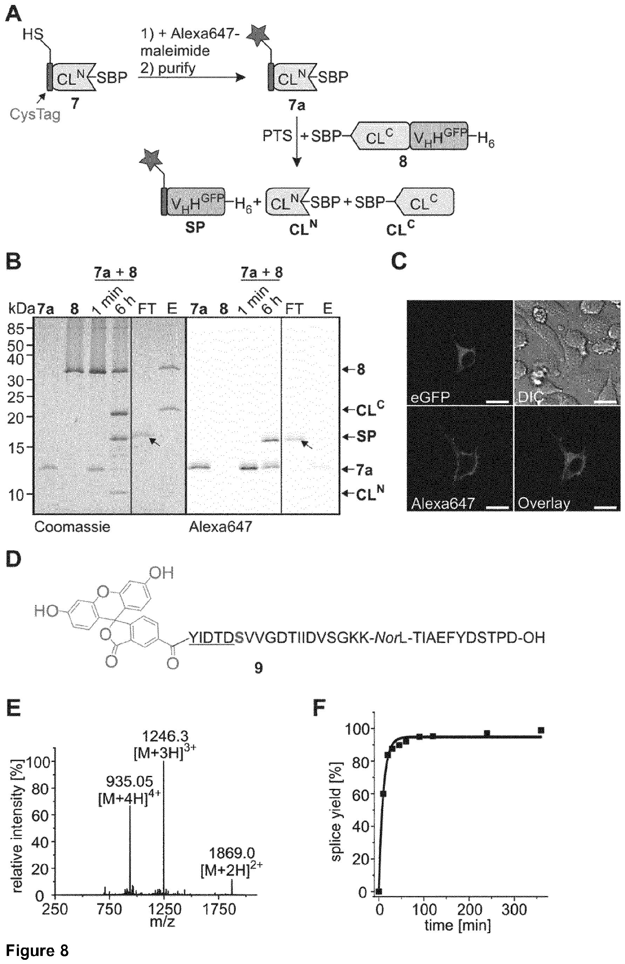

**Figure 8. N-terminal labeling of target proteins using the CL intein under oxidizing conditions.** (A) Reaction scheme. (B) SDS-PAGE analysis of the labeling and purification steps for the generation of Alexa647-labeled V$_H$H$^{GFP}$ nanobody (**SP**). FT and E represent the flow through and elution fractions from Streptactin resin, respectively. The arrows mark the purified splice product (**SP**) that was used for further experiments. (C) Confocal microscopy images of HeLa cells transfected with HA-eGFP-TMD-mCherry (HA= hemagglutinin A tag, TMD= transmembrane domain of the PDGF receptor) that were treated with 1 nM of Alexa647-labeled V$_H$H$^{GFP}$ nanobody (**SP**). Scale bars represent 20 $\mu$m. (D) Sequence of the fluorescein labeled CL$^N$ peptide (**9**) synthesized via SPPS. M15 was replaced with nor-leucine (NorL). (E) Mass spectrometric analysis of the purified peptide **9**. M(calc) 3736.69 Da, M(obs) 3736.03 Da. (F) Time-course of the *trans*-splicing reaction between **9** (40 $\mu$M) and **4** (10 $\mu$M). SP = splice product. Calculated molecular weights: **7**: 9.6 kDa, **7a**: 10.5 kDa, **8**: 35.0 kDa, **SP**: 16.4 kDa, **CL$^N$**: 8.9 kDa, **CL$^C$**: 19.8 kDa.

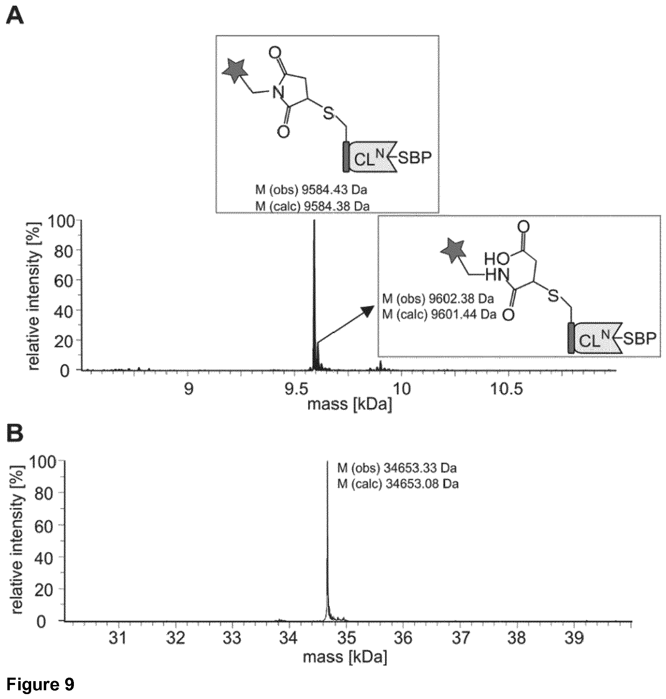

**Figure 9. ESI-MS analysis of proteins used for N-terminal labeling of V$_H$H$^{GFP}$.** (A) Mass spectrum of CysTag-CL$^N$-SBP (**7**) after labeling the unique Cys with Alexa647 maleimide (**7a**). The two peaks correspond to the protein labeled with one molecule of Alexa647 with and without hydrolysis of the succinimide ring as shown in the schemes within the red boxes. (B) Mass spectrum of SBP-CL$^C$-V$_H$H$^{GFP}$-H$_6$ (**8**).

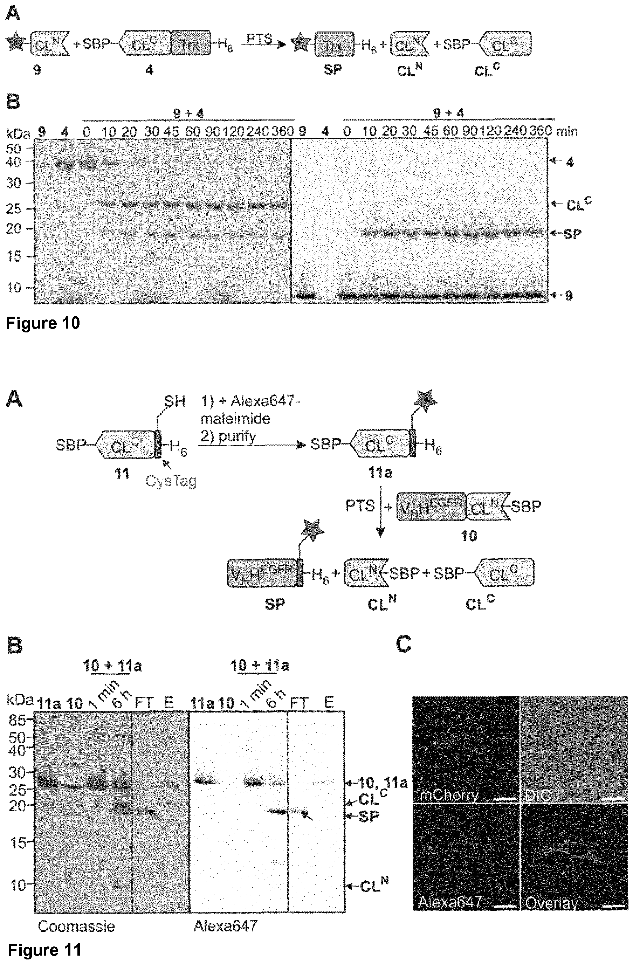

**Figure 10. Splice activity of fluorescein-labeled CL$^N$ synthesized by solid phase peptide synthesis.** (A) Schematic representation of the proteins used for analyzing the splice activity. (B) PAGE analysis flowed by Coomassie staining (left panel) and imaging of fluorescein fluorescence (right panel) for the splice assay showed in (A). 40 $\mu$M of **9** and 10 $\mu$M of **4** was used for the assay. Densitometric analysis of the Coomassie-stained gel was used for the generation of the data shown in Figure 8F. Calculated molecular weights: **9**: 3.7 kDa, **4**: 33.8 kDa, **SP**: 15 kDa, **CL$^N$**: 2.8 kDa, **CL$^C$**: 19.8 kDa.

**Figure 11. C-terminal labeling of a nanobody using the CL intein under oxidizing conditions.** (A) Reaction scheme. (B) SDS-PAGE analysis of the labeling and purification steps for the generation of Alexa647-labeled V$_H$H$^{EGFR}$ nanobody (**SP**). FT and E represent the flow through and elution fractions from Streptactin resin, respectively. The arrows mark the purified splice product (**SP**) that was used for further experiments. (C) Confocal microscopy images of HeLa cells transfected with HA-EGFR-mCherry (HA= hemagglutinin A tag) that were treated with 10 nM Alexa647-labeled V$_H$H$^{EGFR}$ (**SP**). Scale bars represent 20 $\mu$m. SP = splice product. Calculated molecular weights: **10**: 23.4 kDa, **11**: 22.0 kDa, **11a**: 22.9 kDa, **SP** = 18.7, kDa, **CL$^N$** = 7.8 kDa, **CL$^C$** = 19.8 kDa.

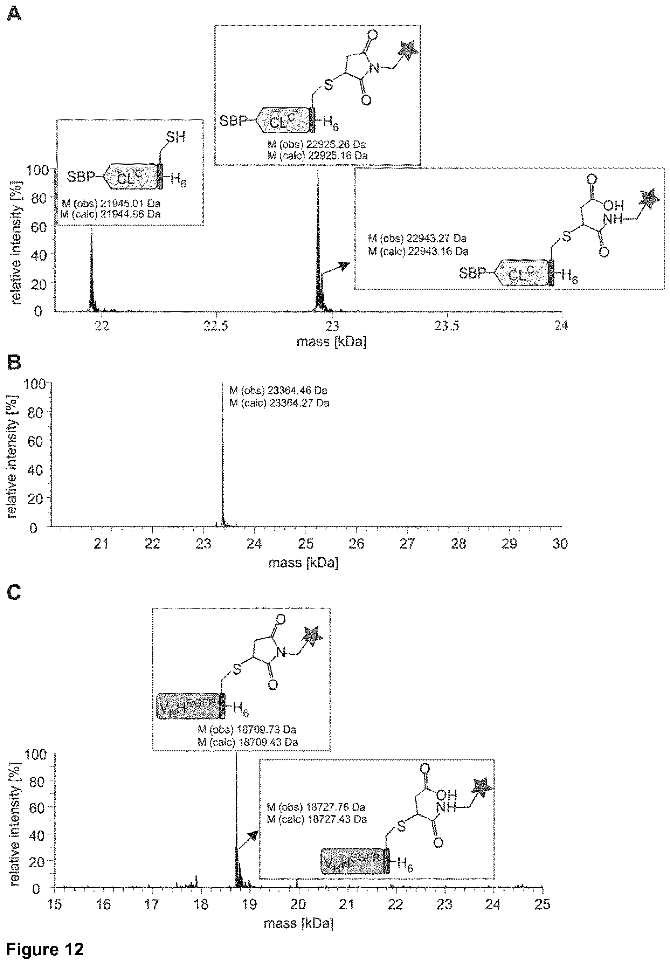

**Figure 12. ESI-MS analysis of proteins used for C-terminal labeling of V$_H$H$^{EGFR}$.** (A) Mass spectrum of SBP-CL$^C$-CysTag-H$_6$ (**11**) after labeling the unique Cys with Alexa647 maleimide (**11a**). The three peaks correspond to the unlabeled protein and protein labeled with one molecule of Alexa647 with and without hydrolysis of the succinimide ring as shown in the schemes within the red boxes. (B) Mass spectrum of V$_H$H$^{EGFR}$-CL$^N$-SBP (**10**). (C) Mass spectrum of the purified splice product of **10** + **11a**. The two peaks correspond to the protein labeled with one molecule of Alexa647 with and without hydrolysis of the succinimide ring as shown in the schemes within the red boxes.

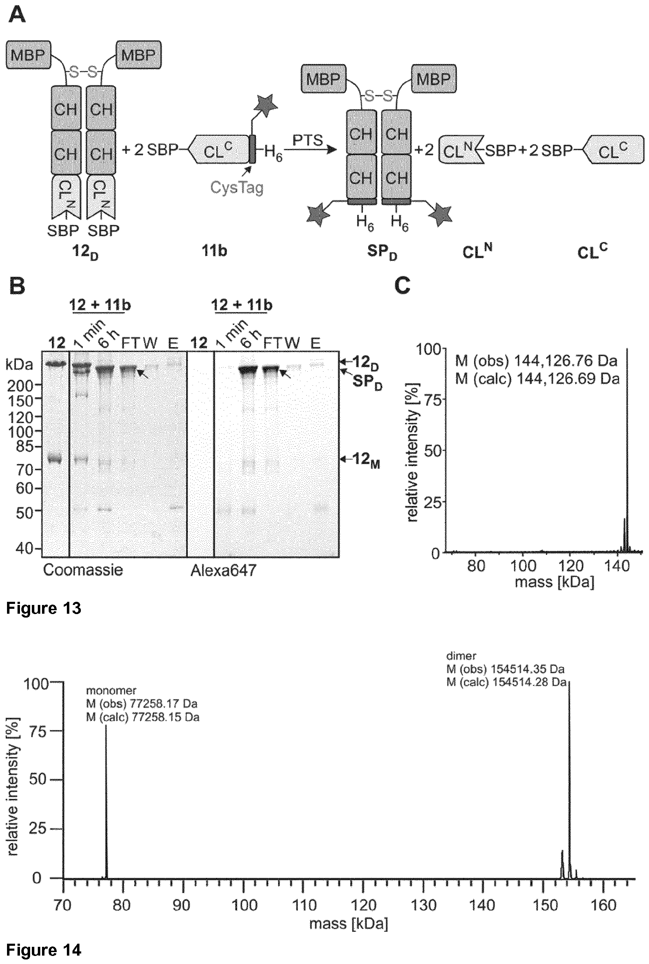

**Figure 13. C-terminal labeling of an antibody Fc dimer fusion protein under oxidizing conditions.** (A) Reaction scheme. The protein SBP-CL$^C$-CysTag-H$_6$ (**11**) was first reacted with fluorescein-maleimide to label the unique thiol on the CysTag resulting in **11b** (not shown). (B) SDS-PAGE analysis of the labeling and purification process. The arrows mark the purified splice product (**SP**) in the FT fraction. The subscripts M and D refer to the monomeric and dimeric forms of the Fc-fusion protein. FT, W and E refer to the flow through, wash and elution fractions from Streptactin resin, respectively. Note that **11b** is not seen on the gel because of its low molecular weight. (C) MS-analysis of the FT fraction containing the desired **SP$_D$** protein. Calculated molecular weights: **11b** = 22.4 kDa, **12$_M$** = 77.3 kDa, **12$_D$** = 154.5 kDa, **SP$_D$** = 144.1 kDa, **CL$^N$** = 7.8 kDa, **CL$^C$** = 19.8 kDa.

**Figure 14. ESI-MS analysis of MBP-Fc-CL$^N$-SBP.** MBP-Fc-CL$^N$-SBP (**12**) was expressed and purified from E. *coli* Origami cells. The observed mass for the dimer corresponds to the presence of one disulfide bond.

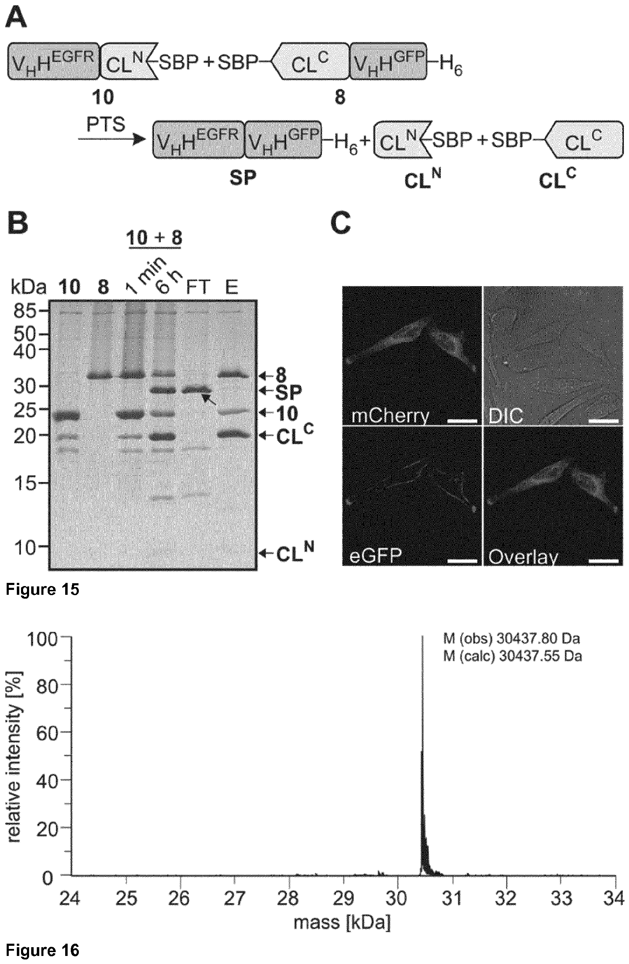

**Figure 15. Creation of a bispecific-nanobody using the CL intein.** (A) Reaction scheme. (B) Analysis of the splicing reaction and purification of V$_H$H$^{EGFR}$-V$_H$H$^{GFP}$ using Coomassie-stained SDS-PAGE. FT and E represent the flow through and elution fractions from Streptactin resin respectively. The arrow marks the purified splice product. (C) Confocal microscopy images of HeLa cells transfected with HA-EGFR-mCherry (HA= hemagglutinin A tag) that were treated with 10 nM V$_H$H$^{EGFR}$-V$_H$H$^{GFP}$ (**SP**), followed by washing and subsequent incubation with by 1 nM eGFP. Scale bars represent 20 μm. **SP** = splice product. Calculated molecular weights: **8**: 35.0 kDa, **10**: 23.4 kDa, **SP**: 30.5 kDa, **CL$^N$**: 7.8 kDa, **CL$^C$**: 19.8 kDa.

**Figure 16. ESI-MS analysis of the purified splice product V$_H$H$^{EGFR}$-V$_H$H$^{GFP}$-H$_6$.**

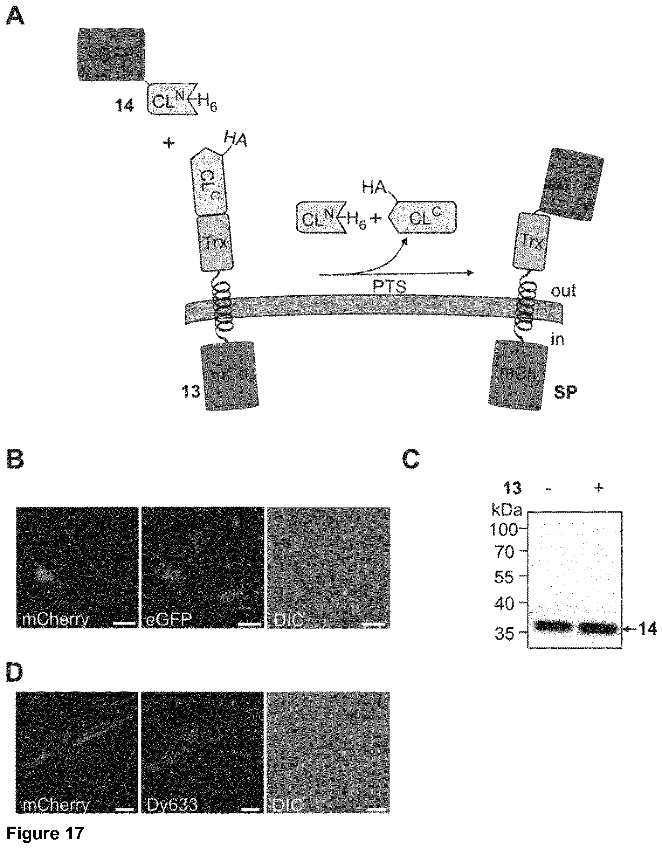

**Figure 17. Low affinity between CL$^N$ and CL$^C$ prevents protein *trans*-splicing to cell surface receptors.** (A) Schematic representation of the strategy for *trans*-splicing on cell surface receptors. (B) Confocal images of HeLa cells expressing **13** that were exposed to 5 μM of **14** for 3 h. Non-specific binding of **14** (eGFP channel) could be observed on cells transfected with **13** (mCherry positive) as well as on untransfected (mCherry negative) cells. (C) Western blot analysis of lysates of cells from the experiment in (B) that were probed against GFP showed a band corresponding to **14** for both untransfected cells and cells transfected with **13**, but no band corresponding to **SP**. (D) Control experiment to confirm correct localization of **13**. Immunostaining of HeLa cells expressing **13** against the extracellular HA-tag on **13** via an anti-HA primary antibody, followed by a Dy633-labeled secondary antibody showed selective Dy633 labeling of cells expressing **13**. A significant fraction of **13** is also present inside cells, presumably due to mislocalization in the secretory pathway caused by overexpression. Scale bars represent 20 μm. HA= hemagglutinin A tag, Trx= thioredoxin, mCh= mCherry. Calculate molecular weights: **13**: 65.1 kDa, **14**: 32.9 kDa, **SP:** 77.4 kDa.

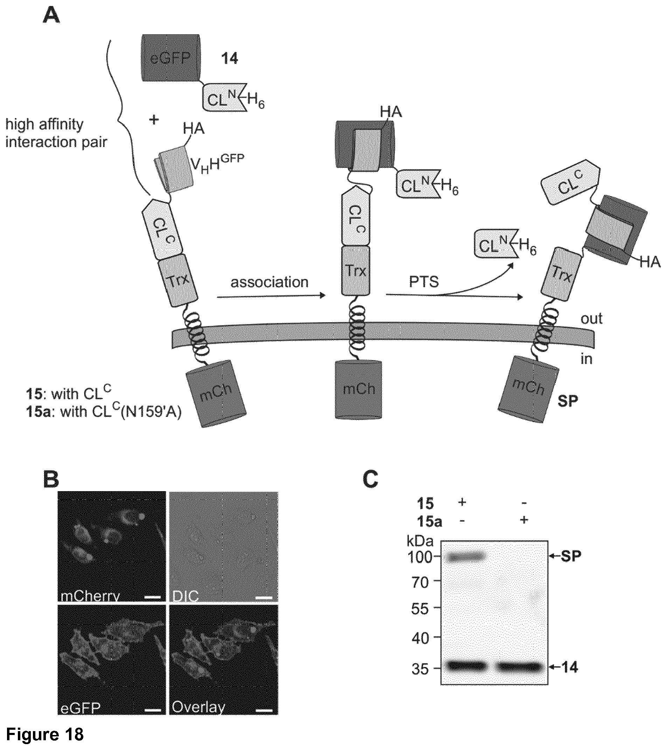

**Figure 18. Nanobody-mediated high affinity interaction enhances *trans*-splicing on a model cell-surface receptor.** (A) Schematic representation of the strategy. (B) Confocal images of HeLa cells expressing **15** that were exposed to 500 nM of **14** for 3 h. Specific binding of **14** (eGFP channel) could be observed on cells transfected with **15** (mCherry positive) but not on untransfected (mCherry negative) cells. Endocytosis of the model receptor led to significant intracellular eGFP signal, although clear membrane localization could also be observed. A significant fraction of **15** is also present inside cells, presumably due to mislocalization in the secretory pathway caused by overexpression. (C) Western blot analysis of lysates of cells from the experiment in (B) that were probed with an anti-GFP antibody showed a band corresponding to **14**, as well as a higher molecular weight band corresponding to **SP**. Exposure of HeLa cells expressing a variant of **15** (**15a**) with a splice-inactivating mutation of CL$^C$ (N159'A) showed a band corresponding to **14**, but not **SP**. Since samples were denatured prior to western blotting, HA-V$_H$H$^{GFP}$-CL$^C$ no longer remained bound to **SP** during western blot analysis. Scale bars represent 20 μm. HA= hemagglutinin A tag, Trx= thioredoxin, mCh=mCherry. Calculate molecular weights: **14: 32.9 kDa, 15/15a: 78.8 kDa, SP: 77.4 kDa.**

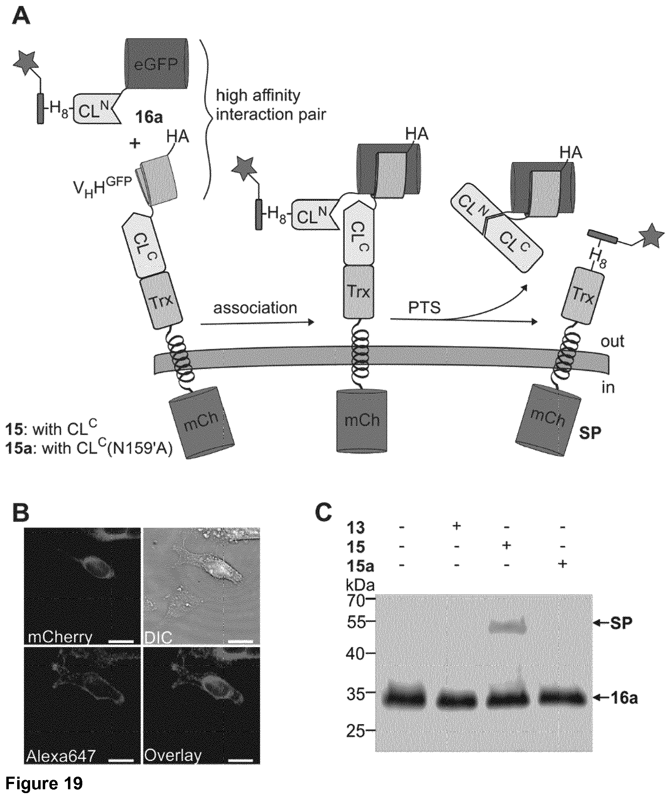

**Figure 19. Traceless fluorescent labeling of a model receptor via *trans*-splicing with the CL intein.** (A) Schematic representation of the strategy for the traceless labeling of a model receptor with Alexa647. The transmembrane domain of **15** was derived from PDGFR. Protein **16** was produced in *E. coli* and site-selectively labeled on a single Cys residue in the CysTag with Alexa647-maleimide to get **16a**. (B) Confocal images of HeLa cells expressing **15** that were exposed to 150 nM of **16a** for 3 h. Specific binding of **16a** (Alexa647 channel) could be observed on cells transfected with **15** (mCherry positive) but not on untransfected (mCherry negative) cells. Endocytosis of the model receptor led to significant intracellular Alexa647 signal, although clear membrane localization could also be observed. A significant fraction of **15** is also present inside cells, presumably due to mislocalization in the secretory pathway caused by overexpression. (C) SDS-PAGE analysis followed by imaging of Alexa647 fluorescence on lysates from either untransfected Hela cells or Hela cells expressing different cell surface receptor constructs (**13, 15** or **15a**) that were treated with 150 nM of **16a** for 3 h. While all experiments showed a band corresponding to **16a**, a band corresponding to **SP** was detected only for cells expressing **15**. Scale bars represent 20 μm. HA= hemagglutinin A tag, Trx= thioredoxin, mCh=mCherry. Calculate molecular weights: **16a: 33.4 kDa, 15/15a: 78.8 kDa, SP: 51.4 kDa.**

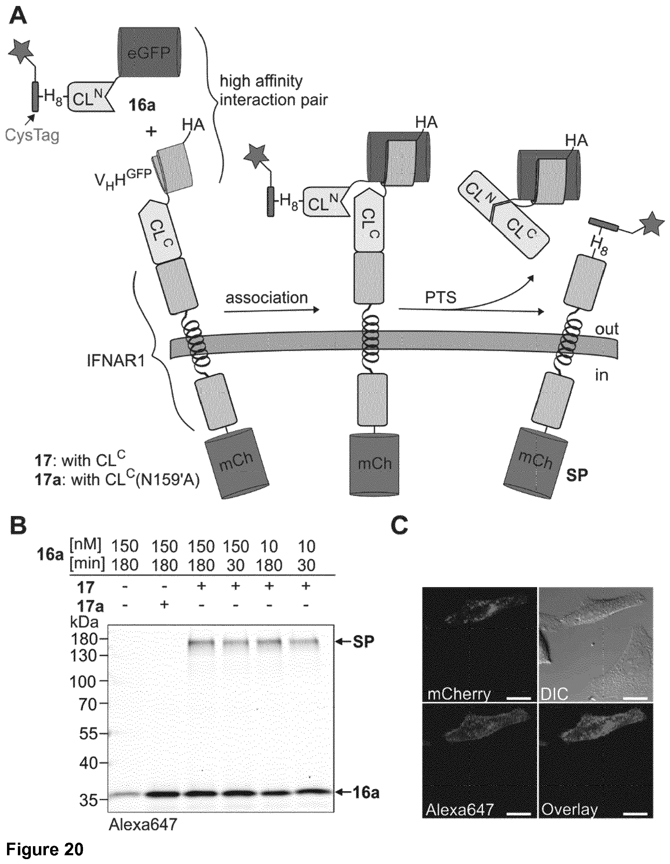

**Figure 20. Traceless fluorescent labeling of IFNAR1 under oxidizing conditions via high-affinity protein *trans*-splicing.** (A) Schematic representation of the labeling strategy. Protein CysTag(Alexa647)-H$_8$-CL$^N$-eGFP(C49S) (**16a**) was obtained by production of **16** in *E. coli* and its subsequent thiol bioconjugation with Alexa647-maleimide (not shown). (B) PAGE analysis of lysates from Hela cells transfected with

17 or **17a** that 24 h later were treated with **16a**. The unusual migration behavior of the splice product (**SP**) stems from the post-translational glycosylation of IFNAR1. (C) Confocal microscopy images of HeLa cells expressing **17** that were exposed to 10 nM of **16a** for 30 min. Specific binding of **16a** could be observed only on cells transfected with **17** but not on untransfected cells. Note that IFNAR1 is strongly endocytosed at 37 °C which results in significant intracellular Alexa647 signal. Scale bars represent 20 $\mu$m. HA= hemagglutinin A tag. mCh = mCherry. Calculated molecular weights: **16a:** 33.4 kDa, **17/17a:** 120 kDa, **SP:** 92.5 kDa.

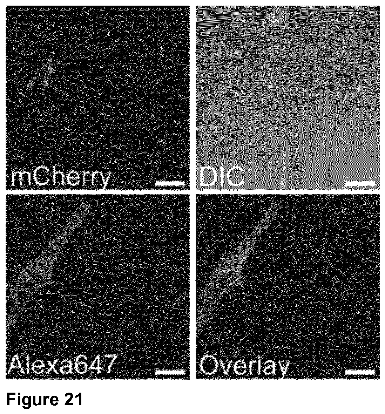

**Figure 21. Traceless fluorescent labeling of IFNAR1 via *trans*-splicing with the CL intein.** (A) Confocal microscopy images of HeLa cells expressing HA-V$_H$H$^{GFP}$-CL$^C$-IFNAR1 (17) that were exposed to 150 nM of **16a** for 3 h. Specific binding of **16a** (Alexa647 channel) could be observed on cells transfected with **17** (mCherry positive) but not on untransfected (mCherry negative) cells. IFNAR1 is strongly endocytosed at 37 °C which resulted in significant intracellular Alexa647 signal. Scale bars represent 20 $\mu$m.

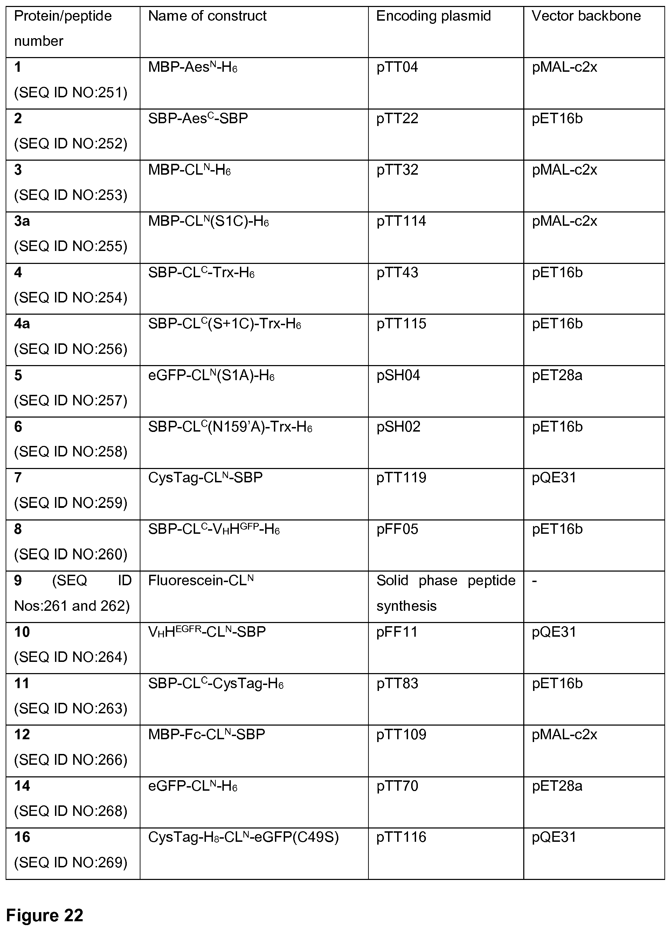

**Figure 22. List of recombinantly produced proteins and synthesized peptides used in this study.**

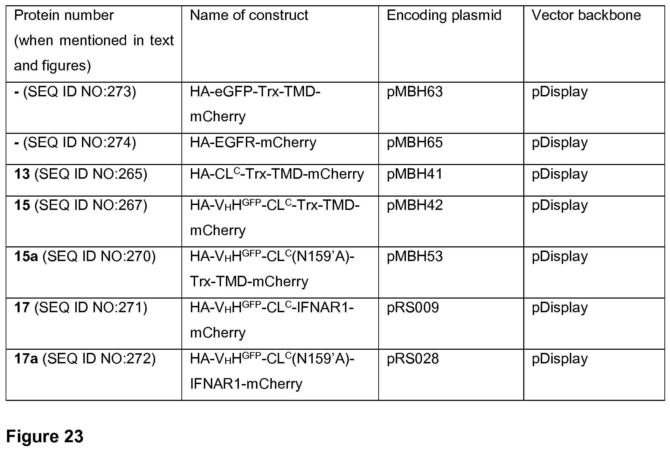

**Figure 23. List of plasmids used for transfection of HeLa cells.**

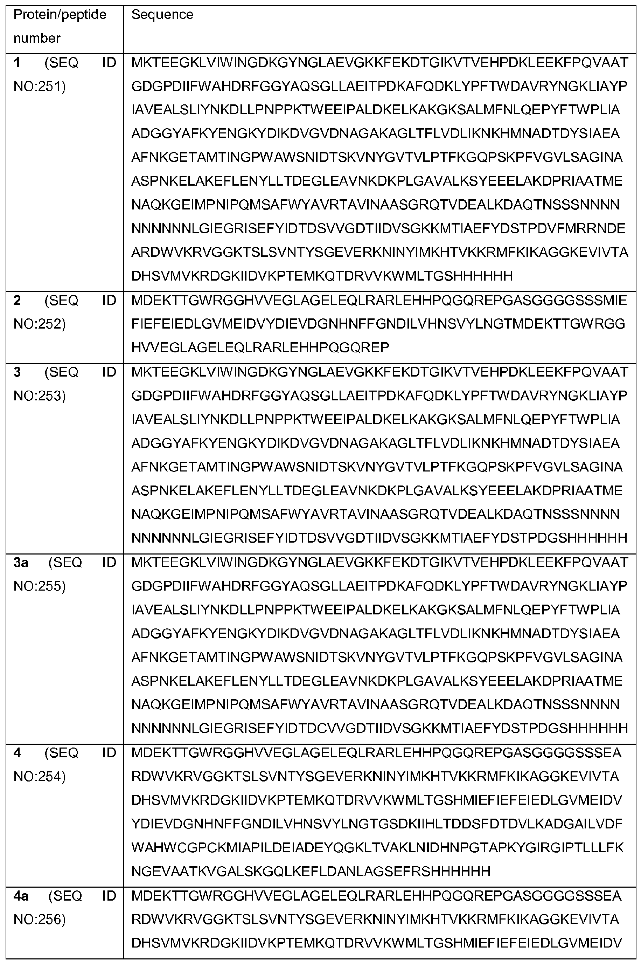

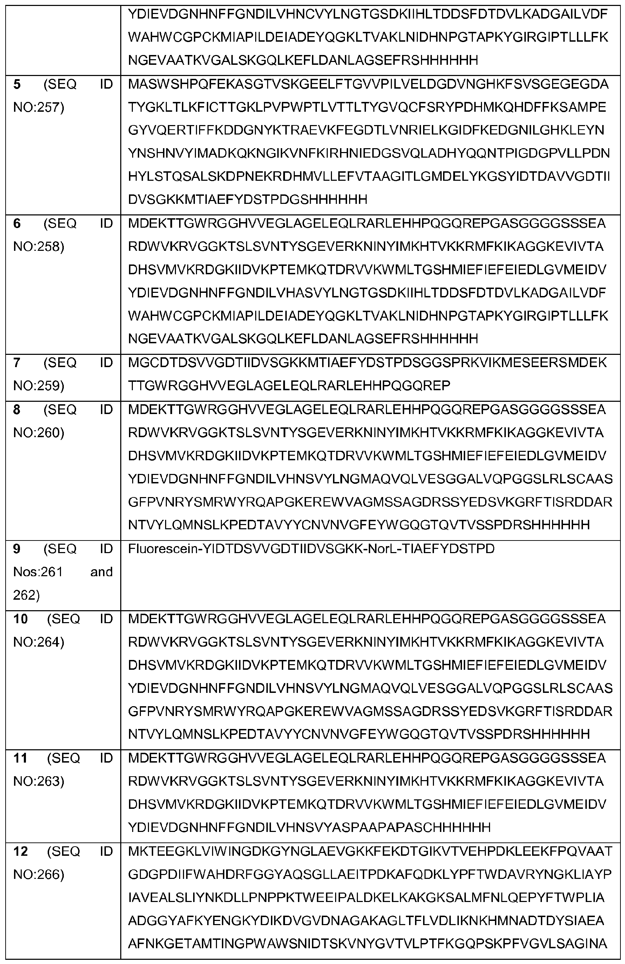

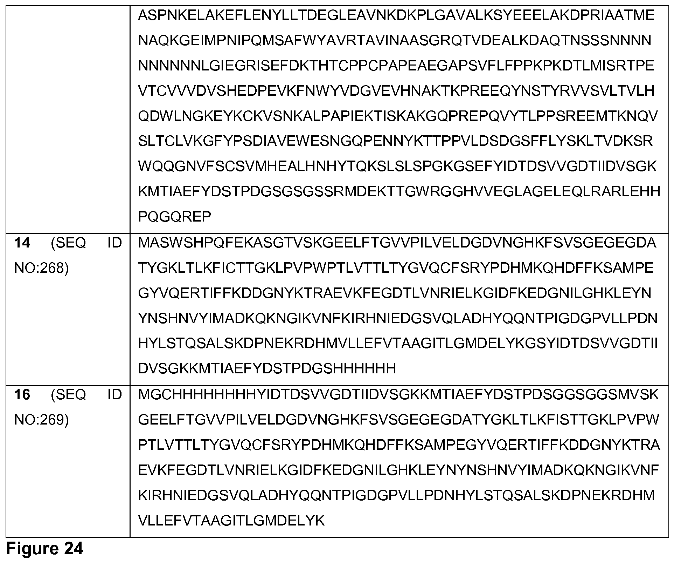

**Figure 24. Amino acid sequences of recombinantly produced proteins and synthesized peptides.**

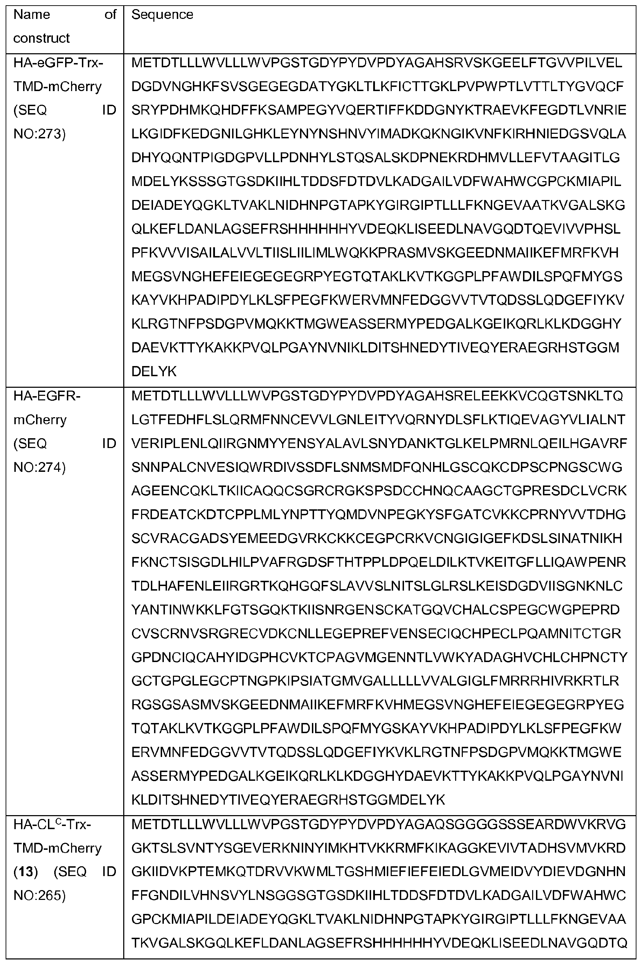

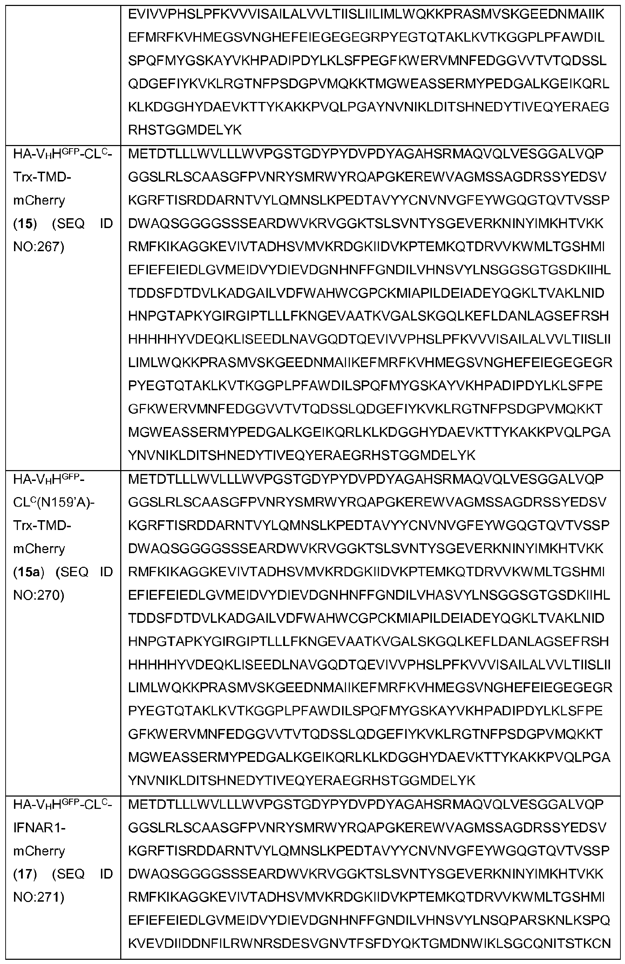

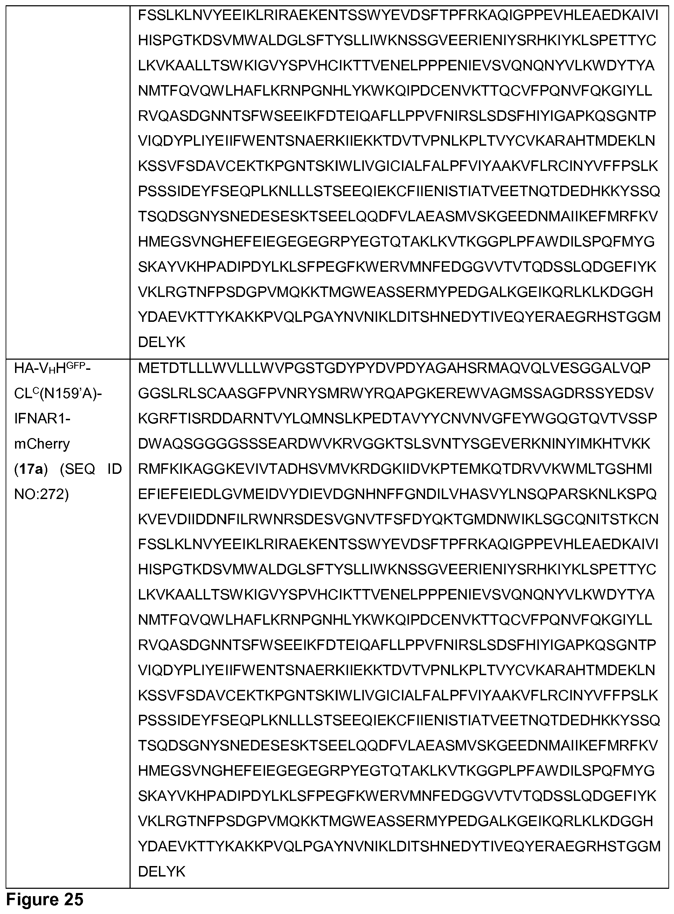

**Figure 25. Amino acid sequences of proteins expressed in HeLa cells.**

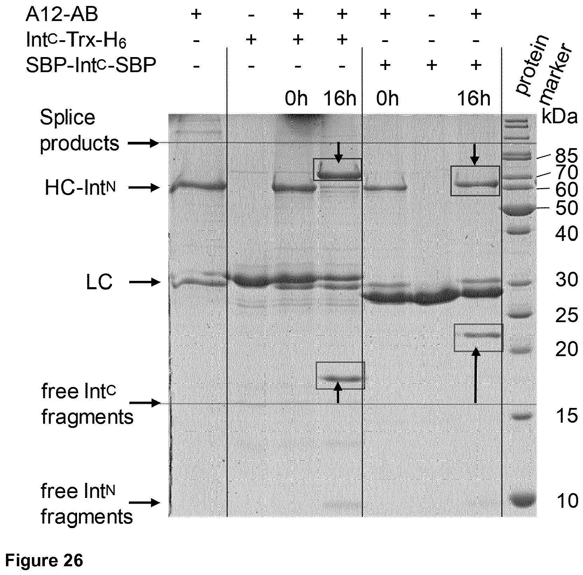

**Figure 26. Splicing of the CL intein with full-length human IgG1 antibody.** Cixutumumab was produced and purified as fusion with the Int$^N$ fragment at the C terminus of the heavy chain (HC), here designated as A12-AB. A12-AB thus consisted of the heavy chain HC-Int$^N$ and the light chain (LC). Shown is a Coomassie brilliant blue-stained SDS-PAGE gel (reducing) of the proteins and the splice reactions as indicated. The HC-Int$^N$ fusion contained a GGSGG linker (HC-GGSGG-Int$^N$). A12-AB was provided at 2 $\mu$M, and each of the Int$^C$ fusion proteins were at 8 $\mu$M. Reactions were performed in Tris-buffer at pH 7.0 and at 25 °C. No reducing agent was present in the protein preparations or the splice reaction.

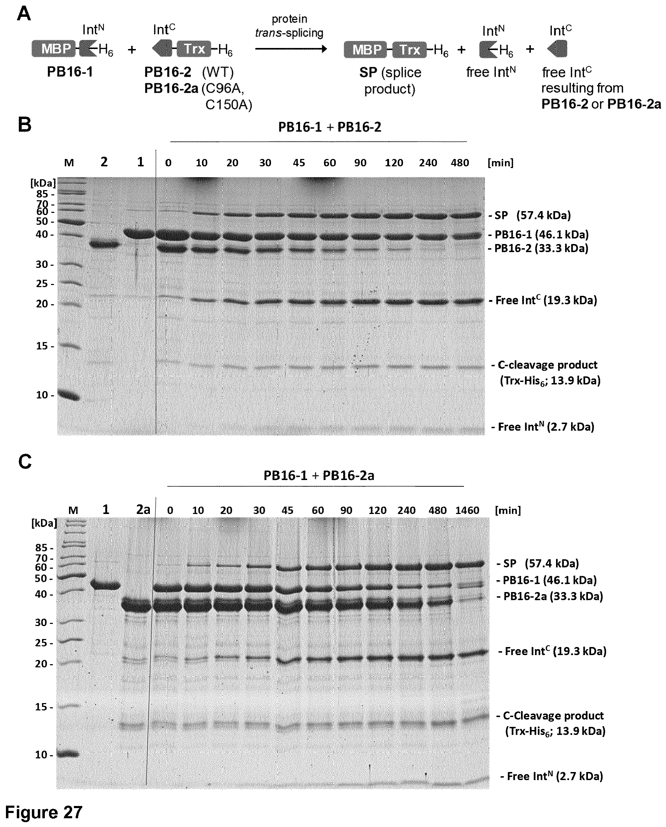

**Figure 27. Splicing of the PolB-16_OarG.** (A) Schematic description of model fusion constructs **PB16-1** and **PB16-2** to investigate splicing of the PolB-16_OarG intein and their protein trans-splicing reaction. (B) and (C) Coomassie brilliant blue-stained SDS-PAGE gels (reducing) of purified proteins and the protein trans-splicing reactions quenched at the indicated time points. (B) shows the reaction of MBP-Int$^N$-H$_6$ (**PB16-1;** 15 $\mu$M) with the wild-type (WT) Int$^C$ fragment (**PB16-2;** 5 $\mu$M) and (C) shows the reaction of MBP-Int$^N$-H$_6$ (**PB16-1;** 5 $\mu$M) with the double-cysteine mutant (Int$^C$(C96A, C150A)) (**PB16-2a;15** $\mu$M). Reactions were performed at 37 °C in Tris-buffer (pH 7.0) with 1 mM TCEP.

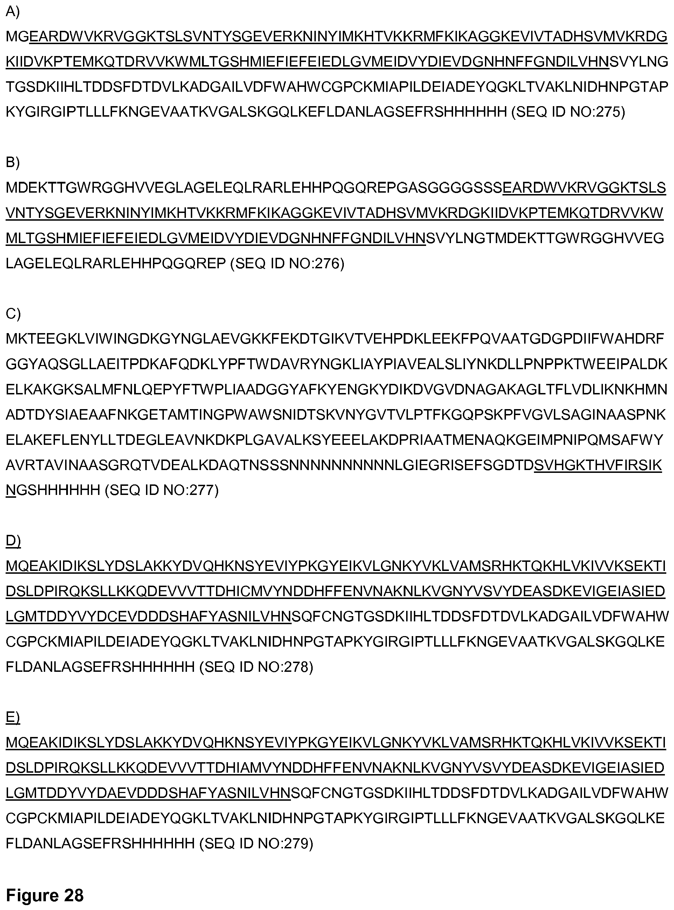

**Figure 28. Specific amino acid sequence constructs of example 2.** (A) Amino acid sequence of CL-intein construct Int$^C$-Trx-H$_6$ (shown in Figure 26); Int$^C$ sequence is underlined, (B) amino acid sequence of CL-intein construct SBP-Int$^C$-SBP (shown in Figure 26); Int$^C$ sequence is underlined, (C) amino acid sequence of PolB-16_OarG-intein construct MBP-Int$^N$-H$_6$ (**PB16-1;** shown in Figure 27); Int$^N$ sequence is underlined, (D) amino acid sequence of PolB-16_OarG-intein construct Int$^C$-Trx-H$_6$ (**PB16-2;** shown in Figure 27); Int$^C$ sequence is underlined, (E) amino acid sequence of PolB-16_OarG-intein construct Int$^C$(C96A, C150A)-Trx-H$_6$ (**PB16-2a;** shown in Figure 27); Int$^C$ sequence is underlined.

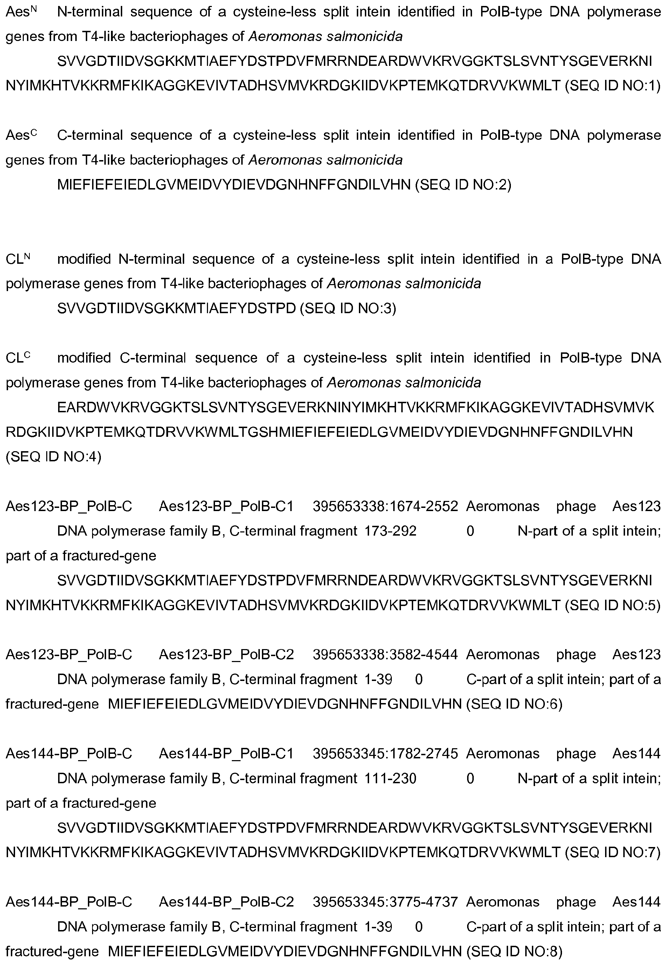

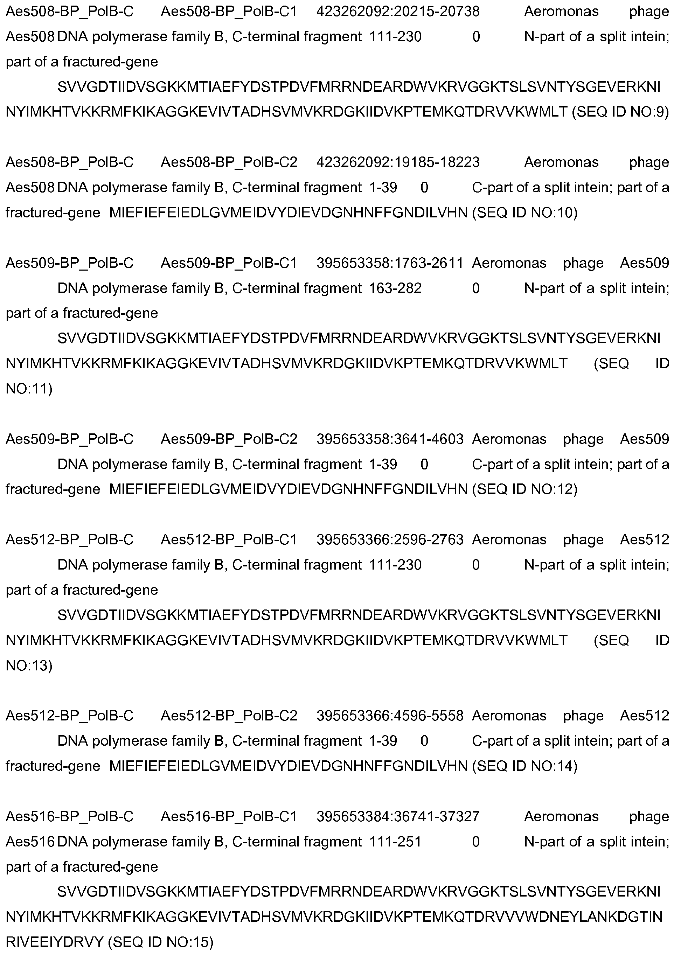

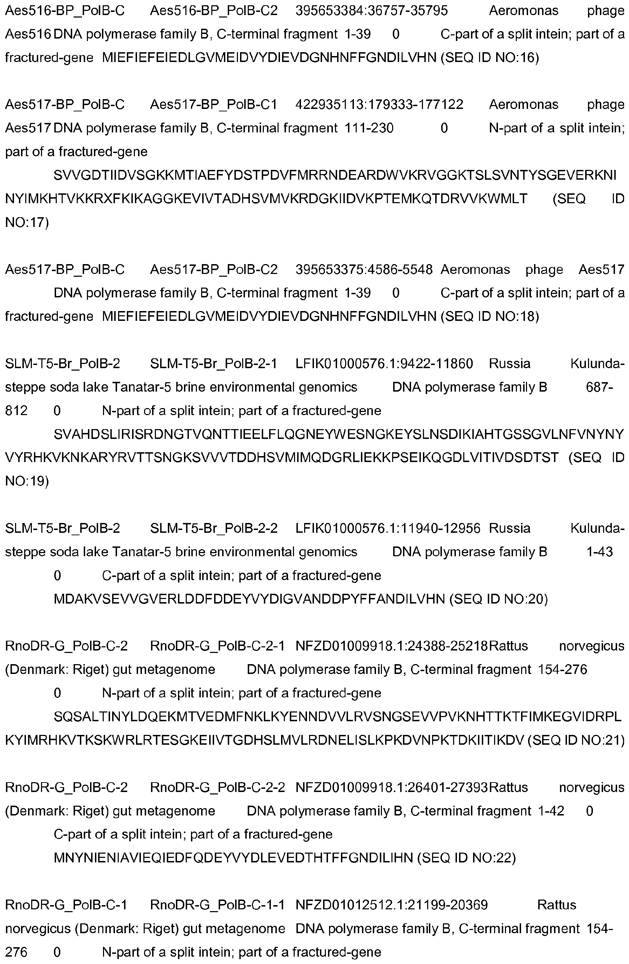

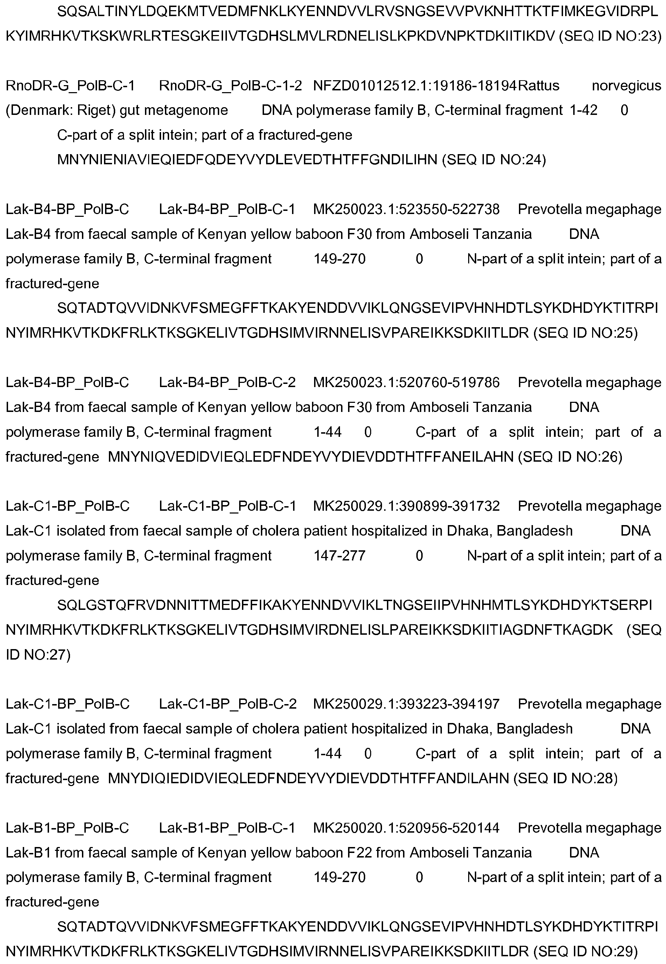

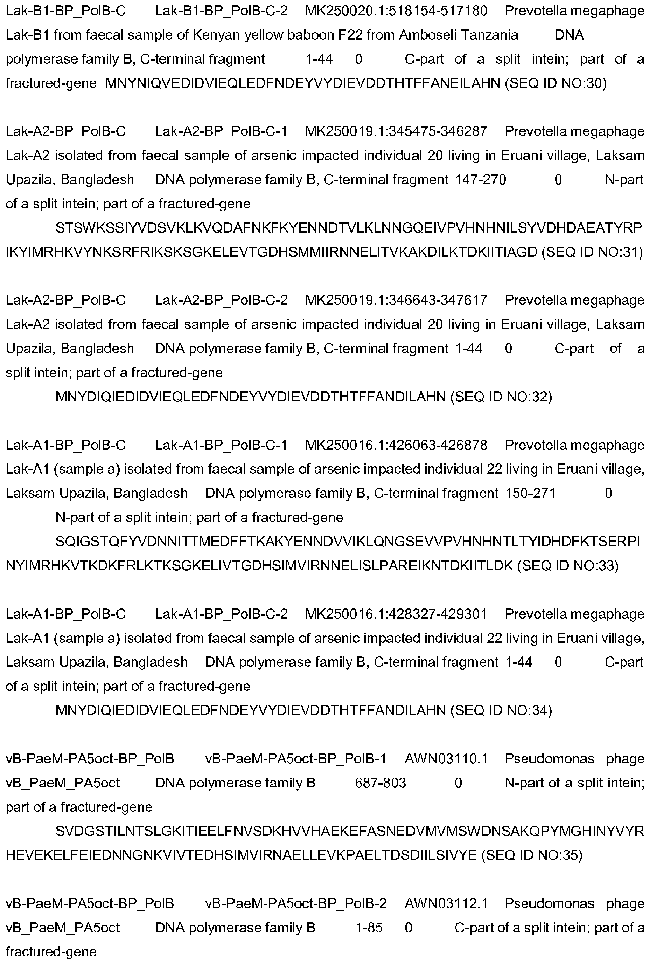

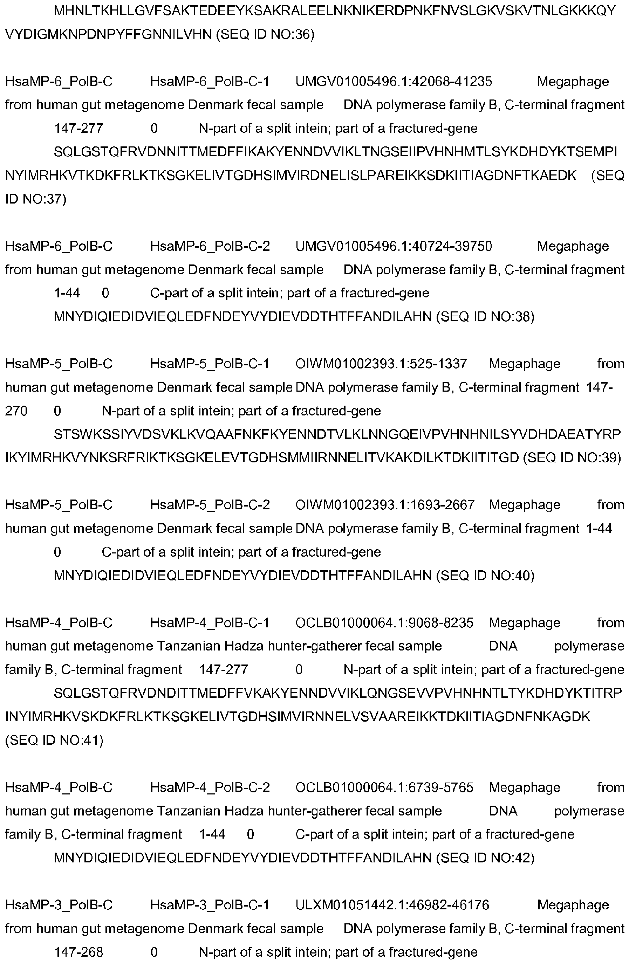

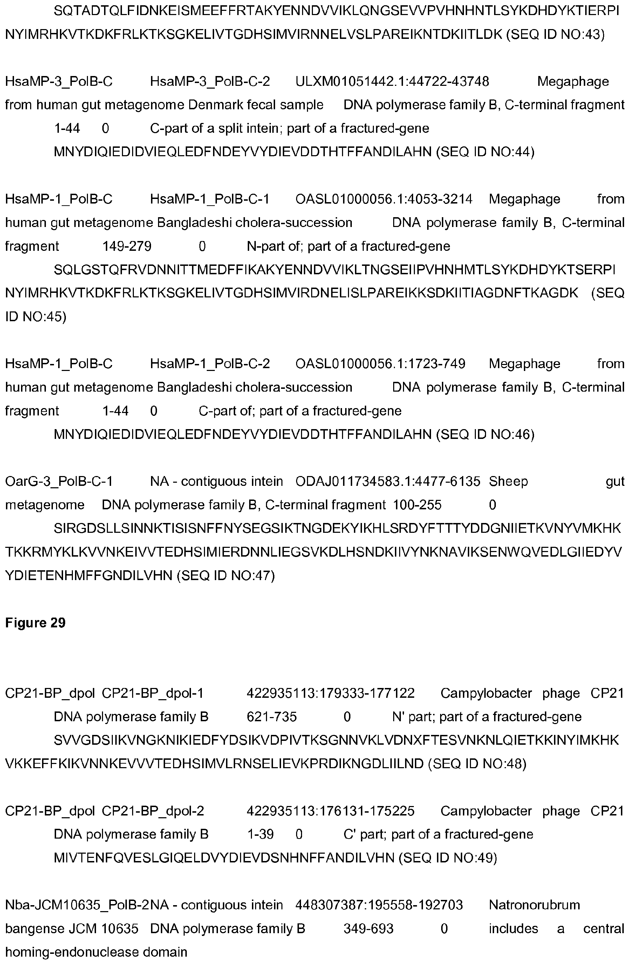

**Figure 29.** Amino acid sequences of N-terminal and C-terminal sequences of split inteins and an amino acid sequence of a contiguous intein sequence (pol-e-integration point alleles/ pol-e type inteins) (with information of intein name, split-intein part name, host protein NCBI accession, species, host type, position in host protein, occuring cysteine residues (positions), comments, aa sequence, in that order).

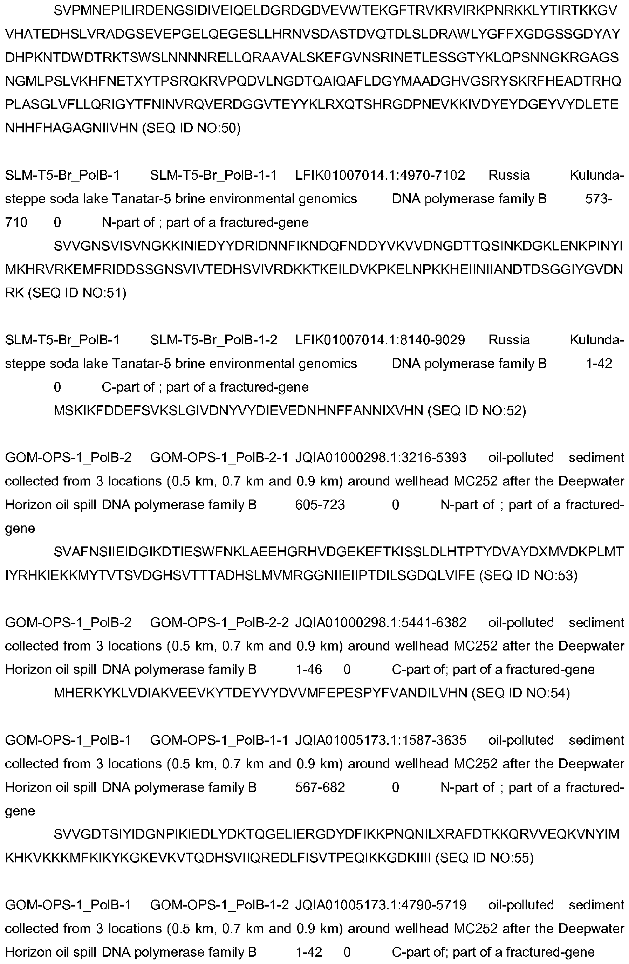

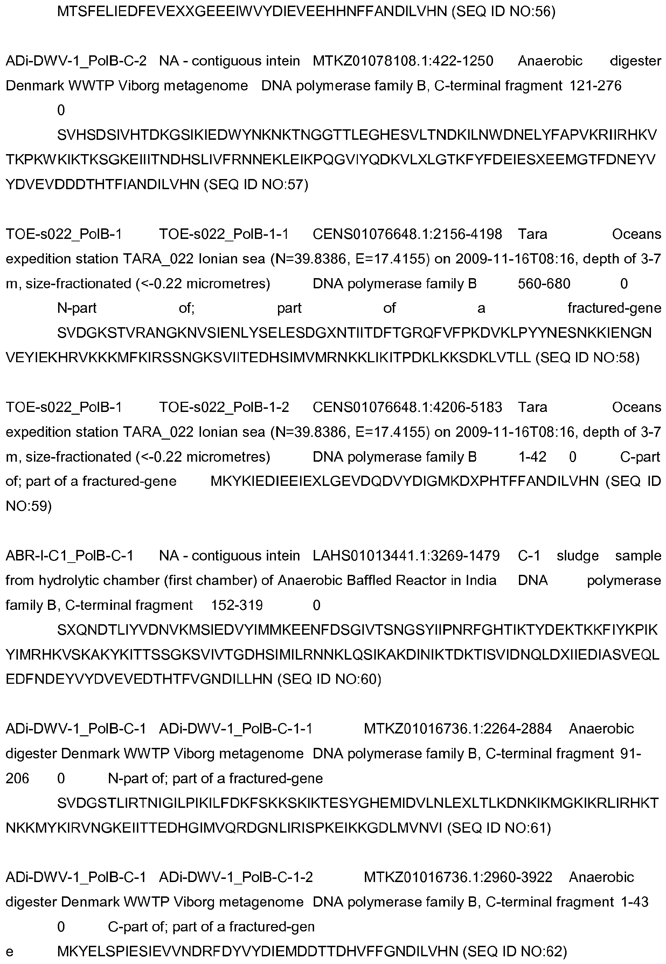

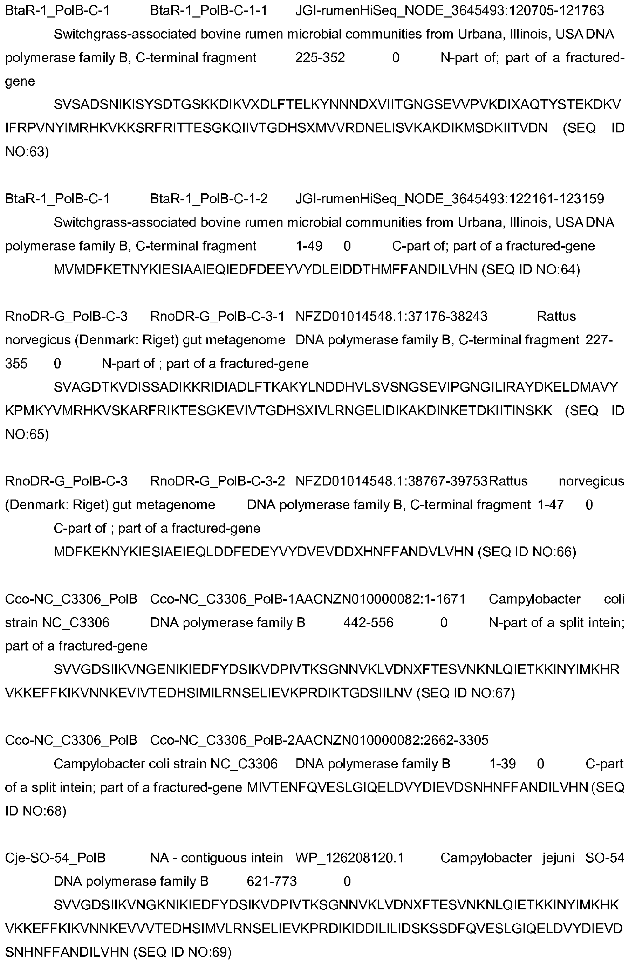

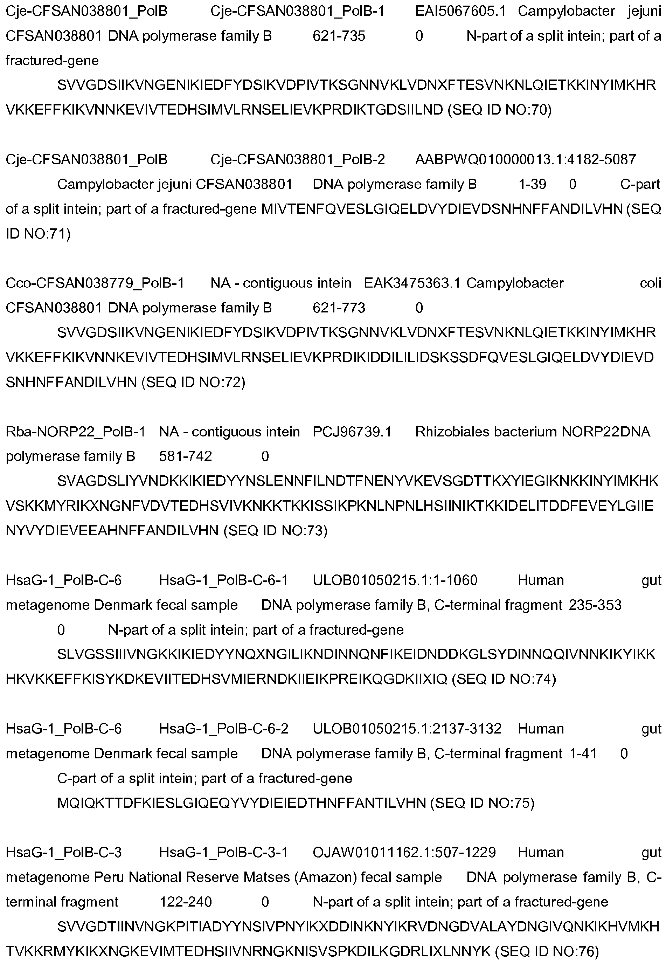

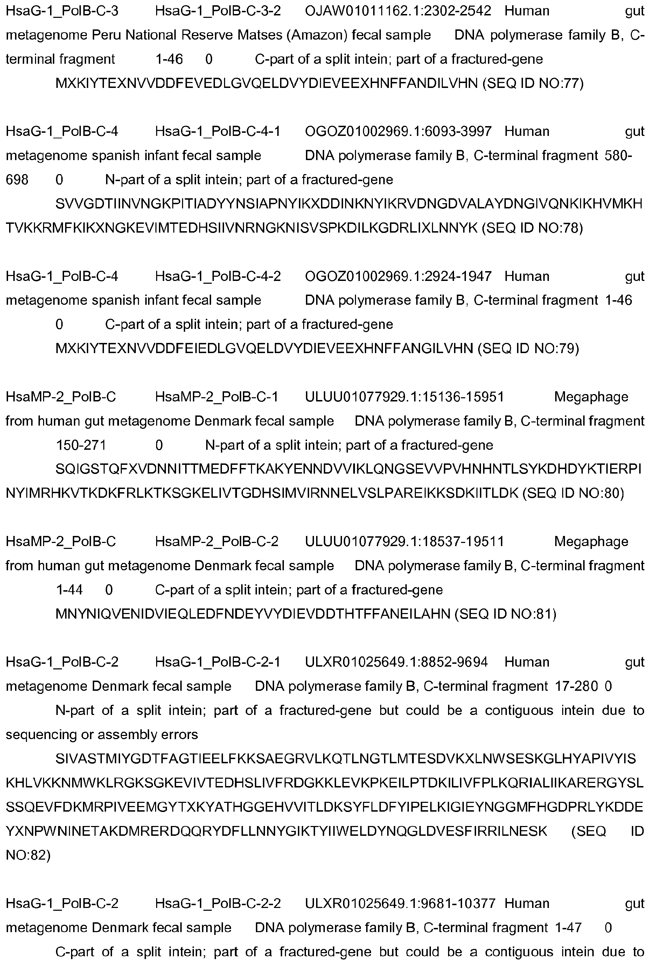

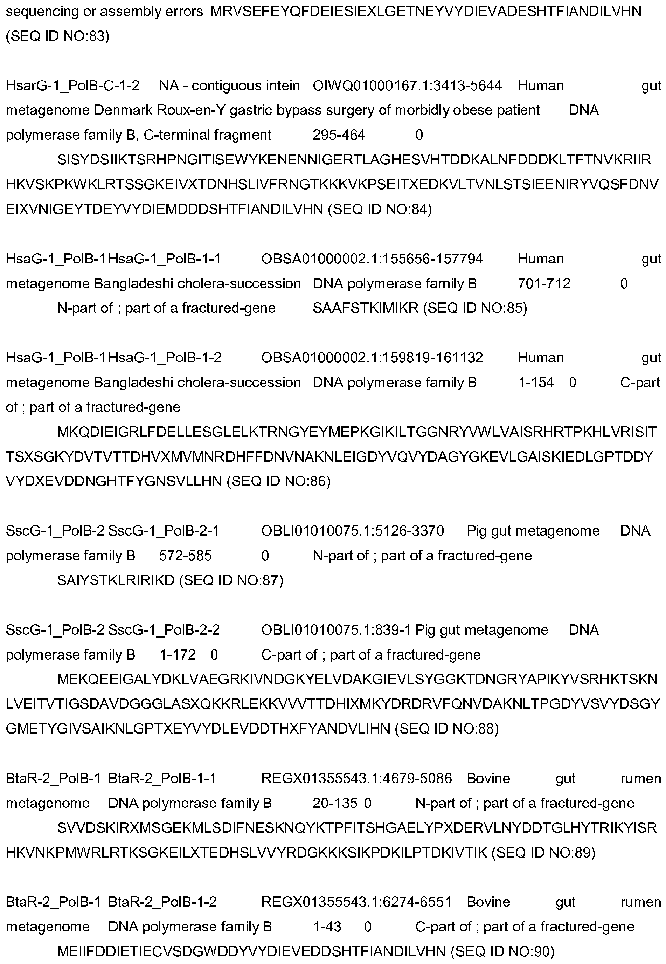

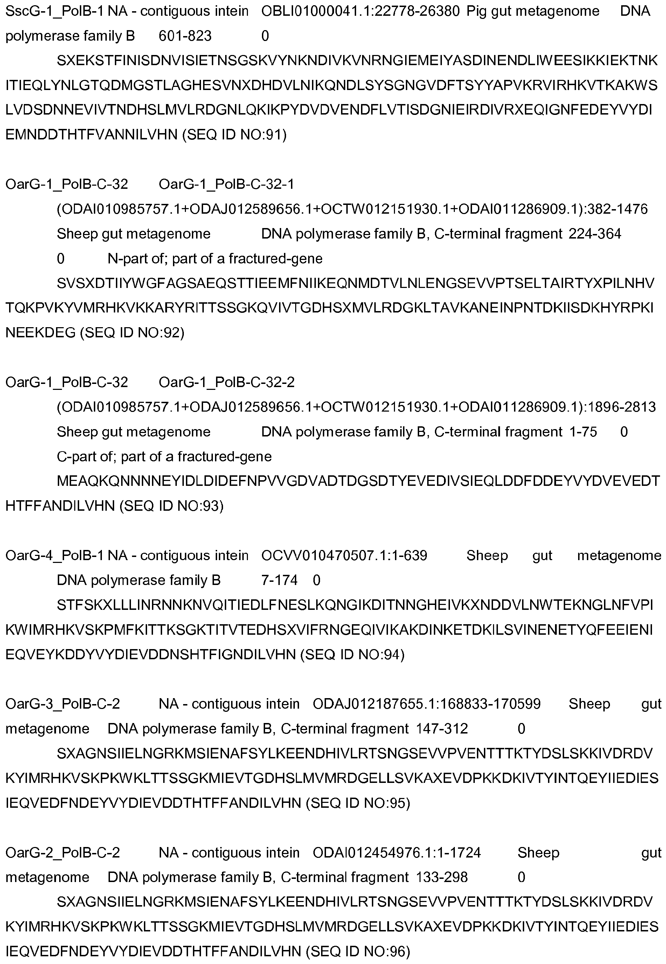

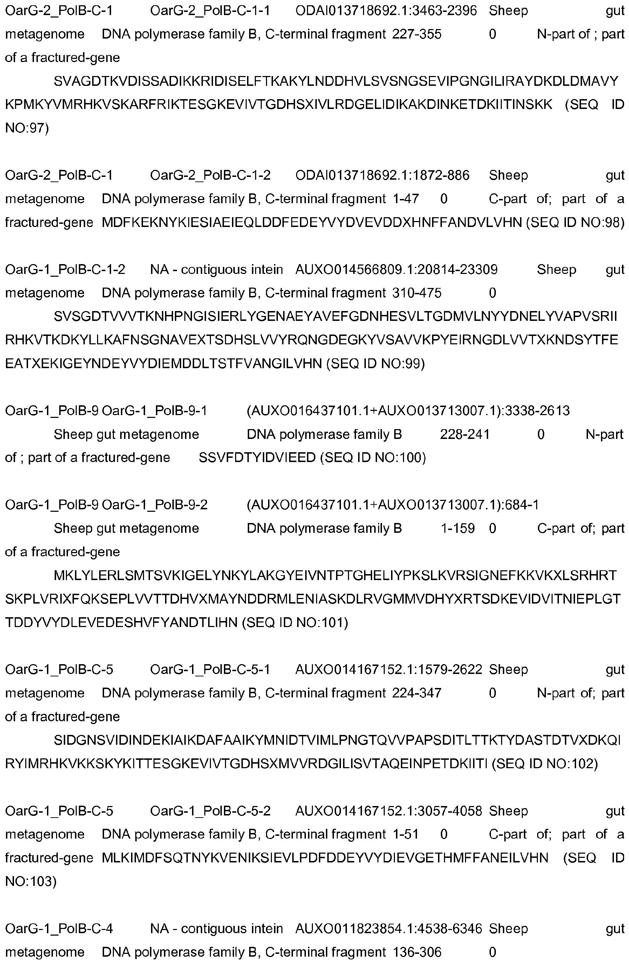

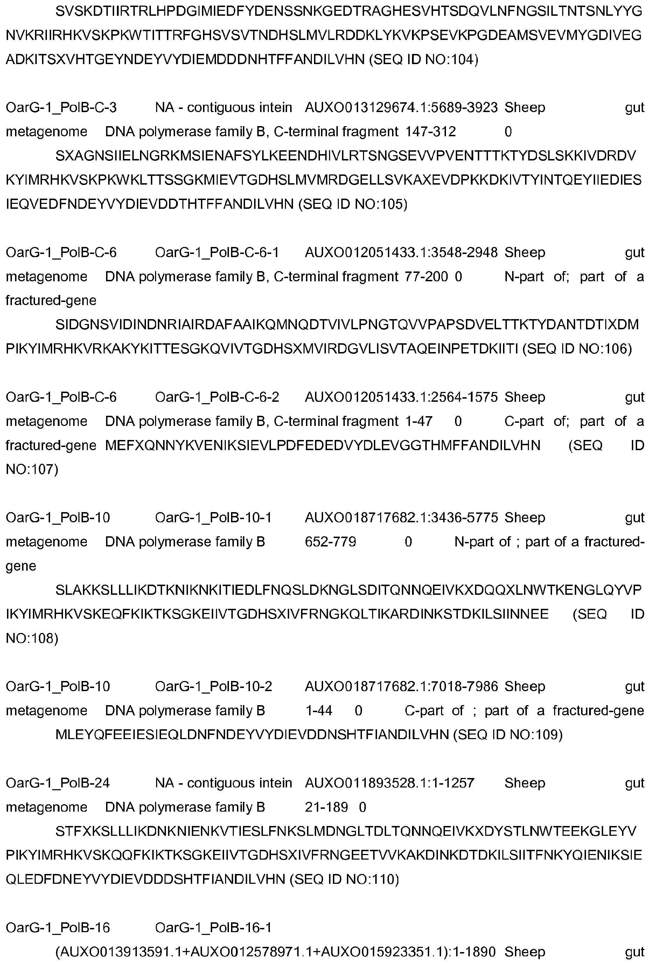

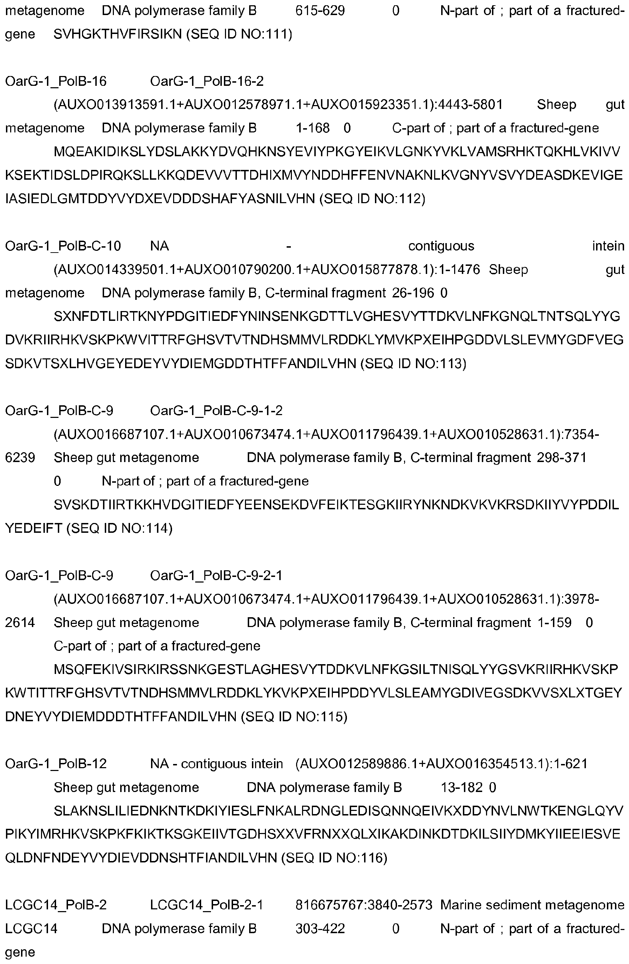

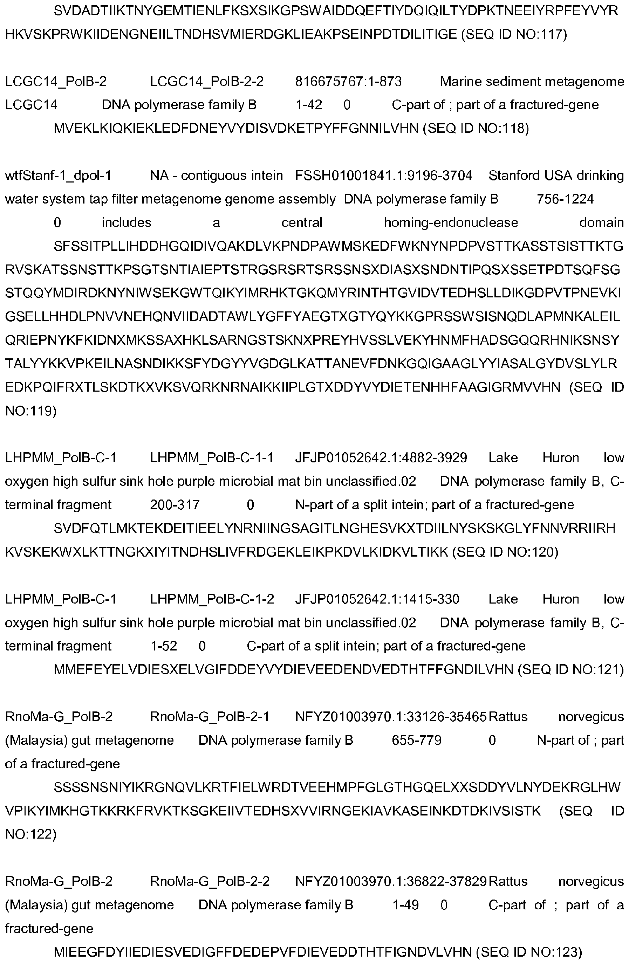

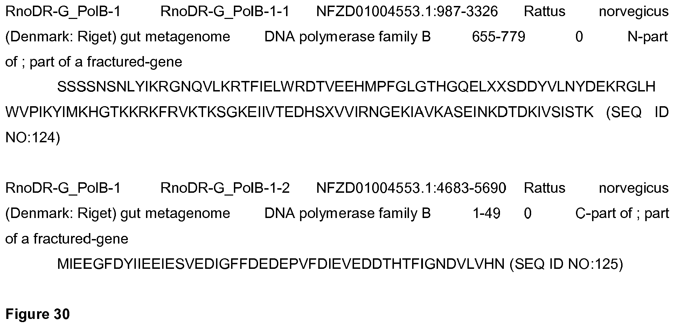

**Figure 30.** Amino acid sequences of N-terminal and C-terminal sequences of split inteins and amino acid sequences of contiguous intein sequences (pol-e integration point alleles/ pol-e type inteins), wherein X is any naturally occuring amino acid residue (with information of intein name, split-intein part name, host protein NCBI accession, species, host type, position in host protein, occuring cysteine residues (positions), comments, aa sequence, in that order). Split intein BtaR-1_PolB-C-1 is from sequence data from the JGI (Joint Genome Institute) (SEQ ID Nos. 63 and 64). All other accessions are from NCBI.

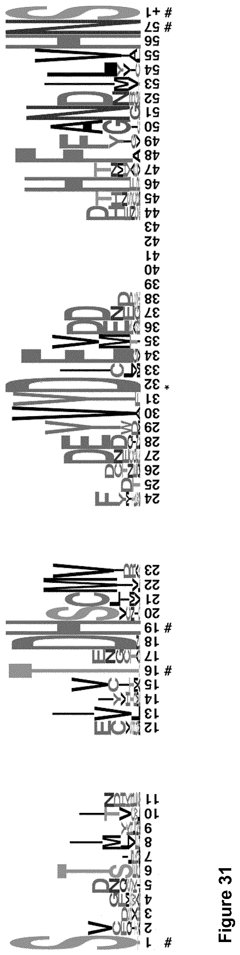

**Figure 31.** Sequence alignment of potential sequence motifs of pol-e type intein sequences of Figures 29 and 30.

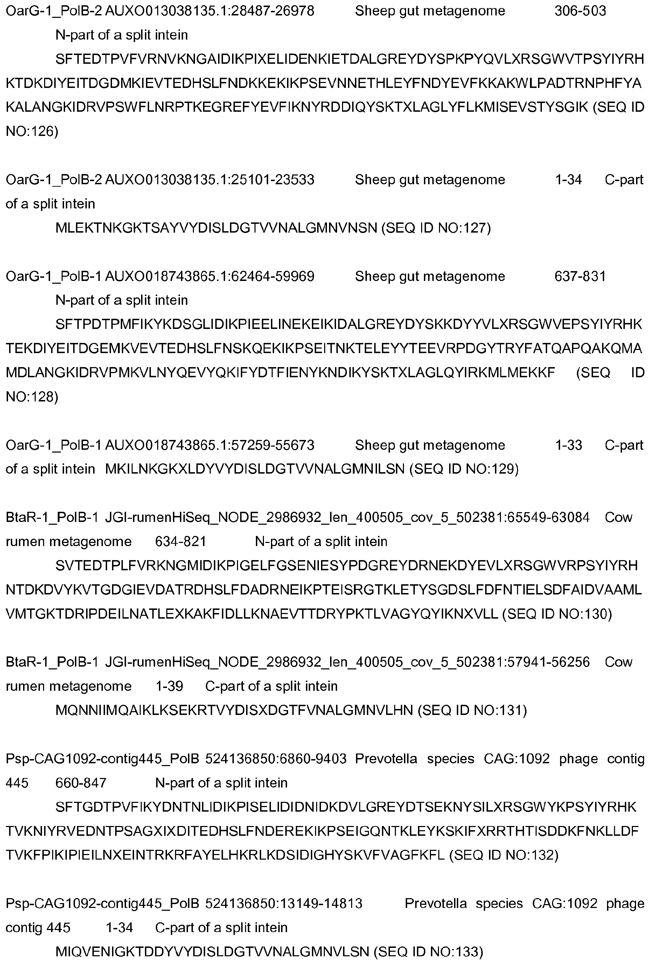

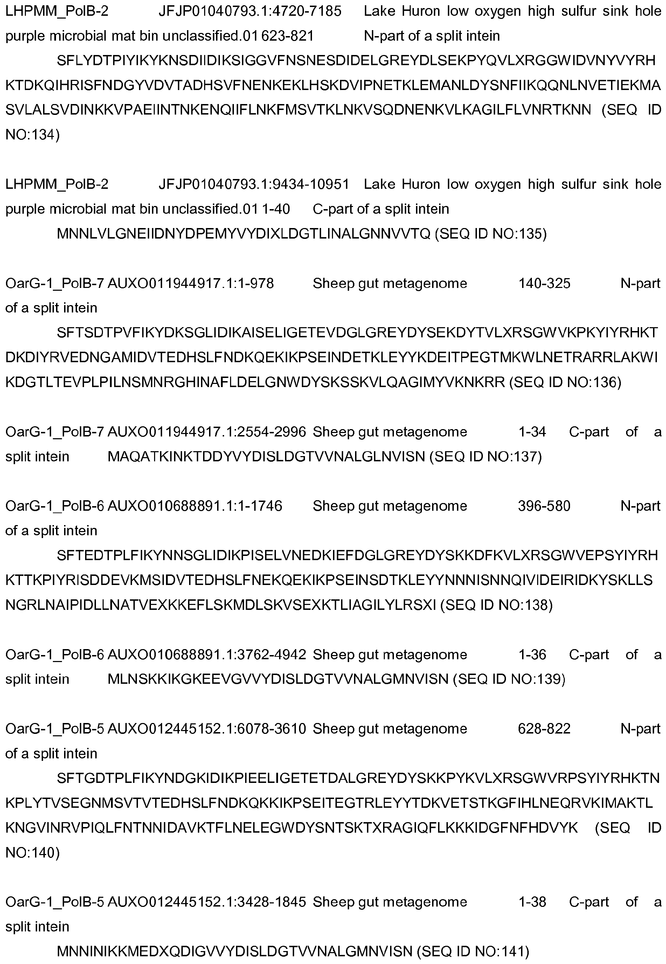

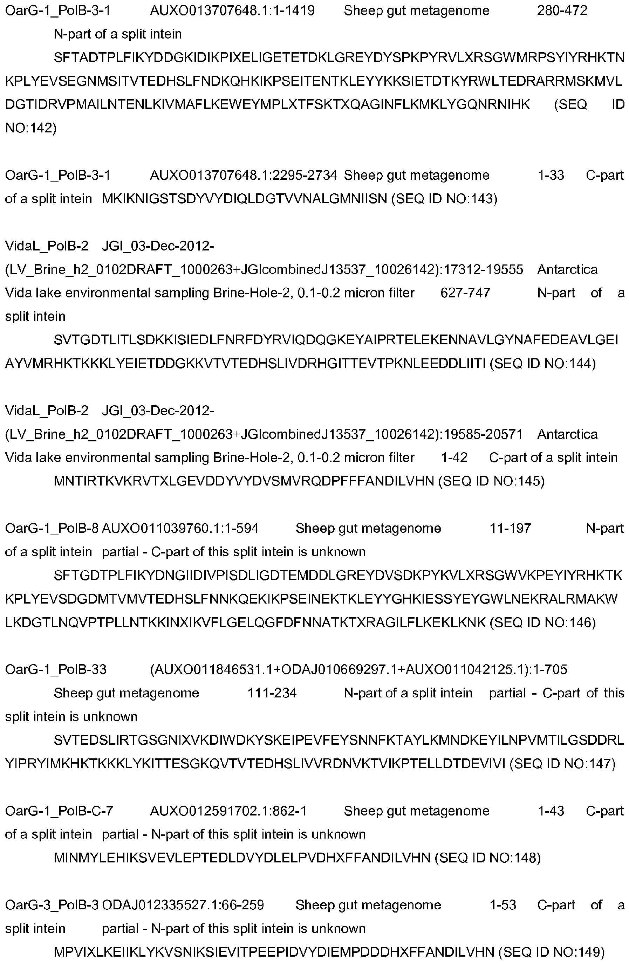

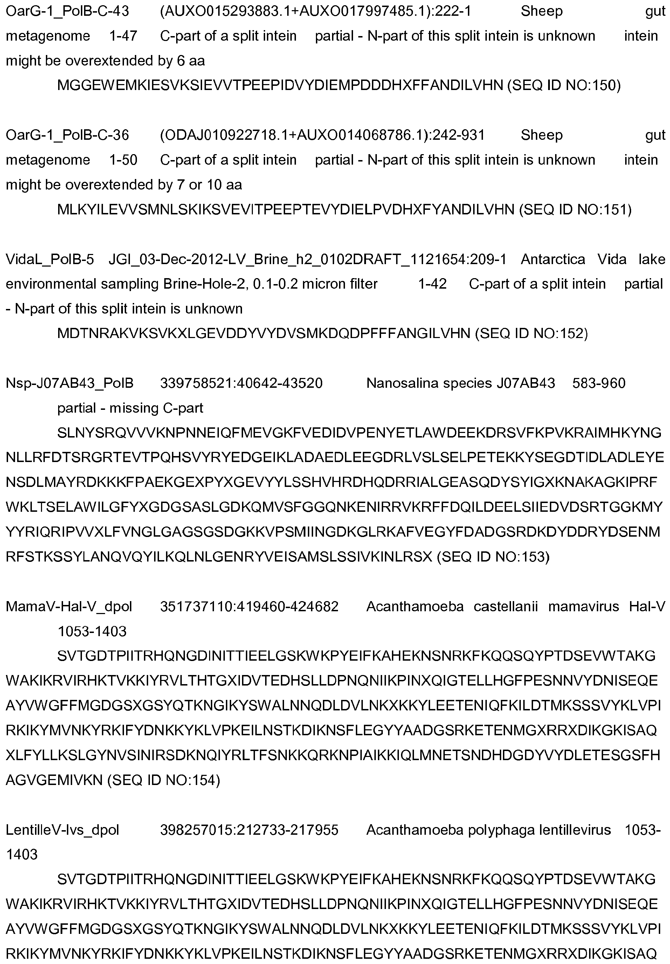

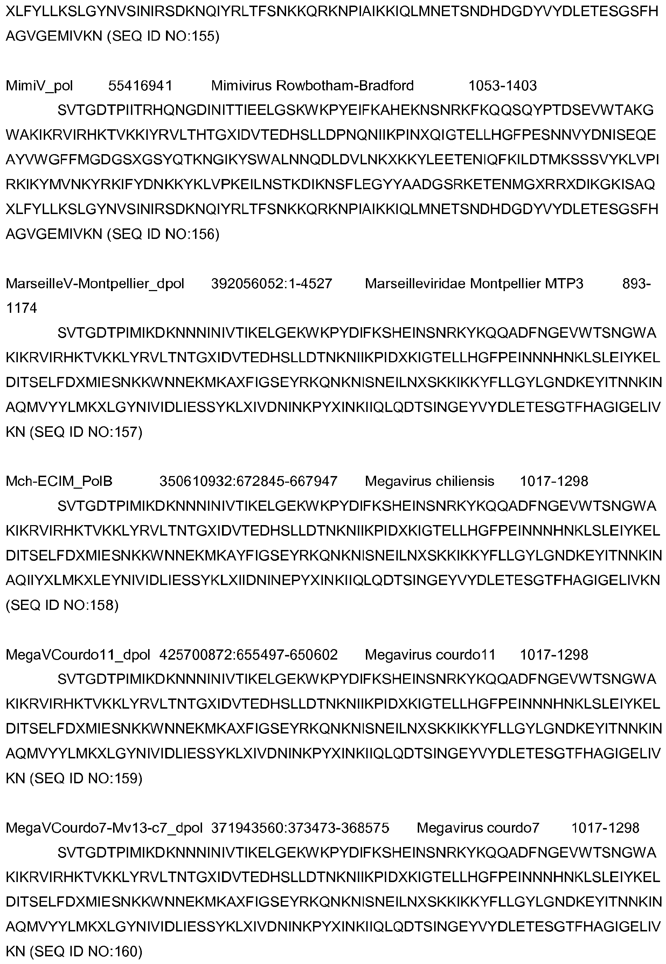

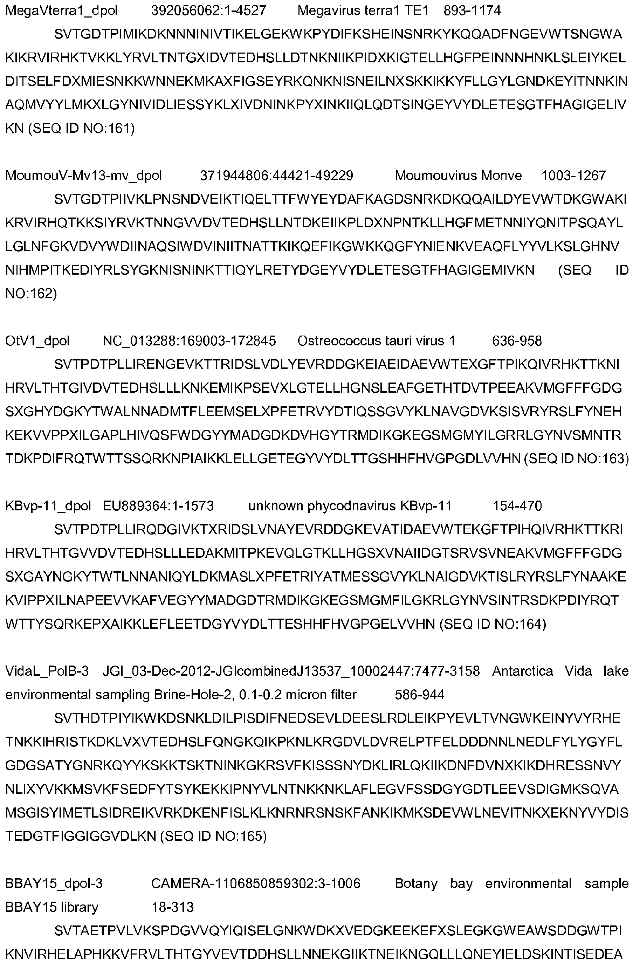

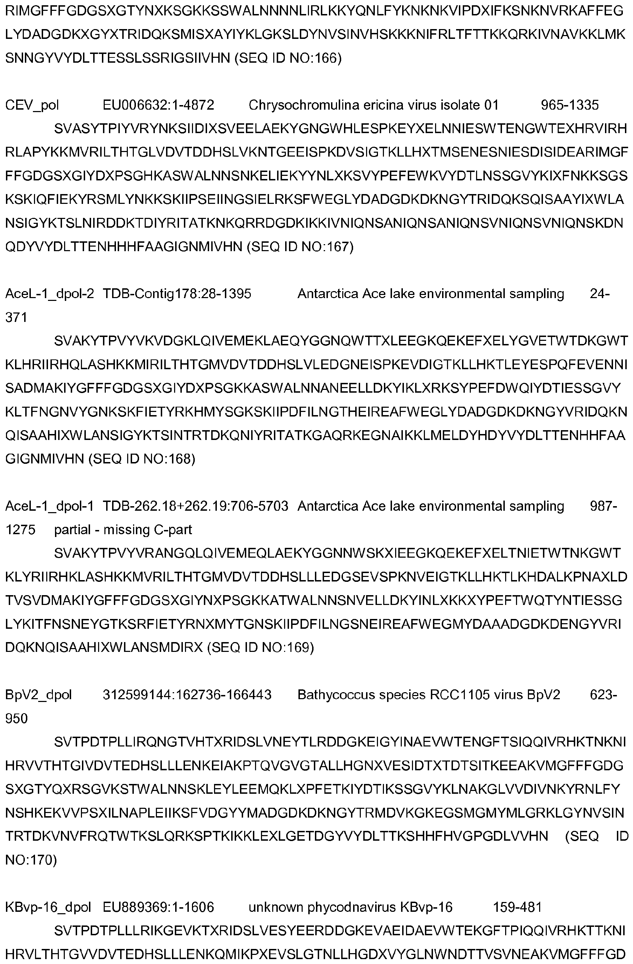

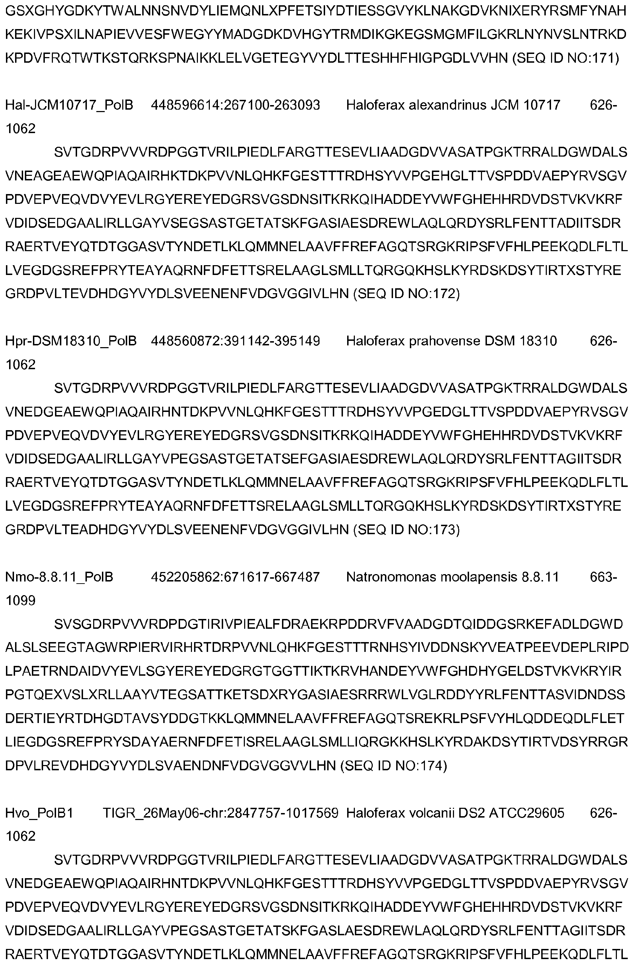

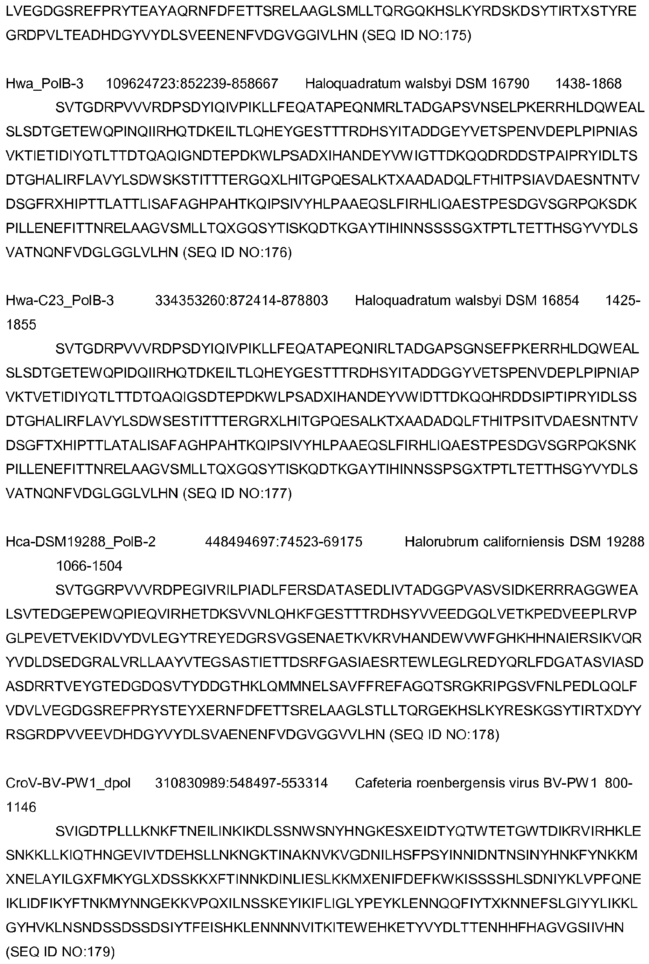

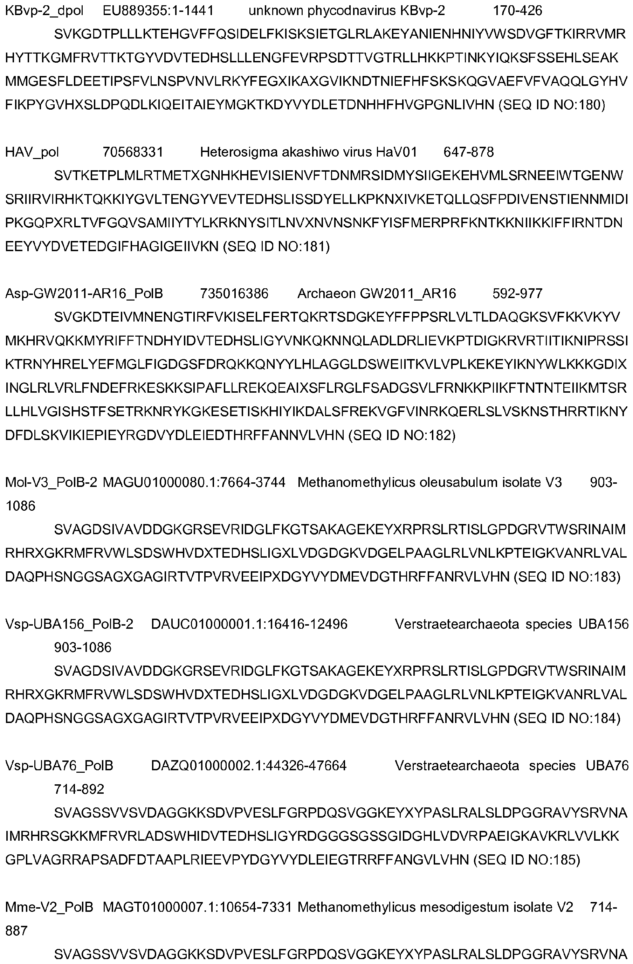

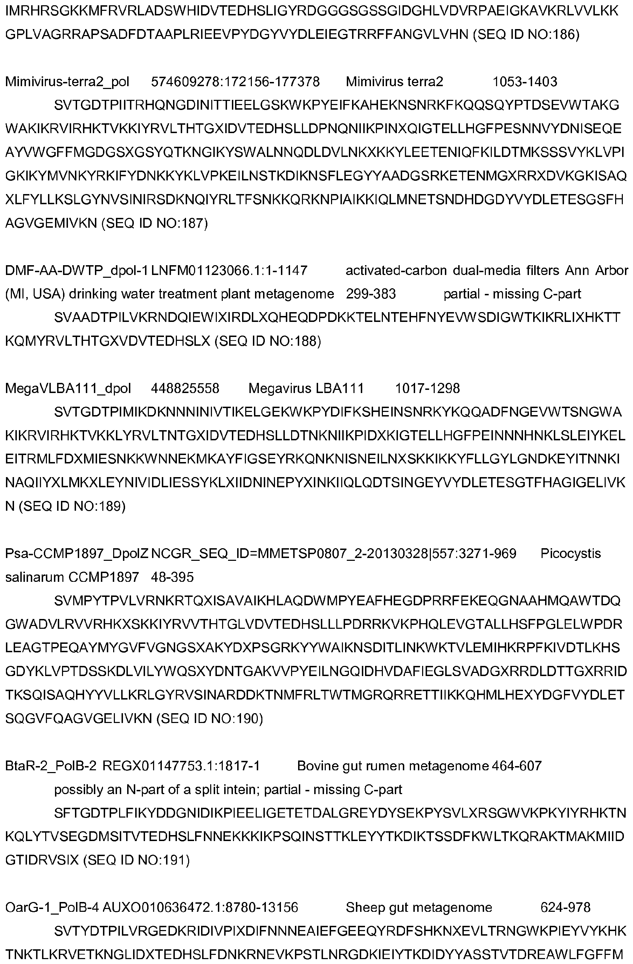

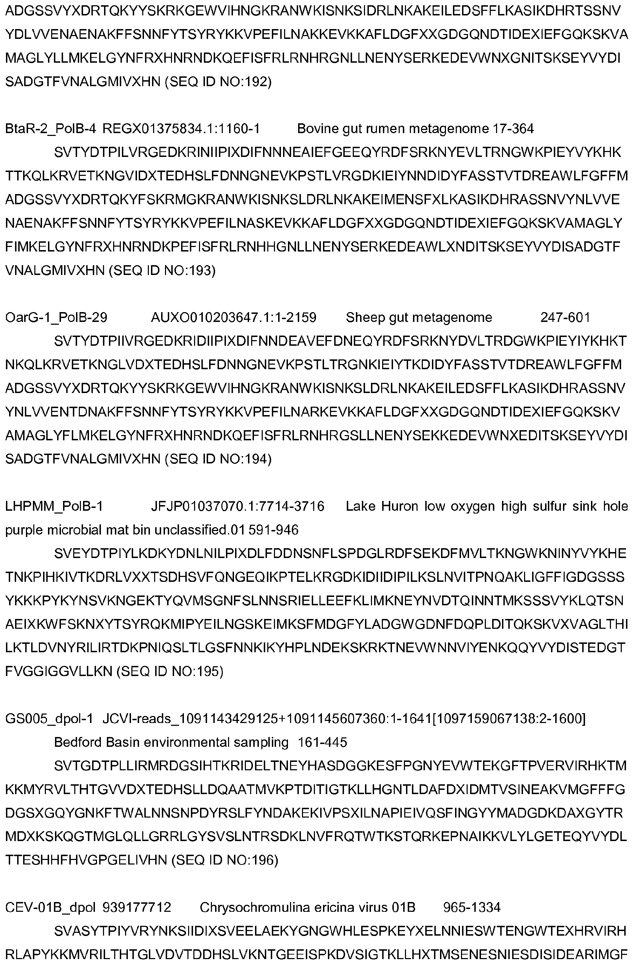

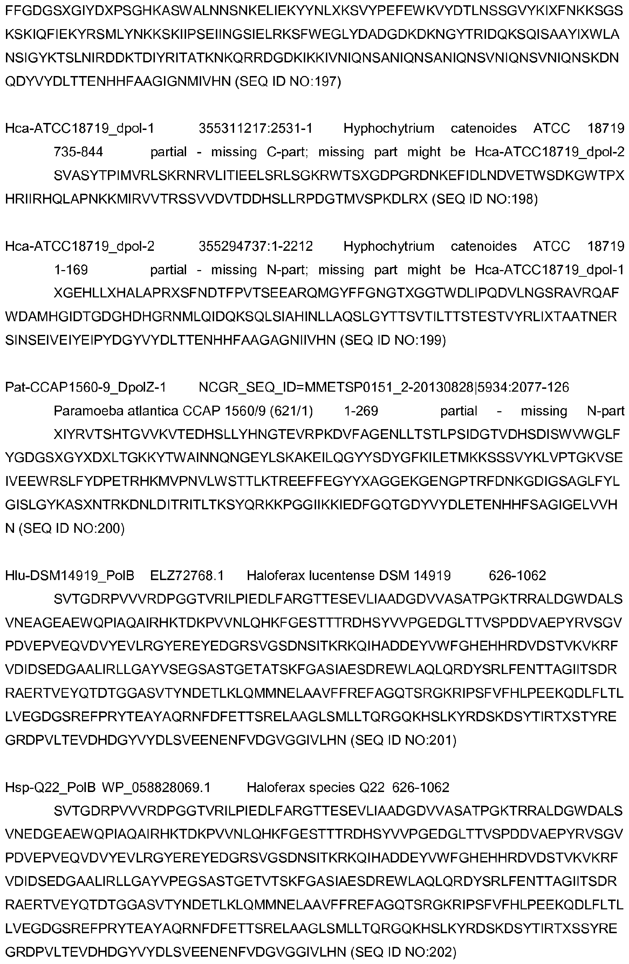

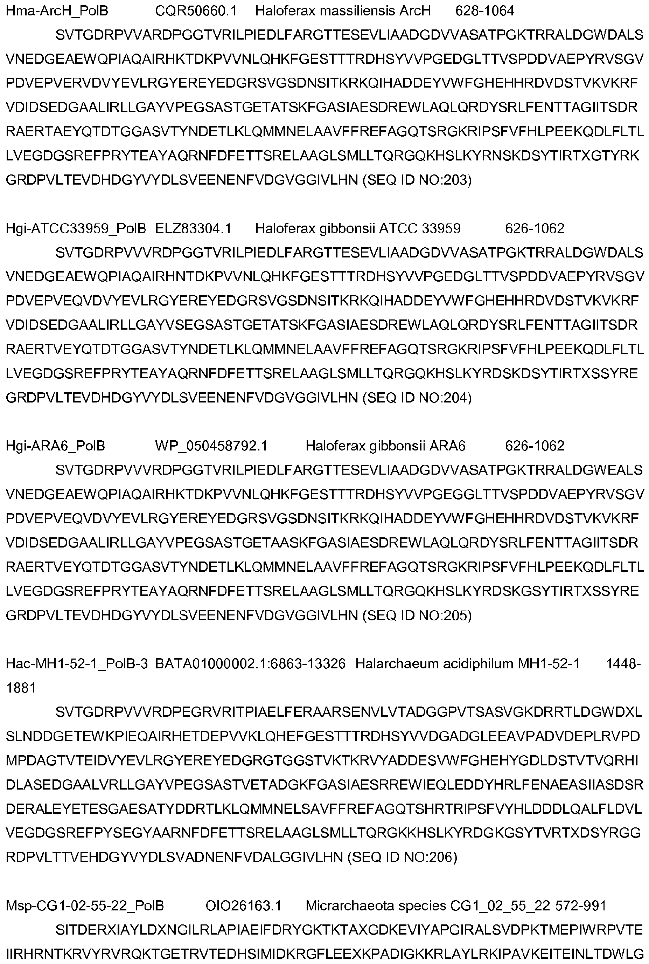

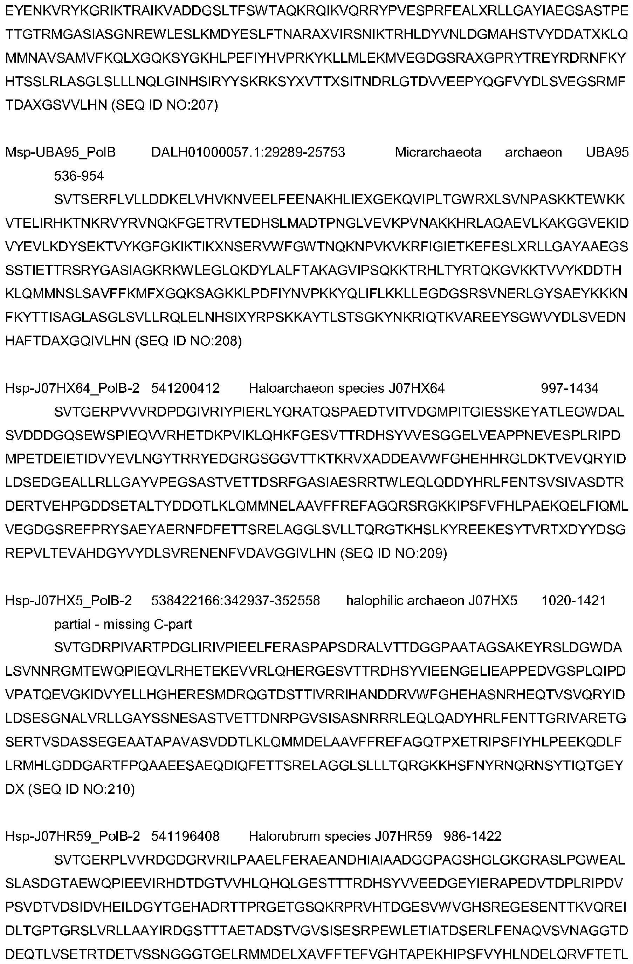

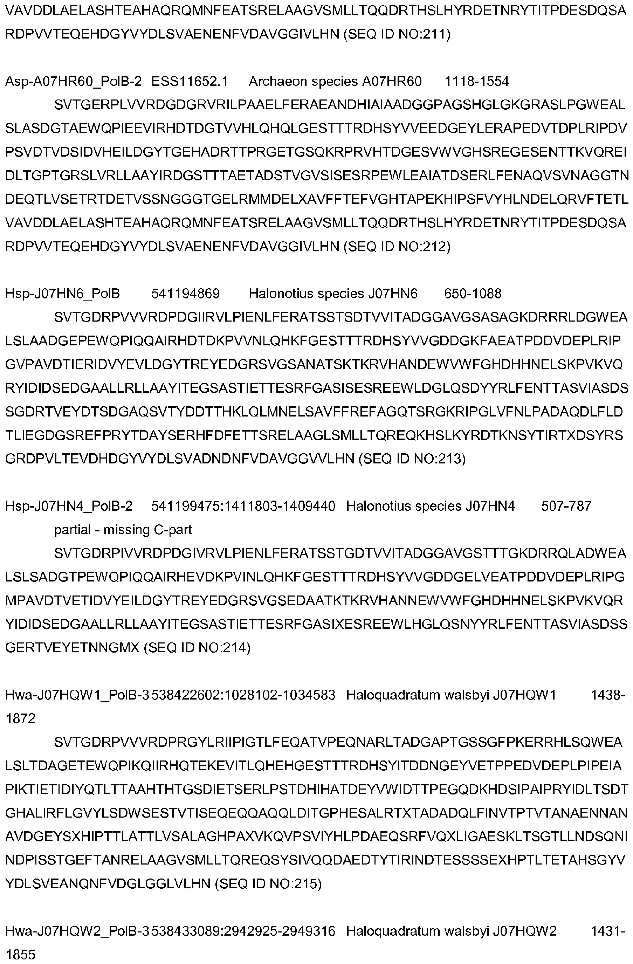

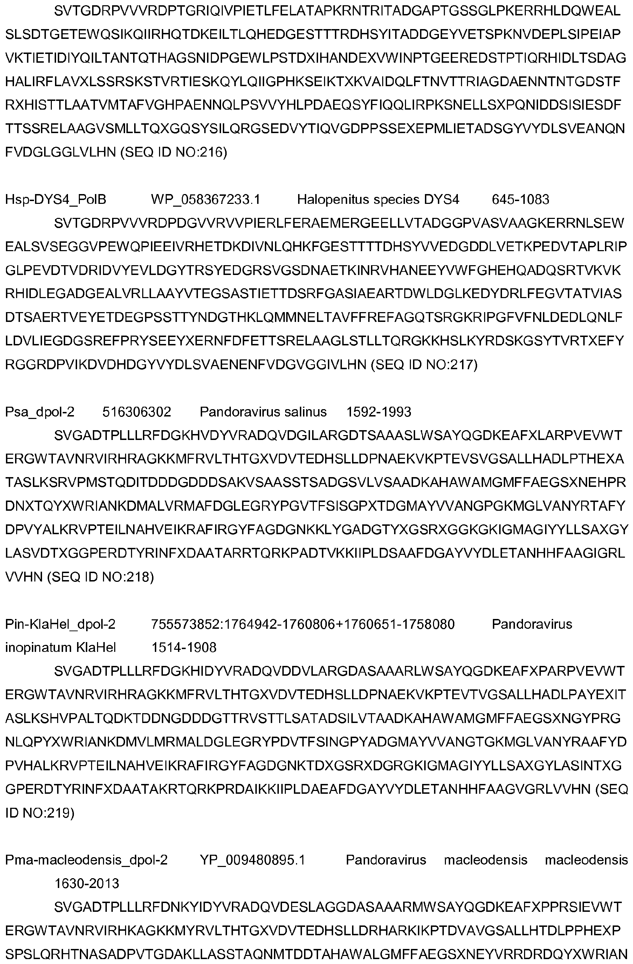

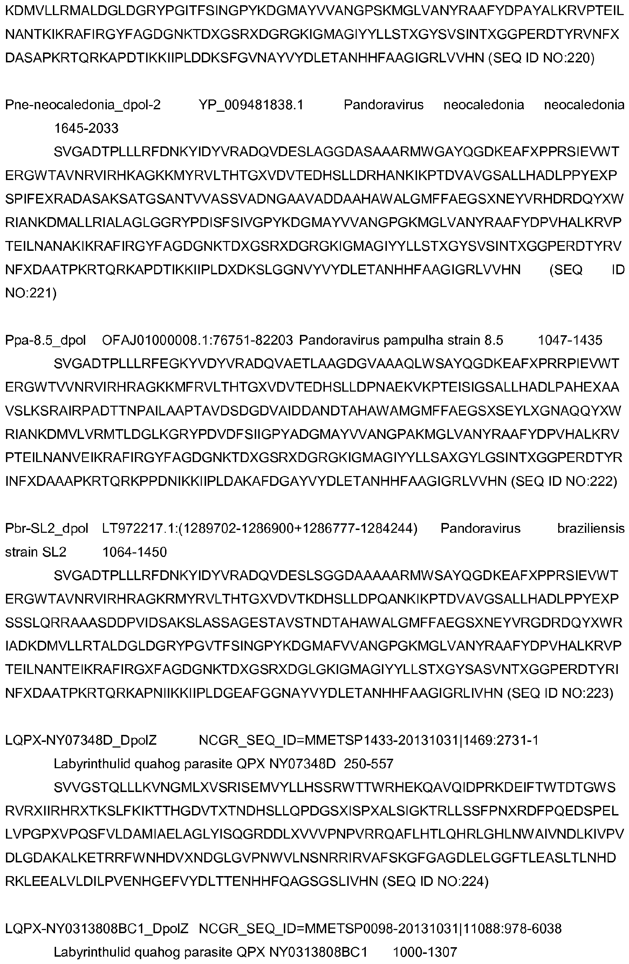

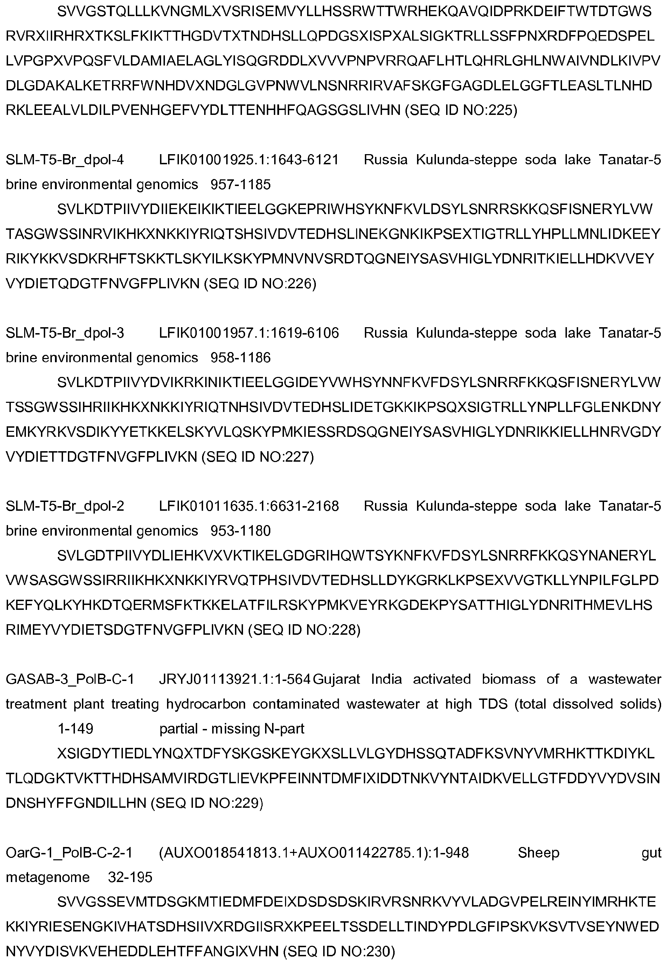

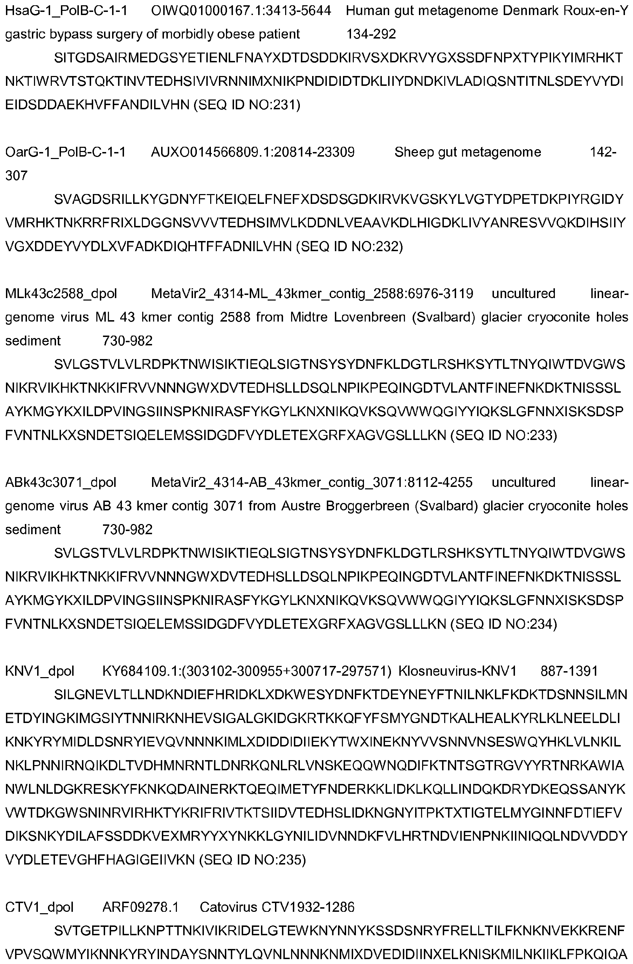

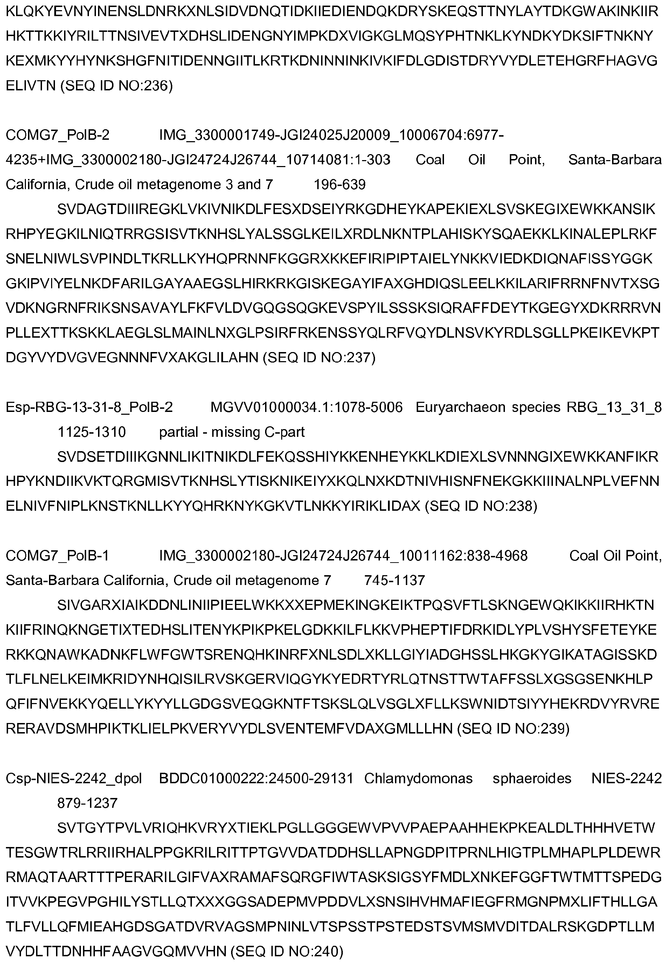

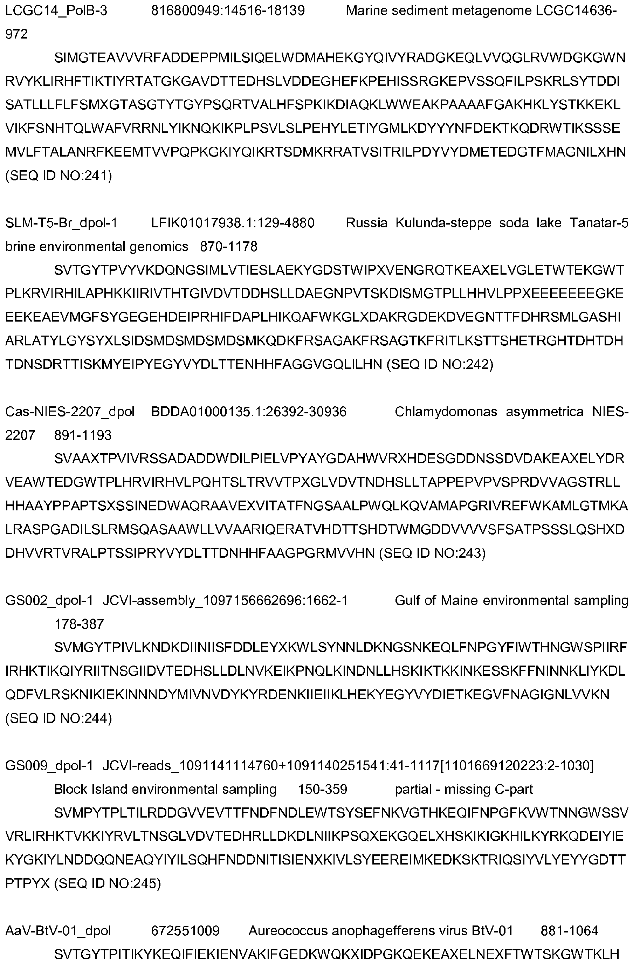

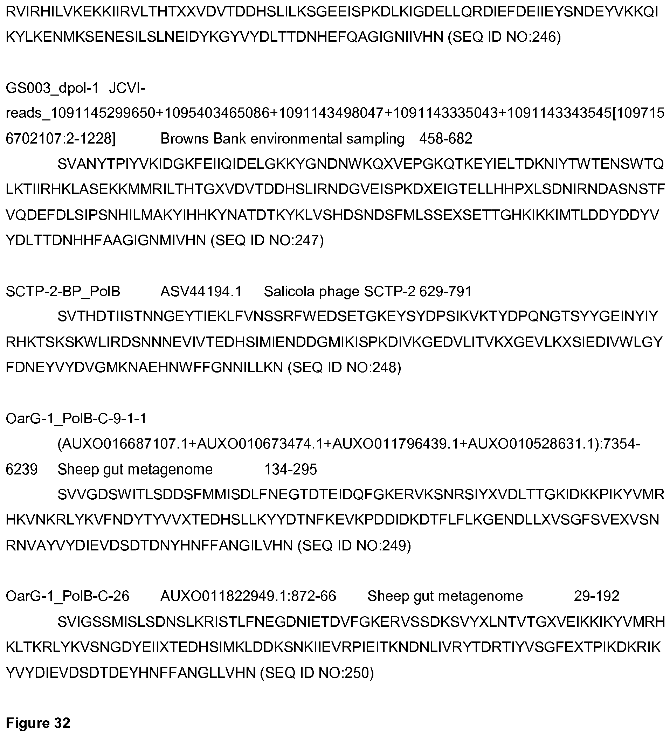

**Figure 32.** Complete and partial amino acid sequences of N-terminal and C-terminal sequences of inteins and amino acid sequences of complete and partial contiguous intein sequences of pol-c-type inteins, wherein X is any naturally occuring amino acid residue (with information of intein name, accession code, source (species or metagenome site), intein position in host, comments, aa sequence).

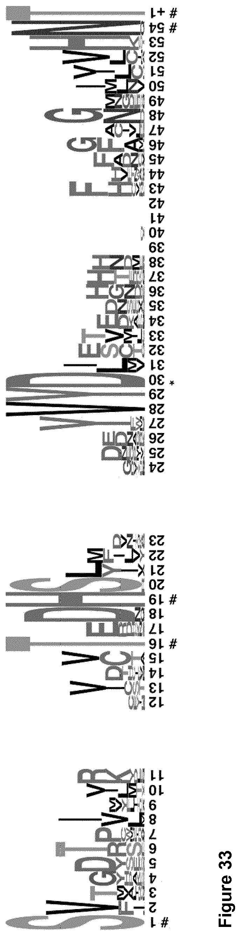

**Figure 33.** Sequence alignment of potential sequence motifs of pol-c type intein sequences of Figures 32.

## DETAILED DESCRIPTION OF THE INVENTION

The present invention is based on the unexpected discovery of the inventors that certain split inteins and genetically engineered intein variants, wherein the split inteins comprise a first intein amino acid sequence and a second intein amino acid sequence with the proviso that the catalytic motifs of the at least one first intein amino acid sequence and/or of the at least one second intein amino acid sequence are free of cysteine residues, are able to splice at ambient temperatures, preferably at temperatures under 40 °C, and/or under oxidizing conditions and/or in the absence of a denaturation step and/or they exhibit excellent splicing yields and rates.

Unless otherwise defined, all technical and scientific terms used herein have the same meaning as commonly understood by one of ordinary skill in the art. The singular terms "a", "an" and "the" include plural referents unless context clearly indicates otherwise. Similarly, the word "or" is intended to include "and" unless the context clearly indicates otherwise. The term "comprises" means "includes". In case of conflict, the present specification, including explanations of terms, will control.

The terms "one or more" or "at least one", as interchangeably used herein, relate to at least 1, or at least 2, 3, 4, 5, 6, 7, 8, 9, 10, 11, 12, 13, 14, 15, 20, 25 or a plurality of species, e.g. at least one first intein amino acid sequence. In this connection, the term "plurality" means more than one, preferably 2 or more, such as up to 1000.

Numeric values specified without decimal places here refer to the full value specified with one decimal place, i.e. for example, 99 % means 99.0 %, unless otherwise defined.

The terms "about" or "approximately" or "approx.", in connection with a numerical value, refer to a variance of $\pm10$ %, preferably $\pm5$ %, more preferably $\pm2$ %, more preferably $\pm1$ %, more preferably $\pm0.1$ %, and most preferably less than $\pm0.1$ %, with respect to the given numerical value.

When an amount, a concentration or other values or parameters (e.g. temperature) is/are expressed in form of a range, a preferable range, or a preferable upper limit value and a preferable lower limit value, it should be understood as that any ranges obtained by combining any upper limit or preferable value with any lower limit or preferable value are specifically disclosed, without considering whether the obtained ranges are clearly mentioned in the context.

"Essentially", for example in "essentially free of" typically means that little amounts of the compound can be present. Preferably, the compounds are comprised in amounts of less than 10 wt.-%, more preferably less than 5 wt.-%, more preferably less than 1 wt.-%, more preferably less than 0.1 wt. %, more preferably less than 0.01 wt.-%, more preferably less than 0.001 wt.-%, most preferably free of these compounds, if not explicitly stated otherwise. Similarly, if something "consists essentially of", the listed compound/component forms the major part, typically at least 50% or more.

In a first aspect the present invention relates to an isolated polypeptide comprising

(a) at least one first intein amino acid sequence (a1) and at least one first extein amino acid sequence (a2); wherein the at least one first intein amino acid sequence is an N-terminal sequence of a split intein or fragment thereof; wherein the at least one first extein sequence is fused to the N-terminus of the at least one first intein sequence and has a length of at least 1, preferably at least 5 amino acids; and

(b) at least one second intein amino acid sequence (b1) and at least one second extein sequence (b2); wherein the at least one second intein sequence is a C-terminal sequence of a split intein or fragment thereof; wherein the at least one second extein sequence is fused to the C-terminus of the at least one second intein sequence and has a length of at least 1, preferably at least 5 amino acids; wherein the catalytic motifs of the at least one first intein amino acid sequence and/or of the at least one second intein amino acid sequence are free of cysteine residues and wherein the first N-terminal amino acid of the second extein sequence (+1 position) is not a cysteine residue; wherein the first and second intein amino acid sequence interact with each other to catalyze the cleavage of the peptide bonds between the C-terminal amino acid of the first extein sequence and the N-terminal amino acid of the first intein sequence and the N-terminal amino acid of the second extein sequence and the C-terminal amino acid of the second intein sequence and to ligate the C-terminal amino acid of the first extein sequence to the N-terminal amino acid of the second extein sequence by a peptide bond.

In a second aspect, the invention relates to a composition comprising

(a) a first isolated polypeptide comprising at least one first intein amino acid sequence (a1) and at least one first extein amino acid sequence (a2); wherein the at least one first intein amino acid sequence is an N-terminal sequence of a split intein or fragment thereof; wherein the at least one first extein sequence is fused to the N-terminus of the at least one first intein sequence and has a length of at least 1, preferably at least 5 amino acids; and

(b) a second isolated polypeptide comprising at least one second intein amino acid sequence (b1), wherein the at least one second intein sequence is a C-terminal sequence of a split intein or fragment thereof and at least one second extein sequence (b2), wherein the at least one second extein sequence is fused to the C-terminus of the at least one second intein sequence and has a length of at least 1, preferably at least 5 amino acids;

wherein the catalytic motifs of the at least one first intein amino acid sequence and/or of the at least one second intein amino acid sequence are free of cysteine residues and wherein the first N-terminal amino acid of the second extein sequence (+1 position) is not a cysteine residue;

wherein the first and second intein amino acid sequence interact with each other to catalyze the cleavage of the peptide bonds between the C-terminal amino acid of the first extein sequence and the N-terminal amino acid of the first intein sequence and the N-terminal amino acid of the second extein sequence and the C-terminal amino acid of the second intein sequence and to ligate the C-terminal amino acid of the first extein sequence to the N-terminal amino acid of the second extein sequence by a peptide bond.

As used herein, the term "isolated polypeptide" refers to a polypeptide, peptide, protein segment or domain or fragments of any of the afore-mentioned, which has been separated from other components that accompany it in nature, e.g., proteins, RNA or DNA or other cellular components that naturally accompany it in a cell, for example by purification. Furthermore, in relation to polypeptide sequences, it can further mean that sequences, which flank it in a naturally occurring state, are no longer present, e.g. it can refer to a polypeptide fragment which has been excised from longer polypeptide sequences, in particular sequences which are normally present adjacent to the fragment in the naturally occurring protein. The isolated polypeptides may be artificial polypeptides in that they do not occur in nature but rather are engineered. The term includes, for example, a recombinant polypeptide, which is encoded by a nucleic acid incorporated into a vector, into an autonomously replicating plasmid or virus, or into the genomic DNA of a prokaryote or eukaryote, or which exists as a separate molecule (e.g., as a cDNA or a genomic or cDNA fragment produced by PCR or restriction enzyme digestion) independent of other sequences. It also includes a recombinant polypeptide, which is part of a hybrid polypeptide comprising additional amino acids.

Moreover, the isolated polypeptide(s) described herein or the at least one nucleic acid molecule encoding it/them may comprise, in addition to all features described herein, regulatory sequences, i.e. segments that on nucleic acid level are capable of increasing or decreasing the expression of specific genes within an organism or that on protein level regulate location, metabolism and the like.

The term "intein" as used herein refers to (a segment of) a protein capable of catalyzing a protein splicing reaction that excises the intein sequence from a precursor protein and joins the flanking sequences (N-and C-exteins) by a peptide bond. Intein and intein-like sequences have been found in a wide variety of organisms and proteins. They are typically 125-650 amino acids in size and may also contain a homing endonuclease domain.

The term "split intein" as used herein refers to any intein, which comprises an N-terminal intein sequence and a C-terminal intein sequence, wherein both sequences must be present in order to interact with each other in order to have (splicing) activity. Particularly, the term "split intein" refers to any intein, in which one or more peptide bond breaks exist between the N-terminal and C-terminal intein amino acid sequence such that the N-terminal and C-terminal sequences become separate fragments that can non-covalently reassociate, or reconstitute, into an intein (domain) that is functional for *trans*-splicing reactions. However, the term "split intein" also refers to a (contiguous) intein, which comprises the N-terminal and the C-terminal amino acid sequence in one contiguous amino acid sequence. Typically, in this case, the C-terminal intein amino acid sequence is linked over its N-terminal end to the C-terminal end of the N-terminal intein amino acid sequence by a peptide bond. Also inteins which comprise a linker sequence, for example of 1 to 10 amino acids in length, between the C-terminal end of the N-terminal intein amino acid sequence and the N-terminal end of the C-terminal intein amino acid sequence are comprised in the scope of the invention. In various non-limiting embodiments, the contiguous intein is functional for *cis*-splicing reactions. The N-terminal amino acid sequence of a split intein is also referred to herein as "first intein amino acid sequence", while the C-terminal amino acid sequence of a split intein is also referred to as "second intein amino acid sequence".

The term "intein fragment" as used herein refers to a separate molecule, e.g. the N-terminal sequence of a split intein or fragment thereof or the C-terminal sequence of a split intein or fragment thereof, typically resulting from peptide bond breaks between the N-terminal and C-terminal amino acid sequences in split inteins.

In preferred embodiments, the first intein amino acid sequence described herein is comparably short (e.g. $CL^N$), whereby the isolated polypeptides are ideally suited for use over a wide range of protein modifications, since the protein of interest-$Int^N$ (POI-$Int^N$) peptide complex can be easily obtained using solid-phase peptide synthesis.

In preferred embodiments according to the invention, the split site between the first intein amino acid sequence,

which is an N-terminal sequence of a split intein or fragment thereof and the second intein amino acid sequence, which is a C-terminal sequence of a split intein or fragment thereof is shifted in the engineered intein variant(s) (e.g. CL intein) in comparison to the wild type intein (e.g. Aes intein). This, preferably, results in a shorter Int$^N$ fragment and a longer Int$^C$ fragment. The described split intein sequences may thus be artificial in that they are split at a different position in relation to their naturally occurring counterpart.

In various non-limiting embodiments of some aspects of the present invention, the split takes place after amino acid 10 to 300, preferably after amino acid 11 to 250, more preferably after amino acid 12 to 200, more preferably after amino acids 25 to 200, more preferably after amino acid 26 to 120, more preferably after amino acid 10 or 11 or 12 or 26 or 120, most preferably after amino acid 26 or 120, as calculated from the intein's N-terminal end.

In a preferred embodiment of some aspects of the present invention, the N-terminal intein sequence is split from the C-terminal intein sequence after 10, 11, 12, 13, 14, 15, 16, 17, 18, 19, 20, 21 , 22, 23, 24, 25, 26, 27, 28, 29, 30, 31 , 32, 33, 34, 35, 36, 37, 38, 39, 40, 41 , 42, 43, 44, 45, 46, 47, 48, 49, 50, 51 , 52, 53, 54, 55, 56, 57, 58, 59 or 60 amino acids as calculated from the intein's N-terminal end. In an especially preferred embodiment, the N-terminal intein sequence is split from the C-terminal intein sequence after 10 to 40 amino acids, preferably after 11 to 40 amino acids, more preferably after 12 to 40 amino acids, more preferably after 25 to 40 amino acids, most preferably after 10, 11, 12 or 26 amino acids, preferably after 26 amino acids from the intein's N-terminal end.

In various non-limiting embodiments, the intein is a split intein with an N-terminal intein sequence of at least 10 or 11 or 14 amino acids, preferably at least 25 amino acids, most preferably 26 amino acids in length. Preferably, the N-terminal intein sequence is an engineered N-terminal intein sequence (e.g. CL$^N$), which is split after 10 to 40 or 11 to 40 or 12 to 40 or 25 to 40 amino acids, most preferably after 26 amino acids from the intein's N-terminal end. More preferably, the engineered N-terminal intein sequence comprises or consists of 26 amino acids in length.

Thus, for example, the N-terminal intein sequence of such an intein or fragment thereof and/or the protein of interest-Int$^N$ peptide complex is shorter and hence better suited for the chemical synthesis, e.g. for solid peptide synthesis, which is faster, easier to perform and much more reliable than protein generation via recombinant protein expression.

In another non-limiting embodiment, the N-terminal intein sequence is split after 100 to 200 amino acids, preferably after 120 amino acids. In various non-limiting embodiments, the intein is a split intein with an N-terminal intein sequence of 120 amino acids in length.

In various non-limiting embodiments of aspect 1 or 2 of the present invention, the intein is a split intein with a C-terminal intein sequence of at least 6 amino acids, preferably of at least 24 amino acids, more preferably of at least 30 amino acids, preferably 39 or 129 amino acids in length.

Preferably, the C-terminal intein sequence is an engineered C-terminal intein sequence (e.g. CL$^C$) and comprises or consists of at least 6 amino acids, more preferably of at least 24 amino acids, more preferably of at least 30 amino acids, more preferably of 129 amino acids in length.

As interchangeably used herein, the terms "N-terminal split intein", "N-terminal intein fragment" and "N-terminal intein sequence" (abbreviated "Int$^N$")" refer to any sequence that comprises an N-terminal amino acid sequence of a split intein or fragment thereof that is functional for (trans-)splicing reactions. Preferably, they refer to the first intein amino acid sequence (a1) as described above. It can comprise a sequence that is a modification of the N-terminal portion of a naturally occurring intein sequence. For example, it can comprise additional amino acid residues and/or mutated residues and/or residues may be deleted as long as the inclusion, modification or deletion of such residues does not render the Int$^N$ non-functional in (trans-)splicing. Preferably, the inclusion, modification or deletion of such residues improves or enhances the (trans-)splicing activity of the Int$^N$.

As interchangeably used herein, the terms "C-terminal split intein", "C-terminal intein fragment" and "C-terminal intein sequence" (abbreviated "Int$^C$")" refer to any sequence that comprises a C-terminal amino acid sequence of a split intein or fragment thereof that is functional for (trans-)splicing reactions. Preferably, they refer to the second intein amino acid sequence (b1) as described above. An Int$^C$ can comprise a sequence that is a modification of the C-terminal portion of a naturally occurring intein sequence. For example, it can comprise additional amino acid residues and/or mutated residues and/or residues may be deleted as long as the inclusion, modification or deletion of such residues does not render the Int$^C$ non-functional in (trans-splicing). Preferably, the inclusion, modification or deletion of such residues improves or enhances the (trans-)splicing activity of the Int$^C$.

Amino acids which may be any naturally occuring amino acid typically include the amino acids: alanine (A), arginine (R), histidine (H), lysine (K), aspartic acid (D), glutamic acid (E), serine (S), threonine (T), asparagine (N), glutamine (Q), cysteine (C), glycine (G), proline (P), valine (V), isoleucine (I), leucine (L), methionine (M), phenylalanine (F), tyrosine (Y), tryptophan (W), selenocysteine (U) and pyrrolysine (O) and natural derivatives thereof, if not explicity stated otherwise.

It is also included within the scope of the invention, but not preferred, that one or more amino acids of N-terminal and/or C-terminal intein amino acid sequences may be unnatural amino acids, preferably in chemically synthetized polypeptides and proteins. This is typically referred to as "genetic code expansion" or "amber stop codon suppression". Suitable unnatural amino acids are, for example, mentioned in the list of reference (71) and their possible synthesis and incorporation is described in the corresponding text of reference (71) and in reference (72), which are hereby incorporated by reference.

In preferred embodiments, amino acids which may be any naturally occuring amino acid include only the 20 proteinogenic amino acids (A), arginine (R), histidine (H), lysine (K), aspartic acid (D), glutamic acid (E), serine (S), threonine (T), asparagine (N), glutamine (Q), cysteine (C), glycine (G), proline (P), valine (V), isoleucine (I), leucine (L), methionine (M), phenylalanine (F), tyrosine (Y) and tryptophan (W), and natural derivatives thereof, if not explicity stated otherwise. It is understood that when a sequence is defined to be cysteine-free that "any amino acid" does not include cysteine.

Typically, one or more sequence motifs of the first and/or second intein amino acid sequence, in particular the catalytic motifs, are free of cysteine residues. In these cases, the term "any naturally occuring amino acid" refers to the 19 proteinogenic amino acids alanine, arginine, histidine, lysine, aspartic acid, glutamic acid, serine, threonine, asparagine, glutamine, glycine, proline, valine, isoleucine, leucine, methionine, phenylalanine, tyrosine and tryptophan and derivatives thereof, excepting cysteine and derivatives thereof. In most preferred embodiments, all sequence motifs of the first and the second intein amino acid sequences are free of cysteine residues.

In cases in which an amino acid is "not present", the adjacent amino acids left and right of the amino acid which is not present are linked to each other by a peptide bond, of not explicitly stated otherwise. "Not present" is thus tantamount to a peptide bond between the adjacent amino acids of the missing/absent amino acid(s).

It is a proviso of the present invention that the catalytic motifs of the at least one first intein amino acid sequence and/or of the at least one second intein amino acid sequence are free of cysteine residues.

In pol-e type intein sequences, typically one or more of the following sequence motifs may be present in the first or second intein amino acid sequence:

In various non-limiting embodiments, the catalytic motif(s) of the at least one first intein amino acid sequence comprise(s) the amino acid sequence (A)

$X_1X_2X_3X_4X_5X_6X_7X_8X_9X_{10}X_{11}$ (A)

wherein

$X_1$ is S or T, most preferably S;

$X_2$ is V, C, F, Q, I, T, S, L, G or A, preferably V, C, F, Q, I or T, more preferably V, F, Q, I or T, most preferably V;

$X_3$ is V, T, L, S, I, N, D, A, Q, R, H, F, E, Y, C, K or P, preferably V, D, S, T, L or A, more preferably V, D or S, most preferably V or D;

$X_4$ is any naturally occuring amino acid or not present, preferably G, A, W, E, S, T, K, N, F, H, Y, R, C, Q, M or not present, more preferably G, A, W, S, F, M or K, more preferably G, A, S, F or K, most preferably G;

$X_5$ is D, S, K, N, L, E, C, T, A, Q or I, preferably D, S, K, N, Q or L, most preferably D or N;

$X_6$ is T, S, V, A, C, M or E, preferably T, S, E or C, more preferably T or S, most preferably T;

$X_7$ is I, Q, S, K, E, T, L, V, H, C, F, A, M, N, Y or P, preferably I, L, Q, K or P, more preferably I, L, Q or K, most preferably I or L;

$X_8$ is any naturally occuring amino acid or not present, preferably I, V, F, L, N, M or not present, more preferably I, V, F, L or M, more preferably I, V, F or L, most preferably I or L;

$X_9$ is D, N, V, R, F, Y, Q, E, K, C, S, I, M, H, W or L, preferably D, Y, K, R, N, L or V, most preferably D or Y;

$X_{10}$ is any naturally occuring amino acid or not present, preferably V, I, L, Y, T, A, C, W or not present, more preferably V, I, L, T, C or W, more preferably V, I, L or T, more preferably V, I or T, most preferably V or I; and

$X_{11}$ is S, N, D, R, K, E, T, M, G, I or H, preferably S, N, D, R, K, E or H, preferably S, N, D, R or K, more preferably S, N or D, most preferably S or N;

wherein the amino acid sequence (A) comprises or consists of at least 8 amino acids ($X_1$-$X_3$, $X_5$-$X_7$, $X_9$, $X_{11}$), preferably at least 9 amino acids ($X_1$-$X_3$, $X_5$-$X_7$, $X_9$-$X_{11}$) or 10 amino acids ($X_1$-$X_3$, $X_5$-$X_{11}$ or $X_1$-$X_9$, $X_{11}$), most preferably it comprises or consists of 11 amino acids ($X_1$-$X_{11}$).

In various preferred embodiments, amino acid sequence (A) is free of cysteine residues.

In various non-limiting embodiments, the catalytic motif(s) of the at least one second intein amino acid sequence comprise(s) the amino acid sequence (B1)

$X_{45}X_{46}X_{47}X_{48}X_{49}X_{50}X_{51}X_{52}X_{53}X_{54}X_{55}X_{56}X_{57}$ (B1)

wherein

$X_{45}$ is N, C, Y, T, D, P, S, H, A, G, M or R, preferably N, C, T, S, or H, more preferably N, T or S, most preferably T or N;

$X_{46}$ is H, P, S or F, preferably H, F or P, most preferably H;

$X_{47}$ is N, T, C, Y, M, V, A, S or H, preferably N, T, Y, M, A or H, most preferably N or T;

$X_{48}$ is F or A, preferably F;

$X_{49}$ is F, V, Y, I or G, preferably F, V, Y or I, most preferably F;

$X_{50}$ is G, A, C or I, preferably G or A;

$X_{51}$ is N, S, D or G, preferably N or G, most preferably N;

$X_{52}$ is D, G, N, E, T, S or R, preferably D, N, G or R, more preferably D, N or G, more preferably D or N, most preferably D;

$X_{53}$ is I, V, M or T, preferably I, M or V, more preferably I or V, most preferably I;

$X_{54}$ is L, C, Y, I or V, preferably L;

$X_{55}$ is V, A, I or L, preferably V;

$X_{56}$ is H; and

$X_{57}$ is N;

wherein the amino acid sequence (B1) comprises or consists of at least 13 amino acids ($X_{45}$-$X_{57}$).

In various preferred embodiments, amino acid sequence (B1) is free of cysteine residues.

In various non-limiting embodiments, the amino acid sequence (B1) can also be amino acid sequence (B1*)

$X_{44}X_{45}X_{46}X_{47}X_{48}X_{49}X_{50}X_{51}X_{52}X_{53}X_{54}X_{55}X_{56}X_{57}$ (B1*)

wherein

$X_{44}$ is D, E, C, N, S, T, G, L or H, preferably D, E or N; and wherein $X_{45}$-$X_{57}$ are as described above.

In various non-limiting embodiments, the catalytic motif(s) of the at least one second intein amino acid sequence comprise(s) the amino acid sequence (B2)

$X_{28}X_{29}X_{30}X_{31}X_{32}X_{33}X_{34}X_{35}X_{36}X_{37}$ (B2)

wherein

$X_{28}$ is I, L, E, V, Q, D or N, preferably I, L, E, Q, D or N, more preferably I, E or D, most preferably I or E;

$X_{29}$ is D, Y, W, F, P, I or M, preferably D, Y or W, most preferably D or Y;

$X_{30}$ is V or A, preferably V;

$X_{31}$ is Y or F, preferably Y;

$X_{32}$ is D;

$X_{33}$ is I, V, L, C, A or M, preferably I, V, L or C, more preferably I, V or L, most preferably I;

$X_{34}$ is E, S, G or V, preferably E;

$X_{35}$ is any naturally occuring amino acid or not present, preferably V, M, I, T or not present, more preferably V or M, most preferably V;

$X_{36}$ is any naturally occuring amino acid or not present, preferably D, E, Q, P, K, F, A, N, G or not present, more preferably D or E, most preferably D; and

$X_{37}$ is any naturally occuring amino acid or not present, preferably G, E, D, K, T, N, S or not present, more preferably G, E, D or N, more preferably G, E or D, most preferably G or D;

wherein the amino acid sequence (B2) comprises or consists of at least 7 amino acids ($X_{28}$-$X_{34}$), preferably at least 8 amino acids ($X_{28}$-$X_{34}$, $X_{37}$ or $X_{28}$-$X_{35}$) or at least 9 amino acids ($X_{28}$-$X_{35}$, $X_{37}$ or $X_{28}$-$X_{36}$), most preferably at least 10 amino acids ($X_{28}$-$X_{37}$).

In various preferred embodiments, amino acid sequence (B2) is free of cysteine residues.

In various non-limiting embodiments, the amino acid sequence (B2) can also be amino acid sequence (B2*)

$X_{24}X_{25}X_{26}X_{27}X_{28}X_{29}X_{30}X_{31}X_{32}X_{33}X_{34}X_{35}X_{36}X_{37}X_{38}$ (B2*)

wherein

$X_{24}$ is F, A, E, I, V, D, Y, K, M, P, T, W, C, S, Q or not present, preferably F, I, Y, M, T or not present; $X_{25}$ is D, T or not present;

$X_{26}$ is N, Q, E, D, V, K, I, R, T, P, C, L or not present, preferably N, Q, E, D, K, T, C or not present;

$X_{27}$ is D, P, N, E, F, K, C, G or not present, preferably D, N, E, C or not present;

$X_{38}$ is P, T, D, S, G or not present, preferably P, D, S or not present; and wherein $X_{28}$-$X_{37}$ are as described above.

In various non-limiting embodiments, it is also possible that the amino acid sequence B1 and the amino acid sequence B2 form a combined sequence motif (B)

$X_{24}X_{25}X_{26}X_{27}X_{28}X_{29}X_{30}X_{31}X_{32}X_{33}X_{34}X_{35}X_{36}X_{37}X_{38}X_{39}X_{40}X_{41}X_{42}X_{43}X_{44}X_{45}X_{46}X_{47}X_{48}X_{49}$

$X_{50}X_{51}X_{52}X_{53}X_{54}X_{55}X_{56}X_{57}$     (B)

wherein

$X_{24}$ is F, A, E, I, V, D, Y, K, M, P, T, W, C, S, Q or not present, preferably F, I, Y, M, T or not present; X25 is D, T or not present;

$X_{26}$ is N, Q, E, D, V, K, I, R, T, P, C, L or not present, preferably N, Q, E, D, K, T, C or not present;

$X_{27}$ is D, P, N, E, F, K, C, G or not present, preferably D, N, E, C or not present;

$X_{28}$ is I, L, E, V, Q, D or N, preferably I, L, E, Q, D or N, more preferably I, E or D, most preferably I or E;

$X_{29}$ is D, Y, W, F, P, I or M, preferably D, Y or W, most preferably D or Y;

$X_{30}$ is V or A, preferably V;

$X_{31}$ is Y or F, preferably Y;

$X_{32}$ is D;

$X_{33}$ is I, V, L, C, A or M, preferably I, V, L or C, more preferably I, V or L, most preferably I;

$X_{34}$ is E, S, G or V, preferably E;

$X_{35}$ is any naturally occuring amino acid or not present, preferably V, M, I, T or not present, more preferably V or M, most preferably V;

$X_{36}$ is any naturally occuring amino acid or not present, preferably D, E, Q, P, K, F, A, N, G or not present, more preferably D or E, most preferably D;

$X_{37}$ is any naturally occuring amino acid or not present, preferably G, E, D, K, T, N, S or not present, more preferably G, E, D or N, more preferably G, E or D, most preferably G or D;

$X_{38}$ is P, T, D, S, G or not present, preferably P, D, S or not present, more preferably P or not present; $X_{39}$ is E, I or not present, preferably I or not present;

$X_{40}$ is N, I or not present, preferably N or not present;

$X_{41}$ is D or not present, preferably not present;

$X_{42}$ is V or not present, preferably not present;

$X_{43}$ is E or not present, preferably not present;

$X_{44}$ is D, E, C, N, S, T, G, L, H or not present, preferably D, E, N, S, H or not present, more preferably D, E or not present, more preferably D or not present;

$X_{45}$ is N, C, Y, T, D, P, S, H, A, G, M or R, preferably N, C, T, S, or H, more preferably N, T or S, most preferably T or N;

$X_{46}$ is H, P, S or F, preferably H, F or P, most preferably H;

$X_{47}$ is N, T, C, Y, M, V, A, S or H, preferably N, T, Y, M, A or H, most preferably N or T;

$X_{48}$ is F or A, preferably F;

$X_{49}$ is F, V, Y, I or G, preferably F, V, Y or I, most preferably F;

$X_{50}$ is G, A, C or I, preferably G or A;

$X_{51}$ is N, S, D or G, preferably N or G, most preferably N;

$X_{52}$ is D, G, N, E, T, S or R, preferably D, N, G or R, more preferably D, N or G, more preferably D or N, most preferably D;

$X_{53}$ is I, V, M or T, preferably I, M or V, more preferably I or V, most preferably I;

$X_{54}$ is L, C, Y, I or V, preferably L;

$X_{55}$ is V, A, I or L, preferably V;

$X_{56}$ is H; and

$X_{57}$ is N;

wherein the amino acid sequence (B) comprises or consists of at least 7 amino acids ($X_{28}$-$X_{34}$), preferably at least 17 amino acids ($X_{28}$-$X_{34}$ and $X_{45}$-$X_{54}$), more preferably at least 20 amino acids ($X_{28}$-$X_{34}$ and $X_{45}$-$X_{57}$), most preferably at least 23 amino acids ($X_{28}$-$X_{37}$ and $X_{45}$-$X_{57}$).

In various preferred embodiments, amino acid sequence (B) is free of cysteine residues.

In various non-limiting embodiments, the catalytic motif(s) of the first or the second intein amino acid sequence comprise(s) the amino acid sequence (C)

$X_{12}X_{13}X_{14}X_{15}X_{16}X_{17}X_{18}X_{19}X_{20}X_{21}X_{22}X_{23}$ (C)

wherein

$X_{12}$ is any naturally occuring amino acid or not present, preferably E, K, T, M, S, P, F, D, V, C, Q, A, Y or not present, more preferably E, C, S, V or P, more preferably E, S, V or P, most preferably E;

$X_{13}$ is V, L, T or I, most preferably V or I;

$X_{14}$ is I, T, E, V, L, F, K, D, S, Y, M or H, preferably I, T, E, V, Y or H, more preferably I, T, E or V, most preferably I;

$X_{15}$ is V, M, I, L, T, C or A, preferably V, T, C or I, more preferably V or I, most preferably V;

$X_{16}$ is T or S, preferably T;

$X_{17}$ is A, E, C, G, N, Q, D, T or S, preferably A, E, C, G, N or T, more preferably A, E, G, N or T, most preferably A, E or G;

$X_{18}$ is D, E or N, preferably D;

$X_{19}$ is H;

$X_{20}$ is S, C, G, I, V or N, preferably S, C, V or N, more preferably S or V, most preferably S;

$X_{21}$ is V, I, L, M, C, or F, preferably V, I, L, M or C, more preferably V, I, C or L, more preferably V, I or L, most preferably V;

$X_{22}$ is any naturally occuring amino acid or not present, preferably M, I, K, V, L or not present, more preferably M, I or V, most preferably M; and

$X_{23}$ is any naturally occuring amino acid or not present, preferably V, I, K, R, A, D or not present, more preferably, V, I, D, R or A, more preferably V, I or A, most preferably V,

wherein the amino acid sequence (C) comprises or consists of at least 9 amino acids ($X_{13}$-$X_{21}$), preferably at least 10 amino acids ($X_{12}$-$X_{21}$) or at least 11 amino acids ($X_{13}$-$X_{23}$), most preferably 12 amino acids ($X_{12}$-$X_{23}$).

In various preferred embodiments, the amino acid sequence (C) is free of cysteine residues.

The terms "any naturally occuring amino acid" or "not present" are as defined above.

In pol-c type intein sequences, typically one or more of the following sequence motifs may be present in the first or second intein amino acid sequence:

In various non-limiting embodiments, the catalytic motif(s) of the at least one first intein amino acid sequence comprise(s) the amino acid sequence (A')

$X_1 X_2 X_3 X_4 X_5 X_6 X_7 X_8 X_9 X_{10} X_{11}$ (A')

wherein

$X_1$ is S;

$X_2$ is V, F, I or L, preferably V, F or I, most preferably V;

$X_3$ is G, A, T, M, L, E, S, V, D, N, I or K, preferably A, T, M, L or V, most preferably T;

$X_4$ is K, G, A, P, S, E, Y, H, D or N, preferably K, G, A, P, Y or H, most preferably G;

$X_5$ is D, S, Y, E, G, N, A, T or C, preferably D, S, Y, E or C, more preferably D, S, Y or E, most preferably D;

$X_6$ is T, S, E or R, preferably T, S or R, more preferably T or S, most preferably T;

$X_7$ is E, I, V, P, Q, L, A, R, D, C, F, W or M, preferably I, V, P, Q, L, C, W or M, more preferably I, V, P, Q, L, W or M, more preferably I, V, P or L, most preferably P;

$X_8$ is I, V, L or M, preferably I, V or L, most preferably I;

$X_9$ is V, A, S, I, M, L, F, Y, T or R, preferably V, I, M, L, F, Y or T, more preferably V, I, M, L or Y, most preferably V, I or L;

$X_{10}$ is M, V, T, I, L, A or Y, preferably M, V, T, I or L, more preferably V, I or L, most preferably V; and $X_{11}$ is any naturally occuring amino acid, preferably N, R, K, Y, S, G, D, E, L or not present, preferably R, K, Y, D or L, more preferably R or K, most preferably R;

wherein the amino acid sequence (A') comprises or consists of at least 10 amino acids ($X_1$-$X_{10}$), preferably it comprises or consists of 11 amino acids ($X_1$-$X_{11}$).

In various preferred embodiments, the amino acid sequence (A') is free of cysteine residues.

In various non-limiting embodiments, the catalytic motif(s) of the at least one second intein amino acid sequence comprise(s) the amino acid sequence (B1')

$X_{43} X_{44} X_{45} X_{46} X_{47} X_{48} X_{49} X_{50} X_{51} X_{52} X_{53} X_{54}$ (B1')

wherein

$X_{43}$ is any naturally occuring amino acid or not present, preferably R, C, F, V, L, P, H, N or not present, more preferably C, F, V or H, more preferably F, V or H, most preferably F;

$X_{44}$ is any naturally occuring amino acid or not present, preferably F, H, Q, V, I, S, A, N, Y, C, T, W or not present, more preferably F, H, V, A, N, C, T or W, more preferably F, H, V, N or A, most preferably F, H, A or V;

$X_{45}$ is F, Y, A, V, N, G, S, I, C or D, preferably F, A, N, C or D, most preferably F, A, N or D;

$X_{46}$ is any naturally occuring amino acid or not present, preferably G, A, R, F, M or not present, preferably G, A or F, most preferably G;

$X_{47}$ is A, V, I, P, L, S, F, G, K or C, preferably A, V, I, P, L or C, more preferably A, V, I, L or C, most preferably A, V, I or L;

$X_{48}$ is any naturally occuring amino acid or not present, preferably N, G, D or not present, most preferably N or G;

$X_{49}$ is N, D, R, G, E, L, M, S, P or Q, preferably N, D, G, E or M, most preferably N, G, E or M;

$X_{50}$ is V, I, M, L or N, preferably I, M or L, most preferably I or L;

$X_{51}$ is L, I, V, D or C, preferably L, I, V or C, most preferably L, I or V;

$X_{52}$ is V, I, N, L, C or A, preferably V, L or C, most preferably V or L;

$X_{53}$ is H, K, S or T, preferably H or K, most preferably H; and

$X_{54}$ is N or Q, preferably N;

wherein the amino acid sequence (B1') comprises or consists of at least 8 amino acids ($X_{45}$, $X_{49}$-$X_{54}$), preferably at least 10 amino acids ($X_{45}$-$X_{54}$) or at least 11 amino acids ($X_{43}$-$X_{45}$, $X_{47}$-$X_{54}$, or $X_{43}$-$X_{47}$, $X_{49}$-$X_{54}$), most preferably it comprises or consists of 12 amino acids ($X_{43}$-$X_{54}$).

In various preferred embodiments, the amino acid sequence (B1') is free of cysteine residues.

In various non-limiting embodiments, the catalytic motif(s) of the at least one second intein amino acid sequence comprise(s) the amino acid sequence (B2')

$X_{27}X_{28}X_{29}X_{30}X_{31}X_{32}X_{33}X_{34}X_{35}X_{36}X_{37}$ (B2')

wherein

$X_{27}$ is D, E, Y, F, V, T, M or W, preferably Y, F, M or W, most preferably Y;

X28 is V;

$X_{29}$ is Y;

$X_{30}$ is D;

$X_{31}$ is L, I, M or V, preferably L or I, most preferably L;

$X_{32}$ is E, T, S, Q, C or G, preferably E, T, S or C, more preferably E, T or S, most preferably E or S;

$X_{33}$ is I, L, M, V, T, C or A, preferably I, L, M, V, T or C, more preferably I, L, M, V or T, most preferably VorT;

$X_{34}$ is any naturally occuring amino acid or not present, preferably E, P, D, G, S, A, Q, T, V, K, N, F, G, R or not present, more preferably E, D, A or K, most preferably E;

$X_{35}$ is any naturally occuring amino acid or not present, preferably V, D, S, Q, N, R, A, C, H, G, E, T or not present, more preferably D, S, N, C or E, more preferably D, S, N or E, most preferably D or N,

$X_{36}$ is any naturally occuring amino acid or not present, preferably D, G, H, Q, N, E, T, S, A or not present, more preferably D, G, H, Q or N, more preferably G, H or N, most preferably G or H,

$X_{37}$ is any naturally occuring amino acid or not present, preferably T, D, S, H, V, E, R, Q, K, N, I or not present, more preferably T, D, H or E, most preferably T or H;

wherein the amino acid sequence (B2') comprises or consists of at least 7 amino acids ($X_{27}$-$X_{33}$), preferably at least 9 amino acids ($X_{27}$-$X_{35}$) or at least 10 amino acids ($X_{27}$-$X_{33}$, $X_{35}$-$X_{37}$ or $X_{27}$-$X_{35}$, $X_{37}$ or $X_{27}$-$X_{34}$, $X_{36}$-$X_{37}$), most preferably at least 11 amino acids ($X_{27}$-$X_{37}$).

In various preferred embodiments, the amino acid sequence (B2') is free of cysteine residues.

In various non-limiting embodiments, the amino acid sequence (B2') can also be amino acid sequence (B2'*)

$X_{24}X_{25}X_{26}X_{27}X_{28}X_{29}X_{30}X_{31}X_{32}X_{33}X_{34}X_{35}X_{36}X_{37}X_{38}$ (B2'*)

wherein

$X_{24}$ is G, L, I, T, K, Q, D, H, V, F, E, S, Y, N, R or not present, preferably G, T, D, V or not present;

$X_{25}$ is N, G, V, M, D, E, L, P, K, I or not present, more preferably N, G, M, D, E, P or not present;

$X_{26}$ is D, E, N, V, A, R, G, L, T, K or not present, preferably D, E, N, A, R, G or not present;

$X_{38}$ is H, R, E, D, N, M, A or not present, preferably H, D, N, M or not present; and

wherein $X_{27}$-$X_{37}$ are as described above.

In various preferred embodiments, the amino acid sequence (B2'*) is free of cysteine residues.

In various non-limiting embodiments, amino acid sequences B1' and B2' may also form a combined sequence motif (B')

$X_{24}X_{25}X_{26}X_{27}X_{28}X_{29}X_{30}X_{31}X_{32}X_{33}X_{34}X_{35}X_{36}X_{37}X_{38}X_{39}X_{40}X_{41}X_{42}X_{43}X_{44}X_{45}X_{46}X_{47}X_{48}X_{49}X_{50}X_{51}X_{52}X_{53}X_{54}$ (B')

wherein

$X_{24}$ is G, L, I, T, K, Q, D, H, V, F, E, S, Y, N, R or not present, preferably G, T, D, V or not present;

$X_{25}$ is N, G, V, M, D, E, L, P, K, I or not present, more preferably N, G, M, D, E, P or not present;

$X_{26}$ is D, E, N, V, A, R, G, L, T, K or not present, preferably D, E, N, A, R, G or not present;

$X_{27}$ is D, E, Y, F, V, T, M or W, preferably Y, F, M or W, most preferably Y;

$X_{28}$ is V;

$X_{29}$ is Y;

$X_{30}$ is D;

$X_{31}$ is L, I, M or V, preferably L or I, most preferably L;

$X_{32}$ is E, T, S, Q, C or G, preferably E, T, S or C, more preferably E, T or S, most preferably E or S; $X_{33}$ is I, L, M, V, T, C or A, preferably I, L, M, V, T or C, more preferably I, L, M, V or T, most preferably V orT;

$X_{34}$ is any naturally occuring amino acid or not present, preferably E, P, D, G, S, A, Q, T, V, K, N, F, G, R or not present, more preferably E, D, A or K, most preferably E;

$X_{35}$ is any naturally occuring amino acid or not present, preferably V, D, S, Q, N, R, A, C, H, G, E, T or not present, more preferably D, S, N, C or E, more preferably D, S, N or E, most preferably D or N, $X_{36}$ is any naturally occuring amino acid or not present, preferably D, G, H, Q, N, E, T, S, A or not present, more preferably D, G, H, Q or N, more preferably G, H or N, most preferably G or H,

$X_{37}$ is any naturally occuring amino acid or not present, preferably T, D, S, H, V, E, R, Q, K, N, I or not present, more preferably T, D, H or E, most preferably T or H;

$X_{38}$ is H, R, E, D, N, M, A or not present, preferably H, D, N, M or not present;

$X_{39}$ is D, N, E or not present, preferably not present;

$X_{40}$ is D, A, Y or not present, preferably not present;

$X_{41}$ is L, E, I or not present, preferably not present;

$X_{42}$ is E, K, Q or not present, preferably not present;

$X_{43}$ is any naturally occuring amino acid or not present, preferably R, C, F, V, L, P, H, N or not present, more preferably C, F, V or H, more preferably F, V or H, most preferably F;

$X_{44}$ is any naturally occuring amino acid or not present, preferably F, H, Q, V, I, S, A, N, Y, C, T, W or not present, more preferably F, H, V, A, N, C, T or W, more preferably F, H, V, N or A, most preferably F, H, A or V;

$X_{45}$ is F, Y, A, V, N, G, S, I, C or D, preferably F, A, N, C or D, most preferably F, A, N or D;

$X_{46}$ is any naturally occuring amino acid or not present, preferably G, A, R, F, M or not present, preferably G, A or F, most preferably G;

$X_{47}$ is A, V, I, P, L, S, F, G, K or C, preferably A, V, I, P, L or C, more preferably A, V, I, L or C, most preferably A, V, I or L;

$X_{48}$ is any naturally occuring amino acid or not present, preferably N, G, D or not present, most preferably N or G;

$X_{49}$ is N, D, R, G, E, L, M, S, P or Q, preferably N, D, G, E or M, most preferably N, G, E or M;

$X_{50}$ is V, I, M, L or N, preferably I, M or L, most preferably I or L;

$X_{51}$ is L, I, V, D or C, preferably L, I, V or C, most preferably L, I or V;

$X_{52}$ is V, I, N, L, C or A, preferably V, L or C, most preferably V or L;

$X_{53}$ is H, K, S or T, preferably H or K, most preferably H; and

$X_{54}$ is N or Q, preferably N;

wherein amino acid sequence (B') comprises or consists of at least 15 amino acids ($X_{27}$-$X_{33}$, $X_{45}$, $X_{47}$, $X_{49}$-$X_{54}$), preferably at least 23 amino acids.

In various preferred embodiments, amino acid sequence (B') is free of cysteine residues.

In various non-limiting embodiments, the catalytic motif(s) of the first or the second intein amino acid sequence comprise(s) the amino acid sequence (C')

$X_{12}X_{13}X_{14}X_{15}X_{16}X_{17}X_{18}X_{19}X_{20}X_{21}X_{22}X_{23}$ (C')

wherein

$X_{12}$ is Y, H, C, V, I, L, M, S, T, K, E, D, Q, W, R or A, preferably Y, C, I, L, K or E, more preferably Y, C or E, more preferably Y or E, most preferably E;

$X_{13}$ is I, V, C, T or S, preferably I, V, C or S, more preferably I, V or S, most preferably V;

$X_{14}$ is D, T, M, E, C, K, H, N, V, S, I or R, preferably D, T, E, C, H, V or I, more preferably D, T or H, more preferably D or T, most preferably D;

$X_{15}$ is V, C, A, I or T, preferably V, C or T, most preferably V;

$X_{16}$ is T;

$X_{17}$ is E, R, A, S, D, K, N, H, C, P or T, preferably E, R, D, H or C, more preferably E, R, D or H, more preferably E or R, most preferably E;

$X_{18}$ is D, N, Q or E, preferably D;

$X_{19}$ is H;

$X_{20}$ is S or R, preferably S;

$X_{21}$ is L, V, A, I or Y, preferably L, I or Y, most preferably L;

$X_{22}$ is I, L, F, V, M or Y, preferably I, L, F, V or M, most preferably I, L, F or V; and

$X_{23}$ is G, D, N, L, Q, K, R, Y, V, I, A, T, S or K, preferably G, D, N, V, I or T, more preferably D or V; wherein the amino acid sequence (C') comprises or consists of 12 amino acids ($X_{12}$-$X_{23}$).

In various preferred embodiments, amino acid sequence (C') is free of cysteine residues.

In various preferred embodiments, the amino acid sequence (A) or (A') forms the N-terminus of the first intein amino acid sequence.

In various preferred embodiments, the amino acid sequence (B1) or (B1') forms the C-terminus of the second intein amino acid sequence. In other embodiments, also one of the amino acid sequences (B1*) or (B) or (B') may form the C-terminus of the second intein amino acid sequence.

In various preferred embodiments, the second extein amino acid sequence (b2) comprises a serine (S) or threonine (T) residue at its N-terminus that is fused to the amino acid of the C-terminus of the second intein amino acid sequence (b1) by a peptide bond. That typically means, that in preferred embodiments, the last amino acid at the C-terminus of the amino acid sequence (B1) or (B1') or (B1*) or (B) or (B') is directly linked to a serine or threonine residue (also referred to as position +1) by a peptide bond, wherein the serine or threonine residue (+1) is part of the second extein amino acid sequence (b2). In preferred embodiments, the serine or threonine residue may also be part of the catalytic motif of (B1) or (B1') or (B1*) or (B) or (B'), since it has an effect on the catalytic activity, in particular the splicing activity, of the intein. In these preferred embodiments, the catalytic motif may be one of:

$X_{45}X_{46}X_{47}X_{48}X_{49}X_{50}X_{51}X_{52}X_{53}X_{54}X_{55}X_{56}X_{57}$(+1) (B1-(+1)), wherein (+1) is S or T, preferably S; or

$X_{44}X_{45}X_{46}X_{47}X_{48}X_{49}X_{50}X_{51}X_{52}X_{53}X_{54}X_{55}X_{56}X_{57}$(+1) (B1*-(+1)), wherein (+1) is S or T, preferably S; or

$X_{24}X_{25}X_{26}X_{27}X_{28}X_{29}X_{30}X_{31}X_{32}X_{33}X_{34}X_{35}X_{36}X_{37}X_{38}X_{39}X_{40}X_{41}X_{42}X_{43}X_{44}X_{45}X_{46}X_{47}X_{48}X_{49}$
$X_{50}X_{51}X_{52}X_{53}X_{54}X_{55}X_{56}X_{57}$(+1) (B-(+1)), wherein (+1) is S or T, preferably S; or

$X_{43}X_{44}X_{45}X_{46}X_{47}X_{48}X_{49}X_{50}X_{51}X_{52}X_{53}X_{54}$(+1) (B1'-(+1)), wherein (+1) is S or T, preferably T; or

$X_{24}X_{25}X_{26}X_{27}X_{28}X_{29}X_{30}X_{31}X_{32}X_{33}X_{34}X_{35}X_{36}X_{37}X_{38}X_{39}X_{40}X_{41}X_{42}X_{43}X_{44}X_{45}X_{46}X_{47}X_{48}X_{49}X_{50}X_{51}X_{52}X_{53}X_{54}$(+1)
(B'-(+1)), wherein (+1) is S or T, preferably T;

wherein $X_{24}$-$X_{57}$ are as described above in the corresponding embodiment.

In various preferred embodiments, the amino acid sequence (B2) is located N-terminally to amino acid sequence (B1) in the second intein amino acid sequence, preferably amino acids $X_{37}$ of (B2) and $X_{45}$ of (B1) are linked to each other by a peptide bond. In this case, the amino acid sequences of (B2) and (B1) may form a combined amino acid sequence (B).

In further preferred embodiments, the amino acid sequence (B2') is located N-terminally to amino acid sequence (B1') in the second intein amino acid sequence, preferably amino acids $X_{37}$ of (B2') and $X_{43}$ of (B1') are linked to each other by a peptide bond. In this case, the amino acid sequences of (B2') and of (B1') may form a combined amino acid sequence (B').

Analogously, the amino acid sequences B1* or B2* or B2'* may be present.

In various preferred embodiments, the amino acid sequence (C), if present in the first intein amino acid sequence, is located C-terminally to amino acid sequence (A), or, if present in the second intein amino acid sequence, is located N-terminally to amino acid sequence (B2) or (B2*) or (B).

In various preferred embodiments, the amino acid sequence (C'), if present in the first intein amino acid sequence, is located C-terminally to amino acid sequence (A'), or, if present in the second intein amino acid sequence, is located N-terminally to amino acid sequence (B2') or (B2'*) or (B').

In various preferred embodiments, the amino acid sequence (A), the amino acid sequence (B1) or (B1*), the amino acid sequence (B2) or (B2*) and/or the amino acid sequence (C), preferably at least two, more preferably at least three, most preferably all four amino acid sequences are free of cysteine residues.

In various preferred embodiments, the amino acid sequence (A'), the amino acid sequence (B1'), the amino acid sequence (B2') or (B2'*) and/or the amino acid sequence (C'), preferably at least two, more preferably at least three, most preferably all four amino acid sequences are free of cysteine residues.

Preferably,

(i) the at least one first intein amino acid sequence is free of cysteine residues, and/or
(ii) the at least one second intein amino acid sequence is free of cysteine residues. This means that the complete sequences, i.e. not only the catalytic motifs, do not contain any cysteine residue.

The term "sequence identity" as used herein refers to peptides that share identical amino acids at corresponding positions or nucleic acids sharing identical nucleotides at corresponding positions. This takes into account of the fact that peptides may exist which do not have significant "sequence identity", as they may not have similar amino acids at corresponding positions, but have the same function, because they contain, e.g., conservative substitutions. The sequence identity is typically given in percent identity, i.e. the portion of identical amino acids relative to the total number of amino acids present. If not indicated otherwise herein, sequence identity is determined based on the entire length of the provided reference sequence. A sequence identity of 90% relative to a 10 aa long reference sequence would thus mean that 9 of 10 amino acids of the corresponding positions in the query sequence are identical to those in the reference sequence.

The determination of sequence identity described herein between two amino acid or nucleotide sequences can be accomplished using a mathematical algorithm. For example, a mathematical algorithm useful for comparing two sequences is the algorithm of Karlin and Altschul (1990, Proc. Natl. Acad. Sci. USA 87:2264-2268), modified as in Karlin and Altschul (1993, Proc. Natl. Acad. Sci. USA 90:5873-5877). This algorithm is incorporated into the BLASTN and BLASTX programs and can be accessed, for example, at the National Center for Biotechnology Information (NCBI) world wide web site having the universal resource locator "www.ncbi.nlm.nih.gov/BLAST". Blast nucleotide searches can be performed with BLASTN program, whereas BLAST protein searches can be performed with BLASTX program or the NCBI "blastp" program. Another algorithm available in the art is the FASTA algorithm. Sequence comparisons (alignments), in particular multiple sequence comparisons, can be generated using computer programs. Commonly used are for example the Clustal series (See, e.g. ,Chenna et al. (2003): Multiple sequence alignment with the Clustal series of programs. Nucleic Acid Research 31, 3497-3500), T-Coffee (See, e.g., Notredame et al. (2000): T-Coffee: A novel method for multiple sequence alignments. J. Mol. Biol. 302, 205-217) or programs based thereon or the respective algorithms. Further possible are sequence comparisons (alignments) with the computer program Vector NTI® Suite 10.3 (Invitrogen Corporation, 1600 Faraday Avenue, Carlsbad, CA, USA) with the pre-set standard parameters, the AlignX-module of which is based on ClustalW. If not explicitly defined otherwise, sequence identity is determined using the BLAST algorithm.

The term "mutant" or "variant" as used herein refers to a polypeptide the sequence of which has one or more amino acids added, deleted, substituted or otherwise chemically modified in comparison to a polypeptide according to one of the claimed sequences, provided that the mutant/variant retains substantially the same properties as the polypeptide according to one of the claimed sequences.

In a further embodiment, the present invention relates to an isolated polypeptide or to a composition of isolated polypeptides as described above, wherein

(i) the at least one first intein amino acid sequence has at least 70 %, at least 80 %, at least 85 %, at least 90 %, at least 95 %, at least 99 % or 100 % sequence identity with any one of the amino acid sequences set forth in SEQ ID Nos. 1, 3, 5, 7, 9, 11, 13, 15, 17, 19, 21, 23, 25, 27, 29, 31, 33, 35, 37, 39, 41, 43, 45, 48, 51, 53, 55, 58, 61, 63, 65, 67, 70, 74, 76, 78, 80, 82, 85, 87, 89, 92, 97, 100, 102, 106, 108, 111, 114, 117, 120, 122, 124, 126, 128, 130, 132, 134, 136, 138, 140, 142, 144, 146 and 147, preferably SEQ ID Nos. 1, 3, 5, 7, 9, 11, 13, 15, 17, 19, 21, 23, 25, 27, 29, 31, 33, 35, 37, 39, 41, 43 and 45, more preferably SEQ ID NO:1 or 3, more preferably SEQ ID NO:3; and/or

(ii) the at least one second intein amino acid sequence has at least 70 %, at least 80 %, at least 85 %, at least 90 %, at least 95 %, at least 99 % or 100 % sequence identity with any one of the amino acid sequences set forth in SEQ ID Nos. 2, 4, 6, 8, 10, 12, 14, 16, 18, 20, 22, 24, 26, 28, 30, 32, 34, 36, 38, 40, 42, 44, 46, 49, 52, 54, 56, 59, 62, 64, 66, 68, 71, 75, 77, 79, 81, 83, 86, 88, 90, 93, 98, 101, 103, 107, 109, 112, 115, 118, 121, 123, 125, 127, 129, 131, 133, 135, 137, 139, 141, 143, 145, 148, 149, 150, 151 and 152, preferably SEQ ID Nos. 2, 4, 6, 8, 10, 12, 14, 16, 18, 20, 22, 24, 26, 28, 30, 32, 34, 36, 38, 40, 42, 44 and 46, more preferably SEQ ID NO:2 or 4, more preferably SEQ ID NO:4; or

(iii) at least one intein amino acid sequence comprising the first intein amino acid sequence and the second intein amino acid sequence in one contiguous amino acid sequence; wherein the C-terminus of the first intein sequence is ligated to the N-terminus of the second intein sequence, and wherein the contiguous amino acid sequence has at least 70 %, at least 80 %, at least 85 %, at least 90 %, at least 95 %, at least 99 % or 100 % sequence identity with any one of the amino acid sequences set forth in SEQ ID Nos. 47, 50, 57, 60, 69, 72, 73, 84, 91, 94, 95, 96, 99, 104, 105, 110, 113, 116, 119, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182,183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 and 250.

In a preferred embodiment, the present invention relates to an isolated polypeptide as described above, wherein

(a) said at least one first intein amino acid sequence has at least 70 %, at least 80 %, at least 85 %, at least 90 %, at least 95 %, at least 99 % or 100 % sequence identity with the amino acid sequence set forth in SEQ ID NO:1 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:2; or

(b) said at least one first intein amino acid sequence has at least 70 %, at least 80 %, at least 85 %, at least 90 %, at least 95 %, at least 99 % or 100 % sequence identity with the amino acid sequence set forth in SEQ ID NO:3 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:4;

(c) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:5 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:6; or

(d) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:7 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:8; or

(e) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:9 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:10; or

(f) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:11 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:12; or

(g) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:13 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:14; or

(h) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:15 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:16; or

(i) wherein said at least one firstl intein amino acid sequence has at least 70 %, at least 80 %, at least 85 %, at least 90 %, at least 95 %, at least 99 % or 100 % sequence identity with the amino acid sequence set forth in SEQ ID NO:17 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:18; or

(j) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:19 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:20; or

(k) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:21 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:22; or

(l) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:23 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:24; or

(m) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:25 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:26; or

(n) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:27 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:28; or

(o) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:29 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:30; or

(p) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:31 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:32; or

(q) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:33 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:34; or

(r) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:35 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:36; or

(s) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:37 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:38; or

(t) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:39 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:40; or

(u) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:41 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:42; or

(v) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:43 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:44; or

(w) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:45 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:46.

In a preferred embodiment, the present invention relates to an isolated polypeptide as described above, wherein

(1) said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:1 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:2; or

(2) said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:3 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:4;

(3) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:5 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:6; or

(4) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:7 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:8; or

(5) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:9 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:10; or

(6) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:11 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:12; or

(7) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:13 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:14; or

(8) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:15 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:16; or

(9) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:17 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:18; or

(10) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:19 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:20; or

(11) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:21 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:22; or

(12) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:23 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:24; or

(13) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:25 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:26; or

(14) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:27 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:28; or

(15) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:29 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:30; or

(16) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:31 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:32; or

(17) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:33 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:34; or

(18) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:35 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:36; or

(19) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:37 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:38; or

(20) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:39 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:40; or

(21) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:41 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:42; or

(22) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:43 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:44; or

(23) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:45 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:46; or

(24) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:48 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:49; or

(25) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:51 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:52; or

(26) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:53 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:54; or

(27) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:55 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:56; or

(28) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:58 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:59; or

(29) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:61 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:62; or

(30) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:63 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:64; or

(31) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:65 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:66; or

(32) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:67 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:68; or

(33) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:70 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:71; or

(34) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:74 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:75; or

(35) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:76 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:77; or

(36) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:78 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:79; or

(37) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:80 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:81; or

(38) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:82 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:83; or

(39) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:85 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:86; or

(40) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:87 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:88; or

(41) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:89 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:90; or

(42) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:92 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:93; or

(43) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:97 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:98; or

(44) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:100 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:101; or

(45) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:102 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:103; or

(46) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:106 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:107; or

(47) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:108 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:109; or

(48) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:111 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:112; or

(49) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:114 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:115; or

(50) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:117 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:118; or

(51) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:120 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:121; or

(52) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:122 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:123; or

(53) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:124 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:125; or

(54) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:126 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:127; or

(55) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:128 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:129; or

(56) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:130 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:131; or

(57) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:132 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:133; or

(58) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:134 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:135; or

(59) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:136 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:137; or

(60) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:138 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:139; or

(61) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:140 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:141; or

(62) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:142 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:143; or

(63) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:144 and said at least one C-terminal intein amino acid sequence has at least 70 %, at least 80 %, at least 85 %, at least 90 %, at least 95 %, at least 99 % or 100 % sequence identity with the amino acid sequence set forth in SEQ ID NO:145; or

(64) wherein said at least one N-terminal intein amino acid sequence has at least 70 %, at least 80 %, at least 85 %, at least 90 %, at least 95 %, at least 99 % or 100 % sequence identity with the amino acid sequence set forth in SEQ ID NO:146 or 147 and said at least one C-terminal intein amino acid sequence has at least 70 %, at least 80 %, at least 85 %, at least 90 %, at least 95 %, at least 99 % or 100 % sequence identity with any one of the amino acid sequence set forth in SEQ ID NO:148-151.

Preferably, the N-terminal intein amino acid sequence is the at least one first intein amino acid sequence (a1) and the C-terminal intein amino acid sequence is the at least one second intein amino acid sequence.

Most preferably, the present invention relates to an isolated polypeptide or a composition comprising isolated polypeptides as described above, wherein

(a) said at least one N-terminal intein amino acid sequence (or at least one first intein amino acid sequence) has at least 70 %, at least 80 %, at least 85 %, at least 90 %, at least 95 %, at least 99 % or 100 % sequence identity with the amino acid sequence set forth in SEQ ID NO:1 and said at least one C-terminal intein amino acid sequence (or at least one second intein amino acid sequence) has at least 70 %, at least 80 %, at least 85 %, at least 90 %, at least 95 %, at least 99 % or 100 % sequence identity with the amino acid sequence set forth in SEQ ID NO:2; or

(b) said at least one N-terminal intein amino acid sequence (or at least one first intein amino acid sequence) has at least 70 %, at least 80 %, at least 85 %, at least 90 %, at least 95 %, at least 99 % or 100 % sequence identity with the amino acid sequence set forth in SEQ ID NO:3 and said at least one C-terminal intein amino acid sequence (or at least one second intein amino acid sequence) has at least 70 %, at least 80 %, at least 85 %, at least 90 %, at least 95 %, at least 99 % or 100 % sequence identity with the amino acid sequence set forth in SEQ ID NO:4.

In a preferred embodiment of the invention, the split intein is formed by two separate polypeptides which are preferably separated by a peptide bond break of the intein's amino acid sequence, i.e. there is one C-terminal intein amino acid sequence and one N-terminal intein amino acid sequence. "Separated by a peptide bond break of the intein's amino acid sequence" thus means that they exist as two separate, not covalently linked molecules, that have a C-terminal and N-terminal end, respectively, which, if covalently coupled to each other, would form the corresponding full intein sequence.

In preferred embodiments according to the invention, the $Int^N$ sequence is an $Aes^N$ sequence or a homolog or variant thereof. Preferably, the $Int^N$ sequence has at least 70 %, at least 75 %, at least 80 %, at least 85 %, at least 90 %, at least 91 %, at least 92 %, at least 93 %, at least 94 %, at least 95 %, at least 96 %, at least 97 %, at least 98 %, at least 99 %, at least 99.5 % or 100 % sequence identity with the amino acid sequence set forth in SEQ ID NO:1.

In preferred embodiments according to the invention, the $Int^C$ sequence is an $Aes^C$ sequence or a homolog or variant thereof. Preferably, the $Int^C$ sequence has at least 70 %, at least 75 %, at least 80 %, at least 85 %, at least 90 %, at least 91 %, at least 92 %, at least 93 %, at least 94 %, at least 95 %, at least 96 %, at least 97 %, at least 98 %, at least 99 %, at least 99.5 % or 100 % sequence identity with the amino acid sequence set forth in SEQ ID NO:2.

In preferred embodiments according to the invention, the $Int^N$ sequence is a $CL^N$ sequence or a homolog or variant thereof. Preferably, the $Int^N$ sequence has at least 70 %, at least 75 %, at least 80 %, at least 85 %, at least 90 %, at least 91 %, at least 92 %, at least 93 %, at least 94 %, at least 95 %, at least 96 %, at least 97 %, at least 98 %, at least 99 %, at least 99.5 % or 100 % sequence identity with the amino acid sequence set forth in SEQ ID NO:3.

In preferred embodiments according to the invention, the $Int^C$ sequence is a $CL^C$ sequence or a homolog or variant thereof. Preferably, the $Int^C$ sequence has at least 70 %, at least 75 %, at least 80 %, at least 85 %, at least 90 %, at least 91 %, at least 92 %, at least 93 %, at least 94 %, at least 95 %, at least 96 %, at least 97 % ,at least 98 %, at least 99 %, at least 99.5 % or 100 % sequence identity with the amino acid sequence set forth in SEQ ID NO:4. "CL intein", as referred to herein is an intein that comprises SEQ ID No. 3 as the N-terminal intein sequence and SEQ ID NO: 4 as the C-terminal intein sequence.

In various non-limiting embodiments according to the invention, the first intein amino acid sequence and the second intein amino acid sequence are not derived from a thermophilic organism. This means that the two sequences are not identical to any intein sequence from a thermophilic organism or are not engineered based on a sequence from such an organism. A "thermophilic organism" or "thermophile" is an organism - a type of extremophile - that thrives at relatively high temperatures, such as between 41 and 122 °C. Thermophiles are often archaea or bacteria.

In various non-limiting embodiments according to the invention

(i) the first and the second intein amino acid sequence, preferably the N-terminal and C-terminal sequence of a split intein or fragment thereof, (trans-)splice under oxidizing conditions, preferably when the isolated polypeptide is exposed to atmospheric oxygen; and/or

(ii) the first and the second intein amino acid sequence, preferably the N-terminal and C-terminal sequence of a split intein or fragment thereof, (trans-)splice under ambient temperatures, preferably at temperatures of 40 °C or less than 40 °C, more preferably in the range of 0 to 37 °C, in particular at 8 °C to 37 °C.

In various non-limiting embodiments, the total temperature range in which the first and the second intein amino acid sequence (trans-)splice is 0°C to 80 °C.

In one non-limiting embodiment, the first and the second intein amino acid sequence (trans-)splice preferably at 0 °C to 40 °C, more preferably at 0 to 37 °C, more preferably at 5 to 10 °C, more preferably at 8 °C.

Further preferred, the first and the second intein amino acid sequence, preferably the N-terminal and C-terminal sequence of a split intein or fragment thereof, *(trans-)splice* at 37 °C.

The feature that the first and the second intein amino acid sequence, preferably the N-terminal and C-terminal sequence of a split intein or fragment thereof, (trans-)splice at the given temperatures means that the splicing efficiency is at least 50 %, preferably at least 75 %, more preferably at least 85 %, more preferably at least 90 % of the maximum splicing efficiency of the intein at its optimum temperature, i.e. the temperature at which it performs best, under otherwise identical conditions.

The feature that the first and the second intein amino acid sequence, preferably the N-terminal and C-terminal sequence of a split intein or fragment thereof, (trans-)splice under oxidizing conditions means that the splicing efficiency is at least 50 %, preferably at least 75 %, more preferably at least 85 %, more preferably at least 90 % of the maximum splicing efficiency of the intein under reducing conditions while the other conditions, temperature etc., are identical.

Preferably, the first and the second intein amino acid sequence, preferably the N-terminal and C-terminal sequence of a split intein or fragment thereof, (trans)-splice in the absence of any reducing agent. In addition, the *trans*-splicing is preferably not dependent on a denaturation step.

The feature that the first and the second intein amino acid sequence, preferably the N-terminal and C-terminal sequence of a split intein or fragment thereof, (trans-)splice in the absence of any reducing agent preferably means that the splicing efficiency is at least 50 %, preferably at least 75 %, more preferably at least 85 %, more preferably at least 90 % of the maximum splicing efficiency of the intein under reducing conditions, i.e. in the presence of a reducing agent, e.g. DTT or TCEP, while the other conditions, temperature etc., are identical.

The feature that the (*trans*-)splicing of the first and the second intein amino acid sequence, preferably of the N-terminal and C-terminal sequence of a split intein or fragment thereof, is not dependent on a denaturation step preferably means that the splicing efficiency is at least 50 %, preferably at least 75 %, more preferably at least 85 %, more preferably at least 90 % of the maximum splicing efficiency of the intein when a denaturation step is present, while the other conditions, temperature etc., are identical.

It is understood that the functional split intein is formed, in various non-limiting embodiments, by two of the isolated polypeptides described herein, one comprising the first intein amino acid sequence and the other one comprising the second intein amino acid sequence, with both being separate molecules, i.e. not being covalently linked by a peptide bond.

The isolated polypeptide(s) further comprise(s) at least one first extein sequence (a2) and at least one second extein sequence (b2). The term "extein" or "extein sequence" as used herein refers to the peptide sequences that link to form a new polypeptide after the intein has excised itself during splicing. In other words, the N-terminal and C-terminal exteins (also termed Ex$^N$ and Ex$^C$) flank the Int$^N$ and Int$^C$ fragments, respectively prior to the (*trans*-)splicing reaction. It is one feature of the invention that the first amino acid of the second extein sequence (b2), i.e. the amino acid in the so-called +1 position, is not a cysteine residue. As described above, it can be preferred that the amino acid in this position is a serine or threonine residue.

In a preferred embodiment, the first and second intein amino acid sequence interact with each other to catalyze the cleavage of the peptide bonds between the C-terminal amino acid of the first extein sequence and the N-terminal amino acid of the first intein sequence and the N-terminal amino acid of the second extein sequence and the C-terminal amino acid of the second intein sequence and to ligate the C-terminal amino acid of the first extein sequence to the N-terminal amino acid of the second extein sequence by a peptide bond

(a) at a temperature of $\leq$ 40 °C, preferably less than 40 °C, more preferably 0 to 37 °C, more preferably 8 °C or 37 °C; and/or
(b) under oxidizing reaction conditions, preferably under atmospheric oxygen.

In various non-limiting embodiments of this aspect of the present invention, the isolated polypeptide comprises exactly one C-terminal extein and exactly one N-terminal extein sequence.

In yet still other non-limiting embodiments of this aspect of the present invention, at least one of the peptide sequences of the isolated polypeptide(s) selected from N-terminal intein, C-terminal intein, C-terminal extein and/or N-terminal extein is a recombinant protein.

Preferably, the first extein amino acid sequence and/or the second extein amino acid sequence is a recombinant protein or a fragment thereof.

In various non-limiting embodiments, the extein sequence is heterologous with respect to the intein sequence or fragment thereof to which it is coupled, i.e. the two sequences do not naturally occur together but have been artificially combined. It is understood that all the embodiments described are similarly applicable to scenarios where the N-terminal intein sequence and the C-terminal intein sequence are part of separate molecules. In such cases, each of the two molecules comprises the respective extein sequence, i.e. the polypeptide comprising the N-terminal intein sequence comprises the N-terminal extein and the polypeptide comprising the C-terminal intein sequence comprises the C-terminal extein or the polypeptide comprising the first intein sequence comprises the first extein sequence and the polypeptide comprising the second intein sequence comprises the second extein sequence. In various embodiments, thus either the first or second or both extein sequences are heterologous to the utilized intein sequences, i.e. do not flank the respective intein sequence in nature.

Through using extein sequences that are heterologous to the chosen intein sequences, the full performance of (split) inteins can be exploited, as the extein sequences will be joined by a peptide bond following the (*trans-*)splicing reaction with virtually no trace of the previously existing intein sequences.

The isolated polypeptides described herein can advantageously be used for example for labelling of a protein. Due to the small size of the N-terminal intein sequence or fragment thereof, the protein of interest-Int$^N$ (POI-Int$^N$) peptide complex can be obtained by using solid-phase peptide synthesis. The label attached to the Int$^C$ sequence can be generated by recombinant protein expression. For example, upon combining the two chimeric protein complexes, i.e. "POI-Int$^N$" and "Int$^C$-label", the (*trans-*)splicing reaction could take place generating "POI-label". Of course, also encompassed are all embodiments wherein N- and C-terminal intein sequences are exchanged, i.e. by coupling the label or any other modifying moiety (that need not be a peptide or protein but only needs to be coupled to an amino acid or amino acid oligomer) to the N-terminal intein sequence and synthesizing the protein of interest as a recombinant fusion protein with the C-terminal intein sequence. It is thus understood that while embodiments may be described herein with reference to only one of these possibilities, the present invention is intended to also cover the respective counterpart where the two intein fragments are exchanged.

In various non-limiting embodiments, the two separate intein sequences are useful by themselves. For example, it is possible, to pre-assemble - possibly in form of a composition - the "Int$^C$-label" fusion protein or merely the Int$^C$ sequence, e.g., for easy protein labelling. The ready "Int$^C$-label" fusion proteins could then be used as soon as a protein of interest is decided upon for easy and robust protein labelling. Of course, the reverse scenario is also possible, where the protein of interest is known and pre-generated fused to the Int$^N$ sequence. As soon as it is decided upon which labels should be used the "Int$^C$-label" fusion proteins could be prepared and protein labelling could be carried out.

Moreover, the label of the fusion protein in this example can of course be readily replaced by any protein of interest or any other non-peptide or non-protein moiety. In case of non-peptide, non-protein moieties are used for the modification of proteins or any other purpose, these are used in form of conjugates with at least one amino acid or a short peptide sequence to facilitate the covalent linkage with the corresponding other extein part by a peptide bond.

In various non-limiting embodiments, the protein of interest is an antibody, a nanobody, a protein hormone, antibody-like molecules, nanobody-like molecules or a fragment or homolog thereof.

In various non-limiting embodiments, including the afore-mentioned, it is preferred that the "Int$^C$-protein" fusion protein, or more generally the "intein sequence-protein of interest" fusion protein, is generated via recombinant expression.

In various non-limiting embodiments of this aspect of the present invention an isolated polypeptide comprises at least one first intein amino acid sequence which is an N-terminal sequence of a split intein or fragment thereof and at least one second intein amino acid sequence which is a C-terminal sequence of a split intein or fragment thereof, wherein

1) the at least one N-terminal intein sequence is selected from SEQ ID NO:1 and the at least one C-terminal intein sequence is selected from SEQ ID NO:2, or

2) the at least one N-terminal intein sequence is selected from SEQ ID NO:3, and the at least one C-terminal intein sequence is selected from SEQ ID NO:4.

In these non-limiting embodiments, the two intein sequences that naturally occur in form of separate molecules may be combined in one molecule. Alternatively, the two sequences may still be parts of separate molecules. In the latter case, the isolated polypeptide is a composition of two isolated polypeptides, one of which comprises the N-terminal intein sequence, as defined above, and the other comprises the C-terminal intein sequence, also as defined above.

In various non-limiting embodiments, the present invention therefore also covers compositions of two isolated polypeptides as described herein, wherein the first polypeptide comprises at least one first intein amino acid sequence (N-terminal intein sequence) and the second polypeptide comprises at least one second intein amino acid sequence (C-terminal intein sequence), wherein

1) the at least one N-terminal intein sequence is selected from any one of SEQ ID Nos. 1, 3, 5, 7, 9, 11, 13, 15, 17, 19, 21, 23, 25, 27, 29, 31, 33, 35, 37, 39, 41, 43, 45, 48, 51, 53, 55, 58, 61, 63, 65, 67, 70, 74, 76, 78, 80, 82, 85, 87, 89, 92, 97, 100, 102, 106, 108, 111, 114, 117, 120, 122, 124, 126, 128, 130, 132, 134, 136, 138, 140, 142, 144, 146 and 147, or a variant thereof and the at least one C-terminal intein sequence is selected from any one of SEQ ID Nos. 2, 4, 6, 8, 10, 12, 14, 16, 18, 20, 22, 24, 26, 28, 30, 32, 34, 36, 38, 40, 42, 44, 46, 49, 52, 54, 56, 59, 62, 64, 66, 68, 71, 75, 77, 79, 81, 83, 86, 88, 90, 93, 98, 101, 103, 107, 109, 112, 115, 118, 121, 123, 125, 127, 129, 131, 133, 135, 137, 139, 141, 143, 145, 148, 149, 150, 151 and 152, or a variant thereof, or

2) the at least one N-terminal intein sequence is selected from SEQ ID NO:1 or 3 or a variant thereof and the at least one C-terminal intein sequence is selected from SEQ ID NO:2 or 4 or a variant thereof.

In various embodiments, the isolated polypeptide comprises at least one first extein amino acid sequence (N-terminal extein amino acid sequence) fused to at least one first intein amino acid sequence (N-terminal intein sequence), wherein the isolated polypeptide has an amino acid sequence having at least 70 %, at least 75 %, at least 80 %, at least 85 %, at least 90 %, at least 91 %, at least 92 %, at least 93 %, at least 94 %, at least 95 %, at least 96 %, at least 97 % ,at least 98 %, at least 99 %, at least 99.5 % or 100 % sequence identity with any one of the amino acid sequences set forth in SEQ ID Nos. 251 **(1)**, 253 **(3)**, 255 **(3a)**, 257 **(5)**, 259 **(7)**, 261 and 262 **(9)**, 264 **(10)**, 266 **(12)**, 268 **(14)**, or 269 **(16)**.

In various non-limiting embodiments, the isolated polypeptide comprises at least one second extein amino acid sequence (C-terminal extein amino acid sequence) fused to at least one second intein amino acid sequence C-terminal intein sequence, wherein the isolated polypeptide has an amino acid sequence having at least 70 %, at least 75 %, at least 80 %, at least 85 %, at least 90 %, at least 91 %, at least 92 %, at least 93 %, at least 94 %, at least 95 %, at least 96 %, at least 97 % ,at least 98 %, at least 99 %, at least 99.5 % or 100 % sequence identity with the amino acid sequences set forth in SEQ ID Nos. 252 **(2)**, 254 **(4)**, 256 **(4a)**, 258 **(6)**, 260 **(8)**, 263 **(11)**, 265 **(13)**, 267 **(15)**, 270 **(15a)**, 271 **(17)** or 272 **(17a)**.

The first and second intein and extein sequences described above can be combined in one molecule or they may be parts of separate molecules. Most preferred, one of the isolated polypeptides comprising at least one N-terminal extein sequence fused to at least one N-terminal intein sequence is used together with one of the isolated polypeptides comprising at least one C-terminal extein sequence fused to at least one C-terminal intein sequence.

Advantageously the resulting polypeptide(s) has/have split intein activity exhibiting excellent splicing yields and/or rates.

Furthermore, of course all applications envisaged for one of the intein fragments also apply for embodiments where both are used/present together.

In preferred embodiments of this aspect of the present invention, the isolated polypeptide comprises exactly one N-terminal intein sequence and exactly one C-terminal intein sequence selected as described above. This similarly applies in case two separate isolated polypeptides are used.

The at least one first extein sequence is fused to the N-terminus of the at least one first intein sequence and has a length of at least 1, preferably at least 5 amino acids. The at least one second extein sequence is fused to the C-terminus of the at least one second intein sequence and has a length of at least 1, preferably at least 5 amino acids. In various non-limiting embodiments which are especially preferred, the first amino acid at the N-terminal end of the second extein amino acid sequence is S (serine residue). That means that the second intein amino acid sequence is preferably fused with its C-terminal end to the serine amino acid, which is the first amino acid at the N-terminal end of the second extein amino acid sequence, e.g. Int^C-S or CL^C-S, or Aes^C-S.

In various non-limiting embodiments, the last amino acid at the C-terminal end of the first extein amino acid sequence is D,

preferably the last two amino acids at the C-terminal end of the first extein amino acid sequence are TD, preferably the last three amino acids at the C-terminal end of the first extein amino acid sequence are DTD,
preferably the last four amino acids at the C-terminal end of the first extein amino acid sequence are IDTD (SEQ ID NO:285),
preferably the last five amino acids at the C-terminal end of the first extein amino acid sequence are YIDTD (SEQ ID NO:283); and/or
wherein the first amino acid at the N-terminal end of the second extein amino acid sequence is S, preferably the first two amino acids at the N-terminal end of the second extein amino acid sequence are SV,
preferably the first three amino acids at the N-terminal end of the second extein amino acid sequence are SVY,
preferably the first four amino acids at the N-terminal end of the second extein amino acid sequence are SVYL (SEQ ID NO:286),
preferably the first five amino acids at the N-terminal end of the second extein amino acid sequence are SVYLN (SEQ ID NO:284). Preferably, the described flanking amino acid sequences flank pol-e type inteins.

For pol-c type inteins, the flanking sequences are preferably as follows:

The last amino acid at the C-terminal end of the first extein amino acid sequence is D or S, preferably D,
preferably the last two amino acids at the C-terminal end of the first extein amino acid sequence are GD,
preferably the last three amino acids at the C-terminal end of the first extein amino acid sequence are YGD, VGD or AGD,
wherein the first amino acid at the N-terminal end of the second extein amino acid sequence is S or T, preferably T,
preferably the first two amino acids at the N-terminal end of the second extein amino acid sequence are TD,
preferably the first three amino acids at the N-terminal end of the second extein amino acid sequence are TDG or TDS,

Preferably, the first 1 to 15, preferably 1 to 10, more preferably 1 to 6, most preferably 1, 2, 3, 4, or 5 amino acids at the C-terminus of the first extein sequence or at the N-terminus of the second extein sequence that are fused to the intein sequence are also referred to as flanking amino acid sequences.

In one preferred embodiment according to the invention, the N-terminal intein sequence is linked at its N-terminal end to a flanking amino acid sequence YIDTD (e.g. YIDTD-Int$^N$) and/or the C-terminal inein sequence is linked at its C-terminal end to a flanking amino acid sequence SVYLN (e.g. Int$^C$-SVYLN), wherein the flanking amino acid sequences are part of the extein sequences.

Such an isolated polypeptide may have the advantage that the autocatalytic reaction - the protein splicing - proceeds with higher efficiency, if some, such as 1-15, or 1-10, or 1-6, or 1 or 2 or 3 or 4 or 5 of the wild type extein residues (also termed flanking amino acid sequences, i.e. sequences flanking the intein) are present. If only 1 residue is present, this typically means the directly adjacent amino acid, 2 means the two amino acids closest to the intein sequence, etc.

If the intein sequences are part of different isolated polypeptides, the respective flanking sequences may be comprised in the respective polypeptide. In this context, it is emphasized that, in general, the intein sequences of this application are shown without the +1 residue following the Int$^C$, as this residue is strictly not part of the intein sequence, but part of the extein sequence. However, it should be noted that this residue is usually involved in the intein's activity and forms part of the intein active site. Preferably, this +1 residue is a serine residue.

In various non-limiting embodiments, the first amino acid at the N-terminal end of the N-terminal intein sequence and/or the first amino acid C-terminally to the C-terminal end of the C-terminal intein sequence (+1) (within the flanking sequence of the extein sequence) is a serine residue.

In yet another non-limiting embodiment, the present invention relates to an isolated polypeptide as described above, wherein said polypeptide N-terminally to the N-terminal end of the at least one N-terminal intein sequence and/or C-terminally to the C-terminal end of the at least one C-terminal intein sequence further comprises a flanking amino acid sequence, wherein said flanking amino acid sequence, which is part of the first and/or second extein sequence, is selected from

1) in the case the N-terminal intein sequence is SEQ ID NO:1 or 3, the flanking amino acid sequence N-terminally to the N-terminal end of the at least one N-terminal intein sequence is SEQ ID NO:283; and/or
2) in the case the C-terminal intein sequence is SEQ ID NO:2 or 4, the flanking amino acid sequence C-terminally to the C-terminal end of the at least one C-terminal intein sequence is SEQ ID NO:284.

In various non-limiting embodiments, the above is to be understood such that, for example, the N-terminal intein sequence of SEQ ID NO:1 or 3 is N-terminally flanked by SEQ ID NO:283, i.e. SEQ ID NO:283 is located N-terminal to SEQ ID NO:1 or 3, and the C-terminal intein sequence or fragment thereof of SEQ ID NO:2 or 4 is C-terminally flanked by SEQ ID NO:284, i.e. SEQ ID NO:284 is located C-terminal to SEQ ID NO:2 or 4.

In another non-limiting embodiment, the first amino acid at the N-terminal end of the second extein amino acid sequence can be T (threonine residue), preferably, if the intein is a pol-c type intein. That means that the second intein amino acid sequence is preferably fused with its C-terminal end to the threonine amino acid, which is the first amino acid at the N-terminal end of the second extein amino acid sequence, e.g. Int$^C$-T.

According to the previous embodiment, preferably, the last amino acid at the C-terminal end of the first extein amino acid sequence is D,

more preferably the last two amino acids at the C-terminal end of the first extein amino acid sequence are CD, MD, GD or AD,

more preferably the last three amino acids at the C-terminal end of the first extein amino acid sequence are VCD, VMD, VGD, YAD or YGD; and/or

wherein the first amino acid at the N-terminal end of the second extein amino acid sequence is T,

more preferably the first two amino acids at the N-terminal end of the second extein amino acid sequence are TD,

more preferably the first three amino acids at the N-terminal end of the second extein amino acid sequence are TDG or TDS. Preferably, the described flanking amino acid sequences flank pol-c type inteins.

Preferably, in case of an isolated polypeptide/isolated polypeptides comprising one N-terminal intein sequence and/or one C-terminal intein sequence , wherein the N-terminal intein sequence has at least 70 %, at least 75 %, at least 80 %, at least 85 %, at least 90 %, at least 91 %, at least 92 %, at least 93 %, at least 94 %, at least 95 %, at least 96 %, at least 97 % ,at least 98 %, at least 99 %, at least 99.5 % or 100 % sequence identity with anyone of SEQ ID Nos. 1, 3, 5, 7, 9, 11, 13, 15, 17, 19, 21, 23, 25, 27, 29, 31, 33, 35, 37, 39, 41, 43, 45, 48, 51, 53, 55, 58, 61, 63, 65, 67, 70, 74, 76, 78, 80, 82, 85, 87, 89, 92, 97, 100, 102, 106, 108, 111, 114, 117, 120, 122, 124, 126, 128, 130, 132, 134, 136, 138, 140, 142, 144, 146 and 147, preferably 1, 3, 5, 7, 9, 11, 13, 15, 17, 19, 21, 23, 25, 27, 29, 31, 33, 35, 37, 39, 41, 43 and 45, more preferably 1 or 3, and/or the C-terminal intein sequence has at least 70 %, at least 75 %, at least 80 %, at least 85 %, at least 90 %, at least 91 %, at least 92 %, at least 93 %, at least 94 %, at least 95 %, at least 96 %, at least 97 % ,at least 98 %, at least 99 %, at least 99.5 % or 100 % sequence identity with any one of SEQ ID Nos. 2, 4, 6, 8, 10, 12, 14, 16, 18, 20, 22, 24, 26, 28, 30, 32, 34, 36, 38, 40, 42, 44, 46, 49, 52, 54, 56, 59, 62, 64, 66, 68, 71, 75, 77, 79, 81, 83, 86, 88, 90, 93, 98, 101, 103, 107, 109, 112, 115, 118, 121, 123, 125, 127, 129, 131, 133, 135, 137, 139, 141, 143, 145, 148, 149, 150, 151 and 152, preferably 2, 4, 6, 8, 10, 12, 14, 16, 18, 20, 22, 24, 26, 28, 30, 32, 34, 36, 38, 40, 42, 44 and 46, more preferably 2 or 4, the (split) intein is active in the temperature range of 0°C to 80 °C, more preferably in the temperature range of ≤40 °C, more preferably 40 °C or less than 40 °C, more preferably in the range of 0 °C to 37 °C, more preferably the intein has an activity maximum at temperatures of 8 °C or 37 °C, in particular 37 °C. This is ideal to preserve potentially fragile proteins of interest for example in *in vitro* protein labelling experiments.

Moreover, it is surprisingly possible to provide modified (split) inteins with increased splicing yields and/or rates even at temperatures in the range of 0 °C to 40 °C, preferably in the range of 0 °C to 37 °C, in particular at 8 °C or 37 °C.

In addition, the (split) intein is preferably active without the addition of any reducing agent or denaturation step.

Thus, in a further non-limiting embodiment, the present invention relates to one or two isolated polypeptide(s) comprising at least one N-terminal intein sequence and at least one C-terminal intein sequence , wherein the N-terminal intein sequence has at least 95 %, 99 % or 100 % sequence identity with a sequence set forth in SEQ ID NO:3, and/or, wherein the C-terminal intein sequence has at least 95 %, 99 %, or 100% sequence identity with a sequence set forth in SEQ ID NO:4. Such isolated polypeptides provide novel split inteins ideally suited for protein modification and semi-synthesis due to their superior splicing yields and/or rates.

In various non-limiting embodiments of this aspect of the invention, the isolated polypeptide comprises at least one N-terminal intein sequence and at least one C-terminal intein sequence, wherein the N-terminal intein sequence is set forth in SEQ ID NO:3 and the C-terminal intein sequence is set forth in SEQ ID NO:4. Preferably, the N-terminal intein sequence and the C-terminal intein sequence are comprised in separate isolated polypeptides.

Besides their superior splicing yields and/or rates, the novel split inteins are capable of efficient splicing at 37 °C. This characteristic is advantageous as it renders the inteins more thermostable, better suited for expression of fusion proteins in organisms such as E. *coli* and in general broadens their practical utility for a range of applications.

In various non-limiting embodiments, a clear improvement over the wild-type splicing reactions of inteins comprising an N-terminal intein sequence of SEQ ID NO:1 and a C-terminal intein sequence of SEQ ID NO:2 at 8 °C or 37 °C could be observed for the mutants/variants (i.e. the engineered intein mutants/variants, e.g. CL intein) of (split) inteins comprising or consisting of an N-terminal intein sequence having at least 70 %, at least 75 %, at least 80 %, at least 85 %, at least 90 %, at least 91 %, at least 92 %, at least 93 %, at least 94 %, at least 95 %, at least 96 %, at least 97 % ,at least 98 %, at least 99 %, at least 99.5 % or 100 % sequence identity with SEQ ID NO:3 and a C-terminal intein sequence having at least 70 %, at least 75 %, at least 80 %, at least 85 %, at least 90 %, at least 91 %, at least 92 %, at least 93 %, at least 94 %, at least 95 %, at least 96 %, at least 97 % ,at least 98 %, at least 99 %, at least 99.5 % or 100 % sequence identity with SEQ ID NO:4 as described above. Splicing yields were preferably increased, more preferably increased to at least 50 %, more preferably at least 60 %, more preferably to at least 70 %, more preferably at least 80 %, more preferably to at least 90 %, most preferably to approx. 90 or 100 % compared to approx. 30 to 40 % for the wild-type intein.

As used herein the term "wild-type" refers to the phenotype of the typical form of a species as it occurs in nature (here preferably the natural occuring Aes intein). In relation to the provided intein sequences it should be noted that the term can also refer to artificially joined sequences, which themselves possess the wild-type succession of amino acids or nucleotides, respectively.

The term "splicing yield" as used herein refers to the amount of splice product produced. Ideally, the intein mediated (*trans-*)splicing reaction would only result in splice product comprising the extein sequences attached to the intein fragments prior to the reaction. However, under unfavorable conditions or when using an intein, which is not suitable, the formation of cleavage side-product may occur. This lowers the overall splicing yield.

The term "splicing rate" as used herein refers to the change in concentration of the products per unit time. It is expressed using a rate constant *k*. The rate constant, k, which is temperature dependent, is a proportionality constant for a given reaction.

In various non-limiting embodiments, these polypeptides exhibit high splicing yields and/or rates, even if the Int$^N$ and/or Int$^C$ sequences were slightly modified. Moreover, one N-terminal intein sequence is not only capable to splice with its corresponding C-terminal intein sequence, but also with modified variants or completely different intein fragments (cross splicing). In addition, one C-terminal intein sequence is not only capable to splice with its corresponding N-terminal intein sequence, but also with modified variants or completely different intein sequences (cross splicing). Cross splicing, is advantageous as it substantially extends the possibilities for use of both intein fragments.

For example, it is preferred that any one of the first intein amino acid sequences (N-terminal intein sequences) having an amino acid sequence with at least 90 %, at least 95 %, at least 99 %, or 100 % sequence identity with any one of the amino acid sequences set forth in SEQ ID Nos. 1, 3, 5, 7, 9, 11, 13, 15, 17, 19, 21, 23, 25, 27, 29, 31, 33, 35, 37, 39, 41, 43, 45, 48, 51, 53, 55, 58, 61, 63, 65, 67, 70, 74, 76, 78, 80, 82, 85, 87, 89, 92, 97, 100, 102, 106, 108, 111, 114, 117, 120, 122, 124, 126, 128, 130, 132, 134, 136, 138, 140, 142, 144, 146 and 147, preferably in SEQ ID Nos. 1, 3, 5, 7, 9, 11, 13, 15, 17, 19, 21, 23, 25, 27, 29, 31, 33, 35, 37, 39, 41, 43 and 45, more preferably in SEQ ID NO:1 or 3, can be combined with any one of the second intein amino acid sequences (C-terminal intein sequences) having an amino acid with at least 90 %, at least 95 % at least 99 % or 100 % sequence identity with any one of the sequences set forth in SEQ ID Nos. 2, 4, 6, 8, 10, 12, 14, 16, 18, 20, 22, 24, 26, 28, 30, 32, 34, 36, 38, 40, 42, 44, 46, 49, 52, 54, 56, 59, 62, 64, 66, 68, 71, 75, 77, 79, 81, 83, 86, 88, 90, 93, 98, 101, 103, 107, 109, 112, 115, 118, 121, 123, 125, 127, 129, 131, 133, 135, 137, 139, 141, 143, 145, 148, 149, 150, 151 and 152, preferably in SEQ ID Nos. 2, 4, 6, 8, 10, 12, 14, 16, 18, 20, 22, 24, 26, 28, 30, 32, 34, 36, 38, 40, 42, 44 and 46, more preferably in SEQ ID NO:2 or 4 to carry out the splicing reaction.

In one aspect of the invention, the invention relates to two isolated polypeptides as described above, in form of a composition, wherein preferably one of the isolated polypeptides comprises at least one N-terminal intein sequence having at least 70 %, at least 75 %, at least 80 %, at least 85 %, at least 90 %, at least 91 %, at least 92 %, at least 93 %, at least 94 %, at least 95 %, at least 96 %, at least 97 %, at least 98 %, at least 99 %, at least 99.5 % or 100 % sequence identity with any one of the amino acid sequences set forth in SEQ ID Nos. 1, 3, 5, 7, 9, 11, 13, 15, 17, 19, 21, 23, 25, 27, 29, 31, 33, 35, 37, 39, 41, 43, 45, 48, 51, 53, 55, 58, 61, 63, 65, 67, 70, 74, 76, 78, 80, 82, 85, 87, 89, 92, 97, 100, 102, 106, 108, 111, 114, 117, 120, 122, 124, 126, 128, 130, 132, 134, 136, 138, 140, 142, 144, 146 and 147, preferably in SEQ ID Nos. 1, 3, 5, 7, 9, 11, 13, 15, 17, 19, 21, 23, 25, 27, 29, 31, 33, 35, 37, 39, 41, 43 and 45, more preferably in SEQ ID NO:1 or 3, and wherein the second one of the isolated polypeptides comprises at least one C-terminal intein sequence having at least 70 %, at least 75 %, at least 80 %, at least 85 %, at least 90 %, at least 91 %, at least 92 %, at least 93 %, at least 94 %, at least 95 %, at least 96 %, at least 97 %, at least 98 %, at least 99 %, at least 99.5 % or 100 % sequence identity with any one of the amino acid sequences set forth in SEQ ID Nos. 2, 4, 6, 8, 10, 12, 14, 16, 18, 20, 22, 24, 26, 28, 30, 32, 34, 36, 38, 40, 42, 44, 46, 49, 52, 54, 56, 59, 62, 64, 66, 68, 71, 75, 77, 79, 81, 83, 86, 88, 90, 93, 98, 101, 103, 107, 109, 112, 115, 118, 121, 123, 125, 127, 129, 131, 133, 135, 137, 139, 141, 143, 145, 148, 149, 150, 151 and 152, preferably in SEQ ID Nos. 2, 4, 6, 8, 10, 12, 14, 16, 18, 20, 22, 24, 26, 28, 30, 32, 34, 36, 38, 40, 42, 44 and 46, more preferably in SEQ ID NO:2 or 4.

In a further aspect according to the invention, the invention relates to two isolated polypeptides as described above, in form of a composition, wherein preferably one of the isolated polypeptides comprises at least one heterologous N-terminal extein fused to an N-terminal intein fragment having at least 70 %, at least 75 %, at least 80 %, at least 85 %, at least 90 %, at least 91 %, at least 92 %, at least 93 %, at least 94 %, at least 95 %, at least 96 %, at least 97 %, at least 98 %, at least 99 %, at least 99.5 % or 100 % sequence identity with any one of the amino acid sequences set forth in SEQ ID Nos. 1, 3, 5, 7, 9, 11, 13, 15, 17, 19, 21, 23, 25, 27, 29, 31, 33, 35, 37, 39, 41, 43, 45, 48, 51, 53, 55, 58, 61, 63, 65, 67, 70, 74, 76, 78, 80, 82, 85, 87, 89, 92, 97, 100, 102, 106, 108, 111, 114, 117, 120, 122, 124, 126, 128, 130, 132, 134, 136, 138, 140, 142, 144, 146 and 147, preferably in SEQ ID Nos. 1, 3, 5, 7, 9, 11, 13, 15, 17, 19, 21, 23, 25, 27, 29, 31, 33, 35, 37, 39, 41, 43 and 45, more preferably in SEQ ID NO:1 or 3, and wherein preferably the second one of the isolated polypeptides comprises at least one C-terminal extein sequence fused to a C-terminal intein fragment having at least 70 %, at least 75 %, at least 80 %, at least 85 %, at least 90 %, at least 91 %, at least 92 %, at least 93 %, at least 94 %, at least 95 %, at least 96 %, at least 97 %, at least 98 %, at least 99 %, at least 99.5 % or 100 % sequence identity with any one of the amino acid sequences set forth in SEQ ID Nos. 2, 4, 6, 8, 10, 12, 14, 16, 18, 20, 22, 24, 26, 28, 30, 32, 34, 36, 38, 40, 42, 44, 46, 49, 52, 54, 56, 59, 62, 64, 66, 68, 71, 75, 77, 79, 81, 83, 86, 88, 90, 93, 98, 101, 103, 107, 109, 112, 115, 118, 121, 123, 125, 127, 129, 131, 133, 135, 137, 139, 141, 143, 145, 148, 149, 150, 151 and 152, preferably in SEQ ID Nos. 2, 4, 6, 8, 10, 12, 14, 16, 18, 20, 22, 24, 26, 28, 30, 32, 34, 36, 38, 40, 42, 44 and 46, more preferably in SEQ ID NO:2 or 4.

All non-limiting embodiments disclosed above in relation to one isolated polypeptide are similarly applicable to such embodiments where two isolated polypeptides in a composition are used. This particularly applies to the above described combinations of intein sequences and their combination with flanking sequences, wherein the flanking sequences are part of the extein sequences.

Advantageously, the complementary $Int^N$ and $Int^C$ sequences with their respective extein sequences are used together, as thereby the full potential of split inteins is realized, e.g. in applications such as modification of a protein, protein modification with a chemically modified tag sequence, preferably with a Cys tag, protein lipidation, protein immobilization, protein backbone semi synthesis, protein or peptide cyclization, artificial control of protein splicing by light and use as a molecular switch. This is especially the case, if the extein sequences are heterologous to the intein sequences and possibly to each other. As mentioned above in such a case the extein sequences, which can be synthesized separately, will be joined by a peptide bond following the (*trans-*)splicing reaction with virtually no trace of the previously existing intein sequences.

In various non-limiting embodiments, the isolated polypeptide(s) may further comprise a cysteine-containing tag (Cys-tag), which preferably comprises one cysteine residue in its amino acid sequence. Preferably, the Cys tag is comprised in the first extein sequence or in the second extein sequence, preferably in the N-terminal extein sequence or in the C-terminal extein sequence, or the Cys tag is present next to the N-terminal extein sequence or next to the C-terminal extein sequence. Preferably, the Cys tag is either comprised in the N-terminal extein sequence or in the C-terminal extein sequence. Alternatively, the Cys tag is present either next to the N-terminal extein sequence or next to the C-terminal extein sequence, wherein the Cys tag can be linked to the extein sequence or to the intein sequence. It is also possible, that the Cys tag is the N-terminal extein sequence or the C-terminal extein sequence. In various embodiments, the cysteine tag is (pre-)modified or (pre-)labeled, preferably with a (fluorescent) label or protein (e.g. with Alexa 647 maleimide) using e.g. cysteine bioconjugation. Preferably, the label binds to the Cys tag over the one cysteine residue of the amino acid sequence. Preferably, the Cys tag is used to modify or label a protein of interest.

In various non-limiting embodiments, the Cys tag comprises 1 to 20 amino acids, preferably 1 to 15, more preferably 6 or 14. Examples of suitable Cys tag sequences are set forth in SEQ ID NO:280 or 281. However, the Cys tag may comprise any different amino acid sequence based on insertion, modification (substitution) or deletion of one or more amino acids in the sequences set forth in SEQ ID NO:280 or 281. Preferably, the Cys tag has at least 10 % or 20 % or 30 % or 40 % or 50 % or 60 % or 70 % or 80 % or 90 % or 100 % sequence identity with SEQ ID NO:280 or 281.

In various non-limiting embodiments, one cysteine of the Cys tag is selectively modified (e.g. with a fluorescent label). This modified Cys tag is preferably part of the first or second extein sequence according to the invention or it is the first or second extein sequence according to the invention, Preferably by (*trans-*)splicing of the split intein, the modified Cys tag is ligated to another extein sequence, e.g. a protein of interest.

In various non-limiting embodiments, the isolated polypeptide can bind over its Cys tag (over the free thiol group) to a maleimido-coated glass surface (immobilization). Upon addition of the protein of interest fused to the complementary intein sequence and *trans*-splicing the protein of interest would remain bound to the glass surface. Thus, it is possible to preassemble such a glass surface, e.g. with the $Int^N$ sequence, in order to later on immobilize any protein of interest fused to the complementary $Int^C$ sequence. Moreover, $Int^N$ sequence preassembled in such a fashion could act as capture probe array.

In preferred embodiments, the at least one protein of interest or the at least one extein sequence (i.e. the first extein sequence and/or the second extein sequence) is a recombinant protein, an antibody, a nanobody, a protein hormone, or a fragment or homolog thereof.

In another non-limiting embodiment, the intein/intein fragments or isolated polypeptides are used for specific fluorescent labeling of membrane receptors on the surface of host cells, preferably mammalian cells, preferably by using a labeled Cys Tag in the isolated polypeptide according to the invention.

In various non-limiting embodiments of all aspect of the present invention, the isolated polypeptide can be a contiguous *cis* splicing polypeptide. The term *"cis* splicing polypeptide" as used herein refers to a polypeptide, which has a continuous polypeptide sequence, wherein a linker of 1 to 10 amino acids can be present between the C-terminal end of the N-terminal intein amino acid sequence and the N-terminal end of the C-terminal intein amino acid sequence. In some preferred embodiments, the C-terminus of the first intein amino acid sequence and the N-terminus of the second intein amino acid sequence are directly linked to each other by a peptide bond without using a linker.

In other preferred embodiments of all aspects of the present invention, the isolated polypeptide is a *trans* splicing polypeptide, i.e. has a discontinuous polypeptide sequence that needs complementation by another *trans* splicing polypeptide to become active. In such embodiments, each of the *trans* splicing polypeptides as such as well as the combination of both, for example in form of a non-covalently associated complex, is intended to be encompassed by the present invention.

In a further non-limiting embodiment, the present invention relates to one isolated polypeptide or a composition comprising two isolated polypeptides, wherein the polypeptides further comprise at least one component selected from a solubility factor, a marker, a linker, an epitope, an affinity tag, a fluorophore or a fluorescent protein, a toxic compound or protein and a small-molecule or a small-molecule binding protein.

As used herein the term "marker" refers to a component allowing detecting directly or indirectly the molecules having said marker. Thus, in some non-limiting embodiments the marker is a label such as a fluorophore.

As used herein the term "linker" refers to a chemical moiety that connects parts of the isolated polypeptide or the nucleic acids described herein. Thus, a linker may be especially employed in the case *of cis* splicing polypeptides. In various non-limiting embodiments of the present invention, the isolated polypeptide(s) comprise(s) a linker sequence inserted between the at least two intein sequences. The use of these linkers may have the advantage that protein expression of the contiguous *cis* splicing polypeptide is improved. In various non-limiting embodiments, the C-terminus of the N-terminal intein sequence and the N-terminus of the C-terminal intein sequence are linked in a contiguous sequence without or with only minor influence on the three-dimensional structure of the folded Int$^N$/Int$^C$ complex. Preferably, the linker is selected in such a way that it has not a negative effect on the splicing activity of the contiguous sequence. In various non-limiting embodiments of this aspect of the present invention, the linker sequence is selected from the amino acid sequence MG or from the amino acid sequence MGSGGSG (SEQ ID NO:282), without being limited to these examples. One further example of a linker, without limitation, is the amino acid sequence GSH, which is preferably used to create an engineered Int$^C$ fragment, more preferably CL$^C$.

As used herein the term "epitope" refers to an immunogenic amino acid sequence.

As used herein the term "affinity tag" refers to a polypeptide sequence, which has affinity for a specific capture reagent and which can be separated from a pool of proteins and thus can be purified on the basis of its affinity for the capture reagent. In some non-limiting embodiments, the affinity tag is a polyhistidine tag or a Streptag II.

As used herein the term "fluorophore" means a compound or group that fluoresces when exposed to and excited by a light source, i.e. it re-emits light. As used herein the term "fluorescent protein" refers to a protein that is fluorescent, e.g., it may exhibit low, medium or intense fluorescence upon exposure to electromagnetic radiation.

As used herein the term "small molecule" refers to any molecule, or chemical entity, with a molecular weight of less than about 1,000 Daltons.

Preferred solubility factors are, for example, maltose binding protein (MPB), SUMO (small-ubiquitin like modifier), SBP (streptavidin binding peptide) and GST (glutathione-S-transferase).

Especially preferred is MBP as an N-terminal tag. This has the advantage that protein expression and solubility are significantly increased.

Preferred markers are green and red fluorescent proteins (EGFP and mRFP, e.g. mCherry), or *Gaussia princeps* luciferase (Gluc).

In various non-limiting embodiments, the polypeptide(s) comprise(s) or consist(s) of an amino acid sequence having at least 70 %, at least 80 %, at least 85 %, at least 90 %, at least 95 %, at least 99 %, or 100 % sequence identity with any one of the sequences set forth in SEQ ID Nos. 251 to 272 and 275 to 279.

In various non-limiting embodiments, the isolated polypeptide comprising the at least one N-terminal intein sequence has at least 70 %, at least 80 %, at least 90 %, at least 95 % or at least 100 % sequence identity with any one of the sequences set forth in SEQ ID Nos. 251 **(1),** 253 (**3**), 255 (**3a**), 257 (**5**), 259 **(7),** 261 and 262 (**9**), 264 (**10**), 266 (**12**), 268 (**14**), or 269 (**16**) (see Figures 22/23 and 24/25).

In various non-limiting embodiments, the isolated polypeptide comprising the at least one C-terminal intein sequence has at least 70 %, at least 80 %, at least 90 %, at least 95 % or at least 100 % sequence identity with the sequences set forth in SEQ ID Nos. 252 **(2),** 254 **(4),** 256 **(4a),** 258 **(6),** 260 **(8),** 263 (**11**), 265 (**13**), 267 (**15**), 270 (**15a**), 271 (**17**) or 272 (**17a**) (see Figures 22/23 and 24/25).

Preferably, the isolated polypeptides comprising the at least one N-terminal intein sequence and the isolated polypeptide comprising the at least one C-terminal intein sequence are used in combination for *trans*-splicing reactions.

In various non-limiting embodiments of all aspect of the present invention, the isolated polypeptide(s) has/have the highest splicing yield and/or splicing rate at 8 °C or 37 °C.

In a further aspect, the present invention relates to at least one isolated nucleic acid molecule comprising at least one nucleotide sequence encoding one or two isolated polypeptide(s) according to the invention, or a homolog, variant or complement thereof.

The term "variant" or "mutant" as used herein refers to a nucleic acid molecule which is substantially similar in structure and biological activity to a nucleic acid molecule according to one of the claimed sequences and/or refers to a nucleic acid molecule, the sequence of which has one or more nucleotides added, deleted, substituted or otherwise chemically modified in comparison to a nucleic acid molecule according to one of the claimed sequences, provided always that the variant/mutant retains substantially the same properties as the nucleic acid molecule according to one of the claimed sequences.

As used herein, the term "complement" refers to the complementary nucleic acid of the used/known/discussed nucleic acid. This is an important concept since in molecular biology, complementarity is a property of double-stranded nucleic acids such as DNA and RNA as well as DNA:RNA duplexes. Each strand is complementary to the other in that the base pairs between them are non-covalently connected via two or three hydrogen bonds. Since there is in principle - exceptions apply for thymine/uracil and the tRNA wobble conformation - only one complementary base for any of the bases found in nucleic acids, one can reconstruct a complementary strand for any single strand.

However, for double stranded DNA the term "complement" can also refer to the complementary DNA (cDNA). cDNA can be synthesized from a mature mRNA template in a reaction catalyzed by the enzyme reverse transcriptase.

In further non-limiting embodiments of this aspect of the invention, the isolated nucleic acid molecule is comprised in a vector, preferably in a plasmid.

The term "vector", as used herein, refers to a molecular vehicle used to transfer foreign genetic material into another cell. The vector itself is generally a DNA sequence that consists of an insert (sequence of interest) and a larger sequence. The purpose of a vector to transfer genetic information to another cell is typically to isolate, multiply, or express the insert in the target cell.

The term "plasmid", as used herein, refers to plasmid vectors, i.e. circular DNA sequences that are capable of autonomous replication within a suitable host due to an origin of replication ("ORI"). Furthermore, a plasmid may comprise a selectable marker to indicate the success of the transformation or other procedures meant to introduce foreign DNA into a cell and a multiple cloning site which includes multiple restriction enzyme consensus sites to enable the insertion of an insert. Plasmid vectors called cloning or donor vectors are used to ease the cloning and to amplify a sequence of interest. Plasmid vectors called expression or acceptor vectors are specifically for the expression of a gene of interest in a defined target cell. Those plasmid vectors generally show an expression cassette, consisting of a promoter, the transgene and a terminator sequence. Expression plasmids can be shuttle plasmids containing elements that enable the propagation and selection in different host cells.

In further non-limiting embodiments of this aspect, the at least one isolated nucleic acid molecule is comprised in a host cell.

The term "host cell", as used herein refers to a transgenic cell, which is used as expression host. Said cell, or its progenitor, has thus been transfected with a suitable vector comprising the cDNA of the protein to be expressed.

In a further non-limiting embodiment, the present invention relates to a protein expression system comprising an isolated polypeptide as described above or an isolated nucleic acid molecule as described above expressed from a plasmid in a host cell, e.g. *E.coli*.

In another non-limiting embodiment, the host cells are mammalian cells, e.g. HeLa cells.

In yet another aspect, the present invention relates to a method for modifying amino acid sequences, preferably ligating two amino acid sequences to each other, using an isolated polypeptide according to the invention or a composition according to the invention or at least one isolated nucleic acid molecule according to the invention, wherein the modification is preferably selected from the group consisting of modification of a protein, protein lipidation, protein immobilization, protein backbone ligation, protein backbone semi-synthesis, protein or peptide cyclization, protein modification with a chemically modified tag sequence, and artificial control of protein splicing by light.

As used herein the term "protein lipidation" refers to the covalent modification of peptides, polypeptides and proteins with a variety of lipids, including fatty acids, isoprenoids, and cholesterol. Lipid modifications play important roles in the localization and function of proteins.

The term "semi-synthesis" as used herein refers to partial chemical synthesis, i.e. a type of chemical synthesis that uses compounds isolated from natural sources (e.g. plant material or bacterial or cell cultures) as starting materials. This is opposed to a total synthesis where large molecules are synthesized from a stepwise combination of small and cheap (usually petrochemical) building blocks.

The term "backbone semi-synthesis" as used herein refers to generation of a protein consisting of a polypeptide segment derived from recombinant protein expression and a segment obtained by organic peptide synthesis.

In a preferred embodiment of this aspect of the present invention, the isolated polypeptide is modified in such a way that it can be specifically induced to cleave, resulting in a separation of the intein and the N-terminal and/or C-terminal extein sequences. Such a modification can, for example, be achieved via point mutations in at least one of the extein sequences or by omitting one of the extein sequences. Such a system could, e.g. be used for the purification of proteins with the subsequent cleavage of the employed affinity tag. Protein *trans*-splicing (PTS) using inteins is especially well suited for these applications since the protein of interest (POI) will be assembled from two parts (fused as extein sequences to the intein fragments) and each of these fusion constructs can be prepared individually before the PTS reaction. Due to the small size of the split intein fragments, one of the intein fusion proteins "POI- Int$^N$" or "Int$^C$-POI" can be obtained by using solid-phase peptide synthesis. Alternatively, both fusion proteins can be recombinant, but treated individually with bioconjugation chemicals to (regioselectively) introduce a synthetic label. In various embodiments, a nanobody-epitope pair is fused to the N- and C-terminal intein fragments. Preferably, the construct may be labeled using the comprised Cys tag.

Moreover, a method of protein modification employing an isolated polypeptide as described above can be carried out in complex systems like a living cell or a cell extract, preferably mammalian or bacterial cells or cell extract.

In various non-limiting embodiments of this aspect of the present invention, the modification is a protein-terminal modification. In various non-limiting embodiments of this aspect of the present invention, the N-terminal modification is protein labelling.

In various non-limiting embodiments, the isolated polypeptide(s), preferably the N-terminal and C-terminal intein sequences, are used for site-specific chemical modification of protein hormones, nanobody-like molecules, antibody-like molecules, nanobodies, antibodies or fragments or homologs thereof, preferably by site-selective *in vitro* bioconjugation of e.g. nanobodies or the antibody Fc domain, by transferring a short, pre-labeled CysTag in the protein-splicing reaction, under strictly oxidizing conditions, preferably provided by the ubiquitous molecular oxygen.

In a further non-limiting embodiment, the present invention relates to a method for the ligation of at least one first peptide to at least one second peptide using an isolated polypeptide or a composition as described above or an isolated nucleic acid molecule as described above. For instance, the first and the second peptide may be antibody fragments.

In this context it should be noted that the isolated polypeptide as described above or the isolated nucleic acid molecule as described above, can be used to generate one of the functional groups for the ligation reaction e.g. for native chemical ligation (NCL) or expressed protein ligation (EPL) reactions or can be employed to create the peptide bonds itself via intein mediated protein *trans*-splicing.

Thus, in various non-limiting embodiments of this aspect of the present invention, the method comprises covalently linking the C-terminus of the first protein to the N-terminus of the second protein, i.e. it is an intein mediated protein *trans*-splicing reaction.

In this context the isolated polypeptide(s) as described above is/are advantageous, because it allows for ligation at low concentrations and in the presence of other components.

In various non-limiting embodiments of the method according to the invention

(i) the polypeptide(s) according to the invention is/are exposed to oxidizing conditions, preferably it is exposed to atmospheric oxygen; and/or
(ii) the polypeptide(s) according to the invention is/are exposed to a temperature of ≤40 °C, preferably 40 °C or less than 40 °C, more preferably 0 to 37 °C, most preferably 8 or 37 °C; and/or
(iii) the method is essentially free of a reducing step or a reducing agent; and/or
(iv) the method is free of a denaturation step.

Some of the known inteins require a denaturation step to induce the assembly of N-terminal and C-terminal intein sequences, which significantly restricts their scope of application to proteins that can be refolded and prohibits it use in living cells.

In preferred embodiments, the method further comprises the step(s) of

(i) protein preparation, preferably recombinant protein expression or *in vitro* translation; and/or
(ii) protein purification; and/or
(iii) dialysis of expressed/purified proteins.

Suitable methods for protein preparation are already described above. Protein purification is preferably conducted by chromatographic methods, e.g affinity chromatography and/or size exclusion chromatography, without being limited to these methods. Dialysis may, for example, be used to replace parts of a buffer or to concentrate a protein solution.

In yet another aspect, the present invention relates to the use of a polypeptide or a composition according to the invention or at least one nucleic acid molecule according to the invention, wherein the polypeptide or the composition or the nucleic acid molecule(s) are preferably used for modification of a protein, protein lipidation, protein immobilization, protein backbone ligation, protein backbone semi-synthesis, protein or peptide cyclization, protein modification with a chemically modified tag sequence , and use as molecular switch.

Preferably, protein modification comprises (regioselective) protein side chain modification, e.g. covalently joining cells to other cells or other surfaces by proteins that present the split intein parts on the cell outer membrane or attached to the other surfaces.

In various non-limiting embodiments, protein backbone ligation refers to the ligation of two proteins by trans-splicing reactions as described above, wherein preferably one or both proteins may be redox-sensitive, e.g. protein backbone ligation may refer to the ligation of an antibody fragment with a cytokine compound.

In various non-limiting embodiments, the inteins and the intein (fragment)-containing polypeptides described herein can be used for modification of proteins that have therapeutic utility, e.g. are used as pharmaceuticals. Such proteins include, without limitation, antibodies, nanobodies, protein hormones, antibody-like molecules, nanobody-like molecules or fragments or homologs thereof.

In various non-limiting embodiment of the present invention, the modification of a protein is selected from site-selective introduction of synthetic moieties into proteins and N-terminal modification.

In various non-limiting embodiment of the invention, the described polypeptide(s) or nucleic acid molecule(s) is/are used for protein engineering or protein semi-synthesis.

Moreover, inteins have also been recognized as molecular switches that mediate protein splicing as an output signal only when a certain input signal is given. The latter represents the condition under which the intein is active. By fusing additional polypeptides that mediate a protein-protein interaction to the "free" termini of the split intein fragments, the systems can be designed to either work as a biosensor or as an experimental tool to control biological activities that rely on the primary structure of the spliced polypeptide or protein.

All non-limiting embodiments and examples described for the isolated polypeptide according to the invention also apply to the composition comprising isolated polypeptides according to the invention and to the isolated nucleic acid molecule, to the method for modifying amino acid sequences, and to the use of said isolated polypeptide or said composition or said at least one isolated nucleic acid molecule according to the invention, and *vice versa*.

Other non-limiting embodiments are within the following non-limiting examples.

## EXAMPLES

## MATERIALS AND METHODS

### General

Solvents and standard chemical reagents were purchased from Sigma Aldrich, Acros Organics, TCI, Alfa Aesar, Iris or Merck and were used without further purification. Restriction enzymes were from Thermo Scientific. Synthetic oligonucleotides were ordered from Biolegio. Plasmids were verified by DNA sequencing by Seqlab. If not further specified, buffer reagents, antibiotics, media components and other compounds used for cell culture or biochemical assays were purchased from Carl Roth, Applichem, Sigma Aldrich, Fluka or Jena Bioscience.

### Identification of the Aes123 PolB1 intein

The putative sequence of the intein was identified in the sequences stored in Genbank accession codes JN377899, JN377900, JN377902, JN377903, JN377904, JN377905, NC_019543 and MF479730.

### Protein production and purification

All proteins except **12** were produced in E. *coli* BL21(DE3) Gold cells. **12** was produced in E. *coli* Origami cells. Cells were cultured at 37 °C in LB-medium with the corresponding antibiotic until an $OD_{600}$ of 0.6 - 0.8 was reached. Protein expression was induced at 28 °C for 4 h by adding IPTG (0.4 mM). Cell pellets were collected by centrifugation, resuspended in the respective purification buffer and stored at -20 °C till further use. Resuspended cells were ruptured using an Emulsiflex C5 emulsifier (Avestin). Insoluble fractions were removed by centrifugation and the supernatant fractions were used to purify the proteins.

For purification via Ni-NTA affinity chromatography of His-tagged proteins, cell pellets were resuspended in Ni-NTA buffer (50 mM Tris/HCl, 300 mM NaCl, pH 8.0). Purification was performed at 4 °C using flow gravity flow columns with a bed volume of 2 mL of Ni-NTA resin (Cube Biotech). For washing, two steps with Ni-NTA buffer + 20 mM imidazole and one step with Ni-NTA buffer + 40 mM imidazole were performed. Proteins were eluted in six fractions (1 mL) with Ni-NTA buffer + 250 mM imidazole.

For purification via amylose affinity chromatography of MBP-fused proteins, cells were resuspended in amylose column buffer (ACB) (20 mM Tris/HCl, 200 mM NaCl, 1 mM EDTA, pH 7.4). Purification was performed at 4 °C using flow gravity flow columns with a bed volume of ca. 2 mL of amylose resin (New England Biolabs). Before elution the column was washed three times with ACB. Proteins were eluted in six fractions (1 mL) with ACB + 10 mM maltose.

For purification via Strep-tactin affinity chromatography of SBP-fused proteins, cells were resuspended in buffer W (100 mM Tris/HCl, 150 mM NaCl, 1 mM EDTA, pH 8.0). Purification was performed at 4 °C using flow gravity flow columns with a bed volume of ca. 2 mL of Strep-tactin resin (IBA Lifesciences). Before elution the column was washed three times with buffer W. Proteins were eluted in six fractions (1 mL) with buffer W + 2.5 mM desthiobiotin.

For purification via anion-exchange the protein solution was dialysed against MonoQ buffer A (20 mM Tris/HCl, pH 8.0). Anion exchange was perfomed on a Mono-Q PE 4.6/100 column at 4 °C using an ÄKTA Purifier (GE Healthcare). The proteins were eluted with a gradient from 0 to 500 mM NaCl at a flow rate of 0.75 mL/min.

Purified proteins were dialyzed three times against a splice buffer (50 mM Tris/HCl, 300 mM NaCl, 1 mM EDTA, pH 7.0), and finally dialyzed against splice buffer + 10 % glycerol before flash freezing in liquid nitrogen and storage at -80 °C. Protein concentrations were determined using the calculated extinction coefficient at 280 nm.

**Splice assays**

Splicing assays were performed in splice buffer using the described concentrations and at the mentioned temperatures. For determination of splicing rates, one of the intein fragments was used at either a three- or four-fold excess in order to carry out the splicing reaction under pseudo-first order conditions. The splicing reaction was initiated by mixing N- and C-terminal intein fragments. The reaction was stopped at the described time points by taking an aliquot of the reaction mixture and boiling (5 min, 96 °C) the aliquots in 4x SDS sample buffer (500 mM Tris/HCl, 8 % (w/v) SDS, 40 % (v/v) glycerol, 20 % (v/v) 2-mercaptoethanol, 5 mg mL$^{-1}$ bromophenol blue, pH 6.8). For assays containing Fc-dimer fusion proteins, 4x SDS sample buffer without 2-mercaptoethanol was used and the samples were not boiled.

**Densitometric analysis and determination of rate constants**

Coomassie-stained SDS gels were scanned and the signal intensity of Coomassie-stained bands was determined using ImageJ. The signal intensity was normalized to the molecular weight of the protein. The ratio x of the normalized intensities of the splice product and precursor proteins was calculated and inserted in the following equation to determine the splice yield:

$$P[\%] = \frac{(100 * x)}{(1 + x)}$$

The splice yield was plotted against the time and fitted to the following pseudo-first-order equation using Graph-Pad Prism (version 6.01):

$$P_t = P_0 * (1 - e^{-kt})$$

with Pt = yield of product at time t, $P_0$ = maximum yield of product, t = time and k = pseudo-first-order reaction constant.

**Microscale thermophoresis**

Microscale thermophoresis (MST) was carried out on the Monolith NT.115 (NanoTemper, Munich) using splice-inactive intein mutants. **6** was successively diluted from 56.4 $\mu$M to 130 nM. **5** was added to each sample of the dilution series at a final concentration of 170 nM. BSA (0.05% (w/v)) and Tween-20 (0.1% (v/v)) were subsequently added to each sample to prevent aggregation and non-specific adsorption to the MST capillaries. The samples were incubated at 25 °C for 15 minutes before the measurements were conducted. Standard treated MST capillaries were then filled with the samples and measurements were performed using 20% laser intensity for both the blue laser for excitation of eGFP and the IR laser for induction of thermophoresis.

Binding was quantified using the ratio of $F_{hot}$ (fluorescence after thermodiffusion) to $F_{cold}$ (initial fluorescence) resulting in $F_{norm}$. The change in fluorescence was plotted against the concentration of **6** on a logarithmic scale and the subsequent plot was fitted to a sigmoidal curve using *Origin9.0* (OriginLab) to determine the $K_D$.

**Fluorescent labeling of CysTag-fusion proteins**

Purified CysTag-fusion proteins (**7, 11** and **16**) were diluted in splice buffer to a concentration of 25 $\mu$M and incubated with EDTA (5 mM) and TCEP (100 $\mu$M) for 20 min at 37 °C. The required fluorophore-maleimide conjugate (Alexa647-C2 maleimide or Fluorescein-5 maleimide, Thermo Fisher Scientific) was added at a final concentration of 200 $\mu$M and the reaction mixture was incubated for 2 hours at 25 °C for **11** and on ice for **7** and **16.** The reaction was quenched with DTT (2 mM) and the unreacted fluorophore was removed by affinity chromatography on Ni-NTA resin.

**Fluorescent labeling of nanobodies**

The fluorescently labeled CysTag-fusion protein (**7a** or **11a**) (15 $\mu$M) and the nanobody-construct (**8** or **10**) (10

μM) were mixed in splice buffer and incubated at 37 °C for 6 hours. The reaction mix was loaded on a Strep-tactin-column and the labeled nanobody was collected in the flow through. The other bound components in the reaction mix were eluted with buffer W + 2.5 mM desthiobiotin. The purified and labeled nanobody was dialyzed against splice buffer.

**Creation of bispecific nanobody**

The nanobody constructs (**8** and **10**) were mixed in splice buffer and incubated at 37 °C for 6 hours. The reaction mix was loaded on a Strep-tactin-column and the labeled nanobody was collected in the flow through. The other bound components in the reaction mix were eluted with buffer W + 2.5 mM desthiobiotin. The purified and labeled nanobody was dialyzed against splice buffer.

**Labeling of Fc-dimer fusion protein**

**12** (10 μM) and 11b (30 μM) were mixed in splice buffer and incubated at 37 °C for 6 hours. The reaction mix was loaded on a Strep-tactin-column and the labeled nanobody was collected in the flow through. The other bound components in the reaction mix were eluted with buffer W + 2.5 mM desthiobiotin. The purified and labeled Fc-dimer fusion protein was dialyzed against splice buffer.

**Solid phase peptide synthesis of Fluorescein-CL$^N$**

The peptide was assembled on Wang resin by stepwise microwave assisted Fmoc-SPPS on a Liberty peptide synthesizer, operating on a 0.1 mmol scale. Activation of entering Fmoc-protected amino acids (Merck Millipore or Iris Biotech) was performed using Oxyma Pure and DIC in DMF (1:1 molar ratio), with a 4 equivalent excess over the initial resin loading. Coupling steps were performed either for 45 minutes at 35 °C, 15 minutes at 50 °C or 5 minutes at 70°C. Capping steps were introduced after each coupling step and performed by treatment with a 0.3 M acetic anhydride/ 0.3 M $i$Pr$_2$NEt solution in DMF. Fmoc-deprotection steps were performed by treatment of the resin with a 20% piperidine solution in DMF for 10 minutes at room temperature. Following each coupling, capping or deprotection step, the resin was washed with DMF. Fluorescein was manually coupled to the peptide by adding a solution of 5(6)-Carboxyfluorescein-OH (1.5 eq.) (Sigma Aldrich), DIC (1.5eq.) and HOAt (1.5 eq.) in DMF to the resin and shaking at room temperature for 16 hours. The resin was subsequently washed with DMF and DCM, and dried under nitrogen flow. The labelled peptide was finally cleaved off the resin by treatment with an ice-cold TFA, TIS, water mixture (90:5:5) and allowed to shake at room temperature for 2 hours, followed by purification by RP-HPLC.

**Cell culture and transfection**

HeLa cells were cultured in MEM supplemented with Earle's salts, that was further supplemented with 10% fetal calf serum, 1% non-essential amino acids, 1% L-glutamine and 1% penicillin/streptomycin at 37 °C under 5 % CO$_2$. Transfection was carried out using Lipofectamine 2000 (Thermo Fisher Scientific) using the manufacturer's instructions.

**Labeling of HeLa cells with nanobodies**

For labeling with CysTag(AF647)-V$_H$H$^{GFP}$-H$_6$, HeLa cells transfected with pMBH63 (encoding eGFP-Trx-TMD-mCherry) were washed with PBS and fresh culture medium containing 1 nM CysTag(AF647)-V$_H$H$^{GFP}$-H6 was added to the cells. The cells were incubated for 5 minutes at 4 °C, followed by washing with PBS.

For labelling with V$_H$H$^{EGFR}$-CysTag(AF647)-H6, HeLa cells transfected with pMBH65 (encoding HA-EGFR-mCherry) were washed with PBS and fresh culture medium containing 10 nM V$_H$H$^{EGFR}$-CysTag(AF647)-H$_6$ was added to the cells. The cells were incubated for 20 minutes at 4 °C, followed by washing with PBS.

For labelling with the bispecific nanobody V$_H$H$^{EGFR}$-V$_H$H$^{GFP}$-H$_6$, HeLa cells transfected with pMBH65 (encoding HA-EGFR-mCherry) were washed with PBS and fresh culture medium containing 10 nM V$_H$H$^{EGFR}$-V$_H$H$^{GFP}$-H$_6$ was added to the cells. The cells were incubated for 20 minutes at 4 °C, followed by washing with PBS. The cells were subsequently incubated with 1 nM eGFP for 5 minutes at 4 °C and washed again with PBS.

**Cell-surface labeling by *trans*-splicing**

HeLa cells transfected with plasmids (pMBH41, pMBH42, pMBH53, pRS009 or pRS028) to express the defined cell surface receptor were washed with PBS and fresh culture medium containing the specified CL$^N$-fusion protein (**14** or **16a**) at the defined concentration was added to them. The cells were then cultured at 37 °C under 5 % CO$_2$ for the specified time durations. The cells were then washed with PBS and further processed either for confocal microscopy or SDS-PAGE analysis and western blotting.

**Detection of cell-surface labeling by western blotting and fluorescence detection**

Cells subjected to cell-surface labeling by trans-splicing were washed three times with PBS and 100 $\mu$l RIPA buffer (Thermo Fisher Scientific) supplemented with Halt Protease Inhibitor (Thermo Fisher Scientific) was added to each cell culture plate. Cells were then scraped off the plates and collected in microcentrifuge tubes. The cell suspension was incubated for 30 minutes at 4 °C under constant agitation to induce cell lysis. Cell lysates were then centrifuged at 15,000 g and the supernatant fraction was collected. Laemmli buffer was then added to the supernatant and the samples were boiled at 98 °C for 10 minutes and subjected to polyacrylamide gel electrophoresis on 15% SDS-PAGE gels. Visualization of fluorescent signals when carrying out labeling assays with **16a** was carried out by imaging the gels using a Typhoon FLA 9500 (GE Healthcare). For western blotting of cell surface labeling assays carried out with **14,** SDS-PAGE separated samples were transferred to PVDF membranes and probed by an anti-GFP primary antibody (Santa Cruz Biotechnology) and detected by chemi-luminescence imaging using an HRP-conjugated secondary antibody (Dako).

**Immunostaining**

HeLa cells transfected with pMBH41 were washed with PBS and fixed with 4 % paraformaldehyde for 20 minutes at room temperature. The cells were washed again with PBS and blocked with 3 % bovine serum albumin (BSA) in PBS for 20 minutes at room temperature. The cells were subsequently incubated with a solution of anti-hemagglutinin A tag primary antibody (Santa Cruz Biotechnology) in PBS containing 3 % BSA for 1 hour at 37 °C, and then washed with PBS. This was flowed by an incubation with a solution of Dy633-labeled anti-mouse secondary antibody (Thermo Fisher Scientific) in PBS containing 3 % BSA for 1 hour at 37 °C. Cells were then washed with PBS and fixed with 4 % paraformaldehyde for 20 minutes at room temperature, followed by washing with PBS. The coverslips on which the cells were plated were finally mounted on slides using Aqua/Poly-Mount mounting solution (Polysciences).

**Confocal laser scanning microscopy**

HeLa cells subjected to cell-surface labeling either with nanobodies or via trans-splicing were washed with PBS and fixed with 4 % paraformaldehyde for 20 minutes at room temperature. Cells were washed three more times with PBS and the coverslips on which the cells were plated were mounted on slides using Aqua/Poly-Mount mounting solution (Polysciences). Confocal microscopy was carried out using a 63X water-immersion objective lens on a Leica DMi8 system.

**Mass spectrometry**

Peptide **9** was analyzed using an Agilent 1260 Infinity series system (Agilent Technologies, Waldbronn, Germany) with a C18 column (ZORBAX SB-C18 RR HT, 3 x 50 mm, 1.8 $\mu$m, Agilent Technologies, Waldbronn, Germany).
Mass analyses of all proteins were carried out using an UltiMate™ 3000 RS system (Thermo Fisher Scientific Inc., MA, USA), along with maXis II UHR-TOF mass spectrometer (Bruker Daltonik GmbH, Bremen, Germany) equipped with a standard ESI source (Apollo, Bruker Daltonik GmbH, Bremen, Germany).
All mass spectroscopic data were analyzed with the software DataAnalysis 4.4 (Bruker Daltonik GmbH, S26 Bremen, Germany).

**Example 1**

**The Aes123 PolB1 intein (Aes intein) is a novel cysteine-less intein split at the standard endonuclease position**

Cysteine-less split inteins were identified in PolB-type DNA polymerase genes from T4-like bacteriophages of *Aeromonas salmonicida*[a] and from megaphages of *Prevotella* in guts of humans, baboons, and pigs (43). The *Aeromonas* bacteriophage Aes123 PolB1 split intein was chosen for further characterization. It was predicted to consist of fragments of lengths 120 aa (Aes$^N$) and 39 aa (Aes$^C$) and contain serines at the catalytic 1 and +1 positions (Figure 2A). The split site position corresponds to the typical break point of split inteins (44), where many contiguous inteins carry an inserted homing endonuclease domain (45, 46). For functional characterization the intein fragments were produced as recombinant fusion proteins in *Escherichia coli* using the model extein sequences maltose-binding protein (MBP) and streptavidin-binding peptide (SBP) (Figure 2B). Mixing of the purified complementary constructs in equimolar ratio led to spontaneous and rapid protein *trans*-splicing (Figure 2C) with no detectable side reactions in form of N- or C-terminal cleavage. However, whereas the Aes$^C$ partner **(2)** could be nearly completely consumed in presence of excess Aes$^N$ fusion protein **(1),** only partial consumption up to 30% of the Aes$^N$ part **(1)** could be achieved when reversing the stoichiometric ratio (Figure 2C). Quantitative analysis of the trans-splicing reaction between 10 $\mu$M **1** and 30 $\mu$M **2** revealed splicing rates of 21.6 $\pm$ 3.9 x 10$^{-3}$s$^{-1}$ and 23.0 $\pm$ 4.8 x 10$^{-3}$s$^{-1}$ at 37 °C and 8 °C, respectively (Figures 2D, Figures 3A and B).

### The CL intein: an engineered high-yielding variant with an atypically split site

To overcome the low yield of protein *trans*-splicing of the Aes$^N$ fragment, presumably a result of partial misfolding, engineering the intein halves by shifting the intein split point was intended. Sequence and structure alignments suggested the region between residues -25-40 to be an insertion compared to the sequence of the Ssp DnaB intein (Figures S2A). Thus, first the native Aes$^N$ and Aes$^C$ fragments were fused with a short linker (glycine-serine-histidine), and then created new fragments, termed CL$^N$ and CL$^C$, encompassing residues 1-26 (CL$^N$) and residues 34-159 (CL$^C$) (Figures 4A and 5B). This engineered variant of the Aes intein is referred to as CL intein (cysteine-less). To our delight, the protein *trans*-splicing reaction of the purified fusion proteins MBP-CL$^N$-H$_6$ **(3)** and SBP-CL$^C$-Trx-H$_6$ (**4,** Trx = thioredoxin, Figure 4B) proceeded to nearly quantitative consumption of each intein half at 37° C (Figure 4C). Only slight amounts of C-terminal cleavage were observed (Figure 4C). Protein *trans*-splicing proceeded robustly over a range of temperatures and even when the proteins were incubated at 0 °C. It was found that at lower temperatures the levels of undesired cleavage reaction were further reduced to give only trace amounts at best (Figure 4D). Analysis of the trans-splicing reaction between 10 $\mu$M **3** and 40 $\mu$M **4** gave splicing rates of 0.60 $\pm$ 0.04 x 10$^{-3}$s$^{-1}$ and 0.14 $\pm$ 0.01 10$^{-3}$s$^{-1}$ at 37 °C and 8 °C, respectively (Figure 4E, Figures 6A and B, respectively). The lower rates of the CL intein compared to the Aes intein could reflect structural perturbations introduced due to the artificial split site and concomitant sequence changes. An affinity of 1.7 $\pm$ 0.1 $\mu$M was determined using microscale thermophoresis for the interaction between the CL$^N$ and CL$^C$ fragments inactivated for splicing by point mutagenesis (Figure 4F). Importantly, the CL intein was completely redox-insensitive. No reducing agent was added to the protein *trans*-splicing reaction and it could also be omitted during the purification steps of the CL$^N$ and CL$^C$ fragments. Reducing agents in typical concentrations (1 mM TCEP) were tolerated and even high concentrations of nucleophilic DTT (50 mM), that typically cleave the intermediary thioesters in protein splicing, had no effect on the splicing of the CL intein (Figures 7C and D). To further mechanistically characterize the intein, both catalytic serines at the upstream and downstream splice junctions were mutated (Ser1 in the CL$^N$ and Ser+1 downstream of the CL$^C$ fragment) to cysteines and protein *trans*-splicing in all four possible combinations was tested. No splicing above negligible levels except for the native Ser1/Ser+1 combination was detected. These findings indicated that the CL intein, and by extension, the Aes inteins, are highly specialized for oxoester intermediates (Figure 7). Together, the engineered CL intein was capable of efficient *trans*-splicing under native non-reducing conditions at a range of temperatures.

### Selective N-terminal chemical modification of proteins under oxidizing conditions using the CL intein

Next, the use to split CL intein for selective and reduction-free protein conjugation on cysteine-containing proteins was aimed using the CysTag approach. Nanobodies are single domain antibody fragments derived from Camelidae species with immense potential for a variety applications in imaging, therapy and basic research (47, 48). They contain one or more disulfide bonds, which in many cases are indispensable for stability and antigen recognition. Figure 8A illustrates the novel implementation of the split CL intein into the protocol for N-terminal chemical modification, as applied to the GFP-binding *enhancer* nanobody (V$_H$H$^{GFP}$) (49). The fusion proteins CysTag-CL$^N$-SBP **(7)** and SBP-CL$^C$-V$_H$H$^{GFP}$-H$_6$ **(8)** were produced in E. *coli* and purified using streptactin and Ni-NTA affinity chromatography, respectively. The short CysTag (6 aa; MGCDTD) contains a single cysteine that was subsequently modified with AlexaFluor647-maleimide to give CysTag(AF647)-CL$^N$-SBP **(7a),** as confirmed by MS (Figure 9A). Subsequently, **7a** was purified by affinity chromatography using streptactin beads to separate off excess labeling reagent and reducing agent. To transfer the labeled CysTag to the POI both proteins were incubated in the absence of reducing agents. Analysis of an SDS gel by fluorescence imaging and Coomassie-staining showed that after 6 h the CL$^N$-containing precursor was consumed by protein trans-splicing and a new protein corresponding to the labeled nanobody CysTag(AF647)-V$_H$H$^{GFP}$-H$_6$ with the expected molecular weight of 16.4 kDa was formed (Figure 8B). The labeled nanobody was then purified from the reaction mixture by an inverse affinity chromatography on streptactin resin (Figure 8B). To test for functionality in binding its GFP epitope, the labeled nanobody (1 nM) was applied on HeLa cells, which were transfected with a plasmid encoding HA-eGFP-Trx-TMD-mCherry (TMD = transmembrane domain of the PDGF receptor) to present eGFP on the cell surface. A specific labeling around the membrane was observed only for the transfected cells (Figure 8C). The efficient binding of the labeled nanobody at such a low concentration close to its K$_d$ value (around 1 nM)(49, 50) indicated that its binding capacity was completely preserved.

It was also sought to facilitate N-terminal chemical modification by using semi-synthetic protein *trans*-splicing with a fully synthetic CL$^N$ peptide. The fluorescein-coupled fragment Fl-CL$^N$ (**9**; Fl = 5,6-carboxyfluorescein) was prepared by solid-phase peptide synthesis (Figures 8D and E) and incubated it with SBP-CL$^C$-Trx-H$_6$ (**4;** Trx = thioredoxin) in the absence of any reducing agents (Figure 10A). Efficient trans-splicing generating the splice product Fl-Trx-H$_6$ was observed by SDS-PAGE analysis followed by fluorescence imaging and Coomassie staining (Figure 10B). It was found that protein *trans*-splicing using 40 $\mu$M **9** and 10 $\mu$M **4** occurred very rapidly with a rate of 1.6 $\pm$ 0.1 x 10$^{-3}$s$^{-1}$ (Figure 8F) and virtually quantitative conversion of the Trx precursor to the product labeled with fluorescein could be achieved.

**Selective C-terminal chemical modification of proteins under oxidizing conditions using the CL intein**

The CL intein provides the potential to perform the two-step CysTag protocol not only for N-terminal but also for C-terminal chemical modification. For C-terminal labeling the CysTag protein SBP-CL$^C$-CysTag-H$_6$ **(11)** was devised, and produced in E. *coli.* Following purification, the single thiol group in the CysTag sequence (14 aa; SVYASPAAPAPASC) was conjugated with AlexaFluor647-maleimide to obtain SBP-CL$^C$-CysTag(AF647)-H$_6$ **(11a;** Figure 11A), as verified by ESI-MS (Figure 12A). The anti-EGFR nanobody EgA1 (V$_H$H$^{EGFR}$) was selected for C-terminal labeling (51). Structural analysis of the complex between this nanobody and EGFR (PDB ID: 4KRO) revealed that the N terminus of the nanobody is located very close to the EGFR surface, suggesting that the conjugation of a fluorophore is likely to be less perturbing on the C terminus of the nanobody. The nanobody was produced as the construct V$_H$H$^{EGFR}$-CL$^N$-SBP **(10)** and it was purified from *E. coli.* It was observed that incubation with **11a** resulted in successful transfer of the pre-labeled CysTag to the nanobody (Figure 11B). It was possible to purify the labeled nanobody using inverse affinity purification on a streptactin resin, as confirmed by SDS-PAGE and ESI-MS analyses (Figures 11B; 12C). To functionally characterize the labeled EgA1 nanobody, it was added at 10 nM concentration to HeLa cells transfected with a plasmid encoding HA-EGFR-mCherry. A staining restricted to transfected cells was observed, clearly demonstrating the specific binding of the nanobody (Figure 11C).

**Preparation of selectively labeled Fc fragment under oxidizing conditions using the CL intein**

Next, it was aimed to chemically modify an Fc fragment derived from a human IgG antibody. Compared to a single-domain nanobody, the disulfide bonds in an Fc fragment are more critical as they are crucial for correct connectivity between the two heavy chains. The Fc fragment of an IgG1 chain including the hinge region as the recombinant fusion protein MBP-Fc-CL$^N$-SBP **(12)** was produced in E. *coli* Origami cells and it was purified using streptactin resin without exposure to any reducing agent. Analysis of the purified **12** by non-reducing SDS-PAGE showed two bands (Figure 13B, left lane). The lower migrating band at -75 kDa correlated with the calculated size of the reduced monomeric chain (77.3 kDa). The upper and more intense band likely corresponded to the desired homodimer. Its unusual migration behavior of > 200 kDa would be a result of the branched connectivity of the two polypeptide chains. The presence of the monomeric and dimeric species was confirmed by ESI-MS analysis (Figure 14). The data is consistent with only one intramolecular disulfide bond in the dimeric species and suggests that the second cysteine in the hinge region remained unpaired, likely due to incomplete oxidation during expression in *E. coli.* The cysteine in the complementary intein construct SBP-CL$^C$-CysTag-H$_6$ **(11)** was biconjugated with 5-carboxyfluorescein maleimide to give SBP-CL$^C$-CysTag(fluorescein)-H$_6$ **(11b)** and then the modified CysTag was transferred to the C terminus of the Fc fragment by the protein *trans*-splicing reaction. By-products of splicing and remaining traces of unspliced precursor were removed by inverse affinity purification on a streptactin column to furnish the virtually pure, C-terminally labeled and homodimeric Fc fragment (Figure 13B). Notably, the minor monomeric Fc species present in the initial preparation had disappeared nearly completely in the final product, most probably due to oxidation by oxygen during storage and the splicing reaction. The formation of the desired, doubly labeled Fc fragment was confirmed by an ESI-MS analysis of the purified protein fraction (Figure 13C).

**Production of bi-specific nanobodies under oxidizing conditions using the CL intein**

Protein trans-splicing under oxidizing conditions should also enable the fusion of two disulfide containing proteins without disulfide scrambling caused by temporary reduction. Therefore, it was attempted to create bi-specific nanobodies by incubating the above-described nanobody constructs V$_H$H$^{EGFR}$-CL$^N$-SBP **(10)** and SBP-CL$^C$-V$_H$H$^{GFP}$-H$_6$ **(8)** (Figure 15A). Indeed, the dimeric nanobody fusion protein V$_H$H$^{EGFR}$-V$_H$H$^{GFP}$-H$_6$ was obtained by protein *trans*-splicing and it was efficiently purified using streptactin resin (Figure 15B), as verified by ESI-MS (Figure 16). 10 nM of the bispecific nanobody were added to HeLa cells transfected with a plasmid encoding HA-EGFR-mCherry, followed by incubation with 1 nM eGFP. Using confocal microscopy, distinct labeling of transfected cells with eGFP was observed, which confirmed that both nanobodies retained their functionality (Figure 15C).

**Chemical modification of cell-surface proteins with a minimal tag using nanobody-mediated high-affinity split intein complementation**

Afterwards, the application of protein trans-splicing to extracellular domains of cell-surface receptors under oxidizing conditions was explored. It was reasoned that a chemical labeling approach that preserves disulfide bonds would be highly valuable for this purpose, given that many extracellular regions of *trans*-membrane or membrane-anchored proteins contain disulfide bonds important or critical for structural integrity. Labeling of receptors with small, bright and photostable fluorophores is very important for fluorescent microscopy techniques, in particular for single molecule studies (52, 53). Prominent existing techniques for this rely on fusion with large proteins such as the HaloTag and SNAP-tag (54, 55). However, these tags are of considerable size and thus potentially interfere with receptor function. Previous reports to overcome this limitation by exploiting virtually traceless protein *trans*-splicing of very short fluorophore-conjugated tags used split inteins that contained catalytic cysteines and hence required the presence of reducing agents (21, 22, 56-58).

To perform receptor labeling with the new CL intein, HeLa cells were first transfected with a plasmid encoding a model receptor consisting of the transmembrane domain of the platelet derived growth factor receptor (TMD) with a C-terminal intracellular mCherry fusion and an N-terminal extracellular HA-CL$^C$-Trx fusion (HA-CL$^C$-Trx-TMD-mCherry, **13**; HA = hemagglutinin A tag, Figure 17A). The sandwiched Trx domain was previously found to support correct localization of such model receptor constructs in the plasma membrane and increase protein splicing activity (19, 21). However, upon treating these cells with the fusion protein eGFP-CL$^N$-H$_6$ **(14)** at concentrations up to 5 $\mu$M, neither any specific eGFP signal on the transfected cells via confocal microscopy (Figure 17B) nor the formation of the splice product by western blot (Figure 17C) were detected. Using immuno-fluorescence analysis, the extracellular N-terminal HA-tag on the model receptor in transfected cells was clearly detected, which confirmed that the receptor was correctly displayed on the cell membrane (Figure 17D). It is hypothesized that the lack of splicing could result from the comparably low interaction affinity between the intein fragments (see above), thus precluding the formation of the splicing-active intein complex at the low levels at which the receptor is present on the cell surface. It is rationalized that complementation of the split intein fragments could be enabled by recombinantly fusing two high affinity interaction partners on either fragment of the CL intein (Figure 18A) (59). In this approach, the two high-affinity interaction partners would first associate leading to a high local concentration of the two intein fragments, thus promoting intein complementation. To this end, the anti-GFP-nanobody Enhancer (V$_H$H$^{GFP}$) (49) was recombinantly fused on the N terminus of the receptor to yield a construct encoding HA-V$_H$H$^{GFP}$-CL$^C$-Trx-TMD-mCherry **(15,** Figure 18A). Upon incubating transfected HeLa cells producing **15** with eGFP-CL$^N$-H$_6$ **(14),** rapid and specific recruitment of eGFP protein to the cell membrane was observed via fluorescence microscopy (Figure 18B). It could be confirmed that the splice product with eGFP was formed by western blot analysis, whereas no splice product could be observed in a control experiment with a N159'A splicing mutant of the CL$^C$ construct **(15a)** (Figure 18C). Splice product formation was still detected with concentrations of **14** as low as 100 nM.

Next, it was attempted to extend this strategy to label receptors with small organic fluorophores using the above-described CysTag concept (Figure 19A). For this, the protein CysTag-H$_8$-CL$^N$-eGFP(C49S) **(16)** was created and labeled with maleimide Alexa647 to give **16a.** In this construct, eGFP was C-terminally fused to the CL$^N$ fragment to have it removed with the intein fragments during the protein *trans*-splicing reaction. The surface-exposed C49 was mutated to avoid chemical labeling. Indeed, when cells expressing the construct HA-V$_H$H$^{GFP}$-CL$^C$-Trx-TMD-mCherry **(15)** were incubated with **16a** (150 nM), a specific Alexa647 signal accumulation on the cell membrane was detected by fluorescence microscopy (Figure 19B). An SDS-PAGE analysis of cell lysates with a fluorescence read-out was performed that showed the occurrence of a new band that corresponded to the fluorescently labeled receptor as the splice product. Significantly, this band was not observed in control reactions using untransfected cells, the model receptor lacking the V$_H$H$^{GFP}$ fusion **(13),** or the model receptor containing an inactive intein mutant **(15a)** (Figure 19C). Together, these results established the successful cell-surface *trans*-splicing of the CL intein mediated by a high affinity nanobody-epitope pair on an artificial model receptor protein.

Finally, a full-length receptor without artificial fusions of solubility-conferring domains was chosen as a biologically relevant test case. The interferon receptor subunit IFNAR1 was selected for these experiments. Heterodimerization of IFNAR1 and IFNAR2, which is mediated by several protein ligands belonging to the Type I interferon family, is known to be crucial for diverse cellular processes, particularly in the context of the anti-viral immune response and cell proliferation (60). HeLa cells were transfected with a plasmid coding for HA-V$_H$H$^{GFP}$-CL$^C$-IFNAR1-mCherry **(17)** (Figure 20A). Notably, in this arrangement the CL$^C$ fragment is fused with a minimal linker of only 11 amino acids directly to the N terminus of IFNAR1. Following addition of CysTag(AF647)-H$_8$-CL$^N$-eGFP(C49S) **(16a)** at 150 nM to the cells, a specific Alexa647 signal on transfected cells could be observed via confocal microscopy (Figure 21). Compared to the model receptor, the labeled IFNAR1 fusion protein was observed to be strongly localized to intracellular vesicular structures. Endocytosis mediated internalization of IFNAR1 is known to occur rapidly even in the absence of interferon stimulation (61), which suggests that the observed vesicular structures were endosomes. A fluorescent band could be detected corresponding to the splice product Alexa647-labeled IFNAR1 following SDS-PAGE analysis of cell lysates from this experiment, but not in a control experiment using protein **17a** with an inactive CL$^C$ fragment (Figure 20B). In order to optimize the experimental conditions, the duration of time used for the splice reaction could be reduced to only 30 min and the concentration of **16a** to 10 nM with an almost equal amount of splice product still being formed (Figures 20B and 20C).

Thus, it was demonstrated that the CL intein can be used to selectively label cell surface receptors with a synthetic fluorophore on a very short peptide tag. The reactions were carried out within short time periods and using very low concentrations of the labeled CL$^N$ fragment, which not only enables low background fluorescence microscopy but also reduces experimental costs. Notably, all reactions were performed in the strict absence of any reducing agents to preserve disulfide bonds in the extracellular domains of cell surface proteins.

In conclusion, the first mesophilic split intein that is completely free of cysteines and can splice under native conditions has been discovered. While the N-terminal fragment of the naturally split Aes123 PolB1 intein could not be consumed to more than 30-40 % in a protein *trans*-splicing reaction, virtually complete splicing was achieved by shifting the split position close to the N-terminal end in a structural reengineering effort. The new split point of the CL$^N$ fragment after 26 amino acids is reminiscent of the fragment architecture of the recently discovered atypically natural split inteins like AceL TerL and GOS TerL inteins, which have Int$^N$ fragments of 25 and 37 amino acids, respectively (10, 62). However, the re-engineering of the split site is remarkable because the introduction of artificial split sites into naturally split inteins typically diminishes their activity (11, 63, 64) and additional protein engineering efforts are required to increase their activity (65). The re-engineering of the Aes123 PolB1 intein was further complicated by the fact that this intein shares only very low sequence similarity with characterized inteins.

The resulting engineered split CL intein is an efficient new tool to link two polypeptide chains in a redox-independent manner. It should prove useful whenever reducing conditions are to be avoided, for example when sensitive proteins or probes are present in an extein, or when reducing conditions cannot be established, for example in oxidative cellular compartments and extracellular spaces of whole organisms or ecological habitats. Furthermore, the absence of thioester intermediates in the protein splicing pathway of the CL intein explains its unusual resistance to 50 mM DTT and hence could be useful to perform protein *trans*-splicing reactions in the presence of high concentrations of nucleophiles.

The CL intein was utilized for efficient and site-selective *in vitro* bioconjugation of nanobodies or antibody domains (e.g. Fc) by transferring a short, pre-labeled CysTag in the protein-splicing reaction under strictly oxidizing conditions provided by the ubiquitous molecular oxygen. Previously, first versions of the CysTag strategy have been reported for site-selective protein C- and N-terminal modification (16, 31, 40, 41). However, while the absence of cysteines in one fragment allowed the use of the CysTag strategy for bioconjugation, the presence of a catalytic cysteine on the complementary fragment necessitated the presence of reducing agents during the *trans*-splicing reaction to transfer the label to the POI. This severe limitation has now been overcome with the split CL intein. An important advantage of the CysTag protocol is that well-established, commercially available and affordable maleimide and iodacetamide reagents can be employed. The CysTag can be as short as a few amino acids and its only strict sequence requirement is the presence of a single cysteine.

Furthermore, a high affinity version of the split CL intein was developed by fusing a nanobody-epitope pair to the N- and C-terminal intein fragments. Importantly, this novel proximity-conferring dimerization system can be encoded fully recombinantly (67), in contrast to previous semi-synthetic interaction pairs (57, 68), and exploits the low nanomolar affinity of the anti-GFP nanobody/eGFP complex (49, 50). Specific fluorescent labeling of membrane receptors on the surface of mammalian cells was possible with the labeled CysTag-intein part added to the growth medium in concentrations as low as 10 nM. While fluorescently labeled high-affinity nanobodies have been previously used to target proteins for microscopic analysis (69, 70), the key advantage of this new approach is the combination with the self-excision property of inteins to enable covalent and nearly traceless labeling. Depending on the desired application, the CysTag approach with the high-affinity split CL intein should therefore be superior or at least complementary to existing technologies like self-labeling enzymes or enzymatically labeled tags (54, 55). Given the membrane-impermeability of the exogenously provided, chemically labeled intein fragment, the reported approach will strictly label receptors on the outside of cells and hence may be used to track uptake mechanisms by endocytosis.

In conclusion, these results show the great potential of the novel split CL intein and significantly advance the scope of protein *trans*-splicing technologies to enable the manipulation of purified proteins and proteins on living cells under oxidizing conditions.

### Example 2

#### 1) Additional data showing that the CL intein can splice with full-length IgG antibodies

To further test the potential of the CL intein for modifying antibodies or antibody fragments a fusion construct of the Int$^N$ fragment with the C-terminus of the heavy chain (HC) of a full length human IgG1 antibody was prepared. The fully human IgG1 monoclonal antibody directed against the human insulin-like growth factor-1 receptor (IGF-1R), also known as Cixutumumab (IMC-A12), was chosen. Two different Int[C] fusion proteins were added to the purified antibody fusion, one containing E. *coli* thioredoxin (Trx) as a prototype for a globular protein, and one containing the streptavidin binding peptide (SBP) as a prototype for a short tag sequence. Figure 26 shows that both constructs spliced virtually quantitatively to give the splice products HC-Trx-His6 and HC-SBP, respectively.

**2) Additional data showing that the PolB-16_OarG is fully active and can be rendered completely free of cysteine residues**

In addition to the above-described CL intein, more natively split inteins without any cysteine residues at the catalytically critical positions were identified. Some of these inteins have one or more cysteine residue(s) at non-conserved positions, whereas other are entirely free of cysteines. The split PolB-16_OarG intein contains two cysteine residues at non-conserved positions in the Int[C] fragment. Its intein fragments were fused to model sequences as shown in Figure 27A to investigate its activity. The proteins were obtained from overproduction in *E. coli* and subsequent affinity purification using Ni-NTA chromatograpy. Figure 27B shows that the unmutated, wild-type intein fragments were fully active in protein trans-splicing. Figure 27C shows that a mutated variant of the Int[C] fragment, in which the two cysteines at position 96 and 150 were mutated to alanine, was also fully active, albeit with a lower reaction rate of the protein trans-splicing reaction.

**REFERENCES**

1. Kane PM, et al. (1990) Protein splicing converts the yeast TFP1 gene product to the 69-kD subunit of the vacuolar H(+)-adenosine triphosphatase. Science (New York, N.Y 250(4981):651-657.

2. Hirata R, et al. (1990) Molecular structure of a gene, VMA1, encoding the catalytic subunit of H(+)-translocating adenosine triphosphatase from vacuolar membranes of Saccharomyces cerevisiae. The Journal of biological chemistry 265(12):6726-6733.

3. Noren CJ, Wang J, & Perler FB (2000) Dissecting the Chemistry of Protein Splicing and Its Applications. Angewandte Chemie (International ed 39(3):450-466.

4. Novikova O, Topilina N, & Belfort M (2014) Enigmatic distribution, evolution, and function of inteins. The Journal of biological chemistry 289(21):14490-14497.

5. Volkmann G & Mootz HD (2013) Recent progress in intein research: from mechanism to directed evolution and applications. Cellular and molecular life sciences : CMLS 70(7):1185-1206.

6. Shah NH & Muir TW (2014) Inteins: nature's gift to protein chemists. Chem Sci 5:446-461.

7. Wood DW & Camarero JA (2014) Intein applications: from protein purification and labeling to metabolic control methods. The Journal of biological chemistry 289(21):14512-14519.

8. Topilina NI & Mills KV (2014) Recent advances in in vivo applications of intein-mediated protein splicing. Mobile DNA 5(1):5.

9. Wu H, Hu Z, & Liu XQ (1998) Protein trans-splicing by a split intein encoded in a split DnaE gene of Synechocystis sp. PCC6803. Proceedings of the National Academy of Sciences of the United States of America 95(16):9226-9231.

10. Thiel IV, Volkmann G, Pietrokovski S, & Mootz HD (2014) An atypical naturally split intein engineered for highly efficient protein labeling. Angewandte Chemie (International ed 53(5):1306-1310.

11. Mootz HD (2009) Split inteins as versatile tools for protein semisynthesis. Chembiochem 10(16):2579-2589.

12. Aranko AS, Wlodawer A, & Iwai H (2014) Nature's recipe for splitting inteins. Protein engineering, design & selection : PEDS 27(8):263-271.

13. Callahan BP, Topilina NI, Stanger MJ, Van Roey P, & Belfort M (2011) Structure of catalytically competent intein caught in a redox trap with functional and evolutionary implications. Nature structural & molecular biology 18(5):630-633.

14. Nicastri MC, et al. (2013) Internal disulfide bond acts as a switch for intein activity. Biochemistry 52(34):5920-5927.

15. Mohlmann S, Bringmann P, Greven S, & Harrenga A (2011) Site-specific modification of ED-B-targeting antibody using intein-fusion technology. BMC biotechnology 11:76.

16. Bachmann AL & Mootz HD (2017) N-terminal chemical protein labeling using the naturally split GOS-TerL intein. Journal of peptide science : an official publication of the European Peptide Society 23(7-8):624-630.

17. Stevens AJ, et al. (2016) Design of a Split Intein with Exceptional Protein Splicing Activity. Journal of the American Chemical Society 138(7):2162-2165.

18. Moll JM, et al. (2017) Split(2) Protein-Ligation Generates Active IL-6-Type Hyper-Cytokines from Inactive Precursors. ACS Synth Biol 6(12):2260-2272.

19. Dhar T & Mootz HD (2011) Modification of transmembrane and GPI-anchored proteins on living cells by efficient protein trans-splicing using the Npu DnaE intein. Chemical communications (Cambridge, England) 47(11):3063-3065.

20. Palei S, Becher KS, Nienberg C, Jose J, & Mootz HD (2019) Bacterial Cell-Surface Display of Semisynthetic Cyclic Peptides. Chembiochem 20(1):72-77.

21. Schütz V & Mootz HD (2014) Click-tag and amine-tag: chemical tag approaches for efficient protein labeling in vitro and on live cells using the naturally split Npu DnaE intein. Angewandte Chemie (International ed 53(16):4113-4117.

22. Lee E, Min K, Chang YT, & Kwon Y (2018) Efficient and wash-free labeling of membrane proteins using engineered Npu DnaE split-inteins. Protein science : a publication of the Protein Society 27(9):1568-1574.

23. Iwai H, Zuger S, Jin J, & Tam PH (2006) Highly efficient protein trans-splicing by a naturally split DnaE intein from Nostoc punctiforme. FEBS letters 580(7):1853-1858.

24. Zettler J, Schütz V, & Mootz HD (2009) The naturally split Npu DnaE intein exhibits an extraordinarily high rate in the protein trans-splicing reaction. FEBS letters 583(5):909-914.

25. Dassa B, Amitai G, Caspi J, Schueler-Furman O, & Pietrokovski S (2007) Trans protein splicing of cyanobacterial split inteins in endogenous and exogenous combinations. Biochemistry 46(1):322-330.

26. Carvajal-Vallejos P, Pallisse R, Mootz HD, & Schmidt SR (2012) Unprecedented rates and efficiencies revealed for new natural split inteins from metagenomic sources. The Journal of biological chemistry 287(34):28686-28696.

27. Tori K, et al. (2010) Splicing of the mycobacteriophage Bethlehem DnaB intein: identification of a new mechanistic class of inteins that contain an obligate block F nucleophile. The Journal of biological chemistry 285(4):2515-2526.

28. Qi X, Wang J, Meng Q, & Liu XQ (2011) Alternative Nucleophilic Residues in Intein Catalysis of Protein Splicing. Protein and peptide letters 18(12):1226-1232.

29. Choi JJ, et al. (2006) Protein trans-splicing and characterization of a split family B-type DNA polymerase from the hyperthermophilic archaeal parasite Nanoarchaeum equitans. Journal of molecular biology 356(5):1093-1106.

30. Southworth MW, et al. (1998) Control of protein splicing by intein fragment reassembly. The EMBO journal 17(4):918-926.

31. Brenzel S, Cebi M, Reiss P, Koert U, & Mootz HD (2009) Expanding the scope of protein trans-splicing to fragment ligation of an integral membrane protein: towards modulation of porin-based ion channels by chemical modification. Chembiochem 10(6):983-986.

32. Dawson PE, Muir TW, Clark-Lewis I, & Kent SB (1994) Synthesis of proteins by native chemical ligation. Science (New York, N. Y 266(5186):776-779.

33. Tamura T & Hamachi I (2019) Chemistry for Covalent Modification of Endogenous/Native Proteins: From Test Tubes to Complex Biological Systems. Journal of the American Chemical Society 141(7):2782-2799.

34. Krall N, da Cruz FP, Boutureira O, & Bernardes GJ (2016) Site-selective protein-modification chemistry for basic biology and drug development. Nature chemistry 8(2):103-113.

35. Gunnoo SB & Madder A (2016) Chemical Protein Modification through Cysteine. Chembiochem 17(7):529-553.

36. Massa S, et al. (2014) Site-specific labeling of cysteine-tagged camelid single-domain antibody-fragments for use in molecular imaging. Bioconjugate chemistry 25(5):979-988.

37. Junutula JR, et al. (2008) Site-specific conjugation of a cytotoxic drug to an antibody improves the therapeutic index. Nature biotechnology 26(8):925-932.

38. Vazquez-Rey M & Lang DA (2011) Aggregates in monoclonal antibody manufacturing processes. Biotechnology and bioengineering 108(7):1494-1508.

39. Li W, et al. (2016) Antibody Aggregation: Insights from Sequence and Structure. antibodies 5:19.

40. Kurpiers T & Mootz HD (2007) Regioselective cysteine bioconjugation by appending a labeled cystein tag to a protein by using protein splicing in trans. Angewandte Chemie (International ed 46(27):5234-5237.

41. Kurpiers T & Mootz HD (2008) Site-specific chemical modification of proteins with a prelabelled cysteine tag using the artificially split Mxe GyrA intein. Chembiochem 9(14):2317-2325.

42. Dai X, Xun Q, Liu XQ, & Meng Q (2015) Cysteine-free non-canonical C-intein for versatile protein C-terminal labeling through trans-splicing. Applied microbiology and biotechnology 99(19):8151-8161.

43. Devoto AE, et al. (2019) Megaphages infect Prevotella and variants are widespread in gut microbiomes. Nat Microbiol 4(4):693-700.

44. Caspi J, Amitai G, Belenkiy O, & Pietrokovski S (2003) Distribution of split DnaE inteins in cyanobacteria. Molecular microbiology 50(5):1569-1577.

45. Dalgaard JZ, Moser MJ, Hughey R, & Mian IS (1997) Statistical modeling, phylogenetic analysis and structure prediction of a protein splicing domain common to inteins and hedgehog proteins. J Comput Biol 4(2):193-214.

46. Pietrokovski S (1998) Modular organization of inteins and C-terminal autocatalytic domains. Protein science : a publication of the Protein Society 7(1):64-71.

47. Hu Y, Liu C, & Muyldermans S (2017) Nanobody-Based Delivery Systems for Diagnosis and Targeted Tumor Therapy. Front Immunol 8:1442.

48. Schumacher D, Helma J, Schneider AFL, Leonhardt H, & Hackenberger CPR (2018) Nanobodies: Chemical Functionalization Strategies and Intracellular Applications. Angewandte Chemie (International ed 57(9):2314-2333.

49. Kirchhofer A, et al. (2010) Modulation of protein properties in living cells using nanobodies. Nature structural & molecular biology 17(1): 133-138.

50. Klein A, et al. (2018) Nanobody-Displaying Flagellar Nanotubes. Scientific reports 8(1):3584.

51. Schmitz KR, Bagchi A, Roovers RC, van Bergen en Henegouwen PM, & Ferguson KM (2013) Structural evaluation of EGFR inhibition mechanisms for nanobodies/VHH domains. Structure 21(7):1214-1224.

52. Ibach J, et al. (2015) Single Particle Tracking Reveals that EGFR Signaling Activity Is Amplified in Clathrin-Coated Pits. PloS one 10(11):e0143162.

53. Richter D, et al. (2017) Ligand-induced type II interleukin-4 receptor dimers are sustained by rapid re-association within plasma membrane microcompartments. Nature communications 8:15976.

54. Los GV, et al. (2008) HaloTag: a novel protein labeling technology for cell imaging and protein analysis. ACS chemical biology 3(6):373-382.

55. Keppler A, et al. (2003) A general method for the covalent labeling of fusion proteins with small molecules in vivo. Nature biotechnology 21(1):86-89.

56. Dhar T, Kurpiers T, & Mootz HD (2011) Extending the scope of site-specific cysteine bioconjugation by appending a prelabeled cysteine tag to proteins using protein trans-splicing. Methods in molecular biology (Clifton, N.J 751:131-142.

57. Ando T, Tsukiji S, Tanaka T, & Nagamune T (2007) Construction of a small-molecule-integrated semisynthetic split intein for in vivo protein ligation. Chemical communications (Cambridge, England) (47):4995-4997.

58. Volkmann G & Liu XQ (2009) Protein C-terminal labeling and biotinylation using synthetic peptide and split-intein. PloS one 4(12):e8381.

59. Mootz HD & Muir TW (2002) Protein splicing triggered by a small molecule. Journal of the American Chemical Society 124(31):9044-9045.

60. Thomas C, et al. (2011) Structural linkage between ligand discrimination and receptor activation by type I interferons. Cell 146(4):621-632.

61. Kumar KG, et al. (2008) Basal ubiquitin-independent internalization of interferon alpha receptor is prevented by Tyk2-mediated masking of a linear endocytic motif. The Journal of biological chemistry 283(27):18566-18572.

62. Bachmann AL & Mootz HD (2015) An Unprecedented Combination of Serine and Cysteine Nucleophiles in a Split Intein with an Atypical Split Site. The Journal of biological chemistry 290(48):28792-28804.

63. Aranko AS, et al. (2014) Structure-based engineering and comparison of novel split inteins for protein ligation. Molecular bioSystems 10(5):1023-1034.

64. Aranko AS, Zuger S, Buchinger E, & Iwai H (2009) In vivo and in vitro protein ligation by naturally occurring and engineered split DnaE inteins. PloS one 4(4):e5185.

65. Appleby-Tagoe JH, et al. (2011) Highly efficient and more general cis- and trans-splicing inteins through sequential directed evolution. The Journal of biological chemistry 286(39):34440-34447.

66. Stevens AJ, Sekar G, Gramespacher JA, Cowburn D, & Muir TW (2018) An Atypical Mechanism of Split Intein Molecular Recognition and Folding. Journal of the American Chemical Society 140(37):11791-11799.

67. Brenzel S & Mootz HD (2005) Design of an intein that can be inhibited with a small molecule ligand. Journal of the American Chemical Society 127(12):4176-4177.

68. Braner M, Kollmannsperger A, Wieneke R, & Tampe R (2016) 'Traceless' tracing of proteins - high-affinity trans-splicing directed by a minimal interaction pair. Chem Sci 7(4):2646-2652.

69. Pleiner T, et al. (2015) Nanobodies: site-specific labeling for super-resolution imaging, rapid epitope-mapping and native protein complex isolation. eLife 4:e11349.

70. Albrecht D, Winterflood CM, & Ewers H (2015) Dual color single particle tracking via nanobodies. Methods Appl Fluoresc 3(2):024001.

71. Liu CC & Schultz PG (2010) Adding new chemistries to the genetic code. Annu Rev Biochem 79:413-444.

72. Chin JW (2017) Expanding and reprogramming the genetic code. Nature 550_53-60.

**Claims**

1. An isolated polypeptide comprising

   (a) at least one first intein amino acid sequence (a1) and at least one first extein amino acid sequence (a2); wherein the at least one first intein amino acid sequence is an N-terminal sequence of a split intein or fragment thereof; wherein the at least one first extein sequence is fused to the N-terminus of the at least one first intein sequence and has a length of at least 1, preferably at least 5 amino acids; and
   (b) at least one second intein amino acid sequence (b1) and at least one second extein sequence (b2); wherein the at least one second intein sequence is a C-terminal sequence of a split intein or fragment thereof; wherein the at least one second extein sequence is fused to the C-terminus of the at least one second intein sequence and has a length of at least 1, preferably at least 5 amino acids;
   wherein the catalytic motifs of the at least one first intein amino acid sequence and/or of the at least one second intein amino acid sequence are free of cysteine residues and wherein the first N-terminal amino acid of the second extein sequence is not a cysteine residue;
   wherein the first and second intein amino acid sequence interact with each other to catalyze the cleavage of the peptide bonds between the C-terminal amino acid of the first extein sequence and the N-terminal amino acid of the first intein sequence and the N-terminal amino acid of the second extein sequence and the C-terminal amino acid of the second intein sequence and to ligate the C-terminal amino acid of the first extein sequence to the N-terminal amino acid of the second extein sequence by a peptide bond.

2. A composition comprising

(a) a first isolated polypeptide comprising at least one first intein amino acid sequence (a1) and at least one first extein amino acid sequence (a2); wherein the at least one first intein amino acid sequence is an N-terminal sequence of a split intein or fragment thereof; wherein the at least one first extein sequence is fused to the N-terminus of the at least one first intein sequence and has a length of at least 1, preferably at least 5 amino acids; and

(b) a second isolated polypeptide comprising at least one second intein amino acid sequence (b1), wherein the at least one second intein sequence is a C-terminal sequence of a split intein or fragment thereof and at least one second extein sequence (b2), wherein the at least one second extein sequence is fused to the C-terminus of the at least one second intein sequence and has a length of at least 1, preferably at least 5 amino acids;

wherein the catalytic motifs of the at least one first intein amino acid sequence and/or of the at least one second intein amino acid sequence are free of cysteine residues and wherein the first N-terminal amino acid of the second extein sequence (+1 position) is not a cysteine residue;

wherein the first and second intein amino acid sequence interact with each other to catalyze the cleavage of the peptide bonds between the C-terminal amino acid of the first extein sequence and the N-terminal amino acid of the first intein sequence and the N-terminal amino acid of the second extein sequence and the C-terminal amino acid of the second intein sequence and to ligate the C-terminal amino acid of the first extein sequence to the N-terminal amino acid of the second extein sequence by a peptide bond.

3. The isolated polypeptide of claim 1 or the composition of claim 2, wherein

(i) the catalytic motif(s) of the at least one first intein amino acid sequence comprise(s) the amino acid sequence(A)

$X_1X_2X_3X_4X_5X_6X_7X_8X_9X_{10}X_{11}$ (A)

wherein

$X_1$ is S or T, most preferably S;

$X_2$ is V, C, F, Q, I, T, S, L, G or A, preferably V, C, F, Q, I or T, more preferably V, F, Q, I or T, most preferably V;

$X_3$ is V, T, L, S, I, N, D, A, Q, R, H, F, E, Y, C, K or P, preferably V, D, S, T, L or A, more preferably V, D or S, most preferably V or D;

$X_4$ is any naturally occuring amino acid or not present, preferably G, A, W, E, S, T, K, N, F, H, Y, R, C, Q, M or not present, more preferably G, A, W, S, F, M or K, more preferably G, A, S, F or K, most preferably G;

$X_5$ is D, S, K, N, L, E, C, T, A, Q or I, preferably D, S, K, N, Q or L, most preferably D or N;

$X_6$ is T, S, V, A, C, M or E, preferably T, S, E or C, more preferably T or S, most preferably T;

$X_7$ is I, Q, S, K, E, T, L, V, H, C, F, A, M, N, Y or P, preferably I, L, Q, K or P, more preferably I, L, Q or K, most preferably I or L;

$X_8$ is any naturally occuring amino acid or not present, preferably I, V, F, L, N, M or not present, more preferably I, V, F, L or M, more preferably I, V, F or L, most preferably I or L;

$X_9$ is D, N, V, R, F, Y, Q, E, K, C, S, I, M, H, W or L, preferably D, Y, K, R, N, L or V, most preferably D or Y;

$X_{10}$ is any naturally occuring amino acid or not present, preferably V, I, L, Y, T, A, C, W or not present, more preferably V, I, L, T, C or W, more preferably V, I, L or T, more preferably V, I or T, most preferably V or I; and

$X_{11}$ is S, N, D, R, K, E, T, M, G, I or H, preferably S, N, D, R, K, E or H, preferably S, N, D, R or K, more preferably S, N or D, most preferably S or N;

wherein the amino acid sequence (A) comprises or consists of at least 8 amino acids ($X_1$-$X_3$, $X_5$-$X_7$, $X_9$, $X_{11}$), preferably at least 9 amino acids (X1-X3, $X_5$-$X_7$, $X_9$-$X_{11}$) or 10 amino acids (X1-X3, $X_5$-$X_{11}$ or $X_1$-$X_9$, $X_{11}$), most preferably it comprises or consists of 11 amino acids ($X_1$-$X_{11}$); or

(ii) the catalytic motif(s) of the at least one first intein amino acid sequence comprise(s) the amino acid sequence (A')

$X_1X_2X_3X_4X_5X_6X_7X_8X_9X_{10}X_{11}$ (A')

wherein

$X_1$ is S;

$X_2$ is V, F, I or L, preferably V, F or I, most preferably V;

$X_3$ is G, A, T, M, L, E, S, V, D, N, I or K, preferably A, T, M, L or V, most preferably T;

$X_4$ is K, G, A, P, S, E, Y, H, D or N, preferably K, G, A, P, Y or H, most preferably G;

$X_5$ is D, S, Y, E, G, N, A, T or C, preferably D, S, Y, E or C, more preferably D, S, Y or E, most preferably D;

$X_6$ is T, S, E or R, preferably T, S or R, more preferably T or S, most preferably T;

$X_7$ is E, I, V, P, Q, L, A, R, D, C, F, W or M, preferably I, V, P, Q, L, C, W or M, more preferably I, V, P, Q, L, W or M, more preferably I, V, P or L, most preferably P;

Xs is I, V, L or M, preferably I, V or L, most preferably I;

$X_9$ is V, A, S, I, M, L, F, Y, T or R, preferably V, I, M, L, F, Y or T, more preferably V, I, M, L or Y, most preferably V, I or L;

$X_{10}$ is M, V, T, I, L, A or Y, preferably M, V, T, I or L, more preferably V, I or L, most preferably V; and $X_{11}$ is any naturally occuring amino acid, preferably N, R, K, Y, S, G, D, E, L or not present, preferably R, K, Y, D or L, more preferably R or K, most preferably R;

wherein the amino acid sequence (A') comprises or consists of at least 10 amino acids ($X_1$-$X_{10}$), preferably it comprises or consists of 11 amino acids ($X_1$-$X_{11}$);

wherein the amino acid sequence (A) or (A') preferably forms the N-terminus of the first intein amino acid sequence.

4. The isolated polypeptide or the composition of any one of claims 1 to 3, wherein

(i) the catalytic motif(s) of the at least one second intein amino acid sequence comprise(s) the amino acid sequence (B1)

$X_{45}X_{46}X_{47}X_{48}X_{49}X_{50}X_{51}X_{52}X_{53}X_{54}X_{55}X_{56}X_{57}$ (B1)

wherein

$X_{45}$ is N, C, Y, T, D, P, S, H, A, G, M or R, preferably N, C, T, S, or H, more preferably N, T or S, most preferably T or N;

$X_{46}$ is H, P, S or F, preferably H, F or P, most preferably H;

$X_{47}$ is N, T, C, Y, M, V, A, S or H, preferably N, T, Y, M, A or H, most preferably N or T;

$X_{48}$ is F or A, preferably F;

$X_{49}$ is F, V, Y, I or G, preferably F, V, Y or I, most preferably F;

$X_{50}$ is G, A, C or I, preferably G or A;

$X_{51}$ is N, S, D or G, preferably N or G, most preferably N;

$X_{52}$ is D, G, N, E, T, S or R, preferably D, N, G or R, more preferably D, N or G, more preferably D or N, most preferably D;

$X_{53}$ is I, V, M or T, preferably I, M or V, more preferably I or V, most preferably I;

$X_{54}$ is L, C, Y, I or V, preferably L;

$X_{55}$ is V, A, I or L, preferably V;

$X_{56}$ is H; and

$X_{57}$ is N;

wherein the amino acid sequence (B1) comprises or consists of at least 13 amino acids ($X_{45}$-$X_{57}$); or

(ii) the catalytic motif(s) of the at least one second intein amino acid sequence comprise(s) the amino acid sequence (B1')

$X_{43}X_{44}X_{45}X_{46}X_{47}X_{48}X_{49}X_{50}X_{51}X_{52}X_{53}X_{54}$ (B1')

wherein

$X_{43}$ is any naturally occuring amino acid or not present, preferably R, C, F, V, L, P, H, N or not present, more preferably C, F, V or H, more preferably F, V or H, most preferably F;

$X_{44}$ is any naturally occuring amino acid or not present, preferably F, H, Q, V, I, S, A, N, Y, C, T, W or not present, more preferably F, H, V, A, N, C, T or W, more preferably F, H, V, N or A, most preferably F, H, A or V;

$X_{45}$ is F, Y, A, V, N, G, S, I, C or D, preferably F, A, N, C or D, most preferably F, A, N or D;

$X_{46}$ is any naturally occuring amino acid or not present, preferably G, A, R, F, M or not present, preferably G, A or F, most preferably G;

$X_{47}$ is A, V, I, P, L, S, F, G, K or C, preferably A, V, I, P, L or C, more preferably A, V, I, L or C, most preferably A, V, I or L;

$X_{48}$ is any naturally occuring amino acid or not present, preferably N, G, D or not present, most preferably N or G;

$X_{49}$ is N, D, R, G, E, L, M, S, P or Q, preferably N, D, G, E or M, most preferably N, G, E or M;

$X_{50}$ is V, I, M, L or N, preferably I, M or L, most preferably I or L;

$X_{51}$ is L, I, V, D or C, preferably L, I, V or C, most preferably L, I or V;

$X_{52}$ is V, I, N, L, C or A, preferably V, L or C, most preferably V or L;

$X_{53}$ is H, K, S or T, preferably H or K, most preferably H; and

$X_{54}$ is N or Q, preferably N;

wherein the amino acid sequence (B1') comprises or consists of at least 8 amino acids (X45, X49-X54), preferably at least 10 amino acids (X45-X54) or at least 11 amino acids ($X_{43}$-$X_{45}$, $X_{47}$-$X_{54}$, or X43-X47, X49-X54), most preferably it comprises or consists of 12 amino acids (X43-X54);

wherein the amino acid sequence (B1) or (B1') preferably forms the C-terminus of the second intein amino acid sequence.

5. The isolated polypeptide or the composition of any one of claims 1 to 4, wherein

(i) the second extein sequence comprises an N-terminal serine or threonine residue that is fused to the C-terminal amino acid of the second intein sequence by a peptide bond; and/or
(ii) the catalytic motif(s) of the at least one second intein amino acid sequence comprise(s) the amino acid sequence (B2)

$X_{28}X_{29}X_{30}X_{31}X_{32}X_{33}X_{34}X_{35}X_{36}X_{37}$ (B2)

wherein

$X_{28}$ is I, L, E, V, Q, D or N, preferably I, L, E, Q, D or N, more preferably I, E or D, most preferably I or E;

$X_{29}$ is D, Y, W, F, P, I or M, preferably D, Y or W, most preferably D or Y;

$X_{30}$ is V or A, preferably V;

$X_{31}$ is Y or F, preferably Y;

X32 is D;

$X_{33}$ is I, V, L, C, A or M, preferably I, V, L or C, more preferably I, V or L, most preferably I;

$X_{34}$ is E, S, G or V, preferably E;

$X_{35}$ is any naturally occuring amino acid or not present, preferably V, M, I, T or not present, more preferably V or M, most preferably V;

$X_{36}$ is any naturally occuring amino acid or not present, preferably D, E, Q, P, K, F, A, N, G or not present, more preferably D or E, most preferably D; and

$X_{37}$ is any naturally occuring amino acid or not present, preferably G, E, D, K, T, N, S or not present, more preferably G, E, D or N, more preferably G, E or D, most preferably G or D;

wherein the amino acid sequence (B2) comprises or consists of at least 7 amino acids ($X_{28}$-$X_{34}$), preferably at least 8 amino acids ($X_{28}$-$X_{34}$, $X_{37}$ or $X_{28}$-$X_{35}$) or at least 9 amino acids ($X_{28}$-$X_{35}$, $X_{37}$ or $X_{28}$-X36), most preferably at least 10 amino acids ($X_{28}$-$X_{37}$); or

the catalytic motif(s) of the at least one second intein amino acid sequence comprise(s) the amino acid sequence (B2')

$X_{27}X_{28}X_{29}X_{30}X_{31}X_{32}X_{33}X_{34}X_{35}X_{36}X_{37}$ (B2')

wherein

$X_{27}$ is D, E, Y, F, V, T, M or W, preferably Y, F, M or W, most preferably Y;

$X_{28}$ is V;

X29 is Y;

$X_{30}$ is D;

$X_{31}$ is L, I, M or V, preferably L or I, most preferably L;

$X_{32}$ is E, T, S, Q, C or G, preferably E, T, S or C, more preferably E, T or S, most preferably E or S;

$X_{33}$ is I, L, M, V, T, C or A, preferably I, L, M, V, T or C, more preferably I, L, M, V or T, most preferably V or T;

$X_{34}$ is any naturally occuring amino acid or not present, preferably E, P, D, G, S, A, Q, T, V, K, N, F, G, R or not present, more preferably E, D, A or K, most preferably E;

$X_{35}$ is any naturally occuring amino acid or not present, preferably V, D, S, Q, N, R, A, C, H, G, E, T or not present, more preferably D, S, N, C or E, more preferably D, S, N or E, most preferably D or N,

$X_{36}$ is any naturally occuring amino acid or not present, preferably D, G, H, Q, N, E, T, S, A or not present, more preferably D, G, H, Q or N, more preferably G, H or N, most preferably G or H,

$X_{37}$ is any naturally occuring amino acid or not present, preferably T, D, S, H, V, E, R, Q, K, N, I or not present, more preferably T, D, H or E, most preferably T or H;

wherein the amino acid sequence (B2') comprises or consists of at least 7 amino acids ($X_{27}$-$X_{33}$),

preferably at least 9 amino acids ($X_{27}$-$X_{35}$) or at least 10 amino acids ($X_{27}$-$X_{33}$, $X_{35}$-$X_{37}$ or $X_{27}$-$X_{35}$, $X_{37}$ or X27-X34, X36-X37), most preferably at least 11 amino acids ($X_{27}$-$X_{37}$),

wherein amino acid sequence (B2) or (B2') is preferably located N-terminally to amino acid sequence (B1) or (B1') in the second intein amino acid sequence, preferably amino acids $X_{37}$ of (B2) and $X_{45}$ of (B1) or amino acids $X_{37}$ of (B2') and $X_{43}$ of (B1') are linked to each other by a peptide bond; and/or

(iii) the catalytic motif(s) of the first or the second intein amino acid sequence comprise(s) the amino acid sequence (C)

$X_{12}X_{13}X_{14}X_{15}X_{16}X_{17}X_{18}X_{19}X_{20}X_{21}X_{22}X_{23}$ (C)

wherein

$X_{12}$ is any naturally occuring amino acid or not present, preferably E, K, T, M, S, P, F, D, V, C, Q, A, Y or not present, more preferably E, C, S, V or P, more preferably E, S, V or P, most preferably E;

$X_{13}$ is V, L, T or I, most preferably V or I;

$X_{14}$ is I, T, E, V, L, F, K, D, S, Y, M or H, preferably I, T, E, V, Y or H, more preferably I, T, E or V, most preferably I;

$X_{15}$ is V, M, I, L, T, C or A, preferably V, T, C or I, more preferably V or I, most preferably V;

$X_{16}$ is T or S, preferably T;

$X_{17}$ is A, E, C, G, N, Q, D, T or S, preferably A, E, C, G, N or T, more preferably A, E, G, N or T, most preferably A, E or G;

$X_{18}$ is D, E or N, preferably D;

X19 is H;

$X_{20}$ is S, C, G, I, V or N, preferably S, C, V or N, more preferably S or V, most preferably S;

$X_{21}$ is V, I, L, M, C, or F, preferably V, I, L, M or C, more preferably V, I, C or L, more preferably V, I or L, most preferably V;

$X_{22}$ is any naturally occuring amino acid or not present, preferably M, I, K, V, L or not present, more preferably M, I or V, most preferably M; and

$X_{23}$ is any naturally occuring amino acid or not present, preferably V, I, K, R, A, D or not present, more preferably, V, I, D, R or A, more preferably V, I or A, most preferably V,

wherein the amino acid sequence (C) comprises or consists of at least 9 amino acids ($X_{13}$-$X_{21}$), preferably at least 10 amino acids ($X_{12}$-$X_{21}$) or at least 11 amino acids ($X_{13}$-$X_{23}$), most preferably 12 amino acids ($X_{12}$-$X_{23}$); or

(ii) the catalytic motif(s) of the first or the second intein amino acid sequence comprise(s) the amino acid sequence (C')

$X_{12}X_{13}X_{14}X_{15}X_{16}X_{17}X_{18}X_{19}X_{20}X_{21}X_{22}X_{23}$ (C')

wherein

$X_{12}$ is Y, H, C, V, I, L, M, S, T, K, E, D, Q, W, R or A, preferably Y, C, I, L, K or E, more preferably Y, C or E, more preferably Y or E, most preferably E;

$X_{13}$ is I, V, C, T or S, preferably I, V, C or S, more preferably I, V or S, most preferably V;

$X_{14}$ is D, T, M, E, C, K, H, N, V, S, I or R, preferably D, T, E, C, H, V or I, more preferably D, T or H, more preferably D or T, most preferably D;

$X_{15}$ is V, C, A, I or T, preferably V, C or T, most preferably V;

X16 is T;

$X_{17}$ is E, R, A, S, D, K, N, H, C, P or T, preferably E, R, D, H or C, more preferably E, R, D or H, more preferably E or R, most preferably E;

$X_{18}$ is D, N, Q or E, preferably D;

$X_{19}$ is H;

$X_{20}$ is S or R, preferably S;

$X_{21}$ is L, V, A, I or Y, preferably L, I or Y, most preferably L;

$X_{22}$ is I, L, F, V, M or Y, preferably I, L, F, V or M, most preferably I, L, F or V; and

$X_{23}$ is G, D, N, L, Q, K, R, Y, V, I, A, T, S or K, preferably G, D, N, V, I or T, more preferably D or V;

wherein the amino acid sequence (C') comprises or consists of 12 amino acids ($X_{12}$-$X_{23}$);

wherein, preferably, the amino acid sequence (C) or (C'), if present in the first intein amino acid sequence, is located C-terminally to amino acid sequence (A) or (A'), or, if present in the second intein amino acid sequence, is located N-terminally to amino acid sequence (B2) or (B2').

6. The isolated polypeptide or the composition of any one of claims 1 to 5, wherein

(i) the amino acid sequence (A) or (A'), the amino acid sequence (B1) or (B1'), the amino acid sequence (B2) or (B2') and/or the amino acid sequence (C) or (C'), preferably at least two, more preferably at least three, most preferably all four amino acid sequences are free of cysteine residues; and/or
(ii) the at least one first intein amino acid sequence is free of cysteine residues, and/or
(iii) the at least one second intein amino acid sequence is free of cysteine residues; and/or
(iv) the first intein amino acid sequence and the second intein amino acid sequence are not derived from a thermophilic organism.

7. The isolated polypeptide or the composition of any one of claims 1 to 6, wherein the first and second intein amino acid sequence interact with each other to catalyze the cleavage of the peptide bonds between the C-terminal amino acid of the first extein sequence and the N-terminal amino acid of the first intein sequence and the N-terminal amino acid of the second extein sequence and the C-terminal amino acid of the second intein sequence and to ligate the C-terminal amino acid of the first extein sequence to the N-terminal amino acid of the second extein sequence by a peptide bond

(a) at a temperature of ≤ 40 °C, preferably less than 40 °C, more preferably 0 to 37 °C, more preferably 8 °C or 37 °C; and/or
(b) under oxidizing reaction conditions, preferably under atmospheric oxygen.

8. The isolated polypeptide or the composition of any one of claims 1 to 7, wherein

(i) said at least one first intein amino acid sequence has at least 70 %, at least 80 %, at least 85 %, at least 90 %, at least 95 %, at least 99 % or 100 % sequence identity with any one of the amino acid sequences set forth in SEQ ID Nos. 1, 3, 5, 7, 9, 11, 13, 15, 17, 19, 21, 23, 25, 27, 29, 31, 33, 35, 37, 39, 41, 43, 45, 48, 51, 53, 55, 58, 61, 63, 65, 67, 70, 74, 76, 78, 80, 82, 85, 87, 89, 92, 97, 100, 102, 106, 108, 111, 114, 117, 120, 122, 124, 126, 128, 130, 132, 134, 136, 138, 140, 142, 144, 146 and 147, preferably SEQ ID Nos. 1, 3, 5, 7, 9, 11, 13, 15, 17, 19, 21, 23, 25, 27, 29, 31, 33, 35, 37, 39, 41, 43 and 45, more preferably SEQ ID NO:1 or 3, more preferably SEQ ID NO:3;
and/or
(ii) said at least one second intein amino acid sequence has at least 70 %, at least 80 %, at least 85 %, at least 90 %, at least 95 %, at least 99 % or 100 % sequence identity with any one of the amino acid sequences set forth in SEQ ID Nos. 2, 4, 6, 8, 10, 12, 14, 16, 18, 20, 22, 24, 26, 28, 30, 32, 34, 36, 38, 40, 42, 44, 46, 49, 52, 54, 56, 59, 62, 64, 66, 68, 71, 75, 77, 79, 81, 83, 86, 88, 90, 93, 98, 101, 103, 107, 109, 112, 115, 118, 121, 123, 125, 127, 129, 131, 133, 135, 137, 139, 141, 143, 145, 148, 149, 150, 151 and 152, preferably SEQ ID Nos. 2, 4, 6, 8, 10, 12, 14, 16, 18, 20, 22, 24, 26, 28, 30, 32, 34, 36, 38, 40, 42, 44 and 46, more preferably SEQ ID NO:2 or 4, more preferably SEQ ID NO:4; or
(iii) at least one intein amino acid sequence comprising the first intein amino acid sequence and the second intein amino acid sequence in one contiguous amino acid sequence; wherein the C-terminus of the first intein sequence is ligated to the N-terminus of the second intein sequence, and wherein the contiguous amino acid sequence has at least 70 %, at least 80 %, at least 85 %, at least 90 %, at least 95 %, at least 99 % or 100 % sequence identity with any one of the amino acid sequences set forth in SEQ ID Nos. 47, 50, 57, 60, 69, 72, 73, 84, 91, 94, 95, 96, 99, 104, 105, 110, 113, 116, 119, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182,183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 and 250; wherein, preferably,

(a) said at least one first intein amino acid sequence has at least 70 %, at least 80 %, at least 85 %, at least 90 %, at least 95 %, at least 99 % or 100 % sequence identity with the amino acid sequence set forth in SEQ ID NO:1 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:2; or
(b) said at least one first intein amino acid sequence has at least 70 %, at least 80 %, at least 85 %, at least 90 %, at least 95 %, at least 99 % or 100 % sequence identity with the amino acid sequence set forth in SEQ ID NO:3 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:4;

(c) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:5 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:6; or

(d) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:7 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:8; or

(e) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:9 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:10; or

(f) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:11 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:12; or

(g) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:13 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:14; or

(h) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:15 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:16; or

(i) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:17 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:18; or

(j) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:19 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:20; or

(k) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:21 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:22; or

(l) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:23 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:24; or

(m) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:25 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:26; or

(n) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:27 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:28; or

(o) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:29 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:30; or

(p) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:31 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:32; or

(q) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:33 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:34; or

(r) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:35 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:36; or

(s) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:37 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:38; or

(t) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:39 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:40; or

(u) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:41 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:42; or

(v) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:43 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:44; or

(w) wherein said at least one first intein amino acid sequence has at least 70 %, at least 80 %, at least 85 %, at least 90 %, at least 95 %, at least 99 % or 100 % sequence identity with the amino acid sequence set forth in SEQ ID NO:45 and said at least one second intein amino acid sequence has at least 70 %, at least 80 %, at least 85 %, at least 90 %, at least 95 %, at least 99 % or 100 % sequence identity with the amino acid sequence set forth in SEQ ID NO:46.

9. The isolated polypeptide or the composition of any one of claims 1 to 8, wherein

(i) the at least one first intein amino acid sequence is at least 10 amino acids, preferably at least 11 amino acids, more preferably at least 12 amino acids, more preferably 26 or 120 amino acids in length; and/or

(ii) the at least one second intein amino acid sequence is at least 6 amino acids, preferably at least 24 amino acids, more preferably at least 30 amino acids, more preferably 39 or 129 amino acids in length; and/or

(iii) the split site between the N-terminal and C-terminal amino acid sequence of the split intein is between residues 10 to 200, more preferably between 11 to 200, more preferably between 12 to 200, more preferably between 25 to 200, more preferably after amino acid 10 or 11 or 12 or 26 or 120, preferably after amino acids 26 or 120 as calculated from the intein's N-terminal end; and/or

(iv) the last amino acid at the C-terminal end of the first extein amino acid sequence is D or S, preferably D, preferably the last two amino acids at the C-terminal end of the first extein amino acid sequence are TD or GD, preferably the last three amino acids at the C-terminal end of the first extein amino acid sequence are DTD,

preferably the last four amino acids at the C-terminal end of the first extein amino acid sequence are IDTD, preferably the last five amino acids at the C-terminal end of the first extein amino acid sequence are YIDTD; and/or wherein the first amino acid at the N-terminal end of the second extein amino acid sequence is S or T, preferably the first two amino acids at the N-terminal end of the second extein amino acid sequence are SV or TD, preferably the first three amino acids at the N-terminal end of the second extein amino acid sequence are SVY or TDG or TDS,
preferably the first four amino acids at the N-terminal end of the second extein amino acid sequence are SVYL, preferably the first five amino acids at the N-terminal end of the second extein amino acid sequence are SVYLN.

**10.** The isolated polypeptide or the composition of any one of claims 1 to 9, wherein

(i) the polypeptide(s), preferably the first and/or second extein amino acid sequence(s) of the polypeptide(s), comprise(s) a recombinant protein, an antibody, a nanobody, a protein hormone, or a fragment or homolog thereof; and/or
(ii) the polypeptide(s), preferably the first and/or second extein amino acid sequence(s) of the polypeptide(s), further comprise(s) a cysteine tag (Cys tag), wherein the cysteine tag comprises a single cysteine residue in its amino acid sequence; and/or
(iii) the polypeptide(s), preferably the first and/or second extein amino acid sequence(s) of the polypeptide(s), further comprise(s) at least one component selected from a solubility factor, a marker, a linker, an epitope, an affinity tag, a fluorophore or a fluorescent protein, a toxic compound or protein and a small-molecule or a small-molecule binding protein; and/or
(iv) the polypeptide(s) comprise(s) or consist(s) of an amino acid sequence having at least 70 %, at least 80 %, at least 85 %, at least 90 %, at least 95 %, at least 99 % or 100 % sequence identity with any one of the sequences set forth in SEQ ID Nos. 251 to 272 and 275 to 279.

**11.** At least one isolated nucleic acid molecule comprising at least one nucleotide sequence encoding an isolated polypeptide according to any one of claims 1 to 10, or a homolog, variant or complement thereof.

**12.** Method for modifying amino acid sequences, preferably ligating two amino acid sequences to each other, using an isolated polypeptide or a composition of any one of claims 1 to 10 or at least one isolated nucleic acid molecule of claim 11, wherein the modification is preferably selected from the group consisting of modification of a protein, protein lipidation, protein immobilization, protein backbone ligation, protein backbone semi-synthesis, protein or peptide cyclization, protein modification with a chemically modified tag sequence, preferably with a cysteine tag, and artificial control of protein splicing by light.

**13.** The method according to claim 12, wherein

(i) the isolated polypeptide or the composition of any one of claims 1 to 10 is exposed to oxidizing conditions, preferably to atmospheric oxygen; and/or
(ii) the isolated polypeptide or the composition of any one of claims 1 to 10 is exposed to a temperature of ≤ 40 °C, preferably less than 40 °C, more preferably 0 to 37 °C, more preferably 8 °C or 37 °C; and/or
(iii) the method is essentially free of or free of a reducing step or a reducing agent; and/or
(iv) the method is free of a denaturation step.

**14.** The method according to claim 12 or 13, wherein the method comprises the step(s) of

(i) protein preparation, preferably recombinant protein expression or *in vitro* translation; and/or
(ii) protein purification; and/or
(iii) dialysis of a prepared/purified protein.

**15.** Use of an isolated polypeptide or a composition of any one of claims 1 to 10 or at least one nucleic acid molecule according to claim 11, wherein the polypeptide or the composition or the nucleic acid molecule is preferably used for modification of a protein, protein lipidation, protein immobilization, protein backbone ligation, protein backbone semi-synthesis, protein or peptide cyclization, protein modification with a chemically modified tag sequence, preferably with a cysteine tag, and as molecular switch.

Figure 1

**A**

Aes$^N$

```
    1                               30
   YIDTDSVVGDTIIDVSGKKMTIAEFYDSTP
   31                              60
   DVFMRRNDEARDWVKRVGGKTSLSVNTYSG
   61                              90
   EVERKNINYIMKHTVKKRMFKIKAGGKEVI
   91                             120
   VTADHSVMVKRDGKIIDVKPTEMKQTDRVV
   121     125
   KWMLT
```

Aes$^C$

```
   1                               30
   MIEFIEFEIEDLGVMEIDVYDIEVDGNHNF
   31          44
   FGNDILVHNSVYLN
```

**B**

MBP Aes$^N$ H$_6$ + SBP Aes$^C$ SBP
  **1**                    **2**

→ PTS → MBP SBP + Aes$^N$ H$_6$ + SBP Aes$^C$
         **SP**        **Aes$^N$**      **Aes$^C$**

**C**

ratio (construct **1:2**)

| | | 1:1 | 3:1 | 1:3 |
| **1** | **2** | 1 360 | 1 360 | 1 360 min |

kDa

85
60 ←**1**
50 ←SP
40
30
25
20 ←**2**
15 ←Aes$^N$
10

**D**

splice yield [%] vs time [min]
■ 8 °C
■ 37 °C

**Figure 2**

**Figure 3**

Figure 4

## A

```
Aes123 PolB1    SVVGDTIIDV--SGKKMTIAEFYDSTPDVFMRRNDEARDWVKRVGGKTSL
Ssp DnaB        CISGDSLISLASTGKRVSIKDLLDEKDFE-------------------IW
                .: **::*.:   :**::* :: *..

Aes123 PolB1    SVNTYSGEVERKNINYIMKHTVKKRMFKIKA-GGKEVIVTADHSVMVKRD
Ssp DnaB        AINEQTMKLESAKVSRVFM-TGKKLVYILKTRLGRTIKATANHRFLTIDG
                ::*   : ::*   ::. ::   * **  :: :*:   *: :  .**:* .:.   .

Aes123 PolB1    GKIID--------------------VKPTEMKQTDRVVKWMLTMIEFIEF
Ssp DnaB        WKRLDELSLKEHIALPRKLESSSLQLSPEIEKLSQSDISWD-SI-----V
                * :*                     :.*   * :: :.* ::       .

Aes123 PolB1    EIEDLGVMEIDVYDIEVDGNHNFFGNDILVHNS
Ssp DnaB        SITETGVEE--VFDLTVPGPHNFVANDIIVHNS
                .* : ** *  *:*: * * ***..***:****
```

## B

Figure 5

**Figure 6**

Figure 7

Figure 8

**A**

**B**

**Figure 9**

**Figure 10**

**Figure 11**

Figure 12

A

B

C

**Figure 13**

**Figure 14**

**Figure 15**

**Figure 16**

Figure 17

Figure 18

**Figure 19**

**Figure 20**

**Figure 21**

| Protein/peptide number | Name of construct | Encoding plasmid | Vector backbone |
|---|---|---|---|
| **1** (SEQ ID NO:251) | MBP-Aes$^N$-H$_6$ | pTT04 | pMAL-c2x |
| **2** (SEQ ID NO:252) | SBP-Aes$^C$-SBP | pTT22 | pET16b |
| **3** (SEQ ID NO:253) | MBP-CL$^N$-H$_6$ | pTT32 | pMAL-c2x |
| **3a** (SEQ ID NO:255) | MBP-CL$^N$(S1C)-H$_6$ | pTT114 | pMAL-c2x |
| **4** (SEQ ID NO:254) | SBP-CL$^C$-Trx-H$_6$ | pTT43 | pET16b |
| **4a** (SEQ ID NO:256) | SBP-CL$^C$(S+1C)-Trx-H$_6$ | pTT115 | pET16b |
| **5** (SEQ ID NO:257) | eGFP-CL$^N$(S1A)-H$_6$ | pSH04 | pET28a |
| **6** (SEQ ID NO:258) | SBP-CL$^C$(N159'A)-Trx-H$_6$ | pSH02 | pET16b |
| **7** (SEQ ID NO:259) | CysTag-CL$^N$-SBP | pTT119 | pQE31 |
| **8** (SEQ ID NO:260) | SBP-CL$^C$-V$_H$H$^{GFP}$-H$_6$ | pFF05 | pET16b |
| **9** (SEQ ID Nos:261 and 262) | Fluorescein-CL$^N$ | Solid phase peptide synthesis | - |
| **10** (SEQ ID NO:264) | V$_H$H$^{EGFR}$-CL$^N$-SBP | pFF11 | pQE31 |
| **11** (SEQ ID NO:263) | SBP-CL$^C$-CysTag-H$_6$ | pTT83 | pET16b |
| **12** (SEQ ID NO:266) | MBP-Fc-CL$^N$-SBP | pTT109 | pMAL-c2x |
| **14** (SEQ ID NO:268) | eGFP-CL$^N$-H$_6$ | pTT70 | pET28a |
| **16** (SEQ ID NO:269) | CysTag-H$_8$-CL$^N$-eGFP(C49S) | pTT116 | pQE31 |

## Figure 22

| Protein number (when mentioned in text and figures) | Name of construct | Encoding plasmid | Vector backbone |
|---|---|---|---|
| - (SEQ ID NO:273) | HA-eGFP-Trx-TMD-mCherry | pMBH63 | pDisplay |
| - (SEQ ID NO:274) | HA-EGFR-mCherry | pMBH65 | pDisplay |
| 13 (SEQ ID NO:265) | HA-CL$^C$-Trx-TMD-mCherry | pMBH41 | pDisplay |
| 15 (SEQ ID NO:267) | HA-V$_H$H$^{GFP}$-CL$^C$-Trx-TMD-mCherry | pMBH42 | pDisplay |
| 15a (SEQ ID NO:270) | HA-V$_H$H$^{GFP}$-CL$^C$(N159'A)-Trx-TMD-mCherry | pMBH53 | pDisplay |
| 17 (SEQ ID NO:271) | HA-V$_H$H$^{GFP}$-CL$^C$-IFNAR1-mCherry | pRS009 | pDisplay |
| 17a (SEQ ID NO:272) | HA-V$_H$H$^{GFP}$-CL$^C$(N159'A)-IFNAR1-mCherry | pRS028 | pDisplay |

**Figure 23**

| Protein/peptide number | Sequence |
|---|---|
| 1 (SEQ ID NO:251) | MKTEEGKLVIWINGDKGYNGLAEVGKKFEKDTGIKVTVEHPDKLEEKFPQVAAT GDGPDIIFWAHDRFGGYAQSGLLAEITPDKAFQDKLYPFTWDAVRYNGKLIAYP IAVEALSLIYNKDLLPNPPKTWEEIPALDKELKAKGKSALMFNLQEPYFTWPLIA ADGGYAFKYENGKYDIKDVGVDNAGAKAGLTFLVDLIKNKHMNADTDYSIAEA AFNKGETAMTINGPWAWSNIDTSKVNYGVTVLPTFKGQPSKPFVGVLSAGINA ASPNKELAKEFLENYLLTDEGLEAVNKDKPLGAVALKSYEEELAKDPRIAATME NAQKGEIMPNIPQMSAFWYAVRTAVINAASGRQTVDEALKDAQTNSSSNNNN NNNNNLGIEGRISEFYIDTDSVVGDTIIDVSGKKMTIAEFYDSTPDVFMRRNDE ARDWVKRVGGKTSLSVNTYSGEVERKNINYIMKHTVKKRMFKIKAGGKEVIVTA DHSVMVKRDGKIIDVKPTEMKQTDRVVKWMLTGSHHHHHH |
| 2 (SEQ ID NO:252) | MDEKTTGWRGGHVVEGLAGELEQLRARLEHHPQGQREPGASGGGGSSSMIE FIEFEIEDLGVMEIDVYDIEVDGNHNFFGNDILVHNSVYLNGTMDEKTTGWRGG HVVEGLAGELEQLRARLEHHPQGQREP |
| 3 (SEQ ID NO:253) | MKTEEGKLVIWINGDKGYNGLAEVGKKFEKDTGIKVTVEHPDKLEEKFPQVAAT GDGPDIIFWAHDRFGGYAQSGLLAEITPDKAFQDKLYPFTWDAVRYNGKLIAYP IAVEALSLIYNKDLLPNPPKTWEEIPALDKELKAKGKSALMFNLQEPYFTWPLIA ADGGYAFKYENGKYDIKDVGVDNAGAKAGLTFLVDLIKNKHMNADTDYSIAEA AFNKGETAMTINGPWAWSNIDTSKVNYGVTVLPTFKGQPSKPFVGVLSAGINA ASPNKELAKEFLENYLLTDEGLEAVNKDKPLGAVALKSYEEELAKDPRIAATME NAQKGEIMPNIPQMSAFWYAVRTAVINAASGRQTVDEALKDAQTNSSSNNNN NNNNNLGIEGRISEFYIDTDSVVGDTIIDVSGKKMTIAEFYDSTPDGSHHHHHH |
| 3a (SEQ ID NO:255) | MKTEEGKLVIWINGDKGYNGLAEVGKKFEKDTGIKVTVEHPDKLEEKFPQVAAT GDGPDIIFWAHDRFGGYAQSGLLAEITPDKAFQDKLYPFTWDAVRYNGKLIAYP IAVEALSLIYNKDLLPNPPKTWEEIPALDKELKAKGKSALMFNLQEPYFTWPLIA ADGGYAFKYENGKYDIKDVGVDNAGAKAGLTFLVDLIKNKHMNADTDYSIAEA AFNKGETAMTINGPWAWSNIDTSKVNYGVTVLPTFKGQPSKPFVGVLSAGINA ASPNKELAKEFLENYLLTDEGLEAVNKDKPLGAVALKSYEEELAKDPRIAATME NAQKGEIMPNIPQMSAFWYAVRTAVINAASGRQTVDEALKDAQTNSSSNNNN NNNNNLGIEGRISEFYIDTDCVVGDTIIDVSGKKMTIAEFYDSTPDGSHHHHHH |
| 4 (SEQ ID NO:254) | MDEKTTGWRGGHVVEGLAGELEQLRARLEHHPQGQREPGASGGGGSSSEA RDWVKRVGGKTSLSVNTYSGEVERKNINYIMKHTVKKRMFKIKAGGKEVIVTA DHSVMVKRDGKIIDVKPTEMKQTDRVVKWMLTGSHMIEFIEFEIEDLGVMEIDV YDIEVDGNHNFFGNDILVHNSVYLNGTGSDKIIHLTDDSFDTDVLKADGAILVDF WAHWCGPCKMIAPILDEIADEYQGKLTVAKLNIDHNPGTAPKYGIRGIPTLLLFK NGEVAATKVGALSKGQLKEFLDANLAGSEFRSHHHHHH |
| 4a (SEQ ID NO:256) | MDEKTTGWRGGHVVEGLAGELEQLRARLEHHPQGQREPGASGGGGSSSEA RDWVKRVGGKTSLSVNTYSGEVERKNINYIMKHTVKKRMFKIKAGGKEVIVTA DHSVMVKRDGKIIDVKPTEMKQTDRVVKWMLTGSHMIEFIEFEIEDLGVMEIDV |

| | |
|---|---|
| | YDIEVDGNHNFFGNDILVHNCVYLNGTGSDKIIHLTDDSFDTDVLKADGAILVDF WAHWCGPCKMIAPILDEIADEYQGKLTVAKLNIDHNPGTAPKYGIRGIPTLLLFK NGEVAATKVGALSKGQLKEFLDANLAGSEFRSHHHHHH |
| 5 (SEQ ID NO:257) | MASWSHPQFEKASGTVSKGEELFTGVVPILVELDGDVNGHKFSVSGEGEGDA TYGKLTLKFICTTGKLPVPWPTLVTTLTYGVQCFSRYPDHMKQHDFFKSAMPE GYVQERTIFFKDDGNYKTRAEVKFEGDTLVNRIELKGIDFKEDGNILGHKLEYN YNSHNVYIMADKQKNGIKVNFKIRHNIEDGSVQLADHYQQNTPIGDGPVLLPDN HYLSTQSALSKDPNEKRDHMVLLEFVTAAGITLGMDELYKGSYIDTDAVVGDTII DVSGKKMTIAEFYDSTPDGSHHHHHH |
| 6 (SEQ ID NO:258) | MDEKTTGWRGGHVVEGLAGELEQLRARLEHHPQGQREPGASGGGGSSSEA RDWVKRVGGKTSLSVNTYSGEVERKNINYIMKHTVKKRMFKIKAGGKEVIVTA DHSVMVKRDGKIIDVKPTEMKQTDRVVKWMLTGSHMIEFIEFEIEDLGVMEIDV YDIEVDGNHNFFGNDILVHASVYLNGTGSDKIIHLTDDSFDTDVLKADGAILVDF WAHWCGPCKMIAPILDEIADEYQGKLTVAKLNIDHNPGTAPKYGIRGIPTLLLFK NGEVAATKVGALSKGQLKEFLDANLAGSEFRSHHHHHH |
| 7 (SEQ ID NO:259) | MGCDTDSVVGDTIIDVSGKKMTIAEFYDSTPDSGGSPRKVIKMESEERSMDEK TTGWRGGHVVEGLAGELEQLRARLEHHPQGQREP |
| 8 (SEQ ID NO:260) | MDEKTTGWRGGHVVEGLAGELEQLRARLEHHPQGQREPGASGGGGSSSEA RDWVKRVGGKTSLSVNTYSGEVERKNINYIMKHTVKKRMFKIKAGGKEVIVTA DHSVMVKRDGKIIDVKPTEMKQTDRVVKWMLTGSHMIEFIEFEIEDLGVMEIDV YDIEVDGNHNFFGNDILVHNSVYLNGMAQVQLVESGGALVQPGGSLRLSCAAS GFPVNRYSMRWYRQAPGKEREWVAGMSSAGDRSSYEDSVKGRFTISRDDAR NTVYLQMNSLKPEDTAVYYCNVNVGFEYWGQGTQVTVSSPDRSHHHHHH |
| 9 (SEQ ID Nos:261 and 262) | Fluorescein-YIDTDSVVGDTIIDVSGKK-NorL-TIAEFYDSTPD |
| 10 (SEQ ID NO:264) | MDEKTTGWRGGHVVEGLAGELEQLRARLEHHPQGQREPGASGGGGSSSEA RDWVKRVGGKTSLSVNTYSGEVERKNINYIMKHTVKKRMFKIKAGGKEVIVTA DHSVMVKRDGKIIDVKPTEMKQTDRVVKWMLTGSHMIEFIEFEIEDLGVMEIDV YDIEVDGNHNFFGNDILVHNSVYLNGMAQVQLVESGGALVQPGGSLRLSCAAS GFPVNRYSMRWYRQAPGKEREWVAGMSSAGDRSSYEDSVKGRFTISRDDAR NTVYLQMNSLKPEDTAVYYCNVNVGFEYWGQGTQVTVSSPDRSHHHHHH |
| 11 (SEQ ID NO:263) | MDEKTTGWRGGHVVEGLAGELEQLRARLEHHPQGQREPGASGGGGSSSEA RDWVKRVGGKTSLSVNTYSGEVERKNINYIMKHTVKKRMFKIKAGGKEVIVTA DHSVMVKRDGKIIDVKPTEMKQTDRVVKWMLTGSHMIEFIEFEIEDLGVMEIDV YDIEVDGNHNFFGNDILVHNSVYASPAAPAPASCHHHHHH |
| 12 (SEQ ID NO:266) | MKTEEGKLVIWINGDKGYNGLAEVGKKFEKDTGIKVTVEHPDKLEEKFPQVAAT GDGPDIIFWAHDRFGGYAQSGLLAEITPDKAFQDKLYPFTWDAVRYNGKLIAYP IAVEALSLIYNKDLLPNPPKTWEEIPALDKELKAKGKSALMFNLQEPYFTWPLIA ADGGYAFKYENGKYDIKDVGVDNAGAKAGLTFLVDLIKNKHMNADTDYSIAEA AFNKGETAMTINGPWAWSNIDTSKVNYGVTVLPTFKGQPSKPFVGVLSAGINA |

| | |
|---|---|
| | ASPNKELAKEFLENYLLTDEGLEAVNKDKPLGAVALKSYEEELAKDPRIAATME NAQKGEIMPNIPQMSAFWYAVRTAVINAASGRQTVDEALKDAQTNSSSNNNN NNNNNNLGIEGRISEFDKTHTCPPCPAPEAEGAPSVFLFPPKPKDTLMISRTPE VTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQV SLTCLVKGFYPSDIAVEWESNGQPENNYKTTPPVLDSDGSFFLYSKLTVDKSR WQQGNVFSCSVMHEALHNHYTQKSLSLSPGKGSEFYIDTDSVVGDTIIDVSGK KMTIAEFYDSTPDGSGSGSSRMDEKTTGWRGGHVVEGLAGELEQLRARLEHH PQGQREP |
| **14** (SEQ ID NO:268) | MASWSHPQFEKASGTVSKGEELFTGVVPILVELDGDVNGHKFSVSGEGEGDA TYGKLTLKFICTTGKLPVPWPTLVTTLTYGVQCFSRYPDHMKQHDFFKSAMPE GYVQERTIFFKDDGNYKTRAEVKFEGDTLVNRIELKGIDFKEDGNILGHKLEYN YNSHNVYIMADKQKNGIKVNFKIRHNIEDGSVQLADHYQQNTPIGDGPVLLPDN HYLSTQSALSKDPNEKRDHMVLLEFVTAAGITLGMDELYKGSYIDTDSVVGDTII DVSGKKMTIAEFYDSTPDGSHHHHHH |
| **16** (SEQ ID NO:269) | MGCHHHHHHHHYIDTDSVVGDTIIDVSGKKMTIAEFYDSTPDSGGSGGSMVSK GEELFTGVVPILVELDGDVNGHKFSVSGEGEGDATYGKLTLKFISTTGKLPVPW PTLVTTLTYGVQCFSRYPDHMKQHDFFKSAMPEGYVQERTIFFKDDGNYKTRA EVKFEGDTLVNRIELKGIDFKEDGNILGHKLEYNYNSHNVYIMADKQKNGIKVNF KIRHNIEDGSVQLADHYQQNTPIGDGPVLLPDNHYLSTQSALSKDPNEKRDHM VLLEFVTAAGITLGMDELYK |

**Figure 24**

| Name of construct | Sequence |
|---|---|
| HA-eGFP-Trx-TMD-mCherry (SEQ ID NO:273) | METDTLLLWVLLLWVPGSTGDYPYDVPDYAGAHSRVSKGEELFTGVVPILVEL DGDVNGHKFSVSGEGEGDATYGKLTLKFICTTGKLPVPWPTLVTTLTYGVQCF SRYPDHMKQHDFFKSAMPEGYVQERTIFFKDDGNYKTRAEVKFEGDTLVNRIE LKGIDFKEDGNILGHKLEYNYNSHNVYIMADKQKNGIKVNFKIRHNIEDGSVQLA DHYQQNTPIGDGPVLLPDNHYLSTQSALSKDPNEKRDHMVLLEFVTAAGITLG MDELYKSSSGTGSDKIIHLTDDSFDTDVLKADGAILVDFWAHWCGPCKMIAPIL DEIADEYQGKLTVAKLNIDHNPGTAPKYGIRGIPTLLLFKNGEVAATKVGALSKG QLKEFLDANLAGSEFRSHHHHHHYVDEQKLISEEDLNAVGQDTQEVIVVPHSL PFKVVVISAILALVVLTIISLIILIMLWQKKPRASMVSKGEEDNMAIIKEFMRFKVH MEGSVNGHEFEIEGEGEGRPYEGTQTAKLKVTKGGPLPFAWDILSPQFMYGS KAYVKHPADIPDYLKLSFPEGFKWERVMNFEDGGVVTVTQDSSLQDGEFIYKV KLRGTNFPSDGPVMQKKTMGWEASSERMYPEDGALKGEIKQRLKLKDGGHY DAEVKTTYKAKKPVQLPGAYNVNIKLDITSHNEDYTIVEQYERAEGRHSTGGM DELYK |
| HA-EGFR-mCherry (SEQ ID NO:274) | METDTLLLWVLLLWVPGSTGDYPYDVPDYAGAHSRELEEKKVCQGTSNKLTQ LGTFEDHFLSLQRMFNNCEVVLGNLEITYVQRNYDLSFLKTIQEVAGYVLIALNT VERIPLENLQIIRGNMYYENSYALAVLSNYDANKTGLKELPMRNLQEILHGAVRF SNNPALCNVESIQWRDIVSSDFLSNMSMDFQNHLGSCQKCDPSCPNGSCWG AGEENCQKLTKIICAQQCSGRCRGKSPSDCCHNQCAAGCTGPRESDCLVCRK FRDEATCKDTCPPLMLYNPTTYQMDVNPEGKYSFGATCVKKCPRNYVVTDHG SCVRACGADSYEMEEDGVRKCKKCEGPCRKVCNGIGIGEFKDSLSINATNIKH FKNCTSISGDLHILPVAFRGDSFTHTPPLDPQELDILKTVKEITGFLLIQAWPENR TDLHAFENLEIIRGRTKQHGQFSLAVVSLNITSLGLRSLKEISDGDVIISGNKNLC YANTINWKKLFGTSGQKTKIISNRGENSCKATGQVCHALCSPEGCWGPEPRD CVSCRNVSRGRECVDKCNLLEGEPREFVENSECIQCHPECLPQAMNITCTGR GPDNCIQCAHYIDGPHCVKTCPAGVMGENNTLVWKYADAGHVCHLCHPNCTY GCTGPGLEGCPTNGPKIPSIATGMVGALLLLLVVALGIGLFMRRRHIVRKRTLR RGSGSASMVSKGEEDNMAIIKEFMRFKVHMEGSVNGHEFEIEGEGEGRPYEG TQTAKLKVTKGGPLPFAWDILSPQFMYGSKAYVKHPADIPDYLKLSFPEGFKW ERVMNFEDGGVVTVTQDSSLQDGEFIYKVKLRGTNFPSDGPVMQKKTMGWE ASSERMYPEDGALKGEIKQRLKLKDGGHYDAEVKTTYKAKKPVQLPGAYNVNI KLDITSHNEDYTIVEQYERAEGRHSTGGMDELYK |
| HA-CL^C-Trx-TMD-mCherry (13) (SEQ ID NO:265) | METDTLLLWVLLLWVPGSTGDYPYDVPDYAGAQSGGGGSSSEARDWVKRVG GKTSLSVNTYSGEVERKNINYIMKHTVKKRMFKIKAGGKEVIVTADHSVMVKRD GKIIDVKPTEMKQTDRVVKWMLTGSHMIEFIEFEIEDLGVMEIDVYDIEVDGNHN FFGNDILVHNSVYLNSGGSGTGSDKIIHLTDDSFDTDVLKADGAILVDFWAHWC GPCKMIAPILDEIADEYQGKLTVAKLNIDHNPGTAPKYGIRGIPTLLLFKNGEVAA TKVGALSKGQLKEFLDANLAGSEFRSHHHHHHYVDEQKLISEEDLNAVGQDTQ |

| | |
|---|---|
| | EVIVVPHSLPFKVVVISAILALVVLTIISLIILIMLWQKKPRASMVSKGEEDNMAIIK EFMRFKVHMEGSVNGHEFEIEGEGEGRPYEGTQTAKLKVTKGGPLPFAWDIL SPQFMYGSKAYVKHPADIPDYLKLSFPEGFKWERVMNFEDGGVVTVTQDSSL QDGEFIYKVKLRGTNFPSDGPVMQKKTMGWEASSERMYPEDGALKGEIKQRL KLKDGGHYDAEVKTTYKAKKPVQLPGAYNVNIKLDITSHNEDYTIVEQYERAEG RHSTGGMDELYK |
| HA-V$_H$H$^{GFP}$-CL$^C$-Trx-TMD-mCherry (15) (SEQ ID NO:267) | METDTLLLWVLLLWVPGSTGDYPYDVPDYAGAHSRMAQVQLVESGGALVQP GGSLRLSCAASGFPVNRYSMRWYRQAPGKEREWVAGMSSAGDRSSYEDSV KGRFTISRDDARNTVYLQMNSLKPEDTAVYYCNVNVGFEYWGQGTQVTVSSP DWAQSGGGGSSSEARDWVKRVGGKTSLSVNTYSGEVERKNINYIMKHTVKK RMFKIKAGGKEVIVTADHSVMVKRDGKIIDVKPTEMKQTDRVVKWMLTGSHMI EFIEFEIEDLGVMEIDVYDIEVDGNHNFFGNDILVHNSVYLNSGGSGTGSDKIIHL TDDSFDTDVLKADGAILVDFWAHWCGPCKMIAPILDEIADEYQGKLTVAKLNID HNPGTAPKYGIRGIPTLLLFKNGEVAATKVGALSKGQLKEFLDANLAGSEFRSH HHHHHYVDEQKLISEEDLNAVGQDTQEVIVVPHSLPFKVVVISAILALVVLTIISLII LIMLWQKKPRASMVSKGEEDNMAIIKEFMRFKVHMEGSVNGHEFEIEGEGEGR PYEGTQTAKLKVTKGGPLPFAWDILSPQFMYGSKAYVKHPADIPDYLKLSFPE GFKWERVMNFEDGGVVTVTQDSSLQDGEFIYKVKLRGTNFPSDGPVMQKKT MGWEASSERMYPEDGALKGEIKQRLKLKDGGHYDAEVKTTYKAKKPVQLPGA YNVNIKLDITSHNEDYTIVEQYERAEGRHSTGGMDELYK |
| HA-V$_H$H$^{GFP}$-CL$^C$(N159'A)-Trx-TMD-mCherry (15a) (SEQ ID NO:270) | METDTLLLWVLLLWVPGSTGDYPYDVPDYAGAHSRMAQVQLVESGGALVQP GGSLRLSCAASGFPVNRYSMRWYRQAPGKEREWVAGMSSAGDRSSYEDSV KGRFTISRDDARNTVYLQMNSLKPEDTAVYYCNVNVGFEYWGQGTQVTVSSP DWAQSGGGGSSSEARDWVKRVGGKTSLSVNTYSGEVERKNINYIMKHTVKK RMFKIKAGGKEVIVTADHSVMVKRDGKIIDVKPTEMKQTDRVVKWMLTGSHMI EFIEFEIEDLGVMEIDVYDIEVDGNHNFFGNDILVHASVYLNSGGSGTGSDKIIHL TDDSFDTDVLKADGAILVDFWAHWCGPCKMIAPILDEIADEYQGKLTVAKLNID HNPGTAPKYGIRGIPTLLLFKNGEVAATKVGALSKGQLKEFLDANLAGSEFRSH HHHHHYVDEQKLISEEDLNAVGQDTQEVIVVPHSLPFKVVVISAILALVVLTIISLII LIMLWQKKPRASMVSKGEEDNMAIIKEFMRFKVHMEGSVNGHEFEIEGEGEGR PYEGTQTAKLKVTKGGPLPFAWDILSPQFMYGSKAYVKHPADIPDYLKLSFPE GFKWERVMNFEDGGVVTVTQDSSLQDGEFIYKVKLRGTNFPSDGPVMQKKT MGWEASSERMYPEDGALKGEIKQRLKLKDGGHYDAEVKTTYKAKKPVQLPGA YNVNIKLDITSHNEDYTIVEQYERAEGRHSTGGMDELYK |
| HA-V$_H$H$^{GFP}$-CL$^C$-IFNAR1-mCherry (17) (SEQ ID NO:271) | METDTLLLWVLLLWVPGSTGDYPYDVPDYAGAHSRMAQVQLVESGGALVQP GGSLRLSCAASGFPVNRYSMRWYRQAPGKEREWVAGMSSAGDRSSYEDSV KGRFTISRDDARNTVYLQMNSLKPEDTAVYYCNVNVGFEYWGQGTQVTVSSP DWAQSGGGGSSSEARDWVKRVGGKTSLSVNTYSGEVERKNINYIMKHTVKK RMFKIKAGGKEVIVTADHSVMVKRDGKIIDVKPTEMKQTDRVVKWMLTGSHMI EFIEFEIEDLGVMEIDVYDIEVDGNHNFFGNDILVHNSVYLNSQPARSKNLKSPQ KVEVDIIDDNFILRWNRSDESVGNVTFSFDYQKTGMDNWIKLSGCQNITSTKCN |

| | |
|---|---|
| | FSSLKLNVYEEIKLRIRAEKENTSSWYEVDSFTPFRKAQIGPPEVHLEAEDKAIVI HISPGTKDSVMWALDGLSFTYSLLIWKNSSGVEERIENIYSRHKIYKLSPETTYC LKVKAALLTSWKIGVYSPVHCIKTTVENELPPPENIEVSVQNQNYVLKWDYTYA NMTFQVQWLHAFLKRNPGNHLYKWKQIPDCENVKTTQCVFPQNVFQKGIYLL RVQASDGNNTSFWSEEIKFDTEIQAFLLPPVFNIRSLSDSFHIYIGAPKQSGNTP VIQDYPLIYEIIFWENTSNAERKIIEKKTDVTVPNLKPLTVYCVKARAHTMDEKLN KSSVFSDAVCEKTKPGNTSKIWLIVGICIALFALPFVIYAAKVFLRCINYVFFPSLK PSSSIDEYFSEQPLKNLLLSTSEEQIEKCFIIENISTIATVEETNQTDEDHKKYSSQ TSQDSGNYSNEDESESKTSEELQQDFVLAEASMVSKGEEDNMAIIKEFMRFKV HMEGSVNGHEFEIEGEGEGRPYEGTQTAKLKVTKGGPLPFAWDILSPQFMYG SKAYVKHPADIPDYLKLSFPEGFKWERVMNFEDGGVVTVTQDSSLQDGEFIYK VKLRGTNFPSDGPVMQKKTMGWEASSERMYPEDGALKGEIKQRLKLKDGGH YDAEVKTTYKAKKPVQLPGAYNVNIKLDITSHNEDYTIVEQYERAEGRHSTGGM DELYK |
| HA-V<sub>H</sub>H<sup>GFP</sup>-CL<sup>C</sup>(N159'A)-IFNAR1-mCherry **(17a)** (SEQ ID NO:272) | METDTLLLWVLLLWVPGSTGDYPYDVPDYAGAHSRMAQVQLVESGGALVQP GGSLRLSCAASGFPVNRYSMRWYRQAPGKEREWVAGMSSAGDRSSYEDSV KGRFTISRDDARNTVYLQMNSLKPEDTAVYYCNVNVGFEYWGQGTQVTVSSP DWAQSGGGGSSSEARDWVKRVGGKTSLSVNTYSGEVERKNINYIMKHTVKK RMFKIKAGGKEVIVTADHSVMVKRDGKIIDVKPTEMKQTDRVVKWMLTGSHMI EFIEFEIEDLGVMEIDVYDIEVDGNHNFFGNDILVHASVYLNSQPARSKNLKSPQ KVEVDIIDDNFILRWNRSDESVGNVTFSFDYQKTGMDNWIKLSGCQNITSTKCN FSSLKLNVYEEIKLRIRAEKENTSSWYEVDSFTPFRKAQIGPPEVHLEAEDKAIVI HISPGTKDSVMWALDGLSFTYSLLIWKNSSGVEERIENIYSRHKIYKLSPETTYC LKVKAALLTSWKIGVYSPVHCIKTTVENELPPPENIEVSVQNQNYVLKWDYTYA NMTFQVQWLHAFLKRNPGNHLYKWKQIPDCENVKTTQCVFPQNVFQKGIYLL RVQASDGNNTSFWSEEIKFDTEIQAFLLPPVFNIRSLSDSFHIYIGAPKQSGNTP VIQDYPLIYEIIFWENTSNAERKIIEKKTDVTVPNLKPLTVYCVKARAHTMDEKLN KSSVFSDAVCEKTKPGNTSKIWLIVGICIALFALPFVIYAAKVFLRCINYVFFPSLK PSSSIDEYFSEQPLKNLLLSTSEEQIEKCFIIENISTIATVEETNQTDEDHKKYSSQ TSQDSGNYSNEDESESKTSEELQQDFVLAEASMVSKGEEDNMAIIKEFMRFKV HMEGSVNGHEFEIEGEGEGRPYEGTQTAKLKVTKGGPLPFAWDILSPQFMYG SKAYVKHPADIPDYLKLSFPEGFKWERVMNFEDGGVVTVTQDSSLQDGEFIYK VKLRGTNFPSDGPVMQKKTMGWEASSERMYPEDGALKGEIKQRLKLKDGGH YDAEVKTTYKAKKPVQLPGAYNVNIKLDITSHNEDYTIVEQYERAEGRHSTGGM DELYK |

**Figure 25**

Figure 26

Figure 27

A)

MGEARDWVKRVGGKTSLSVNTYSGEVERKNINYIMKHTVKKRMFKIKAGGKEVIVTADHSVMVKRDG
KIIDVKPTEMKQTDRVVKWMLTGSHMIEFIEFEIEDLGVMEIDVYDIEVDGNHNFFGNDILVHNSVYLNG
TGSDKIIHLTDDSFDTDVLKADGAILVDFWAHWCGPCKMIAPILDEIADEYQGKLTVAKLNIDHNPGTAP
KYGIRGIPTLLLFKNGEVAATKVGALSKGQLKEFLDANLAGSEFRSHHHHHH (SEQ ID NO:275)

B)

MDEKTTGWRGGHVVEGLAGELEQLRARLEHHPQGQREPGASGGGGSSSEARDWVKRVGGKTSLS
VNTYSGEVERKNINYIMKHTVKKRMFKIKAGGKEVIVTADHSVMVKRDGKIIDVKPTEMKQTDRVVKW
MLTGSHMIEFIEFEIEDLGVMEIDVYDIEVDGNHNFFGNDILVHNSVYLNGTMDEKTTGWRGGHVVEG
LAGELEQLRARLEHHPQGQREP (SEQ ID NO:276)

C)

MKTEEGKLVIWINGDKGYNGLAEVGKKFEKDTGIKVTVEHPDKLEEKFPQVAATGDGPDIIFWAHDRF
GGYAQSGLLAEITPDKAFQDKLYPFTWDAVRYNGKLIAYPIAVEALSLIYNKDLLPNPPKTWEEIPALDK
ELKAKGKSALMFNLQEPYFTWPLIAADGGYAFKYENGKYDIKDVGVDNAGAKAGLTFLVDLIKNKHMN
ADTDYSIAEAAFNKGETAMTINGPWAWSNIDTSKVNYGVTVLPTFKGQPSKPFVGVLSAGINAASPNK
ELAKEFLENYLLTDEGLEAVNKDKPLGAVALKSYEEELAKDPRIAATMENAQKGEIMPNIPQMSAFWY
AVRTAVINAASGRQTVDEALKDAQTNSSSNNNNNNNNNNLGIEGRISEFSGDTDSVHGKTHVFIRSIK
NGSHHHHHH (SEQ ID NO:277)

D)

MQEAKIDIKSLYDSLAKKYDVQHKNSYEVIYPKGYEIKVLGNKYVKLVAMSRHKTQKHLVKIVVKSEKTI
DSLDPIRQKSLLKKQDEVVVTTDHICMVYNDDHFFENVNAKNLKVGNYVSVYDEASDKEVIGEIASIED
LGMTDDYVYDCEVDDDSHAFYASNILVHNSQFCNGTGSDKIIHLTDDSFDTDVLKADGAILVDFWAHW
CGPCKMIAPILDEIADEYQGKLTVAKLNIDHNPGTAPKYGIRGIPTLLLFKNGEVAATKVGALSKGQLKE
FLDANLAGSEFRSHHHHHH (SEQ ID NO:278)

E)

MQEAKIDIKSLYDSLAKKYDVQHKNSYEVIYPKGYEIKVLGNKYVKLVAMSRHKTQKHLVKIVVKSEKTI
DSLDPIRQKSLLKKQDEVVVTTDHIAMVYNDDHFFENVNAKNLKVGNYVSVYDEASDKEVIGEIASIED
LGMTDDYVYDAEVDDDSHAFYASNILVHNSQFCNGTGSDKIIHLTDDSFDTDVLKADGAILVDFWAHW
CGPCKMIAPILDEIADEYQGKLTVAKLNIDHNPGTAPKYGIRGIPTLLLFKNGEVAATKVGALSKGQLKE
FLDANLAGSEFRSHHHHHH (SEQ ID NO:279)

**Figure 28**

Aes[N]   N-terminal sequence of a cysteine-less split intein identified in PolB-type DNA polymerase genes from T4-like bacteriophages of *Aeromonas salmonicida*

SVVGDTIIDVSGKKMTIAEFYDSTPDVFMRRNDEARDWVKRVGGKTSLSVNTYSGEVERKNI NYIMKHTVKKRMFKIKAGGKEVIVTADHSVMVKRDGKIIDVKPTEMKQTDRVVKWMLT (SEQ ID NO:1)

Aes[C]   C-terminal sequence of a cysteine-less split intein identified in PolB-type DNA polymerase genes from T4-like bacteriophages of *Aeromonas salmonicida*

MIEFIEFEIEDLGVMEIDVYDIEVDGNHNFFGNDILVHN (SEQ ID NO:2)

CL[N]   modified N-terminal sequence of a cysteine-less split intein identified in a PolB-type DNA polymerase genes from T4-like bacteriophages of *Aeromonas salmonicida*

SVVGDTIIDVSGKKMTIAEFYDSTPD (SEQ ID NO:3)

CL[C]   modified C-terminal sequence of a cysteine-less split intein identified in PolB-type DNA polymerase genes from T4-like bacteriophages of *Aeromonas salmonicida*

EARDWVKRVGGKTSLSVNTYSGEVERKNINYIMKHTVKKRMFKIKAGGKEVIVTADHSVMVK RDGKIIDVKPTEMKQTDRVVKWMLTGSHMIEFIEFEIEDLGVMEIDVYDIEVDGNHNFFGNDILVHN (SEQ ID NO:4)

Aes123-BP_PolB-C   Aes123-BP_PolB-C1   395653338:1674-2552 Aeromonas   phage   Aes123
    DNA polymerase family B, C-terminal fragment 173-292        0       N-part of a split intein;
part of a fractured-gene
    SVVGDTIIDVSGKKMTIAEFYDSTPDVFMRRNDEARDWVKRVGGKTSLSVNTYSGEVERKNI NYIMKHTVKKRMFKIKAGGKEVIVTADHSVMVKRDGKIIDVKPTEMKQTDRVVKWMLT (SEQ ID NO:5)

Aes123-BP_PolB-C   Aes123-BP_PolB-C2   395653338:3582-4544 Aeromonas   phage   Aes123
    DNA polymerase family B, C-terminal fragment 1-39    0       C-part of a split intein; part of a
fractured-gene  MIEFIEFEIEDLGVMEIDVYDIEVDGNHNFFGNDILVHN (SEQ ID NO:6)

Aes144-BP_PolB-C   Aes144-BP_PolB-C1   395653345:1782-2745 Aeromonas   phage   Aes144
    DNA polymerase family B, C-terminal fragment 111-230        0       N-part of a split intein;
part of a fractured-gene
    SVVGDTIIDVSGKKMTIAEFYDSTPDVFMRRNDEARDWVKRVGGKTSLSVNTYSGEVERKNI NYIMKHTVKKRMFKIKAGGKEVIVTADHSVMVKRDGKIIDVKPTEMKQTDRVVKWMLT (SEQ ID NO:7)

Aes144-BP_PolB-C   Aes144-BP_PolB-C2   395653345:3775-4737 Aeromonas   phage   Aes144
    DNA polymerase family B, C-terminal fragment 1-39    0       C-part of a split intein; part of a
fractured-gene  MIEFIEFEIEDLGVMEIDVYDIEVDGNHNFFGNDILVHN (SEQ ID NO:8)

Aes508-BP_PolB-C    Aes508-BP_PolB-C1    423262092:20215-20738        Aeromonas    phage Aes508 DNA polymerase family B, C-terminal fragment 111-230        0        N-part of a split intein; part of a fractured-gene

SVVGDTIIDVSGKKMTIAEFYDSTPDVFMRRNDEARDWVKRVGGKTSLSVNTYSGEVERKNI NYIMKHTVKKRMFKIKAGGKEVIVTADHSVMVKRDGKIIDVKPTEMKQTDRVVKWMLT (SEQ ID NO:9)

Aes508-BP_PolB-C    Aes508-BP_PolB-C2    423262092:19185-18223        Aeromonas    phage Aes508 DNA polymerase family B, C-terminal fragment 1-39    0        C-part of a split intein; part of a fractured-gene  MIEFIEFEIEDLGVMEIDVYDIEVDGNHNFFGNDILVHN (SEQ ID NO:10)

Aes509-BP_PolB-C    Aes509-BP_PolB-C1    395653358:1763-2611 Aeromonas    phage    Aes509 DNA polymerase family B, C-terminal fragment 163-282        0        N-part of a split intein; part of a fractured-gene

SVVGDTIIDVSGKKMTIAEFYDSTPDVFMRRNDEARDWVKRVGGKTSLSVNTYSGEVERKNI NYIMKHTVKKRMFKIKAGGKEVIVTADHSVMVKRDGKIIDVKPTEMKQTDRVVKWMLT    (SEQ    ID NO:11)

Aes509-BP_PolB-C    Aes509-BP_PolB-C2    395653358:3641-4603 Aeromonas    phage    Aes509 DNA polymerase family B, C-terminal fragment 1-39    0        C-part of a split intein; part of a fractured-gene  MIEFIEFEIEDLGVMEIDVYDIEVDGNHNFFGNDILVHN (SEQ ID NO:12)

Aes512-BP_PolB-C    Aes512-BP_PolB-C1    395653366:2596-2763 Aeromonas    phage    Aes512 DNA polymerase family B, C-terminal fragment 111-230        0        N-part of a split intein; part of a fractured-gene

SVVGDTIIDVSGKKMTIAEFYDSTPDVFMRRNDEARDWVKRVGGKTSLSVNTYSGEVERKNI NYIMKHTVKKRMFKIKAGGKEVIVTADHSVMVKRDGKIIDVKPTEMKQTDRVVKWMLT    (SEQ    ID NO:13)

Aes512-BP_PolB-C    Aes512-BP_PolB-C2    395653366:4596-5558 Aeromonas    phage    Aes512 DNA polymerase family B, C-terminal fragment 1-39    0        C-part of a split intein; part of a fractured-gene  MIEFIEFEIEDLGVMEIDVYDIEVDGNHNFFGNDILVHN (SEQ ID NO:14)

Aes516-BP_PolB-C    Aes516-BP_PolB-C1    395653384:36741-37327        Aeromonas    phage Aes516 DNA polymerase family B, C-terminal fragment 111-251        0        N-part of a split intein; part of a fractured-gene

SVVGDTIIDVSGKKMTIAEFYDSTPDVFMRRNDEARDWVKRVGGKTSLSVNTYSGEVERKNI NYIMKHTVKKRMFKIKAGGKEVIVTADHSVMVKRDGKIIDVKPTEMKQTDRVVVWDNEYLANKDGTIN RIVEEIYDRVY (SEQ ID NO:15)

Aes516-BP_PolB-C    Aes516-BP_PolB-C2    395653384:36757-35795    Aeromonas    phage Aes516 DNA polymerase family B, C-terminal fragment 1-39    0    C-part of a split intein; part of a fractured-gene    MIEFIEFEIEDLGVMEIDVYDIEVDGNHNFFGNDILVHN (SEQ ID NO:16)

Aes517-BP_PolB-C    Aes517-BP_PolB-C1    422935113:179333-177122    Aeromonas    phage Aes517 DNA polymerase family B, C-terminal fragment 111-230    0    N-part of a split intein; part of a fractured-gene

SVVGDTIIDVSGKKMTIAEFYDSTPDVFMRRNDEARDWVKRVGGKTSLSVNTYSGEVERKNI NYIMKHTVKKRXFKIKAGGKEVIVTADHSVMVKRDGKIIDVKPTEMKQTDRVVKWMLT    (SEQ    ID NO:17)

Aes517-BP_PolB-C    Aes517-BP_PolB-C2    395653375:4586-5548 Aeromonas    phage    Aes517 DNA polymerase family B, C-terminal fragment 1-39    0    C-part of a split intein; part of a fractured-gene    MIEFIEFEIEDLGVMEIDVYDIEVDGNHNFFGNDILVHN (SEQ ID NO:18)

SLM-T5-Br_PolB-2    SLM-T5-Br_PolB-2-1    LFIK01000576.1:9422-11860    Russia    Kulunda-steppe soda lake Tanatar-5 brine environmental genomics    DNA polymerase family B    687-812    0    N-part of a split intein; part of a fractured-gene

SVAHDSLIRISRDNGTVQNTTIEELFLQGNEYWESNGKEYSLNSDIKIAHTGSSGVLNFVNYNY VYRHKVKNKARYRVTTSNGKSVVVTDDHSVMIMQDGRLIEKKPSEIKQGDLVITIVDSDTST (SEQ    ID NO:19)

SLM-T5-Br_PolB-2    SLM-T5-Br_PolB-2-2    LFIK01000576.1:11940-12956 Russia    Kulunda-steppe soda lake Tanatar-5 brine environmental genomics    DNA polymerase family B    1-43    0    C-part of a split intein; part of a fractured-gene    MDAKVSEVVGVERLDDFDDEYVYDIGVANDDPYFFANDILVHN (SEQ ID NO:20)

RnoDR-G_PolB-C-2    RnoDR-G_PolB-C-2-1    NFZD01009918.1:24388-25218 Rattus    norvegicus (Denmark: Riget) gut metagenome    DNA polymerase family B, C-terminal fragment 154-276    0    N-part of a split intein; part of a fractured-gene    SQSALTINYLDQEKMTVEDMFNKLKYENNDVVLRVSNGSEVVPVKNHTTKTFIMKEGVIDRPL KYIMRHKVTKSKWRLRTESGKEIIVTGDHSLMVLRDNELISLKPKDVNPKTDKIITIKDV (SEQ ID NO:21)

RnoDR-G_PolB-C-2    RnoDR-G_PolB-C-2-2    NFZD01009918.1:26401-27393 Rattus    norvegicus (Denmark: Riget) gut metagenome    DNA polymerase family B, C-terminal fragment 1-42    0    C-part of a split intein; part of a fractured-gene    MNYNIENIAVIEQIEDFQDEYVYDLEVEDTHTFFGNDILHN (SEQ ID NO:22)

RnoDR-G_PolB-C-1    RnoDR-G_PolB-C-1-1    NFZD01012512.1:21199-20369    Rattus norvegicus (Denmark: Riget) gut metagenome    DNA polymerase family B, C-terminal fragment 154-276    0    N-part of a split intein; part of a fractured-gene

SQSALTINYLDQEKMTVEDMFNKLKYENNDVVLRVSNGSEVVPVKNHTTKTFIMKEGVIDRPL
KYIMRHKVTKSKWRLRTESGKEIIVTGDHSLMVLRDNELISLKPKDVNPKTDKIITIKDV (SEQ ID NO:23)

RnoDR-G_PolB-C-1    RnoDR-G_PolB-C-1-2  NFZD01012512.1:19186-18194 Rattus    norvegicus
(Denmark: Riget) gut metagenome    DNA polymerase family B, C-terminal fragment 1-42    0
C-part of a split intein; part of a fractured-gene
MNYNIENIAVIEQIEDFQDEYVYDLEVEDTHTFFGNDILIHN (SEQ ID NO:24)

Lak-B4-BP_PolB-C    Lak-B4-BP_PolB-C-1    MK250023.1:523550-522738    Prevotella megaphage
Lak-B4 from faecal sample of Kenyan yellow baboon F30 from Amboseli Tanzania    DNA
polymerase family B, C-terminal fragment    149-270    0    N-part of a split intein; part of a
fractured-gene
SQTADTQVVIDNKVFSMEGFFTKAKYENDDVVIKLQNGSEVIPVHNHDTLSYKDHDYKTITRPI
NYIMRHKVTKDKFRLKTKSGKELIVTGDHSIMVIRNNELISVPAREIKKSDKIITLDR (SEQ ID NO:25)

Lak-B4-BP_PolB-C    Lak-B4-BP_PolB-C-2    MK250023.1:520760-519786    Prevotella megaphage
Lak-B4 from faecal sample of Kenyan yellow baboon F30 from Amboseli Tanzania    DNA
polymerase family B, C-terminal fragment    1-44    0    C-part of a split intein; part of a
fractured-gene  MNYNIQVEDIDVIEQLEDFNDEYVYDIEVDDTHTFFANEILAHN (SEQ ID NO:26)

Lak-C1-BP_PolB-C    Lak-C1-BP_PolB-C-1    MK250029.1:390899-391732    Prevotella megaphage
Lak-C1 isolated from faecal sample of cholera patient hospitalized in Dhaka, Bangladesh    DNA
polymerase family B, C-terminal fragment    147-277    0    N-part of a split intein; part of a
fractured-gene
SQLGSTQFRVDNNITTMEDFFIKAKYENNDVVIKLTNGSEIIPVHNHMTLSYKDHDYKTSERPI
NYIMRHKVTKDKFRLKTKSGKELIVTGDHSIMVIRDNELISLPAREIKKSDKIITIAGDNFTKAGDK    (SEQ
ID NO:27)

Lak-C1-BP_PolB-C    Lak-C1-BP_PolB-C-2    MK250029.1:393223-394197    Prevotella megaphage
Lak-C1 isolated from faecal sample of cholera patient hospitalized in Dhaka, Bangladesh    DNA
polymerase family B, C-terminal fragment    1-44    0    C-part of a split intein; part of a
fractured-gene  MNYDIQIEDIDVIEQLEDFNDEYVYDIEVDDTHTFFANDILAHN (SEQ ID NO:28)

Lak-B1-BP_PolB-C    Lak-B1-BP_PolB-C-1    MK250020.1:520956-520144    Prevotella megaphage
Lak-B1 from faecal sample of Kenyan yellow baboon F22 from Amboseli Tanzania    DNA
polymerase family B, C-terminal fragment    149-270    0    N-part of a split intein; part of a
fractured-gene
SQTADTQVVIDNKVFSMEGFFTKAKYENDDVVIKLQNGSEVIPVHNHDTLSYKDHDYKTITRPI
NYIMRHKVTKDKFRLKTKSGKELIVTGDHSIMVIRNNELISVPAREIKKSDKIITLDR (SEQ ID NO:29)

Lak-B1-BP_PolB-C    Lak-B1-BP_PolB-C-2    MK250020.1:518154-517180    Prevotella megaphage Lak-B1 from faecal sample of Kenyan yellow baboon F22 from Amboseli Tanzania    DNA polymerase family B, C-terminal fragment    1-44    0    C-part of a split intein; part of a fractured-gene  MNYNIQVEDIDVIEQLEDFNDEYVYDIEVDDTHTFFANEILAHN (SEQ ID NO:30)

Lak-A2-BP_PolB-C    Lak-A2-BP_PolB-C-1    MK250019.1:345475-346287    Prevotella megaphage Lak-A2 isolated from faecal sample of arsenic impacted individual 20 living in Eruani village, Laksam Upazila, Bangladesh    DNA polymerase family B, C-terminal fragment 147-270    0    N-part of a split intein; part of a fractured-gene

STSWKSSIYVDSVKLKVQDAFNKFKYENNDTVLKLNNGQEIVPVHNHNILSYVDHDAEATYRP IKYIMRHKVYNKSRFRIKSKSGKELEVTGDHSMMIIRNNELITVKAKDILKTDKIITIAGD (SEQ ID NO:31)

Lak-A2-BP_PolB-C    Lak-A2-BP_PolB-C-2    MK250019.1:346643-347617    Prevotella megaphage Lak-A2 isolated from faecal sample of arsenic impacted individual 20 living in Eruani village, Laksam Upazila, Bangladesh    DNA polymerase family B, C-terminal fragment 1-44    0    C-part of a split intein; part of a fractured-gene

MNYDIQIEDIDVIEQLEDFNDEYVYDIEVDDTHTFFANDILAHN (SEQ ID NO:32)

Lak-A1-BP_PolB-C    Lak-A1-BP_PolB-C-1    MK250016.1:426063-426878    Prevotella megaphage Lak-A1 (sample a) isolated from faecal sample of arsenic impacted individual 22 living in Eruani village, Laksam Upazila, Bangladesh    DNA polymerase family B, C-terminal fragment 150-271    0    N-part of a split intein; part of a fractured-gene

SQIGSTQFYVDNNITTMEDFFTKAKYENNDVVIKLQNGSEVVPVHNHNTLTYIDHDFKTSERPI NYIMRHKVTKDKFRLKTKSGKELIVTGDHSIMVIRNNELISLPAREIKNTDKIITLDK (SEQ ID NO:33)

Lak-A1-BP_PolB-C    Lak-A1-BP_PolB-C-2    MK250016.1:428327-429301    Prevotella megaphage Lak-A1 (sample a) isolated from faecal sample of arsenic impacted individual 22 living in Eruani village, Laksam Upazila, Bangladesh    DNA polymerase family B, C-terminal fragment 1-44    0    C-part of a split intein; part of a fractured-gene

MNYDIQIEDIDVIEQLEDFNDEYVYDIEVDDTHTFFANDILAHN (SEQ ID NO:34)

vB-PaeM-PA5oct-BP_PolB    vB-PaeM-PA5oct-BP_PolB-1    AWN03110.1    Pseudomonas phage vB_PaeM_PA5oct    DNA polymerase family B    687-803    0    N-part of a split intein; part of a fractured-gene

SVDGSTILNTSLGKITIEELFNVSDKHVVHAEKEFASNEDVMVMSWDNSAKQPYMGHINYVYR HEVEKELFEIEDNNGNKVIVTEDHSIMVIRNAELLEVKPAELTDSDIILSIVYE (SEQ ID NO:35)

vB-PaeM-PA5oct-BP_PolB    vB-PaeM-PA5oct-BP_PolB-2    AWN03112.1    Pseudomonas phage vB_PaeM_PA5oct    DNA polymerase family B    1-85    0    C-part of a split intein; part of a fractured-gene

MHNLTKHLLGVFSAKTEDEEYKSAKRALEELNKNIKERDPNKFNVSLGKVSKVTNLGKKKQY
VYDIGMKNPDNPYFFGNNILVHN (SEQ ID NO:36)


HsaMP-6_PolB-C    HsaMP-6_PolB-C-1    UMGV01005496.1:42068-41235    Megaphage
from human gut metagenome Denmark fecal sample    DNA polymerase family B, C-terminal fragment
147-277    0    N-part of a split intein; part of a fractured-gene
SQLGSTQFRVDNNITTMEDFFIKAKYENNDVVIKLTNGSEIIPVHNHMTLSYKDHDYKTSEMPI
NYIMRHKVTKDKFRLKTKSGKELIVTGDHSIMVIRDNELISLPAREIKKSDKIITIAGDNFTKAEDK    (SEQ
ID NO:37)


HsaMP-6_PolB-C    HsaMP-6_PolB-C-2    UMGV01005496.1:40724-39750    Megaphage
from human gut metagenome Denmark fecal sample    DNA polymerase family B, C-terminal fragment
1-44    0    C-part of a split intein; part of a fractured-gene
MNYDIQIEDIDVIEQLEDFNDEYVYDIEVDDTHTFFANDILAHN (SEQ ID NO:38)


HsaMP-5_PolB-C    HsaMP-5_PolB-C-1    OIWM01002393.1:525-1337    Megaphage    from
human gut metagenome Denmark fecal sample DNA polymerase family B, C-terminal fragment  147-
270    0    N-part of a split intein; part of a fractured-gene
STSWKSSIYVDSVKLKVQAAFNKFKYENNDTVLKLNNGQEIVPVHNHNILSYVDHDAEATYRP
IKYIMRHKVYNKSRFRIKTKSGKELEVTGDHSMMIIRNNELITVKAKDILKTDKIITITGD (SEQ ID NO:39)


HsaMP-5_PolB-C    HsaMP-5_PolB-C-2    OIWM01002393.1:1693-2667    Megaphage    from
human gut metagenome Denmark fecal sample DNA polymerase family B, C-terminal fragment  1-44
0    C-part of a split intein; part of a fractured-gene
MNYDIQIEDIDVIEQLEDFNDEYVYDIEVDDTHTFFANDILAHN (SEQ ID NO:40)


HsaMP-4_PolB-C    HsaMP-4_PolB-C-1    OCLB01000064.1:9068-8235    Megaphage    from
human gut metagenome Tanzanian Hadza hunter-gatherer fecal sample    DNA    polymerase
family B, C-terminal fragment    147-277    0    N-part of a split intein; part of a fractured-gene
SQLGSTQFRVDNDITTMEDFFVKAKYENNDVVIKLQNGSEVVPVHNHNTLTYKDHDYKTITRP
INYIMRHKVSKDKFRLKTKSGKELIVTGDHSIMVIRNNELVSVAAREIKKTDKIITIAGDNFNKAGDK
(SEQ ID NO:41)


HsaMP-4_PolB-C    HsaMP-4_PolB-C-2    OCLB01000064.1:6739-5765    Megaphage    from
human gut metagenome Tanzanian Hadza hunter-gatherer fecal sample    DNA    polymerase
family B, C-terminal fragment    1-44    0    C-part of a split intein; part of a fractured-gene
MNYDIQIEDIDVIEQLEDFNDEYVYDIEVDDTHTFFANDILAHN (SEQ ID NO:42)


HsaMP-3_PolB-C    HsaMP-3_PolB-C-1    ULXM01051442.1:46982-46176    Megaphage
from human gut metagenome Denmark fecal sample    DNA polymerase family B, C-terminal fragment
147-268    0    N-part of a split intein; part of a fractured-gene

SQTADTQLFIDNKEISMEEFFRTAKYENNDVVIKLQNGSEVVPVHNHNTLSYKDHDYKTIERPI
NYIMRHKVTKDKFRLKTKSGKELIVTGDHSIMVIRNNELVSLPAREIKNTDKIITLDK (SEQ ID NO:43)

HsaMP-3_PolB-C    HsaMP-3_PolB-C-2    ULXM01051442.1:44722-43748    Megaphage
from human gut metagenome Denmark fecal sample    DNA polymerase family B, C-terminal fragment
1-44    0    C-part of a split intein; part of a fractured-gene
MNYDIQIEDIDVIEQLEDFNDEYVYDIEVDDTHTFFANDILAHN (SEQ ID NO:44)

HsaMP-1_PolB-C    HsaMP-1_PolB-C-1    OASL01000056.1:4053-3214    Megaphage    from
human gut metagenome Bangladeshi cholera-succession    DNA polymerase family B, C-terminal
fragment    149-279    0    N-part of; part of a fractured-gene
SQLGSTQFRVDNNITTMEDFFIKAKYENNDVVIKLTNGSEIIPVHNHMTLSYKDHDYKTSERPI
NYIMRHKVTKDKFRLKTKSGKELIVTGDHSIMVIRDNELISLPAREIKKSDKIITIAGDNFTKAGDK    (SEQ
ID NO:45)

HsaMP-1_PolB-C    HsaMP-1_PolB-C-2    OASL01000056.1:1723-749    Megaphage    from
human gut metagenome Bangladeshi cholera-succession    DNA polymerase family B, C-terminal
fragment    1-44    0    C-part of; part of a fractured-gene
MNYDIQIEDIDVIEQLEDFNDEYVYDIEVDDTHTFFANDILAHN (SEQ ID NO:46)

OarG-3_PolB-C-1    NA - contiguous intein    ODAJ011734583.1:4477-6135    Sheep    gut
metagenome    DNA polymerase family B, C-terminal fragment 100-255    0
SIRGDSLLSINNKTISISNFFNYSEGSIKTNGDEKYIKHLSRDYFTTTYDDGNIIETKVNYVMKHK
TKKRMYKLKVVNKEIVVTEDHSIMIERDNNLIEGSVKDLHSNDKIIVYNKNAVIKSENWQVEDLGIIEDYV
YDIETENHMFFGNDILVHN (SEQ ID NO:47)

**Figure 29**

CP21-BP_dpol  CP21-BP_dpol-1    422935113:179333-177122    Campylobacter  phage  CP21
DNA polymerase family B    621-735    0    N' part; part of a fractured-gene
SVVGDSIIKVNGKNIKIEDFYDSIKVDPIVTKSGNNVKLVDNXFTESVNKNLQIETKKINYIMKHK
VKKEFFKIKVNNKEVVVTEDHSIMVLRNSELIEVKPRDIKNGDLIILND (SEQ ID NO:48)

CP21-BP_dpol  CP21-BP_dpol-2    422935113:176131-175225    Campylobacter  phage  CP21
DNA polymerase family B    1-39    0    C' part; part of a fractured-gene
MIVTENFQVESLGIQELDVYDIEVDSNHNFFANDILVHN (SEQ ID NO:49)

Nba-JCM10635_PolB-2NA - contiguous intein  448307387:195558-192703    Natronorubrum
bangense JCM 10635  DNA polymerase family B    349-693    0    includes  a  central
homing-endonuclease domain

SVPMNEPILIRDENGSIDIVEIQELDGRDGDVEVWTEKGFTRVKRVIRKPNRKKLYTIRTKKGV
VHATEDHSLVRADGSEVEPGELQEGESLLHRNVSDASTDVQTDLSLDRAWLYGFFXGDGSSGDYAY
DHPKNTDWDTRKTSWSLNNNNRELLQRAAVALSKEFGVNSRINETLESSGTYKLQPSNNGKRGAGS
NGMLPSLVKHFNETXYTPSRQKRVPQDVLNGDTQAIQAFLDGYMAADGHVGSRYSKRFHEADTRHQ
PLASGLVFLLQRIGYTFNINVRQVERDGGVTEYYKLRXQTSHRGDPNEVKKIVDYEYDGEYVYDLETE
NHHFHAGAGNIIVHN (SEQ ID NO:50)

SLM-T5-Br_PolB-1     SLM-T5-Br_PolB-1-1   LFIK01007014.1:4970-7102   Russia       Kulunda-
steppe soda lake Tanatar-5 brine environmental genomics     DNA polymerase family B     573-
710    0      N-part of ; part of a fractured-gene

SVVGNSVISVNGKKINIEDYYDRIDNNFIKNDQFNDDYVKVVDNGDTTQSINKDGKLENKPINYI
MKHRVRKEMFRIDDSSGNSVIVTEDHSVIVRDKKTKEILDVKPKELNPKKHEIINIIANDTDSGGIYGVDN
RK (SEQ ID NO:51)

SLM-T5-Br_PolB-1     SLM-T5-Br_PolB-1-2   LFIK01007014.1:8140-9029   Russia       Kulunda-
steppe soda lake Tanatar-5 brine environmental genomics     DNA polymerase family B     1-42
0      C-part of ; part of a fractured-gene

MSKIKFDDEFSVKSLGIVDNYVYDIEVEDNHNFFANNIXVHN (SEQ ID NO:52)

GOM-OPS-1_PolB-2   GOM-OPS-1_PolB-2-1   JQIA01000298.1:3216-5393   oil-polluted   sediment
collected from 3 locations (0.5 km, 0.7 km and 0.9 km) around wellhead MC252 after the Deepwater
Horizon oil spill DNA polymerase family B     605-723      0     N-part of ; part of a fractured-
gene

SVAFNSIIEIDGIKDTIESWFNKLAEEHGRHVDGEKEFTKISSLDLHTPTYDVAYDXMVDKPLMT
IYRHKIEKKMYTVTSVDGHSVTTTADHSLMVMRGGNIIEIIPTDILSGDQLVIFE (SEQ ID NO:53)

GOM-OPS-1_PolB-2   GOM-OPS-1_PolB-2-2   JQIA01000298.1:5441-6382   oil-polluted   sediment
collected from 3 locations (0.5 km, 0.7 km and 0.9 km) around wellhead MC252 after the Deepwater
Horizon oil spill DNA polymerase family B     1-46   0      C-part of; part of a fractured-gene

MHERKYKLVDIAKVEEVKYTDEYVYDVVMFEPESPYFVANDILVHN (SEQ ID NO:54)

GOM-OPS-1_PolB-1   GOM-OPS-1_PolB-1-1   JQIA01005173.1:1587-3635   oil-polluted   sediment
collected from 3 locations (0.5 km, 0.7 km and 0.9 km) around wellhead MC252 after the Deepwater
Horizon oil spill DNA polymerase family B     567-682      0     N-part of ; part of a fractured-
gene

SVVGDTSIYIDGNPIKIEDLYDKTQGELIERGDYDFIKKPNQNILXRAFDTKKQRVVEQKVNYIM
KHKVKKKMFKIKYKGKEVKVTQDHSVIIQREDLFISVTPEQIKKGDKIIII (SEQ ID NO:55)

GOM-OPS-1_PolB-1   GOM-OPS-1_PolB-1-2   JQIA01005173.1:4790-5719   oil-polluted   sediment
collected from 3 locations (0.5 km, 0.7 km and 0.9 km) around wellhead MC252 after the Deepwater
Horizon oil spill DNA polymerase family B     1-42   0      C-part of; part of a fractured-gene

MTSFELIEDFEVEXXGEEEIWVYDIEVEEHHNFFANDILVHN (SEQ ID NO:56)


ADi-DWV-1_PolB-C-2   NA - contiguous intein   MTKZ01078108.1:422-1250   Anaerobic   digester Denmark WWTP Viborg metagenome   DNA polymerase family B, C-terminal fragment 121-276

0

SVHSDSIVHTDKGSIKIEDWYNKNKTNGGTTLEGHESVLTNDKILNWDNELYFAPVKRIIRHKV TKPKWKIKTKSGKEIIITNDHSLIVFRNNEKLEIKPQGVIYQDKVLXLGTKFYFDEIESXEEMGTFDNEYV YDVEVDDDTHTFIANDILVHN (SEQ ID NO:57)


TOE-s022_PolB-1   TOE-s022_PolB-1-1   CENS01076648.1:2156-4198   Tara   Oceans expedition station TARA_022 Ionian sea (N=39.8386, E=17.4155) on 2009-11-16T08:16, depth of 3-7 m, size-fractionated (<-0.22 micrometres)   DNA polymerase family B   560-680   0

N-part   of;   part   of   a   fractured-gene

SVDGKSTVRANGKNVSIENLYSELESDGXNTIITDFTGRQFVFPKDVKLPYYNESNKKIENGN VEYIEKHRVKKKMFKIRSSNGKSVIITEDHSIMVMRNKKLIKITPDKLKKSDKLVTLL (SEQ ID NO:58)


TOE-s022_PolB-1   TOE-s022_PolB-1-2   CENS01076648.1:4206-5183   Tara   Oceans expedition station TARA_022 Ionian sea (N=39.8386, E=17.4155) on 2009-11-16T08:16, depth of 3-7 m, size-fractionated (<-0.22 micrometres)   DNA polymerase family B   1-42   0   C-part of; part of a fractured-gene   MKYKIEDIEEIEXLGEVDQDVYDIGMKDXPHTFFANDILVHN (SEQ ID NO:59)


ABR-I-C1_PolB-C-1   NA - contiguous intein   LAHS01013441.1:3269-1479   C-1   sludge   sample from hydrolytic chamber (first chamber) of Anaerobic Baffled Reactor in India   DNA   polymerase family B, C-terminal fragment   152-319   0

SXQNDTLIYVDNVKMSIEDVYIMMKEENFDSGIVTSNGSYIIPNRFGHTIKTYDEKTKKFIYKPIK YIMRHKVSKAKYKITTSSGKSVIVTGDHSIMILRNNKLQSIKAKDINIKTDKTISVIDNQLDXIIEDIASVEQL EDFNDEYVYDVEVEDTHTFVGNDILLHN (SEQ ID NO:60)


ADi-DWV-1_PolB-C-1   ADi-DWV-1_PolB-C-1-1   MTKZ01016736.1:2264-2884   Anaerobic digester Denmark WWTP Viborg metagenome   DNA polymerase family B, C-terminal fragment 91-206   0   N-part of; part of a fractured-gene

SVDGSTLIRTNIGILPIKILFDKFSKKSKIKTESYGHEMIDVLNLEXLTLKDNKIKMGKIKRLIRHKT NKKMYKIRVNGKEIITTEDHGIMVQRDGNLIRISPKEIKKGDLMVNVI (SEQ ID NO:61)


ADi-DWV-1_PolB-C-1   ADi-DWV-1_PolB-C-1-2   MTKZ01016736.1:2960-3922   Anaerobic digester Denmark WWTP Viborg metagenome   DNA polymerase family B, C-terminal fragment 1-43

0   C-part of; part of a fractured-gen

e   MKYELSPIESIEVVNDRFDYVYDIEMDDTTDHVFFGNDILVHN (SEQ ID NO:62)

BtaR-1_PolB-C-1    BtaR-1_PolB-C-1-1    JGI-rumenHiSeq_NODE_3645493:120705-121763
Switchgrass-associated bovine rumen microbial communities from Urbana, Illinois, USA DNA polymerase family B, C-terminal fragment    225-352    0    N-part of; part of a fractured-gene

SVSADSNIKISYSDTGSKKDIKVXDLFTELKYNNNDXVIITGNGSEVVPVKDIXAQTYSTEKDKV
IFRPVNYIMRHKVKKSRFRITTESGKQIIVTGDHSXMVVRDNELISVKAKDIKMSDKIITVDN    (SEQ    ID
NO:63)

BtaR-1_PolB-C-1    BtaR-1_PolB-C-1-2    JGI-rumenHiSeq_NODE_3645493:122161-123159
Switchgrass-associated bovine rumen microbial communities from Urbana, Illinois, USA DNA polymerase family B, C-terminal fragment    1-49    0    C-part of; part of a fractured-gene
MVMDFKETNYKIESIAAIEQIEDFDEEYVYDLEIDDTHMFFANDILVHN (SEQ ID NO:64)

RnoDR-G_PolB-C-3    RnoDR-G_PolB-C-3-1    NFZD01014548.1:37176-38243    Rattus
norvegicus (Denmark: Riget) gut metagenome    DNA polymerase family B, C-terminal fragment 227-
355    0    N-part of ; part of a fractured-gene
SVAGDTKVDISSADIKKRIDIADLFTKAKYLNDDHVLSVSNGSEVIPGNGILIRAYDKELDMAVY
KPMKYVMRHKVSKARFRIKTESGKEVIVTGDHSXIVLRNGELIDIKAKDINKETDKIITINSKK    (SEQ    ID
NO:65)

RnoDR-G_PolB-C-3    RnoDR-G_PolB-C-3-2    NFZD01014548.1:38767-39753 Rattus    norvegicus
(Denmark: Riget) gut metagenome    DNA polymerase family B, C-terminal fragment 1-47    0
C-part of ; part of a fractured-gene
MDFKEKNYKIESIAEIEQLDDFEDEYVYDVEVDDXHNFFANDVLVHN (SEQ ID NO:66)

Cco-NC_C3306_PolB  Cco-NC_C3306_PolB-1AACNZN010000082:1-1671    Campylobacter    coli
strain NC_C3306    DNA polymerase family B    442-556    0    N-part of a split intein;
part of a fractured-gene
SVVGDSIIKVNGENIKIEDFYDSIKVDPIVTKSGNNVKLVDNXFTESVNKNLQIETKKINYIMKHR
VKKEFFKIKVNNKEVIVTEDHSIMILRNSELIEVKPRDIKTGDSIILNV (SEQ ID NO:67)

Cco-NC_C3306_PolB  Cco-NC_C3306_PolB-2AACNZN010000082:2662-3305
Campylobacter coli strain NC_C3306    DNA polymerase family B    1-39    0    C-part
of a split intein; part of a fractured-gene MIVTENFQVESLGIQELDVYDIEVDSNHNFFANDILVHN (SEQ
ID NO:68)

Cje-SO-54_PolB    NA - contiguous intein  WP_126208120.1    Campylobacter    jejuni    SO-54
DNA polymerase family B    621-773    0
SVVGDSIIKVNGKNIKIEDFYDSIKVDPIVTKSGNNVKLVDNXFTESVNKNLQIETKKINYIMKHK
VKKEFFKIKVNNKEVVVTEDHSIMVLRNSELIEVKPRDIKIDDILILIDSKSSDFQVESLGIQELDVYDIEVD
SNHNFFANDILVHN (SEQ ID NO:69)

Cje-CFSAN038801_PolB        Cje-CFSAN038801_PolB-1        EAI5067605.1  Campylobacter  jejuni CFSAN038801 DNA polymerase family B        621-735        0        N-part of a split intein; part of a fractured-gene

SVVGDSIIKVNGENIKIEDFYDSIKVDPIVTKSGNNVKLVDNXFTESVNKNLQIETKKINYIMKHR VKKEFFKIKVNNKEVIVTEDHSIMVLRNSELIEVKPRDIKTGDSIILND (SEQ ID NO:70)


Cje-CFSAN038801_PolB        Cje-CFSAN038801_PolB-2        AABPWQ010000013.1:4182-5087

Campylobacter jejuni CFSAN038801        DNA polymerase family B        1-39   0        C-part of a split intein; part of a fractured-gene MIVTENFQVESLGIQELDVYDIEVDSNHNFFANDILVHN (SEQ ID NO:71)


Cco-CFSAN038779_PolB-1        NA - contiguous intein   EAK3475363.1 Campylobacter                  coli CFSAN038801 DNA polymerase family B        621-773        0

SVVGDSIIKVNGENIKIEDFYDSIKVDPIVTKSGNNVKLVDNXFTESVNKNLQIETKKINYIMKHR VKKEFFKIKVNNKEVIVTEDHSIMVLRNSELIEVKPRDIKIDDILILIDSKSSDFQVESLGIQELDVYDIEVD SNHNFFANDILVHN (SEQ ID NO:72)


Rba-NORP22_PolB-1   NA - contiguous intein   PCJ96739.1        Rhizobiales bacterium NORP22DNA polymerase family B        581-742        0

SVAGDSLIYVNDKKIKIEDYYNSLENNFILNDTFNENYVKEVSGDTTKXYIEGIKNKKINYIMKHK VSKKMYRIKXNGNFVDVTEDHSVIVKNKKTKKISSIKPKNLNPNLHSIINIKTKKIDELITDDFEVEYLGIIE NYVYDIEVEEAHNFFANDILVHN (SEQ ID NO:73)


HsaG-1_PolB-C-6        HsaG-1_PolB-C-6-1        ULOB01050215.1:1-1060        Human                  gut metagenome Denmark fecal sample        DNA polymerase family B, C-terminal fragment 235-353        0        N-part of a split intein; part of a fractured-gene

SLVGSSIIIVNGKKIKIEDYYNQXNGILIKNDINNQNFIKEIDNDDKGLSYDINNQQIVNNKIKYIKK HKVKKEFFKISYKDKEVIITEDHSVMIERNDKIIEIKPREIKQGDKIIXIQ (SEQ ID NO:74)


HsaG-1_PolB-C-6        HsaG-1_PolB-C-6-2        ULOB01050215.1:2137-3132        Human                  gut metagenome Denmark fecal sample        DNA polymerase family B, C-terminal fragment 1-41   0        C-part of a split intein; part of a fractured-gene MQIQKTTDFKIESLGIQEQYVYDIEIEDTHNFFANTILVHN (SEQ ID NO:75)


HsaG-1_PolB-C-3        HsaG-1_PolB-C-3-1        OJAW01011162.1:507-1229        Human                  gut metagenome Peru National Reserve Matses (Amazon) fecal sample        DNA polymerase family B, C-terminal fragment        122-240        0        N-part of a split intein; part of a fractured-gene

SVVGDTIINVNGKPITIADYYNSIVPNYIKXDDINKNYIKRVDNGDVALAYDNGIVQNKIKHVMKH TVKKRMYKIKXNGKEVIMTEDHSIIVNRNGKNISVSPKDILKGDRLIXLNNYK (SEQ ID NO:76)

HsaG-1_PolB-C-3    HsaG-1_PolB-C-3-2    OJAW01011162.1:2302-2542   Human         gut metagenome Peru National Reserve Matses (Amazon) fecal sample    DNA polymerase family B, C-terminal fragment    1-46    0    C-part of a split intein; part of a fractured-gene

MXKIYTEXNVVDDFEVEDLGVQELDVYDIEVEEXHNFFANDILVHN (SEQ ID NO:77)

HsaG-1_PolB-C-4    HsaG-1_PolB-C-4-1    OGOZ01002969.1:6093-3997   Human         gut metagenome spanish infant fecal sample    DNA polymerase family B, C-terminal fragment   580-698    0    N-part of a split intein; part of a fractured-gene

SVVGDTIINVNGKPITIADYYNSIAPNYIKXDDINKNYIKRVDNGDVALAYDNGIVQNKIKHVMKH TVKKRMFKIKXNGKEVIMTEDHSIIVNRNGKNISVSPKDILKGDRLIXLNNYK (SEQ ID NO:78)

HsaG-1_PolB-C-4    HsaG-1_PolB-C-4-2    OGOZ01002969.1:2924-1947   Human         gut metagenome spanish infant fecal sample    DNA polymerase family B, C-terminal fragment   1-46    0    C-part of a split intein; part of a fractured-gene

MXKIYTEXNVVDDFEIEDLGVQELDVYDIEVEEXHNFFANGILVHN (SEQ ID NO:79)

HsaMP-2_PolB-C    HsaMP-2_PolB-C-1    ULUU01077929.1:15136-15951        Megaphage from human gut metagenome Denmark fecal sample    DNA polymerase family B, C-terminal fragment   150-271    0    N-part of a split intein; part of a fractured-gene

SQIGSTQFXVDNNITTMEDFFTKAKYENNDVVIKLQNGSEVVPVHNHNTLSYKDHDYKTIERPI NYIMRHKVTKDKFRLKTKSGKELIVTGDHSIMVIRNNELVSLPAREIKKSDKIITLDK (SEQ ID NO:80)

HsaMP-2_PolB-C    HsaMP-2_PolB-C-2    ULUU01077929.1:18537-19511        Megaphage from human gut metagenome Denmark fecal sample    DNA polymerase family B, C-terminal fragment   1-44    0    C-part of a split intein; part of a fractured-gene

MNYNIQVENIDVIEQLEDFNDEYVYDIEVDDTHTFFANEILAHN (SEQ ID NO:81)

HsaG-1_PolB-C-2    HsaG-1_PolB-C-2-1    ULXR01025649.1:8852-9694   Human         gut metagenome Denmark fecal sample    DNA polymerase family B, C-terminal fragment   17-280   0    N-part of a split intein; part of a fractured-gene but could be a contiguous intein due to sequencing or assembly errors

SIVASTMIYGDTFAGTIEELFKKSAEGRVLKQTLNGTLMTESDVKXLNWSESKGLHYAPIVYIS KHLVKKNMWKLRGKSGKEVIVTEDHSLIVFRDGKKLEVKPKEILPTDKILIVFPLKQRIALIIKARERGYSL SSQEVFDKMRPIVEEMGYTXKYATHGGEHVVITLDKSYFLDFYIPELKIGIEYNGGMFHGDPRLYKDDE YXNPWNINETAKDMRERDQQRYDFLLNNYGIKTYIIWELDYNQGLDVESFIRRILNESK    (SEQ    ID NO:82)

HsaG-1_PolB-C-2    HsaG-1_PolB-C-2-2    ULXR01025649.1:9681-10377   Human         gut metagenome Denmark fecal sample    DNA polymerase family B, C-terminal fragment   1-47    0    C-part of a split intein; part of a fractured-gene but could be a contiguous intein due to

sequencing or assembly errors  MRVSEFEYQFDEIESIEXLGETNEYVYDIEVADESHTFIANDILVHN (SEQ ID NO:83)

HsarG-1_PolB-C-1-2    NA - contiguous intein   OIWQ01000167.1:3413-5644    Human            gut metagenome Denmark Roux-en-Y gastric bypass surgery of morbidly obese patient      DNA polymerase family B, C-terminal fragment      295-464        0

SISYDSIIKTSRHPNGITISEWYKENENNIGERTLAGHESVHTDDKALNFDDDKLTFTNVKRIIR HKVSKPKWKLRTSSGKEIVXTDNHSLIVFRNGTKKKVKPSEITXEDKVLTVNLSTSIEENIRYVQSFDNV EIXVNIGEYTDEYVYDIEMDDDSHTFIANDILVHN (SEQ ID NO:84)

HsaG-1_PolB-1 HsaG-1_PolB-1-1      OBSA01000002.1:155656-157794        Human           gut metagenome Bangladeshi cholera-succession   DNA polymerase family B      701-712       0

N-part of ; part of a fractured-gene      SAAFSTKIMIKR (SEQ ID NO:85)

HsaG-1_PolB-1 HsaG-1_PolB-1-2      OBSA01000002.1:159819-161132        Human           gut metagenome Bangladeshi cholera-succession   DNA polymerase family B      1-154  0      C-part of ; part of a fractured-gene

MKQDIEIGRLFDELLESGLELKTRNGYEYMEPKGIKILTGGNRYVWLVAISRHRTPKHLVRISIT TSXSGKYDVTVTTDHVXMVMNRDHFFDNVNAKNLEIGDYVQVYDAGYGKEVLGAISKIEDLGPTDDY VYDXEVDDNGHTFYGNSVLLHN (SEQ ID NO:86)

SscG-1_PolB-2 SscG-1_PolB-2-1      OBLI01010075.1:5126-3370     Pig gut metagenome    DNA polymerase family B     572-585        0       N-part of ; part of a fractured-gene

SAIYSTKLRIRIKD (SEQ ID NO:87)

SscG-1_PolB-2 SscG-1_PolB-2-2      OBLI01010075.1:839-1 Pig gut metagenome     DNA polymerase family B     1-172   0       C-part of ; part of a fractured-gene

MEKQEEIGALYDKLVAEGRKIVNDGKYELVDAKGIEVLSYGGKTDNGRYAPIKYVSRHKTSKN LVEITVTIGSDAVDGGGLASXQKKRLEKKVVVTTDHIXMKYDRDRVFQNVDAKNLTPGDYVSVYDSGY GMETYGIVSAIKNLGPTXEYVYDLEVDDTHXFYANDVLIHN (SEQ ID NO:88)

BtaR-2_PolB-1 BtaR-2_PolB-1-1      REGX01355543.1:4679-5086 Bovine        gut        rumen metagenome    DNA polymerase family B      20-135 0        N-part of ; part of a fractured-gene

SVVDSKIRXMSGEKMLSDIFNESKNQYKTPFITSHGAELYPXDERVLNYDDTGLHYTRIKYISR HKVNKPMWRLRTKSGKEILXTEDHSLVVYRDGKKKSIKPDKILPTDKIVTIK (SEQ ID NO:89)

BtaR-2_PolB-1 BtaR-2_PolB-1-2      REGX01355543.1:6274-6551 Bovine        gut        rumen metagenome    DNA polymerase family B      1-43    0       C-part of ; part of a fractured-gene

MEIIFDDIETIECVSDGWDDYVYDIEVEDDSHTFIANDILVHN (SEQ ID NO:90)

SscG-1_PolB-1 NA - contiguous intein    OBLI01000041.1:22778-26380  Pig gut metagenome    DNA polymerase family B    601-823    0

      SXEKSTFINISDNVISIETNSGSKVYNKNDIVKVNRNGIEMEIYASDINENDLIWEESIKKIEKTNK ITIEQLYNLGTQDMGSTLAGHESVNXDHDVLNIKQNDLSYSGNGVDFTSYYAPVKRVIRHKVTKAKWS LVDSDNNEVIVTNDHSLMVLRDGNLQKIKPYDVDVENDFLVTISDGNIEIRDIVRXEQIGNFEDEYVYDI EMNDDTHTFVANNILVHN (SEQ ID NO:91)

OarG-1_PolB-C-32    OarG-1_PolB-C-32-1

      (ODAI010985757.1+ODAJ012589656.1+OCTW012151930.1+ODAI011286909.1):382-1476 Sheep gut metagenome    DNA polymerase family B, C-terminal fragment 224-364

      0    N-part of; part of a fractured-gene

      SVSXDTIIYWGFAGSAEQSTTIEEMFNIIKEQNMDTVLNLENGSEVVPTSELTAIRTYXPILNHV TQKPVKYVMRHKVKKARYRITTSSGKQVIVTGDHSXMVLRDGKLTAVKANEINPNTDKIISDKHYRPKI NEEKDEG (SEQ ID NO:92)

OarG-1_PolB-C-32    OarG-1_PolB-C-32-2

      (ODAI010985757.1+ODAJ012589656.1+OCTW012151930.1+ODAI011286909.1):1896-2813 Sheep gut metagenome    DNA polymerase family B, C-terminal fragment 1-75    0

      C-part of; part of a fractured-gene

      MEAQKQNNNNEYIDLDIDEFNPVVGDVADTDGSDTYEVEDIVSIEQLDDFDDEYVYDVEVEDT HTFFANDILVHN (SEQ ID NO:93)

OarG-4_PolB-1 NA - contiguous intein    OCVV010470507.1:1-639    Sheep    gut    metagenome DNA polymerase family B    7-174    0

      STFSKXLLLINRNNKNVQITIEDLFNESLKQNGIKDITNNGHEIVKXNDDVLNWTEKNGLNFVPI KWIMRHKVSKPMFKITTKSGKTITVTEDHSXVIFRNGEQIVIKAKDINKETDKILSVINENETYQFEEIENI EQVEYKDDYVYDIEVDDNSHTFIGNDILVHN (SEQ ID NO:94)

OarG-3_PolB-C-2    NA - contiguous intein    ODAJ012187655.1:168833-170599    Sheep    gut metagenome    DNA polymerase family B, C-terminal fragment 147-312    0

      SXAGNSIIELNGRKMSIENAFSYLKEENDHIVLRTSNGSEVVPVENTTTKTYDSLSKKIVDRDV KYIMRHKVSKPKWKLTTSSGKMIEVTGDHSLMVMRDGELLSVKAXEVDPKKDKIVTYINTQEYIIEDIES IEQVEDFNDEYVYDIEVDDTHTFFANDILVHN (SEQ ID NO:95)

OarG-2_PolB-C-2    NA - contiguous intein    ODAI012454976.1:1-1724    Sheep    gut metagenome    DNA polymerase family B, C-terminal fragment 133-298    0

      SXAGNSIIELNGRKMSIENAFSYLKEENDHIVLRTSNGSEVVPVENTTTKTYDSLSKKIVDRDV KYIMRHKVSKPKWKLTTSSGKMIEVTGDHSLMVMRDGELLSVKAXEVDPKKDKIVTYINTQEYIIEDIES IEQVEDFNDEYVYDIEVDDTHTFFANDILVHN (SEQ ID NO:96)

OarG-2_PolB-C-1     OarG-2_PolB-C-1-1     ODAI013718692.1:3463-2396   Sheep          gut
metagenome   DNA polymerase family B, C-terminal fragment 227-355       0     N-part of ; part
of a fractured-gene

SVAGDTKVDISSADIKKRIDISELFTKAKYLNDDHVLSVSNGSEVIPGNGILIRAYDKDLDMAVY
KPMKYVMRHKVSKARFRIKTESGKEVIVTGDHSXIVLRDGELIDIKAKDINKETDKIITINSKK   (SEQ   ID
NO:97)

OarG-2_PolB-C-1     OarG-2_PolB-C-1-2     ODAI013718692.1:1872-886    Sheep          gut
metagenome   DNA polymerase family B, C-terminal fragment 1-47    0     C-part of;  part  of  a
fractured-gene   MDFKEKNYKIESIAEIEQLDDFEDEYVYDVEVDDXHNFFANDVLVHN (SEQ ID NO:98)

OarG-1_PolB-C-1-2     NA - contiguous intein   AUXO014566809.1:20814-23309         Sheep     gut
metagenome   DNA polymerase family B, C-terminal fragment 310-475        0

SVSGDTVVVTKNHPNGISIERLYGENAEYAVEFGDNHESVLTGDMVLNYYDNELYVAPVSRII
RHKVTKDKYLLKAFNSGNAVEXTSDHSLVVYRQNGDEGKYVSAVVKPYEIRNGDLVVTXKNDSYTFE
EATXEKIGEYNDEYVYDIEMDDLTSTFVANGILVHN (SEQ ID NO:99)

OarG-1_PolB-9 OarG-1_PolB-9-1     (AUXO016437101.1+AUXO013713007.1):3338-2613
          Sheep gut metagenome          DNA polymerase family B     228-241     0     N-part
of ; part of a fractured-gene      SSVFDTYIDVIEED (SEQ ID NO:100)

OarG-1_PolB-9 OarG-1_PolB-9-2     (AUXO016437101.1+AUXO013713007.1):684-1
          Sheep gut metagenome          DNA polymerase family B     1-159  0     C-part of;  part
of a fractured-gene

MKLYLERLSMTSVKIGELYNKYLAKGYEIVNTPTGHELIYPKSLKVRSIGNEFKKVKXLSRHRT
SKPLVRIXFQKSEPLVVTTDHVXMAYNDDRMLENIASKDLRVGMMVDHYXRTSDKEVIDVITNIEPLGT
TDDYVYDLEVEDESHVFYANDTLIHN (SEQ ID NO:101)

OarG-1_PolB-C-5     OarG-1_PolB-C-5-1     AUXO014167152.1:1579-2622   Sheep          gut
metagenome   DNA polymerase family B, C-terminal fragment 224-347        0     N-part of;  part
of a fractured-gene

SIDGNSVIDINDEKIAIKDAFAAIKYMNIDTVIMLPNGTQVVPAPSDITLTTKTYDASTDTVXDKQI
RYIMRHKVKKSKYKITTESGKEVIVTGDHSXMVVRDGILISVTAQEINPETDKIITI (SEQ ID NO:102)

OarG-1_PolB-C-5     OarG-1_PolB-C-5-2     AUXO014167152.1:3057-4058   Sheep          gut
metagenome   DNA polymerase family B, C-terminal fragment 1-51    0     C-part of;  part  of  a
fractured-gene   MLKIMDFSQTNYKVENIKSIEVLPDFDDEYVYDIEVGETHMFFANEILVHN   (SEQ   ID
NO:103)

OarG-1_PolB-C-4     NA - contiguous intein   AUXO011823854.1:4538-6346   Sheep          gut
metagenome   DNA polymerase family B, C-terminal fragment 136-306        0

SVSKDTIIRTRLHPDGIMIEDFYDENSSNKGEDTRAGHESVHTSDQVLNFNGSILTNTSNLYYG
NVKRIIRHKVSKPKWTITTRFGHSVSVTNDHSLMVLRDDKLYKVKPSEVKPGDEAMSVEVMYGDIVEG
ADKITSXVHTGEYNDEYVYDIEMDDDNHTFFANDILVHN (SEQ ID NO:104)


OarG-1_PolB-C-3          NA - contiguous intein   AUXO013129674.1:5689-3923  Sheep          gut
metagenome    DNA polymerase family B, C-terminal fragment 147-312         0
     SXAGNSIIELNGRKMSIENAFSYLKEENDHIVLRTSNGSEVVPVENTTTKTYDSLSKKIVDRDV
KYIMRHKVSKPKWKLTTSSGKMIEVTGDHSLMVMRDGELLSVKAXEVDPKKDKIVTYINTQEYIIEDIES
IEQVEDFNDEYVYDIEVDDTHTFFANDILVHN (SEQ ID NO:105)


OarG-1_PolB-C-6          OarG-1_PolB-C-6-1     AUXO012051433.1:3548-2948  Sheep          gut
metagenome    DNA polymerase family B, C-terminal fragment 77-200 0         N-part  of;  part  of  a
fractured-gene
     SIDGNSVIDINDNRIAIRDAFAAIKQMNQDTVIVLPNGTQVVPAPSDVELTTKTYDANTDTIXDM
PIKYIMRHKVRKAKYKITTESGKQVIVTGDHSXMVIRDGVLISVTAQEINPETDKIITI (SEQ ID NO:106)


OarG-1_PolB-C-6          OarG-1_PolB-C-6-2     AUXO012051433.1:2564-1575  Sheep          gut
metagenome    DNA polymerase family B, C-terminal fragment 1-47    0         C-part  of;  part  of  a
fractured-gene   MEFXQNNYKVENIKSIEVLPDFEDEDVYDLEVGGTHMFFANDILVHN         (SEQ    ID
NO:107)


OarG-1_PolB-10          OarG-1_PolB-10-1     AUXO018717682.1:3436-5775  Sheep          gut
metagenome    DNA polymerase family B         652-779         0         N-part of ; part of a fractured-
gene
     SLAKKSLLLIKDTKNIKNKITIEDLFNQSLDKNGLSDITQNNQEIVKXDQQXLNWTKENGLQYVP
IKYIMRHKVSKEQFKIKTKSGKEIIVTGDHSXIVFRNGKQLTIKARDINKSTDKILSIINNEE         (SEQ    ID
NO:108)


OarG-1_PolB-10          OarG-1_PolB-10-2     AUXO018717682.1:7018-7986  Sheep          gut
metagenome    DNA polymerase family B         1-44    0         C-part of ; part of a fractured-gene
     MLEYQFEEIESIEQLDNFNDEYVYDIEVDDNSHTFIANDILVHN (SEQ ID NO:109)


OarG-1_PolB-24          NA - contiguous intein   AUXO011893528.1:1-1257         Sheep          gut
metagenome    DNA polymerase family B         21-189 0
     STFXKSLLLIKDNKNIENKVTIESLFNKSLMDNGLTDLTQNNQEIVKXDYSTLNWTEEKGLEYV
PIKYIMRHKVSKQQFKIKTKSGKEIIVTGDHSXIVFRNGEETVVKAKDINKDTDKILSIITFNKYQIENIKSIE
QLEDFDNEYVYDIEVDDDSHTFIANDILVHN (SEQ ID NO:110)


OarG-1_PolB-16          OarG-1_PolB-16-1
     (AUXO013913591.1+AUXO012578971.1+AUXO015923351.1):1-1890  Sheep          gut

metagenome    DNA polymerase family B       615-629       0       N-part of ; part of a fractured-gene    SVHGKTHVFIRSIKN (SEQ ID NO:111)

OarG-1_PolB-16       OarG-1_PolB-16-2

(AUXO013913591.1+AUXO012578971.1+AUXO015923351.1):4443-5801       Sheep       gut metagenome    DNA polymerase family B       1-168  0       C-part of ; part of a fractured-gene

MQEAKIDIKSLYDSLAKKYDVQHKNSYEVIYPKGYEIKVLGNKYVKLVAMSRHKTQKHLVKIVV KSEKTIDSLDPIRQKSLLKKQDEVVVTTDHIXMVYNDDHFFENVNAKNLKVGNYVSVYDEASDKEVIGE IASIEDLGMTDDYVYDXEVDDDSHAFYASNILVHN (SEQ ID NO:112)

OarG-1_PolB-C-10       NA       -       contiguous       intein

(AUXO014339501.1+AUXO010790200.1+AUXO015877878.1):1-1476 Sheep       gut metagenome    DNA polymerase family B, C-terminal fragment 26-196 0

SXNFDTLIRTKNYPDGITIEDFYNINSENKGDTTLVGHESVYTTDKVLNFKGNQLTNTSQLYYG DVKRIIRHKVSKPKWVITTRFGHSVTVTNDHSMMVLRDDKLYMVKPXEIHPGDDVLSLEVMYGDFVEG SDKVTSXLHVGEYEDEYVYDIEMGDDTHTFFANDILVHN (SEQ ID NO:113)

OarG-1_PolB-C-9       OarG-1_PolB-C-9-1-2

(AUXO016687107.1+AUXO010673474.1+AUXO011796439.1+AUXO010528631.1):7354-6239    Sheep gut metagenome       DNA polymerase family B, C-terminal fragment 298-371       0       N-part of ; part of a fractured-gene

SVSKDTIIRTKKHVDGITIEDFYEENSEKDVFEIKTESGKIIRYNKNDKVKVKRSDKIIYVYPDDIL YEDEIFT (SEQ ID NO:114)

OarG-1_PolB-C-9       OarG-1_PolB-C-9-2-1

(AUXO016687107.1+AUXO010673474.1+AUXO011796439.1+AUXO010528631.1):3978-2614    Sheep gut metagenome       DNA polymerase family B, C-terminal fragment 1-159   0       C-part of ; part of a fractured-gene

MSQFEKIVSIRKIRSSNKGESTLAGHESVYTDDKVLNFKGSILTNISQLYYGSVKRIIRHKVSKP KWTITTRFGHSVTVTNDHSMMVLRDDKLYKVKPXEIHPDDYVLSLEAMYGDIVEGSDKVVSXLXTGEY DNEYVYDIEMDDDTHTFFANDILVHN (SEQ ID NO:115)

OarG-1_PolB-12       NA - contiguous intein   (AUXO012589886.1+AUXO016354513.1):1-621 Sheep gut metagenome       DNA polymerase family B       13-182 0

SLAKNSLILIEDNKNTKDKIYIESLFNKALRDNGLEDISQNNQEIVKXDDYNVLNWTKENGLQYV PIKYIMRHKVSKPKFKIKTKSGKEIIVTGDHSXXVFRNXXQLXIKAKDINKDTDKILSIIYDMKYIIEEIESVE QLDNFNDEYVYDIEVDDNSHTFIANDILVHN (SEQ ID NO:116)

LCGC14_PolB-2       LCGC14_PolB-2-1       816675767:3840-2573 Marine sediment metagenome LCGC14       DNA polymerase family B       303-422       0       N-part of ; part of a fractured-gene

SVDADTIIKTNYGEMTIENLFKSXSIKGPSWAIDDQEFTIYDQIQILTYDPKTNEEIYRPFEYVYR HKVSKPRWKIIDENGNEIILTNDHSVMIERDGKLIEAKPSEINPDTDILITIGE (SEQ ID NO:117)

LCGC14_PolB-2    LCGC14_PolB-2-2    816675767:1-873    Marine sediment metagenome LCGC14    DNA polymerase family B    1-42    0    C-part of ; part of a fractured-gene
MVEKLKIQKIEKLEDFDNEYVYDISVDKETPYFFGNNILVHN (SEQ ID NO:118)

wtfStanf-1_dpol-1    NA - contiguous intein    FSSH01001841.1:9196-3704    Stanford USA drinking water system tap filter metagenome genome assembly    DNA polymerase family B    756-1224    0    includes    a    central    homing-endonuclease    domain
SFSSITPLLIHDDHGQIDIVQAKDLVKPNDPAWMSKEDFWKNYNPDPVSTTKASSTSISTTKTG RVSKATSSNSTTKPSGTSNTIAIEPTSTRGSRSRTSRSSNSXDIASXSNDNTIPQSXSSETPDTSQFSG STQQYMDIRDKNYNIWSEKGWTQIKYIMRHKTGKQMYRINTHTGVIDVTEDHSLLDIKGDPVTPNEVKI GSELLHHDLPNVVNEHQNVIIDADTAWLYGFFYAEGTXGTYQYKKGPRSSWSISNQDLAPMNKALEIL QRIEPNYKFKIDNXMKSSAXHKLSARNGSTSKNXPREYHVSSLVEKYHNMFHADSGQQRHNIKSNSY TALYYKKVPKEILNASNDIKKSFYDGYYVGDGLKATTANEVFDNKGQIGAAGLYYIASALGYDVSLYLR EDKPQIFRXTLSKDTKXVKSVQRKNRNAIKKIIPLGTXDDYVYDIETENHHFAAGIGRMVVHN (SEQ ID NO:119)

LHPMM_PolB-C-1    LHPMM_PolB-C-1-1    JFJP01052642.1:4882-3929    Lake    Huron    low oxygen high sulfur sink hole purple microbial mat bin unclassified.02    DNA polymerase family B, C-terminal fragment    200-317    0    N-part of a split intein; part of a fractured-gene
SVDFQTLMKTEKDEITIEELYNRNIINGSAGITLNGHESVKXTDIILNYSKSKGLYFNNVRRIIRH KVSKEKWXLKTTNGKXIYITNDHSLIVFRDGEKLEIKPKDVLKIDKVLTIKK (SEQ ID NO:120)

LHPMM_PolB-C-1    LHPMM_PolB-C-1-2    JFJP01052642.1:1415-330    Lake    Huron    low oxygen high sulfur sink hole purple microbial mat bin unclassified.02    DNA polymerase family B, C-terminal fragment    1-52    0    C-part of a split intein; part of a fractured-gene
MMEFEYELVDIESXELVGIFDDEYVYDIEVEEDENDVEDTHTFFGNDILVHN (SEQ ID NO:121)

RnoMa-G_PolB-2    RnoMa-G_PolB-2-1    NFYZ01003970.1:33126-35465 Rattus    norvegicus (Malaysia) gut metagenome    DNA polymerase family B    655-779    0    N-part of ; part of a fractured-gene
SSSSNSNIYIKRGNQVLKRTFIELWRDTVEEHMPFGLGTHGQELXXSDDYVLNYDEKRGLHW VPIKYIMKHGTKKRKFRVKTKSGKEIIVTEDHSXVVIRNGEKIAVKASEINKDTDKIVSISTK    (SEQ    ID NO:122)

RnoMa-G_PolB-2    RnoMa-G_PolB-2-2    NFYZ01003970.1:36822-37829 Rattus    norvegicus (Malaysia) gut metagenome    DNA polymerase family B    1-49    0    C-part of ; part of a fractured-gene
MIEEGFDYIIEDIESVEDIGFFDEDEPVFDIEVEDDTHTFIGNDVLVHN (SEQ ID NO:123)

RnoDR-G_PolB-1    RnoDR-G_PolB-1-1    NFZD01004553.1:987-3326    Rattus    norvegicus (Denmark: Riget) gut metagenome    DNA polymerase family B    655-779    0    N-part of ; part of a fractured-gene

SSSSNSNLYIKRGNQVLKRTFIELWRDTVEEHMPFGLGTHGQELXXSDDYVLNYDEKRGLH WVPIKYIMKHGTKKRKFRVKTKSGKEIIVTEDHSXVVIRNGEKIAVKASEINKDTDKIVSISTK (SEQ ID NO:124)


RnoDR-G_PolB-1    RnoDR-G_PolB-1-2    NFZD01004553.1:4683-5690    Rattus    norvegicus (Denmark: Riget) gut metagenome    DNA polymerase family B    1-49    0    C-part of ; part of a fractured-gene

MIEEGFDYIIEEIESVEDIGFFDEDEPVFDIEVEDDTHTFIGNDVLVHN (SEQ ID NO:125)


**Figure 30**

Figure 31

OarG-1_PolB-2 AUXO013038135.1:28487-26978          Sheep gut metagenome          306-503
N-part of a split intein

SFTEDTPVFVRNVKNGAIDIKPIXELIDENKIETDALGREYDYSPKPYQVLXRSGWVTPSYIYRH
KTDKDIYEITDGDMKIEVTEDHSLFNDKKEKIKPSEVNNETHLEYFNDYEVFKKAKWLPADTRNPHFYA
KALANGKIDRVPSWFLNRPTKEGREFYEVFIKNYRDDIQYSKTXLAGLYFLKMISEVSTYSGIK (SEQ ID
NO:126)


OarG-1_PolB-2 AUXO013038135.1:25101-23533          Sheep gut metagenome          1-34     C-part
of a split intein

MLEKTNKGKTSAYVYDISLDGTVVNALGMNVNSN (SEQ ID NO:127)


OarG-1_PolB-1 AUXO018743865.1:62464-59969          Sheep gut metagenome          637-831
N-part of a split intein

SFTPDTPMFIKYKDSGLIDIKPIEELINEKEIKIDALGREYDYSKKDYYVLXRSGWVEPSYIYRHK
TEKDIYEITDGEMKVEVTEDHSLFNSKQEKIKPSEITNKTELEYYTEEVRPDGYTRYFATQAPQAKQMA
MDLANGKIDRVPMKVLNYQEVYQKIFYDTFIENYKNDIKYSKTXLAGLQYIRKMLMEKKF          (SEQ      ID
NO:128)


OarG-1_PolB-1 AUXO018743865.1:57259-55673          Sheep gut metagenome          1-33     C-part
of a split intein   MKILNKGKXLDYVYDISLDGTVVNALGMNILSN (SEQ ID NO:129)


BtaR-1_PolB-1 JGI-rumenHiSeq_NODE_2986932_len_400505_cov_5_502381:65549-63084     Cow
rumen metagenome          634-821          N-part of a split intein

SVTEDTPLFVRKNGMIDIKPIGELFGSENIESYPDGREYDRNEKDYEVLXRSGWVRPSYIYRH
NTDKDVYKVTGDGIEVDATRDHSLFDADRNEIKPTEISRGTKLETYSGDSLFDFNTIELSDFAIDVAAML
VMTGKTDRIPDEILNATLEXKAKFIDLLKNAEVTTDRYPKTLVAGYQYIKNXVLL (SEQ ID NO:130)


BtaR-1_PolB-1 JGI-rumenHiSeq_NODE_2986932_len_400505_cov_5_502381:57941-56256     Cow
rumen metagenome          1-39     C-part of a split intein

MQNNIIMQAIKLKSEKRTVYDISXDGTFVNALGMNVLHN (SEQ ID NO:131)


Psp-CAG1092-contig445_PolB 524136850:6860-9403 Prevotella species CAG:1092 phage contig
445     660-847          N-part of a split intein

SFTGDTPVFIKYDNTNLIDIKPISELIDIDNIDKDVLGREYDTSEKNYSILXRSGWYKPSYIYRHK
TVKNIYRVEDNTPSAGXIXDITEDHSLFNDEREKIKPSEIGQNTKLEYKSKIFXRRTHTISDDKFNKLLDF
TVKFPIKIPIEILNXEINTRKRFAYELHKRLKDSIDIGHYSKVFVAGFKFL (SEQ ID NO:132)


Psp-CAG1092-contig445_PolB 524136850:13149-14813          Prevotella species CAG:1092 phage
contig 445     1-34     C-part of a split intein

MIQVENIGKTDDYVYDISLDGTVVNALGMNVLSN (SEQ ID NO:133)

LHPMM_PolB-2        JFJP01040793.1:4720-7185    Lake Huron low oxygen high sulfur sink hole purple microbial mat bin unclassified.01 623-821        N-part of a split intein

SFLYDTPIYIKYKNSDIIDIKSIGGVFNSNESDIDELGREYDLSEKPYQVLXRGGWIDVNYVYRH KTDKQIHRISFNDGYVDVTADHSVFNENKEKLHSKDVIPNETKLEMANLDYSNFIIKQQNLNVETIEKMA SVLALSVDINKKVPAEIINTNKENQIIFLNKFMSVTKLNKVSQDNENKVLKAGILFLVNRTKNN (SEQ ID NO:134)


LHPMM_PolB-2        JFJP01040793.1:9434-10951   Lake Huron low oxygen high sulfur sink hole purple microbial mat bin unclassified.01 1-40     C-part of a split intein

MNNLVLGNEIIDNYDPEMYVYDIXLDGTLINALGNNVVTQ (SEQ ID NO:135)


OarG-1_PolB-7 AUXO011944917.1:1-978        Sheep gut metagenome        140-325        N-part of a split intein

SFTSDTPVFIKYDKSGLIDIKAISELIGETEVDGLGREYDYSEKDYTVLXRSGWVKPKYIYRHKT DKDIYRVEDNGAMIDVTEDHSLFNDKQEKIKPSEINDETKLEYYKDEITPEGTMKWLNETRARRLAKWI KDGTLTEVPLPILNSMNRGHINAFLDELGNWDYSKSSKVLQAGIMYVKNKRR (SEQ ID NO:136)


OarG-1_PolB-7 AUXO011944917.1:2554-2996 Sheep gut metagenome        1-34   C-part of a split intein        MAQATKINKTDDYVYDISLDGTVVNALGLNVISN (SEQ ID NO:137)


OarG-1_PolB-6 AUXO010688891.1:1-1746        Sheep gut metagenome        396-580        N-part of a split intein

SFTEDTPLFIKYNNSGLIDIKPISELVNEDKIEFDGLGREYDYSKKDFKVLXRSGWVEPSYIYRH KTTKPIYRISDDEVKMSIDVTEDHSLFNEKQEKIKPSEINSDTKLEYYNNNISNNQIVIDEIRIDKYSKLLS NGRLNAIPIDLLNATVEXKKEFLSKMDLSKVSEXKTLIAGILYLRSXI (SEQ ID NO:138)


OarG-1_PolB-6 AUXO010688891.1:3762-4942 Sheep gut metagenome        1-36   C-part of a split intein        MLNSKKIKGKEEVGVVYDISLDGTVVNALGMNVISN (SEQ ID NO:139)


OarG-1_PolB-5 AUXO012445152.1:6078-3610 Sheep gut metagenome        628-822        N-part of a split intein

SFTGDTPLFIKYNDGKIDIKPIEELIGETETDALGREYDYSKKPYKVLXRSGWVRPSYIYRHKTN KPLYTVSEGNMSVTVTEDHSLFNDKQKKIKPSEITEGTRLEYYTDKVETSTKGFIHLNEQRVKIMAKTL KNGVINRVPIQLFNTNNIDAVKTFLNELEGWDYSNTSKTXRAGIQFLKKKIDGFNFHDVYK (SEQ ID NO:140)


OarG-1_PolB-5 AUXO012445152.1:3428-1845 Sheep gut metagenome        1-38   C-part of a split intein        MNNINIKKMEDXQDIGVVYDISLDGTVVNALGMNVISN (SEQ ID NO:141)

OarG-1_PolB-3-1    AUXO013707648.1:1-1419    Sheep gut metagenome    280-472
     N-part of a split intein

SFTADTPLFIKYDDGKIDIKPIXELIGETETDKLGREYDYSPKPYRVLXRSGWMRPSYIYRHKTN
KPLYEVSEGNMSITVTEDHSLFNDKQHKIKPSEITENTKLEYYKKSIETDTKYRWLTEDRARRMSKMVL
DGTIDRVPMAILNTENLKIVMAFLKEWEYMPLXTFSKTXQAGINFLKMKLYGQNRNIHK     (SEQ    ID
NO:142)


OarG-1_PolB-3-1    AUXO013707648.1:2295-2734 Sheep gut metagenome    1-33    C-part
of a split intein   MKIKNIGSTSDYVYDIQLDGTVVNALGMNIISN (SEQ ID NO:143)


VidaL_PolB-2    JGI_03-Dec-2012-
(LV_Brine_h2_0102DRAFT_1000263+JGIcombinedJ13537_10026142):17312-19555    Antarctica
Vida lake environmental sampling Brine-Hole-2, 0.1-0.2 micron filter    627-747    N-part    of    a
split intein

SVTGDTLITLSDKKISIEDLFNRFDYRVIQDQGKEYAIPRTELEKENNAVLGYNAFEDEAVLGEI
AYVMRHKTKKKLYEIETDDGKKVTVTEDHSLIVDRHGITTEVTPKNLEEDDLIITI (SEQ ID NO:144)


VidaL_PolB-2    JGI_03-Dec-2012-
(LV_Brine_h2_0102DRAFT_1000263+JGIcombinedJ13537_10026142):19585-20571    Antarctica
Vida lake environmental sampling Brine-Hole-2, 0.1-0.2 micron filter    1-42    C-part of a split intein
     MNTIRTKVKRVTXLGEVDDYVYDVSMVRQDPFFFANDILVHN (SEQ ID NO:145)


OarG-1_PolB-8 AUXO011039760.1:1-594    Sheep gut metagenome    11-197    N-part
of a split intein   partial - C-part of this split intein is unknown

SFTGDTPLFIKYDNGIIDIVPISDLIGDTEMDDLGREYDVSDKPYKVLXRSGWVKPEYIYRHKTK
KPLYEVSDGDMTVMVTEDHSLFNNKQEKIKPSEINEKTKLEYYGHKIESSYEYGWLNEKRALRMAKW
LKDGTLNQVPTPLLNTKKINXIKVFLGELQGFDFNNATKTXRAGILFLKEKLKNK (SEQ ID NO:146)


OarG-1_PolB-33    (AUXO011846531.1+ODAJ010669297.1+AUXO011042125.1):1-705
     Sheep gut metagenome    111-234    N-part of a split intein    partial - C-part of this
split intein is unknown

SVTEDSLIRTGSGNIXVKDIWDKYSKEIPEVFEYSNNFKTAYLKMNDKEYILNPVMTILGSDDRL
YIPRYIMKHKTKKKLYKITTESGKQVTVTEDHSLIVVRDNVKTVIKPTELLDTDEVIVI (SEQ ID NO:147)


OarG-1_PolB-C-7    AUXO012591702.1:862-1    Sheep gut metagenome    1-43    C-part
of a split intein   partial - N-part of this split intein is unknown
     MINMYLEHIKSVEVLEPTEDLDVYDLELPVDHXFFANDILVHN (SEQ ID NO:148)


OarG-3_PolB-3 ODAJ012335527.1:66-259    Sheep gut metagenome    1-53    C-part of    a
split intein    partial - N-part of this split intein is unknown
     MPVIXLKEIIKLYKVSNIKSIEVITPEEPIDVYDIEMPDDDHXFFANDILVHN (SEQ ID NO:149)

OarG-1_PolB-C-43    (AUXO015293883.1+AUXO017997485.1):222-1    Sheep    gut
metagenome    1-47    C-part of a split intein    partial - N-part of this split intein is unknown    intein
might be overextended by 6 aa

MGGEWEMKIESVKSIEVVTPEEPIDVYDIEMPDDDHXFFANDILVHN (SEQ ID NO:150)


OarG-1_PolB-C-36    (ODAJ010922718.1+AUXO014068786.1):242-931    Sheep    gut
metagenome    1-50    C-part of a split intein    partial - N-part of this split intein is unknown    intein
might be overextended by 7 or 10 aa

MLKYILEVVSMNLSKIKSVEVITPEEPTEVYDIELPVDHXFYANDILVHN (SEQ ID NO:151)


VidaL_PolB-5    JGI_03-Dec-2012-LV_Brine_h2_0102DRAFT_1121654:209-1    Antarctica    Vida    lake
environmental sampling Brine-Hole-2, 0.1-0.2 micron filter    1-42    C-part of a split intein    partial
- N-part of this split intein is unknown

MDTNRAKVKSVKXLGEVDDYVYDVSMKDQDPFFFANGILVHN (SEQ ID NO:152)


Nsp-J07AB43_PolB    339758521:40642-43520    Nanosalina species J07AB43    583-960
partial - missing C-part

SLNYSRQVVVKNPNNEIQFMEVGKFVEDIDVPENYETLAWDEEKDRSVFKPVKRAIMHKYNG
NLLRFDTSRGRTEVTPQHSVYRYEDGEIKLADAEDLEEGDRLVSLSELPETEKKYSEGDTIDLADLEYE
NSDLMAYRDKKKFPAEKGEXPYXGEVYYLSSHVHRDHQDRRIALGEASQDYSYIGXKNAKAGKIPRF
WKLTSELAWILGFYXGDGSASLGDKQMVSFGGQNKENIRRVKRFFDQILDEELSIIEDVDSRTGGKMY
YYRIQRIPVVXLFVNGLGAGSGSDGKKVPSMIINGDKGLRKAFVEGYFDADGSRDKDYDDRYDSENM
RFSTKSSYLANQVQYILKQLNLGENRYVEISAMSLSSIVKINLRSX (SEQ ID NO:153)


MamaV-Hal-V_dpol    351737110:419460-424682    Acanthamoeba castellanii mamavirus Hal-V
1053-1403

SVTGDTPIITRHQNGDINITTIEELGSKWKPYEIFKAHEKNSNRKFKQQSQYPTDSEVWTAKG
WAKIKRVIRHKTVKKIYRVLTHTGXIDVTEDHSLLDPNQNIIKPINXQIGTELLHGFPESNNVYDNISEQE
AYVWGFFMGDGSXGSYQTKNGIKYSWALNNQDLDVLNKXKKYLEETENIQFKILDTMKSSSVYKLVPI
RKIKYMVNKYRKIFYDNKKYKLVPKEILNSTKDIKNSFLEGYYAADGSRKETENMGXRRXDIKGKISAQ
XLFYLLKSLGYNVSINIRSDKNQIYRLTFSNKKQRKNPIAIKKIQLMNETSNDHDGDYVYDLETESGSFH
AGVGEMIVKN (SEQ ID NO:154)


LentilleV-lvs_dpol    398257015:212733-217955    Acanthamoeba polyphaga lentillevirus    1053-
1403

SVTGDTPIITRHQNGDINITTIEELGSKWKPYEIFKAHEKNSNRKFKQQSQYPTDSEVWTAKG
WAKIKRVIRHKTVKKIYRVLTHTGXIDVTEDHSLLDPNQNIIKPINXQIGTELLHGFPESNNVYDNISEQE
AYVWGFFMGDGSXGSYQTKNGIKYSWALNNQDLDVLNKXKKYLEETENIQFKILDTMKSSSVYKLVPI
RKIKYMVNKYRKIFYDNKKYKLVPKEILNSTKDIKNSFLEGYYAADGSRKETENMGXRRXDIKGKISAQ

XLFYLLKSLGYNVSINIRSDKNQIYRLTFSNKKQRKNPIAIKKIQLMNETSNDHDGDYVYDLETESGSFH
AGVGEMIVKN (SEQ ID NO:155)

MimiV_pol        55416941        Mimivirus Rowbotham-Bradford        1053-1403
        SVTGDTPIITRHQNGDINITTIEELGSKWKPYEIFKAHEKNSNRKFKQQSQYPTDSEVWTAKG
WAKIKRVIRHKTVKKIYRVLTHTGXIDVTEDHSLLDPNQNIIKPINXQIGTELLHGFPESNNVYDNISEQE
AYVWGFFMGDGSXGSYQTKNGIKYSWALNNQDLDVLNKXKKYLEETENIQFKILDTMKSSSVYKLVPI
RKIKYMVNKYRKIFYDNKKYKLVPKEILNSTKDIKNSFLEGYYAADGSRKETENMGXRRXDIKGKISAQ
XLFYLLKSLGYNVSINIRSDKNQIYRLTFSNKKQRKNPIAIKKIQLMNETSNDHDGDYVYDLETESGSFH
AGVGEMIVKN (SEQ ID NO:156)

MarseilleV-Montpellier_dpol        392056052:1-4527        Marseilleviridae Montpellier MTP3        893-
1174
        SVTGDTPIMIKDKNNNINIVTIKELGEKWKPYDIFKSHEINSNRKYKQQADFNGEVWTSNGWA
KIKRVIRHKTVKKLYRVLTNTGXIDVTEDHSLLDTNKNIIKPIDXKIGTELLHGFPEINNNHNKLSLEIYKEL
DITSELFDXMIESNKKWNNEKMKAXFIGSEYRKQNKNISNEILNXSKKIKKYFLLGYLGNDKEYITNNKIN
AQMVYYLMKXLGYNIVIDLIESSYKLXIVDNINKPYXINKIIQLQDTSINGEYVYDLETESGTFHAGIGELIV
KN (SEQ ID NO:157)

Mch-ECIM_PolB        350610932:672845-667947        Megavirus chiliensis        1017-1298
        SVTGDTPIMIKDKNNNINIVTIKELGEKWKPYDIFKSHEINSNRKYKQQADFNGEVWTSNGWA
KIKRVIRHKTVKKLYRVLTNTGXIDVTEDHSLLDTNKNIIKPIDXKIGTELLHGFPEINNNHNKLSLEIYKEL
DITSELFDXMIESNKKWNNEKMKAYFIGSEYRKQNKNISNEILNXSKKIKKYFLLGYLGNDKEYITNNKIN
AQIIYXLMKXLEYNIVIDLIESSYKLXIIDNINEPYXINKIIQLQDTSINGEYVYDLETESGTFHAGIGELIVKN
(SEQ ID NO:158)

MegaVCourdo11_dpol  425700872:655497-650602        Megavirus courdo11    1017-1298
        SVTGDTPIMIKDKNNNINIVTIKELGEKWKPYDIFKSHEINSNRKYKQQADFNGEVWTSNGWA
KIKRVIRHKTVKKLYRVLTNTGXIDVTEDHSLLDTNKNIIKPIDXKIGTELLHGFPEINNNHNKLSLEIYKEL
DITSELFDXMIESNKKWNNEKMKAXFIGSEYRKQNKNISNEILNXSKKIKKYFLLGYLGNDKEYITNNKIN
AQMVYYLMKXLGYNIVIDLIESSYKLXIVDNINKPYXINKIIQLQDTSINGEYVYDLETESGTFHAGIGELIV
KN (SEQ ID NO:159)

MegaVCourdo7-Mv13-c7_dpol  371943560:373473-368575        Megavirus courdo7        1017-1298
        SVTGDTPIMIKDKNNNINIVTIKELGEKWKPYDIFKSHEINSNRKYKQQADFNGEVWTSNGWA
KIKRVIRHKTVKKLYRVLTNTGXIDVTEDHSLLDTNKNIIKPIDXKIGTELLHGFPEINNNHNKLSLEIYKEL
DITSELFDXMIESNKKWNNEKMKAXFIGSEYRKQNKNISNEILNXSKKIKKYFLLGYLGNDKEYITNNKIN
AQMVYYLMKXLGYNIVIDLIESSYKLXIVDNINKPYXINKIIQLQDTSINGEYVYDLETESGTFHAGIGELIV
KN (SEQ ID NO:160)

MegaVterra1_dpol    392056062:1-4527    Megavirus terra1 TE1  893-1174

SVTGDTPIMIKDKNNNINIVTIKELGEKWKPYDIFKSHEINSNRKYKQQADFNGEVWTSNGWA
KIKRVIRHKTVKKLYRVLTNTGXIDVTEDHSLLDTNKNIIKPIDXKIGTELLHGFPEINNNHNKLSLEIYKEL
DITSELFDXMIESNKKWNNEKMKAXFIGSEYRKQNKNISNEILNXSKKIKKYFLLGYLGNDKEYITNNKIN
AQMVYYLMKXLGYNIVIDLIESSYKLXIVDNINKPYXINKIIQLQDTSINGEYVYDLETESGTFHAGIGELIV
KN (SEQ ID NO:161)

MoumouV-Mv13-mv_dpol    371944806:44421-49229    Moumouvirus Monve    1003-1267

SVTGDTPIIVKLPNSNDVEIKTIQELTTFWYEYDAFKAGDSNRKDKQQAILDYEVWTDKGWAKI
KRVIRHQTKKSIYRVKTNNGVVDVTEDHSLLNTDKEIIKPLDXNPNTKLLHGFMETNNIYQNITPSQAYL
LGLNFGKVDVYWDIINAQSIWDVINIITNATTKIKQEFIKGWKKQGFYNIENKVEAQFLYYVLKSLGHNV
NIHMPITKEDIYRLSYGKNISNINKTTIQYLRETYDGEYVYDLETESGTFHAGIGEMIVKN    (SEQ    ID
NO:162)

OtV1_dpol    NC_013288:169003-172845    Ostreococcus tauri virus 1    636-958

SVTPDTPLLIRENGEVKTTRIDSLVDLYEVRDDGKEIAEIDAEVWTEXGFTPIKQIVRHKTTKNI
HRVLTHTGIVDVTEDHSLLLKNKEMIKPSEVXLGTELLHGNSLEAFGETHTDVTPEEAKVMGFFFGDG
SXGHYDGKYTWALNNADMTFLEEMSELXPFETRVYDTIQSSGVYKLNAVGDVKSISVRYRSLFYNEH
KEKVVPPXILGAPLHIVQSFWDGYYMADGDKDVHGYTRMDIKGKEGSMGMYILGRRLGYNVSMNTR
TDKPDIFRQTWTTSSQRKNPIAIKKLELLGETEGYVYDLTTGSHHFHVGPGDLVVHN (SEQ ID NO:163)

KBvp-11_dpol  EU889364:1-1573    unknown phycodnavirus KBvp-11    154-470

SVTPDTPLLIRQDGIVKTXRIDSLVNAYEVRDDGKEVATIDAEVWTEKGFTPIHQIVRHKTTKRI
HRVLTHTGVVDVTEDHSLLLEDAKMITPKEVQLGTKLLHGSXVNAIIDGTSRVSVNEAKVMGFFFGDG
SXGAYNGKYTWTLNNANIQYLDKMASLXPFETRIYATMESSGVYKLNAIGDVKTISLRYRSLFYNAAKE
KVIPPXILNAPEEVVKAFVEGYYMADGDTRMDIKGKEGSMGMFILGKRLGYNVSINTRSDKPDIYRQT
WTTYSQRKEPXAIKKLEFLEETDGYVYDLTTESHHFHVGPGELVVHN (SEQ ID NO:164)

VidaL_PolB-3  JGI_03-Dec-2012-JGIcombinedJ13537_10002447:7477-3158  Antarctica  Vida  lake
environmental sampling Brine-Hole-2, 0.1-0.2 micron filter    586-944

SVTHDTPIYIKWKDSNKLDILPISDIFNEDSEVLDEESLRDLEIKPYEVLTVNGWKEINYVYRHE
TNKKIHRISTKDKLVXVTEDHSLFQNGKQIKPKNLKRGDVLDVRELPTFELDDDNNLNEDLFYLYGYFL
GDGSATYGNRKQYYKSKKTSKTNINKGKRSVFKISSSNYDKLIRLQKIIKDNFDVNXKIKDHRESSNVY
NLIXYVKKMSVKFSEDFYTSYKEKKIPNYVLNTNKKNKLAFLEGVFSSDGYGDTLEEVSDIGMKSQVA
MSGISYIMETLSIDREIKVRKDKENFISLKLKNRNRSNSKFANKIKMKSDEVWLNEVITNKXEKNYVYDIS
TEDGTFIGGIGGVDLKN (SEQ ID NO:165)

BBAY15_dpol-3    CAMERA-1106850859302:3-1006    Botany  bay  environmental  sample
BBAY15 library    18-313

SVTAETPVLVKSPDGVVQYIQISELGNKWDKXVEDGKEEKEFXSLEGKGWEAWSDDGWTPI
KNVIRHELAPHKKVFRVLTHTGYVEVTDDHSLLNNEKGIIKTNEIKNGQLLLQNEYIELDSKINTISEDEA

RIMGFFFGDGSXGTYNXKSGKKSSWALNNNNLIRLKKYQNLFYKNKNKVIPDXIFKSNKNVRKAFFEG
LYDADGDKXGYXTRIDQKSMISXAYIYKLGKSLDYNVSINVHSKKKNIFRLTFTTKKQRKIVNAVKKLMK
SNNGYVYDLTTESSLSSRIGSIIVHN (SEQ ID NO:166)


CEV_pol     EU006632:1-4872     Chrysochromulina ericina virus isolate 01     965-1335
          SVASYTPIYVRYNKSIIDIXSVEELAEKYGNGWHLESPKEYXELNNIESWTENGWTEXHRVIRH
RLAPYKKMVRILTHTGLVDVTDDHSLVKNTGEEISPKDVSIGTKLLHXTMSENESNIESDISIDEARIMGF
FFGDGSXGIYDXPSGHKASWALNNSNKELIEKYYNLXKSVYPEFEWKVYDTLNSSGVYKIXFNKKSGS
KSKIQFIEKYRSMLYNKKSKIIPSEIINGSIELRKSFWEGLYDADGDKDKNGYTRIDQKSQISAAYIXWLA
NSIGYKTSLNIRDDKTDIYRITATKNKQRRDGDKIKKIVNIQNSANIQNSANIQNSVNIQNSVNIQNSKDN
QDYVYDLTTENHHHFAAGIGNMIVHN (SEQ ID NO:167)


AceL-1_dpol-2  TDB-Contig178:28-1395     Antarctica Ace lake environmental sampling     24-
371
          SVAKYTPVYVKVDGKLQIVEMEKLAEQYGGNQWTTXLEEGKQEKEFXELYGVETWTDKGWT
KLHRIIRHQLASHKKMIRILTHTGMVDVTDDHSLVLEDGNEISPKEVDIGTKLLHKTLEYESPQFEVENNI
SADMAKIYGFFFGDGSXGIYDXPSGKKASWALNNANEELLDKYIKLXRKSYPEFDWQIYDTIESSGVY
KLTFNGNVYGNKSKFIETYRKHMYSGKSKIIPDFILNGTHEIREAFWEGLYDADGDKDKNGYVRIDQKN
QISAAHIXWLANSIGYKTSINTRTDKQNIYRITATKGAQRKEGNAIKKLMELDYHDYVYDLTTENHHFAA
GIGNMIVHN (SEQ ID NO:168)


AceL-1_dpol-1  TDB-262.18+262.19:706-5703  Antarctica Ace lake environmental sampling     987-
1275    partial - missing C-part
          SVAKYTPVYVRANGQLQIVEMEQLAEKYGGNNWSKXIEEGKQEKEFXELTNIETWTNKGWT
KLYRIIRHKLASHKKMVRILTHTGMVDVTDDHSLLLEDGSEVSPKNVEIGTKLLHKTLKHDALKPNAXLD
TVSVDMAKIYGFFFGDGSXGIYNXPSGKKATWALNNSNVELLDKYINLXKKXYPEFTWQTYNTIESSG
LYKITFNSNEYGTKSRFIETYRNXMYTGNSKIIPDFILNGSNEIREAFWEGMYDAAADGDKDENGYVRI
DQKNQISAAHIXWLANSMDIRX (SEQ ID NO:169)


BpV2_dpol     312599144:162736-166443     Bathycoccus species RCC1105 virus BpV2     623-
950
          SVTPDTPLLIRQNGTVHTXRIDSLVNEYTLRDDGKEIGYINAEVWTENGFTSIQQIVRHKTNKNI
HRVVTHTGIVDVTEDHSLLLENKEIAKPTQVGVGTALLHGNXVESIDTXTDTSITKEEAKVMGFFFGDG
SXGTYQXRSGVKSTWALNNSKLEYLEEMQKLXPFETKIYDTIKSSGVYKLNAKGLVVDIVNKYRNLFY
NSHKEKVVPSXILNAPLEIIKSFVDGYYMADGDKDKNGYTRMDVKGKEGSMGMYMLGRKLGYNVSIN
TRTDKVNVFRQTWTKSLQRKSPTKIKKLEXLGETDGYVYDLTTKSHHFHVGPGDLVVHN     (SEQ     ID
NO:170)


KBvp-16_dpol  EU889369:1-1606     unknown phycodnavirus KBvp-16     159-481
          SVTPDTPLLLRIKGEVKTXRIDSLVESYEERDDGKEVAEIDAEVWTEKGFTPIQQIVRHKTTKNI
HRVLTHTGVVDVTEDHSLLLENKQMIKPXEVSLGTNLLHGDXVYGLNWNDTTVSVNEAKVMGFFFGD

GSXGHYGDKYTWALNNSNVDYLIEMQNLXPFETSIYDTIESSGVYKLNAKGDVKNIXERYRSMFYNAH
KEKIVPSXILNAPIEVVESFWEGYYMADGDKDVHGYTRMDIKGKEGSMGMFILGKRLNYNVSLNTRKD
KPDVFRQTWTKSTQRKSPNAIKKLELVGETEGYVYDLTTESHHFHIGPGDLVVHN (SEQ ID NO:171)

Hal-JCM10717_PolB    448596614:267100-263093    Haloferax alexandrinus JCM 10717    626-
1062

    SVTGDRPVVVRDPGGTVRILPIEDLFARGTTESEVLIAADGDVVASATPGKTRRALDGWDALS
VNEAGEAEWQPIAQAIRHKTDKPVVNLQHKFGESTTTRDHSYVVPGEHGLTTVSPDDVAEPYRVSGV
PDVEPVEQVDVYEVLRGYEREYEDGRSVGSDNSITKRKQIHADDEYVWFGHEHHRDVDSTVKVKRF
VDIDSEDGAALIRLLGAYVSEGSASTGETATSKFGASIAESDREWLAQLQRDYSRLFENTTADIITSDR
RAERTVEYQTDTGGASVTYNDETLKLQMMNELAAVFFREFAGQTSRGKRIPSFVFHLPEEKQDLFLTL
LVEGDGSREFPRYTEAYAQRNFDFETTSRELAAGLSMLLTQRGQKHSLKYRDSKDSYTIRTXSTYRE
GRDPVLTEVDHDGYVYDLSVEENENFVDGVGGIVLHN (SEQ ID NO:172)

Hpr-DSM18310_PolB    448560872:391142-395149    Haloferax prahovense DSM 18310    626-
1062

    SVTGDRPVVVRDPGGTVRILPIEDLFARGTTESEVLIAADGDVVASATPGKTRRALDGWDALS
VNEDGEAEWQPIAQAIRHNTDKPVVNLQHKFGESTTTRDHSYVVPGEDGLTTVSPDDVAEPYRVSGV
PDVEPVEQVDVYEVLRGYEREYEDGRSVGSDNSITKRKQIHADDEYVWFGHEHHRDVDSTVKVKRF
VDIDSEDGAALIRLLGAYVPEGSASTGETATSEFGASIAESDREWLAQLQRDYSRLFENTTAGIITSDR
RAERTVEYQTDTGGASVTYNDETLKLQMMNELAAVFFREFAGQTSRGKRIPSFVFHLPEEKQDLFLTL
LVEGDGSREFPRYTEAYAQRNFDFETTSRELAAGLSMLLTQRGQKHSLKYRDSKDSYTIRTXSTYRE
GRDPVLTEADHDGYVYDLSVEENENFVDGVGGIVLHN (SEQ ID NO:173)

Nmo-8.8.11_PolB    452205862:671617-667487    Natronomonas moolapensis 8.8.11    663-
1099

    SVSGDRPVVVRDPDGTIRIVPIEALFDRAEKRPDDRVFVAADGDTQIDDGSRKEFADLDGWD
ALSLSEEGTAGWRPIERVIRHRTDRPVVNLQHKFGESTTTRNHSYIVDDNSKYVEATPEEVDEPLRIPD
LPAETRNDAIDVYEVLSGYEREYEDGRGTGGTTIKTKRVHANDEYVWFGHDHYGELDSTVKVKRYIR
PGTQEXVSLXRLLAAYVTEGSATTKETSDXRYGASIAESRRRWLVGLRDDYYRLFENTTASVIDNDSS
DERTIEYRTDHGDTAVSYDDGTKKLQMMNELAAVFFREFAGQTSREKRLPSFVYHLQDDEQDLFLET
LIEGDGSREFPRYSDAYAERNFDFETISRELAAGLSMLLIQRGKKHSLKYRDAKDSYTIRTVDSYRRGR
DPVLREVDHDGYVYDLSVAENDNFVDGVGGVVLHN (SEQ ID NO:174)

Hvo_PolB1    TIGR_26May06-chr:2847757-1017569    Haloferax volcanii DS2 ATCC29605    626-
1062

    SVTGDRPVVVRDPGGTVRILPIEDLFARGTTESEVLIAADGDVVASATPGKTRRALDGWDALS
VNEDGEAEWQPIAQAIRHNTDKPVVNLQHKFGESTTTRDHSYVVPGEDGLTTVSPDDVAEPYRVSGV
PDVEPVEQVDVYEVLRGYEREYEDGRSVGSDNSITKRKQIHADDEYVWFGHEHHRDVDSTVKVKRF
VDIDSEDGAALIRLLGAYVPEGSASTGETATSKFGASLAESDREWLAQLQRDYSRLFENTTAGIITSDR
RAERTVEYQTDTGGASVTYNDETLKLQMMNELAAVFFREFAGQTSRGKRIPSFVFHLPEEKQDLFLTL

LVEGDGSREFPRYTEAYAQRNFDFETTSRELAAGLSMLLTQRGQKHSLKYRDSKDSYTIRTXSTYRE
GRDPVLTEADHDGYVYDLSVEENENFVDGVGGIVLHN (SEQ ID NO:175)


Hwa_PolB-3    109624723:852239-858667    Haloquadratum walsbyi DSM 16790    1438-1868

SVTGDRPVVVRDPSDYIQIVPIKLLFEQATAPEQNMRLTADGAPSVNSELPKERRHLDQWEAL
SLSDTGETEWQPINQIIRHQTDKEILTLQHEYGESTTTRDHSYITADDGEYVETSPENVDEPLPIPNIAS
VKTIETIDIYQTLTTDTQAQIGNDTEPDKWLPSADXIHANDEYVWIGTTDKQQDRDDSTPAIPRYIDLTS
DTGHALIRFLAVYLSDWSKSTITTTERGQXLHITGPQESALKTXAADADQLFTHITPSIAVDAESNTNTV
DSGFRXHIPTTLATTLISAFAGHPAHTKQIPSIVYHLPAAEQSLFIRHLIQAESTPESDGVSGRPQKSDK
PILLENEFITTNRELAAGVSMLLTQXGQSYTISKQDTKGAYTIHINNSSSSGXTPTLTETTHSGYVYDLS
VATNQNFVDGLGGLVLHN (SEQ ID NO:176)


Hwa-C23_PolB-3    334353260:872414-878803    Haloquadratum walsbyi DSM 16854    1425-
1855

SVTGDRPVVVRDPSDYIQIVPIKLLFEQATAPEQNIRLTADGAPSGNSEFPKERRHLDQWEAL
SLSDTGETEWQPIDQIIRHQTDKEILTLQHEYGESTTTRDHSYITADDGGYVETSPENVDEPLPIPNIAP
VKTVETIDIYQTLTTDTQAQIGSDTEPDKWLPSADXIHANDEYVWIDTTDKQQHRDDSIPTIPRYIDLSS
DTGHALIRFLAVYLSDWSESTITTTERGRXLHITGPQESALKTXAADADQLFTHITPSITVDAESNTNTV
DSGFTXHIPTTLATALISAFAGHPAHTKQIPSIVYHLPAAEQSLFIRHLIQAESTPESDGVSGRPQKSNK
PILLENEFITTNRELAAGVSMLLTQXGQSYTISKQDTKGAYTIHINNSSPSGXTPTLTETTHSGYVYDLS
VATNQNFVDGLGGLVLHN (SEQ ID NO:177)


Hca-DSM19288_PolB-2    448494697:74523-69175    Halorubrum californiensis DSM 19288
1066-1504

SVTGGRPVVVRDPEGIVRILPIADLFERSDATASEDLIVTADGGPVASVSIDKERRRAGGWEA
LSVTEDGEPEWQPIEQVIRHETDKSVVNLQHKFGESTTTRDHSYVVEEDGQLVETKPEDVEEPLRVP
GLPEVETVEKIDVYDVLEGYTREYEDGRSVGSENAETKVKRVHANDEWVWFGHKHHNAIERSIKVQR
YVDLDSEDGRALVRLLAAYVTEGSASTIETTDSRFGASIAESRTEWLEGLREDYQRLFDGATASVIASD
ASDRRTVEYGTEDGDQSVTYDDGTHKLQMMNELSAVFFREFAGQTSRGKRIPGSVFNLPEDLQQLF
VDVLVEGDGSREFPRYSTEYXERNFDFETTSRELAAGLSTLLTQRGEKHSLKYRESKGSYTIRTXDYY
RSGRDPVVEEVDHDGYVYDLSVAENENFVDGVGGVVLHN (SEQ ID NO:178)


CroV-BV-PW1_dpol    310830989:548497-553314    Cafeteria roenbergensis virus BV-PW1  800-
1146

SVIGDTPLLLKNKFTNEILINKIKDLSSNWSNYHNGKESXEIDTYQTWTETGWTDIKRVIRHKLE
SNKKLLKIQTHNGEVIVTDEHSLLNKNGKTINAKNVKVGDNILHSFPSYINNIDNTNSINYHNKFYNKKM
XNELAYILGXFMKYGLXDSSKKXFTINNKDINLIESLKKMXENIFDEFKWKISSSSHLSDNIYKLVPFQNE
IKLIDFIKYFTNKMYNNGEKKVPQXILNSSKEYIKIFLIGLYPEYKLENNQQFIYTXKNNEFSLGIYYLIKKL
GYHVKLNSNDSSDSSDSIYTFEISHKLENNNNVITKITEWEHKETYVYDLTTENHHFHAGVGSIIVHN
(SEQ ID NO:179)

KBvp-2_dpol    EU889355:1-1441    unknown phycodnavirus KBvp-2        170-426

SVKGDTPLLLKTEHGVFFQSIDELFKISKSIETGLRLAKEYANIENHNIYVWSDVGFTKIRRVMR
HYTTKGMFRVTTKTGYVDVTEDHSLLLENGFEVRPSDTTVGTRLLHKKPTINKYIQKSFSSEHLSEAK
MMGESFLDEETIPSFVLNSPVNVLRKYFEGXIKAXGVIKNDTNIEFHFSKSKQGVAEFVFVAQQLGYHV
FIKPYGVHXSLDPQDLKIQEITAIEYMGKTKDYVYDLETDNHHFHVGPGNLIVHN (SEQ ID NO:180)


HAV_pol        70568331      Heterosigma akashiwo virus HaV01      647-878

SVTKETPLMLRTMETXGNHKHEVISIENVFTDNMRSIDMYSIIGEKEHVMLSRNEEIWTGENW
SRIIRVIRHKTQKKIYGVLTENGYVEVTEDHSLISSDYELLKPKNXIVKETQLLQSFPDIVENSTIENNMIDI
PKGQPXRLTVFGQVSAMIIYTYLKRKNYSITLNVXNVNSNKFYISFMERPRFKNTKKNIIKKIFFIRNTDN
EEYVYDVETEDGIFHAGIGEIIVKN (SEQ ID NO:181)


Asp-GW2011-AR16_PolB        735016386      Archaeon GW2011_AR16        592-977

SVGKDTEIVMNENGTIRFVKISELFERTQKRTSDGKEYFFPPSRLVLTLDAQGKSVFKKVKYV
MKHRVQKKMYRIFFTNDHYIDVTEDHSLIGYVNKQKNNQLADLDRLIEVKPTDIGKRVRTIITIKNIPRSSI
KTRNYHRELYEFMGLFIGDGSFDRQKKQNYYLHLAGGLDSWEIITKVLVPLKEKEYIKNYWLKKKGDIX
INGLRLVRLFNDEFRKESKKSIPAFLLREKQEAIXSFLRGLFSADGSVLFRNKKPIIKFTNTNTEIIKMTSR
LLHLVGISHSTFSETRKNRYKGKESETISKHIYIKDALSFREKVGFVINRKQERLSLVSKNSTHRRTIKNY
DFDLSKVIKIEPIEYRGDVYDLEIEDTHRFFANNVLVHN (SEQ ID NO:182)


Mol-V3_PolB-2 MAGU01000080.1:7664-3744  Methanomethylicus oleusabulum isolate V3      903-
1086

SVAGDSIVAVDDGKGRSEVRIDGLFKGTSAKAGEKEYXRPRSLRTISLGPDGRVTWSRINAIM
RHRXGKRMFRVWLSDSWHVDXTEDHSLIGXLVDGDGKVDGELPAAGLRLVNLKPTEIGKVANRLVAL
DAQPHSNGGSAGXGAGIRTVTPVRVEEIPXDGYVYDMEVDGTHRFFANRVLVHN (SEQ ID NO:183)


Vsp-UBA156_PolB-2    DAUC01000001.1:16416-12496        Verstraetearchaeota species UBA156
903-1086

SVAGDSIVAVDDGKGRSEVRIDGLFKGTSAKAGEKEYXRPRSLRTISLGPDGRVTWSRINAIM
RHRXGKRMFRVWLSDSWHVDXTEDHSLIGXLVDGDGKVDGELPAAGLRLVNLKPTEIGKVANRLVAL
DAQPHSNGGSAGXGAGIRTVTPVRVEEIPXDGYVYDMEVDGTHRFFANRVLVHN (SEQ ID NO:184)


Vsp-UBA76_PolB        DAZQ01000002.1:44326-47664        Verstraetearchaeota species UBA76
714-892

SVAGSSVVSVDAGGKKSDVPVESLFGRPDQSVGGKEYXYPASLRALSLDPGGRAVYSRVNA
IMRHRSGKKMFRVRLADSWHIDVTEDHSLIGYRDGGGSGSSGIDGHLVDVRPAEIGKAVKRLVVLKK
GPLVAGRRAPSADFDTAAPLRIEEVPYDGYVYDLEIEGTRRFFANGVLVHN (SEQ ID NO:185)


Mme-V2_PolB  MAGT01000007.1:10654-7331 Methanomethylicus mesodigestum isolate V2    714-
887

SVAGSSVVSVDAGGKKSDVPVESLFGRPDQSVGGKEYXYPASLRALSLDPGGRAVYSRVNA

IMRHRSGKKMFRVRLADSWHIDVTEDHSLIGYRDGGGSGSSGIDGHLVDVRPAEIGKAVKRLVVLKK
GPLVAGRRAPSADFDTAAPLRIEEVPYDGYVYDLEIEGTRRFFANGVLVHN (SEQ ID NO:186)


Mimivirus-terra2_pol    574609278:172156-177378    Mimivirus terra2    1053-1403

    SVTGDTPIITRHQNGDINITTIEELGSKWKPYEIFKAHEKNSNRKFKQQSQYPTDSEVWTAKG
WAKIKRVIRHKTVKKIYRVLTHTGXIDVTEDHSLLDPNQNIIKPINXQIGTELLHGFPESNNVYDNISEQE
AYVWGFFMGDGSXGSYQTKNGIKYSWALNNQDLDVLNKXKKYLEETENIQFKILDTMKSSSVYKLVPI
GKIKYMVNKYRKIFYDNKKYKLVPKEILNSTKDIKNSFLEGYYAADGSRKETENMGXRRXDVKGKISAQ
XLFYLLKSLGYNVSINIRSDKNQIYRLTFSNKKQRKNPIAIKKIQLMNETSNDHDGDYVYDLETESGSFH
AGVGEMIVKN (SEQ ID NO:187)


DMF-AA-DWTP_dpol-1 LNFM01123066.1:1-1147    activated-carbon dual-media filters Ann Arbor
(MI, USA) drinking water treatment plant metagenome  299-383    partial - missing C-part

    SVAADTPILVKRNDQIEWIXIRDLXQHEQDPDKKTELNTEHFNYEVWSDIGWTKIKRLIXHKTT
KQMYRVLTHTGXVDVTEDHSLX (SEQ ID NO:188)


MegaVLBA111_dpol    448825558    Megavirus LBA111    1017-1298

    SVTGDTPIMIKDKNNNINIVTIKELGEKWKPYDIFKSHEINSNRKYKQQADFNGEVWTSNGWA
KIKRVIRHKTVKKLYRVLTNTGXIDVTEDHSLLDTNKNIIKPIDXKIGTELLHGFPEINNNHNKLSLEIYKEL
EITRMLFDXMIESNKKWNNEKMKAYFIGSEYRKQNKNISNEILNXSKKIKKYFLLGYLGNDKEYITNNKI
NAQIIYXLMKXLEYNIVIDLIESSYKLXIIDNINEPYXINKIIQLQDTSINGEYVYDLETESGTFHAGIGELIVK
N (SEQ ID NO:189)


Psa-CCMP1897_DpolZ NCGR_SEQ_ID=MMETSP0807_2-20130328|557:3271-969    Picocystis
salinarum CCMP1897  48-395

    SVMPYTPVLVRNKRTQXISAVAIKHLAQDWMPYEAFHEGDPRRFEKEQGNAAHMQAWTDQ
GWADVLRVVRHKXSKKIYRVVTHTGLVDVTEDHSLLLPDRRKVKPHQLEVGTALLHSFPGLELWPDR
LEAGTPEQAYMYGVFVGNGSXAKYDXPSGRKYYWAIKNSDITLINKWKTVLEMIHKRPFKIVDTLKHS
GDYKLVPTDSSKDLVILYWQSXYDNTGAKVVPYEILNGQIDHVDAFIEGLSVADGXRRDLDTTGXRRID
TKSQISAQHYYVLLKRLGYRVSINARDDKTNMFRLTWTMGRQRRETTIIKKQHMLHEXYDGFVYDLET
SQGVFQAGVGELIVKN (SEQ ID NO:190)


BtaR-2_PolB-2 REGX01147753.1:1817-1    Bovine gut rumen metagenome 464-607
    possibly an N-part of a split intein; partial - missing C-part
    SFTGDTPLFIKYDDGNIDIKPIEELIGETETDALGREYDYSEKPYSVLXRSGWVKPKYIYRHKTN
KQLYTVSEGDMSITVTEDHSLFNNEKKKIKPSQINSTTKLEYYTKDIKTSSDFKWLTKQRAKTMAKMIID
GTIDRVSIX (SEQ ID NO:191)


OarG-1_PolB-4 AUXO010636472.1:8780-13156    Sheep gut metagenome    624-978
    SVTYDTPILVRGEDKRIDIVPIXDIFNNNEAIEFGEEQYRDFSHKNXEVLTRNGWKPIEYVYKHK
TNKTLKRVETKNGLIDXTEDHSLFDNKRNEVKPSTLNRGDKIEIYTKDIDYYASSTVTDREAWLFGFFM

ADGSSVYXDRTQKYYSKRKGEWVIHNGKRANWKISNKSIDRLNKAKEILEDSFFLKASIKDHRTSSNV
YDLVVENAENAKFFSNNFYTSYRYKKVPEFILNAKKEVKKAFLDGFXXGDGQNDTIDEXIEFGQKSKVA
MAGLYLLMKELGYNFRXHNRNDKQEFISFRLRNHRGNLLNENYSERKEDEVWNXGNITSKSEYVYDI
SADGTFVNALGMIVXHN (SEQ ID NO:192)

BtaR-2_PolB-4 REGX01375834.1:1160-1        Bovine gut rumen metagenome 17-364
        SVTYDTPILVRGEDKRINIIPIXDIFNNNEAIEFGEEQYRDFSRKNYEVLTRNGWKPIEYVYKHK
TTKQLKRVETKNGVIDXTEDHSLFDNNGNEVKPSTLVRGDKIEIYNNDIDYFASSTVTDREAWLFGFFM
ADGSSVYXDRTQKYFSKRMGKRANWKISNKSLDRLNKAKEIMENSFXLKASIKDHRASSNVYNLVVE
NAENAKFFSNNFYTSYRYKKVPEFILNASKEVKKAFLDGFXXGDGQNDTIDEXIEFGQKSKVAMAGLY
FIMKELGYNFRXHNRNDKPEFISFRLRNHHGNLLNENYSERKEDEAWLXNDITSKSEYVYDISADGTF
VNALGMIVXHN (SEQ ID NO:193)

OarG-1_PolB-29        AUXO010203647.1:1-2159        Sheep gut metagenome        247-601
        SVTYDTPIIVRGEDKRIDIIPIXDIFNNDEAVEFDNEQYRDFSRKNYDVLTRDGWKPIEYIYKHKT
NKQLKRVETKNGLVDXTEDHSLFDNNGNEVKPSTLTRGNKIEIYTKDIDYFASSTVTDREAWLFGFFM
ADGSSVYXDRTQKYYSKRKGEWVIHNGKRANWKISNKSLDRLNKAKEILEDSFFLKASIKDHRASSNV
YNLVVENTDNAKFFSNNFYTSYRYKKVPEFILNARKEVKKAFLDGFXXGDGQNDTIDEXIEFGQKSKV
AMAGLYFLMKELGYNFRXHNRNDKQEFISFRLRNHRGSLLNENYSEKKEDEVWNXEDITSKSEYVYDI
SADGTFVNALGMIVXHN (SEQ ID NO:194)

LHPMM_PolB-1        JFJP01037070.1:7714-3716        Lake Huron low oxygen high sulfur sink hole
purple microbial mat bin unclassified.01 591-946
        SVEYDTPIYLKDKYDNLNILPIXDLFDDNSNFLSPDGLRDFSEKDFMVLTKNGWKNINYVYKHE
TNKPIHKIVTKDRLVXXTSDHSVFQNGEQIKPTELKRGDKIDIIDIPILKSLNVITPNQAKLIGFFIGDGSSS
YKKKPYKYNSVKNGEKTYQVMSGNFSLNNSRIELLEEFKLIMKNEYNVDTQINNTMKSSSVYKLQTSN
AEIXKWFSKNXYTSYRQKMIPYEILNGSKEIMKSFMDGFYLADGWGDNFDQPLDITQKSKVXVAGLTHI
LKTLDVNYRILIRTDKPNIQSLTLGSFNNKIKYHPLNDEKSKRKTNEVWNNVIYENKQQYVYDISTEDGT
FVGGIGGVLLKN (SEQ ID NO:195)

GS005_dpol-1  JCVI-reads_1091143429125+1091145607360:1-1641[1097159067138:2-1600]
        Bedford Basin environmental sampling  161-445
        SVTGDTPLLIRMRDGSIHTKRIDELTNEYHASDGGKESFPGNYEVWTEKGFTPVERVIRHKTM
KKMYRVLTHTGVVDXTEDHSLLDQAATMVKPTDITIGTKLLHGNTLDAFDXIDMTVSINEAKVMGFFFG
DGSXGQYGNKFTWALNNSNPDYRSLFYNDAKEKIVPSXILNAPIEIVQSFINGYYMADGDKDAXGYTR
MDXKSKQGTMGLQLLGRRLGYSVSLNTRSDKLNVFRQTWTKSTQRKEPNAIKKVLYLGETEQYVYDL
TTESHHFHVGPGELIVHN (SEQ ID NO:196)

CEV-01B_dpol 939177712        Chrysochromulina ericina virus 01B        965-1334
        SVASYTPIYVRYNKSIIDIXSVEELAEKYGNGWHLESPKEYXELNNIESWTENGWTEXHRVIRH
RLAPYKKMVRILTHTGLVDVTDDHSLVKNTGEEISPKDVSIGTKLLHXTMSENESNIESDISIDEARIMGF

FFGDGSXGIYDXPSGHKASWALNNSNKELIEKYYNLXKSVYPEFEWKVYDTLNSSGVYKIXFNKKSGS
KSKIQFIEKYRSMLYNKKSKIIPSEIINGSIELRKSFWEGLYDADGDKDKNGYTRIDQKSQISAAYIXWLA
NSIGYKTSLNIRDDKTDIYRITATKNKQRRDGDKIKKIVNIQNSANIQNSANIQNSVNIQNSVNIQNSKDN
QDYVYDLTTENHHFAAGIGNMIVHN (SEQ ID NO:197)


Hca-ATCC18719_dpol-1        355311217:2531-1     Hypochytrium   catenoides   ATCC   18719
    735-844        partial - missing C-part; missing part might be Hca-ATCC18719_dpol-2
        SVASYTPIMVRLSKRNRVLITIEELSRLSGKRWTSXGDPGRDNKEFIDLNDVETWSDKGWTPX
HRIIRHQLAPNKKMIRVVTRSSVVDVTDDHSLLRPDGTMVSPKDLRX (SEQ ID NO:198)


Hca-ATCC18719_dpol-2        355294737:1-2212     Hypochytrium   catenoides   ATCC   18719
    1-169        partial - missing N-part; missing part might be Hca-ATCC18719_dpol-1
        XGEHLLXHALAPRXSFNDTFPVTSEEARQMGYFFGNGTXGGTWDLIPQDVLNGSRAVRQAF
WDAMHGIDTGDGHDHGRNMLQIDQKSQLSIAHINLLAQSLGYTTSVTILTTSTESTVYRLIXTAATNER
SINSEIVEIYEIPYDGYVYDLTTENHHFAAGAGNIIVHN (SEQ ID NO:199)


Pat-CCAP1560-9_DpolZ-1      NCGR_SEQ_ID=MMETSP0151_2-20130828|5934:2077-126
    Paramoeba atlantica CCAP 1560/9 (621/1)    1-269       partial  -  missing   N-part
        XIYRVTSHTGVVKVTEDHSLLYHNGTEVRPKDVFAGENLLTSTLPSIDGTVDHSDISWVWGLF
YGDGSXGYXDXLTGKKYTWAINNQNGEYLSKAKEILQGYYSDYGFKILETMKKSSSVYKLVPTGKVSE
IVEEWRSLFYDPETRHKMVPNVLWSTTLKTREEFFEGYYXAGGEKGENGPTRFDNKGDIGSAGLFYL
GISLGYKASXNTRKDNLDITRITLTKSYQRKKPGGIIKKIEDFGQTGDYVYDLETENHHFSAGIGELVVH
N (SEQ ID NO:200)


Hlu-DSM14919_PolB  ELZ72768.1    Haloferax lucentense DSM 14919      626-1062
        SVTGDRPVVVRDPGGTVRILPIEDLFARGTTESEVLIAADGDVVASATPGKTRRALDGWDALS
VNEAGEAEWQPIAQAIRHKTDKPVVNLQHKFGESTTTRDHSYVVPGEDGLTTVSPDDVAEPYRVSGV
PDVEPVEQVDVYEVLRGYEREYEDGRSVGSDNSITKRKQIHADDEYVWFGHEHHRDVDSTVKVKRF
VDIDSEDGAALIRLLGAYVSEGSASTGETATSKFGASIAESDREWLAQLQRDYSRLFENTTAGIITSDR
RAERTVEYQTDTGGASVTYNDETLKLQMMNELAAVFFREFAGQTSRGKRIPSFVFHLPEEKQDLFLTL
LVEGDGSREFPRYTEAYAQRNFDFETTSRELAAGLSMLLTQRGQKHSLKYRDSKDSYTIRTXSTYRE
GRDPVLTEVDHDGYVYDLSVEENENFVDGVGGIVLHN (SEQ ID NO:201)


Hsp-Q22_PolB WP_058828069.1      Haloferax species Q22 626-1062
        SVTGDRPVVVRDPGGTVRILPIEDLFARGTTESEVLIAADGDVVASATPGKTRRALDGWDALS
VNEDGEAEWQPIAQAIRHKTDKPVVNLQHKFGESTTTRDHSYVVPGEDGLTTVSPDDVAEPYRVSGV
PDVEPVEQVDVYEVLRGYEREYEDGRSVGSDNSITKRKQIHADDEYVWFGHEHHRDVDSTVKVKRF
VDIDSEDGAALIRLLGAYVPEGSASTGETVTSKFGASIAESDREWLAQLQRDYSRLFENTTAGIITSDR
RAERTVEYQTDTGGASVTYNDETLKLQMMNELAAVFFREFAGQTSRGKRIPSFVFHLPEEKQDLFLTL
LVEGDGSREFPRYTEAYAQRNFDFETTSRELAAGLSMLLTQRGQKHSLKYRDSKDSYTIRTXSSYRE
GRDPVLTEVDHDGYVYDLSVEENENFVDGVGGIVLHN (SEQ ID NO:202)

Hma-ArcH_PolB       CQR50660.1    Haloferax massiliensis ArcH    628-1064

SVTGDRPVVARDPGGTVRILPIEDLFARGTTESEVLIAADGDVVASATPGKTRRALDGWDALS
VNEDGEAEWQPIAQAIRHKTDKPVVNLQHKFGESTTTRDHSYVVPGEDGLTTVSPDDVAEPYRVSGV
PDVEPVERVDVYEVLRGYEREYEDGRSVGSDNSITKRKQIHADDEYVWFGHEHHRDVDSTVKVKRF
VDIDSEDGAALIRLLGAYVPEGSASTGETATSKFGASIAESDREWLAQLQRDYSRLFENTTAGIITSDR
RAERTAEYQTDTGGASVTYNDETLKLQMMNELAAVFFREFAGQTSRGKRIPSFVFHLPEEKQDLFLTL
LVEGDGSREFPRYTEAYAQRNFDFETTSRELAAGLSMLLTQRGQKHSLKYRNSKDSYTIRTXGTYRK
GRDPVLTEVDHDGYVYDLSVEENENFVDGVGGIVLHN (SEQ ID NO:203)


Hgi-ATCC33959_PolB  ELZ83304.1    Haloferax gibbonsii ATCC 33959     626-1062

SVTGDRPVVVRDPGGTVRILPIEDLFARGTTESEVLIAADGDVVASATPGKTRRALDGWDALS
VNEDGEAEWQPIAQAIRHNTDKPVVNLQHKFGESTTTRDHSYVVPGEDGLTTVSPDDVAEPYRVSGV
PDVEPVEQVDVYEVLRGYEREYEDGRSVGSDNSITKRKQIHADDEYVWFGHEHHRDVDSTVKVKRF
VDIDSEDGAALIRLLGAYVSEGSASTGETATSKFGASIAESDREWLAQLQRDYSRLFENTTAGIITSDR
RAERTVEYQTDTGGASVTYNDETLKLQMMNELAAVFFREFAGQTSRGKRIPSFVFHLPEEKQDLFLTL
LVEGDGSREFPRYTEAYAQRNFDFETTSRELAAGLSMLLTQRGQKHSLKYRDSKDSYTIRTXSSYRE
GRDPVLTEVDHDGYVYDLSVEENENFVDGVGGIVLHN (SEQ ID NO:204)


Hgi-ARA6_PolB       WP_050458792.1    Haloferax gibbonsii ARA6    626-1062

SVTGDRPVVVRDPGGTVRILPIEDLFARGTTESEVLIAADGDVVASATPGKTRRALDGWEALS
VNEDGEAEWQPIAQAIRHKTDKPVVNLQHKFGESTTTRDHSYVVPGEGGLTTVSPDDVAEPYRVSGV
PDVEPVEQVDVYEVLRGYEREYEDGRSVGSDNSITKRKQIHADDEYVWFGHEHHRDVDSTVKVKRF
VDIDSEDGAALIRLLGAYVPEGSASTGETAASKFGASIAESDREWLAQLQRDYSRLFENTTAGIITSDR
RAERTVEYQTDTGGASVTYNDETLKLQMMNELAAVFFREFAGQTSRGKRIPSFVFHLPEEKQDLFLTL
LVEGDGSREFPRYTEAYAQRNFDFETTSRELAAGLSMLLTQRGQKHSLKYRDSKGSYTIRTXSSYRE
GRDPVLTEVDHDGYVYDLSVEENENFVDGVGGIVLHN (SEQ ID NO:205)


Hac-MH1-52-1_PolB-3  BATA01000002.1:6863-13326  Halarchaeum acidiphilum MH1-52-1    1448-1881

SVTGDRPVVVRDPEGRVRITPIAELFERAARSENVLVTADGGPVTSASVGKDRRTLDGWDXL
SLNDDGETEWKPIEQAIRHETDEPVVKLQHEFGESTTTRDHSYVVDGADGLEEAVPADVDEPLRVPD
MPDAGTVTEIDVYEVLRGYEREYEDGRGTGGSTVKTKRVYADDESVWFGHEHYGDLDSTVTVQRHI
DLASEDGAALVRLLGAYVPEGSASTVETADGKFGASIAESRREWIEQLEDDYHRLFENAEASIIASDSR
DERALEYETESGAESATYDDRTLKLQMMNELSAVFFREFAGQTSHRTRIPSFVYHLDDDLQALFLDVL
VEGDGSREFPYSEGYAARNFDFETTSRELAAGLSMLLTQRGKKHSLKYRDGKGSYTVRTXDSYRGG
RDPVLTTVEHDGYVYDLSVADNENFVDALGGIVLHN (SEQ ID NO:206)


Msp-CG1-02-55-22_PolB    OIO26163.1    Micrarchaeota species CG1_02_55_22 572-991

SITDERXIAYLDXNGILRLAPIAEIFDRYGKTKTAXGDKEVIYAPGIRALSVDPKTMEPIWRPVTE
IIRHRNTKRVYRVRQKTGETRVTEDHSIMIDKRGFLEEXKPADIGKKRLAYLRKIPAVKEITEINLTDWLG

EYENKVRYKGRIKTRAIKVADDGSLTFSWTAQKRQIKVQRRYPVESPRFEALXRLLGAYIAEGSASTPE
TTGTRMGASIASGNREWLESLKMDYESLFTNARAXVIRSNIKTRHLDYVNLDGMAHSTVYDDATXKLQ
MMNAVSAMVFKQLXGQKSYGKHLPEFIYHVPRKYKLLMLEKMVEGDGSRAXGPRYTREYRDRNFKY
HTSSLRLASGLSLLLNQLGINHSIRYYSKRKSYXVTTXSITNDRLGTDVVEEPYQGFVYDLSVEGSRMF
TDAXGSVVLHN (SEQ ID NO:207)


Msp-UBA95_PolB       DALH01000057.1:29289-25753       Micrarchaeota    archaeon    UBA95
536-954

SVTSERFLVLLDDKELVHVKNVEELFEENAKHLIEXGEKQVIPLTGWRXLSVNPASKKTEWKK
VTELIRHKTNKRVYRVNQKFGETRVTEDHSLMADTPNGLVEVKPVNAKKHRLAQAEVLKAKGGVEKID
VYEVLKDYSEKTVYKGFGKIKTIKXNSERVWFGWTNQKNPVKVKRFIGIETKEFESLXRLLGAYAAEGS
SSTIETTRSRYGASIAGKRKWLEGLQKDYLALFTAKAGVIPSQKKTRHLTYRTQKGVKKTVVYKDDTH
KLQMMNSLSAVFFKMFXGQKSAGKKLPDFIYNVPKKYQLIFLKKLLEGDGSRSVNERLGYSAEYKKKN
FKYTTISAGLASGLSVLLRQLELNHSIXYRPSKKAYTLSTSGKYNKRIQTKVAREEYSGWVYDLSVEDN
HAFTDAXGQIVLHN (SEQ ID NO:208)


Hsp-J07HX64_PolB-2   541200412    Haloarchaeon species J07HX64              997-1434
SVTGERPVVVRDPDGIVRIYPIERLYQRATQSPAEDTVITVDGMPITGIESSKEYATLEGWDAL
SVDDDGQSEWSPIEQVVRHETDKPVIKLQHKFGESVTTRDHSYVVESGGELVEAPPNEVESPLRIPD
MPETDEIETIDVYEVLNGYTRRYEDGRGSGGVTTKTKRVXADDEAVWFGHEHHRGLDKTVEVQRYID
LDSEDGEALLRLLGAYVPEGSASTVETTDSRFGASIAESRRTWLEQLQDDYHRLFENTSVSIVASDTR
DERTVEHPGDDSETALTYDDQTLKLQMMNELAAVFFREFAGQRSRGKKIPSFVFHLPAEKQELFIQML
VEGDGSREFPRYSAEYAERNFDFETTSRELAGGLSVLLTQRGTKHSLKYREEKESYTVRTXDYYDSG
REPVLTEVAHDGYVYDLSVRENENFVDAVGGIVLHN (SEQ ID NO:209)


Hsp-J07HX5_PolB-2    538422166:342937-352558    halophilic archaeon J07HX5    1020-1421
partial - missing C-part
SVTGDRPIVARTPDGLIRIVPIEELFERASPAPSDRALVTTDGGPAATAGSAKEYRSLDGWDA
LSVNNRGMTEWQPIEQVLRHETEKEVVRLQHERGESVTTRDHSYVIEENGELIEAPPEDVGSPLQIPD
VPATQEVGKIDVYELLHGHERESMDRQGTDSTTIVRRIHANDDRVWFGHEHASNRHEQTVSVQRYID
LDSESGNALVRLLGAYSSNESASTVETTDNRPGVSISASNRRRLEQLQADYHRLFENTTGRIVARETG
SERTVSDASSEGEAATAPAVASVDDTLKLQMMDELAAVFFREFAGQTPXETRIPSFIYHLPEEKQDLF
LRMHLGDDGARTFPQAAEESAEQDIQFETTSRELAGGLSLLLTQRGKKHSFNYRNQRNSYTIQTGEY
DX (SEQ ID NO:210)


Hsp-J07HR59_PolB-2   541196408    Halorubrum species J07HR59   986-1422
SVTGERPLVVRDGDGRVRILPAAELFERAEANDHIAIAADGGPAGSHGLGKGRASLPGWEAL
SLASDGTAEWQPIEEVIRHDTDGTVVHLQHQLGESTTTRDHSYVVEEDGEYIERAPEDVTDPLRIPDV
PSVDTVDSIDVHEILDGYTGEHADRTTPRGETGSQKRPRVHTDGESVWVGHSREGESENTTKVQREI
DLTGPTGRSLVRLLAAYIRDGSTTTAETADSTVGVSISESRPEWLETIATDSERLFENAQVSVNAGGTD
DEQTLVSETRTDETVSSNGGGTGELRMMDELXAVFFTEFVGHTAPEKHIPSFVYHLNDELQRVFTETL

VAVDDLAELASHTEAHAQRQMNFEATSRELAAGVSMLLTQQDRTHSLHYRDETNRYTITPDESDQSA
RDPVVTEQEHDGYVYDLSVAENENFVDAVGGIVLHN (SEQ ID NO:211)


Asp-A07HR60_PolB-2  ESS11652.1  Archaeon species A07HR60  1118-1554

      SVTGERPLVVRDGDGRVRILPAAELFERAEANDHIAIAADGGPAGSHGLGKGRASLPGWEAL
SLASDGTAEWQPIEEVIRHDTDGTVVHLQHQLGESTTTRDHSYVVEEDGEYLERAPEDVTDPLRIPDV
PSVDTVDSIDVHEILDGYTGEHADRTTPRGETGSQKRPRVHTDGESVWVGHSREGESENTTKVQREI
DLTGPTGRSLVRLLAAYIRDGSTTTAETADSTVGVSISESRPEWLEAIATDSERLFENAQVSVNAGGTN
DEQTLVSETRTDETVSSNGGGTGELRMMDELXAVFFTEFVGHTAPEKHIPSFVYHLNDELQRVFTETL
VAVDDLAELASHTEAHAQRQMNFEATSRELAAGVSMLLTQQDRTHSLHYRDETNRYTITPDESDQSA
RDPVVTEQEHDGYVYDLSVAENENFVDAVGGIVLHN (SEQ ID NO:212)


Hsp-J07HN6_PolB  541194869  Halonotius species J07HN6  650-1088

      SVTGDRPVVVRDPDGIIRVLPIENLFERATSSTSDTVVITADGGAVGSASAGKDRRRLDGWEA
LSLAADGEPEWQPIQQAIRHDTDKPVVNLQHKFGESTTTRDHSYVVGDDGKFAEATPDDVDEPLRIP
GVPAVDTIERIDVYEVLDGYTREYEDGRSVGSANATSKTKRVHANDEWVWFGHDHHNELSKPVKVQ
RYIDIDSEDGAALLRLLAAYITEGSASTIETTESRFGASISESREEWLDGLQSDYYRLFENTTASVIASDS
SGDRTVEYDTSDGAQSVTYDDTTHKLQLMNELSAVFFREFAGQTSRGKRIPGLVFNLPADAQDLFLD
TLIEGDGSREFPRYTDAYSERHFDFETTSRELAAGLSMLLTQREQKHSLKYRDTKNSYTIRTXDSYRS
GRDPVLTEVDHDGYVYDLSVADNDNFVDAVGGVVLHN (SEQ ID NO:213)


Hsp-J07HN4_PolB-2  541199475:1411803-1409440  Halonotius species J07HN4  507-787
      partial - missing C-part

      SVTGDRPIVVRDPDGIVRVLPIENLFERATSSTGDTVVITADGGAVGSTTTGKDRRQLADWEA
LSLSADGTPEWQPIQQAIRHEVDKPVINLQHKFGESTTTRDHSYVVGDDGELVEATPDDVDEPLRIPG
MPAVDTVETIDVYEILDGYTREYEDGRSVGSEDAATKTKRVHANNEWVWFGHDHHNELSKPVKVQR
YIDIDSEDGAALLRLLAAYITEGSASTIETTESRFGASIXESREEWLHGLQSNYYRLFENTTASVIASDSS
GERTVEYETNNGMX (SEQ ID NO:214)


Hwa-J07HQW1_PolB-3 538422602:1028102-1034583  Haloquadratum walsbyi J07HQW1  1438-
1872

      SVTGDRPVVVRDPRGYLRIIPIGTLFEQATVPEQNARLTADGAPTGSSGFPKERRHLSQWEA
LSLTDAGETEWQPIKQIIRHQTEKEVITLQHEHGESTTTRDHSYITDDNGEYVETPPEDVDEPLPIPEIA
PIKTIETIDIYQTLTTAAHTHTGSDIETSERLPSTDHIHATDEYVWIDTTPEGQDKHDSIPAIPRYIDLTSDT
GHALIRFLGVYLSDWSESTVTISEQEQQAQQLDITGPHESALRTXTADADQLFINVTPTVTANAENNAN
AVDGEYSXHIPTTLATTLVSALAGHPAXVKQVPSVIYHLPDAEQSRFVQXLIGAESKLTSGTLLNDSQNI
NDPISSTGEFTANRELAAGVSMLLTQREQSYSIVQQDAEDTYTIRINDTESSSSEXHPTLTETAHSGYV
YDLSVEANQNFVDGLGGLVLHN (SEQ ID NO:215)


Hwa-J07HQW2_PolB-3 538433089:2942925-2949316  Haloquadratum walsbyi J07HQW2  1431-
1855

SVTGDRPVVVRDPTGRIQIVPIETLFELATAPKRNTRITADGAPTGSSGLPKERRHLDQWEAL
SLSDTGETEWQSIKQIIRHQTDKEILTLQHEDGESTTTRDHSYITADDGEYVETSPKNVDEPLSIPEIAP
VKTIETIDIYQILTANTQTHAGSNIDPGEWLPSTDXIHANDEXVWINPTGEEREDSTPTIQRHIDLTSDAG
HALIRFLAVXLSSRSKSTVRTIESKQYLQIIGPHKSEIKTXKVAIDQLFTNVTTRIAGDAENNTNTGDSTF
RXHISTTLAATVMTAFVGHPAENNQLPSVVYHLPDAEQSYFIQQLIRPKSNELLSXPQNIDDSISIESDF
TTSSRELAAGVSMLLTQXGQSYSILQRGSEDVYTIQVGDPPSSEXEPMLIETADSGYVYDLSVEANQN
FVDGLGGLVLHN (SEQ ID NO:216)

Hsp-DYS4_PolB        WP_058367233.1        Halopenitus species DYS4        645-1083
SVTGDRPVVVRDPDGVVRVVPIERLFERAEMERGEELLVTADGGPVASVAAGKERRNLSEW
EALSVSEGGVPEWQPIEEIVRHETDKDIVNLQHKFGESTTTTDHSYVVEDGDDLVETKPEDVTAPLRIP
GLPEVDTVDRIDVYEVLDGYTRSYEDGRSVGSDNAETKINRVHANEEYVWFGHEHQADQSRTVKVK
RHIDLEGADGEALVRLLAAYVTEGSASTIETTDSRFGASIAEARTDWLDGLKEDYDRLFEGVTATVIAS
DTSAERTVEYETDEGPSSTTYNDGTHKLQMMNELTAVFFREFAGQTSRGKRIPGFVFNLDEDLQNLF
LDVLIEGDGSREFPRYSEEYXERNFDFETTSRELAAGLSTLLTQRGKKHSLKYRDSKGSYTVRTXEFY
RGGRDPVIKDVDHDGYVYDLSVAENENFVDGVGGIVLHN (SEQ ID NO:217)

Psa_dpol-2        516306302        Pandoravirus salinus        1592-1993
SVGADTPLLLRFDGKHVDYVRADQVDGILARGDTSAAASLWSAYQGDKEAFXLARPVEVWT
ERGWTAVNRVIRHRAGKKMFRVLTHTGXVDVTEDHSLLDPNAEKVKPTEVSVGSALLHADLPTHEXA
TASLKSRVPMSTQDITDDDGDDDSAKVSAASSTSADGSVLVSAADKAHAWAMGMFFAEGSXNEHPR
DNXTQYXWRIANKDMALVRMAFDGLEGRYPGVTFSISGPXTDGMAYVVANGPGKMGLVANYRTAFY
DPVYALKRVPTEILNAHVEIKRAFIRGYFAGDGNKKLYGADGTYXGSRXGGKGKIGMAGIYYLLSAXGY
LASVDTXGGPERDTYRINFXDAATARRTQRKPADTVKKIIPLDSAAFDGAYVYDLETANHHFAAGIGRL
VVHN (SEQ ID NO:218)

Pin-KlaHel_dpol-2        755573852:1764942-1760806+1760651-1758080        Pandoravirus
inopinatum KlaHel        1514-1908
SVGADTPLLLRFDGKHIDYVRADQVDDVLARGDASAAARLWSAYQGDKEAFXPARPVEVWT
ERGWTAVNRVIRHRAGKKMFRVLTHTGXVDVTEDHSLLDPNAEKVKPTEVTVGSALLHADLPAYEXIT
ASLKSHVPALTQDKTDDNGDDDGTTRVSTTLSATADSILVTAADKAHAWAMGMFFAEGSXNGYPRG
NLQPYXWRIANKDMVLMRMALDGLEGRYPDVTFSINGPYADGMAYVVANGTGKMGLVANYRAAFYD
PVHALKRVPTEILNAHVEIKRAFIRGYFAGDGNKTDXGSRXDGRGKIGMAGIYYLLSAXGYLASINTXG
GPERDTYRINFXDAATAKRTQRKPRDAIKKIIPLDAEAFDGAYVYDLETANHHFAAGVGRLVVHN (SEQ
ID NO:219)

Pma-macleodensis_dpol-2        YP_009480895.1        Pandoravirus    macleodensis    macleodensis
1630-2013
SVGADTPLLLRFDNKYIDYVRADQVDESLAGGDASAAARMWSAYQGDKEAFXPPRSIEVWT
ERGWTAVNRVIRHKAGKKMYRVLTHTGXVDVTEDHSLLDRHARKIKPTDVAVGSALLHTDLPPHEXP
SPSLQRHTNASADPVTGDAKLLASSTAQNMTDDTAHAWALGMFFAEGSXNEYVRRDRDQYXWRIAN

KDMVLLRMALDGLDGRYPGITFSINGPYKDGMAYVVANGPSKMGLVANYRAAFYDPAYALKRVPTEIL
NANTKIKRAFIRGYFAGDGNKTDXGSRXDGRGKIGMAGIYYLLSTXGYSVSINTXGGPERDTYRVNFX
DASAPKRTQRKAPDTIKKIIPLDDKSFGVNAYVYDLETANHHFAAGIGRLVVHN (SEQ ID NO:220)


Pne-neocaledonia_dpol-2     YP_009481838.1     Pandoravirus    neocaledonia    neocaledonia
1645-2033

SVGADTPLLLRFDNKYIDYVRADQVDESLAGGDASAAARMWGAYQGDKEAFXPPRSIEVWT
ERGWTAVNRVIRHKAGKKMYRVLTHTGXVDVTEDHSLLDRHANKIKPTDVAVGSALLHADLPPYEXP
SPIFEXRADASAKSATGSANTVVASSVADNGAAVADDAAHAWALGMFFAEGSXNEYVRHDRDQYXW
RIANKDMALLRIALAGLGGRYPDISFSIVGPYKDGMAYVVANGPGKMGLVANYRAAFYDPVHALKRVP
TEILNANAKIKRAFIRGYFAGDGNKTDXGSRXDGRGKIGMAGIYYLLSTXGYSVSINTXGGPERDTYRV
NFXDAATPKRTQRKAPDTIKKIIPLDXDKSLGGNVYVYDLETANHHFAAGIGRLVVHN       (SEQ     ID
NO:221)


Ppa-8.5_dpol    OFAJ01000008.1:76751-82203 Pandoravirus pampulha strain 8.5        1047-1435
SVGADTPLLLRFEGKYVDYVRADQVAETLAAGDGVAAAQLWSAYQGDKEAFXPRRPIEVWT
ERGWTVVNRVIRHRAGKKMFRVLTHTGXVDVTEDHSLLDPNAEKVKPTEISIGSALLHADLPAHEXAA
VSLKSRAIRPADTTNPAILAAPTAVDSDGDVAIDDANDTAHAWAMGMFFAEGSXSEYLXGNAQQYXW
RIANKDMVLVRMTLDGLKGRYPDVDFSIIGPYADGMAYVVANGPAKMGLVANYRAAFYDPVHALKRV
PTEILNANVEIKRAFIRGYFAGDGNKTDXGSRXDGRGKIGMAGIYYLLSAXGYLGSINTXGGPERDTYR
INFXDAAAPKRTQRKPPDNIKKIIPLDAKAFDGAYVYDLETANHHFAAGIGRLVVHN (SEQ ID NO:222)


Pbr-SL2_dpol    LT972217.1:(1289702-1286900+1286777-1284244)     Pandoravirus     braziliensis
strain SL2     1064-1450
SVGADTPLLLRFDNKYIDYVRADQVDESLSGGDAAAAARMWSAYQGDKEAFXPPRSIEVWT
ERGWTAVNRVIRHRAGKRMYRVLTHTGXVDVTKDHSLLDPQANKIKPTDVAVGSALLHADLPPYEXP
SSSLQRRAAASDDPVIDSAKSLASSAGESTAVSTNDTAHAWALGMFFAEGSXNEYVRGDRDQYXWR
IADKDMVLLRTALDGLDGRYPGVTFSINGPYKDGMAFVVANGPGKMGLVANYRAAFYDPVHALKRVP
TEILNANTEIKRAFIRGXFAGDGNKTDXGSRXDGLGKIGMAGIYYLLSTXGYSASVNTXGGPERDTYRI
NFXDAATPKRTQRKAPNIIKKIIPLDGEAFGGNAYVYDLETANHHFAAGIGRLIVHN (SEQ ID NO:223)


LQPX-NY07348D_DpolZ       NCGR_SEQ_ID=MMETSP1433-20131031|1469:2731-1
Labyrinthulid quahog parasite QPX NY07348D  250-557
SVVGSTQLLLKVNGMLXVSRISEMVYLLHSSRWTTWRHEKQAVQIDPRKDEIFTWTDTGWS
RVRXIIRHRXTKSLFKIKTTHGDVTXTNDHSLLQPDGSXISPXALSIGKTRLLSSFPNXRDFPQEDSPEL
LVPGPXVPQSFVLDAMIAELAGLYISQGRDDLXVVVPNPVRRQAFLHTLQHRLGHLNWAIVNDLKIVPV
DLGDAKALKETRRFWNHDVXNDGLGVPNWVLNSNRRIRVAFSKGFGAGDLELGGFTLEASLTLNHD
RKLEEALVLDILPVENHGEFVYDLTTENHHFQAGSGSLIVHN (SEQ ID NO:224)


LQPX-NY0313808BC1_DpolZ  NCGR_SEQ_ID=MMETSP0098-20131031|11088:978-6038
Labyrinthulid quahog parasite QPX NY0313808BC1     1000-1307

SVVGSTQLLLKVNGMLXVSRISEMVYLLHSSRWTTWRHEKQAVQIDPRKDEIFTWTDTGWS
RVRXIIRHRXTKSLFKIKTTHGDVTXTNDHSLLQPDGSXISPXALSIGKTRLLSSFPNXRDFPQEDSPEL
LVPGPXVPQSFVLDAMIAELAGLYISQGRDDLXVVVPNPVRRQAFLHTLQHRLGHLNWAIVNDLKIVPV
DLGDAKALKETRRFWNHDVXNDGLGVPNWVLNSNRRIRVAFSKGFGAGDLELGGFTLEASLTLNHD
RKLEEALVLDILPVENHGEFVYDLTTENHHFQAGSGSLIVHN (SEQ ID NO:225)

SLM-T5-Br_dpol-4     LFIK01001925.1:1643-6121     Russia Kulunda-steppe soda lake Tanatar-5
brine environmental genomics   957-1185

SVLKDTPIIVYDIIEKEIKIKTIEELGGKEPRIWHSYKNFKVLDSYLSNRRSKKQSFISNERYLVW
TASGWSSINRVIKHKXNKKIYRIQTSHSIVDVTEDHSLINEKGNKIKPSEXTIGTRLLYHPLLMNLIDKEEY
RIKYKKVSDKRHFTSKKTLSKYILKSKYPMNVNVSRDTQGNEIYSASVHIGLYDNRITKIELLHDKVVEY
VYDIETQDGTFNVGFPLIVKN (SEQ ID NO:226)

SLM-T5-Br_dpol-3     LFIK01001957.1:1619-6106     Russia Kulunda-steppe soda lake Tanatar-5
brine environmental genomics   958-1186

SVLKDTPIIVYDVIKRKINIKTIEELGGIDEYVWHSYNNFKVFDSYLSNRRFKKQSFISNERYLVW
TSSGWSSIHRIIKHKXNKKIYRIQTNHSIVDVTEDHSLIDETGKKIKPSQXSIGTRLLYNPLLFGLENKDNY
EMKYRKVSDIKYYETKKELSKYVLQSKYPMKIESSRDSQGNEIYSASVHIGLYDNRIKKIELLHNRVGDY
VYDIETTDGTFNVGFPLIVKN (SEQ ID NO:227)

SLM-T5-Br_dpol-2     LFIK01011635.1:6631-2168     Russia Kulunda-steppe soda lake Tanatar-5
brine environmental genomics   953-1180

SVLGDTPIIVYDLIEHKVXVKTIKELGDGRIHQWTSYKNFKVFDSYLSNRRFKKQSYNANERYL
VWSASGWSSIRRIIKHKXNKKIYRVQTPHSIVDVTEDHSLLDYKGRKLKPSEXVVGTKLLYNPILFGLPD
KEFYQLKYHKDTQERMSFKTKKELATFILRSKYPMKVEYRKGDEKPYSATTHIGLYDNRITHMEVLHS
RIMEYVYDIETSDGTFNVGFPLIVKN (SEQ ID NO:228)

GASAB-3_PolB-C-1     JRYJ01113921.1:1-564 Gujarat India activated biomass of a wastewater
treatment plant treating hydrocarbon contaminated wastewater at high TDS (total dissolved solids)
       1-149       partial - missing N-part

XSIGDYTIEDLYNQXTDFYSKGSKEYGKXSLLVLGYDHSSQTADFKSVNYVMRHKTTKDIYKL
TLQDGKTVKTTHDHSAMVIRDGTLIEVKPFEINNTDMFIXIDDTNKVYNTAIDKVELLGTFDDYVYDVSIN
DNSHYFFGNDILLHN (SEQ ID NO:229)

OarG-1_PolB-C-2-1     (AUXO018541813.1+AUXO011422785.1):1-948       Sheep       gut
metagenome   32-195

SVVGSSEVMTDSGKMTIEDMFDEIXDSDSDSKIRVRSNRKVYVLADGVPELREINYIMRHKTE
KKIYRIESENGKIVHATSDHSIIVXRDGIISRXKPEELTSSDELLTINDYPDLGFIPSKVKSVTVSEYNWED
NYVYDISVKVEHEDDLEHTFFANGIXVHN (SEQ ID NO:230)

HsaG-1_PolB-C-1-1    OIWQ01000167.1:3413-5644    Human gut metagenome Denmark Roux-en-Y gastric bypass surgery of morbidly obese patient        134-292

SITGDSAIRMEDGSYETIENLFNAYXDTDSDDKIRVSXDKRVYGXSSDFNPXTYPIKYIMRHKT NKTIWRVTSTQKTINVTEDHSIVIVRNNIMXNIKPNDIDIDTDKLIIYDNDKIVLADIQSNTITNLSDEYVYDI EIDSDDAEKHVFFANDILVHN (SEQ ID NO:231)


OarG-1_PolB-C-1-1    AUXO014566809.1:20814-23309        Sheep gut metagenome        142-307

SVAGDSRILLKYGDNYFTKEIQELFNEFXDSDSGDKIRVKVGSKYLVGTYDPETDKPIYRGIDY VMRHKTNKRRFRIXLDGGNSVVVTEDHSIMVLKDDNLVEAAVKDLHIGDKLIVYANRESVVQKDIHSIIY VGXDDEYVYDLXVFADKDIQHTFFADNILVHN (SEQ ID NO:232)


MLk43c2588_dpol    MetaVir2_4314-ML_43kmer_contig_2588:6976-3119    uncultured    linear-genome virus ML 43 kmer contig 2588 from Midtre Lovenbreen (Svalbard) glacier cryoconite holes sediment        730-982

SVLGSTVLVLRDPKTNWISIKTIEQLSIGTNSYSYDNFKLDGTLRSHKSYTLTNYQIWTDVGWS NIKRVIKHKTNKKIFRVVNNNGWXDVTEDHSLLDSQLNPIKPEQINGDTVLANTFINEFNKDKTNISSSL AYKMGYKXILDPVINGSIINSPKNIRASFYKGYLKNXNIKQVKSQVWWQGIYYIQKSLGFNNXISKSDSP FVNTNLKXSNDETSIQELEMSSIDGDFVYDLETEXGRFXAGVGSLLLKN (SEQ ID NO:233)


ABk43c3071_dpol    MetaVir2_4314-AB_43kmer_contig_3071:8112-4255    uncultured    linear-genome virus AB 43 kmer contig 3071 from Austre Broggerbreen (Svalbard) glacier cryoconite holes sediment        730-982

SVLGSTVLVLRDPKTNWISIKTIEQLSIGTNSYSYDNFKLDGTLRSHKSYTLTNYQIWTDVGWS NIKRVIKHKTNKKIFRVVNNNGWXDVTEDHSLLDSQLNPIKPEQINGDTVLANTFINEFNKDKTNISSSL AYKMGYKXILDPVINGSIINSPKNIRASFYKGYLKNXNIKQVKSQVWWQGIYYIQKSLGFNNXISKSDSP FVNTNLKXSNDETSIQELEMSSIDGDFVYDLETEXGRFXAGVGSLLLKN (SEQ ID NO:234)


KNV1_dpol    KY684109.1:(303102-300955+300717-297571) Klosneuvirus-KNV1    887-1391

SILGNEVLTLLNDKNDIEFHRIDKLXDKWESYDNFKTDEYNEYFTNILNKLFKDKTDSNNSILMN ETDYINGKIMGSIYTNNIRKNHEVSIGALGKIDGKRTKKQFYFSMYGNDTKALHEALKYRLKLNEELDLI KNKYRYMIDLDSNRYIEVQVNNNKIMLXDIDDIDIIEKYTWXINEKNYVVSNNVNSESWQYHKLVLNKIL NKLPNNIRNQIKDLTVDHMNRNTLDNRKQNLRLVNSKEQQWNQDIFKTNTSGTRGVYYRTNRKAWIA NWLNLDGKRESKYFKNKQDAINERKTQEQIMETYFNDERKKLIDKLKQLLINDQKDRYDKEQSSANYK VVWTDKGWSNINRVIRHKTYKRIFRIVTKTSIIDVTEDHSLIDKNGNYITPKTXTIGTELMYGINNFDTIEFV DIKSNKYDILAFSSDDKVEXMRYYXYNKKLGYNILIDVNNDKFVLHRTNDVIENPNKIINIQQLNDVVDDY VYDLETEVGHFHAGIGEIIVKN (SEQ ID NO:235)


CTV1_dpol    ARF09278.1    Catovirus CTV1932-1286

SVTGETPILLKNPTTNKIVIKRIDELGTEWKNYNNYKSSDSNRYFRELLTILFKNKNVEKKRENF VPVSQWMYIKNNKYRYINDAYSNNTYLQVNLNNNKNMIXDVEDIDIINXELKNISKMILNKIIKLFPKQIQA

KLQKYEVNYINENSLDNRKXNLSIDVDNQTIDKIIEDIENDQKDRYSKEQSTTNYLAYTDKGWAKINKIIR
HKTTKKIYRILTTNSIVEVTXDHSLIDENGNYIMPKDXVIGKGLMQSYPHTNKLKYNDKYDKSIFTNKNY
KEXMKYYHYNKSHGFNITIDENNGIITLKRTKDNINNINKIVKIFDLGDISTDRYVYDLETEHGRFHAGVG
ELIVTN (SEQ ID NO:236)


COMG7_PolB-2        IMG_3300001749-JGI24025J20009_10006704:6977-
4235+IMG_3300002180-JGI24724J26744_10714081:1-303    Coal    Oil    Point,    Santa-Barbara
California, Crude oil metagenome 3 and 7        196-639
     SVDAGTDIIIREGKLVKIVNIKDLFESXDSEIYRKGDHEYKAPEKIEXLSVSKEGIXEWKKANSIK
RHPYEGKILNIQTRRGSISVTKNHSLYALSSGLKEILXRDLNKNTPLAHISKYSQAEKKLKINALEPLRKF
SNELNIWLSVPINDLTKRLLKYHQPRNNFKGGRXKKEFIRIPIPTAIELYNKKVIEDKDIQNAFISSYGGK
GKIPVIYELNKDFARILGAYAAEGSLHIRKRKGISKEGAYIFAXGHDIQSLEELKKILARIFRRNFNVTXSG
VDKNGRNFRIKSNSAVAYLFKFVLDVGQGSQGKEVSPYILSSSKSIQRAFFDEYTKGEGYXDKRRRVN
PLLEXTTKSKKLAEGLSLMAINLNXGLPSIRFRKENSSYQLRFVQYDLNSVKYRDLSGLLPKEIKEVKPT
DGYVYDVGVEGNNNFVXAKGLILAHN (SEQ ID NO:237)


Esp-RBG-13-31-8_PolB-2        MGVV01000034.1:1078-5006    Euryarchaeon species RBG_13_31_8
    1125-1310        partial - missing C-part
     SVDSETDIIIKGNNLIKITNIKDLFEKQSSHIYKKENHEYKKLKDIEXLSVNNNGIXEWKKANFIKR
HPYKNDIIKVKTQRGMISVTKNHSLYTISKNIKEIYXKQLNXKDTNIVHISNFNEKGKKIIINALNPLVEFNN
ELNIVFNIPLKNSTKNLLKYYQHRKNYKGKVTLNKKYIRIKLIDAX (SEQ ID NO:238)


COMG7_PolB-1        IMG_3300002180-JGI24724J26744_10011162:838-4968        Coal Oil Point,
Santa-Barbara California, Crude oil metagenome 7        745-1137
     SIVGARXIAIKDDNLINIIPIEELWKKXXEPMEKINGKEIKTPQSVFTLSKNGEWQKIKKIIRHKTN
KIIFRINQKNGETIXTEDHSLITENYKPIKPKELGDKKILFLKKVPHEPTIFDRKIDLYPLVSHYSFETEYKE
RKKQNAWKADNKFLWFGWTSRENQHKINRFXNLSDLXKLLGIYIADGHSSLHKGKYGIKATAGISSKD
TLFLNELKEIMKRIDYNHQISILRVSKGERVIQGYKYEDRTYRLQTNSTTWTAFFSSLXGSGSENKHLP
QFIFNVEKKYQELLYKYYLLGDGSVEQGKNTFTSKSLQLVSGLXFLLKSWNIDTSIYYHEKRDVYRVRE
RERAVDSMHPIKTKLIELPKVERYVYDLSVENTEMFVDAXGMLLLHN (SEQ ID NO:239)


Csp-NIES-2242_dpol   BDDC01000222:24500-29131   Chlamydomonas    sphaeroides    NIES-2242
    879-1237
     SVTGYTPVLVRIQHKVRYXTIEKLPGLLGGGEWVPVVPAEPAAHHEKPKEALDLTHHHVETW
TESGWTRLRRIIRHALPPGKRILRITTPTGVVDATDDHSLLAPNGDPITPRNLHIGTPLMHAPLPLDEWR
RMAQTAARTTTPERARILGIFVAXRAMAFSQRGFIWTASKSIGSYFMDLXNKEFGGFTWTMTTSPEDG
ITVVKPEGVPGHILYSTLLQTXXXGGSADEPMVPDDVLXSNSIHVHMAFIEGFRMGNPMXLIFTHLLGA
TLFVLLQFMIEAHGDSGATDVRVAGSMPNINLVTSPSSTPSTEDSTSVMSMVDITDALRSKGDPTLLM
VYDLTTDNHHFAAGVGQMVVHN (SEQ ID NO:240)

LCGC14_PolB-3    816800949:14516-18139    Marine sediment metagenome LCGC14636-972

SIMGTEAVVVRFADDEPPMILSIQELWDMAHEKGYQIVYRADGKEQLVVQGLRVWDGKGWN RVYKLIRHFTIKTIYRTATGKGAVDTTEDHSLVDDEGHEFKPEHISSRGKEPVSSQFILPSKRLSYTDDI SATLLLFLFSMXGTASGTYTGYPSQRTVALHFSPKIKDIAQKLWWEAKPAAAAFGAKHKLYSTKKEKL VIKFSNHTQLWAFVRRNLYIKNQKIKPLPSVLSLPEHYLETIYGMLKDYYYNFDEKTKQDRWTIKSSSE MVLFTALANRFKEEMTVVPQPKGKIYQIKRTSDMKRRATVSITRILPDYVYDMETEDGTFMAGNILXHN (SEQ ID NO:241)

SLM-T5-Br_dpol-1    LFIK01017938.1:129-4880    Russia Kulunda-steppe soda lake Tanatar-5 brine environmental genomics    870-1178

SVTGYTPVYVKDQNGSIMLVTIESLAEKYGDSTWIPXVENGRQTKEAXELVGLETWTEKGWT PLKRVIRHILAPHKKIIRIVTHTGIVDVTDDHSLLDAEGNPVTSKDISMGTPLLHHVLPPXEEEEEEEEGKE EEKEAEVMGFSYGEGEHDEIPRHIFDAPLHIKQAFWKGLXDAKRGDEKDVEGNTTFDHRSMLGASHI ARLATYLGYSYXLSIDSMDSMDSMDSMKQDKFRSAGAKFRSAGTKFRITLKSTTSHETRGHTDHTDH TDNSDRTTISKMYEIPYEGYVYDLTTENHHFAGGVGQLILHN (SEQ ID NO:242)

Cas-NIES-2207_dpol    BDDA01000135.1:26392-30936    Chlamydomonas asymmetrica NIES-2207    891-1193

SVAAXTPVIVRSSADADDWDILPIELVPYAYGDAHWVRXHDESGDDNSSDVDAKEAXELYDR VEAWTEDGWTPLHRVIRHVLPQHTSLTRVVTPXGLVDVTNDHSLLTAPPEPVPVSPRDVVAGSTRLL HHAAYPPAPTSXSSINEDWAQRAAVEXVITATFNGSAALPWQLKQVAMAPGRIVREFWKAMLGTMKA LRASPGADILSLRMSQASAAWLLVVAARIQERATVHDTTSHDTWMGDDVVVVSFSATPSSSLQSHXD DHVVRTVRALPTSSIPRYVYDLTTDNHHFAAGPGRMVVHN (SEQ ID NO:243)

GS002_dpol-1 JCVI-assembly_1097156662696:1662-1    Gulf of Maine environmental sampling    178-387

SVMGYTPIVLKNDKDIINIISFDDLEYXKWLSYNNLDKNGSNKEQLFNPGYFIWTHNGWSPIIRF IRHKTIKQIYRIITNSGIIDVTEDHSLLDLNVKEIKPNQLKINDNLLHSKIKTKKINKESSKFFNINNKLIYKDL QDFVLRSKNIKIEKINNNDYMIVNVDYKYRDENKIIEIIKLHEKYEGYVYDIETKEGVFNAGIGNLVVKN (SEQ ID NO:244)

GS009_dpol-1 JCVI-reads_1091141114760+1091140251541:41-1117[1101669120223:2-1030]    Block Island environmental sampling    150-359    partial - missing C-part

SVMPYTPLTILRDDGVVEVTTFNDFNDLEWTSYSEFNKVGTHKEQIFNPGFKVWTNNGWSSV VRLIRHKTVKKIYRVLTNSGLVDVTEDHRLLDKDLNIIKPSQXEKGQELXHSKIKIGKHILKYRKQDEIYIE KYGKIYLNDDQQNEAQYIYILSQHFNDDNITISIENXKIVLSYEEREIMKEDKSKTRIQSIYVLYEYYGDTT PTPYX (SEQ ID NO:245)

AaV-BtV-01_dpol    672551009    Aureococcus anophagefferens virus BtV-01    881-1064

SVTGYTPITIKYKEQIFIEKIENVAKIFGEDKWQKXIDPGKQEKEAXELNEXFTWTSKGWTKLH

RVIRHILVKEKKIIRVLTHTXXVDVTDDHSLILKSGEEISPKDLKIGDELLQRDIEFDEIIEYSNDEYVKKQI
KYLKENMKSENESILSLNEIDYKGYVYDLTTDNHEFQAGIGNIIVHN (SEQ ID NO:246)


GS003_dpol-1  JCVI-
reads_1091145299650+1095403465086+1091143498047+1091143335043+1091143343545[109715
6702107:2-1228]      Browns Bank environmental sampling    458-682
        SVANYTPIYVKIDGKFEIIQIDELGKKYGNDNWKQXVEPGKQTKEYIELTDKNIYTWTENSWTQ
LKTIIRHKLASEKKMMRILTHTGXVDVTDDHSLIRNDGVEISPKDXEIGTELLHHPXLSDNIRNDASNSTF
VQDEFDLSIPSNHILMAKYIHHKYNATDTKYKLVSHDSNDSFMLSSEXSETTGHKIKKIMTLDDYDDYV
YDLTTDNHHFAAGIGNMIVHN (SEQ ID NO:247)


SCTP-2-BP_PolB      ASV44194.1    Salicola phage SCTP-2 629-791
        SVTHDTIISTNNGEYTIEKLFVNSSRFWEDSETGKEYSYDPSIKVKTYDPQNGTSYYGEINYIY
RHKTSKSKWLIRDSNNNEVIVTEDHSIMIENDDGMIKISPKDIVKGEDVLITVKXGEVLKXSIEDIVWLGY
FDNEYVYDVGMKNAEHNWFFGNNILLKN (SEQ ID NO:248)


OarG-1_PolB-C-9-1-1
        (AUXO016687107.1+AUXO010673474.1+AUXO011796439.1+AUXO010528631.1):7354-
6239    Sheep gut metagenome          134-295
        SVVGDSWITLSDDSFMMISDLFNEGTDTEIDQFGKERVKSNRSIYXVDLTTGKIDKKPIKYVMR
HKVNKRLYKVFNDYTYVVXTEDHSLLKYYDTNFKEVKPDDIDKDTFLFLKGENDLLXVSGFSVEXVSN
RNVAYVYDIEVDSDTDNYHNFFANGILVHN (SEQ ID NO:249)


OarG-1_PolB-C-26      AUXO011822949.1:872-66      Sheep gut metagenome          29-192
        SVIGSSMISLSDNSLKRISTLFNEGDNIETDVFGKERVSSDKSVYXLNTVTGXVEIKKIKYVMRH
KLTKRLYKVSNGDYEIIXTEDHSIMKLDDKSNKIIEVRPIEITKNDNLIVRYTDRTIYVSGFEXTPIKDKRIK
YVVYDIEVDSDTDEYHNFFANGLLVHN (SEQ ID NO:250)


**Figure 32**

Figure 33

Europäisches
Patentamt

European
Patent Office

Office européen
des brevets

# EUROPEAN SEARCH REPORT

Application Number

EP 19 20 1176

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | CHOI J J ET AL: "Protein Trans-splicing and Characterization of a Split Family B-type DNA Polymerase from the Hyperthermophilic Archaeal Parasite Nanoarchaeum equitans", JOURNAL OF MOLECULAR BIOLOGY, ACADEMIC PRESS, UNITED KINGDOM, vol. 356, no. 5, 10 March 2006 (2006-03-10), pages 1093-1106, XP024950739, ISSN: 0022-2836, DOI: 10.1016/J.JMB.2005.12.036 [retrieved on 2006-03-10] * page 1095, column 1, paragraph 2 - column 2, paragraph 2; figure 2 * ----- | 1-15 | INV. C07K14/005 |
| X | DATABASE UniProt [Online] 3 October 2012 (2012-10-03), Petrov V.M., Ratnayaka S., Karam J.D.: "Diversification of the DNA polymerase (gp43) of T4-related phages of Aeromonas salmonicida; INTEIN_C_TER", XP002798913, retrieved from EBI accession no. UNIPROT:I6ZIH7 Database accession no. I6ZIH7 * sequence * ----- -/-- | 1-15 | TECHNICAL FIELDS SEARCHED (IPC) C07K C12N |

~~The present search report has been drawn up for all claims~~

| Place of search | Date of completion of the search | Examiner |
|---|---|---|
| Munich | 11 May 2020 | Burkhardt, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 4

Europäisches
Patentamt

European
Patent Office

Office européen
des brevets

# EUROPEAN SEARCH REPORT

Application Number

EP 19 20 1176

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProt [Online]<br><br>3 October 2012 (2012-10-03),<br>Petrov V.M., Ratnayaka S., Karam J.D.:<br>"Diversification of the DNA polymerase (gp43) of T4-related phages of Aeromonas salmonicida; INTEIN_N_TER",<br>XP055691405,<br>retrieved from EBI accession no. UNIPROT:I6YMS9<br>Database accession no. I6YMS9<br>* sequence * | 1-15 | |
| X | KAZUO TORI ET AL: "Splicing of the Mycobacteriophage Bethlehem DnaB Intein: IDENTIFICATION OF A NEW MECHANISTIC CLASS OF INTEINS THAT CONTAIN AN OBLIGATE BLOCK F NUCLEOPHILE",<br>JOURNAL OF BIOLOGICAL CHEMISTRY,<br>vol. 285, no. 4,<br>22 January 2010 (2010-01-22), pages 2515-2526, XP055691450,<br>US<br>ISSN: 0021-9258, DOI:<br>10.1074/jbc.M109.069567<br>* page 2520, column 2, last paragraph - page 2523, column 3, paragraph 1; figures 6,7; tables 1,2 * | 1-15 | TECHNICAL FIELDS SEARCHED (IPC) |

-----

-/--

The present search report has been drawn up for all claims

| Place of search | Date of completion of the search | Examiner |
|---|---|---|
| Munich | 11 May 2020 | Burkhardt, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 19 20 1176

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | QI XINGMEI ET AL: "Alternative Nucleophilic Residues in Intein Catalysis of Protein Splicing", PROTEIN AND PEPTIDE LETTERS, vol. 18, no. 12, December 2011 (2011-12), pages 1226-1232, XP009520238, ISSN: 0929-8665 * page 1227, column 2, paragraph 2 - page 1228, column 2, last paragraph; figures 1-3; table 1 * | 1-15 | |
| A | DAI XUDONG ET AL: "Cysteine-free non-canonical C-intein for versatile protein C-terminal labeling throughtrans-splicing", APPLIED MICROBIOLOGY AND BIOTECHNOLOGY, SPRINGER BERLIN HEIDELBERG, BERLIN/HEIDELBERG, vol. 99, no. 19, 31 July 2015 (2015-07-31), pages 8151-8161, XP035541979, ISSN: 0175-7598, DOI: 10.1007/S00253-015-6796-6 [retrieved on 2015-07-31] * the whole document * | 1-15 | |
| A | WO 2015/086825 A1 (WESTFÄLISCHE WILHELMS UNIVERSITÄT MÜNSTER [DE]; YEDA RES & DEV [IL]) 18 June 2015 (2015-06-18) | 1-15 | |
| A,D | NEEL H. SHAH ET AL: "Inteins: nature's gift to protein chemists", CHEMICAL SCIENCE, vol. 5, no. 2, 1 January 2014 (2014-01-01), pages 446-461, XP055240209, United Kingdom ISSN: 2041-6520, DOI: 10.1039/C3SC52951G * page 449, column 2, last paragraph - page 451, column 2, paragraph 1 * | 1-15 | |

TECHNICAL FIELDS
SEARCHED        (IPC)

-/--

~~The present search report has been drawn up for all claims~~

| Place of search | Date of completion of the search | Examiner |
|---|---|---|
| Munich | 11 May 2020 | Burkhardt, Peter |

page 3 of 4

Europäisches
Patentamt

European
Patent Office

Office européen
des brevets

## EUROPEAN SEARCH REPORT

Application Number

EP 19 20 1176

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2016/207965 A1 (WOOD DAVID WEBSTER [US] ET AL) 21 July 2016 (2016-07-21) * paragraph [0103]; claims; examples * ----- | 1-15 | |
| T | MANIRAJ BHAGAWATI ET AL: "A mesophilic cysteine-less split intein for protein trans -splicing applications under oxidizing conditions", PROCEEDINGS OF THE NATIONAL ACADEMY OF SCIENCES, vol. 116, no. 44, 14 October 2019 (2019-10-14), pages 22164-22172, XP055691500, US ISSN: 0027-8424, DOI: 10.1073/pnas.1909825116 * the whole document * ----- | | |

TECHNICAL FIELDS
SEARCHED (IPC)

~~The present search report has been drawn up for all claims~~

| Place of search | Date of completion of the search | Examiner |
|---|---|---|
| Munich | 11 May 2020 | Burkhardt, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 4 of 4

Europäisches
Patentamt

European
Patent Office

Office européen
des brevets

**Application Number**

EP 19 20 1176

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-15(partially)

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 19 20 1176

The Search Division considers that the present European patent application does not comply with the
requirements of unity of invention and relates to several inventions or groups of inventions, namely:

```
1. claims: 1-15(partially)

      relating to a split intein that is free of cysteine and
      wherein Int-N has SEQ ID NO:1 and Int-C has SEQ ID NO:2 and
      methods and products comprising said intein
                          ---

2-65. claims: 1-15(partially)

      as invention 1 but relating to SEQ ID NOs:3, 5, 7, 9, 11,
      13, 15, 17, 19, 21, 23, 25, 27, 29, 31, 33, 35, 37, 39, 41,
      43, 45, 48, 51, 53, 55, 58, 61, 63, 65, 67, 70, 74, 76, 78,
      80, 82, 85, 87, 89, 92, 97, 100, 102, 106, 108, 111, 114,
      117, 120, 122, 124, 126, 128, 130, 132, 134, 136, 138, 140,
      142, 144, 146 or 147 (Int-N) and/or SEQ ID NOs:4, 6, 8, 10,
      12, 14, 16, 18, 20, 22, 24, 26, 28, 30, 32, 34, 36, 38, 40,
      42, 44, 46, 49, 52, 54, 56, 59, 62, 64, 66, 68, 71, 75, 77,
      79, 81, 83, 86, 88, 90, 93, 98, 101, 103, 107, 109, 112,
      115, 118, 121, 123, 125, 127, 129, 131, 133, 135, 137, 139,
      141, 143, 145, 148, 149, 150, 151 and 152 (Int-C)
                          ---
```

**EP 3 750 911 A1**

## ANNEX TO THE EUROPEAN SEARCH REPORT
## ON EUROPEAN PATENT APPLICATION NO.

EP 19 20 1176

11-05-2020

| Patent document cited in search report | | Publication date | Patent family member(s) | | Publication date |
|---|---|---|---|---|---|
| WO 2015086825 | A1 | 18-06-2015 | EP | 2883953 A1 | 17-06-2015 |
| | | | EP | 3080255 A1 | 19-10-2016 |
| | | | US | 2016319287 A1 | 03-11-2016 |
| | | | WO | 2015086825 A1 | 18-06-2015 |
| US 2016207965 | A1 | 21-07-2016 | US | 2016207965 A1 | 21-07-2016 |
| | | | US | 2020024370 A1 | 23-01-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KARLIN ; ALTSCHUL.** *Proc. Natl. Acad. Sci. USA,* 1990, vol. 87, 2264-2268
- **KARLIN ; ALTSCHUL.** *Proc. Natl. Acad. Sci. USA,* 1993, vol. 90, 5873-5877
- **CHENNA et al.** Multiple sequence alignment with the Clustal series of programs. *Nucleic Acid Research,* 2003, vol. 31, 3497-3500
- **NOTREDAME et al.** T-Coffee: A novel method for multiple sequence alignments. *J. Mol. Biol.,* 2000, vol. 302, 205-217
- **KANE PM et al.** Protein splicing converts the yeast TFP1 gene product to the 69-kD subunit of the vacuolar H(+)-adenosine triphosphatase. *Science,* 1990, vol. 250 (4981), 651-657
- **HIRATA R et al.** Molecular structure of a gene, VMA1, encoding the catalytic subunit of H(+)-translocating adenosine triphosphatase from vacuolar membranes of Saccharomyces cerevisiae. *The Journal of biological chemistry,* 1990, vol. 265 (12), 6726-6733
- **NOREN CJ ; WANG J ; PERLER FB.** Dissecting the Chemistry of Protein Splicing and Its Applications. *Angewandte Chemie (International ed,* 2000, vol. 39 (3), 450-466
- **NOVIKOVA O ; TOPILINA N ; BELFORT M.** Enigmatic distribution, evolution, and function of inteins. *The Journal of biological chemistry,* 2014, vol. 289 (21), 14490-14497
- **VOLKMANN G ; MOOTZ HD.** Recent progress in intein research: from mechanism to directed evolution and applications. *Cellular and molecular life sciences : CMLS,* 2013, vol. 70 (7), 1185-1206
- **SHAH NH ; MUIR TW.** Inteins: nature's gift to protein chemists. *Chem Sci,* 2014, vol. 5, 446-461
- **WOOD DW ; CAMARERO JA.** Intein applications: from protein purification and labeling to metabolic control methods. *The Journal of biological chemistry,* 2014, vol. 289 (21), 14512-14519
- **TOPILINA NI ; MILLS KV.** Recent advances in in vivo applications of intein-mediated protein splicing. *Mobile DNA,* 2014, vol. 5 (1), 5
- **WU H ; HU Z ; LIU XQ.** Protein trans-splicing by a split intein encoded in a split DnaE gene of Synechocystis sp. PCC6803. *Proceedings of the National Academy of Sciences of the United States of America,* 1998, vol. 95 (16), 9226-9231

- **THIEL IV ; VOLKMANN G ; PIETROKOVSKI S ; MOOTZ HD.** An atypical naturally split intein engineered for highly efficient protein labeling. *Angewandte Chemie (International ed,* 2014, vol. 53 (5), 1306-1310
- **MOOTZ HD.** Split inteins as versatile tools for protein semisynthesis. *Chembiochem,* 2009, vol. 10 (16), 2579-2589
- **ARANKO AS ; WLODAWER A ; IWAI H.** Nature's recipe for splitting inteins. *Protein engineering, design & selection : PEDS,* 2014, vol. 27 (8), 263-271
- **CALLAHAN BP ; TOPILINA NI ; STANGER MJ ; VAN ROEY P ; BELFORT M.** Structure of catalytically competent intein caught in a redox trap with functional and evolutionary implications. *Nature structural & molecular biology,* 2011, vol. 18 (5), 630-633
- **NICASTRI MC et al.** Internal disulfide bond acts as a switch for intein activity. *Biochemistry,* 2013, vol. 52 (34), 5920-5927
- **MOHLMANN S ; BRINGMANN P ; GREVEN S ; HARRENGA A.** Site-specific modification of ED-B-targeting antibody using intein-fusion technology. *BMC biotechnology,* 2011, vol. 11, 76
- **BACHMANN AL ; MOOTZ HD.** N-terminal chemical protein labeling using the naturally split GOS-TerL intein. *Journal of peptide science : an official publication of the European Peptide Society,* 2017, vol. 23 (7-8), 624-630
- **STEVENS AJ et al.** Design of a Split Intein with Exceptional Protein Splicing Activity. *Journal of the American Chemical Society,* 2016, vol. 138 (7), 2162-2165
- **MOLL JM et al.** Split(2) Protein-Ligation Generates Active IL-6-Type Hyper-Cytokines from Inactive Precursors. *ACS Synth Biol,* 2017, vol. 6 (12), 2260-2272
- **DHAR T ; MOOTZ HD.** Modification of transmembrane and GPI-anchored proteins on living cells by efficient protein trans-splicing using the Npu DnaE intein. *Chemical communications,* 2011, vol. 47 (11), 3063-3065
- **PALEI S ; BECHER KS ; NIENBERG C ; JOSE J ; MOOTZ HD.** Bacterial Cell-Surface Display of Semisynthetic Cyclic Peptides. *Chembiochem,* 2019, vol. 20 (1), 72-77

- **SCHÜTZ V ; MOOTZ HD.** Click-tag and amine-tag: chemical tag approaches for efficient protein labeling in vitro and on live cells using the naturally split Npu DnaE intein. *Angewandte Chemie (International ed,* 2014, vol. 53 (16), 4113-4117
- **LEE E ; MIN K ; CHANG YT ; KWON Y.** Efficient and wash-free labeling of membrane proteins using engineered Npu DnaE split-inteins. *Protein science : a publication of the Protein Society,* 2018, vol. 27 (9), 1568-1574
- **IWAI H ; ZUGER S ; JIN J ; TAM PH.** Highly efficient protein trans-splicing by a naturally split DnaE intein from Nostoc punctiforme. *FEBS letters,* 2006, vol. 580 (7), 1853-1858
- **ZETTLER J ; SCHÜTZ V ; MOOTZ HD.** The naturally split Npu DnaE intein exhibits an extraordinarily high rate in the protein trans-splicing reaction. *FEBS letters,* 2009, vol. 583 (5), 909-914
- **DASSA B ; AMITAI G ; CASPI J ; SCHUELER-FURMAN O ; PIETROKOVSKI S.** Trans protein splicing of cyanobacterial split inteins in endogenous and exogenous combinations. *Biochemistry,* 2007, vol. 46 (1), 322-330
- **CARVAJAL-VALLEJOS P ; PALLISSE R ; MOOTZ HD ; SCHMIDT SR.** Unprecedented rates and efficiencies revealed for new natural split inteins from metagenomic sources. *The Journal of biological chemistry,* 2012, vol. 287 (34), 28686-28696
- **TORI K et al.** Splicing of the mycobacteriophage Bethlehem DnaB intein: identification of a new mechanistic class of inteins that contain an obligate block F nucleophile. *The Journal of biological chemistry,* 2010, vol. 285 (4), 2515-2526
- **QI X ; WANG J ; MENG Q ; LIU XQ.** Alternative Nucleophilic Residues in Intein Catalysis of Protein Splicing. *Protein and peptide letters,* 2011, vol. 18 (12), 1226-1232
- **CHOI JJ et al.** Protein trans-splicing and characterization of a split family B-type DNA polymerase from the hyperthermophilic archaeal parasite Nanoarchaeum equitans. *Journal of molecular biology,* 2006, vol. 356 (5), 1093-1106
- **SOUTHWORTH MW et al.** Control of protein splicing by intein fragment reassembly. *The EMBO journal,* 1998, vol. 17 (4), 918-926
- **BRENZEL S ; CEBI M ; REISS P ; KOERT U ; MOOTZ HD.** Expanding the scope of protein trans-splicing to fragment ligation of an integral membrane protein: towards modulation of porin-based ion channels by chemical modification. *Chembiochem,* 2009, vol. 10 (6), 983-986
- **DAWSON PE ; MUIR TW ; CLARK-LEWIS I ; KENT SB.** Synthesis of proteins by native chemical ligation. *Science,* 1994, vol. 266 (5186), 776-779
- **TAMURA T ; HAMACHI I.** Chemistry for Covalent Modification of Endogenous/Native Proteins: From Test Tubes to Complex Biological Systems. *Journal of the American Chemical Society,* 2019, vol. 141 (7), 2782-2799
- **KRALL N ; DA CRUZ FP ; BOUTUREIRA O ; BERNARDES GJ.** Site-selective protein-modification chemistry for basic biology and drug development. *Nature chemistry,* 2016, vol. 8 (2), 103-113
- **GUNNOO SB ; MADDER A.** Chemical Protein Modification through Cysteine. *Chembiochem,* 2016, vol. 17 (7), 529-553
- **MASSA S et al.** Site-specific labeling of cysteine-tagged camelid single-domain antibody-fragments for use in molecular imaging. *Bioconjugate chemistry,* 2014, vol. 25 (5), 979-988
- **JUNUTULA JR et al.** Site-specific conjugation of a cytotoxic drug to an antibody improves the therapeutic index. *Nature biotechnology,* 2008, vol. 26 (8), 925-932
- **VAZQUEZ-REY M ; LANG DA.** Aggregates in monoclonal antibody manufacturing processes. *Biotechnology and bioengineering,* 2011, vol. 108 (7), 1494-1508
- **LI W et al.** Antibody Aggregation: Insights from Sequence and Structure. *antibodies,* 2016, vol. 5, 19
- **KURPIERS T ; MOOTZ HD.** Regioselective cysteine bioconjugation by appending a labeled cystein tag to a protein by using protein splicing in trans. *Angewandte Chemie (International ed,* 2007, vol. 46 (27), 5234-5237
- **KURPIERS T ; MOOTZ HD.** Site-specific chemical modification of proteins with a prelabelled cysteine tag using the artificially split Mxe GyrA intein. *Chembiochem,* 2008, vol. 9 (14), 2317-2325
- **DAI X ; XUN Q ; LIU XQ ; MENG Q.** Cysteine-free non-canonical C-intein for versatile protein C-terminal labeling through trans-splicing. *Applied microbiology and biotechnology,* 2015, vol. 99 (19), 8151-8161
- **DEVOTO AE et al.** Megaphages infect Prevotella and variants are widespread in gut microbiomes. *Nat Microbiol,* 2019, vol. 4 (4), 693-700
- **CASPI J ; AMITAI G ; BELENKIY O ; PIETROKOVSKI S.** Distribution of split DnaE inteins in cyanobacteria. *Molecular microbiology,* 2003, vol. 50 (5), 1569-1577
- **DALGAARD JZ ; MOSER MJ ; HUGHEY R ; MIAN IS.** Statistical modeling, phylogenetic analysis and structure prediction of a protein splicing domain common to inteins and hedgehog proteins. *J Comput Biol,* 1997, vol. 4 (2), 193-214
- **PIETROKOVSKI S.** Modular organization of inteins and C-terminal autocatalytic domains. *Protein science : a publication of the Protein Society,* 1998, vol. 7 (1), 64-71

- **HU Y ; LIU C ; MUYLDERMANS S.** Nanobody-Based Delivery Systems for Diagnosis and Targeted Tumor Therapy. *Front Immunol,* 2017, vol. 8, 1442
- **SCHUMACHER D ; HELMA J ; SCHNEIDER AFL ; LEONHARDT H ; HACKENBERGER CPR.** Nanobodies: Chemical Functionalization Strategies and Intracellular Applications. *Angewandte Chemie (International ed,* 2018, vol. 57 (9), 2314-2333
- **KIRCHHOFER A et al.** Modulation of protein properties in living cells using nanobodies. *Nature structural & molecular biology,* 2010, vol. 17 (1), 133-138
- **KLEIN A et al.** Nanobody-Displaying Flagellar Nanotubes. *Scientific reports,* 2018, vol. 8 (1), 3584
- **SCHMITZ KR ; BAGCHI A ; ROOVERS RC ; VAN BERGEN EN HENEGOUWEN PM ; FERGUSON KM.** Structural evaluation of EGFR inhibition mechanisms for nanobodies/VHH domains. *Structure,* 2013, vol. 21 (7), 1214-1224
- **IBACH J et al.** Single Particle Tracking Reveals that EGFR Signaling Activity Is Amplified in Clathrin-Coated Pits. *PloS one,* 2015, vol. 10 (11), e0143162
- **RICHTER D et al.** Ligand-induced type II interleukin-4 receptor dimers are sustained by rapid re-association within plasma membrane microcompartments. *Nature communications,* 2017, vol. 8, 15976
- **LOS GV et al.** HaloTag: a novel protein labeling technology for cell imaging and protein analysis. *ACS chemical biology,* 2008, vol. 3 (6), 373-382
- **KEPPLER A et al.** A general method for the covalent labeling of fusion proteins with small molecules in vivo. *Nature biotechnology,* 2003, vol. 21 (1), 86-89
- **DHAR T ; KURPIERS T ; MOOTZ HD.** Extending the scope of site-specific cysteine bioconjugation by appending a prelabeled cysteine tag to proteins using protein trans-splicing. *Methods in molecular biology,* 2011, vol. 751, 131-142
- **ANDO T ; TSUKIJI S ; TANAKA T ; NAGAMUNE T.** Construction of a small-molecule-integrated semi-synthetic split intein for in vivo protein ligation. *Chemical communications,* 2007, vol. 47, 4995-4997
- **VOLKMANN G ; LIU XQ.** Protein C-terminal labeling and biotinylation using synthetic peptide and split-intein. *PloS one,* 2009, vol. 4 (12), e8381
- **MOOTZ HD ; MUIR TW.** Protein splicing triggered by a small molecule. *Journal of the American Chemical Society,* 2002, vol. 124 (31), 9044-9045
- **THOMAS C et al.** Structural linkage between ligand discrimination and receptor activation by type I interferons. *Cell,* 2011, vol. 146 (4), 621-632
- **KUMAR KG et al.** Basal ubiquitin-independent internalization of interferon alpha receptor is prevented by Tyk2-mediated masking of a linear endocytic motif. *The Journal of biological chemistry,* 2008, vol. 283 (27), 18566-18572
- **BACHMANN AL ; MOOTZ HD.** An Unprecedented Combination of Serine and Cysteine Nucleophiles in a Split Intein with an Atypical Split Site. *The Journal of biological chemistry,* 2015, vol. 290 (48), 28792-28804
- **ARANKO AS et al.** Structure-based engineering and comparison of novel split inteins for protein ligation. *Molecular bioSystems,* 2014, vol. 10 (5), 1023-1034
- **ARANKO AS ; ZUGER S ; BUCHINGER E ; IWAI H.** In vivo and in vitro protein ligation by naturally occurring and engineered split DnaE inteins. *PloS one,* 2009, vol. 4 (4), e5185
- **APPLEBY-TAGOE JH et al.** Highly efficient and more general cis- and trans-splicing inteins through sequential directed evolution. *The Journal of biological chemistry,* 2011, vol. 286 (39), 34440-34447
- **STEVENS AJ ; SEKAR G ; GRAMESPACHER JA ; COWBURN D ; MUIR TW.** An Atypical Mechanism of Split Intein Molecular Recognition and Folding. *Journal of the American Chemical Society,* 2018, vol. 140 (37), 11791-11799
- **BRENZEL S ; MOOTZ HD.** Design of an intein that can be inhibited with a small molecule ligand. *Journal of the American Chemical Society,* 2005, vol. 127 (12), 4176-4177
- **BRANER M ; KOLLMANNSPERGER A ; WIENEKE R ; TAMPE R.** Traceless' tracing of proteins - high-affinity trans-splicing directed by a minimal interaction pair. *Chem Sci,* 2016, vol. 7 (4), 2646-2652
- **PLEINER T et al.** Nanobodies: site-specific labeling for super-resolution imaging, rapid epitope-mapping and native protein complex isolation. *eLife,* 2015, vol. 4, e11349
- **ALBRECHT D ; WINTERFLOOD CM ; EWERS H.** Dual color single particle tracking via nanobodies. *Methods Appl Fluoresc,* 2015, vol. 3 (2), 024001
- **LIU CC ; SCHULTZ PG.** Adding new chemistries to the genetic code. *Annu Rev Biochem,* 2010, vol. 79, 413-444
- **CHIN JW.** Expanding and reprogramming the genetic code. *Nature,* 2017, vol. 550, 53-60